(19) Europäisches Patentamt
European Patent Office
Office européen des brevets

(11) **EP 4 488 271 A1**

(12) **EUROPEAN PATENT APPLICATION**
published in accordance with Art. 153(4) EPC

(43) Date of publication:
**08.01.2025 Bulletin 2025/02**

(21) Application number: **23760138.0**

(22) Date of filing: **27.02.2023**

(51) International Patent Classification (IPC):
***C07D 403/04*** (2006.01) ***A61K 31/416*** (2006.01)
***A61K 31/4439*** (2006.01) ***A61K 31/444*** (2006.01)
***A61K 31/506*** (2006.01) ***A61K 31/5377*** (2006.01)
***A61P 9/00*** (2006.01) ***A61P 11/00*** (2006.01)
***A61P 11/06*** (2006.01) ***A61P 21/00*** (2006.01)
***A61P 37/08*** (2006.01) ***C07D 231/56*** (2006.01)
***C07D 401/04*** (2006.01) ***C07D 401/14*** (2006.01)
***C07D 403/14*** (2006.01) ***C07D 405/14*** (2006.01)
***C07D 409/14*** (2006.01) ***C07D 413/14*** (2006.01)
***C07D 451/04*** (2006.01) ***C07D 471/04*** (2006.01)

(52) Cooperative Patent Classification (CPC):
**A61K 31/416; A61K 31/4439; A61K 31/444; A61K 31/506; A61K 31/5377; A61P 9/00; A61P 11/00; A61P 11/06; A61P 21/00; A61P 37/08; C07D 231/56; C07D 401/04; C07D 401/14; C07D 403/04; C07D 403/14;** (Cont.)

(86) International application number:
**PCT/JP2023/006921**

(87) International publication number:
**WO 2023/163140 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2022 JP 2022029771**
**18.11.2022 JP 2022185318**

(71) Applicant: **Japan Tobacco Inc.**
**Tokyo 105-6927 (JP)**

(72) Inventors:
- **NAGAMOTO, Yuki**
**Takatsuki-shi, Osaka 569-1125 (JP)**
- **TAKAGI, Masaki**
**Takatsuki-shi, Osaka 569-1125 (JP)**
- **MATSUMURA, Koji**
**Takatsuki-shi, Osaka 569-1125 (JP)**
- **ITO, Hirotsugu**
**Takatsuki-shi, Osaka 569-1125 (JP)**
- **ITO, Keisuke**
**Takatsuki-shi, Osaka 569-1125 (JP)**
- **OYAMA, Yuki**
**Takatsuki-shi, Osaka 569-1125 (JP)**

(74) Representative: **Vossius & Partner Patentanwälte Rechtsanwälte mbB**
**Siebertstrasse 3**
**81675 München (DE)**

(54) **INDAZOLE COMPOUND AND PHARMACEUTICAL USE THEREOF**

(57) The present invention aims to provide a compound having H-PGDS inhibitory activity. The present invention relates to a compound of the formula [I]:

EP 4 488 271 A1

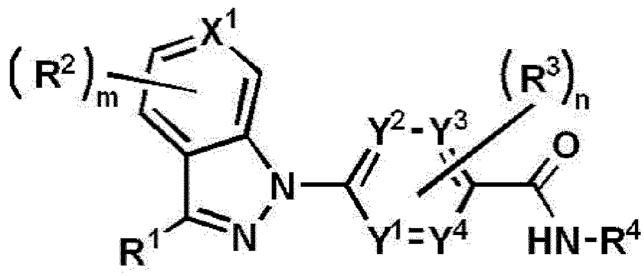

[I]

wherein each symbol is as defined in the DESCRIPTION, or a pharmaceutically acceptable salt thereof.

(52) Cooperative Patent Classification (CPC): (Cont.)
**C07D 405/14; C07D 409/14; C07D 413/14; C07D 451/04; C07D 471/04**

## Description

[Technical Field]

The present invention relates to an indazole compound or a pharmaceutically acceptable salt thereof having hematopoietic prostaglandin D synthase (hereinafter to be abbreviated as "H-PGDS") inhibitory activity, a pharmaceutical composition containing the same, a pharmaceutical use thereof, and the like.

[Background Art]

Prostaglandin D synthase (PGDS) is an enzyme that synthesizes prostaglandin $D_2$ ($PGD_2$) by using prostaglandin $H_2$ ($PGH_2$) as a substrate. There are two types of PGDS: hematopoietic PGDS (H-PGDS) and lipocalin-type PGDS (L-PGDS). These two types of enzymes show no homology in amino acid sequence, and are different in tissue distribution and cell localization. H-PGDS is predominantly expressed in mast cells and hematopoietic cells such as neutrophils and macrophages, and is involved in allergy and inflammation. On the other hand, expression of L-PGDS is mainly found in the central nervous system and L-PGDS plays a role in sleep regulation and pain regulation.
The use of the H-PGDS inhibitor is described below.

(1) Peripheral arterial disease (PAD) and cardiovascular disease (CAD)

In the femoral artery ligation model, which is a PAD model, an increase in lower leg blood flow was observed in H-PGDS knockout mouse as compared with wild-type mouse, suggesting that collateral blood flow pathways are involved in this increase in the lower leg blood flow (Patent Document 1). In addition, it has been reported that some PAD patients having well-developed collateral blood flow pathways show higher walking function (Non-patent Document 1). Furthermore, collateral blood flow pathways are also present in the heart, and some CAD patients having well-developed collateral blood flow pathways have been reported to show better prognosis (Non-patent Document 2). Based on these findings, H-PGDS inhibitors are expected to achieve therapeutic effects on PAD and CAD by increasing the blood flow to lower legs and heart via collateral blood flow pathways. PAD is classified into mild intermittent claudication (IC: Intermittent Claudication) and severe comprehensive chronic limb threatening ischemia (CLTI: Chronic Limb Threatening Ischemia), and H-PGDS inhibitors are expected to provide therapeutic drugs for these disease conditions (Non-patent Documents 8, 9).
Peripheral arterial disease is also called chronic arterial occlusive disease or arteriosclerosis obliterans.

(2) Allergic asthma and chronic obstructive pulmonary disease

It has been reported that the D-prostanoid (DP) receptor, which is the receptor for $PGD_2$, and the chemo-triggered receptor-like molecule (CRTH2) receptor expressed in Th2 cells are involved in allergic asthma and chronic obstructive pulmonary diseases (COPD). In an asthma model, reduced allergic asthma symptoms such as reduced infiltration of eosinophils and lymphocytes into the lungs and decreased airway hyperresponsiveness have been found in DP receptor knockout mice, as compared with wild-type mice (Non-patent Document 3). It has also been reported that $PGD_2$ exacerbates inflammation by recruiting eosinophils into the airways via the CRTH2 receptor (Non-patent Document 4), and that CRTH2 receptor inhibitors show bronchial inflammation improving effects in COPD model mouse (Non-patent Document 5). From these findings, H-PGDS inhibitors are expected to show therapeutic effects on allergic asthma and COPD by suppressing $PGD_2$ production and suppressing inflammation in the airways.

(3) Duchenne muscular dystrophy

It has been reported that the expression of H-PGDS increases in the muscle tissue of Duchenne muscular dystrophy (DMD) patients and mdx mice, which are DMD model mice (Non-patent Document 6). Furthermore, it has also been reported that the H-PGDS inhibitor HQL-79 suppresses myonecrosis and increases muscle strength in mdx mice (Non-patent Document 7). From these findings, H-PGDS inhibitors may also show therapeutic effects on DMD.

(4) Allergic rhinitis

In an animal model of allergic rhinitis, it has been reported that the PGD2 concentration in nasal lavage fluid increases and the administration of an H-PGDS inhibitor improves nasal obstruction (Non-patent Document 10). From the above results, H-PGDS inhibitors are considered to provide therapeutic agents for allergic rhinitis.

(5) Sarcopenia

In sarcopenia model animals, a muscle atrophy suppressive action by the administration of H-PGDS inhibitors has been reported (Patent Document 2). From the above results, H-PGDS inhibitors are considered to provide therapeutic agents for sarcopenia.

[Document List]

[Patent Document]

[Patent Document 1] WO 2011/052628
[Patent Document 2] WO 2019/203296

[Non-patent Document]

[Non-patent Document 1] McDermott MM, et al., JACC Cardiovasc Imaging. 2013 Jun; 6(6):687-94.
[Non-patent Document 2] Steg PG, et al., Circulation. 2010 Jun 29; 121(25):2724-30.
[Non-patent Document 3] Matsuoka T, et al., Science. 2000 Mar 17; 287(5460):2013-7.
[Non-patent Document 4] George L, et al., Ther Adv Chronic Dis. 2016 Jan; 7(1):34-51.
[Non-patent Document 5] Sargent C, et al., Br J Pharmacol 7 (2009): 003P.
[Non-patent Document 6] Okinaga T, et al., Acta Neuropathol. 2002 Oct; 104(4):377-84.
[Non-patent Document 7] Mohri I, et al., Am J Pathol. 2009 May; 174(5):1735-44.
[Non-patent Document 8] Hamburg NM, et al., Circulation J 2017; 81:281-289
[Non-patent Document 9] Tran B, Heart 2021; 107: 1835-1843
[Non-patent Document 10] Kajiwara D, et al., European Journal of Pharmacology 667 (2011) 389-395.

[Summary of Invention]

The present invention provides an indazole compound having H-PGDS inhibitory activity or a pharmaceutically acceptable salt thereof, a pharmaceutical composition containing the same, a pharmaceutical use thereof, and the like. Accordingly, the present invention provides the following.

[Item 1]

A compound selected from the group consisting of the following structural formulas:

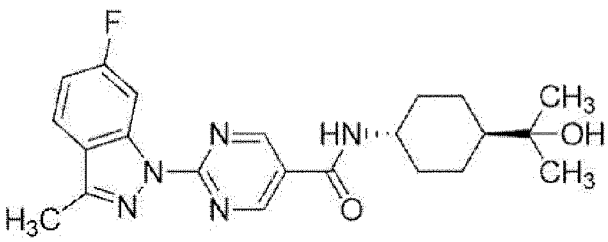

、

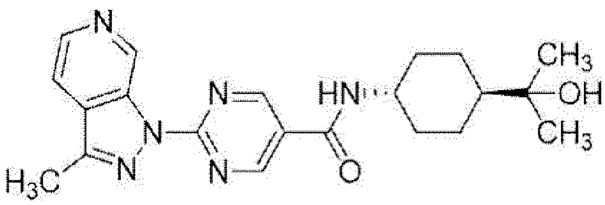

、

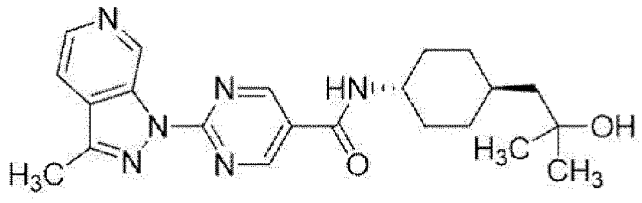
,

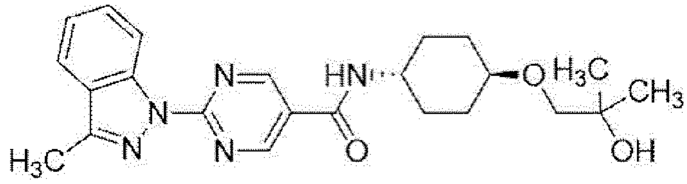
,

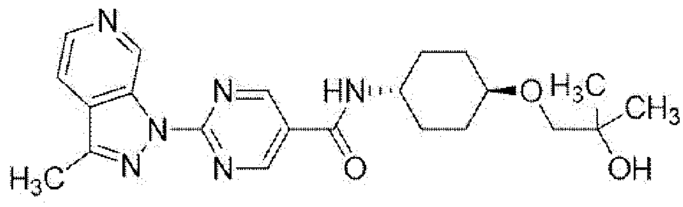
,

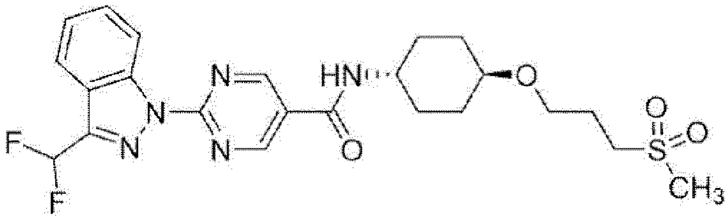
,

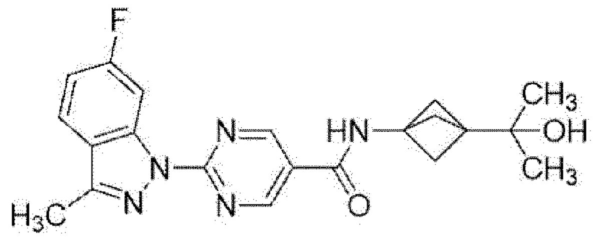
,

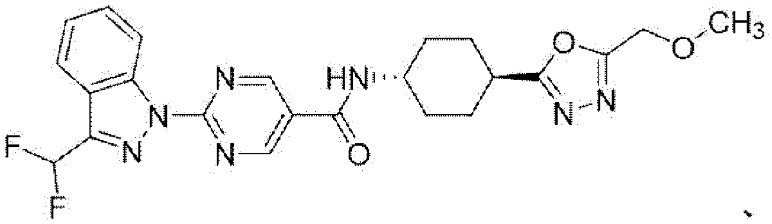
,

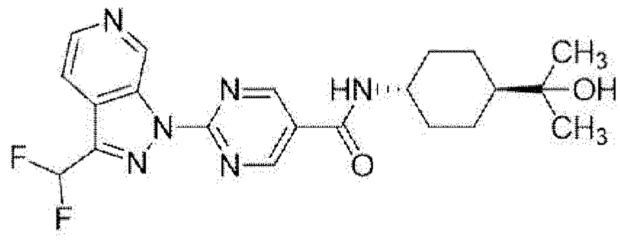

and

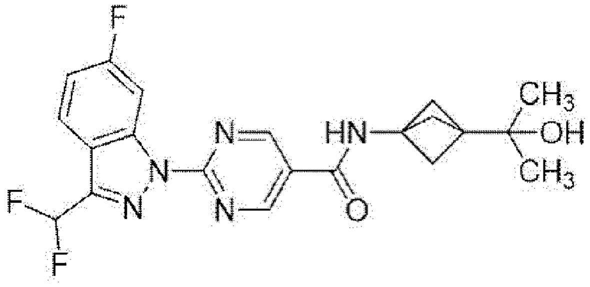

,

or a pharmaceutically acceptable salt thereof.

[Item 2]

A pharmaceutical composition comprising the compound of Item 1 or a pharmaceutically acceptable salt thereof, and a pharmaceutically acceptable carrier.

[Item 3]

An H-PGDS inhibitor comprising the compound of Item 1 or a pharmaceutically acceptable salt thereof.

[Item 4]

A therapeutic or prophylactic agent for a disease selected from the group consisting of peripheral arterial diseases, cardiovascular diseases, allergic asthma, chronic obstructive pulmonary diseases, and Duchenne muscular dystrophy, the agent comprising the compound of Item 1 or a pharmaceutically acceptable salt thereof.

[Item 5]

A method for inhibiting H-PGDS in a mammal, comprising administering a pharmaceutically effective amount of the compound of Item 1 or a pharmaceutically acceptable salt thereof to the mammal.

[Item 6]

A method for treating or preventing a disease selected from the group consisting of peripheral arterial diseases, cardiovascular diseases, allergic asthma, chronic obstructive pulmonary diseases, and Duchenne muscular dystrophy in a mammal, comprising administering a pharmaceutically effective amount of the compound of Item 1 or a pharmaceutically acceptable salt thereof to the mammal.

[Item 7]

Use of the compound of Item 1 or a pharmaceutically acceptable salt thereof in producing an H-PGDS inhibitor.

[Item 8]

Use of the compound of Item 1 or a pharmaceutically acceptable salt thereof in producing a therapeutic or

prophylactic agent for a disease selected from the group consisting of peripheral arterial diseases, cardiovascular diseases, allergic asthma, chronic obstructive pulmonary diseases, and Duchenne muscular dystrophy.

[Item 9]

The compound of Item 1 or a pharmaceutically acceptable salt thereof for use in inhibiting H-PGDS.

[Item 10]

The compound of Item 1 or a pharmaceutically acceptable salt thereof for use in the treatment or prophylaxis of a disease selected from the group consisting of peripheral arterial diseases, cardiovascular diseases, allergic asthma, chronic obstructive pulmonary diseases, and Duchenne muscular dystrophy.

[Item 11]

A commercial package comprising the pharmaceutical composition of Item 2 and a written matter associated therewith, the written matter stating that the pharmaceutical composition can be used for treatment or prophylaxis of a disease selected from the group consisting of peripheral arterial diseases, cardiovascular diseases, allergic asthma, chronic obstructive pulmonary diseases, and Duchenne muscular dystrophy.

[Item 12]

A kit comprising the pharmaceutical composition of Item 2 and a written matter associated therewith, the written matter stating that the pharmaceutical composition can be used for treatment or prophylaxis of a disease selected from the group consisting of peripheral arterial diseases, cardiovascular diseases, allergic asthma, chronic obstructive pulmonary diseases, and Duchenne muscular dystrophy.

[Item 1A]

A compound of the formula [II]:

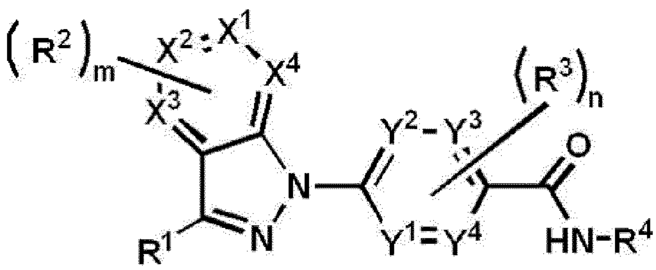

[II]

wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2),
m is 0, 1 or 2,
n is 0, 1 or 2,
$R^1$ is

(1) hydroxy,
(2) cyano,
(3) $C_{1-4}$ alkyl (wherein the alkyl is optionally substituted by hydroxy or $C_{1-4}$ alkoxy),
(4) $C_{1-4}$ alkoxy,
(5) halogen,
(6) $C_{1-4}$ haloalkyl, or
(7) $C_{3-6}$ cycloalkyl,

$R^2$ in the number of m are each independently

(1) cyano,
(2) $C_{1-4}$ alkyl,
(3) $C_{1-4}$ alkoxy,
(4) halogen, or
(5) $C_{1-4}$ haloalkyl,

$R^3$ in the number of n are each independently

(1) $C_{1-4}$ alkyl,
(2) $C_{1-4}$ alkoxy, or
(3) halogen, and

$R^4$ is

(1) ring Cy [wherein the ring Cy is

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

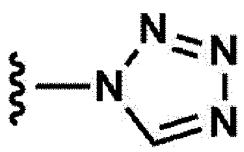
),

(V) $C_{1-6}$ alkoxy (wherein the alkoxy is optionally substituted by hydroxy or $-SO_2$-$C_{1-4}$ alkyl),
(VI) carboxy,
(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or -CO-$C_{1-6}$ alkyl (wherein the alkyl is optionally substituted by cyano)},
(IX) -O-$C_{1-6}$ haloalkyl,
(X) a group represented by the formula:

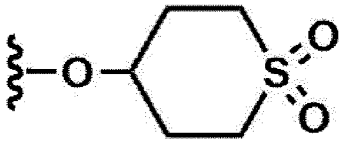,

(XI) a group represented by the formula:

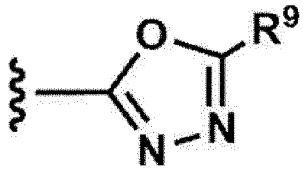

{wherein $R^9$ is $C_{1-4}$ alkyl (wherein the alkyl is optionally substituted by $C_{1-4}$ alkoxy)}, and
(XII) a group represented by the formula:

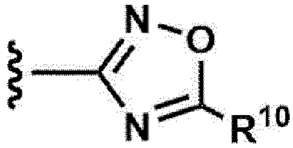

(wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

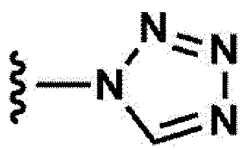),

(III) $C_{1-6}$ alkoxy (wherein the alkoxy is optionally substituted by hydroxy or $-SO_2-C_{1-4}$ alkyl),
(IV) carboxy,
(V) $-CO-C_{1-4}$ alkoxy,
(VI) $-O-C_{1-6}$ haloalkyl, or
(VII) a group represented by the formula:

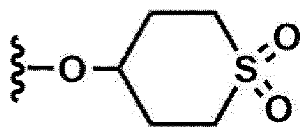},

(c) 5- or 6-membered heterocycloalkyl containing one nitrogen atom [wherein the heterocycloalkyl is optionally substituted by 1 to 3 substituents independently selected from the group consisting of

(I) oxo,

(II) $C_{1-6}$ alkyl,
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

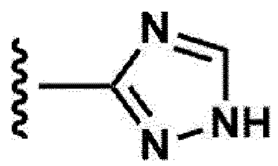

],

(d) 8-membered bridged heterocycloalkyl containing one nitrogen atom {wherein the bridged heterocycloalkyl is optionally substituted by -CO-$R^{11}$},
(e) 7- to 11-membered spiro heterocycloalkyl containing one nitrogen atom {wherein the spiro heterocycloalkyl is optionally substituted by -CO-$R^{11}$},
(f) 6- to 9-membered saturated or partially unsaturated fused ring group containing 1 or 2 nitrogen atoms (wherein the fused ring group is optionally substituted by -CO-$R^{11}$),
(g) a group represented by the formula:

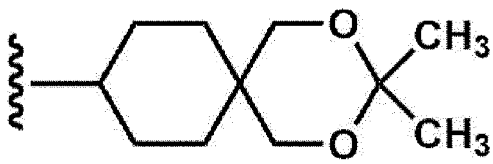

,

(h) a group represented by the formula:

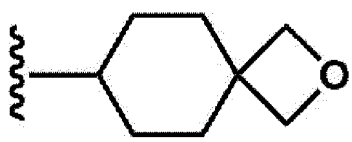

,

or
(i) a group represented by the formula:

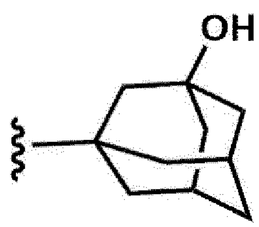

],

(2) $C_{1-4}$ alkyl {wherein the alkyl is optionally substituted by

(a) $C_{3-6}$ cycloalkyl (wherein the cycloalkyl is optionally substituted by 1 or 2 substituents independently selected from the group consisting of hydroxy and $C_{1-4}$ alkyl optionally substituted by hydroxy), or
(b) phenyl}, or

(3) $C_{1-4}$ haloalkyl (wherein the haloalkyl is optionally substituted by $C_{3-6}$ cycloalkyl optionally substituted by hydroxy)],

or a pharmaceutically acceptable salt thereof.

[Item 2A]

The compound of Item 1A, which is represented by the formula [I]:

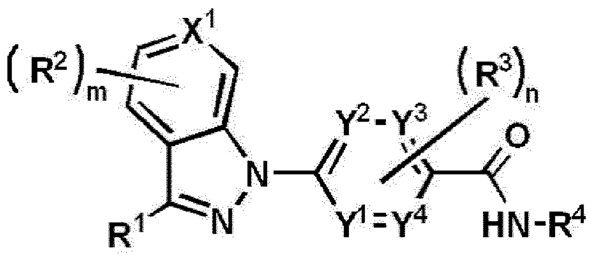

[I]

(wherein each symbol is as defined for Item 1A),
or a pharmaceutically acceptable salt thereof.

[Item 3A]

The compound of Item 1A or 2A, wherein the total number of the nitrogen atoms for $Y^1$, $Y^2$, $Y^3$ and $Y^4$ is 1 or 2, or a pharmaceutically acceptable salt thereof.

[Item 4A]

The compound of Item 1A, which is represented by the formula [IA]:

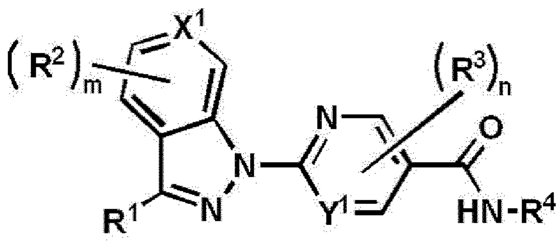

[IA]

(wherein each symbol is as defined for Item 1A),
or a pharmaceutically acceptable salt thereof.

[Item 5A]

The compound of any one of Items 1A to 4A, wherein $R^3$ is halogen, or a pharmaceutically acceptable salt thereof.

[Item 6A]

The compound of any one of Items 1A to 5A, wherein $R^4$ is ring Cy, or a pharmaceutically acceptable salt thereof.

[Item 7A]

The compound of Item 1A, which is represented by the formula [IB]:

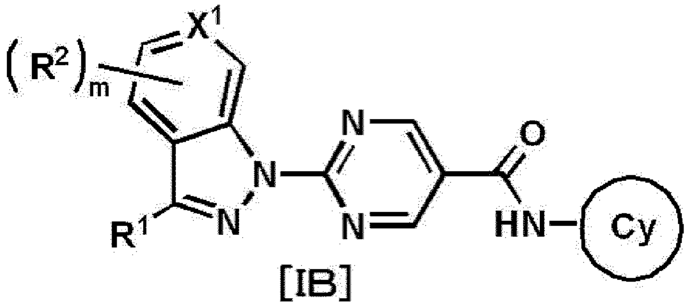

[IB]

(wherein each symbol is as defined for Item 1A),
or a pharmaceutically acceptable salt thereof.

[Item 8A]

The compound of any one of Items 1A to 7A, wherein $R^1$ is

(1) $C_{1-4}$ alkyl (wherein the alkyl is optionally substituted by hydroxy or $C_{1-4}$ alkoxy),
(2) $C_{1-4}$ alkoxy,
(3) halogen, or
(4) $C_{1-4}$ haloalkyl,

or a pharmaceutically acceptable salt thereof.

[Item 9A]

The compound of any one of Items 1A to 8A, wherein $R^2$ is halogen, or a pharmaceutically acceptable salt thereof.

[Item 10A]

The compound of any one of Items 1A to 9A, wherein ring Cy is

(1) $C_{4-6}$ cycloalkyl (wherein the cycloalkyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) $-CONH_2$,
(IV) -CO-$C_{1-4}$ alkoxy,
(V) $-SO_2-C_{1-4}$ alkyl, or
(VI) a group represented by the formula:

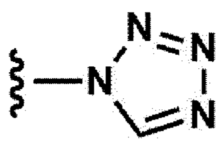
),

(e) $C_{1-6}$ alkoxy (wherein the alkoxy is optionally substituted by hydroxy or $-SO_2-C_{1-4}$ alkyl),
(f) carboxy,
(g) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl, or $-CO-C_{1-6}$ alkyl (wherein the alkyl is optionally substituted by cyano)},
(i) $-O-C_{1-6}$ haloalkyl,
(j) a group represented by the formula:

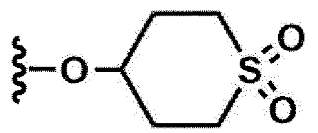

(k) a group represented by the formula:

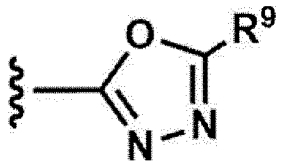

{wherein $R^9$ is $C_{1-4}$ alkyl (wherein the alkyl is optionally substituted by $C_{1-4}$ alkoxy)}, and
(m) a group represented by the formula:

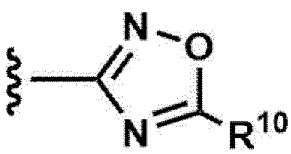

wherein $R^{10}$ is $C_{1-4}$ alkyl)),

(2) $C_{5-8}$ bridged cycloalkyl {wherein the bridged cycloalkyl is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

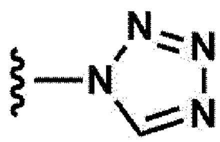 ),

(c) $C_{1-6}$ alkoxy (wherein the alkoxy is optionally substituted by hydroxy or $-SO_2-C_{1-4}$ alkyl),
(d) carboxy,
(e) $-CO-C_{1-4}$ alkoxy,
(f) $-O-C_{1-6}$ haloalkyl, or
(g) a group represented by the formula:

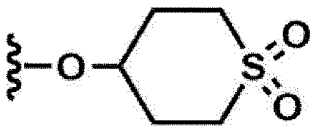 },

(3) 5- or 6-membered heterocycloalkyl containing one nitrogen atom [wherein the heterocycloalkyl is optionally substituted by 1 to 3 substituents independently selected from the group consisting of

(a) oxo,
(b) $C_{1-6}$ alkyl,
(c) $-CO-R^{11}$ {wherein $R^{11}$ is

(I) $C_{1-6}$ alkyl (wherein the alkyl is optionally substituted by

(i) hydroxy,
(ii) cyano, or
(iii) $C_{1-4}$ alkoxy),

(II) $C_{1-6}$ alkoxy,
(III) $C_{1-6}$ haloalkyl,
(IV) $C_{3-6}$ cycloalkyl (wherein the cycloalkyl is optionally substituted by halogen), or
(V) 4- to 6-membered heterocycloalkyl containing one oxygen atom (wherein the heterocycloalkyl is optionally substituted by halogen)}, and

(d) a group represented by the formula:

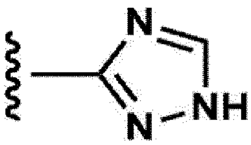 ],

(4) 8-membered bridged heterocycloalkyl containing one nitrogen atom {wherein the bridged heterocycloalkyl is optionally substituted by $-CO-R^{11}$},
(5) 7- to 11-membered spiro heterocycloalkyl containing one nitrogen atom {wherein the spiro heterocycloalkyl is optionally substituted by $-CO-R^{11}$}, or
(6) a 6- to 9-membered saturated or partially unsaturated fused ring group containing 1 or 2 nitrogen atoms (wherein the fused ring group is optionally substituted by $-CO-R^{11}$),

or a pharmaceutically acceptable salt thereof.

[Item 11A]

The compound of any one of Items 1A to 9A, wherein ring Cy is

(1) $C_{4-6}$ cycloalkyl (wherein the cycloalkyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

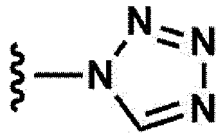
),

(e) $C_{1-6}$ alkoxy (wherein the alkoxy is optionally substituted by hydroxy or $-SO_2-C_{1-4}$ alkyl),
(f) carboxy,
(g) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl, or $-CO-C_{1-6}$ alkyl (wherein the alkyl is optionally substituted by cyano)},
(i) $-O-C_{1-6}$ haloalkyl,
(j) a group represented by the formula:

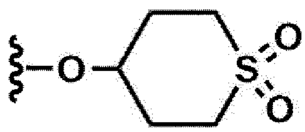
,

(k) a group represented by the formula:

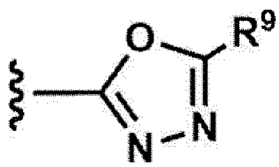

{wherein $R^9$ is $C_{1-4}$ alkyl (wherein the alkyl is optionally substituted by $C_{1-4}$ alkoxy)}, and
(m) a group represented by the formula:

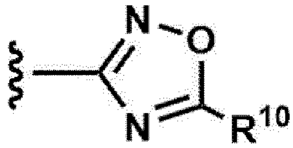

wherein $R^{10}$ is $C_{1-4}$ alkyl)),

(2) $C_{5-8}$ bridged cycloalkyl {wherein the bridged cycloalkyl is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) -CO-$C_{1-4}$ alkoxy,
(V) $-SO_2$-$C_{1-4}$ alkyl, or
(VI) a group represented by the formula:

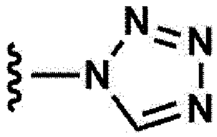

),

(c) $C_{1-6}$ alkoxy (wherein the alkoxy is optionally substituted by hydroxy or $-SO_2$-$C_{1-4}$ alkyl),
(d) carboxy,
(e) -CO-$C_{1-4}$ alkoxy,
(f) -O-$C_{1-6}$ haloalkyl, or
(g) a group represented by the formula:

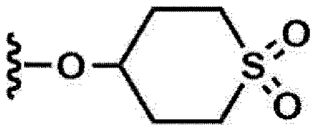

},

(3) 5- or 6-membered heterocycloalkyl containing one nitrogen atom [wherein the heterocycloalkyl is optionally substituted by 1 to 3 substituents independently selected from the group consisting of

(a) oxo,
(b) $C_{1-6}$ alkyl,
(c) -CO-$R^{11}$ {wherein $R^{11}$ is

(I) $C_{1-6}$ alkyl (wherein the alkyl is optionally substituted by

(i) hydroxy,
(ii) cyano, or
(iii) $C_{1-4}$ alkoxy),

(II) $C_{1-6}$ alkoxy,
(III) $C_{1-6}$ haloalkyl,
(IV) $C_{3-6}$ cycloalkyl (wherein the cycloalkyl is optionally substituted by halogen), or
(V) 4- to 6-membered heterocycloalkyl containing one oxygen atom (wherein the heterocycloalkyl is optionally substituted by halogen)}, and

(d) a group represented by the formula:

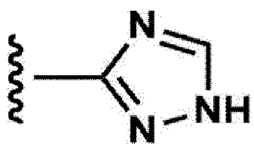

],

or

(4) 8-membered bridged heterocycloalkyl containing one nitrogen atom {wherein the bridged heterocycloalkyl is optionally substituted by -CO-$R^{11}$},

or a pharmaceutically acceptable salt thereof.

[Item 12A]

The compound of any one of Items 1A to 9A, wherein ring Cy is

(1) $C_{4-6}$ cycloalkyl (wherein the cycloalkyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) $-CONH_2$,
(IV) -CO-$C_{1-4}$ alkoxy,
(V) $-SO_2-C_{1-4}$ alkyl, or
(VI) a group represented by the formula:

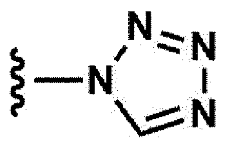 ),

(e) $C_{1-6}$ alkoxy (wherein the alkoxy is optionally substituted by hydroxy or $-SO_2-C_{1-4}$ alkyl),
(f) carboxy,
(g) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl, or -CO-$C_{1-6}$ alkyl (wherein the alkyl is optionally substituted by cyano)},
(i) -O-$C_{1-6}$ haloalkyl,
(j) a group represented by the formula:

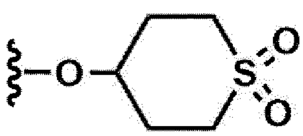 ,

(k) a group represented by the formula:

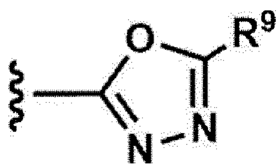

{wherein $R^9$ is $C_{1-4}$ alkyl (wherein the alkyl is optionally substituted by $C_{1-4}$ alkoxy)}, and
(m) a group represented by the formula:

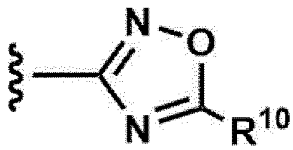

wherein $R^{10}$ is $C_{1-4}$ alkyl)), or

(2) $C_{5-8}$ bridged cycloalkyl {wherein the bridged cycloalkyl is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) -CO-$C_{1-4}$ alkoxy,
(V) $-SO_2-C_{1-4}$ alkyl, or
(VI) a group represented by the formula:

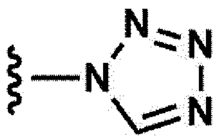 ),

(c) $C_{1-6}$ alkoxy (wherein the alkoxy is optionally substituted by hydroxy or $-SO_2-C_{1-4}$ alkyl),
(d) carboxy,
(e) -CO-$C_{1-4}$ alkoxy,
(f) -O-$C_{1-6}$ haloalkyl, or
(g) a group represented by the formula:

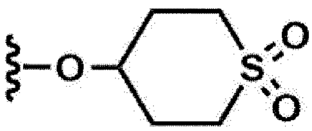 },

or a pharmaceutically acceptable salt thereof.

[Item 13A]

The compound of any one of Items 1A to 9A, wherein ring Cy is

(1) cyclohexyl (wherein the cyclohexyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) $-CONH_2$,
(IV) -CO-$C_{1-4}$ alkoxy,
(V) $-SO_2-C_{1-4}$ alkyl, or
(VI) a group represented by the formula:

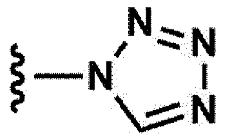
),

(e) $C_{1-6}$ alkoxy (wherein the alkoxy is optionally substituted by hydroxy or $-SO_2-C_{1-4}$ alkyl),
(f) carboxy,
(g) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl, or $-CO-C_{1-6}$ alkyl (wherein the alkyl is optionally substituted by cyano)},
(i) $-O-C_{1-6}$ haloalkyl,
(j) a group represented by the formula:

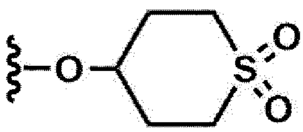
,

(k) a group represented by the formula:

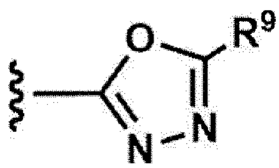

{wherein $R^9$ is $C_{1-4}$ alkyl (wherein the alkyl is optionally substituted by $C_{1-4}$ alkoxy)}, and
(m) a group represented by the formula:

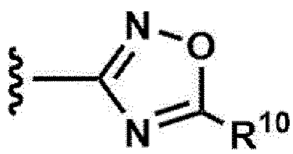

wherein $R^{10}$ is $C_{1-4}$ alkyl)), or

(2) a group represented by the formula:

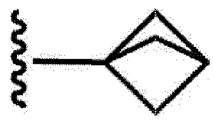

{wherein the group represented by the formula is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

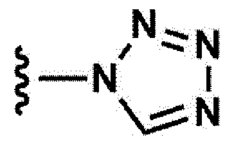
),

(c) $C_{1-6}$ alkoxy (wherein the alkoxy is optionally substituted by hydroxy or $-SO_2-C_{1-4}$ alkyl),
(d) carboxy,
(e) $-CO-C_{1-4}$ alkoxy,
(f) $-O-C_{1-6}$ haloalkyl, or
(g) a group represented by the formula:

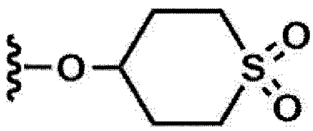
},

or a pharmaceutically acceptable salt thereof.

[Item 14A]

The compound of any one of Items 1A to 9A, wherein ring Cy is

(1) a group represented by the formula:

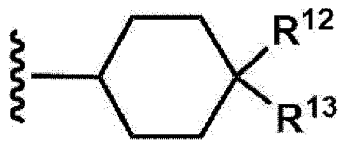

wherein $R^{12}$ and $R^{13}$ are each independently

(a) hydrogen,
(b) hydroxy,
(c) cyano,
(d) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

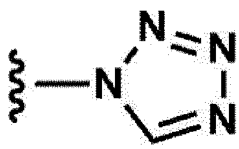
),

(e) $C_{1-6}$ alkoxy (wherein the alkoxy is optionally substituted by hydroxy or $-SO_2-C_{1-4}$ alkyl),

(f) carboxy,
(g) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) -$NR^7R^8$ {wherein $R^7$ and $R^8$ are each independently $C_{1-4}$ alkyl or -CO-$C_{1-6}$ alkyl (wherein the alkyl is optionally substituted by cyano)},
(i) -O-$C_{1-6}$ haloalkyl,
(j) a group represented by the formula:

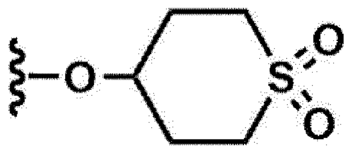

(k) a group represented by the formula:

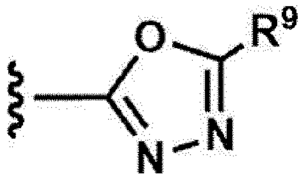

{wherein $R^9$ is $C_{1-4}$ alkyl (wherein the alkyl is optionally substituted by $C_{1-4}$ alkoxy)}, or
(m) a group represented by the formula:

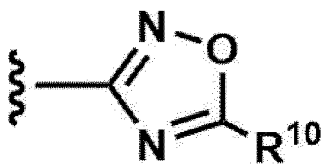

wherein $R^{10}$ is $C_{1-4}$ alkyl), or $R^{12}$ and $R^{13}$ are optionally joined to form oxo), or

(2) a group represented by the formula:

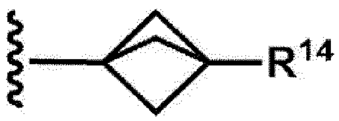

wherein $R^{14}$ is

(a) hydrogen,
(b) hydroxy,
(c) $C_{1-6}$ alkyl (wherein the alkyl is optionally substituted by

(I) hydroxy,
(II) carboxy,
(III) -$CONH_2$,
(IV) -CO-$C_{1-4}$ alkoxy,

(V) $-SO_2-C_{1-4}$ alkyl, or

(VI) a group represented by the formula:

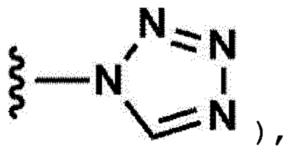

(d) $C_{1-6}$ alkoxy (wherein the alkoxy is optionally substituted by hydroxy or $-SO_2-C_{1-4}$ alkyl),

(e) carboxy,

(f) $-CO-C_{1-4}$ alkoxy,

(g) $-O-C_{1-6}$ haloalkyl, or

(h) a group represented by the formula:

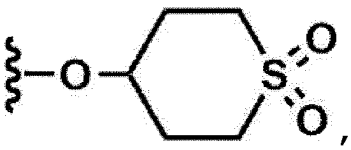

or a pharmaceutically acceptable salt thereof.

[Item 15A]

The compound of Item 1A or 14A, which is represented by the formula [IC]:

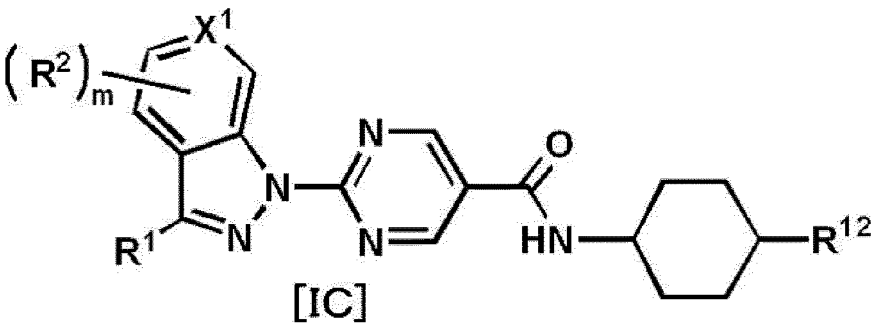

[IC]

wherein

$X^1$, $R^1$, $R^2$ and m are as defined for Item 1A, and
$R^{12}$ is as defined for Item 14A,
or a pharmaceutically acceptable salt thereof.

[Item 16A]

The compound of Item 15A, wherein
$R^{12}$ is

(1) hydroxy,

(2) cyano,

(3) $C_{1-6}$ alkyl (wherein the alkyl is optionally substituted by

(a) hydroxy,

(b) carboxy,

(c) $-CONH_2$,

(d) $-CO-C_{1-4}$ alkoxy,

(e) $-SO_2-C_{1-4}$ alkyl, or

(f) a group represented by the formula:

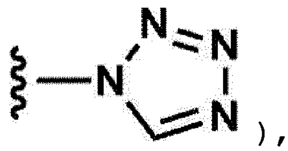

(4) $C_{1-6}$ alkoxy (wherein the alkoxy is optionally substituted by hydroxy or $-SO_2-C_{1-4}$ alkyl),
(5) $-CO-NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],
(6) $-NR^7R^8$ {wherein $R^7$ and $R^8$ are each independently $C_{1-4}$ alkyl or $-CO-C_{1-6}$ alkyl (wherein the alkyl is optionally substituted by cyano) },
(7) a group represented by the formula:

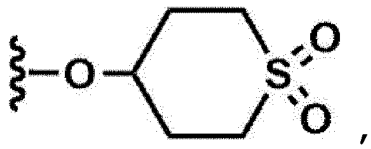

(8) a group represented by the formula:

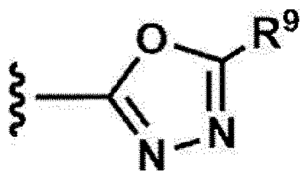

{wherein $R^9$ is $C_{1-4}$ alkyl (wherein the alkyl is optionally substituted by $C_{1-4}$ alkoxy)}, or
(9) a group represented by the formula:

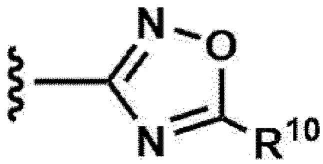

wherein $R^{10}$ is $C_{1-4}$ alkyl,
or a pharmaceutically acceptable salt thereof.

[Item 17A]

The compound of Item 1A or 14A, which is represented by the formula [ID]:

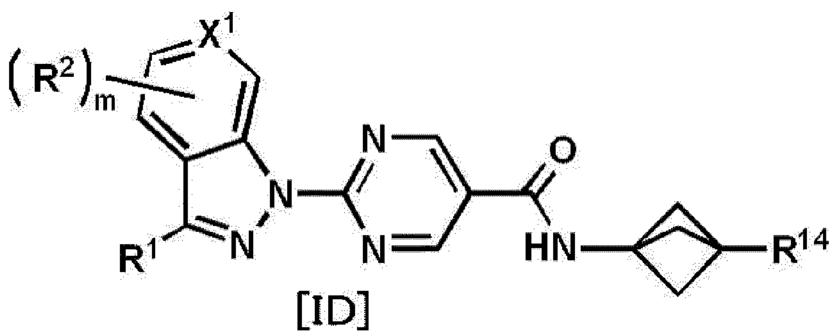

[ID]

wherein

$X^1$, $R^1$, $R^2$ and m are as defined for Item 1A, and
$R^{14}$ is as defined for Item 14A,
or a pharmaceutically acceptable salt thereof.

[Item 18A]

The compound of Item 17A, wherein $R^{14}$ is $C_{1-6}$ alkyl (wherein the alkyl is optionally substituted by hydroxy), or a pharmaceutically acceptable salt thereof.

[Item 19A]

The compound of Item 1A which is selected from the group consisting of the following structural formulas:

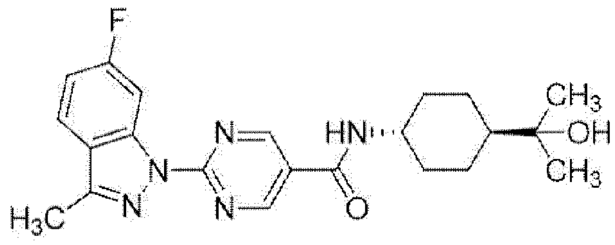

,

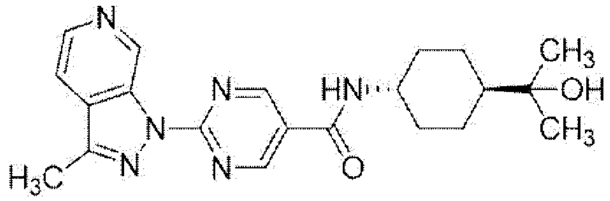

,

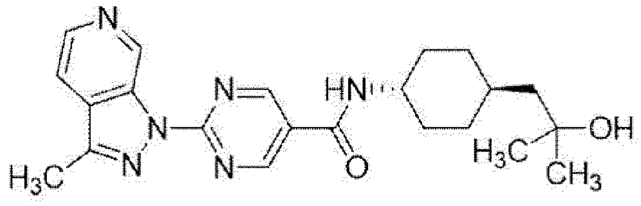

,

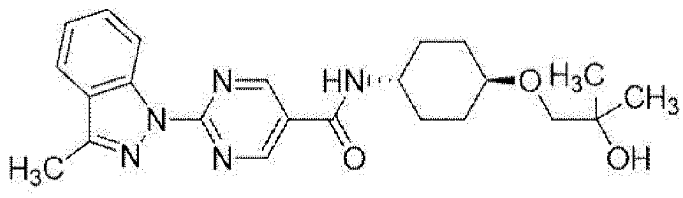

,

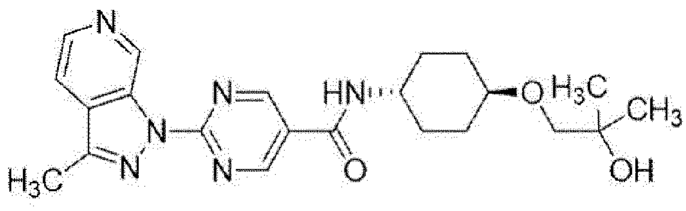

,

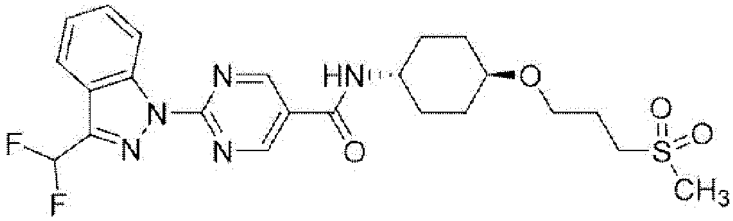

,

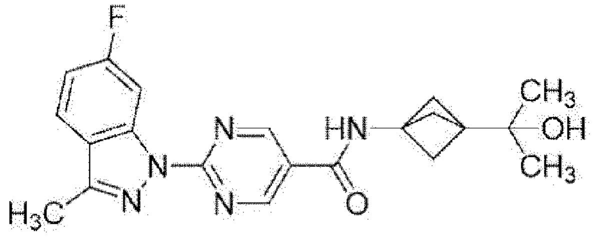

,

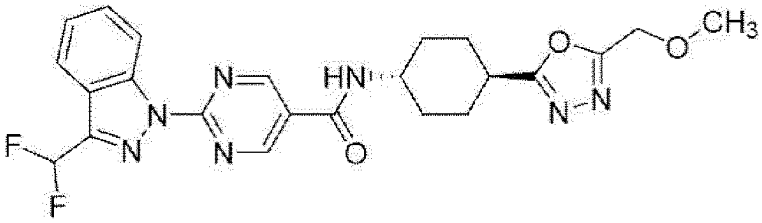

,

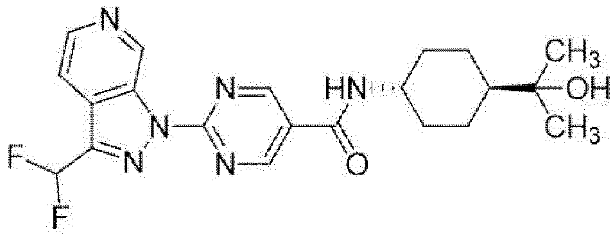

,

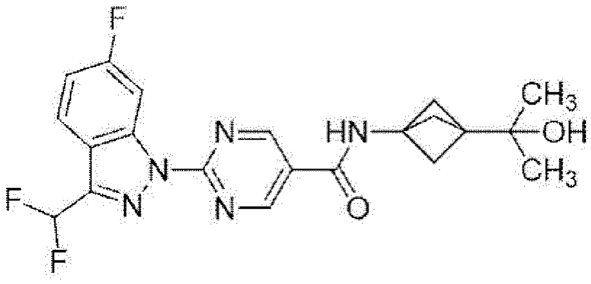

,

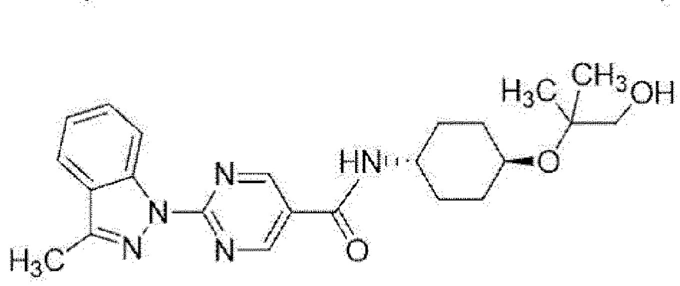

,

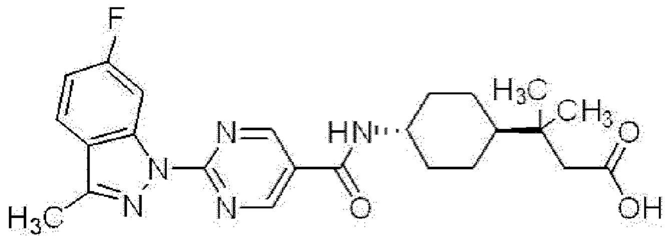

and

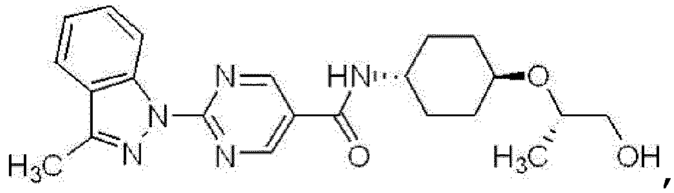

or a pharmaceutically acceptable salt thereof.

[Item 20A]

A compound of the formula [III]:

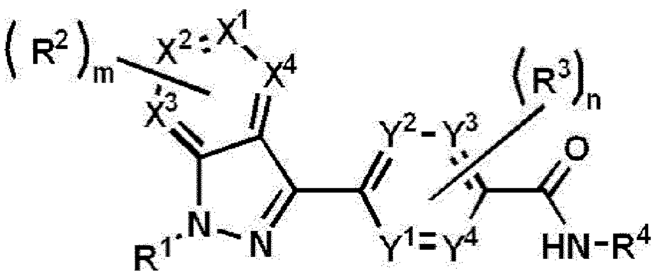

[III]

{wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2), and other symbols are as defined for Item 1A},
or a pharmaceutically acceptable salt thereof.

[Item 21A]

A compound of the formula [IV]:

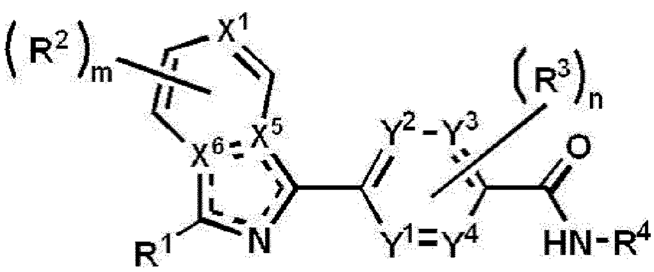

[IV]

{wherein

$X^1$, $X^5$, $X^6$, $Y^1$, $Y^2$, $Y^3$ and $Y^4$ are each independently a carbon or a nitrogen atom (wherein the total number of the nitrogen atoms for $X^5$ and $X^6$ is 1, and the total number of the nitrogen atoms for $Y^1$, $Y^2$, $Y^3$ and $Y^4$ is 0, 1 or 2), and other symbols are as defined for Item 1A},
or a pharmaceutically acceptable salt thereof.

[Item 22A]

A pharmaceutical composition comprising the compound of any one of Items 1A to 21A or a pharmaceutically acceptable salt thereof, and a pharmaceutically acceptable carrier.

[Item 23A]

An H-PGDS inhibitor comprising the compound of any one of Items 1A to 21A or a pharmaceutically acceptable salt thereof.

[Item 24A]

A therapeutic or prophylactic agent for a disease selected from the group consisting of peripheral arterial diseases, cardiovascular diseases, allergic asthma, chronic obstructive pulmonary diseases, and Duchenne muscular dystrophy, the agent comprising the compound of any one of Items 1A to 21A or a pharmaceutically acceptable salt thereof.

[Item 25A]

A method for inhibiting H-PGDS in a mammal, comprising administering a pharmaceutically effective amount of the compound of any one of Items 1A to 21A or a pharmaceutically acceptable salt thereof to the mammal.

[Item 26A]

A method for treating or preventing a disease selected from the group consisting of peripheral arterial diseases, cardiovascular diseases, allergic asthma, chronic obstructive pulmonary diseases, and Duchenne muscular dystrophy in a mammal, comprising administering a pharmaceutically effective amount of the compound of any one of Items 1A to 21A or a pharmaceutically acceptable salt thereof to the mammal.

[Item 27A]

Use of the compound of any one of Items 1A to 21A or a pharmaceutically acceptable salt thereof in producing an H-PGDS inhibitor.

[Item 28A]

Use of the compound of any one of Items 1A to 21A or a pharmaceutically acceptable salt thereof in producing a therapeutic or prophylactic agent for a disease selected from the group consisting of peripheral arterial diseases, cardiovascular diseases, allergic asthma, chronic obstructive pulmonary diseases, and Duchenne muscular dystrophy.

[Item 29A]

The compound of any one of Items 1A to 21A or a pharmaceutically acceptable salt thereof for use in inhibiting H-PGDS.

[Item 30A]

The compound of any one of Items 1A to 21A or a pharmaceutically acceptable salt thereof for use in the treatment or prophylaxis of a disease selected from the group consisting of peripheral arterial diseases, cardiovascular diseases, allergic asthma, chronic obstructive pulmonary diseases, and Duchenne muscular dystrophy.

[Item 31A]

A commercial package comprising the pharmaceutical composition of Item 22A and a written matter associated therewith, the written matter stating that the pharmaceutical composition can be used for treatment or prophylaxis of a disease selected from the group consisting of peripheral arterial diseases, cardiovascular diseases, allergic asthma, chronic obstructive pulmonary diseases, and Duchenne muscular dystrophy.

[Item 32A]

A kit comprising the pharmaceutical composition of Item 22A and a written matter associated therewith, the written matter stating that the pharmaceutical composition can be used for treatment or prophylaxis of a disease selected from the group consisting of peripheral arterial diseases, cardiovascular diseases, allergic asthma, chronic obstructive pulmonary diseases, and Duchenne muscular dystrophy.

[Item 33A]

A compound selected from the group consisting of the following structural formulas:

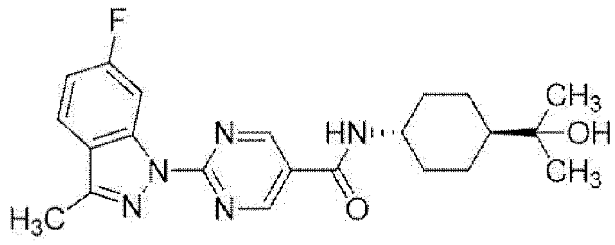

,

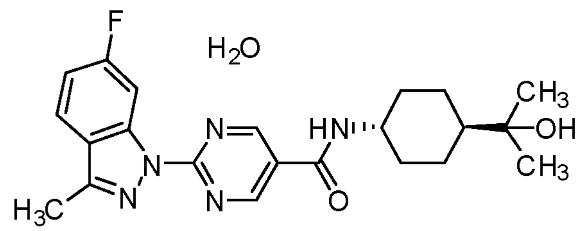

,

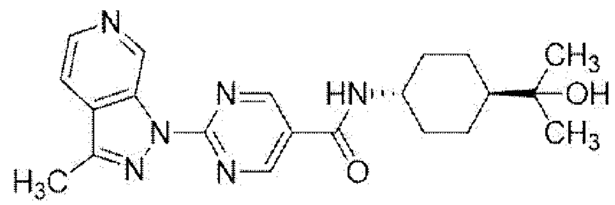

,

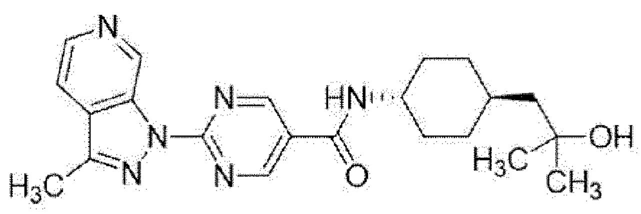

,

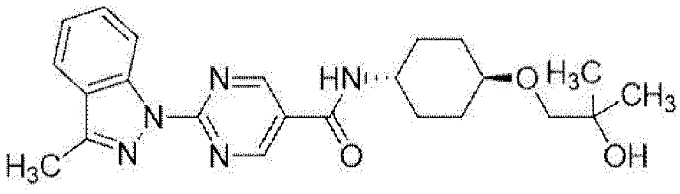

,

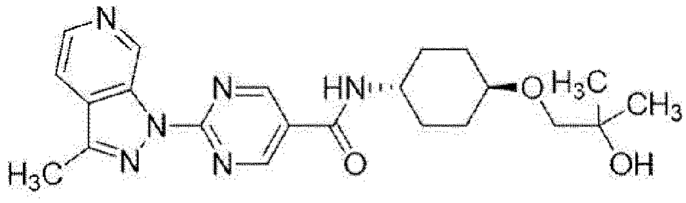

,

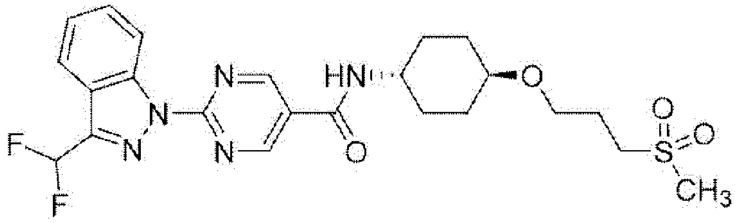

,

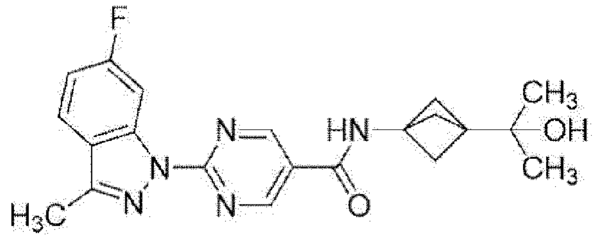

,

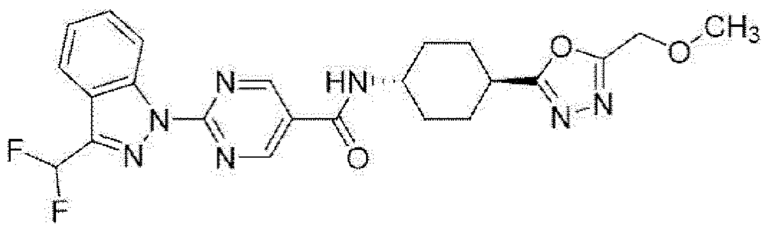

,

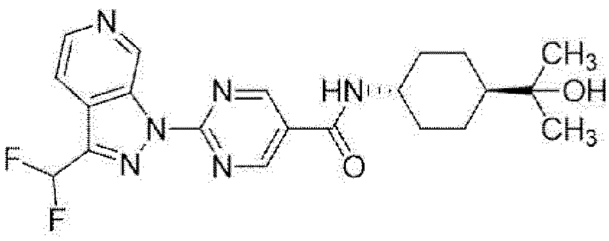

,

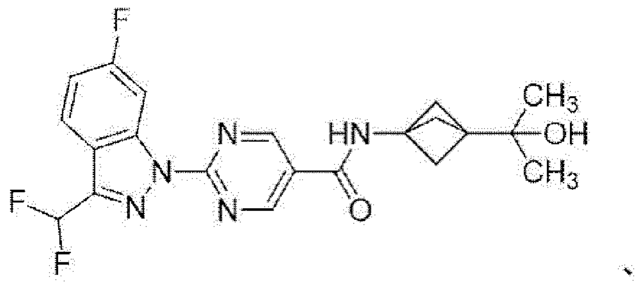

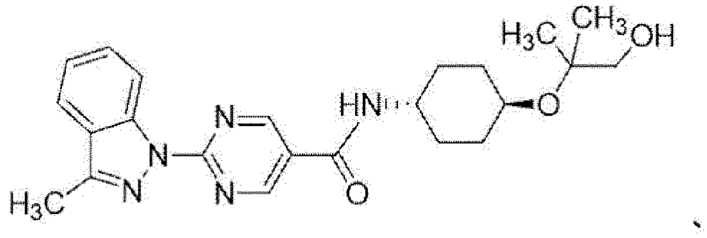

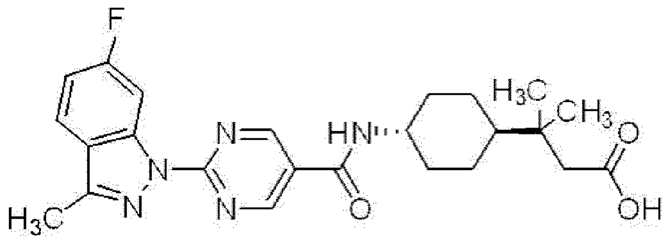

and

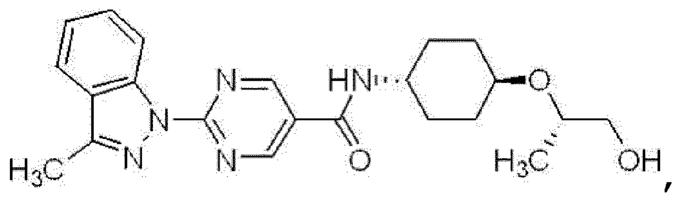

or a pharmaceutically acceptable salt thereof.

[Item 34A]

A pharmaceutical composition comprising the compound of any one of Items 1A to 21A and Item 33A or a pharmaceutically acceptable salt thereof, and a pharmaceutically acceptable carrier.

[Item 35A]

An H-PGDS inhibitor comprising the compound of any one of Items 1A to 21A and Item 33A or a pharmaceutically acceptable salt thereof.

[Item 36A]

A therapeutic or prophylactic agent for a disease selected from the group consisting of peripheral arterial diseases, cardiovascular diseases, allergic asthma, chronic obstructive pulmonary diseases, allergic rhinitis, sarcopenia, and Duchenne muscular dystrophy, the agent comprising the compound of any one of Items 1A to 21A and Item 33A or a pharmaceutically acceptable salt thereof.

[Item 37A]

The therapeutic or prophylactic agent of Item 36A, wherein the peripheral arterial disease is intermittent claudication or comprehensive severe chronic lower extremity ischemia based on PAD.

[Item 38A]

A method for inhibiting H-PGDS in a mammal, comprising administering a pharmaceutically effective amount of the compound of any one of Items 1A to 21A and Item 33A or a pharmaceutically acceptable salt thereof to the mammal.

[Item 39A]

A method for treating or preventing a disease selected from the group consisting of peripheral arterial diseases, cardiovascular diseases, allergic asthma, chronic obstructive pulmonary diseases, allergic rhinitis, sarcopenia, and Duchenne muscular dystrophy in a mammal, comprising administering a pharmaceutically effective amount of the compound of any one of Items 1A to 21A and Item 33A or a pharmaceutically acceptable salt thereof to the mammal.

[Item 40A]

The method of Item 39A, wherein the peripheral arterial disease is intermittent claudication or comprehensive severe chronic lower extremity ischemia based on PAD.

[Item 41A]

Use of the compound of any one of Items 1A to 21A and Item 33A or a pharmaceutically acceptable salt thereof in producing an H-PGDS inhibitor.

[Item 42A]

Use of the compound of any one of Items 1A to 21A and Item 33A or a pharmaceutically acceptable salt thereof in producing a therapeutic or prophylactic agent for a disease selected from the group consisting of peripheral arterial diseases, cardiovascular diseases, allergic asthma, chronic obstructive pulmonary diseases, allergic rhinitis, sarcopenia, and Duchenne muscular dystrophy.

[Item 43A]

The use of Item 42A, wherein the peripheral arterial disease is intermittent claudication or comprehensive severe chronic lower extremity ischemia based on PAD.

[Item 44A]

The compound of any one of Items 1A to 21A and Item 33A or a pharmaceutically acceptable salt thereof for use in inhibiting H-PGDS.

[Item 45A]

The compound of any one of Items 1A to 21A and Item 33A and Item 33A or a pharmaceutically acceptable salt thereof for use in the treatment or prophylaxis of a disease selected from the group consisting of peripheral arterial diseases, cardiovascular diseases, allergic asthma, chronic obstructive pulmonary diseases, allergic rhinitis, sarcopenia, and Duchenne muscular dystrophy.

[Item 46A]

The compound of Item 45A or a pharmaceutically acceptable salt thereof, wherein the peripheral arterial disease is intermittent claudication or comprehensive severe chronic lower extremity ischemia based on PAD.

[Item 47A]

A commercial package comprising the pharmaceutical composition of Item 34A and a written matter associated therewith, the written matter stating that the pharmaceutical composition can be used for treatment or prophylaxis of a disease selected from the group consisting of peripheral arterial diseases, cardiovascular diseases, allergic asthma, chronic obstructive pulmonary diseases, allergic rhinitis, sarcopenia, and Duchenne muscular dystrophy.

[Item 48A]

A kit comprising the pharmaceutical composition of Item 22A and a written matter associated therewith, the written matter stating that the pharmaceutical composition can be used for treatment or prophylaxis of a disease selected from the group consisting of peripheral arterial diseases, cardiovascular diseases, allergic asthma, chronic obstructive pulmonary diseases, allergic rhinitis, sarcopenia, and Duchenne muscular dystrophy.

[Description of Embodiments]

The definitions of the terms in the present specification are as follows.
In the chemical formulas, the wavy line shown by the following:

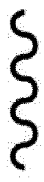

indicates a binding site in the structures or groups represented by the chemical formulas.
In the chemical formulas, the bond shown by the following:



is a single bond or a double bond.
The "$C_{1-4}$ alkyl" means a linear or branched chain saturated hydrocarbon group having 1 to 4 carbon atoms. The "$C_{1-4}$ alkyl" includes methyl, ethyl, n-propyl, isopropyl, n-butyl, sec-butyl, isobutyl, and tert-butyl. Preferred "$C_{1-4}$ alkyl" is methyl, ethyl, or isopropyl. More preferred "$C_{1-4}$ alkyl" is methyl or ethyl.
The "$C_{1-6}$ alkyl" means a linear or branched chain saturated hydrocarbon group having 1 to 6 carbon atoms. The "$C_{1-6}$ alkyl" includes, for example, methyl, ethyl, n-propyl, isopropyl, n-butyl, sec-butyl, isobutyl, tert-butyl, n-pentyl, isopentyl, neopentyl, 2-methylbutyl, 1,1-dimethylpropyl, 1-ethylpropyl, n-hexyl, isohexyl, 1,1-dimethylbutyl, 2,2-dimethylbutyl, 3,3-dimethylbutyl, and 2-ethylbutyl. Preferred "$C_{1-6}$ alkyl" is methyl, ethyl, isopropyl, isobutyl, tert-butyl, or isopentyl. More preferred "$C_{1-6}$ alkyl" is methyl, ethyl, isopropyl, isobutyl, or tert-butyl.
The "$C_{1-4}$ alkoxy" means a group in which the above-mentioned "$C_{1-4}$ alkyl" is bonded to an oxygen atom. The "$C_{1-4}$ alkoxy" includes methoxy, ethoxy, n-propoxy, isopropoxy, n-butoxy, sec-butoxy, isobutoxy, and tert-butoxy. Preferred "$C_{1-4}$ alkoxy" is methoxy or ethoxy.
The "$C_{1-6}$ alkoxy" means a group in which the above-mentioned "$C_{1-6}$ alkyl" is bonded to an oxygen atom. The "$C_{1-6}$ alkoxy" includes, for example, methoxy, ethoxy, n-propoxy, isopropoxy, n-butoxy, sec-butoxy, isobutoxy, tert-butoxy, pentyloxy, isopentyloxy, neopentyloxy, 2-methylbutoxy, 1,1-dimethylpropoxy, 1-ethylpropoxy, hexyloxy, isohexyloxy, 1,1-dimethylbutoxy, 2,2-dimethylbutoxy, 3,3-dimethylbutoxy, and 2-ethylbutoxy. Preferred "$C_{1-6}$ alkoxy" is methoxy, ethoxy, n-propoxy, isopropoxy, isobutoxy, tert-butoxy, or isopentyloxy.
The "halogen" includes, for example, fluorine, chlorine, bromine, and iodine. Preferred "halogen" is fluorine or chlorine.
The "$C_{1-4}$ haloalkyl" means the above-mentioned "$C_{1-4}$ alkyl" substituted by 1 to 7 halogens independently selected from the group of the above-mentioned group of "halogen". The "$C_{1-4}$ haloalkyl" includes, for example, monofluoromethyl, difluoromethyl, trifluoromethyl, chlorofluoromethyl, 2-fluoroethyl, 2-chloroethyl, 2-bromoethyl, 1,1-difluoroethyl, 2,2-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, and 4,4,4-trifluorobutyl. Preferred "$C_{1-4}$ haloalkyl" is difluoromethyl, trifluoromethyl, or 2,2-difluoroethyl.
The "$C_{1-6}$ haloalkyl" means the above-mentioned "$C_{1-6}$ alkyl" substituted by 1 to 9 halogens independently selected from the group of the above-mentioned "halogen". The "$C_{1-6}$ haloalkyl" includes, for example, monofluoromethyl,

difluoromethyl, trifluoromethyl, chlorofluoromethyl, 2-fluoroethyl, 2-chloroethyl, 2-bromoethyl, 1,1-difluoroethyl, 2,2-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, 4,4,4-trifluorobutyl, 5,5,5-trifluoropentyl, and 6,6,6-trifluorohexyl. Preferred "$C_{1-6}$ haloalkyl" is difluoromethyl, trifluoromethyl, 1-fluoro-1-methylethyl, or 2,2,2-trifluoroethyl.

The "$C_{4-6}$ cycloalkyl" means a monocyclic saturated hydrocarbon ring group having 4 to 6 carbon atoms. The "$C_{4-6}$ cycloalkyl" includes, for example, cyclobutyl, cyclopentyl, and cyclohexyl. Preferred "$C_{4-6}$ cycloalkyl" is cyclobutyl or cyclohexyl. More preferred "$C_{4-6}$ cycloalkyl" is cyclohexyl.

The "$C_{3-6}$ cycloalkyl" means a monocyclic saturated hydrocarbon ring group having 3 to 6 carbon atoms. The "$C_{3-6}$ cycloalkyl" includes, for example, cyclopropyl, cyclobutyl, cyclopentyl, and cyclohexyl. Preferred "$C_{3-6}$ cycloalkyl" is cyclopropyl, cyclobutyl, or cyclohexyl.

The "4- to 6-membered heterocycloalkyl containing 1 or 2 hetero atoms independently selected from the group consisting of nitrogen and oxygen atoms" means a 4- to 6-membered monocyclic saturated heterocyclic group containing, as a ring-constituting atom besides carbon atom, 1 or 2 hetero atoms independently selected from the group consisting of nitrogen and oxygen. The "4- to 6-membered heterocycloalkyl containing 1 or 2 hetero atoms independently selected from the group consisting of nitrogen and oxygen atoms" includes, for example, azetidinyl, oxetanyl, diazetidinyl, dioxetanyl, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, tetrahydro-1,2-oxazinyl, and dioxanyl. Preferred "4- to 6-membered heterocycloalkyl containing 1 or 2 hetero atoms independently selected from the group consisting of nitrogen and oxygen atoms" is azetidinyl, pyrrolidinyl, or morpholinyl.

The "5- or 6-membered heterocycloalkyl containing one nitrogen atom" means a 5- or 6-membered monocyclic saturated heterocyclic group containing one nitrogen atom as a ring-constituting atom besides carbon atom. The "5- or 6-membered heterocycloalkyl containing one nitrogen atom" includes, for example, pyrrolidinyl and piperidinyl. Preferred "5- or 6-membered heterocycloalkyl containing one nitrogen atom" is piperidinyl.

The "4- to 6-membered heterocycloalkyl containing one oxygen atom" means a 4- to 6-membered monocyclic saturated heterocyclic group containing one oxygen atom as a ring-constituting atom besides carbon atom. The "4- to 6-membered heterocycloalkyl containing one oxygen atom" includes, for example, oxetanyl, tetrahydrofuranyl, and tetrahydropyranyl. Preferred "4- to 6-membered heterocycloalkyl containing one oxygen atom" is oxetanyl or tetrahydropyranyl.

The "$C_{5-8}$ bridged cycloalkyl" means a 5- to 8-membered, bridged cyclic saturated hydrocarbon group. The "$C_{5-8}$ bridged cycloalkyl" includes, for example, bicyclo[1.1.1]pentyl, bicyclo[2.2.1]heptyl, and bicyclo[2.2.2]octyl. Preferred "$C_{5-8}$ bridged cycloalkyl" is bicyclo[1.1.1]pentyl or bicyclo[2.2.2]octyl. More preferred "$C_{5-8}$ bridged cycloalkyl" is bicyclo[1.1.1]pentyl.

The "8-membered bridged heterocycloalkyl containing one nitrogen atom" means a 8-membered bridged saturated heterocyclic group containing one nitrogen atom as a ring-constituting atom besides carbon atom. The "8-membered bridged heterocycloalkyl containing one nitrogen atom" includes, for example,
the following groups:

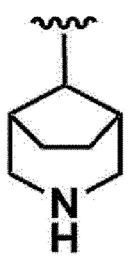
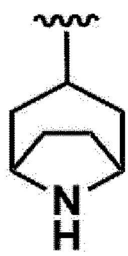
.

The "7- to 11-membered spiro heterocycloalkyl containing one nitrogen atom" means a 7- to 11-membered spiro-type saturated heterocyclic group containing one nitrogen atom as a ring-constituting atom besides carbon atom. The "7- to 11-membered spiro heterocycloalkyl containing one nitrogen atom" includes, for example,
the following groups:

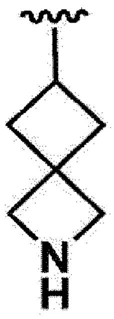 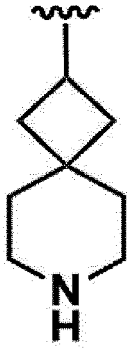 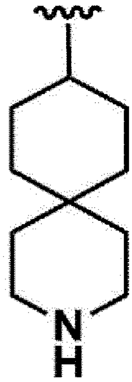.

The "6- to 9-membered saturated or partially unsaturated fused ring group containing 1 or 2 nitrogen atoms" means a 6-to 9-membered fused heterocyclic group containing 1 or 2 nitrogen atoms as a ring-constituting atom besides carbon atom, and means a fused ring group containing at least one saturated ring in the ring constituting the fused ring. The "6- to 9-membered saturated or partially unsaturated fused ring group containing 1 or 2 nitrogen atoms" includes, for example,
the following groups:

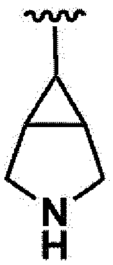 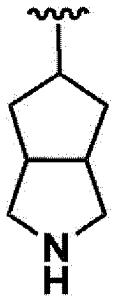 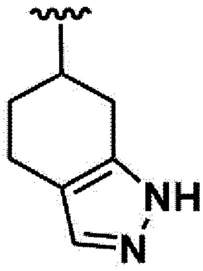 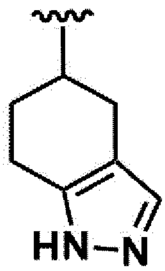 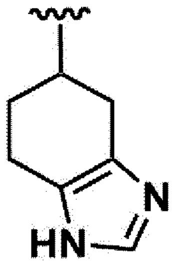.

That α is "optionally substituted" by β means that α is unsubstituted or any substitutable hydrogen of α is replaced by β. For example, "$C_{1-6}$ alkyl optionally substituted by hydroxy" means that $C_{1-6}$ alkyl is unsubstituted or any hydrogen in $C_{1-6}$ alkyl is replaced by hydroxy.
Specific embodiments of each substituent of the compound of formula [II] are exemplified below. However, each substituent of the compound of formula [II] is not limited to the specific embodiments. In addition, the compound of the formula [II] also includes an embodiment in which any two or more of the specific embodiments of each substituent are combined.
$Y^1$ is preferably a nitrogen atom.
$Y^2$ is preferably a nitrogen atom.
$Y^3$ is preferably a carbon atom.
$Y^4$ is preferably a carbon atom.
Preferred embodiment of $R^1$ is

(1) hydroxy,
(2) $C_{1-4}$ alkyl (wherein the alkyl is optionally substituted by hydroxy or $C_{1-4}$ alkoxy),
(3) $C_{1-4}$ alkoxy,
(4) halogen, or
(5) $C_{1-4}$ haloalkyl.

More preferred embodiment of $R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl.

Preferred specific example of $R^1$ is methyl, fluorine, chlorine, difluoromethyl, or trifluoromethyl.
Preferred embodiments of $R^2$ in the number of m are each independently halogen.

Preferred specific examples of $R^2$ in the number of m include fluorine and chlorine.
m is preferably 0 or 1.
Preferred embodiments of $R^3$ in the number of n are each independently halogen.
Preferred specific examples of $R^3$ in the number of n include fluorine and chlorine.
n is preferably 0 or 1.
$R^4$ is preferably

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

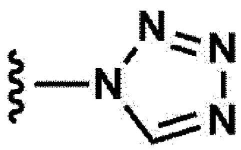 ),

(V) $C_{1-6}$ alkoxy (wherein the alkoxy is optionally substituted by hydroxy or $-SO_2-C_{1-4}$ alkyl),
(VI) carboxy,
(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,
(X) a group represented by the formula:

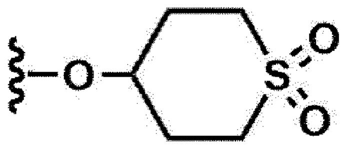 ,

(XI) a group represented by the formula:

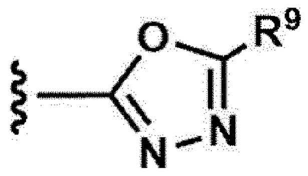

{wherein $R^9$ is $C_{1-4}$ alkyl (wherein the alkyl is optionally substituted by $C_{1-4}$ alkoxy)}, and
(XII) a group represented by the formula:

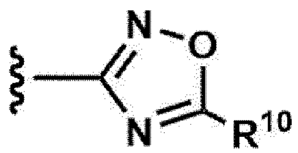

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2-C_{1-4}$ alkyl, or
(vi) a group represented by the formula:

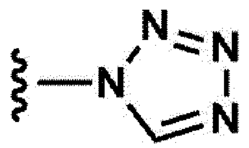
),

(III) $C_{1-6}$ alkoxy (wherein the alkoxy is optionally substituted by hydroxy or $-SO_2-C_{1-4}$ alkyl),
(IV) carboxy,
(V) -CO-$C_{1-4}$ alkoxy,
(VI) -O-$C_{1-6}$ haloalkyl, or
(VII) a group represented by the formula:

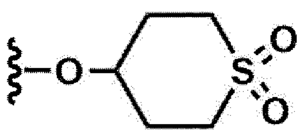
},

(c) 5- or 6-membered heterocycloalkyl containing one nitrogen atom [wherein the heterocycloalkyl is

(I) oxo,
(II) $C_{1-6}$ alkyl,
(III) -CO-$R^{11}$ {wherein $R^{11}$ is optionally substituted by 1 to 3 substituents independently selected from the group consisting of

(i) $C_{1-6}$ alkyl (wherein the alkyl is optionally substituted by

(A) hydroxy,
(B) cyano, or

(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

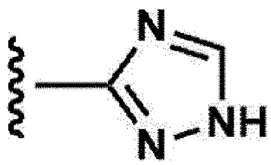
],

(d) 8-membered bridged heterocycloalkyl containing one nitrogen atom {wherein the bridged heterocycloalkyl is optionally substituted by -CO-$R^{11}$},
(e) 7- to 11-membered spiro heterocycloalkyl containing one nitrogen atom {wherein the spiro heterocycloalkyl is optionally substituted by -CO-$R^{11}$}, or
(f) 6- to 9-membered saturated or partially unsaturated fused ring group containing 1 or 2 nitrogen atoms (wherein the fused ring group is optionally substituted by -CO-$R^{11}$).

$R^4$ is more preferably

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) -$CONH_2$,
(iv) -CO-$C_{1-4}$ alkoxy,
(v) -$SO_2$-$C_{1-4}$ alkyl, or
(vi) a group represented by the formula:

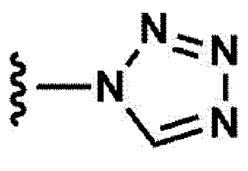
),

(V) $C_{1-6}$ alkoxy (wherein the alkoxy is optionally substituted by hydroxy or -$SO_2$-$C_{1-4}$ alkyl),
(VI) carboxy,
(VII) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) -$NR^7R^8$ {wherein $R^7$ and $R^8$ are each independently $C_{1-4}$ alkyl or -CO-$C_{1-6}$ alkyl (wherein the alkyl is

optionally substituted by cyano)},
(IX) -O-$C_{1-6}$ haloalkyl,
(X) a group represented by the formula:

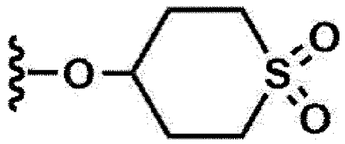

,

(XI) a group represented by the formula:

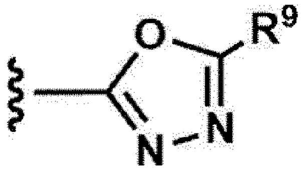

{wherein $R^9$ is $C_{1-4}$ alkyl (wherein the alkyl is optionally substituted by $C_{1-4}$ alkoxy)}, and
(XII) a group represented by the formula:

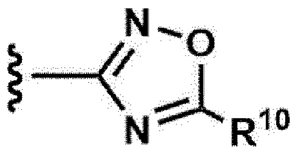

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy, or
(ii) -$SO_2$-$C_{1-4}$ alkyl),

(III) carboxy, or
(IV) -CO-$C_{1-4}$ alkoxy},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl, and
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}],

(d) 8-membered bridged heterocycloalkyl containing one nitrogen atom {wherein the bridged heterocycloalkyl is optionally substituted by -CO-$R^{11a}$ {wherein $R^{11a}$ is

(i) $C_{1-6}$ alkyl (wherein the alkyl is optionally substituted by

(A) hydroxy, or
(B) cyano), or

(ii) $C_{1-6}$ alkoxy,}},

(e) 7- to 11-membered spiro heterocycloalkyl containing one nitrogen atom {wherein the spiro heterocycloalkyl is optionally substituted by -CO-$R^{11a}$}, or
(f) 6- to 9-membered saturated or partially unsaturated fused ring group containing 1 or 2 nitrogen atoms (wherein the fused ring group is optionally substituted by -CO-$C_{1-6}$ alkyl (wherein the alkyl is optionally substituted by hydroxy)).

$R^4$ is further preferably

(a) $C_{4-6}$ cycloalkyl (wherein the cycloalkyl is optionally substituted by

(I) hydroxy,
(II) cyano,
(III) $C_{1-6}$ alkyl (wherein the alkyl is optionally substituted by

(i) hydroxy,
(ii) carboxy,
(iii) -$CONH_2$,
(iv) -CO-$C_{1-4}$ alkoxy,
(v) -$SO_2$-$C_{1-4}$ alkyl, or
(vi) a group represented by the formula:

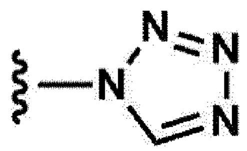
),

(IV) $C_{1-6}$ alkoxy (wherein the alkoxy is optionally substituted by hydroxy or -$SO_2$-$C_{1-4}$ alkyl),
(V) -CO-$NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],
(VI) -$NR^7R^8$ {wherein $R^7$ and $R^8$ are each independently $C_{1-4}$ alkyl or -CO-$C_{1-6}$ alkyl (wherein the alkyl is optionally substituted by cyano)},
(VII) a group represented by the formula:

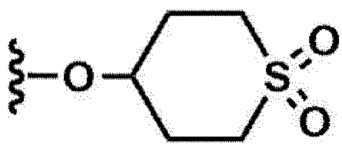
,

(VIII) a group represented by the formula:

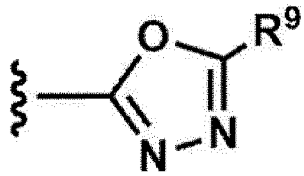

{wherein $R^9$ is $C_{1-4}$ alkyl (wherein the alkyl is optionally substituted by $C_{1-4}$ alkoxy)}, or
(IX) a group represented by the formula:

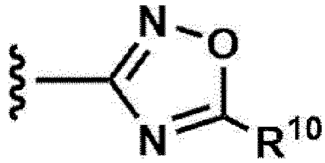

wherein $R^{10}$ is $C_{1-4}$ alkyl)),

(b) $C_{5-8}$ bridged cycloalkyl {wherein the bridged cycloalkyl is optionally substituted by $C_{1-6}$ alkyl (wherein the alkyl is optionally substituted by hydroxy)},
(c) 5- or 6-membered heterocycloalkyl containing one nitrogen atom [wherein the heterocycloalkyl is optionally substituted by -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}], or

(d) 8-membered bridged heterocycloalkyl containing one nitrogen atom {wherein the bridged heterocycloalkyl is optionally substituted by -CO-$C_{1-6}$ alkyl (wherein the alkyl is optionally substituted by

(A) hydroxy, or
(B) cyano)}.

Specific embodiments of each substituent of the compound of formula [I] are exemplified below. However, each substituent of the compound of formula [I] is not limited to the specific embodiments. In addition, the compound of the formula [I] also includes an embodiment in which any two or more of the specific embodiments of each substituent are combined.
$Y^1$ is preferably a nitrogen atom.
$Y^2$ is preferably a nitrogen atom.
$Y^3$ is preferably a carbon atom.
$Y^4$ is preferably a carbon atom.
Preferred embodiment of $R^1$ is

(1) hydroxy,
(2) $C_{1-4}$ alkyl (wherein the alkyl is optionally substituted by hydroxy or $C_{1-4}$ alkoxy),
(3) $C_{1-4}$ alkoxy,
(4) halogen, or
(5) $C_{1-4}$ haloalkyl.

More preferred embodiment of $R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl.

Preferred specific example of $R^1$ is methyl, fluorine, chlorine, difluoromethyl, or trifluoromethyl.
Preferred embodiments of $R^2$ in the number of m are each independently halogen.
Preferred specific examples of $R^2$ in the number of m include fluorine and chlorine.
m is preferably 0 or 1.
Preferred embodiments of $R^3$ in the number of n are each independently halogen.
Preferred specific examples of $R^3$ in the number of n include fluorine and chlorine.
n is preferably 0 or 1.
$R^4$ is preferably

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

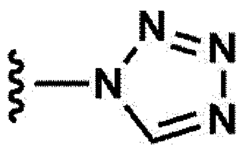
),

(V) $C_{1-6}$ alkoxy (wherein the alkoxy is optionally substituted by hydroxy or $-SO_2-C_{1-4}$ alkyl),
(VI) carboxy,
(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,
(X) a group represented by the formula:

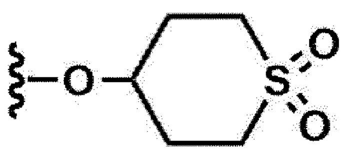
,

(XI) a group represented by the formula:

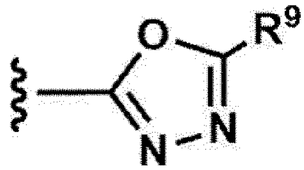

{wherein $R^9$ is $C_{1-4}$ alkyl (wherein the alkyl is optionally substituted by $C_{1-4}$ alkoxy)}, and
(XII) a group represented by the formula:

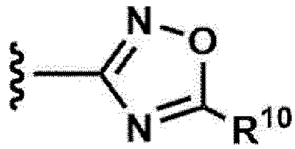

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

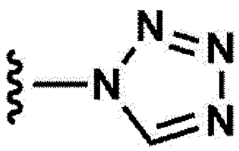
),

(III) $C_{1-6}$ alkoxy (wherein the alkoxy is optionally substituted by hydroxy or $-SO_2$-$C_{1-4}$ alkyl),
(IV) carboxy,
(V) -CO-$C_{1-4}$ alkoxy,
(VI) -O-$C_{1-6}$ haloalkyl, or
(VII) a group represented by the formula:

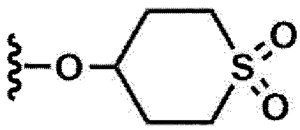
},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl,
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

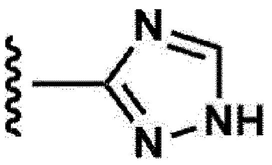
],

(d) 8-membered bridged heterocycloalkyl containing one nitrogen atom {wherein the bridged heterocycloalkyl is optionally substituted by -CO-$R^{11}$},
(e) 7- to 11-membered spiro heterocycloalkyl containing one nitrogen atom {wherein the spiro heterocycloalkyl is optionally substituted by -CO-$R^{11}$}, or
(f) 6- to 9-membered saturated or partially unsaturated fused ring group containing 1 or 2 nitrogen atoms (wherein the fused ring group is optionally substituted by -CO-$R^{11}$).

$R^4$ is more preferably

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) -$CONH_2$,
(iv) -CO-$C_{1-4}$ alkoxy,
(v) -$SO_2$-$C_{1-4}$ alkyl, or
(vi) a group represented by the formula:

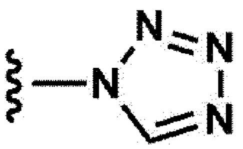
),

(V) $C_{1-6}$ alkoxy (wherein the alkoxy is optionally substituted by hydroxy or -$SO_2$-$C_{1-4}$ alkyl),
(VI) carboxy,
(VII) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,
(X) a group represented by the formula:

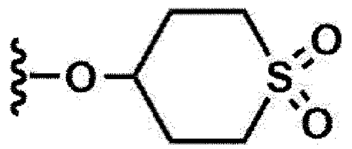

(XI) a group represented by the formula:

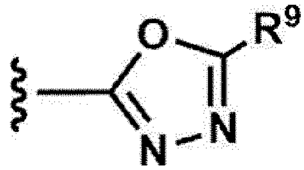

{wherein $R^9$ is $C_{1-4}$ alkyl (wherein the alkyl is optionally substituted by $C_{1-4}$ alkoxy)}, and
(XII) a group represented by the formula:

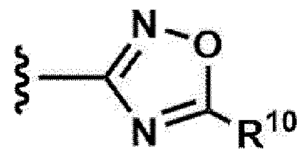

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy, or
(ii) $-SO_2-C_{1-4}$ alkyl),

(III) carboxy, or
(IV) $-CO-C_{1-4}$ alkoxy},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl, and
(III) $-CO-R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is

optionally substituted by halogen)}],

(d) 8-membered bridged heterocycloalkyl containing one nitrogen atom {wherein the bridged heterocycloalkyl is optionally substituted by -CO-$R^{11a}$ {wherein $R^{11a}$ is

(i) $C_{1-6}$ alkyl (wherein the alkyl is optionally substituted by

(A) hydroxy, or
(B) cyano), or

(ii) $C_{1-6}$ alkoxy,}},

(e) 7- to 11-membered spiro heterocycloalkyl containing one nitrogen atom {wherein the spiro heterocycloalkyl is optionally substituted by -CO-$R^{11a}$}, or
(f) 6- to 9-membered saturated or partially unsaturated fused ring group containing 1 or 2 nitrogen atoms (wherein the fused ring group is optionally substituted by -CO-$C_{1-6}$ alkyl (wherein the alkyl is optionally substituted by hydroxy)).

$R^4$ is further preferably

(a) $C_{4-6}$ cycloalkyl (wherein the cycloalkyl is optionally substituted by

(I) hydroxy,
(II) cyano,
(III) $C_{1-6}$ alkyl (wherein the alkyl is optionally substituted by

(i) hydroxy,
(ii) carboxy,
(iii) -$CONH_2$,
(iv) -CO-$C_{1-4}$ alkoxy,
(v) -$SO_2$-$C_{1-4}$ alkyl, or
(vi) a group represented by the formula:

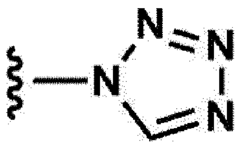
),

(IV) $C_{1-6}$ alkoxy (wherein the alkoxy is optionally substituted by hydroxy or -$SO_2$-$C_{1-4}$ alkyl),
(V) -CO-$NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],
(VI) -$NR^7R^8$ {wherein $R^7$ and $R^8$ are each independently $C_{1-4}$ alkyl or -CO-$C_{1-6}$ alkyl (wherein the alkyl is optionally substituted by cyano)},
(VII) a group represented by the formula:

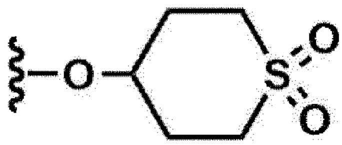
,

(VIII) a group represented by the formula:

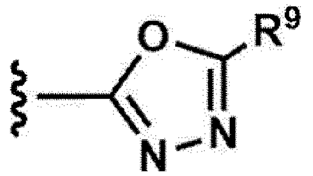

{wherein $R^9$ is $C_{1-4}$ alkyl (wherein the alkyl is optionally substituted by $C_{1-4}$ alkoxy)}, or
(IX) a group represented by the formula:

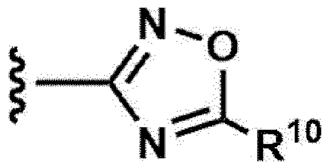

wherein $R^{10}$ is $C_{1-4}$ alkyl)),

(b) $C_{5-8}$ bridged cycloalkyl {wherein the bridged cycloalkyl is optionally substituted by $C_{1-6}$ alkyl (wherein the alkyl is optionally substituted by hydroxy)},
(c) 5- or 6-membered heterocycloalkyl containing one nitrogen atom [wherein the heterocycloalkyl is optionally substituted by -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}], or

(d) 8-membered bridged heterocycloalkyl containing one nitrogen atom {wherein the bridged heterocycloalkyl is optionally substituted by -CO-$C_{1-6}$ alkyl (wherein the alkyl is optionally substituted by

(A) hydroxy, or
(B) cyano)}.

In a partial structure represented by the formula:

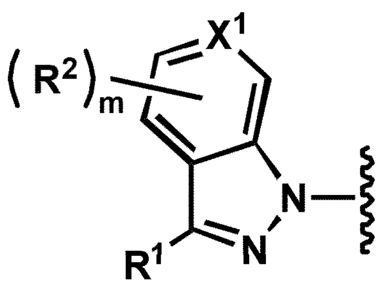

in the formula [I], preferably,

$X^1$ is carbon or a nitrogen atom;
m is 0 or 1;
$R^1$ is

(1) hydroxy,
(2) $C_{1-4}$ alkyl (wherein the alkyl is optionally substituted by hydroxy or $C_{1-4}$ alkoxy),
(3) $C_{1-4}$ alkoxy,
(4) halogen, or
(5) $C_{1-4}$ haloalkyl; and

$R^2$ in the number of m are each independently halogen,
more preferably,
$X^1$ is carbon or a nitrogen atom;
m is 0 or 1;
$R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl; and

$R^2$ in the number of m are each independently halogen.

Specific embodiments of each substituent of the compound of formula [III] are exemplified below. However, each substituent of the compound of formula [III] is not limited to the specific embodiments. In addition, the compound of the formula [III] also includes an embodiment in which any two or more of the specific embodiments of each substituent are combined.

Preferred embodiment of $R^1$ is

(1) $C_{1-4}$ alkyl,
(2) $C_{1-4}$ haloalkyl, or
(3) $C_{3-6}$ cycloalkyl.

More preferred embodiment of $R^1$ is $C_{1-4}$ alkyl.

Specific preferable example of $R^1$ is methyl.

Preferred embodiments of $R^2$ in the number of m are each independently

(1) cyano,
(2) $C_{1-4}$ alkyl,
(3) $C_{1-4}$ alkoxy, or
(4) halogen.

More preferred embodiments of $R^2$ in the number of m are each independently halogen.

Preferred specific examples of $R^2$ in the number of m include fluorine and chlorine.

m is preferably 0 or 1.

Preferred embodiments of $R^3$ in the number of n are each independently halogen.

Preferred specific examples of $R^3$ in the number of n include fluorine and chlorine.

$R^4$ is preferably

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,

(v) $-SO_2-C_{1-4}$ alkyl, or

(vi) a group represented by the formula:

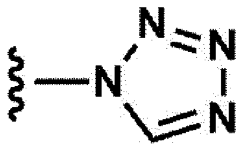 ),

(V) $C_{1-6}$ alkoxy (wherein the alkoxy is optionally substituted by hydroxy or $-SO_2-C_{1-4}$ alkyl),

(VI) carboxy,

(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and

(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},

(IX) $-O-C_{1-6}$ haloalkyl,

(X) a group represented by the formula:

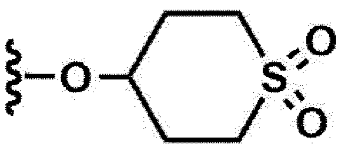 ,

(XI) a group represented by the formula:

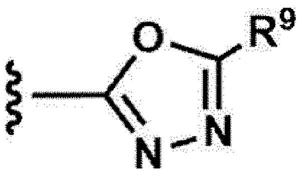

{wherein $R^9$ is $C_{1-4}$ alkyl (wherein the alkyl is optionally substituted by $C_{1-4}$ alkoxy)}, and

(XII) a group represented by the formula:

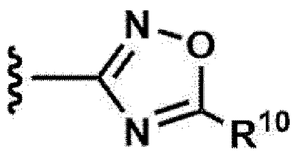

wherein $R^{10}$ is $C_{1-4}$ alkyl)),

(b) $C_{5-8}$ bridged cycloalkyl {wherein the bridged cycloalkyl is optionally substituted by

(I) hydroxy,

(II) $C_{1-6}$ alkyl (wherein the alkyl is optionally substituted by

(i) hydroxy,

(ii) carboxy,

(iii) $-CONH_2$,

(iv) -CO-$C_{1-4}$ alkoxy,
(v) -$SO_2$-$C_{1-4}$ alkyl, or
(vi) a group represented by the formula:

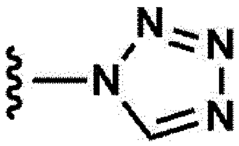

),

(III) $C_{1-6}$ alkoxy (wherein the alkoxy is optionally substituted by hydroxy or -$SO_2$-$C_{1-4}$ alkyl),
(IV) carboxy,
(V) -CO-$C_{1-4}$ alkoxy,
(VI) -O-$C_{1-6}$ haloalkyl, or
(VII) a group represented by the formula:

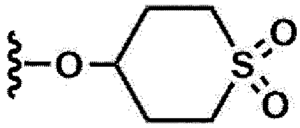

},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl,
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

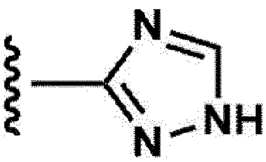

],

(d) 6- to 9-membered saturated or partially unsaturated fused ring group containing 1 or 2 nitrogen atoms {wherein the fused ring group is optionally substituted by -CO-$R^{11}$}, or
(e) a group represented by the formula:

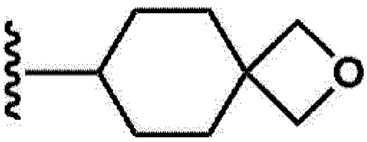

.

$R^4$ is more preferably

(a) $C_{4-6}$ cycloalkyl (wherein the cycloalkyl is optionally substituted by 1 or 2 substituents independently selected from the group consisting of

(I) hydroxy,
(II) cyano,
(III) $C_{1-6}$ alkyl (wherein the alkyl is optionally substituted by

(i) hydroxy,
(ii) carboxy, or
(iii) $-SO_2-C_{1-4}$ alkyl),

(IV) $-O-C_{1-6}$ haloalkyl,
(V) a group represented by the formula:

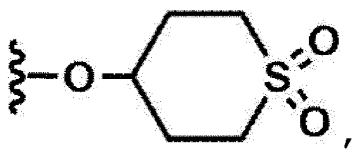

and
(VI) a group represented by the formula:

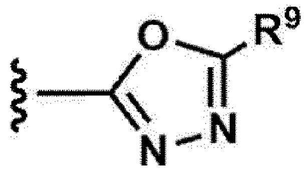

{wherein $R^9$ is $C_{1-4}$ alkyl}),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy, or
(iii) $-CO-C_{1-4}$ alkoxy), or

(III) carboxy},

(c) 5- or 6-membered heterocycloalkyl containing one nitrogen atom [wherein the heterocycloalkyl is optionally substituted by a group represented by the formula:

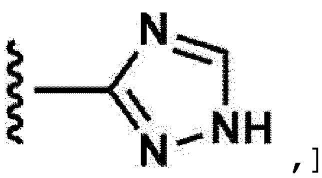

(d) 6- to 9-membered saturated or partially unsaturated fused ring group containing 1 or 2 nitrogen atoms, or
(e) a group represented by the formula:

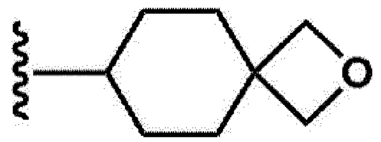

Specific embodiments of each substituent of the compound of formula [IV] are exemplified below. However, each substituent of the compound of formula [IV] is not limited to the specific embodiments. In addition, the compound of the formula [IV] also includes an embodiment in which any two or more of the specific embodiments of each substituent are combined.

$Y^1$ is preferably a nitrogen atom.

$Y^2$ is preferably a nitrogen atom.

$Y^3$ is preferably a carbon atom.

$Y^4$ is preferably a carbon atom.

Preferred embodiment of $R^1$ is $C_{1-4}$ alkyl.

Preferred specific example of $R^1$ is methyl.

m is preferably 0.

Preferred embodiments of $R^3$ in the number of n are each independently halogen.

Preferred specific example of $R^3$ in the number of n is fluorine.

n is preferably 0 or 1.

$R^4$ is preferably $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

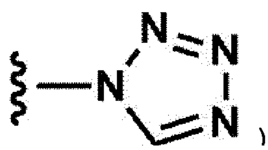

),

(V) $C_{1-6}$ alkoxy (wherein the alkoxy is optionally substituted by hydroxy or $-SO_2$-$C_{1-4}$ alkyl),
(VI) carboxy,
(VII) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or -CO-$C_{1-6}$ alkyl (wherein the alkyl is optionally substituted by cyano)},
(IX) -O-$C_{1-6}$ haloalkyl,
(X) a group represented by the formula:

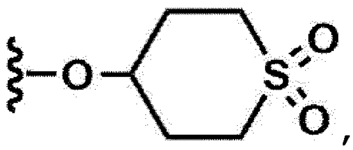

(XI) a group represented by the formula:

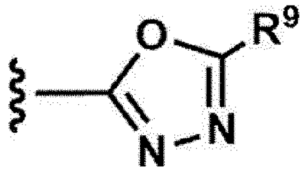

{wherein $R^9$ is $C_{1-4}$ alkyl (wherein the alkyl is optionally substituted by $C_{1-4}$ alkoxy)}, and
(XII) a group represented by the formula:

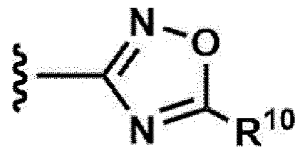

wherein $R^{10}$ is $C_{1-4}$ alkyl)) .

$R^4$ is more preferably $C_{4-6}$ cycloalkyl (wherein the cycloalkyl is

(I) hydroxy, or
(II) $C_{1-6}$ alkyl (wherein the alkyl is optionally substituted by

(i) hydroxy, or
(ii) carboxy).

One of the preferred embodiments of the compound of the formula [II] is a compound of the formula [II] wherein

$X^1$, $X^2$, $X^3$ and $X^4$ are each independently a carbon or a nitrogen atom (wherein the total number of the nitrogen atoms for $X^1$, $X^2$, $X^3$ and $X^4$ is 0 or 1);
$Y^1$ and $Y^2$ are each a nitrogen atom;
$Y^3$ and $Y^4$ are each a carbon atom;
m is 0 or 1;
n is 0 or 1;
$R^1$ is

(1) hydroxy,
(2) $C_{1-4}$ alkyl (wherein the alkyl is optionally substituted by hydroxy or $C_{1-4}$ alkoxy),
(3) $C_{1-4}$ alkoxy,
(4) halogen, or
(5) $C_{1-4}$ haloalkyl;

$R^2$ in the number of m are each independently halogen;
$R^3$ in the number of n are each independently halogen;
$R^4$ is

(a) $C_{4-6}$ cycloalkyl (wherein the cycloalkyl is optionally substituted by 1 or 2 substituents independently selected from the group consisting of

(I) hydroxy,
(II) cyano,

(III) oxo,
(IV) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

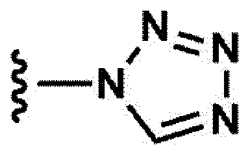

),

(V) $C_{1-6}$ alkoxy (wherein the alkoxy is optionally substituted by hydroxy or $-SO_2$-$C_{1-4}$ alkyl),
(VI) carboxy,
(VII) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or -CO-$C_{1-6}$ alkyl (wherein the alkyl is optionally substituted by cyano)},
(IX) -O-$C_{1-6}$ haloalkyl,
(X) a group represented by the formula:

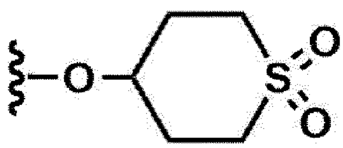

,

(XI) a group represented by the formula:

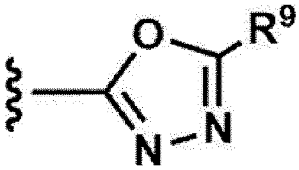

{wherein $R^9$ is $C_{1-4}$ alkyl (wherein the alkyl is optionally substituted by $C_{1-4}$ alkoxy)}, and
(XII) a group represented by the formula:

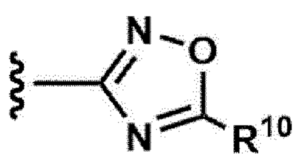

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

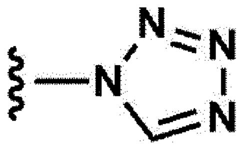
),

(III) $C_{1-6}$ alkoxy (wherein the alkoxy is optionally substituted by hydroxy or $-SO_2-C_{1-4}$ alkyl),
(IV) carboxy,
(V) $-CO-C_{1-4}$ alkoxy,
(VI) $-O-C_{1-6}$ haloalkyl, or
(VII) a group represented by the formula:

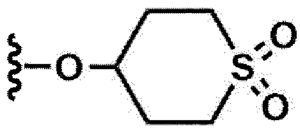
},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl,
(III) $-CO-R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

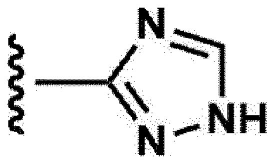
],

(d) 8-membered bridged heterocycloalkyl containing one nitrogen atom {wherein the bridged heterocycloalkyl is optionally substituted by $-CO-R^{11}$},
(e) 7- to 11-membered spiro heterocycloalkyl containing one nitrogen atom {wherein the spiro heterocycloalkyl is

optionally substituted by $-CO-R^{11}$}, or
(f) 6- to 9-membered saturated or partially unsaturated fused ring group containing 1 or 2 nitrogen atoms (wherein the fused ring group is optionally substituted by $-CO-R^{11}$).

One of the more preferred embodiments of the compound of the formula [II] is a compound of the formula [II] wherein

$X^1$, $X^2$, $X^3$ and $X^4$ are each independently a carbon or a nitrogen atom (wherein the total number of the nitrogen atoms for $X^1$, $X^2$, $X^3$ and $X^4$ is 0 or 1);
$Y^1$ and $Y^2$ are each a nitrogen atom;
$Y^3$ and $Y^4$ are each a carbon atom;
m is 0 or 1;
n is 0 or 1;
$R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl;

$R^2$ in the number of m are each independently halogen;
$R^3$ in the number of n are each independently halogen;
$R^4$ is

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

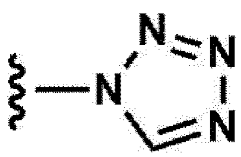
),

(V) $C_{1-6}$ alkoxy (wherein the alkoxy is optionally substituted by hydroxy or $-SO_2-C_{1-4}$ alkyl),
(VI) carboxy,
(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,

(X) a group represented by the formula:

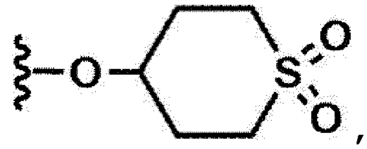

(XI) a group represented by the formula:

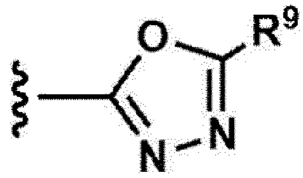

{wherein $R^9$ is $C_{1-4}$ alkyl (wherein the alkyl is optionally substituted by $C_{1-4}$ alkoxy)}, and
(XII) a group represented by the formula:

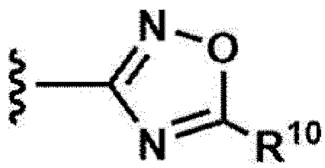

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy, or
(ii) $-SO_2-C_{1-4}$ alkyl),

(III) carboxy, or
(IV) $-CO-C_{1-4}$ alkoxy},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl, and
(III) $-CO-R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}],

(d) 8-membered bridged heterocycloalkyl containing one nitrogen atom {wherein the bridged heterocycloalkyl is -CO-$R^{11a}$ {wherein $R^{11a}$ is optionally substituted by

(i) $C_{1-6}$ alkyl (wherein the alkyl is optionally substituted by

(A) hydroxy, or
(B) cyano), or

(ii) $C_{1-6}$ alkoxy}},

(e) 7- to 11-membered spiro heterocycloalkyl containing one nitrogen atom {wherein the spiro heterocycloalkyl is optionally substituted by -CO-$R^{11a}$}, or
(f) 6- to 9-membered saturated or partially unsaturated fused ring group containing 1 or 2 nitrogen atoms (wherein the fused ring group is optionally substituted by -CO-$C_{1-6}$ alkyl (wherein the alkyl is optionally substituted by hydroxy)).

One of the further preferred embodiments of the compound of the formula [II] is a compound of the formula [II] wherein $X^1$, $X^2$, $X^3$ and $X^4$ are each independently a carbon or a nitrogen atom (wherein the total number of the nitrogen atoms for $X^1$, $X^2$, $X^3$ and $X^4$ is 0 or 1);

$Y^1$ and $Y^2$ are each a nitrogen atom;
$Y^3$ and $Y^4$ are each a carbon atom;
m is 0 or 1;
n is 0 or 1;
$R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl;

$R^2$ in the number of m are each independently halogen;
$R^3$ in the number of n are each independently halogen;
$R^4$ is

(a) $C_{4-6}$ cycloalkyl (wherein the cycloalkyl is optionally substituted by

(I) hydroxy,
(II) cyano,
(III) $C_{1-6}$ alkyl (wherein the alkyl is optionally substituted by

(i) hydroxy,
(ii) carboxy,
(iii) -$CONH_2$,
(iv) -CO-$C_{1-4}$ alkoxy,
(v) -$SO_2$-$C_{1-4}$ alkyl, or
(vi) a group represented by the formula:

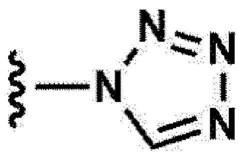 ),

(IV) $C_{1-6}$ alkoxy (wherein the alkoxy is optionally substituted by hydroxy or -$SO_2$-$C_{1-4}$ alkyl),
(V) -CO-$NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],

(VI) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(VII) a group represented by the formula:

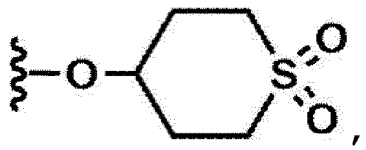

(VIII) a group represented by the formula:

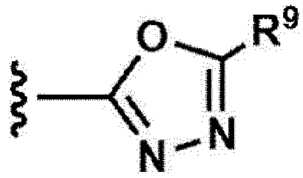

{wherein $R^9$ is $C_{1-4}$ alkyl (wherein the alkyl is optionally substituted by $C_{1-4}$ alkoxy)}, or
(IX) a group represented by the formula:

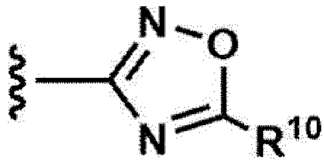

wherein $R^{10}$ is $C_{1-4}$ alkyl)),

(b) $C_{5-8}$ bridged cycloalkyl {wherein the bridged cycloalkyl is optionally substituted by $C_{1-6}$ alkyl (wherein the alkyl is optionally substituted by hydroxy)},
(c) 5- or 6-membered heterocycloalkyl containing one nitrogen atom [wherein the heterocycloalkyl is optionally substituted by $-CO-R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}], or

(d) 8-membered bridged heterocycloalkyl containing one nitrogen atom {wherein the bridged heterocycloalkyl is optionally substituted by $-CO-C_{1-6}$ alkyl (wherein the alkyl is optionally substituted by

(A) hydroxy, or
(B) cyano)}.

One of the preferred embodiments of the compound of the formula [I] is a compound of the formula [I] wherein

$X^1$ is carbon or a nitrogen atom;
$Y^1$ and $Y^2$ are each a nitrogen atom;
$Y^3$ and $Y^4$ are each a carbon atom;

m is 0 or 1;
n is 0 or 1;
$R^1$ is

(1) hydroxy,
(2) $C_{1-4}$ alkyl (wherein the alkyl is optionally substituted by hydroxy or $C_{1-4}$ alkoxy),
(3) $C_{1-4}$ alkoxy,
(4) halogen, or
(5) $C_{1-4}$ haloalkyl;

$R^2$ in the number of m are each independently halogen;
$R^3$ in the number of n are each independently halogen;
$R^4$ is

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

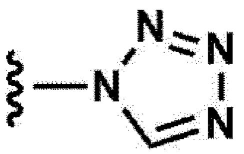
),

(V) $C_{1-6}$ alkoxy (wherein the alkoxy is optionally substituted by hydroxy or $-SO_2$-$C_{1-4}$ alkyl),
(VI) carboxy,
(VII) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or -CO-$C_{1-6}$ alkyl (wherein the alkyl is optionally substituted by cyano)},
(IX) -O-$C_{1-6}$ haloalkyl,
(X) a group represented by the formula:

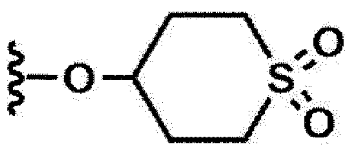
,

(XI) a group represented by the formula:

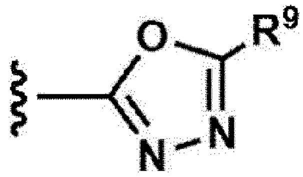

{wherein $R^9$ is $C_{1-4}$ alkyl (wherein the alkyl is optionally substituted by $C_{1-4}$ alkoxy)}, and
(XII) a group represented by the formula:

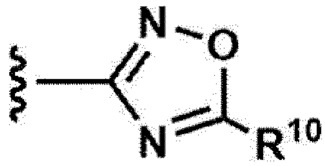

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

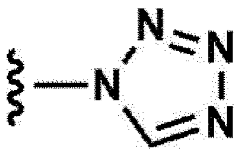

),

(III) $C_{1-6}$ alkoxy (wherein the alkoxy is optionally substituted by hydroxy or $-SO_2$-$C_{1-4}$ alkyl),
(IV) carboxy,
(V) -CO-$C_{1-4}$ alkoxy,
(VI) -O-$C_{1-6}$ haloalkyl, or
(VII) a group represented by the formula:

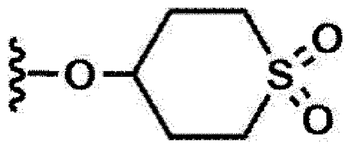

},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl,
(III) -CO-$R^{11}$ {wherein $R^{11}$ is

(i) $C_{1-6}$ alkyl (wherein the alkyl is optionally substituted by

(A) hydroxy,

(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

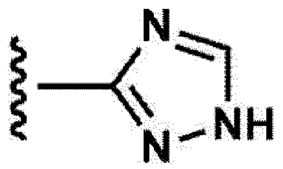
],

(d) 8-membered bridged heterocycloalkyl containing one nitrogen atom {wherein the bridged heterocycloalkyl is optionally substituted by -CO-$R^{11}$},
(e) 7- to 11-membered spiro heterocycloalkyl containing one nitrogen atom {wherein the spiro heterocycloalkyl is optionally substituted by -CO-$R^{11}$}, or
(f) 6- to 9-membered saturated or partially unsaturated fused ring group containing 1 or 2 nitrogen atoms (wherein the fused ring group is optionally substituted by -CO-$R^{11}$).

One of the more preferred embodiments of the compound of the formula [I] is a compound of the formula [I] wherein

$X^1$ is carbon or a nitrogen atom;
$Y^1$ and $Y^2$ are each a nitrogen atom;
$Y^3$ and $Y^4$ are each a carbon atom;
m is 0 or 1;
n is 0 or 1;
$R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl,;

$R^2$ in the number of m are each independently halogen;
$R^3$ in the number of n are each independently halogen;
$R^4$ is

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2-C_{1-4}$ alkyl, or
(vi) a group represented by the formula:

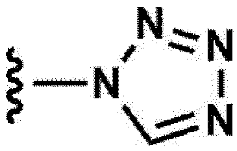

),

(V) $C_{1-6}$ alkoxy (wherein the alkoxy is optionally substituted by hydroxy or $-SO_2-C_{1-4}$ alkyl),
(VI) carboxy,
(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,
(X) a group represented by the formula:

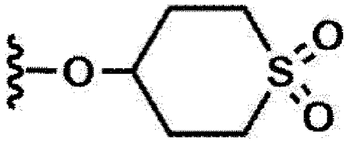

,

(XI) a group represented by the formula:

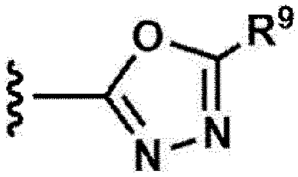

{wherein $R^9$ is $C_{1-4}$ alkyl (wherein the alkyl is optionally substituted by $C_{1-4}$ alkoxy)}, and
(XII) a group represented by the formula:

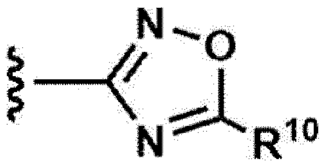

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy, or
(ii) $-SO_2-C_{1-4}$ alkyl,),

(III) carboxy, or
(IV) $-CO-C_{1-4}$ alkoxy,},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl, and
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}],

(d) 8-membered bridged heterocycloalkyl containing one nitrogen atom {wherein the bridged heterocycloalkyl is optionally substituted by -CO-$R^{11a}$ {wherein $R^{113}$ is

(i) $C_{1-6}$ alkyl (wherein the alkyl is optionally substituted by

(A) hydroxy, or
(B) cyano), or

(ii) $C_{1-6}$ alkoxy}},

(e) 7- to 11-membered spiro heterocycloalkyl containing one nitrogen atom {wherein the spiro heterocycloalkyl is optionally substituted by -CO-$R^{11a}$}, or
(f) 6- to 9-membered saturated or partially unsaturated fused ring group containing 1 or 2 nitrogen atoms (wherein the fused ring group is optionally substituted by -CO-$C_{1-6}$ alkyl (wherein the alkyl is optionally substituted by hydroxy)).

One of the further preferred embodiments of the compound of the formula [I] is a compound of the formula [I] wherein

$X^1$ is carbon or a nitrogen atom;
$Y^1$ and $Y^2$ are each a nitrogen atom;
$Y^3$ and $Y^4$ are each a carbon atom;
m is 0 or 1;
n is 0 or 1;

$R^1$ is

(1) $C_{1-4}$ alkyl (wherein the alkyl is optionally substituted by hydroxy or $C_{1-4}$ alkoxy),
(2) halogen, or
(3) $C_{1-4}$ haloalkyl;

$R^2$ in the number of m are each independently halogen;
$R^3$ in the number of n are each independently halogen;
$R^4$ is

(a) $C_{4-6}$ cycloalkyl (wherein the cycloalkyl is optionally substituted by

(I) hydroxy,
(II) cyano,

(III) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

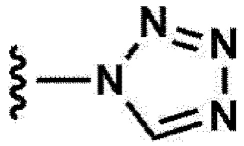

),

(IV) $C_{1-6}$ alkoxy (wherein the alkoxy is optionally substituted by hydroxy or $-SO_2$-$C_{1-4}$ alkyl),
(V) $-CO-NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],
(VI) $-NR^7R^8$ {wherein $R^7$ and $R^8$ are each independently $C_{1-4}$ alkyl or -CO-$C_{1-6}$ alkyl (wherein the alkyl is optionally substituted by cyano)},
(VII) a group represented by the formula:

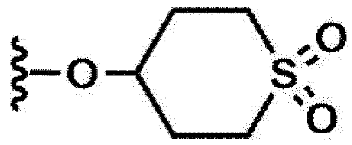

,

(VIII) a group represented by the formula:

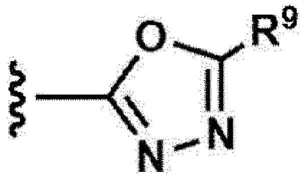

{wherein $R^9$ is $C_{1-4}$ alkyl (wherein the alkyl is optionally substituted by $C_{1-4}$ alkoxy)}, or
(IX) a group represented by the formula:

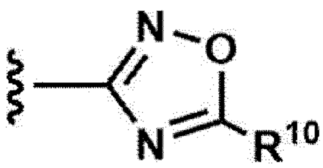

wherein $R^{10}$ is $C_{1-4}$ alkyl)),

(b) $C_{5-8}$ bridged cycloalkyl {wherein the bridged cycloalkyl is optionally substituted by $C_{1-6}$ alkyl (wherein the alkyl is optionally substituted by hydroxy)},
(c) 5- or 6-membered heterocycloalkyl containing one nitrogen atom [wherein the heterocycloalkyl is optionally substituted by $-CO-R^{11}$ {wherein $R^{11}$ is

(i) $C_{1-6}$ alkyl (wherein the alkyl is optionally substituted by

(A) hydroxy,

(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}], or

(d) 8-membered bridged heterocycloalkyl containing one nitrogen atom {wherein the bridged heterocycloalkyl is optionally substituted by -CO-$C_{1-6}$ alkyl (wherein the alkyl is optionally substituted by

(A) hydroxy, or
(B) cyano)}.

One of the preferred embodiments of the compound of the formula [IC] is a compound represented by the formula [ICa]:

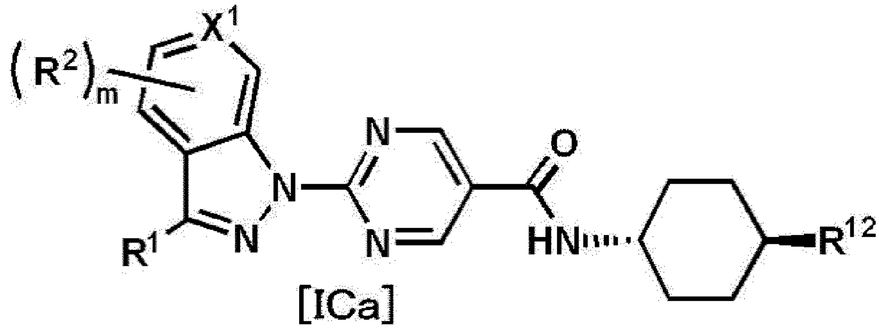

[ICa]

wherein $X^1$, $R^1$, $R^2$, $R^{12}$ and m are as defined above.
One of the more preferred embodiments of the compound of the formula [IC] is a compound of the formula [ICa], wherein $X^1$, $R^1$, $R^2$ and m are as defined above:

$R^{12}$ is

(1) hydroxy,
(2) cyano,
(3) $C_{1-6}$ alkyl (wherein the alkyl is optionally substituted by

(a) hydroxy,
(b) carboxy,
(c) -$CONH_2$,
(d) -CO-$C_{1-4}$ alkoxy,
(e) -$SO_2$-$C_{1-4}$ alkyl, or
(f) a group represented by the formula:

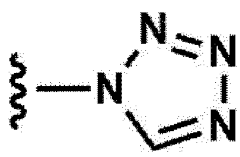

),

(4) $C_{1-6}$ alkoxy (wherein the alkoxy is optionally substituted by hydroxy or -$SO_2$-$C_{1-4}$ alkyl),
(5) -CO-$NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],

(6) $-NR^7R^8$ {wherein $R^7$ and $R^8$ are each independently $C_{1-4}$ alkyl or $-CO-C_{1-6}$ alkyl (wherein the alkyl is optionally substituted by cyano) },
(7) a group represented by the formula:

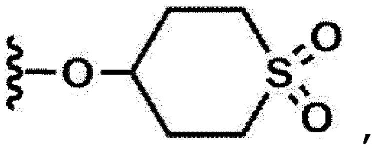

(8) a group represented by the formula:

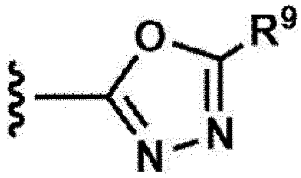

{wherein $R^9$ is $C_{1-4}$ alkyl (wherein the alkyl is optionally substituted by $C_{1-4}$ alkoxy)}, or
(9) a group represented by the formula:

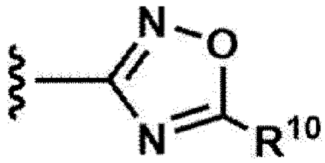

wherein $R^{10}$ is $C_{1-4}$ alkyl) .

One of the preferred embodiments of the compound of the formula [III] is a compound of the formula [III] wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2);
m is 0 or 1;
n is 0, 1 or 2;
$R^1$ is

(1) $C_{1-4}$ alkyl,
(2) $C_{1-4}$ haloalkyl, or
(3) $C_{3-6}$ cycloalkyl;

$R^2$ in the number of m are each independently

(1) cyano,
(2) $C_{1-4}$ alkyl,
(3) $C_{1-4}$ alkoxy, or
(4) halogen;

$R^3$ in the number of n are each independently halogen;
$R^4$ is

(a) $C_{4-6}$ cycloalkyl (wherein the cycloalkyl is optionally substituted by 1 or 2 substituents independently selected from the group consisting of

(I) hydroxy,
(II) cyano,

(III) oxo,

(IV) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

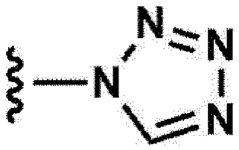
),

(V) $C_{1-6}$ alkoxy (wherein the alkoxy is optionally substituted by hydroxy or $-SO_2-C_{1-4}$ alkyl),

(VI) carboxy,

(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},

(IX) $-O-C_{1-6}$ haloalkyl,

(X) a group represented by the formula:

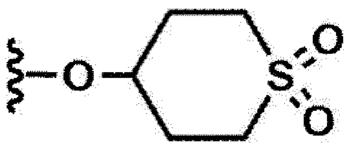
,

(XI) a group represented by the formula:

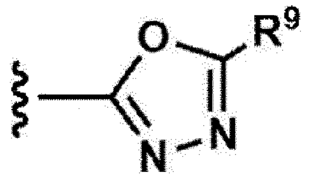

{wherein $R^9$ is $C_{1-4}$ alkyl (wherein the alkyl is optionally substituted by $C_{1-4}$ alkoxy)}, and

(XII) a group represented by the formula:

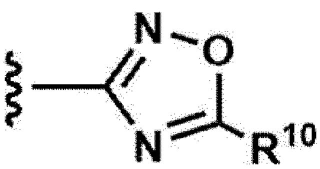

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

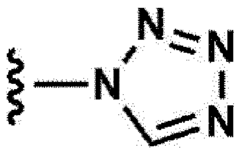
),

(III) $C_{1-6}$ alkoxy (wherein the alkoxy is optionally substituted by hydroxy or $-SO_2$-$C_{1-4}$ alkyl),
(IV) carboxy,
(V) -CO-$C_{1-4}$ alkoxy,
(VI) -O-$C_{1-6}$ haloalkyl, or
(VII) a group represented by the formula:

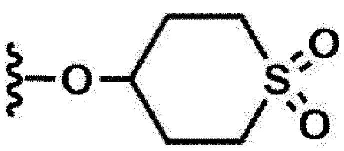
},

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl,
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

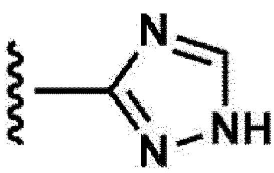
],

(d) a 6- to 9-membered saturated or partially unsaturated fused ring group containing 1 or 2 nitrogen atoms (wherein the fused ring group is optionally substituted by $-CO-R^{11}$), or

(e) a group represented by the formula:

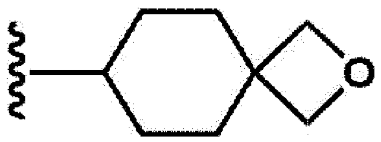

.

One of the more preferred embodiments of the compound of the formula [III] is a compound of the formula [III] wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2);
m is 0 or 1;
n is 0, 1 or 2;
$R^1$ is $C_{1-4}$ alkyl;
$R^2$ in the number of m are each independently halogen;
$R^3$ in the number of n are each independently halogen;
$R^4$ is

(a) $C_{4-6}$ cycloalkyl (wherein the cycloalkyl is optionally substituted by 1 or 2 substituents independently selected from the group consisting of

(I) hydroxy,
(II) cyano,
(III) $C_{1-6}$ alkyl (wherein the alkyl is optionally substituted by

(i) hydroxy,
(ii) carboxy, or
(iii) $-SO_2-C_{1-4}$ alkyl,),

(IV) $-O-C_{1-6}$ haloalkyl,
(V) a group represented by the formula:

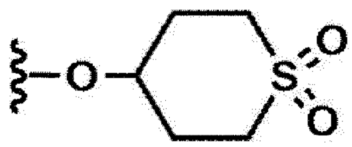

,

and
(VI) a group represented by the formula:

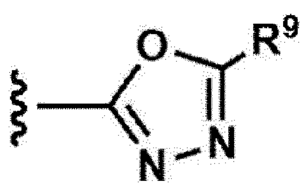

{wherein $R^9$ is $C_{1-4}$ alkyl}),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy, or
(iii) -CO-$C_{1-4}$ alkoxy), or

(III) carboxy},

(c) 5- or 6-membered heterocycloalkyl containing one nitrogen atom [wherein the heterocycloalkyl is optionally substituted by a group represented by the formula:

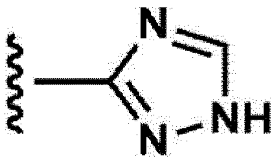
],

(d) a 6- to 9-membered saturated or partially unsaturated fused ring group containing 1 or 2 nitrogen atoms, or
(e) a group represented by the formula:

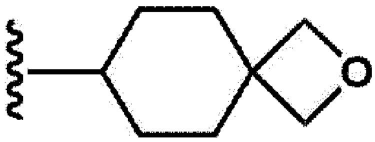
.

One of the preferred embodiments of the compound of the formula [IV] is a compound of the formula [IV] wherein

$X^1$, $X^5$ and $X^6$ are each independently a carbon or a nitrogen atom (wherein the total number of the nitrogen atoms for $X^5$ and $X^6$ is 1);
$Y^1$ and $Y^2$ are each a nitrogen atom;
$Y^3$ and $Y^4$ are each a carbon atom;
m is 0;
n is 0 or 1;
$R^1$ is $C_{1-4}$ alkyl;
$R^3$ in the number of n are each independently halogen;
$R^4$ is $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) -$CONH_2$,
(iv) -CO-$C_{1-4}$ alkoxy,
(v) -$SO_2$-$C_{1-4}$ alkyl, or
(vi) a group represented by the formula:

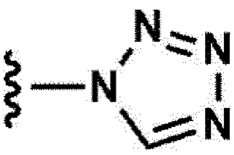
),

(V) $C_{1-6}$ alkoxy (wherein the alkoxy is optionally substituted by hydroxy or -$SO_2$-$C_{1-4}$ alkyl),
(VI) carboxy,
(VII) -CO-$NR^5R^6$ [wherein $R^5$ and $R^6$ are each independently $C_{1-4}$ alkyl (wherein the alkyl is optionally substituted

by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,
(X) a group represented by the formula:

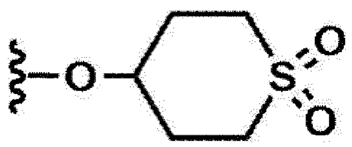

,

(XI) a group represented by the formula:

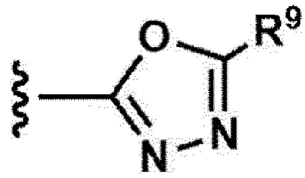

{wherein $R^9$ is $C_{1-4}$ alkyl (wherein the alkyl is optionally substituted by $C_{1-4}$ alkoxy)}, and
(XII) a group represented by the formula:

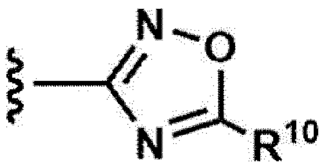

wherein $R^{10}$ is $C_{1-4}$ alkyl)).

One of the more preferred embodiments of the compound of the formula [IV] is a compound of the formula [IV] wherein

$X^1$, $X^5$ and $X^6$ are each independently a carbon or a nitrogen atom (wherein the total number of the nitrogen atoms for $X^5$ and $X^6$ is 1);
$Y^1$ and $Y^2$ are each a nitrogen atom;
$Y^3$ and $Y^4$ are each a carbon atom;
m is 0;
n is 0 or 1;
$R^1$ is $C_{1-4}$ alkyl;
$R^3$ in the number of n are each independently halogen;
$R^4$ is $C_{4-6}$ cycloalkyl (wherein the cycloalkyl is optionally substituted by

(I) hydroxy, or
(II) $C_{1-6}$ alkyl (wherein the alkyl is optionally substituted by

(i) hydroxy, or
(ii) carboxy)).

The "pharmaceutically acceptable salt" may be any salt known in the art that does not accompany excessive toxicity. Specifically, salts with inorganic acids, salts with organic acid, salts with inorganic bases, salts with organic bases and the like can be mentioned. Various forms of pharmaceutically acceptable salts are well-known in the pertinent technical field and are described, for example, in the following reference documents:

(a) Berge et al., J. Pharm. Sci., 66, p1-19 (1977),
(b) Stahl et al., "Handbook of Pharmaceutical Salts: Properties, Selection, and Use" (Wiley-VCH, Weinheim, Germany, 2002),
(c) Paulekuhn et al., J. Med. Chem., 50, p6665-6672 (2007).

Pharmaceutically acceptable salts of the compound of the present invention can be each obtained by reacting the compound of the present invention with an inorganic acid, an organic acid, an inorganic base, or an organic base according to a method known per se.

Examples of the salt with inorganic acid include salts with hydrofluoric acid, hydrochloric acid, hydrobromic acid, hydroiodic acid, nitric acid, phosphoric acid, and sulfuric acid.

Examples of the salt with organic acid include salts with acetic acid, adipic acid, alginic acid, 4-aminosalicylic acid, anhydromethylenecitric acid, benzoic acid, benzenesulfonic acid, calcium edetate, camphoric acid, 10-camphorsulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glycolylarsanilic acid, hexylresorcinic acid, hydroxy-naphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis (salicylic acid), galactaric acid, naphthalene-2-sulfonic acid, 2-naphthoic acid, 1,5-naphthalene disulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid, and glutamic acid.

Examples of the salt with inorganic base include salts with lithium, sodium, potassium, magnesium, calcium, barium, aluminum, zinc, bismuth, and ammonium.

Examples of the salt with organic base include salts with arecoline, betaine, choline, clemizole, ethylenediamine, N-methylglucamine, N-benzylphenethylamine, tris(hydroxymethyl)methylamine, arginine, and lysine.

Preferred embodiments of the "pharmaceutically acceptable salt" are as follows.

Examples of the salts with inorganic acid include salts with hydrochloric acid, nitric acid, sulfuric acid, phosphoric acid, and hydrobromic acid.

Examples of the salts with organic acid include salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid, and 2-hydroxy-1-ethanesulfonic acid.

Examples of the salts with inorganic base include salts with sodium, potassium, calcium, magnesium, and zinc.

Examples of the salts with organic base include salts with tris(hydroxymethyl)methylamine, N-methylglucamine, and lysine.

The compound of the present invention or a pharmaceutically acceptable salt thereof may exist as a solvate thereof. The "solvate" refers to the compound of the present invention or a pharmaceutically acceptable salt thereof with which a solvent molecule is coordinated, and also includes hydrates. Such solvates are preferably pharmaceutically acceptable solvates. Such solvates include, for example, hydrate, ethanol solvate, dimethylsulfoxide-solvate, and the like of the compound of the present invention or a pharmaceutically acceptable salt thereof.

Specific examples include hemihydrate, monohydrate, dihydrate or mono ethanol solvate of the compound of the present invention or a monohydrate of a sodium salt of the compound of the present invention, 2/3 ethanol solvate of dihydrochloride of the compound of the present invention, and the like. These solvates can be obtained according to conventional methods.

The compound of the present invention may exist as a tautomer. In this case, the compound of the present invention can be a single tautomer or a mixture of individual tautomers. For example, the structure represented by the following formula:

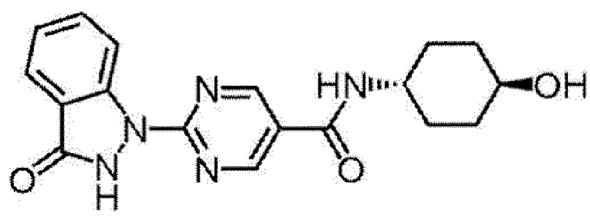

is, unless otherwise indicated, present as

(1)

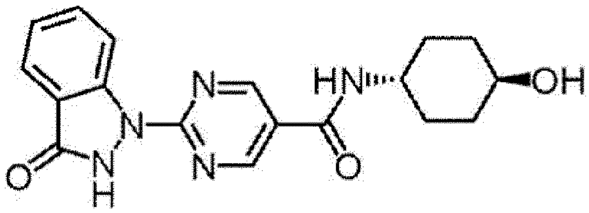

(2)

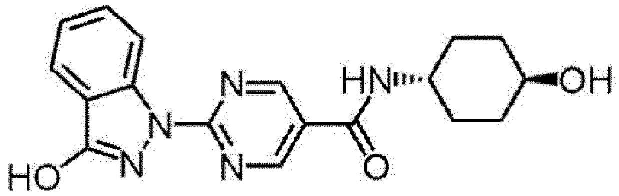

or

(3) a mixture of these.

The compound of the present invention may have a carbon double bond. In this case, the compound of the present invention can be present as E form, Z form, or a mixture of E form and Z form.

The compound of the present invention may contain a stereoisomer that should be recognized as a cis/trans isomer. In this case, the compound of the present invention can be present as a cis form, a trans form, or a mixture of a cis form and a trans form.

The compound of the present invention may contain one or more asymmetric carbons. In this case, the compound of the present invention may be present as a single enantiomer, a single diastereomer, a mixture of enantiomers or a mixture of diastereomers.

The compound of the present invention may be present as an atropisomer. In this case, the compound of the present invention may be present as an individual atropisomer or a mixture of atropisomers.

The compound of the present invention may simultaneously contain plural structural characteristics that produce the above-mentioned isomers. Moreover, the compound of the present invention may contain the above-mentioned isomers at any ratio.

In the absence of other reference such as annotation and the like, the formulae, chemical structures and compound names indicated in the present specification without specifying the stereochemistry thereof encompass all the above-mentioned isomers that may exist.

A diastereomeric mixture can be separated into each diastereomer by conventional methods such as chromatography, crystallization and the like. In addition, each diastereomer can also be formed by using a stereochemically single starting material, or by a synthetic method using a stereoselective reaction.

An enantiomeric mixture can be separated into each single enantiomer by a method well-known in the pertinent technical field.

For example, from a diastereomeric mixture formed by reacting a mixture of enantiomers with a compound known as a chiral auxiliary, which is a substantially pure enantiomer, the isomer ratio can be increased or a single substantially pure diastereomer can be separated from the diastereomeric mixture. The separated diastereomer can be converted to a desired enantiomer by removing the added chiral auxiliary by a cleavage reaction. Alternatively, a mixture of enantiomers can be directly separated by a chromatographic method using a chiral stationary phase, as is well-known in the pertinent technical field. Alternatively, either enantiomer can be obtained by using substantially pure optically active starting materials or by stereoselective synthesis (asymmetric induction) of a prochiral intermediate using a chiral auxiliary and an asymmetric catalyst.

The absolute steric configuration can be determined based on the X-ray crystal analysis of the resultant crystalline product or intermediate. In this case, a resultant crystalline product or intermediate derivatized with a reagent having an asymmetric center with a known steric configuration may be used where necessary.

The compound of the present invention may be labeled with an isotope element ($^2H$(D), $^3H$, $^{14}C$, $^{18}F$, $^{35}S$, etc.). For example, when the compound of the formula [I] has a methyl group, the methyl group is replaced with $-CD_3$ group, and the thus-obtained compound is also encompassed in the present invention.

As the compound of the present invention or a pharmaceutically acceptable salt thereof, the compound of the present invention or a pharmaceutically acceptable salt thereof, each of which is substantially purified, is preferred. More preferred is the compound of the present invention or a pharmaceutically acceptable salt thereof, each of which is purified to a chemical purity of not less than 80%.

The pharmaceutical composition of the present invention may be produced according to a method known in the technical field of pharmaceutical preparations, by mixing the compound of the present invention or a pharmaceutically acceptable salt thereof with a suitable amount of at least one kind of pharmaceutically acceptable carrier and the like as appropriate. While the content of the compound of the present invention or a pharmaceutically acceptable salt thereof in the pharmaceutical composition varies depending on the dosage form, dose and the like, it is, for example, 0.1 to 100 wt% of the whole composition.

The dosage form of a pharmaceutical composition containing the compound of the present invention or a pharmaceutically acceptable salt thereof (hereinafter also to be referred to as "the pharmaceutical composition of the present invention" in the present specification) include oral preparations such as tablet, capsule, granule, powder, troche, syrup, emulsion, suspension and the like, and parenteral agents such as external preparation, suppository, injection, eye drop, nasal preparations, pulmonary preparation and the like.

Examples of the "pharmaceutically acceptable carrier" include various organic or inorganic carrier substances conventionally used as preparation materials, for example, excipient, disintegrant, binder, fluidizer, lubricant, and the like for solid preparations, solvent, solubilizing agent, suspending agent, isotonicity agent, buffering agent, soothing agent and the like for liquid preparations, and base, emulsifier, wetting agent, stabilizer, stabilizing agent, dispersing agent, plasticizer, pH regulator, absorption promoter, gelling agent, antiseptic, filler, dissolving agent, solubilizing agent, suspending agent, and the like for semisolid preparations. Where necessary, moreover, additives such as preservative, antioxidant, colorant, sweetening agent, and the like may be used.

Examples of the "excipient" include lactose, sucrose, D-mannitol, D-sorbitol, cornstarch, dextrin, microcrystalline cellulose, crystalline cellulose, carmellose, carmellose calcium, sodium carboxymethyl starch, low-substituted hydroxypropyl cellulose, gum arabic, and the like.

Examples of the "disintegrant" include carmellose, carmellose calcium, carmellose sodium, sodium carboxymethyl starch, croscarmellose sodium, crospovidone, low-substituted hydroxypropyl cellulose, hydroxypropylmethylcellulose, crystalline cellulose, and the like.

Examples of the "binder" include hydroxypropyl cellulose, hydroxypropylmethylcellulose, povidone, crystalline cellulose, sucrose, dextrin, starch, gelatin, carmellose sodium, gum arabic, and the like.

Examples of the "fluidizer" include light anhydrous silicic acid, magnesium stearate, and the like.

Examples of the "lubricant" include magnesium stearate, calcium stearate, talc, and the like.

Examples of the "solvent" include purified water, ethanol, propylene glycol, macrogol, sesame oil, corn oil, olive oil, and the like.

Examples of the "solubilizing agents" include propylene glycol, D-mannitol, benzyl benzoate, ethanol, triethanolamine, sodium carbonate, sodium citrate, and the like.

Examples of the "suspending agent" include benzalkonium chloride, carmellose, hydroxypropylcellulose, propylene glycol, povidone, methylcellulose, glycerol monostearate, and the like.

Examples of the "isotonic agent" include glucose, D-sorbitol, sodium chloride, D-mannitol, and the like.

Examples of the "buffering agent" include sodium hydrogen phosphate, sodium acetate, sodium carbonate, sodium citrate, and the like.

Examples of the "soothing agent" include benzyl alcohol, and the like.

Examples of the "base" include water, animal and vegetable oils (olive oil, corn oil, peanut oil, sesame oil, castor oil, etc.), lower alcohols (ethanol, propanol, propylene glycol, 1,3-butyleneglycol, phenol, etc.), higher fatty acid and ester thereof, waxes, higher alcohol, polyhydric alcohol, hydrocarbons (white petrolatum, liquid paraffin, paraffin, etc.), hydrophilic petrolatum, purified lanolin, absorption ointment, hydrolyzed lanolin, hydrophilic ointment, starch, pullulan, gum arabic, gum tragacanth, gelatin, dextran, cellulose derivative (methyl cellulose, carboxymethyl cellulose, hydroxyethyl cellulose, hydroxypropyl cellulose, etc.), synthetic polymers (carboxyvinyl polymer, sodium polyacrylate, polyvinyl alcohol, polyvinylpyrrolidone, etc.), propylene glycol, macrogol (macrogol 200-600, etc.), and combinations of two or more kinds thereof.

Examples of the "preservative" include ethyl parahydroxybenzoate, chlorobutanol, benzyl alcohol, sodium dehydroacetate, sorbic acid, and the like.

Examples of the "antioxidant" include sodium sulfite, ascorbic acid and the like.

Examples of the "colorant" include food colors (e.g., Food Color Red No. 2 or 3, Food Color yellow No. 4 or 5, etc.), β-carotene, and the like.

Examples of the "sweetening agent" include saccharin sodium, dipotassium glycyrrhizinate, aspartame, and the like.

The pharmaceutical composition of the present invention can be administered orally or parenterally (topical, rectal, intravenous administration, intramuscular, subcutaneous, etc.) to human as well as mammals other than human (e.g., mouse, rat, hamster, guinea pig, rabbit, cat, dog, swine, bovine, horse, sheep, monkey, etc.). The dose of the compound of the present invention or a pharmaceutically acceptable salt thereof (hereinafter also to be referred to as a "pharmaceutically effective amount" in the present specification) may appropriately vary depending on the subject of administration, administration route, target disease, symptoms, severity of the disease, and combinations of these. For example, the daily dose for oral administration to an adult patient is generally within the range of about 0.01 mg to 1 g, based on the compound of the present invention as the active ingredient. This amount can be administered in one to several portions.

Since the compound of the present invention or a pharmaceutically acceptable salt thereof has an H-PGDS inhibitory action, it is useful for the treatment and/or prophylaxis of various diseases or conditions that can be expected to be improved by regulation of H-PGDS activity. Examples of the various diseases or conditions that can be expected to be improved by regulation of H-PGDS activity include peripheral arterial diseases (e.g., intermittent claudication and comprehensive severe chronic lower extremity ischemia), cardiovascular diseases, allergic asthma, chronic obstructive pulmonary diseases, allergic rhinitis, sarcopenia, and Duchenne muscular dystrophy.

To "inhibit H-PGDS" means to inhibit the function of H-PGDS and eliminate or attenuate the activity. For example, it means to inhibit the function of H-PGDS based on the below-mentioned Experimental Example 1. To "inhibit H-PGDS", human H-PGDS is preferably inhibited.

The "H-PGDS inhibitor" means a substance that inhibits the function of H-PGDS. For example, an H-PGDS inhibitor is expected to be useful for the treatment and/or prophylaxis of a disease selected from the group consisting of peripheral arterial diseases (e.g., intermittent claudication and comprehensive severe chronic lower extremity ischemia), cardiovascular diseases, allergic asthma, chronic obstructive pulmonary diseases, allergic rhinitis, sarcopenia, and Duchenne muscular dystrophy.

In the present specification, the "treatment" includes improvement of symptoms, prevention of aggravation, maintenance of remission, prevention of relapse, and further, prevention of recurrence.

In the present specification, "prophylaxis" means suppression of the onset of symptoms.

Unless there is a contradiction between one embodiment disclosed in a certain part of the specification and embodiments disclosed in other parts, any combination of two or more of thereof is intended to be encompassed in the present invention.

Preferred specific embodiment of the active ingredient in the H-PGDS inhibitor or pharmaceutical composition of the present invention is the compound of the present invention or a pharmaceutically acceptable salt thereof.

[General production methods of the compound of the present invention or a pharmaceutically acceptable salt thereof]

A general method for producing the compound of the formula [I] or a pharmaceutically acceptable salt thereof, the compound of the formula [II] or a pharmaceutically acceptable salt thereof, the compound of the formula [III] or a pharmaceutically acceptable salt thereof, or the compound of formula [IV] or a pharmaceutically acceptable salt thereof is exemplified below. However, the production method of the compound of the formula [I] or a pharmaceutically acceptable salt thereof, the compound of the formula [II] or a pharmaceutically acceptable salt thereof, the compound of the formula [III] or a pharmaceutically acceptable salt thereof, or the compound of the formula [IV] or a pharmaceutically acceptable salt thereof is not limited thereto.

The compound obtained in each step can be isolated and/or purified as necessary by known methods such as distillation, recrystallization, and column chromatography. In some cases, the compound can proceed to the next step without isolation and/or purification.

In the present specification, the room temperature refers to a temperature in the state where the temperature is not controlled, and one embodiment is 1°C to 40°C.

Production method A1: Production method of compound [II] or a salt thereof

Compound [II] or a salt thereof can be produced, for example, by production method A1 shown below:

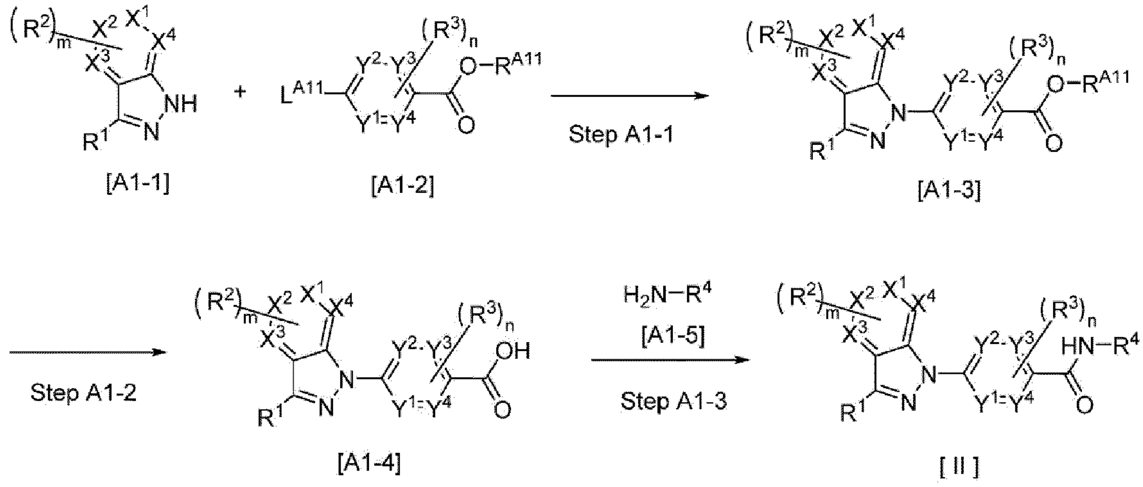


[A1-1] [A1-2] [A1-3] [A1-4] [ II ]

wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2),
$R^{A11}$ is a carboxy-protecting group (e.g., methyl, ethyl and tert-butyl),
$L^{A11}$ is a leaving group {for example, halogen, boronic acid, and boronic acid ester (e.g., boronic acid pinacol ester)}, and
$R^1$, $R^2$, $R^3$, $R^4$, m and n are as defined above.

(Step A1-1)

Compound [A1-3] or a salt thereof can be obtained by reacting compound [A1-1] or a salt thereof with compound [A1-2] or a salt thereof in a solvent in the presence of a base by adding a metal catalyst as necessary.
Examples of the solvent include tetrahydrofuran, N,N-dimethylformamide, N,N-dimethylacetamide, dimethyl sulfoxide, dichloromethane and mixed solvents thereof. Preferred solvent is N,N-dimethylformamide.
Examples of the base include potassium tert-butoxide, potassium carbonate, cesium carbonate and pyridine. Preferred base is potassium carbonate.
Examples of the metal catalyst include copper iodide (I) and copper acetate (II).
The reaction temperature is, for example, 20°C to 120°C, preferably 20°C to 90°C.
Compound [A1-1] or a salt thereof may be a commercially available product, or produced from a commercially available product by a known method.
Compound [A1-2] or a salt thereof may be a commercially available product, or produced from a commercially available product by a known method.

(Step A1-2)

Compound [A1-4] or a salt thereof can be produced by removing $R^{A11}$ of compound [A1-3] or a salt thereof by a deprotection reaction. The deprotection reaction may be carried out under conditions suitable for the kind of $R^{A11}$. For example, when $R^{A11}$ is ethyl, compound [A1-4] or a salt thereof can be produced by hydrolyzing compound [A1-3] or a salt thereof in a solvent in the presence of a base.
Examples of the base include lithium hydroxide·monohydrate, sodium hydroxide, and potassium hydroxide.
Examples of the solvent include methanol, tetrahydrofuran, water, and mixed solvents thereof. Preferred solvent is a mixed solvent of tetrahydrofuran and water.
The reaction temperature is, for example, 20°C to 80°C, preferably 20°C to 30°C.

(Step A1-3)

Compound [II] or a salt thereof can be produced by reacting compound [A1-4] or a salt thereof with compound [A1-5] or a salt thereof in a solvent in the presence of a condensing agent and a base.
Examples of the condensing agent include dicyclohexylcarbodiimide (DCC), 1-ethyl-3-(3-dimethylaminopropyl)carbodiimide hydrochloride (WSC·HCl), diisopropyl carbodiimide, 1,1'-carbonyldiimidazole (CDI), 1-hydroxy-7-azabenzotriazole, O-(7-azabenzotriazol-1-yl)-N,N,N',N'-tetramethyluronium hexafluorophosphate (HATU), hexafluorophosphoric acid (benzotriazol-1-yloxy)tripyrrolidinophosphonium (PyBOP), (1-cyano-2-ethoxy-2-oxoethylideneaminooxy)dimethylaminomorpholinocarbenium hexafluorophosphate (COMU), and diphenylphosphoryl azide. Preferred condensing agent is 1-ethyl-3-(3-dimethylaminopropyl)carbodiimide hydrochloride (WSC·HCl).

Examples of the base include triethylamine and N,N-diisopropylethylamine. Preferred base is triethylamine.

Examples of the solvent include tetrahydrofuran, N,N-dimethylformamide, N,N-dimethylacetamide, dimethyl sulfoxide, acetonitrile, and mixed solvents thereof. Preferred solvent is N,N-dimethylformamide.

The reaction temperature is, for example, 20°C to 30°C.

Compound [A1-5] or a salt thereof may be a commercially available product, or produced from a commercially available product by a known method. For example, it can be obtained by the below-mentioned production methods B1 to B4.

Compound [II] or a salt thereof may also be produced by performing this production method by using, instead of compound [A1-5] or a salt thereof, a compound having a functional group or a protected functional group or a salt thereof that can be converted to compound [A1-5] or a salt thereof by a known reaction to obtain a compound corresponding to compound [II] or a salt thereof, and converting the functional group thereof.

Production method A1a: Production method of compound [I] or a salt thereof

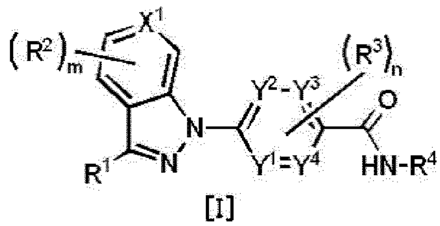

[I]

wherein each symbol is as defined above.

Compound [I] or a salt thereof can be produced by performing production method A1 by using compound [A1a-1] or a salt thereof instead of compound [A1-1] or a salt thereof:

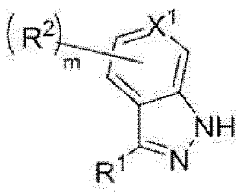

[A1a-1]

wherein each symbol is as defined above.

Compound [A1a-1] or a salt thereof may be a commercially available product, or produced from a commercially available product by a known method.

Production method A2: Another method for producing compound [II] or a salt thereof

Compound [II] or a salt thereof can also be produced by, for example, the following production method A2.

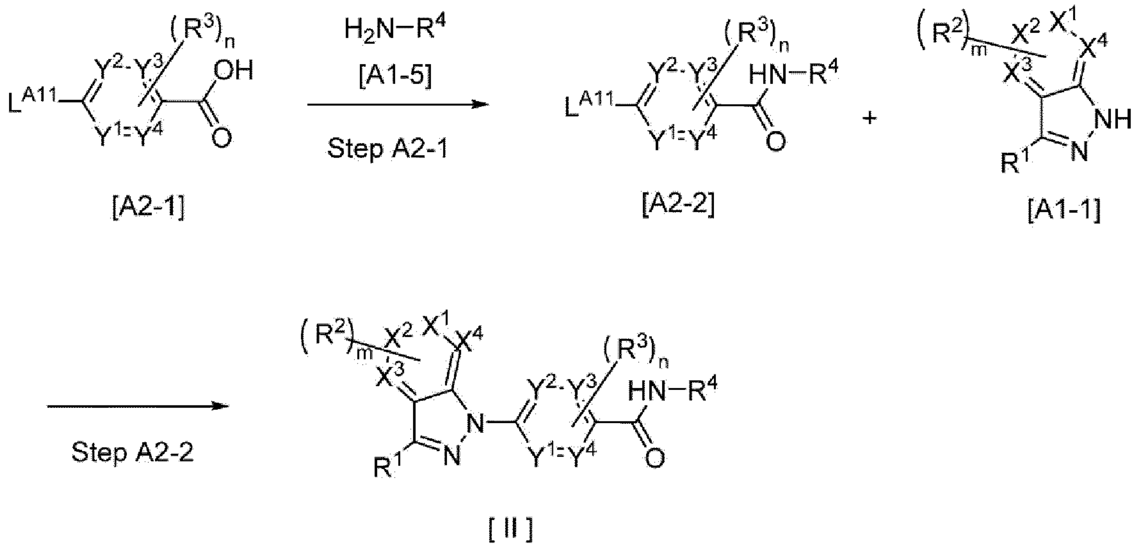


[A2-1] [A2-2] [A1-1]

[II]

wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2), and

$L^{A11}$, $R^1$, $R^2$, $R^3$, $R^4$, m and n are as defined above.

(Step A2-1)

Compound [A2-2] or a salt thereof can be produced by reacting compound [A2-1] or a salt thereof with compound [A1-5] or a salt thereof, according to step A1-3.

Compound [A2-1] or a salt thereof may be a commercially available product, or produced from a commercially available product by a known method.

(Step A2-2)

Compound [II] or a salt thereof can be produced by reacting compound [A2-2] or a salt thereof with compound [A1-1] or a salt thereof according to step A1-1.

Compound [A2-1] or a salt thereof may be a commercially available product, or produced from a commercially available product by a known method.

Compound [II] or a salt thereof may also be produced by performing this production method by using, instead of compound [A1-5] or a salt thereof, a compound having a functional group or a protected functional group or a salt thereof that can be converted to compound [A1-5] or a salt thereof by a known reaction to obtain a compound corresponding to compound [II] or a salt thereof, and converting the functional group thereof.

Production method A2a: Another method for producing compound [I] or a salt thereof

Compound [I] or a salt thereof can be produced by performing production method A2 by using compound [A1a-1] or a salt thereof instead of Compound [A1-1] or a salt thereof:

Production method A3: Production method of compound [III] or a salt thereof

Compound [III] or a salt thereof can be produced, for example, by the following production method A3.

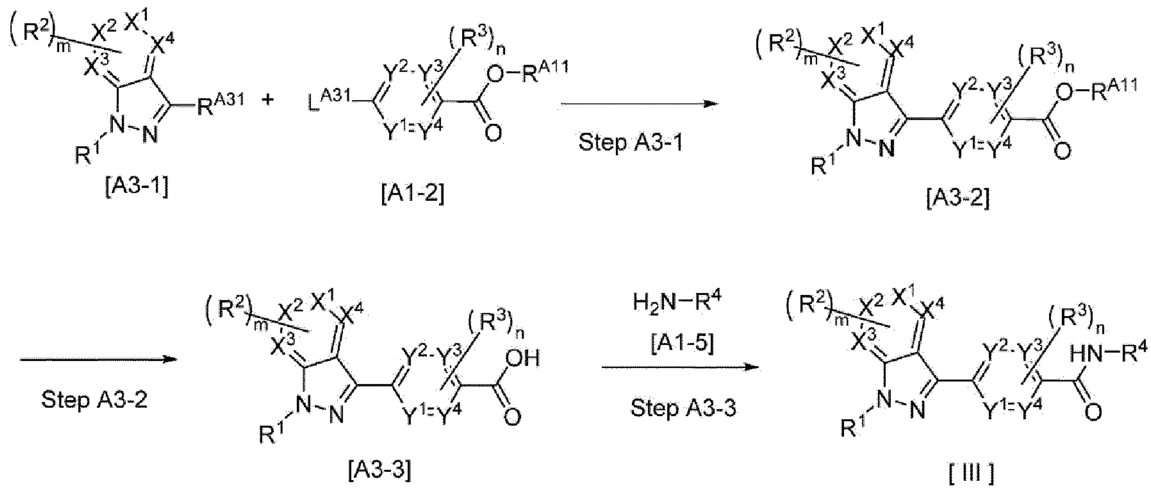


wherein

$X^1$, $X^2$, $X^3$, $X^4$, $Y^1$, $Y^2$, $Y^3$ and $Y^4$ are each independently a carbon or a nitrogen atom (wherein the total number of the nitrogen atoms for $X^1$, $X^2$, $X^3$ and $X^4$ is 0 or 1, and the total number of the nitrogen atoms for $Y^1$, $Y^2$, $Y^3$ and $Y^4$ is 0, 1 or 2),
$L^{A31}$ is a leaving group {for example, halogen, boronic acid, and boronic acid ester (e.g., boronic acid pinacol ester)},
$R^{A31}$ is halogen, boronic acid, or boronic acid ester (e.g., boronic acid pinacol ester)} (wherein when $L^{A31}$ is halogen, $R^{A31}$ is boronic acid or boronic acid ester, and when $L^{A31}$ is boronic acid or boronic acid ester, $R^{A31}$ is halogen), and
$R^1$, $R^2$, $R^3$, $R^4$, $R^{A11}$, m and n are as defined above.

(Step A3-1)

Compound [A3-2] or a salt thereof can be obtained by reacting compound [A3-1] or a salt thereof with compound [A1-2] or a salt thereof in a solvent in the presence of a base and a catalyst.
Examples of the solvent include toluene, 1,4-dioxane, tetrahydrofuran, 1,2-dimethoxyethane, cyclopentyl methyl ether, N,N-dimethylformamide, N,N-dimethylacetamide, acetonitrile, and mixed solvents thereof with water. Preferred solvent is a mixed solvent of 1,2-dimethoxyethane and water.
Examples of the base include potassium phosphate, sodium carbonate, sodium hydrogen carbonate, potassium carbonate, and tetrabutylammonium acetate. Preferred base is potassium phosphate.
Examples of the catalyst include palladium acetate, tetrakis triphenylphosphine palladium, bis(triphenylphosphine) palladium dichloride, (bis (diphenylphosphino) ferrocene) palladium dichloride-dichloromethane complex, and (2-dicyclohexylphosphino-2',4',6'-triisopropyl-1,1'-biphenyl) [2-(2'-amino-1,1'-biphenyl)]palladium(II) methanesulfonate. Preferred catalyst is (bis(diphenylphosphino)ferrocene)palladium dichloride-dichloromethane complex.
The reaction temperature is, for example, 70°C to 100°C.
Compound [A3-1] or a salt thereof may be a commercially available product, or produced from a commercially available product by a known method.

(Step A3-2)

Compound [A3-3] or a salt thereof can be produced by reacting compound [A3-2] or a salt thereof according to step A1-2.

(Step A3-3)

Compound [III] or a salt thereof can be produced by reacting compound [A3-3] or a salt thereof with compound [A1-5] or a salt thereof, according to step A1-3.
Compound [III] or a salt thereof may also be produced by performing this production method by using, instead of compound [A1-5] or a salt thereof, a compound having a functional group or a protected functional group or a salt thereof that can be converted to compound [A1-5] or a salt thereof by a known reaction to obtain a compound corresponding to compound [III] or a salt thereof, and converting the functional group thereof.

Production method A4: Production method of compound [IV] or a salt thereof

Compound [IV] or a salt thereof can be produced, for example, by the following production method A4.

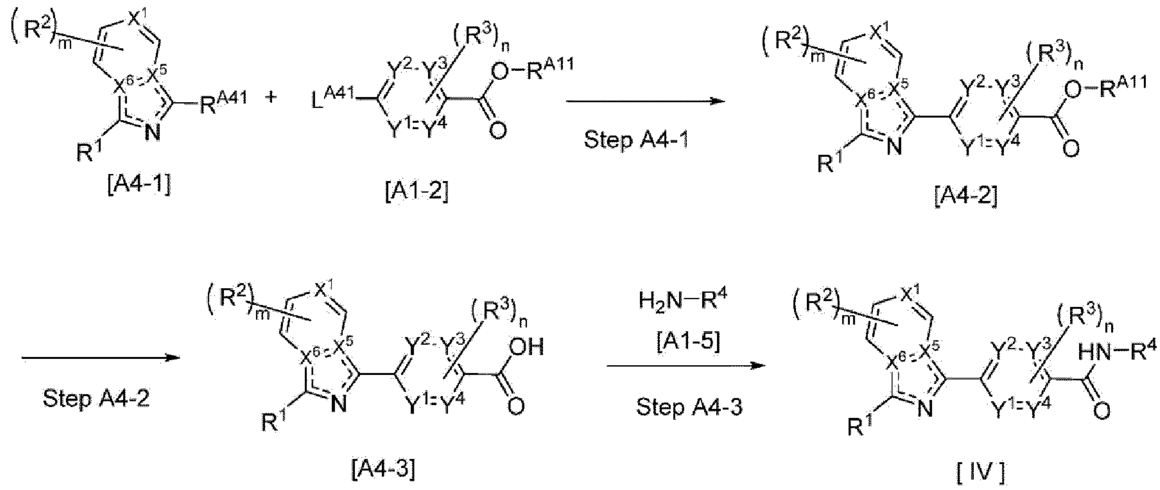


wherein

$X^1$, $X^5$, $X^6$, $Y^1$, $Y^2$, $Y^3$ and $Y^4$ are each independently a carbon or a nitrogen atom (wherein the total number of the nitrogen atoms for $X^5$ and $X^6$ is 1, and the total number of the nitrogen atoms for $Y^1$, $Y^2$, $Y^3$ and $Y^4$ is 0, 1 or 2),
$L^{A41}$ is a leaving group {for example, halogen, boronic acid, and boronic acid ester (e.g., boronic acid pinacol ester)},
$R^{A41}$ is hydrogen, halogen, boronic acid, or boronic acid ester (e.g., boronic acid pinacol ester)}(wherein when $L^{A41}$ is halogen, $R^{A41}$ is hydrogen, boronic acid or boronic acid ester, and when $L^{A41}$ is boronic acid or boronic acid ester, $R^{A41}$ is hydrogen or halogen), and
$R^1$, $R^2$, $R^3$, $R^4$, $R^{A11}$, m and n are as defined above.

(Step A4-1)

Compound [A4-2] or a salt thereof can be produced by reacting compound [A4-1] or a salt thereof with compound [A1-2] or a salt thereof, according to step A3-1.
Compound [A4-1] or a salt thereof may be a commercially available product, or produced from a commercially available product by a known method.

(Step A4-2)

Compound [A4-3] or a salt thereof can be produced by reacting compound [A4-2] or a salt thereof according to step A1-2.

(Step A4-3)

Compound [IV] or a salt thereof can be produced by reacting compound [A4-3] or a salt thereof with compound [A1-5] or a salt thereof, according to step A1-3.
Compound [IV] or a salt thereof may also be produced by performing this production method by using, instead of compound [A1-5] or a salt thereof, a compound having a functional group or a protected functional group or a salt thereof that can be converted to compound [A1-5] or a salt thereof by a known reaction to obtain a compound corresponding to compound [IV] or a salt thereof, and converting the functional group thereof.

Production method B: Production method of compound [A1-5] or a salt thereof

$H_2N-R^4$

[A1-5]

wherein $R^4$ is as defined above.

In one embodiment, [A1-5] or a salt thereof can be obtained by the following production methods B1 to B4. In any production method, the starting material may be a commercially available product, or produced from a commercially available product by a known method.

Production method B1: Production method of compound [A1-5-B1] or a salt thereof

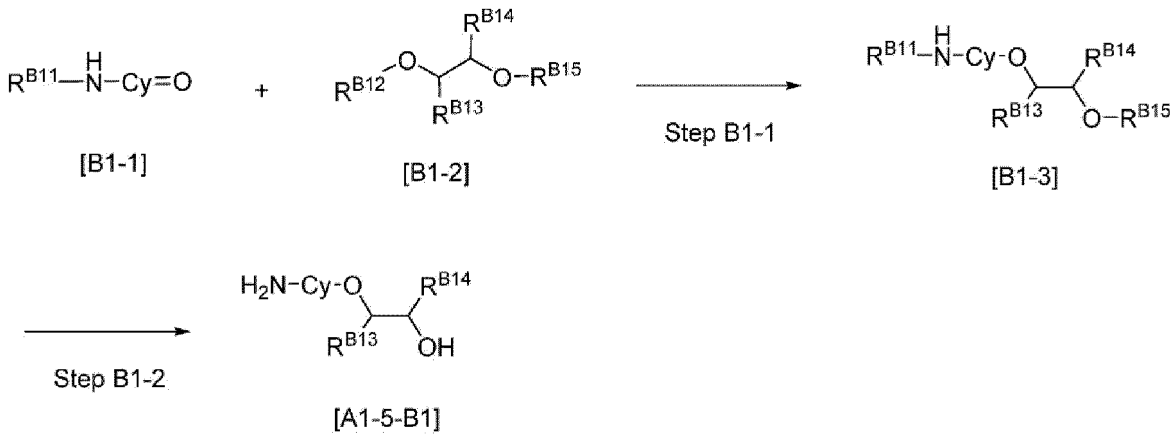


[B1-1] [B1-2] [B1-3] [A1-5-B1]

wherein

- $R^{B11}$ is an amino-protecting group (e.g., benzyloxycarbonyl),
- $R^{B12}$ is a hydroxy-protecting group (e.g., trimethylsilyl),
- $R^{B13}$ and $R^{B14}$ are each independently hydrogen or $C_{1-4}$ alkyl,
- $R^{B15}$ is a hydroxy-protecting group (e.g., benzyl),
- Cy=O is a group in which two hydrogens on the same carbon atom constituting ring Cy are substituted by oxo, and ring Cy is as defined above.

(Step B1-1)

Compound [B1-3] or a salt thereof can be produced by reacting compound [B1-1] or a salt thereof with compound [B1-2] or a salt thereof in a solvent in the presence of an acid and a reducing agent.

Examples of the acid include trimethylsilyl trifluoromethanesulfonate.

Examples of the reducing agent include triethylsilane and trimethylsilane. Preferred reducing agent is triethylsilane.

Examples of the solvent include acetonitrile and dichloromethane. Preferred solvent is acetonitrile.

The reaction temperature is, for example, 0°C to 10°C.

Compound [B1-1] or a salt thereof may be a commercially available product, or produced from a commercially available product by a known method.

Compound [B1-2] or a salt thereof, commercially available product, or produced from a commercially available product by a known method.

(Step B1-2)

Compound [A1-5-B1] or a salt thereof can be produced by removing $R^{B11}$ and $R^{B15}$ of compound [B1-3] or a salt thereof by a deprotection reaction. The deprotection reaction can be carried out under conditions suitable for the kind of each of $R^{B11}$ and $R^{B15}$. For example, when $R^{B11}$ is benzyloxycarbonyl and $R^{B15}$ is benzyl, compound [A1-5-B1] or a salt thereof can be obtained by subjecting compound [B1-3] or a salt thereof to a catalytic hydrogenation reaction. For example, compound [A1-5-B1] or a salt thereof can be obtained by reacting compound [B1-2] or a salt thereof in a solvent under a hydrogen atmosphere in the presence of a catalyst.

Examples of the solvent include methanol, ethanol, tetrahydrofuran, ethyl acetate, acetic acid, and mixed

solvents thereof. Preferred solvent is methanol.

The pressure of hydrogen gas is, for example, 1 to 10 atm, preferably 1 to 4 atm.

Examples of the catalyst include palladium-carbon and palladium hydroxide-carbon. Preferred catalyst is palladium hydroxide-carbon.

The reaction temperature is, for example, 20°C to 30°C.

Production method B2: Production method of compound [A1-5-B2] or a salt thereof

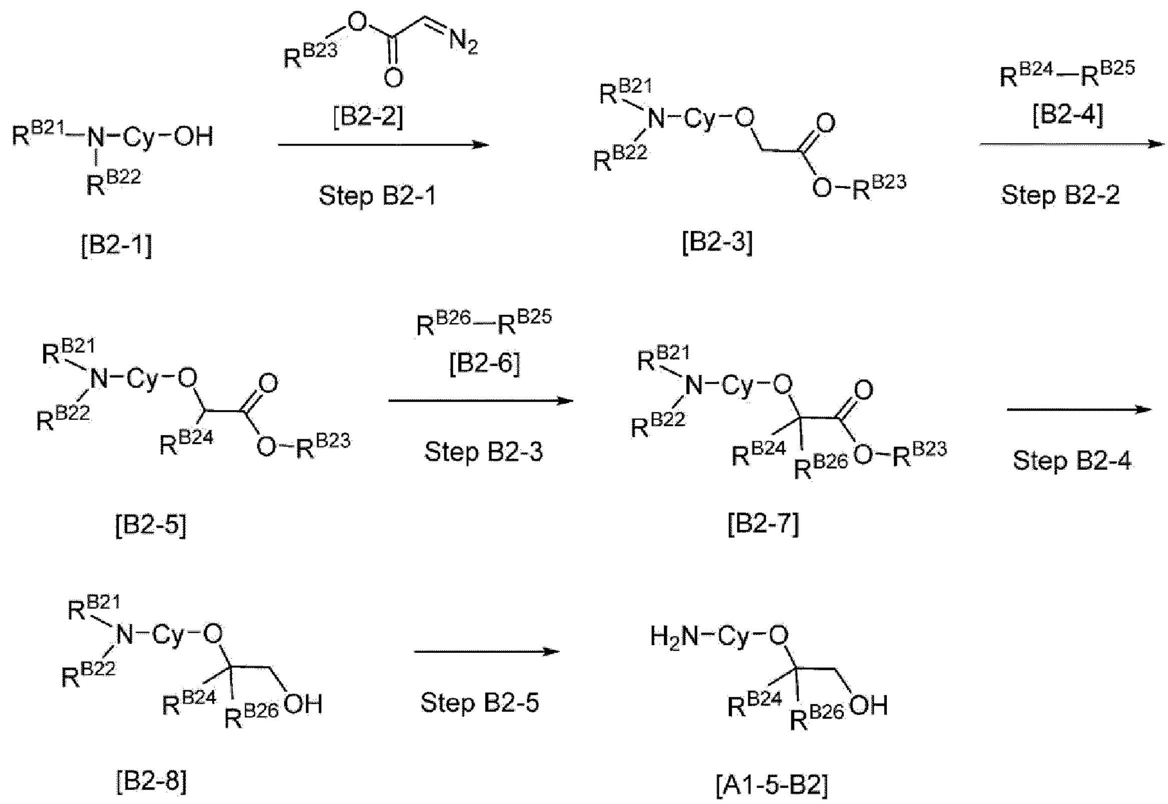


[B2-1] [B2-3] [B2-5] [B2-7] [B2-8] [A1-5-B2]

wherein

- $R^{B21}$ and $R^{B22}$ are each independently an amino-protecting group (e.g., benzyl),
- $R^{B23}$, $R^{B24}$ and $R^{B26}$ are each independently $C_{1-4}$ alkyl,
- $R^{B25}$ are each independently halogen, and
- ring Cy is as defined above.

(Step B2-1)

Compound [B2-3] or a salt thereof can be obtained by reacting compound [B2-1] or a salt thereof with compound [B2-2] in a solvent in the presence of a metal catalyst.

Examples of the solvent include dichloromethane.

Examples of the metal catalyst include rhodium (II) acetate and rhodium (II) octanoate. Preferred metal catalyst is rhodium (II) acetate.

The reaction temperature is, for example, 20°C to 40°C.

Compound [B2-1] or a salt thereof may be a commercially available product, or produced from a commercially available product by a known method.

Compound [B2-2] may be a commercially available product, or produced from a commercially available product by a known method.

(Step B2-2)

Compound [B2-5] or a salt thereof can be obtained by reacting compound [B2-3] or a salt thereof with compound [B2-4] in a solvent in the presence of a base.
Examples of the solvent include diethyl ether, tetrahydrofuran, dimethoxyethane, benzene, toluene, hexamethylphosphoric triamide, and mixed solvents thereof. Preferred solvent is tetrahydrofuran.
Examples of the base include potassium hexamethyl disilazide, sodium hexamethyl disilazide, lithium hexamethyl disilazide, and lithium diisopropylamide.
The reaction temperature is, for example, -78°C to 30°C.
Compound [B2-4] may be a commercially available product, or produced from a commercially available product by a known method.

(Step B2-3)

Compound [B2-7] or a salt thereof can be obtained by reacting compound [B2-5] or a salt thereof with compound [B2-6], according to step B2-2.
Compound [B2-6] may be a commercially available product, or produced from a commercially available product by a known method.

(Step B2-4)

Compound [B2-8] or a salt thereof can be produced by reducing compound [B2-7] or a salt thereof in a solvent.
Examples of the reducing agent include lithium aluminum hydride and sodium borohydride. Preferred reducing agent is lithium aluminum hydride.
Examples of the solvent include tetrahydrofuran.
The reaction temperature is, for example, 0°C to 20°C, preferably 0°C to 10°C.

(Step B2-5)

Compound [A1-5-B2] or a salt thereof can be produced by removing $R^{B21}$ and $R^{B22}$ of compound [B2-8] or a salt thereof by a deprotection reaction. The deprotection reaction can be carried out under conditions suitable for the kind of each of $R^{B21}$ and $R^{B22}$. For example, when $R^{B21}$ and $R^{B22}$ are each benzyl, compound [A1-5-B2] or a salt thereof can be obtained by reacting compound [B2-8] or a salt thereof in a solvent under a hydrogen atmosphere in the presence of a catalyst.
Examples of the solvent include methanol, ethanol, tetrahydrofuran, ethyl acetate, acetic acid, and mixed solvents thereof. Preferred solvent is a mixed solvent of methanol and tetrahydrofuran.
The pressure of hydrogen gas is, for example, 1 to 10 atm, preferably 1 to 4 atm.
Examples of the catalyst include palladium-carbon and palladium hydroxide-carbon. Preferred catalyst is palladium hydroxide-carbon.
The reaction temperature is, for example, 20°C to 30°C.

<u>Production method B3: Production method of compound [A1-5-B3]</u> _ <u>or a salt thereof</u>

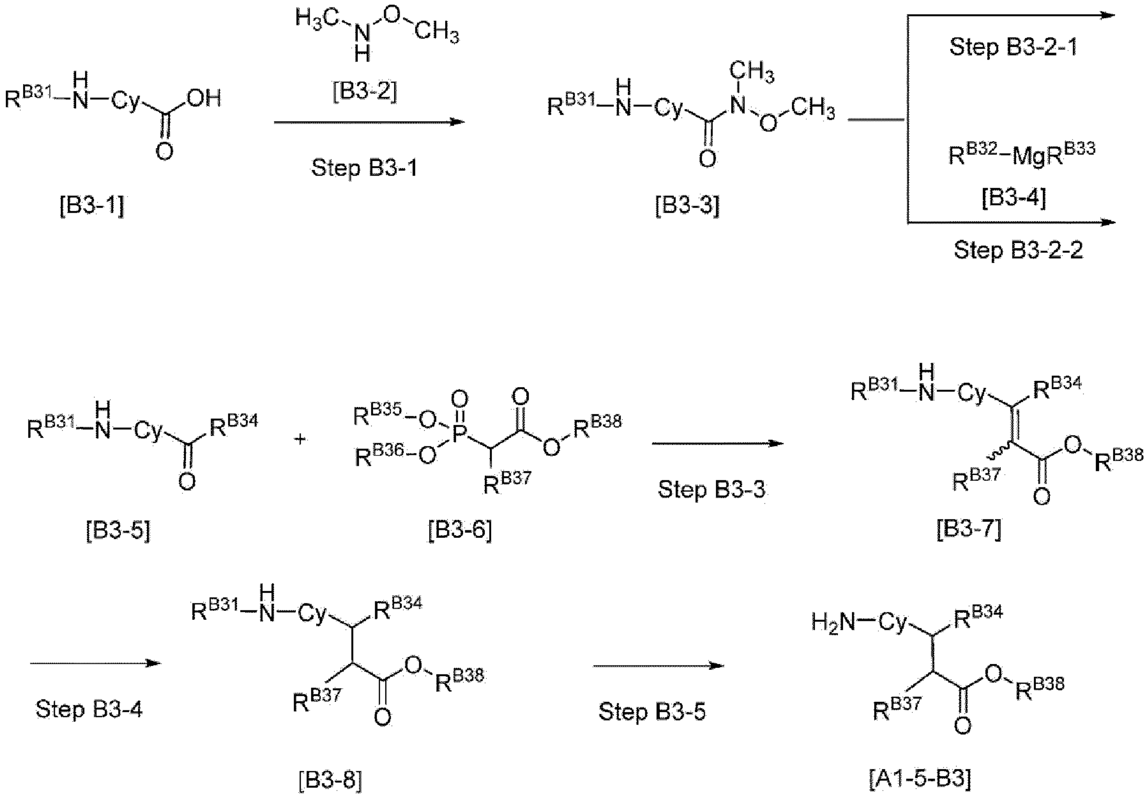


wherein

$R^{B31}$ is an amino-protecting group (e.g., tert-butoxycarbonyl or benzyloxycarbonyl, preferably tert-butoxycarbonyl),
$R^{B32}$ and $R^{B38}$ are each independently $C_{1-4}$ alkyl,
$R^{B33}$ is halogen,
$R^{B34}$, $R^{B35}$, $R^{B36}$ and $R^{B37}$ are each independently hydrogen or $C_{1-4}$ alkyl, and
ring Cy is as defined above.

(Step B3-1)

Compound [B3-3] or a salt thereof can be obtained by reacting compound [B3-1] or a salt thereof with compound [B3-2] or a salt thereof in a solvent in the presence of a condensing agent and a base.
Examples of the solvent include 1,4-dioxane, tetrahydrofuran, diethyl ether, 1,2-dimethoxyethane, cyclopentyl methyl ether, toluene, hexane, xylene, dichloromethane, chloroform, N,N-dimethylformamide, N,N-dimethylacetamide, dimethyl sulfoxide, acetonitrile, pyridine, and mixed solvents thereof. Preferred solvent is N,N-dimethylformamide.
Examples of the condensing agent include dicyclohexylcarbodiimide (DCC), 1-ethyl-3-(3-dimethylaminopropyl) carbodiimide hydrochloride (WSC·HCl), diisopropyl carbodiimide, 1,1'-carbonyldiimidazole (CDI), O-(7-azabenzotriazol-1-yl)-N,N,N',N'-tetramethyluronium hexafluorophosphate (HATU), hexafluorophosphoric acid (benzotriazol-1-yloxy) tripyrrolidinophosphonium (PyBOP), (1-cyano-2-ethoxy-2-oxoethylideneaminooxy)dimethylaminomorpholinocarbenium hexafluorophosphate (COMU), and diphenylphosphoryl azide. Preferred condensing agent is 1-ethyl-3-(3-dimethylaminopropyl)carbodiimide hydrochloride (WSC·HCl).
Examples of the base include pyridine, triethylamine, and N,N-diisopropylethylamine. Preferred base is N,N-diisopropylethylamine.
The reaction temperature is, for example, 0°C to 60°C, preferably 0°C to 40°C.
Compound [B3-1] or a salt thereof may be a commercially available product, or produced from a commercially available product by a known method.
Compound [B3-2] or a salt thereof may be a commercially available product, or produced from a commercially available product by a known method.

(Step B3-2-1)

When $R^{B34}$ is hydrogen, compound [B3-5] or a salt thereof can be obtained by reducing compound [B3-3] or a salt thereof in a solvent.

Examples of the solvent include 1,4-dioxane, tetrahydrofuran, diethyl ether, 1,2-dimethoxyethane, cyclopentyl methyl ether, and mixed solvents thereof. Preferred solvent is tetrahydrofuran.

Examples of the reducing agent include diisobutylaluminum hydride and lithium aluminum hydride. Preferred reducing agent is lithium aluminum hydride.

The reaction temperature is, for example, -78°C to 40°C, preferably -78°C to 0°C.

(Step B3-2-2)

When $R^{B34}$ is $C_{1-4}$ alkyl, compound [B3-5] or a salt thereof can be obtained by reacting compound [B3-3] or a salt thereof with compound [B3-4] in a solvent.

Examples of the solvent include 1,4-dioxane, tetrahydrofuran, diethyl ether, 1,2-dimethoxyethane, cyclopentyl methyl ether, and mixed solvents thereof. Preferred solvent is tetrahydrofuran.

The reaction temperature is, for example, -78°C to 40°C, preferably 0°C to 20°C.

Compound [B3-4] may be a commercially available product, or produced from a commercially available product by a known method.

(Step B3-3)

Compound [B3-7] or a salt thereof can be obtained by reacting compound [B3-5] or a salt thereof with compound [B3-6] or a salt thereof in a solvent in the presence of a base.

Examples of the solvent include tetrahydrofuran, dimethoxyethane, benzene, toluene, methanol, ethanol, and mixed solvents thereof. Preferred solvent is tetrahydrofuran.

Examples of the base include sodium hydride, sodium methoxide, sodium ethoxide, and n-butyllithium. Preferred base is sodium hydride.

The reaction temperature is, for example, -78°C to 100°C, preferably 0°C to 70°C.

Compound [B3-6] or a salt thereof may be a commercially available product, or produced from a commercially available product by a known method.

(Step B3-4)

Compound [B3-8] or a salt thereof can be obtained by subjecting compound [B3-7] or a salt thereof to a catalytic hydrogenation reaction. For example, compound [B3-8] or a salt thereof can be obtained by reacting compound [B3-7] or a salt thereof in a solvent under a hydrogen atmosphere in the presence of a catalyst.

Examples of the solvent include methanol, ethanol, tetrahydrofuran, ethyl acetate, acetic acid, and mixed solvents thereof. Preferred solvent is a mixed solvent of methanol and tetrahydrofuran.

The pressure of hydrogen gas is, for example, 1 to 10 atm, preferably 1 to 4 atm.

Examples of the catalyst include palladium-carbon and palladium hydroxide-carbon. Preferred palladium catalyst is palladium-carbon.

The reaction temperature is, for example, 0°C to 70°C, preferably 0°C to 40°C.

(Step B3-5)

Compound [A1-5-B3] or a salt thereof can be produced by removing $R^{B31}$ of compound [B3-8] or a salt thereof by a deprotection reaction. The deprotection reaction can be carried out under conditions suitable for the kind of $R^{B31}$. For example, when $R^{B31}$ is tert-butoxycarbonyl, compound [A1-5-B3] or a salt thereof can be obtained by reacting compound [B3-8] or a salt thereof with an acid in a solvent.

Examples of the solvent include ethyl acetate, 1,4-dioxane, methanol, cyclopentyl methyl ether, and mixed solvents thereof. Preferred solvent is ethyl acetate, methanol, or a mixed solvent thereof.

Examples of the acid include hydrogen chloride, sulfuric acid, and trifluoroacetic acid. Preferred acid is hydrogen chloride.

The reaction temperature is, for example, 0°C to 50°C, preferably 0°C to 30°C.

Production method B4: Production method of compound [A1-5-B4] or a salt thereof

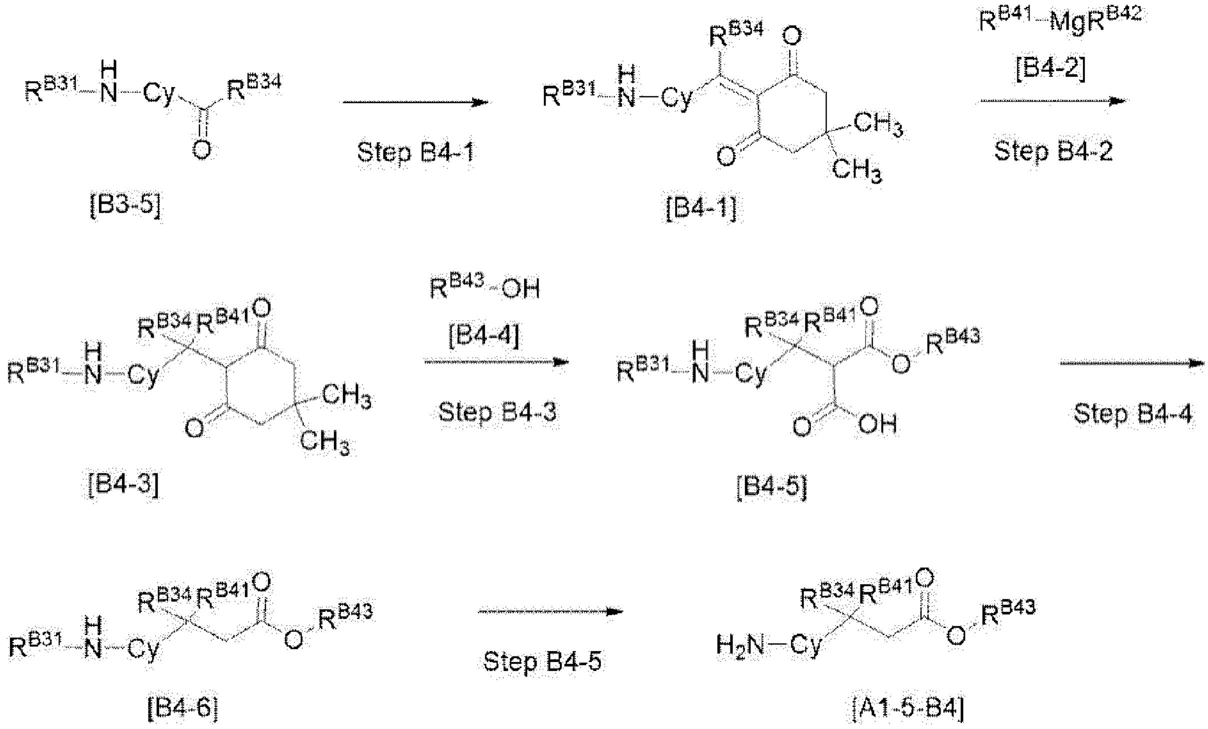


wherein

$R^{B41}$ and $R^{B43}$ are each independently $C_{1-4}$ alkyl,
$R^{B42}$ is halogen, and
$R^{B31}$, $R^{B34}$ and ring Cy are as defined above.

(Step B4-1)

Compound [B4-1] or a salt thereof can be obtained by reacting compound [B3-5] or a salt thereof with Meldrum's acid in a solvent in the presence of Lewis acid and a base.
Examples of the solvent include benzene, toluene, dichloromethane, chloroform, tetrahydrofuran, and mixed solvents thereof. Preferred solvent is tetrahydrofuran.
Examples of the Lewis acid include titanium tetrachloride.
Examples of the base include piperidine, morpholine, and pyridine. Preferred base is pyridine.
The reaction temperature is, for example, 0°C to 50°C, preferably 0°C to 30°C.
Compound [B3-5] or a salt thereof can be obtained according to production method B3, step B3-1, and step B3-2-1 or B3-2-2.

(Step B4-2)

Compound [B4-3] or a salt thereof can be obtained by reacting compound [B4-1] or a salt thereof with compound [B4-2] in a solvent. When $R^{B34}$ is $C_{1-4}$ alkyl, it is preferable to add copper(I) bromide to the reaction system.
Examples of the solvent include diethyl ether, tetrahydrofuran, dimethoxyethane, and mixed solvents thereof. Preferred solvent is tetrahydrofuran.
The reaction temperature is, for example, 0°C to 40°C.
Compound [B4-2] may be a commercially available product, or produced from a commercially available product by a known method.

(Step B4-3)

Compound [B4-5] or a salt thereof can be obtained by reacting compound [B4-3] or a salt thereof in solvent [B4-4].
Examples of the solvent [B4-4] include methanol, ethanol, and tert-butyl alcohol. Preferred solvent [B4-4] is methanol.
The reaction temperature is, for example, 20°C to 110°C, preferably 60°C to 100°C.

(Step B4-4)

Compound [B4-6] or a salt thereof can be obtained by reacting compound [B4-5] or a salt thereof in a solvent in the presence of sodium chloride.
Examples of the solvent include dimethyl sulfoxide, water, and mixed solvents thereof. Preferred solvent is a mixed solvent of dimethyl sulfoxide and water.
The reaction temperature is, for example, 100°C to 160°C, preferably 120°C to 150°C.

(Step B4-5)

Compound [A1-5-B4] or a salt thereof can be produced by removing $R^{B31}$ of compound [B4-6] or a salt thereof by a deprotection reaction according to step B3-5.

[Examples]

The method for producing the compound of the present invention or a pharmaceutically acceptable salt thereof is specifically described below. However, the production method of the compound of the present invention or a pharmaceutically acceptable salt thereof is not limited to the following.
The compound obtained in each step can be isolated and/or purified as necessary by known methods such as distillation, recrystallization, and column chromatography. In some cases, the compound can proceed to the next step without isolation and/or purification.
In the present specification, the room temperature refers to a temperature in the state where the temperature is not controlled, and one embodiment is 1°C to 40°C.
NMR was measured at 400 MHz.

[Production Example 1] <u>Example 1: Production of 2-(6-fluoro-3-methyl-1H-indazol-1-yl)-N-(trans-4-(2-hydroxypropan-2-yl)cyclohexyl)pyrimidine-5-carboxamide</u>

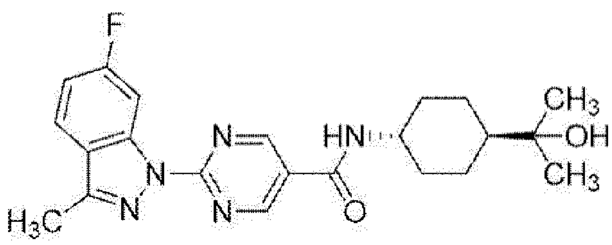

Step 1-1: Ethyl 2-(6-fluoro-3-methyl-1H-indazol-1-yl)pyrimidine-5-carboxylate

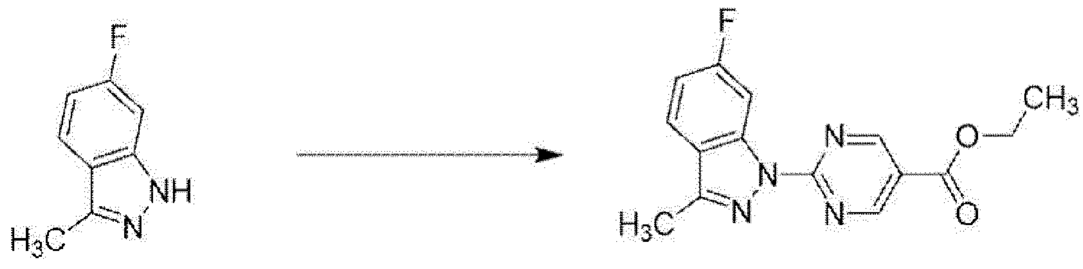

A suspension of 6-fluoro-3-methyl-1H-indazole (200 mg), ethyl 2-chloropyrimidine-carboxylate (161 mg) and cesium carbonate (698 mg) in N,N-dimethylformamide (2.0 mL) was stirred at 90°C for 4 hr. To the reaction mixture was added water at room temperature, and the precipitated solid was collected by filtration and dried under reduced pressure at 70°C to give the title compound (158 mg).
$^1$H-NMR (DMSO-$D_6$) δ: 9.28 (2H, s), 8.42 (1H, dd, J = 10.5, 2.2 Hz), 7.94 (1H, dd, J = 8.2, 5.2 Hz), 7.30 (1H, ddd, J = 10.5, 8.2, 2.2 Hz), 4.40 (2H, q, J = 7.2 Hz), 2.61 (3H, s), 1.37 (3H, t, J = 7.2 Hz).

Step 1-2: 2-(6-Fluoro-3-methyl-1H-indazol-1-yl)pyrimidine-5-carboxylic acid

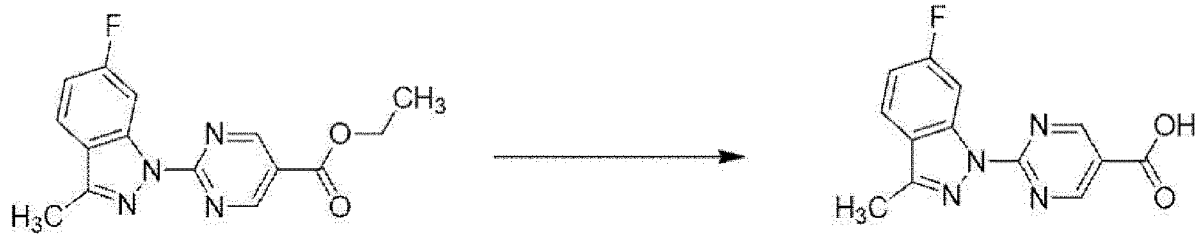

To a suspension of ethyl 2-(6-fluoro-3-methyl-1H-indazol-1-yl)pyrimidine-5-carboxylate (158 mg) in methanol/-tetrahydrofuran (2.37 mL, methanol/tetrahydrofuran=1/2) was added 4 M aqueous sodium hydroxide solution (0.263 mL) at room temperature, and the mixture was stirred for 2 hr. To the reaction mixture were added 2 M hydrochloric acid (0.526 mL) and water at room temperature, and the precipitated solid was collected by filtration and dried under reduced pressure at 70°C to give a crude product (65.6 mg) containing the title compound.

Step 1-3: 2-(6-Fluoro-3-methyl-1H-indazol-1-yl)-N-(trans-4-(2-hydroxypropan-2-yl)cyclohexyl)pyrimidine-5-carboxamide

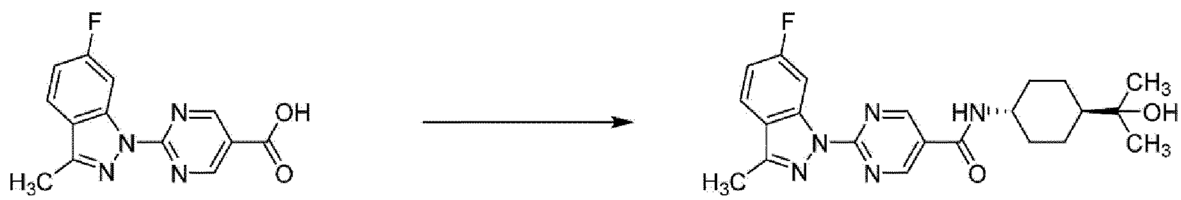

To a suspension of a crude product (30 mg) containing 2-(6-fluoro-3-methyl-1H-indazol-1-yl)pyrimidine-5-carboxylic acid, 2-(trans-4-aminocyclohexyl)propan-2-ol (22.53 mg) and triethylamine (0.023 mL) in N,N-dimethylformamide (0.6 mL) were added 1-hydroxy-7-azabenzotriazole (4.5 mg) and 1-ethyl-3-(3-dimethylaminopropyl)carbodiimide hydrochloride (31.7 mg) at room temperature, and the mixture was stirred for 30 min, left standing overnight, and further stirred for 6 hr. To the reaction mixture were added water and ethyl acetate at room temperature, and the layers were separated. The aqueous layer was extracted once with ethyl acetate. The combined organic layer was washed twice with water and concentrated under reduced pressure. To the residue was added ethyl acetate, and slurry purification was performed. The solid was collected by filtration and dried under reduced pressure at 70°C to give the title compound (8.5 mg).
$^1$H-NMR (DMSO-$D_6$)δ: 9.23 (2H, s), 8.54 (1H, d, J = 8.2 Hz), 8.40 (1H, dd, J = 10.2, 2.0 Hz), 7.94 (1H, dd, J = 8.6, 5.6 Hz), 7.29 (1H, ddd, J = 10.2, 8.6, 2.0 Hz), 4.05 (1H, br s), 3.81-3.68 (1H, m), 2.61 (3H, s), 1.96 (2H, d, J = 10.5 Hz), 1.86 (2H, d, J =

10.5 Hz), 1.40-1.26 (2H, m), 1.25-1.00 (3H, m), 1.06 (6H, s). MS(M+H) :412 MS(M-H) :410

Step 1-4: 2-(6-Fluoro-3-methyl-1H-indazol-1-yl)pyrimidine-5-carboxylic acid

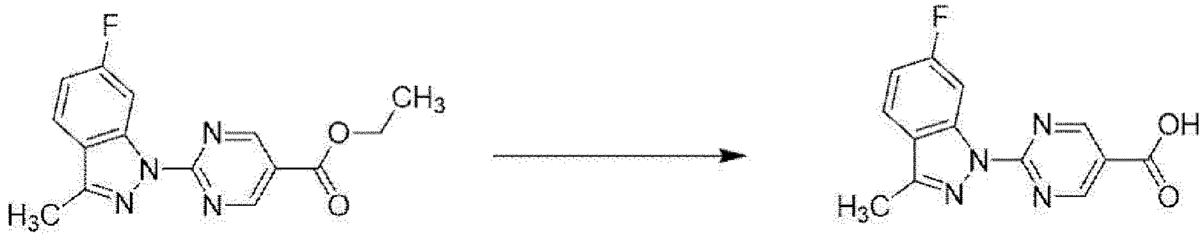

To a suspension of ethyl 2-(6-fluoro-3-methyl-1H-indazol-1-yl)pyrimidine-5-carboxylate (177.68 g) synthesized in the same manner as in step 1-1 in tetrahydrofuran/water (2.132 L, tetrahydrofuran/water=2/1) was added dropwise 4 M aqueous lithium hydroxide solution (192 mL) at room temperature, and the mixture was stirred under a nitrogen stream for 2 hr. To the reaction mixture were successively added dropwise 2 M hydrochloric acid (385 mL) and water (1060 mL) at room temperature, and the mixture was stirred for 6 hr and left standing overnight. The precipitated solid was collected by filtration, washed with water (1440 mL), air dried for 3 days, and dried under reduced pressure at 60°C to give the title compound (166.22 g).

Step 1-5: 2-(6-Fluoro-3-methyl-1H-indazol-1-yl)-N-(trans-4-(2-hydroxypropan-2-yl)cyclohexyl)pyrimidine-5-carboxamide monohydrate

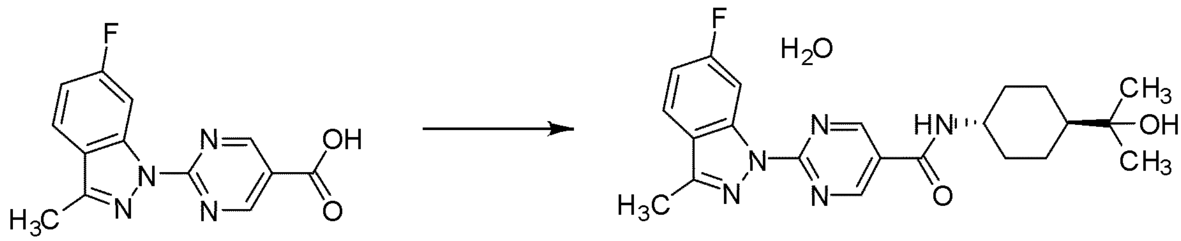

To a suspension of 2-(6-fluoro-3-methyl-1H-indazol-1-yl)pyrimidine-5-carboxylic acid (150 g), 2-(trans-4-aminocyclohexyl)propan-2-ol (104 g) and 1-hydroxy-7-azabenzotriazole (75 g) in N,N-dimethylformamide (1.95 L) were added triethylamine (115 mL), 1-ethyl-3-(3-dimethylaminopropyl)carbodiimide hydrochloride (158 g) and N,N-dimethylformamide (75 mL) at room temperature, and the mixture was stirred under a nitrogen stream for 5 hr. To the reaction mixture were added 1-ethyl-3-(3-dimethylaminopropyl)carbodiimide hydrochloride (52.8 g) and N,N-dimethylformamide (75 mL) at room temperature, and the mixture was stirred under a nitrogen stream overnight. To the reaction mixture was added dropwise 5.0 wt% aqueous sodium hydrogen carbonate solution (4.2 L) at room temperature, and the mixture was stirred for 2 hr. The solid was collected by filtration, washed with water (1.5 L), air dried for 2 days, and dried under reduced pressure at 60°C to give the title compound as crystals (α crystal, 219.92 g).

Step 1-6: 2-(6-Fluoro-3-methyl-1H-indazol-1-yl)-N-(trans-4-(2-hydroxypropan-2-yl)cyclohexyl)pyrimidine-5-carboxamide

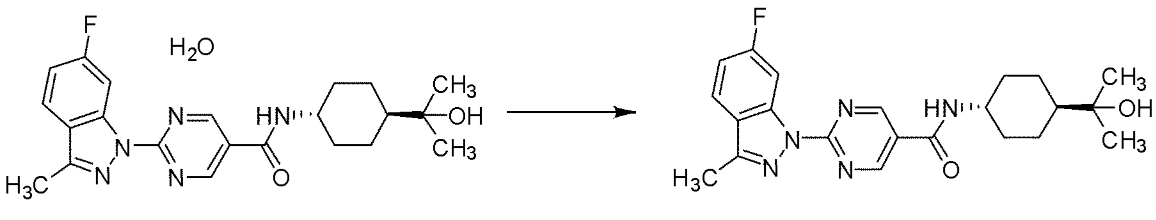

A suspension of 2-(6-fluoro-3-methyl-1H-indazol-1-yl)-N-(trans-4-(2-hydroxypropan-2-yl)cyclohexyl)pyrimidine-5-carboxamide monohydrate crystal (α crystal, 37.4 g) in acetonitrile (748 mL) was stirred for 2 days under an argon atmosphere at room temperature. The precipitated solid was collected by pressure filtration with nitrogen and dried under reduced pressure at 60°C to give the title compound as crystals (β crystal, 34.42 g).

Step 1-7: 2-(6-Fluoro-3-methyl-1H-indazol-1-yl)-N-(trans-4-(2-hydroxypropan-2-yl)cyclohexyl)pyrimidine-5-carboxamide monohydrate

A suspension of 2-(6-fluoro-3-methyl-1H-indazol-1-yl)-N-(trans-4-(2-hydroxypropan-2-yl)cyclohexyl)pyrimidine-5-carboxamide monohydrate crystal (α crystal, 500 mg) in tetrahydrofuran/water (6.0 mL, tetrahydrofuran/-water=5/1) was stirred at 75°C until it became a solution. To the reaction mixture was added dropwise water (4.0 mL) at the same temperature, and the mixture was stirred for 30 min, cooled to room temperature, and stirred for 1 hr. To the reaction mixture was added dropwise water (5.0 mL) at room temperature, and the mixture was stirred for 3 hr. The precipitated solid was collected by filtration and dried under reduced pressure to give the title compound as crystals (γ crystal, 466.6 mg).

[Production Example 2] Example 2: Production of N-(trans-4-(2-hydroxy-2-methylpropyl)cyclohexyl)-2-(3-methyl-1H-pyrazolo[3,4-c]pyridin-1-yl)pyrimidine-5-carboxamide

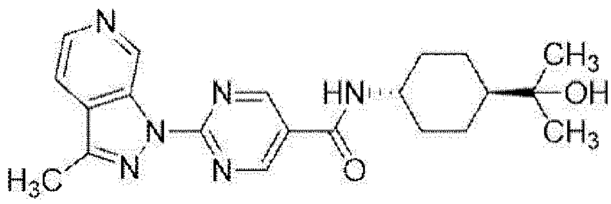

Step 2-1: tert-Butyl 2-chloropyrimidine-5-carboxylate

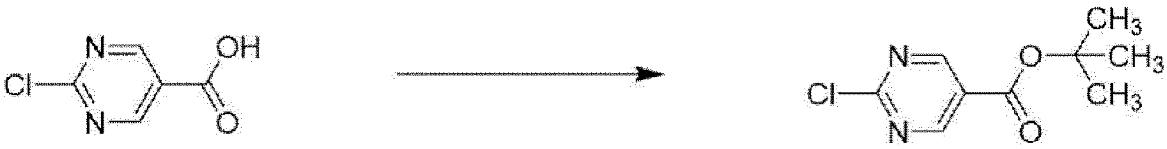

To a solution of 2-chloropyrimidine-5-carboxylic acid (2.0 g) and N,N-dimethyl-4-aminopyridine (0.462 g) in tert-butyl alcohol/chloroform (30 mL, tert-butyl alcohol/chloroform=2/1) was added di-tert-butyl bicarbonate (5.51 g) at room temperature, and the mixture was stirred at 60°C for 90 min and left standing overnight. To the reaction mixture was added ethyl acetate at room temperature, and the organic layer was washed successively with saturated aqueous sodium hydrogen carbonate, aqueous citric acid solution and saturated brine. The organic layer was dried over sodium sulfate, sodium sulfate was removed by filtration, and the filtrate was concentrated under reduced pressure. The residue was purified by silica gel column chromatography (eluent: n-hexane/ethyl acetate) to give the title compound (990 mg). $^1$H-NMR (DMSO-D$_6$) δ: 9.13 (2H, s), 1.57 (9H, s).

Step 2-2: tert-Butyl 2-(3-methyl-1H-pyrazolo[3,4-c]pyridin-1-yl)pyrimidine-5-carboxylate

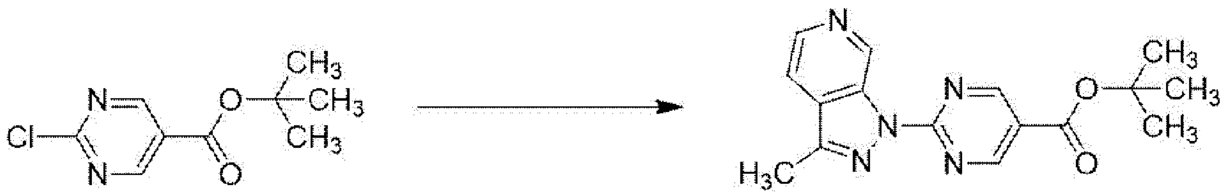

A suspension of tert-butyl 2-chloropyrimidine-5-carboxylate (100 mg), 3-methyl-1H-pyrazolo[3,4-c]pyridine (62 mg) and cesium carbonate (182 mg) in N,N-dimethylformamide (1.0 mL) was stirred at room temperature for 30 min and left standing overnight. To the reaction mixture was added water at room temperature, and the precipitated solid was collected by filtration and dried under reduced pressure at 70°C to give the title compound (130 mg).
$^1$H-NMR (DMSO-$D_6$) δ: 10.01 (1H, s), 9.29 (2H, s), 8.55 (1H, d, J = 5.2 Hz), 7.95 (1H, d, J = 5.2 Hz), 2.67 (3H, s), 1.61 (9H, s).

Step 2-3: 2-(3-Methyl-1H-pyrazolo[3,4-c]pyridin-1-yl)pyrimidine-5-carboxylic acid monohydrochloride

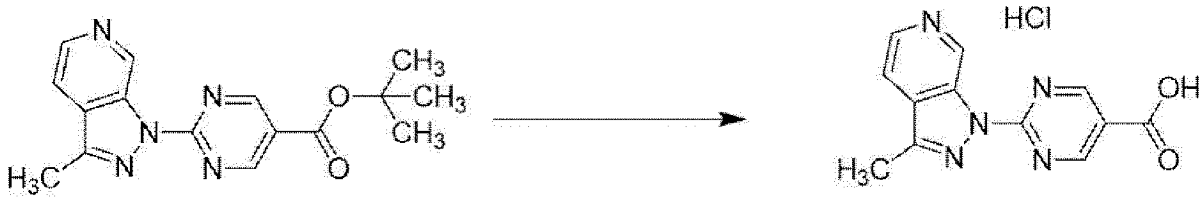

To a suspension of tert-butyl 2-(3-methyl-1H-pyrazolo[3,4-c]pyridin-1-yl)pyrimidine-5-carboxylate (130 mg) in ethyl acetate (0.26 mL) was added 4 M hydrogen chloride/ethyl acetate solution (1.3 mL) at room temperature, and the mixture was stirred for 6 hr and left standing overnight. The reaction mixture was concentrated under reduced pressure. To the residue was added trifluoroacetic acid (0.65 mL) at room temperature, and the mixture was stirred for 5 hr and left standing overnight. To the reaction mixture was added 4 M hydrogen chloride/ethyl acetate solution at room temperature, and the precipitated solid was collected by filtration, and dried under reduced pressure at 70°C to give the title compound (97.8 mg).
$^1$H-NMR (DMSO-$D_6$) δ: 13.73 (1H, br s), 10.06 (1H, s), 9.34 (2H, s), 8.62 (1H, d, J = 5.5 Hz), 8.15-8.08 (1H, m), 2.70 (3H, s).

Step 2-4: N-(Trans-4-(2-hydroxy-2-methylpropyl)cyclohexyl)-2-(3-methyl-1H-pyrazolo[3,4-c]pyridin-1-yl)pyrimidine-5-carboxamide

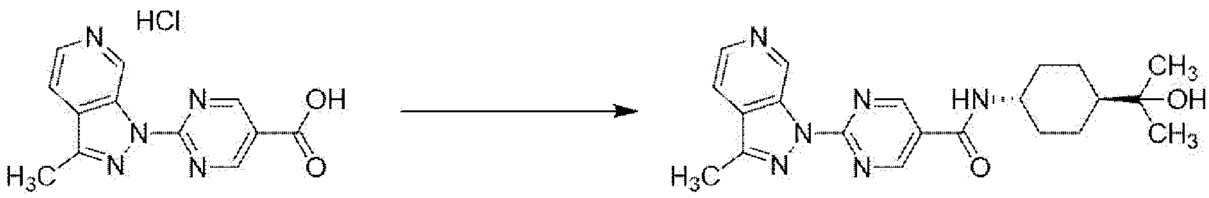

To a suspension of 2-(3-methyl-1H-pyrazolo[3,4-c]pyridin-1-yl)pyrimidine-5-carboxylic acid monohydrochloride (30 mg), 2-(trans-4-aminocyclohexyl)propan-2-ol (19.41 mg), 1-hydroxy-7-azabenzotriazole (4.2 mg) and 1-ethyl-3-(3-dimethylaminopropyl)carbodiimide hydrochloride (29.6 mg) in N,N-dimethylformamide (0.6 mL) was added triethylamine (0.0215 mL) at room temperature, and the mixture was stirred for 45 min and left standing overnight. The reaction mixture was purified by reversed-phase silica gel column chromatography (eluent: water/acetonitrile=95/5 to 0/100) to give the title compound (35.1 mg).
$^1$H-NMR (DMSO-$D_6$) δ: 9.99 (1H, s), 9.28 (2H, s), 8.58 (1H, d, J = 7.6 Hz), 8.53 (1H, d, J = 5.3 Hz), 7.94 (1H, d, J = 5.3 Hz), 4.05 (1H, br s), 3.81-3.68 (1H, m), 2.66 (3H, s), 1.97 (2H, d, J = 10.2 Hz), 1.86 (2H, d, J = 10.2 Hz), 1.40-1.26 (2H, m), 1.25-1.03 (3H, m), 1.06 (6H, s). MS(M+H):395 MS(M-H):393

[Production Example 3] Example 3: Production of N-(trans-4-(2-hydroxy-2-methylpropyl)cyclohexyl)-2-(3-methyl-1H-pyrazolo[3,4-c]pyridin-1-yl)pyrimidine-5-carboxamide

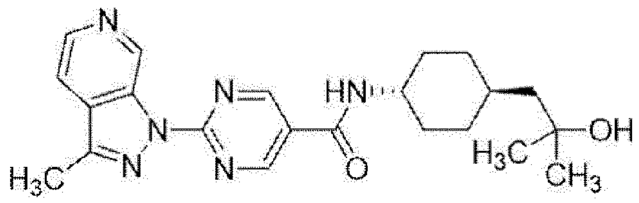

Step 3-1: tert-Butyl 2-chloropyrimidine-5-carboxylate

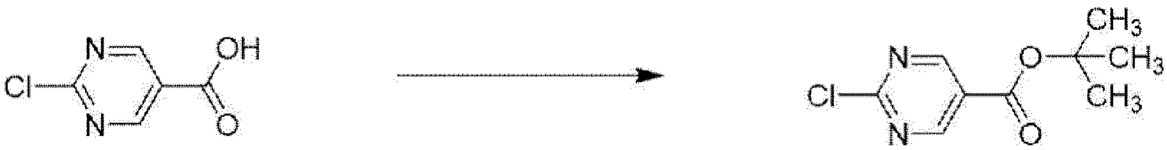

To a solution of 2-chloropyrimidine-5-carboxylic acid (2.0 g) and N,N-dimethyl-4-aminopyridine (0.462 g) in tert-butyl alcohol/chloroform (30 mL, tert-butyl alcohol/chloroform=2/1) was added di-tert-butyl bicarbonate (5.51 g) at room temperature, and the mixture was stirred at 60°C for 90 min and left standing overnight. To the reaction mixture was added ethyl acetate at room temperature, and the organic layer was washed successively with saturated aqueous sodium hydrogen carbonate, aqueous citric acid solution and saturated brine. The organic layer was dried over sodium sulfate, sodium sulfate was removed by filtration, and the filtrate was concentrated under reduced pressure. The residue was purified by silica gel column chromatography (eluent: n-hexane/ethyl acetate) to give the title compound (990 mg). $^1$H-NMR (DMSO-$D_6$) δ: 9.13 (2H, s), 1.57 (9H, s).

Step 3-2: tert-Butyl 2-(3-methyl-1H-pyrazolo[3,4-c]pyridin-1-yl)pyrimidine-5-carboxylate

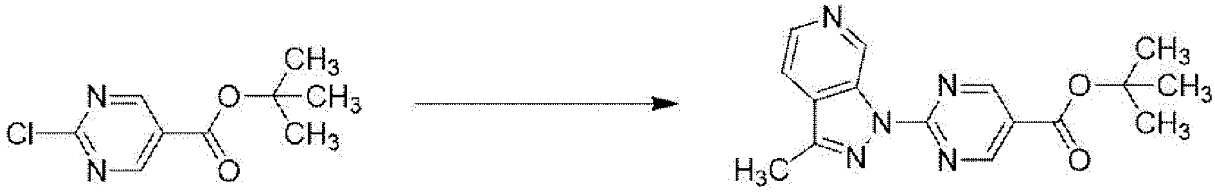

A suspension of tert-butyl 2-chloropyrimidine-5-carboxylate (100 mg), 3-methyl-1H-pyrazolo[3,4-c]pyridine (62 mg) and cesium carbonate (182 mg) in N,N-dimethylformamide (1.0 mL) was stirred at room temperature for 30 min and left standing overnight. To the reaction mixture was added water at room temperature, and the precipitated solid was collected by filtration and dried under reduced pressure at 70°C to give the title compound (130 mg).
$^1$H-NMR (DMSO-$D_6$) δ: 10.01 (1H, s), 9.29 (2H, s), 8.55 (1H, d, J = 5.2 Hz), 7.95 (1H, d, J = 5.2 Hz), 2.67 (3H, s), 1.61 (9H, s).

Step 3-3: 2-(3-Methyl-1H-pyrazolo[3,4-c]pyridin-1-yl)pyrimidine-5-carboxylic acid monohydrochloride

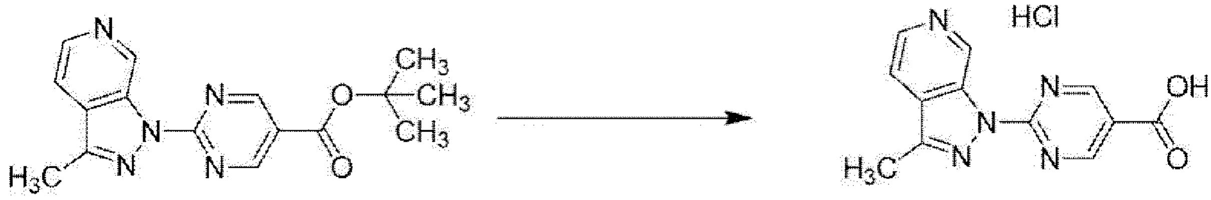

To a suspension of tert-butyl 2-(3-methyl-1H-pyrazolo[3,4-c]pyridin-1-yl)pyrimidine-5-carboxylate (130 mg) in ethyl acetate (0.26 mL) was added 4 M hydrogen chloride/ethyl acetate solution (1.3 mL) at room temperature, and the mixture was stirred for 6 hr and left standing overnight. The reaction mixture was concentrated under reduced pressure. To

the residue was added trifluoroacetic acid (0.65 mL) at room temperature, and the mixture was stirred for 5 hr and left standing overnight. To the reaction mixture was added 4 M hydrogen chloride/ethyl acetate solution at room temperature, and the precipitated solid was collected by filtration and dried under reduced pressure at 70°C to give the title compound (97.8 mg).
$^1$H-NMR (DMSO-$D_6$) δ: 13.73 (1H, br s), 10.06 (1H, s), 9.34 (2H, s), 8.62 (1H, d, J = 5.5 Hz), 8.15-8.08 (1H, m), 2.70 (3H, s).

Step 3-4: Ethyl 2-(trans-4-(dibenzylamino)cyclohexyl)acetate

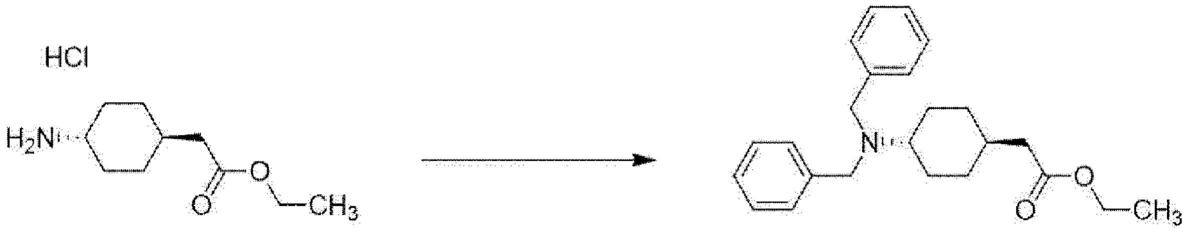

To a suspension of ethyl 2-(trans-4-aminocyclohexyl)acetate monohydrochloride (3.0 g) and potassium carbonate (5.99 g) in acetonitrile (30 mL) was added benzyl bromide (3.4 mL) at room temperature, and the mixture was stirred overnight. To the reaction mixture were added water (30 mL) and ethyl acetate (30 mL) at room temperature, and the layers were separated. The aqueous layer was extracted with ethyl acetate (10 mL). The combined organic layer was washed with saturated brine, and concentrated under reduced pressure. The residue was purified by silica gel column chromatography (eluent: n-hexane/ethyl acetate=96/4 to 74/26) to give a crude product (5.068 g) containing the title compound.

Step 3-5: 1-(Trans-4-(dibenzylamino)cyclohexyl)-2-methylpropan-2-ol

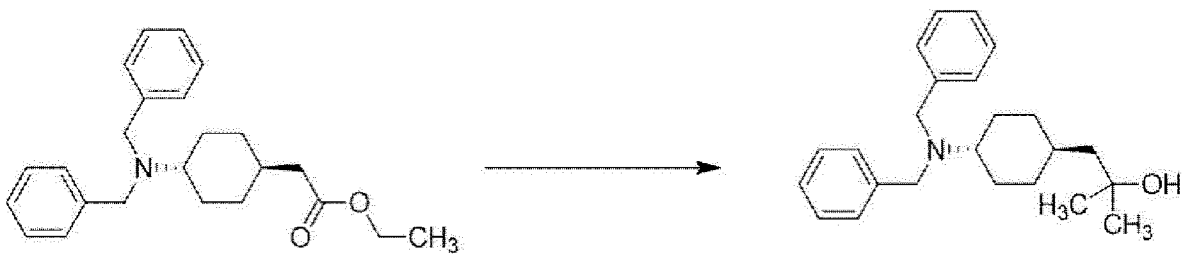

To a solution of the crude product containing ethyl 2-(trans-4-(dibenzylamino)cyclohexyl)acetate crude product (3.847 g) in tetrahydrofuran (20 mL) was added 3.0 M tetrahydrofuran solution (11 mL) of methylmagnesium bromide under ice-cooling, and the mixture was allowed to gradually return to room temperature and stirred overnight. To the reaction mixture were added saturated aqueous ammonium chloride solution (15 mL) and ethyl acetate (20 mL) under ice-cooling. After partitioning, the organic layer was washed with saturated brine, and concentrated under reduced pressure. The residue was purified by silica gel column chromatography (eluent: n-hexane/ethyl acetate=94/6 to 53/47) to give the title compound (1.45 g).
$^1$H-NMR (DMSO-$D_6$) δ: 7.35-7.26 (8H, m), 7.21-7.17 (2H, m), 3.96 (1H, s), 3.57 (4H, s), 2.38-2.31 (1H, m), 1.85-1.76 (4H, m), 1.42-1.31 (3H, m), 1.21-1.15 (2H, m), 1.06 (6H, s), 0.82-0.73 (2H, m).

Step 3-6: 1-(Trans-4-aminocyclohexyl)-2-methylpropan-2-ol

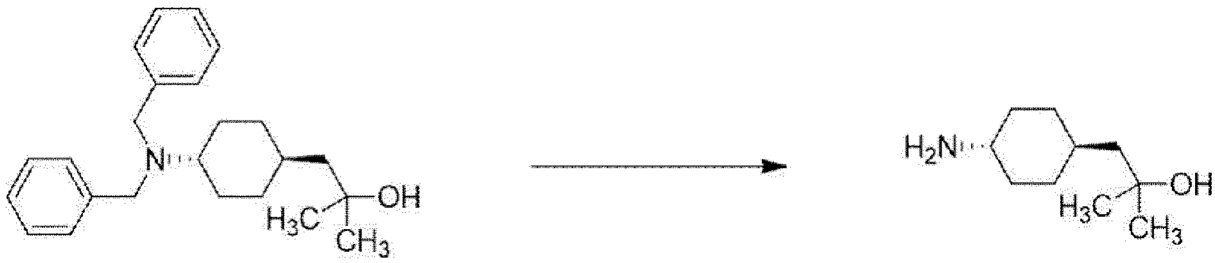

To a solution of 1-(trans-4-(dibenzylamino)cyclohexyl)-2-methylpropan-2-ol (1.45 g) in methanol (20 mL) was added 20% palladium hydroxide-carbon (368 mg) at room temperature. Under 1 atm hydrogen atmosphere, the mixture was stirred at room temperature overnight. The reaction mixture was filtered through celite, and the filtrate was concentrated under reduced pressure to give the title compound (692 mg).
$^1$H-NMR (DMSO-$D_6$) $\delta$: 3.98 (1H, br s), 2.44-2.37 (1H, m), 1.76-1.67 (4H, m), 1.47 (2H, br s), 1.34-1.22 (4H, m), 1.07 (6H, s), 0.99-0.84 (3H, m).

Step 3-7: N-(Trans-4-(2-hydroxy-2-methylpropyl)cyclohexyl)-2-(3-methyl-1H-pyrazolo[3,4-c]pyridin-1-yl)pyrimidine-5-carboxamide

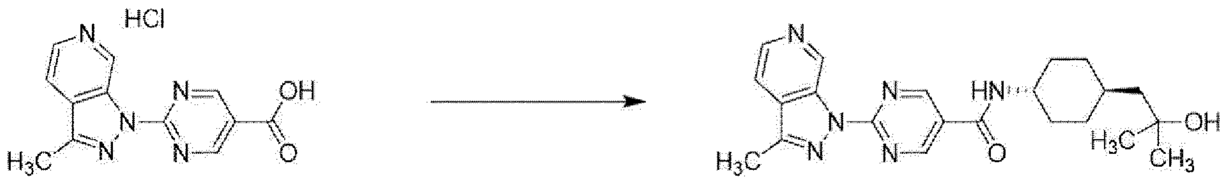

To a suspension of 2-(3-methyl-1H-pyrazolo[3,4-c]pyridin-1-yl)pyrimidine-5-carboxylic acid monohydrochloride (30 mg), 1-(trans-4-aminocyclohexyl)-2-methylpropan-2-ol (21.14 mg), 1-hydroxy-7-azabenzotriazole (4.5 mg) and 1-ethyl-3-(3-dimethylaminopropyl)carbodiimide hydrochloride (31.7 mg) in N,N-dimethylformamide (0.6 mL) was added triethylamine (0.0215 mL) at room temperature, and the mixture was stirred for 45 min and left standing overnight. The reaction mixture was purified by reversed-phase silica gel column chromatography (eluent: water/acetonitrile=95/5 to 0/100) to give the title compound (22.4 mg).

$^1$H-NMR (DMSO-$D_6$) $\delta$: 9.99 (1H, s), 9.27 (2H, s), 8.56 (1H, d, J = 7.6 Hz), 8.53 (1H, d, J = 5.3 Hz), 7.94 (1H, d, J = 5.3 Hz), 4.04 (1H, br s), 3.82-3.69 (1H, m), 2.66 (3H, s), 1.88 (4H, d, J = 10.6 Hz), 1.50-1.22 (5H, m), 1.21-0.98 (2H, m), 1.11 (6H, s).
MS (M+H) :409 MS (M-H) :407

[Production Example 4] Example 4: Production of N-(trans-4-(2-hydroxy-2-methylpropoxy)cyclohexyl)-2-(3-methyl-1H-indazol-1-yl)pyrimidine-5-carboxamide

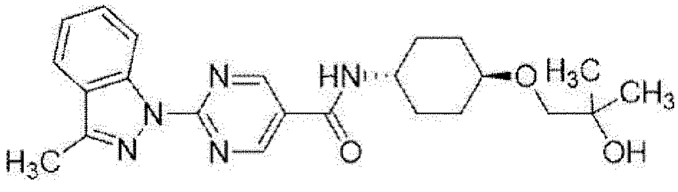

Step 4-1: Ethyl 2-(3-methyl-1H-indazol-1-yl)pyrimidine-5-carboxylate

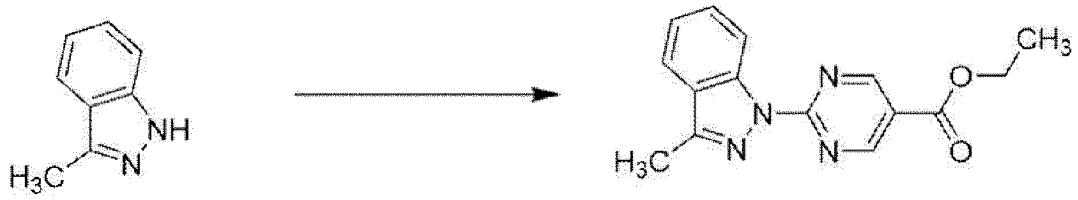

A suspension of 3-methyl-1H-indazole (5.0 g), ethyl 2-chloropyrimidine-carboxylate (8.47 g) and cesium carbonate (24.65 g) in N,N-dimethylformamide (25 mL) was stirred at 90°C for 3 hr. To the reaction mixture were added water, ethyl acetate and chloroform at room temperature, and the layers were separated. The aqueous layer was extracted once with ethyl acetate. The combined organic layer was washed with saturated brine, and dried over magnesium sulfate. Magnesium sulfate was removed by filtration, and the filtrate was concentrated under reduced pressure. The residue was purified by silica gel column chromatography (eluent: n-hexane/ethyl acetate=85/15 to 0/100) to give a crude product (9.5 g) containing the title compound. To the crude product (9.5 g) containing the title compound was added n-hexane/ethyl acetate (30 mL, n-hexane/ethyl acetate=1/2), and slurry purification was performed. The solid was collected by filtration and dried under reduced pressure to give a crude product (7.8 g) containing the title compound. A solution of the crude product (7.8 g) containing the title compound in chloroform was purified by silica gel column chromatography (eluent: chloroform) to give the title compound (6.4 g).
$^1$H-NMR (DMSO-$D_6$) δ: 9.26 (2H, s), 8.69-8.67 (1H, m), 7.90-7.88 (1H, m), 7.66-7.62 (1H, m), 7.43-7.39 (1H, m), 4.39 (2H, q, J = 7.1 Hz), 2.62 (3H, s), 1.37 (3H, t, J = 7.2 Hz).

Step 4-2: 2-(3-Methyl-1H-indazol-1-yl)pyrimidine-5-carboxylic acid

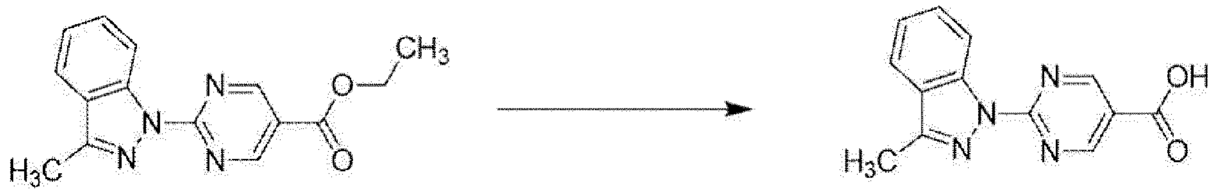

To a solution of ethyl 2-(3-methyl-1H-indazol-1-yl)pyrimidine-5-carboxylate (3.0 g) in tetrahydrofuran (60 mL) was added 2 M aqueous sodium hydroxide solution (15.94 mL) at room temperature, and the mixture was stirred for 3 hr. To the reaction mixture was added 2 M aqueous sodium hydroxide solution (5.31 mL) at room temperature, and the mixture was stirred for 1 hr and left standing overnight. To the reaction mixture was added 2 M hydrochloric acid (21.25 mL) at room temperature, and the mixture was stirred for 30 min. To the reaction mixture was added water at room temperature, and the mixture was stirred for 30 min. The precipitated solid was collected by filtration and dried under reduced pressure to give the title compound (2.47 g).
$^1$H-NMR (DMSO-$D_6$) δ: 13.64 (1H, br s), 9.26 (2H, s), 8.70 (1H, d, J = 8.2 Hz), 7.89 (1H, d, J = 7.5 Hz), 7.66-7.62 (1H, m), 7.41 (1H, t, J = 7.5 Hz), 2.62 (3H, s).

Step 4-3: Trans-4-(dibenzylamino)cyclohexan-1-ol

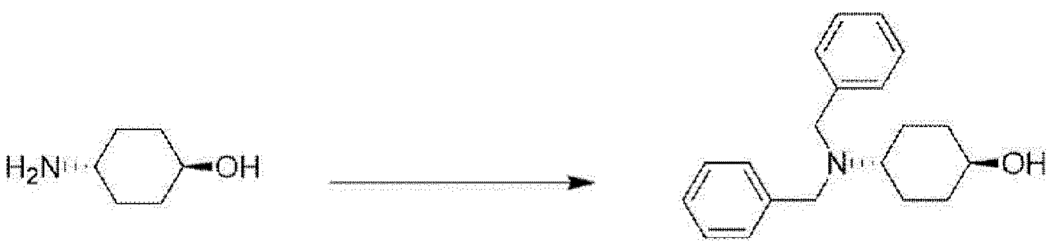

To a suspension of trans-4-aminocyclohexan-1-ol (5.0 g) and potassium carbonate (13.2 g) in acetonitrile (80 mL) was added benzyl bromide (10.9 mL) at room temperature, and the mixture was stirred overnight. To the reaction

mixture was added water (100 mL) at room temperature, and the precipitated solid was collected by filtration, washed three times with water (40 mL), and dried under reduced pressure at 80°C to give the title compound (12.88 g). The title compound (10.38 g) was obtained from the starting material, trans-4-aminocyclohexan-1-ol (5.0 g), by a similar production method.
$^1$H-NMR (DMSO-$D_6$) δ: 7.35-7.27 (8H, m), 7.21-7.17 (2H, m), 4.43 (1H, br s), 3.55 (4H, s), 3.36-3.29 (1H, m), 2.39-2.31 (1H, m), 1.83-1.76 (4H, m), 1.43-1.37 (2H, m), 1.03-0.93 (2H, m).

Step 4-4: Ethyl 2-{[trans-4-(dibenzylamino)cyclohexyl]oxy}acetate

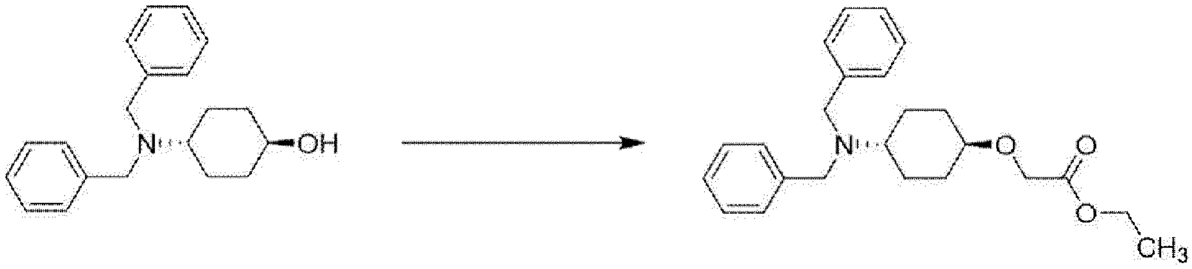

To a solution of trans-4-(dibenzylamino)cyclohexan-1-ol (5.0 g) in dichloromethane (50 mL) was added rhodium (II) acetate dimer (22.4 mg) at room temperature, 15 wt% ethyl diazoacetate/toluene solution (57.5 mL) was added dropwise over 2 hr, and the mixture was stirred for 2 hr. The reaction mixture was concentrated under reduced pressure. The residue was purified by silica gel column chromatography (eluent: n-hexane/ethyl acetate=93/7 to 40/60) to give the title compound (5.779 g).
$^1$H-NMR ($CDCl_3$) δ: 7.38-7.32 (4H, m), 7.31-7.24 (4H, m), 7.23-7.17 (2H, m), 4.20 (2H, q, J = 7.4 Hz), 4.07 (2H, s), 3.60 (4H, s), 3.32-3.20 (1H, m), 2.58-2.47 (1H, m), 2.13-2.05 (2H, m), 1.96-1.87 (2H, m), 1.43-1.16 (7H, m).

Step 4-5: 1-{[Trans-4-(dibenzylamino)cyclohexyl]oxy}-2-methylpropan-2-ol

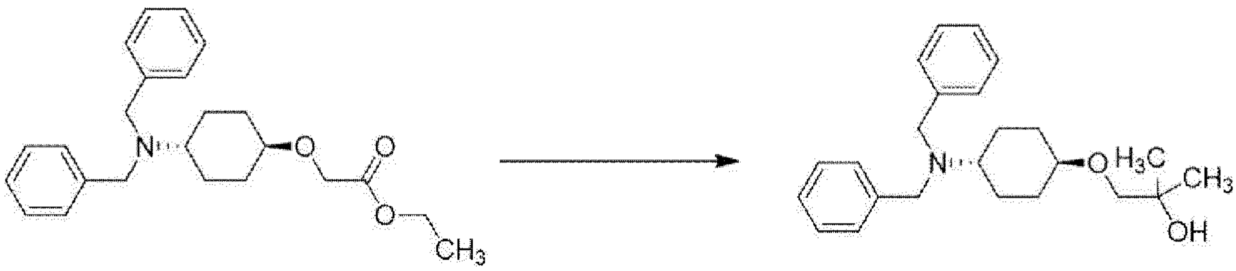

To a solution of ethyl 2-{[trans-4-(dibenzylamino)cyclohexyl]oxy}acetate (5.779 g) in tetrahydrofuran (57.8 mL) was added 1.02 M tetrahydrofuran solution (44.6 mL) of methylmagnesium bromide under ice-cooling, and the mixture was stirred at the same temperature for 2 hr. To the reaction mixture was added saturated aqueous ammonium chloride solution under ice-cooling. To the reaction mixture was added ethyl acetate at room temperature, and the layers were separated. The aqueous layer was extracted once with ethyl acetate. The combined organic layer was washed successively with water and saturated brine, and dried over sodium sulfate. Sodium sulfate was removed by filtration, and the filtrate was concentrated under reduced pressure. The residue was purified by silica gel column chromatography (eluent: n-hexane/ethyl acetate=93/7 to 40/60) to give the title compound (4.04 g).
$^1$H-NMR ($CDCl_3$) δ: 7.38-7.33 (4H, m), 7.32-7.25 (4H, m), 7.23-7.17 (2H, m), 3.60 (4H, s), 3.24 (2H, s), 3.22-3.12 (1H, m), 2.57-2.47 (1H, m), 2.33 (1H, s), 2.10-2.01 (2H, m), 1.96-1.86 (2H, m), 1.43-1.33 (2H, m), 1.20-1.10 (8H, m).

Step 4-6: 1-((Trans-4-aminocyclohexyl)oxy)-2-methylpropan-2-ol

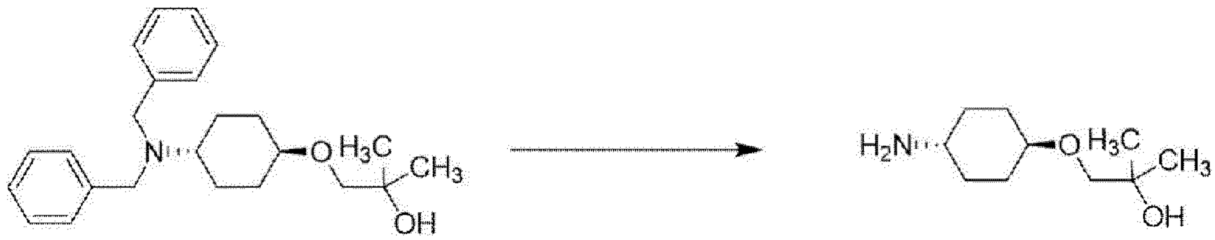

To a solution of 1-{[trans-4-(dibenzylamino)cyclohexyl]oxy}-2-methylpropan-2-ol (3.715 g) in methanol/tetrahydrofuran (40 mL, methanol/tetrahydrofuran=1/1) was added 20% palladium hydroxide-carbon (310 mg) at room temperature. Under 1 atm hydrogen atmosphere, the mixture was stirred at room temperature overnight. The reaction mixture was filtered through celite, and the filtrate was concentrated under reduced pressure to give the title compound (1.926 g). $^1$H-NMR (DMSO-$D_6$) $\delta$: 4.17 (1H, br s), 3.18-3.11 (2H, m), 2.55-2.48 (2H, m), 1.92-1.86 (2H, m), 1.75-1.68 (2H, m), 1.47 (2H, br s), 1.20-1.09 (2H, m), 1.06-0.96 (2H, m), 1.04 (6H, s).

Step 4-7: N-(Trans-4-(2-hydroxy-2-methylpropoxy)cyclohexyl)-2-(3-methyl-1H-indazol-1-yl)pyrimidine-5-carboxamide

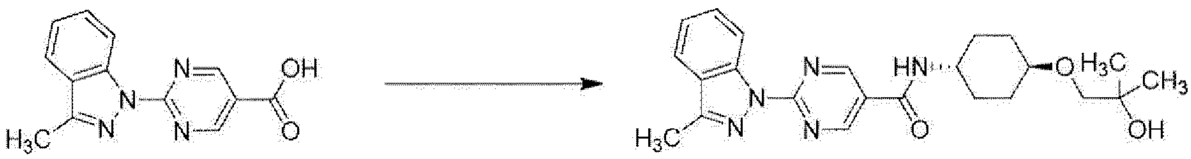

A suspension of 2-(3-methyl-1H-indazol-1-yl)pyrimidine-5-carboxylic acid (30 mg), 1-((trans-4-aminocyclohexyl)oxy)-2-methylpropan-2-ol (27 mg), 1-hydroxy-7-azabenzotriazole (16 mg) and 1-ethyl-3-(3-dimethylaminopropyl) carbodiimide hydrochloride (35 mg) in N,N-dimethylformamide (1.0 mL) was stirred overnight. To the reaction mixture were added saturated aqueous sodium hydrogen carbonate (2.0 mL) and water (3.0 mL) at room temperature, and the precipitated solid was collected by filtration and dried under reduced pressure to give the title compound (44.8 mg).

$^1$H-NMR (DMSO-$D_6$) $\delta$: 9.22 (2H, s), 8.67 (1H, d, J = 8.6 Hz), 8.53 (1H, d, J = 7.6 Hz), 7.89 (1H, d, J = 7.9 Hz), 7.65-7.61 (1H, m), 7.42-7.38 (1H, m), 4.22 (1H, s), 3.85-3.76 (1H, m), 3.29-3.22 (1H, m), 3.19 (2H, s), 2.62 (3H, s), 2.06-2.01 (2H, m), 1.95-1.91 (2H, m), 1.45-1.23 (4H, m), 1.08 (6H, s).
MS(M+H):424 MS(M-H):422

[Production Example 5] Example 5: Production of N-(trans-4-(2-hydroxy-2-methylpropyl)cyclohexyl)-2-(3-methyl-1H-pyrazolo[3,4-c]pyridin-1-yl)pyrimidine-5-carboxamide

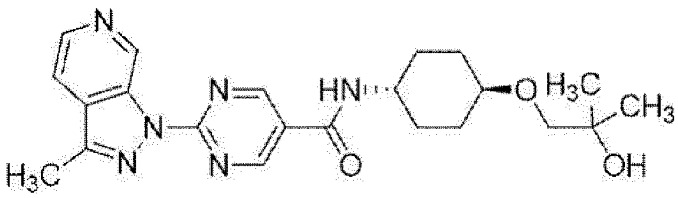

Step 5-1: tert-Butyl 2-chloropyrimidine-5-carboxylate

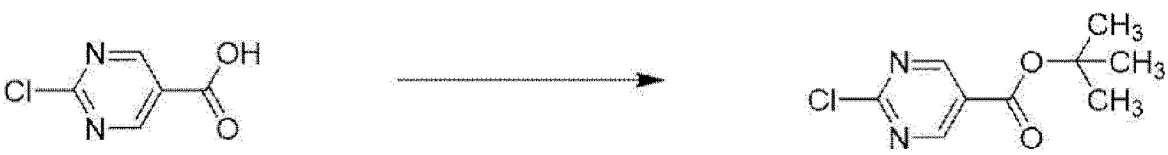

To a solution of 2-chloropyrimidine-5-carboxylic acid (2.0 g) and N,N-dimethyl-4-aminopyridine (0.462 g) in tert-butyl alcohol/chloroform (30 mL, tert-butyl alcohol/chloroform=2/1) was added di-tert-butyl bicarbonate (5.51 g) at room temperature, and the mixture was stirred at 60°C for 90 min and left standing overnight. To the reaction mixture was added ethyl acetate at room temperature, and the organic layer was washed successively with saturated aqueous sodium hydrogen carbonate, aqueous citric acid solution and saturated brine. The organic layer was dried over sodium sulfate, sodium sulfate was removed by filtration, and the filtrate was concentrated under reduced pressure. The residue was purified by silica gel column chromatography (eluent: n-hexane/ethyl acetate) to give the title compound (990 mg). $^1$H-NMR (DMSO-$D_6$) δ: 9.13 (2H, s), 1.57 (9H, s).

Step 5-2: tert-Butyl 2-(3-methyl-1H-pyrazolo[3,4-c]pyridin-1-yl)pyrimidine-5-carboxylate

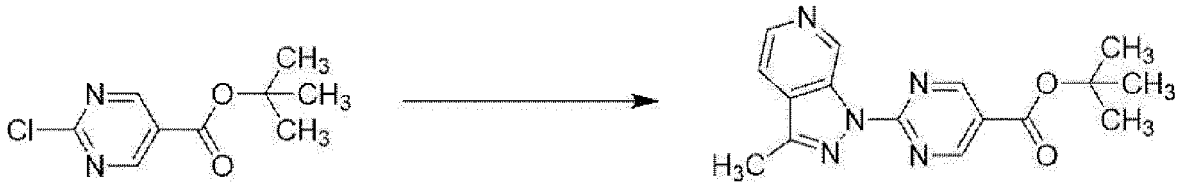

A suspension of tert-butyl 2-chloropyrimidine-5-carboxylate (500 mg), 3-methyl-1H-pyrazolo[3,4-c]pyridine (310 mg) and cesium carbonate (911 mg) in N,N-dimethylformamide (5.0 mL) was stirred at room temperature for 1 hr and left standing overnight. To the reaction mixture was added water at room temperature, and the precipitated solid was collected by filtration and dried under reduced pressure at 70°C to give the title compound (618 mg).
$^1$H-NMR (DMSO-$D_6$) δ: 10.01 (1H, s), 9.29 (2H, s), 8.55 (1H, d, J = 5.3 Hz), 7.95 (1H, dd, J = 5.3, 0.9 Hz), 2.67 (3H, s), 1.60 (9H, s).

Step 5-3: 2-(3-Methyl-1H-pyrazolo[3,4-c]pyridin-1-yl)pyrimidine-5-carboxylic acid monohydrochloride

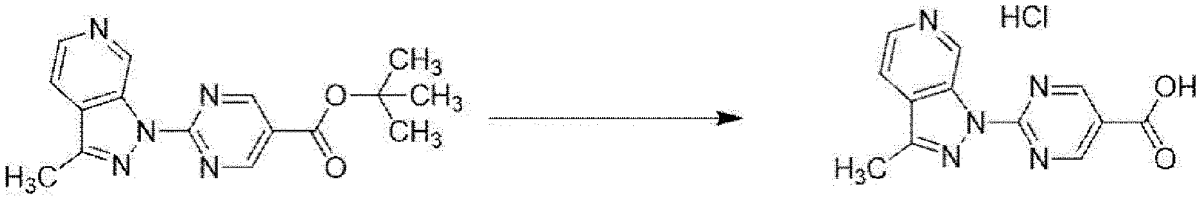

A solution of tert-butyl 2-(3-methyl-1H-pyrazolo[3,4-c]pyridin-1-yl)pyrimidine-5-carboxylate (618 mg) in trifluoroacetic acid (3.09 mL) was stirred for 2 hr and left standing overnight. To the reaction mixture was added 4 M hydrogen chloride/ethyl acetate solution at room temperature, and the mixture was stirred for 1 hr. The precipitated solid was collected by filtration, and dried under reduced pressure at 70°C to give the title compound (563 mg).
$^1$H-NMR (DMSO-$D_6$) δ: 13.77 (1H, br s), 10.08 (1H, s), 9.35 (2H, s), 8.66 (1H, d, J = 5.2 Hz), 8.23 (1H, d, J = 5.2 Hz), 2.72 (3H, s).

Step 5-4: Trans-4-(dibenzylamino)cyclohexan-1-ol

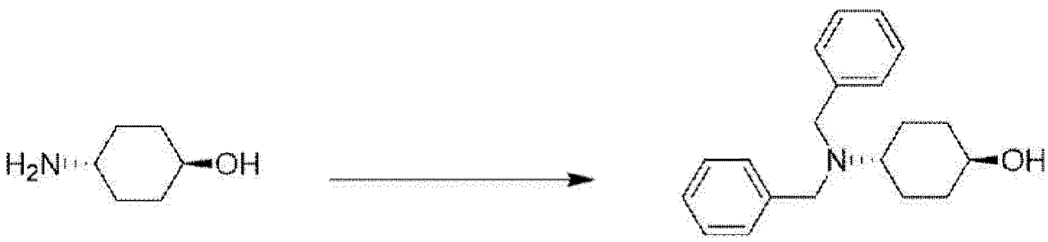

To a suspension of trans-4-aminocyclohexan-1-ol (5.0 g) and potassium carbonate (13.2 g) in acetonitrile (80 mL) was added benzyl bromide (10.9 mL) at room temperature, and the mixture was stirred overnight. To the reaction mixture was added water (100 mL) at room temperature, and the precipitated solid was collected by filtration, washed 3 times with water (40 mL), and dried under reduced pressure at 80°C to give the title compound (12.88 g).
$^1$H-NMR (DMSO-D$_6$) δ: 7.35-7.27 (8H, m), 7.21-7.17 (2H, m), 4.43 (1H, br s), 3.55 (4H, s), 3.36-3.29 (1H, m), 2.39-2.31 (1H, m), 1.83-1.76 (4H, m), 1.43-1.37 (2H, m), 1.03-0.93 (2H, m).

Step 5-5: ethyl 2-{[trans-4-(dibenzylamino)cyclohexyl]oxy}acetate

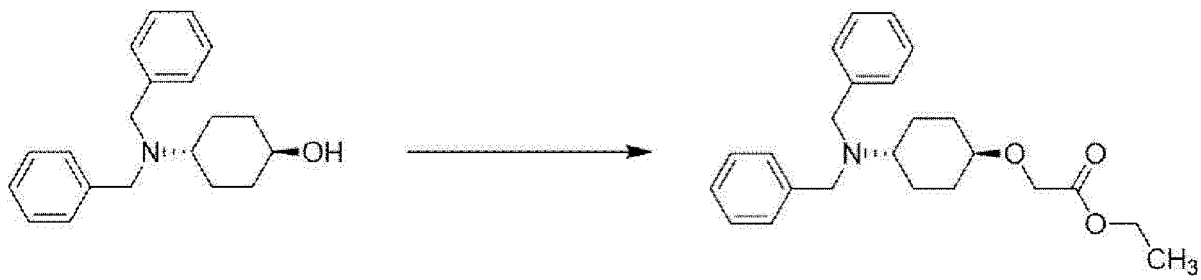

To a solution of trans-4-(dibenzylamino)cyclohexan-1-ol (5.0 g) in dichloromethane (50 mL) was added rhodium (II) acetate dimer (22.4 mg) at room temperature, 15 wt% ethyl diazoacetate/toluene solution (57.5 mL) was added dropwise over 2 hr, and the mixture was stirred for 2 hr. The reaction mixture was concentrated under reduced pressure. The residue was purified by silica gel column chromatography (eluent: n-hexane/ethyl acetate=93/7 to 40/60) to give the title compound (5.779 g).
$^1$H-NMR (CDCl$_3$) δ: 7.38-7.32 (4H, m), 7.31-7.24 (4H, m), 7.23-7.17 (2H, m), 4.20 (2H, q, J = 7.4 Hz), 4.07 (2H, s), 3.60 (4H, s), 3.32-3.20 (1H, m), 2.58-2.47 (1H, m), 2.13-2.05 (2H, m), 1.96-1.87 (2H, m), 1.43-1.16 (7H, m).

Step 5-6: 1-{[Trans-4-(dibenzylamino)cyclohexyl]oxy}-2-methylpropan-2-ol

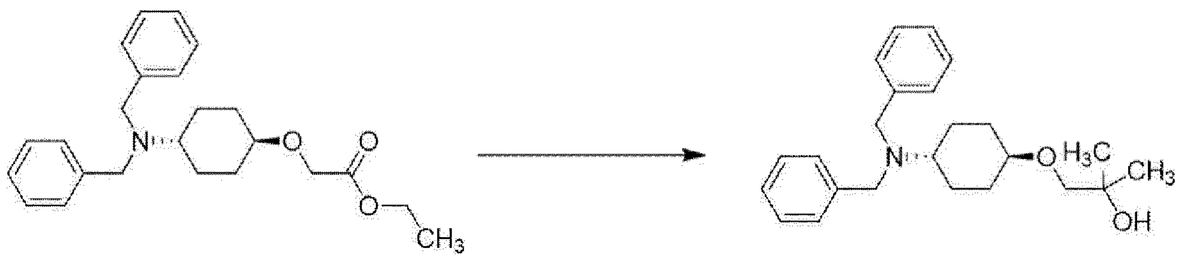

To a solution of ethyl 2-{[trans-4-(dibenzylamino)cyclohexyl]oxy}acetate (5.779 g) in tetrahydrofuran (57.8 mL) was added 1.02 M tetrahydrofuran solution (44.6 mL) of methylmagnesium bromide under ice-cooling, and the mixture was stirred at the same temperature for 2 hr. To the reaction mixture was added saturated aqueous ammonium chloride solution under ice-cooling. To the reaction mixture was added ethyl acetate at room temperature. After partitioning, the aqueous layer was extracted once with ethyl acetate. The combined organic layer was washed successively with water and saturated brine, and dried over sodium sulfate. Sodium sulfate was removed by filtration, and the filtrate was concentrated under reduced pressure. The residue was purified by silica gel column chromatography (eluent: n-hexane/ethyl acetate=93/7 to 40/60) to give the title compound (4.04 g).
$^1$H-NMR (CDCl$_3$) δ: 7.38-7.33 (4H, m), 7.32-7.25 (4H, m), 7.23-7.17 (2H, m), 3.60 (4H, s), 3.24 (2H, s), 3.22-3.12 (1H, m), 2.57-2.47 (1H, m), 2.33 (1H, s), 2.10-2.01 (2H, m), 1.96-1.86 (2H, m), 1.43-1.33 (2H, m), 1.20-1.10 (8H, m).

Step 5-7: 1-((Trans-4-aminocyclohexyl)oxy)-2-methylpropan-2-ol

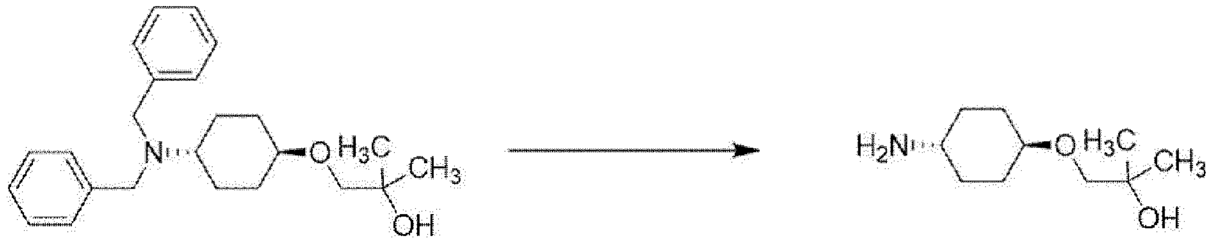

To a solution of 1-{[trans-4-(dibenzylamino)cyclohexyl]oxy}-2-methylpropan-2-ol (3.715 g) in methanol (20 mL) was added 20% palladium hydroxide-carbon (310 mg) at room temperature. Under 1 atm hydrogen atmosphere, the mixture was stirred at room temperature overnight. The reaction mixture was filtered through celite, and the filtrate was concentrated under reduced pressure to give the title compound (1.926 g).
$^1$H-NMR (DMSO-$D_6$) δ: 4.17 (1H, br s), 3.18-3.11 (2H, m), 2.55-2.48 (2H, m), 1.92-1.86 (2H, m), 1.75-1.68 (2H, m), 1.47 (2H, br s), 1.20-1.09 (2H, m), 1.06-0.96 (2H, m), 1.04 (6H, s).

Step 5-8: N-(Trans-4-(2-hydroxy-2-methylpropyl)cyclohexyl)-2-(3-methyl-1H-pyrazolo[3,4-c]pyridin-1-yl)pyrimidine-5-carboxamide

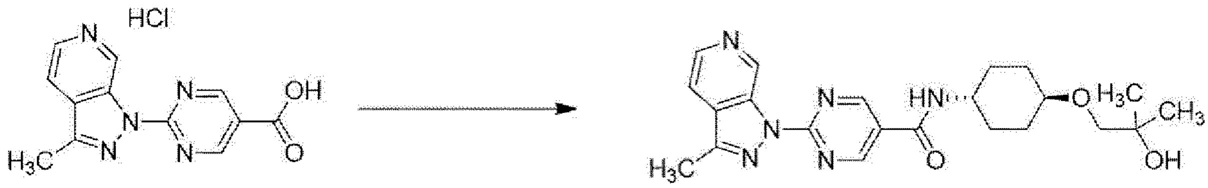

To a suspension of 2-(3-methyl-1H-pyrazolo[3,4-c]pyridin-1-yl)pyrimidine-5-carboxylic acid monohydrochloride (30 mg), 1-((trans-4-aminocyclohexyl)oxy)-2-methylpropan-2-ol (23.11 mg), 1-hydroxy-7-azabenzotriazole (4.2 mg) and 1-ethyl-3-(3-dimethylaminopropyl)carbodiimide hydrochloride (29.6 mg) in N,N-dimethylformamide (0.6 mL) was added triethylamine (0.0215 mL) at room temperature, and the mixture was stirred for 2 hr and left standing overnight. To the reaction mixture was added water at room temperature, and the precipitated solid was collected by filtration and dried under reduced pressure at 70°C to give the title compound (41.4 mg).

$^1$H-NMR (DMSO-$D_6$) δ: 9.99 (1H, s), 9.27 (2H, s), 8.58 (1H, d, J = 7.5 Hz), 8.54 (1H, d, J = 5.2 Hz), 7.94 (1H, d, J = 5.2 Hz), 4.20 (1H, br s), 3.87-3.74 (1H, m), 3.51-3.22 (1H, m), 3.19 (2H, s), 2.67 (3H, s), 2.04 (2H, d, J = 10.5 Hz), 1.93 (2H, d, J = 10.5 Hz), 1.48-1.20 (4H, m), 1.07 (6H, s).
MS(M+H):425 MS(M-H):423

[Production Example 6] Example 6: Production of 2-(3-(difluoromethyl)-1H-indazol-1-yl)-N-(trans-4-(3-(methylsulfonyl)propoxy)cyclohexyl)pyrimidine-5-carboxamide

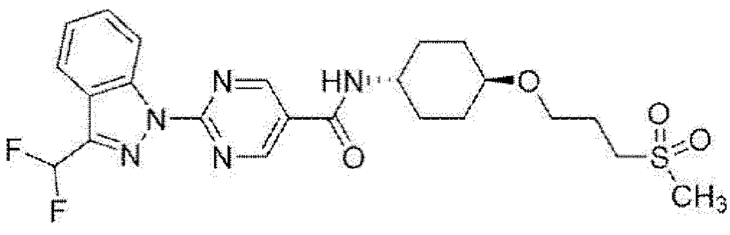

Step 6-1: 3-(Difluoromethyl)-1H-indazole

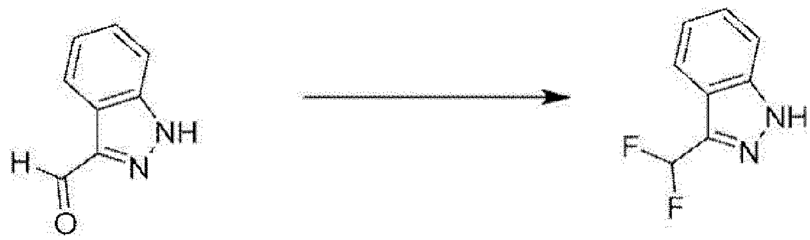

To a solution of 1H-indazole-3-carbaldehyde (1.5 g) in dichloromethane (10 mL) was added N,N-diethylaminosulfur trifluoride (2.71 mL) under ice-cooling, and the mixture was allowed to gradually return to room temperature and stirred for 5 hr. To the reaction mixture was added dropwise saturated aqueous sodium hydrogen carbonate under ice-cooling, ethyl acetate was added, and the layers were separated. The aqueous layer was extracted once with ethyl acetate. The combined organic layer was washed with saturated brine, and dried over magnesium sulfate. Magnesium sulfate was removed by filtration, and the filtrate was concentrated under reduced pressure. The residue was purified by silica gel column chromatography (eluent: n-hexane/ethyl acetate=95/5 to 80/20) and silica gel column chromatography (eluent: n-hexane/ethyl acetate=100/0 to 85/15) to give the title compound (343 mg). The title compound (673 mg) was obtained from the starting material, 1H-indazole-3-carboxaldehyde, by a similar production method.
$^1$H-NMR (DMSO-D$_6$) δ: 13.59 (1H, s), 7.84 (1H, d, J = 8.2 Hz), 7.64 (1H, d, J = 9.0 Hz), 7.46 (1H, t, J = 7.5 Hz), 7.34 (1H, t, J = 53.9 Hz), 7.25 (1H, t, J = 7.5 Hz).

Step 6-2: Ethyl 2-(3-(difluoromethyl)-1H-indazol-1-yl)pyrimidine-5-carboxylate

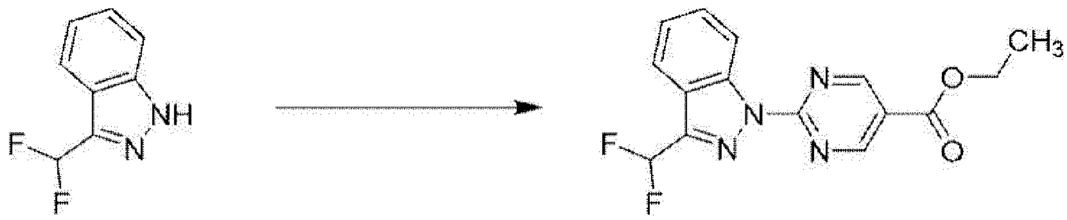

A suspension of 3-(difluoromethyl)-1H-indazole (0.95 g), ethyl 2-chloropyrimidine-carboxylate (1.1 g) and cesium carbonate (2.2 g) in N,N-dimethylformamide (10 mL) was stirred at room temperature for 4 hr. To the reaction mixture was added water under ice-cooling, and the mixture was stirred for 1 hr. The precipitated solid was collected by filtration and dried under reduced pressure to give the title compound (1.73 g).
$^1$H-NMR (DMSO-D$_6$) δ: 9.38 (2H, s), 8.81 (1H, d, J = 8.6 Hz), 8.01 (1H, d, J = 8.1 Hz), 7.77 (1H, t, J = 8.4 Hz), 7.57 (1H, t, J = 54.0 Hz), 7.54 (1H, t, J = 7.6 Hz), 4.42 (2H, q, J = 7.1 Hz), 1.38 (3H, t, J = 7.1 Hz).

Step 6-3: 2-(3-(Difluoromethyl)-1H-indazol-1-yl)pyrimidine-5-carboxylic acid

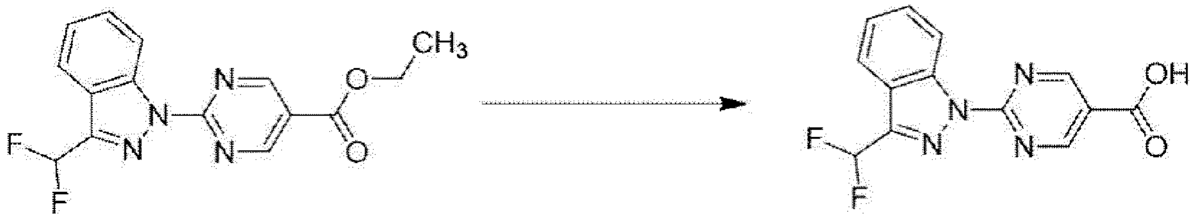

To a solution of ethyl 2-(3-(difluoromethyl)-1H-indazol-1-yl)pyrimidine-5-carboxylate (1.73 g) in tetrahydrofuran (10 mL) was added 2 M aqueous sodium hydroxide solution (5.03 mL) under ice-cooling, and the mixture was allowed to gradually return to room temperature and stirred for 6 hr. To the reaction mixture was added 2 M hydrochloric acid (5.0 mL) under ice-cooling, and the mixture was stirred for 1 hr. The precipitated solid was collected by filtration and dried under reduced pressure to give the title compound (1.45 g).
$^1$H-NMR (DMSO-D$_6$) δ: 13.80 (1H, br s), 9.35 (2H, s), 8.82 (1H, d, J = 8.5 Hz), 8.01 (1H, d, J = 7.9 Hz), 7.78-7.74 (1H, m),

7.56 (1H, t, J = 54.0 Hz), 7.56-7.51 (1H, m).

Step 6-4: Methyl (E)-3-((trans-4-((tert-butoxycarbonyl)amino)cyclohexyl)oxy)acrylate

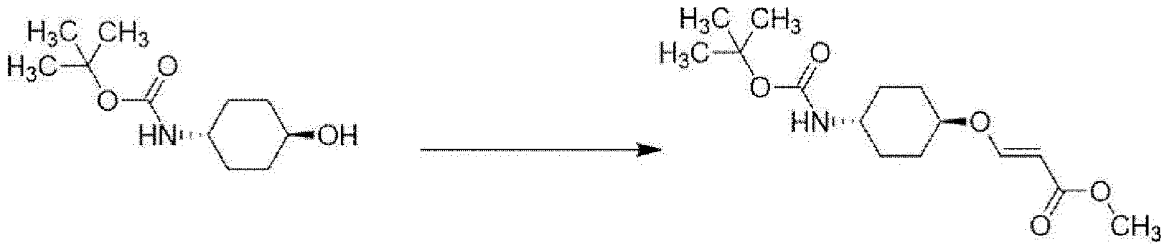

To a suspension of tert-butyl (trans-4-hydroxycyclohexyl)carbamate (5.0 g) and 1,4-diazabicyclo[2,2,2]octane (0.261 g) in tetrahydrofuran (50 mL) was added dropwise methyl propiolate (1.935 mL) at room temperature, and the mixture was stirred for 1 hr and left standing overnight. To the reaction mixture was added ethyl acetate at room temperature, and the organic layer was washed successively twice with saturated aqueous ammonium chloride solution and once with saturated brine. The organic layer was dried over sodium sulfate, sodium sulfate was removed by filtration, and the filtrate was concentrated under reduced pressure to give a crude product (7.42 g) containing the title compound.

Step 6-5: Methyl 3-((trans-4-((tert-butoxycarbonyl)amino)cyclohexyl)oxy)propionate

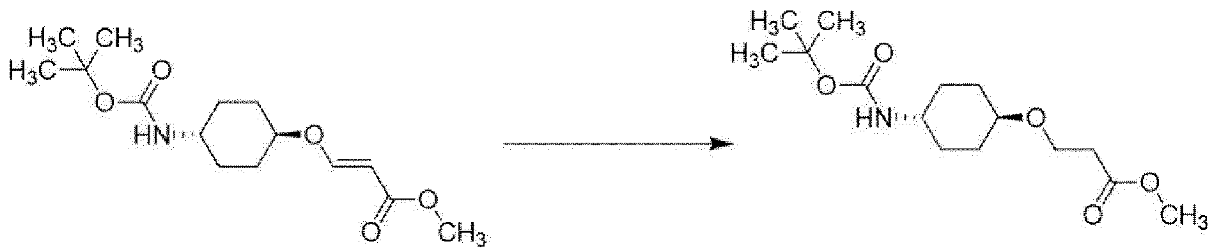

To a solution of a crude product (2.0 g) containing methyl (E)-3-((trans-4-((tert-butoxycarbonyl)amino)cyclohexyl)oxy)acrylate in methanol (9.37 mL) was added 10% palladium-carbon (187 mg) at room temperature. Under 1 atm hydrogen atmosphere, the mixture was stirred at room temperature for 3 hr. The reaction mixture was filtered through celite, and the filtrate was concentrated under reduced pressure to give a crude product (1.71 g) containing the title compound.

Step 6-6: 3-((Trans-4-((tert-butoxycarbonyl)amino)cyclohexyl)oxy)propionic acid

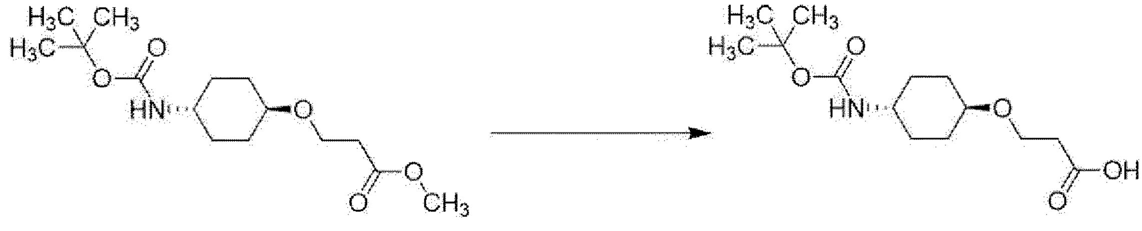

To a solution of a crude product (0.974 mg) containing methyl 3-((trans-4-((tert-butoxycarbonyl)amino)cyclohexyl)oxy)propionate in methanol (9.14 mL) was added 4 M aqueous sodium hydroxide solution (1.13 mL) at room temperature, and the mixture was stirred for 45 min and left standing overnight. The reaction mixture was concentrated under reduced pressure. To the residue were added water and ethyl acetate, the layers were separated, to the aqueous layer was added 2 M hydrochloric acid (2.4 mL), and the mixture was extracted twice with ethyl acetate. The combined

organic layer was washed with saturated brine, and dried over sodium sulfate. Sodium sulfate was removed by filtration, and the filtrate was concentrated under reduced pressure to give the title compound (0.94 g).
$^1$H-NMR (DMSO-$D_6$) δ: 12.04 (1H, br s), 6.69 (1H, d, J = 7.4 Hz), 3.58 (2H, t, J = 6.4 Hz), 3.22-3.10 (2H, m), 2.39 (2H, t, J = 6.4 Hz), 1.94-1.85 (2H, m), 1.79-1.70 (2H, m), 1.37 (9H, s), 1.24-1.07 (4H, m).

Step 6-7: tert-Butyl (trans-4-(3-hydroxypropoxy) cyclohexyl) carbamate

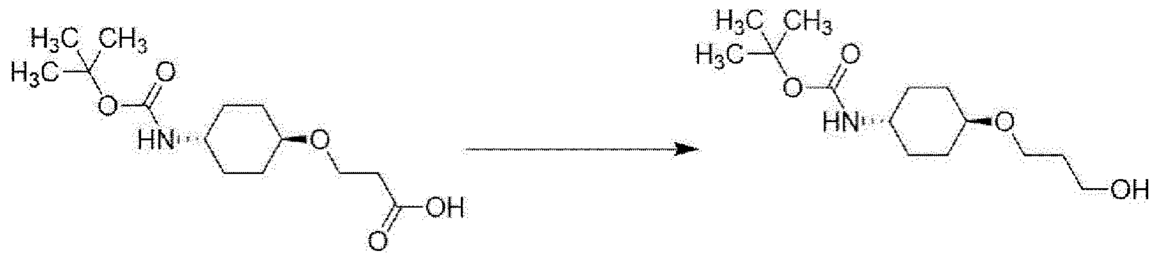

To a solution of 3-((trans-4-((tert-butoxycarbonyl)amino)cyclohexyl)oxy)propionic acid (0.94 g) and triethylamine (0.384 mL) in tetrahydrofuran (7.548 mL) was added isobutyl chloroformate (0.362 mL) under ice-cooling, and the mixture was stirred for 30 min. The reaction mixture was filtered to remove insoluble materials. To the filtrate was added dropwise sodium borohydride (0.298 g)/0.05 M aqueous sodium hydroxide solution (0.3774 mL) under ice-cooling. The mixture was stirred for 2 hr, allowed to gradually return to room temperature, and stirred for 90 min. To the reaction mixture were added saturated aqueous sodium hydrogen carbonate, water and ethyl acetate, and the layers were separated. The aqueous layer was extracted with ethyl acetate. The combined organic layer was washed successively with water and saturated brine, and dried over sodium sulfate. Sodium sulfate was removed by filtration, and the filtrate was concentrated under reduced pressure to give the title compound (0.774 g).
$^1$H-NMR (DMSO-$D_6$) δ: 6.68 (1H, d, J = 7.9 Hz), 4.32 (1H, t, J = 5.1 Hz), 3.46-3.38 (4H, m), 3.23-3.05 (2H, m), 1.95-1.86 (2H, m), 1.80-1.70 (2H, m), 1.65-1.55 (2H, m), 1.37 (9H, s), 1.22-1.04 (4H, m).

Step 6-8: 3-((Trans-4-((tert-butyl-butoxycarbonyl)amino)cyclohexyl)oxy)propylmethanesulfonate

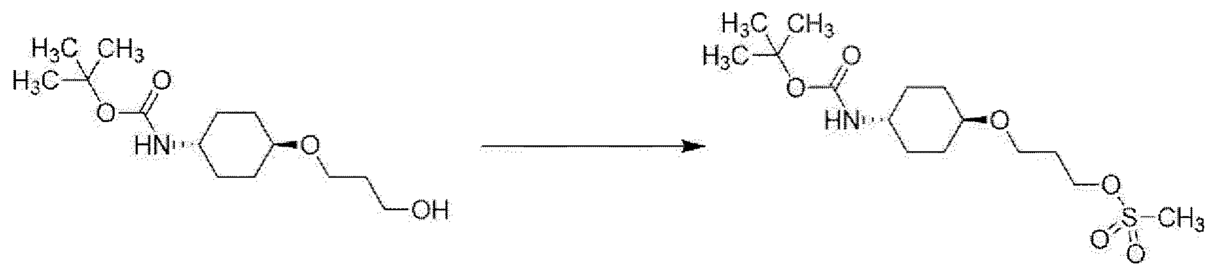

To a solution of tert-butyl (trans-4-(3-hydroxypropoxy)cyclohexyl)carbamate (370 mg) and triethylamine (0.238 mL) in tetrahydrofuran (1.55 mL) was added methanesulfonic anhydride (297 mg) under ice-cooling. The mixture was allowed to gradually return to room temperature, and stirred for 90 min. To the reaction mixture were added water and ethyl acetate at room temperature, and the layers were separated. The aqueous layer was extracted with ethyl acetate. The combined organic layer was washed successively with saturated aqueous sodium hydrogen carbonate and saturated brine, and dried over sodium sulfate. Sodium sulfate was removed by filtration, and the filtrate was concentrated under reduced pressure to give the title compound (420 mg).
$^1$H-NMR (DMSO-$D_6$) δ: 6.70 (1H, d, J = 7.2 Hz), 4.23 (2H, t, J = 6.4 Hz), 3.46 (2H, t, J = 6.2 Hz), 3.23-3.06 (2H, m), 3.15 (3H, s), 1.97-1.81 (4H, m), 1.80-1.69 (2H, m), 1.37 (9H, s), 1.23-1.05 (4H, m).

Step 6-9: tert-Butyl (trans-4-(3-(methylsulfonyl)propoxy)cyclohexyl)carbamate

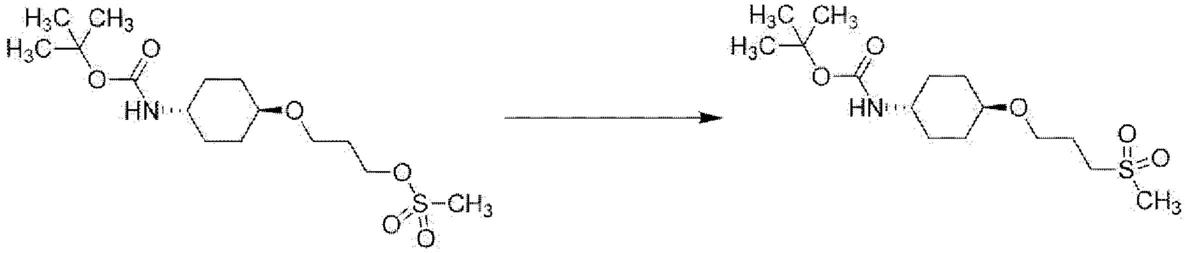

A suspension of 3-((trans-4-((tert-butyl-butoxycarbonyl)amino)cyclohexyl)oxy)propyl methanesulfonate (420 mg), sodium methanesulfinate (242 mg) and sodium iodide (186 mg) in N,N-dimethylformamide (1.987 mL) was stirred at 70°C for 1 hr, and at 100°C for 3 hr. To the reaction mixture was added water at room temperature, and the precipitated solid was collected by filtration, and dried under reduced pressure at 60°C to give the title compound (220 mg). Step 6-8 and step 6-9 were separately performed to give the title compound (47.2 mg).
$^1$H-NMR (DMSO-$D_6$) δ: 6.70 (1H, d, J = 7.5 Hz), 3.47 (2H, t, J = 6.4 Hz), 3.24-3.06 (4H, m), 2.97 (3H, s), 1.98-1.81 (4H, m), 1.81-1.67 (2H, m), 1.37 (9H, s), 1.23-1.07 (4H, m).

Step 6-10: Trans-4-(3-(methylsulfonyl)propoxy)cyclohexan-1-amine monohydrochloride

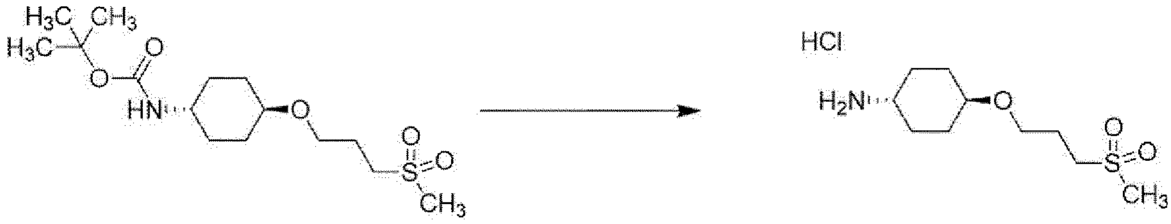

To a suspension of tert-butyl (trans-4-(3-(methylsulfonyl)propoxy)cyclohexyl)carbamate (253 mg) in ethyl acetate (1.265 mL) was added 4 M hydrogen chloride/ethyl acetate solution (1.265 mL) at room temperature, and the mixture was stirred for 2 hr and left standing overnight. The precipitated solid was collected by filtration, washed with ethyl acetate, and dried under reduced pressure at 60°C for 1 hr to give the title compound (202 mg).
$^1$H-NMR (DMSO-$D_6$) δ: 7.87 (3H, br s), 3.50 (2H, t, J = 6.2 Hz), 3.24-3.15 (1H, m), 3.14-3.07 (2H, m), 3.03-2.91 (1H, m), 2.97 (3H, s), 2.04-1.82 (6H, m), 1.40-1.27 (2H, m), 1.26-1.13 (2H, m).

Step 6-11: 2-(3-(Difluoromethyl)-1H-indazol-1-yl)-N-(trans-4-(3-(methylsulfonyl)propoxy)cyclohexyl)pyrimidine-5-carboxamide

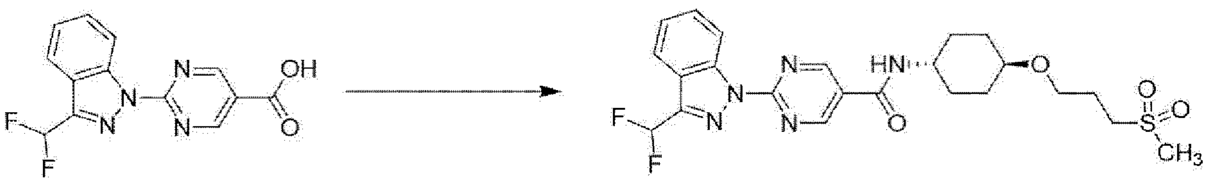

To a suspension of 2-(3-(difluoromethyl)-1H-indazol-1-yl)pyrimidine-5-carboxylic acid (30 mg), trans-4-(3-(methylsulfonyl)propoxy)cyclohexan-1-amine monohydrochloride (33.7 mg) and triethylamine (0.0261 mL) in N,N-dimethylformamide (0.5 mL) were added 1-hydroxy-7-azabenzotriazole (4.22 mg) and 1-ethyl-3-(3-dimethylaminopropyl)carbodiimide hydrochloride (29.7 mg) at room temperature, and the mixture was stirred overnight. To the reaction mixture was added water at room temperature, and the precipitated solid was collected by filtration and dried under reduced pressure. The solid was dissolved in dimethyl sulfoxide and purified by reversed-phase silica gel column chromatography (eluent: water/acetonitrile=95/5 to 0/100) to give the title compound (38.5 mg).

$^1$H-NMR (DMSO-$D_6$) δ: 9.30 (2H, s), 8.78 (1H, d, J = 8.6 Hz), 8.63 (1H, d, J = 7.5 Hz), 8.00 (1H, d, J = 8.1 Hz), 7.75 (1H, t, J = 8.1 Hz), 7.56 (1H, t, J = 53.9 Hz), 7.52 (1H, t, J = 7.5 Hz), 3.86-3.76 (1H, m), 3.53 (2H, t, J = 6.2 Hz), 3.30-3.25 (1H,

m), 3.16-3.11 (2H, m), 2.99 (3H, s), 2.07-2.02 (2H, m), 1.97-1.87 (4H, m), 1.46-1.36 (2H, m), 1.33-1.23 (2H, m). MS(M+H):508 MS(M-H):506

[Production Example 7] Example 7: Production of 2-(6-fluoro-3-methyl-1H-indazol-1-yl)-N-(3-(2-hydroxypropan-2-yl)bicyclo[1.1.1]pentan-1-yl)pyrimidine-5-carboxamide

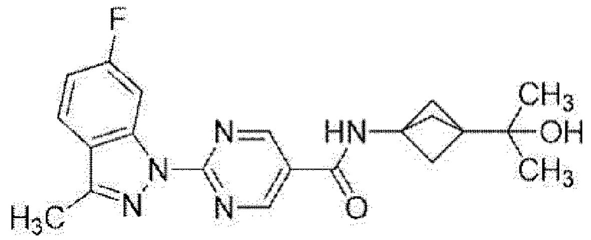

Step 7-1: Ethyl 2-(6-fluoro-3-methyl-1H-indazol-1-yl)pyrimidine-5-carboxylate

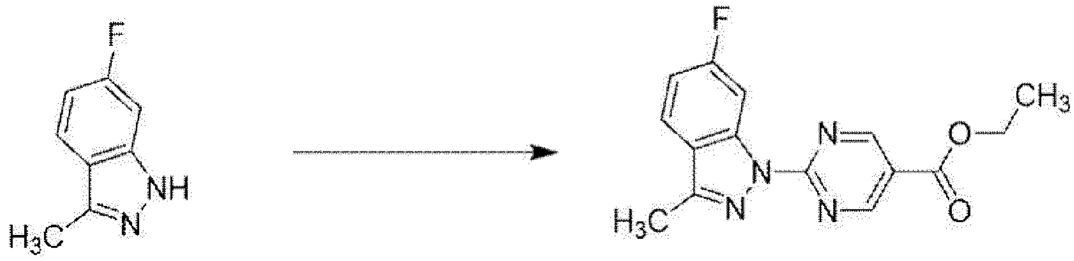

A suspension of 6-fluoro-3-methyl-1H-indazole (1.5 g), ethyl 2-chloropyrimidine-carboxylate (2.05 g) and cesium carbonate (6.51 g) in N,N-dimethylformamide (10 mL) was stirred for 3 hr and left standing overnight at room temperature . To the reaction mixture was added water at room temperature, and the precipitated solid was collected by filtration , and dried under reduced pressure. The solid was dissolved in chloroform, and purified by silica gel column chromatography (eluent: chloroform) to give the title compound (2.65 g).
$^1$H-NMR (DMSO-$D_6$) δ: 9.30 (2H, s), 8.44 (1H, dd, J = 10.4, 2.1 Hz), 7.95 (1H, dd, J = 8.8, 5.3 Hz), 7.31 (1H, td, J = 8.9, 2.2 Hz), 4.40 (2H, q, J = 7.1 Hz), 2.61 (3H, s), 1.37 (3H, t, J = 7.1 Hz).

Step 7-2: 2-(6-Fluoro-3-methyl-1H-indazol-1-yl)pyrimidine-5-carboxylic acid

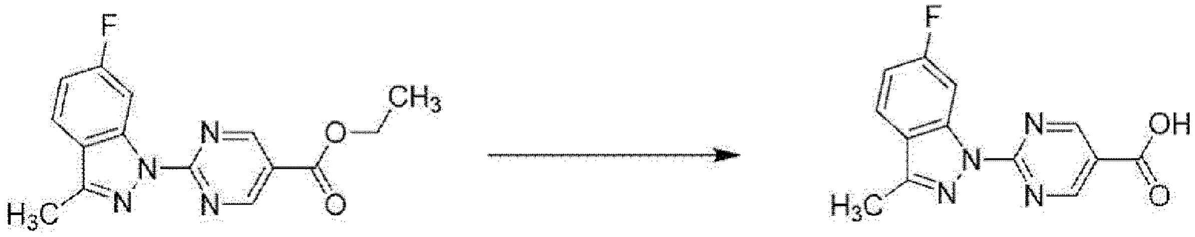

To a solution of ethyl 2-(6-fluoro-3-methyl-1H-indazol-1-yl)pyrimidine-5-carboxylate (2.65 g) in tetrahydrofuran (53 mL) was added 2 M aqueous sodium hydroxide solution (13.24 mL) at room temperature, and the mixture was stirred for 3 hr and left standing overnight. To the reaction mixture was added 2 M aqueous sodium hydroxide solution (4.41 mL) at room temperature, and the mixture was stirred for 1 hr. To the reaction mixture was added 2 M hydrochloric acid (17.65 mL) at room temperature. The mixture was stirred for 30 min and left standing overnight, and concentrated under reduced pressure. To the residue was added water, and the mixture was stirred for 30 min, and the precipitated solid was collected by filtration and dried under reduced pressure to give the title compound (2.38 g).

$^1$H-NMR (DMSO-$D_6$) δ: 13.68 (1H, br s), 9.27 (2H, s), 8.44 (1H, dd, J = 10.5, 2.2 Hz), 7.95 (1H, dd, J = 9.0, 5.2 Hz), 7.31 (1H, td, J = 8.6, 2.0 Hz), 2.61 (3H, s).

Step 7-3: tert-Butyl (3-(2-hydroxypropan-2-yl)bicyclo[1.1.1]pentan-1-yl)carbamate

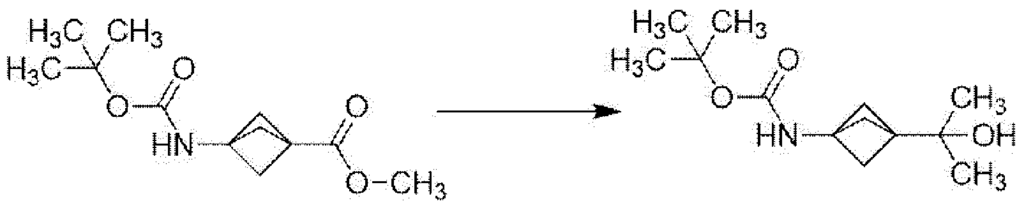

To a solution of methyl 3-((tert-butoxycarbonyl)amino)bicyclo[1.1.1]pentane-1-carboxylate (2.0 g) in tetrahydrofuran (10 mL) were added 1.02 M tetrahydrofuran solution (33 mL) of methylmagnesium bromide under ice-cooling, and the mixture was stirred at the same temperature for 1 hr, allowed to gradually return to room temperature, and stirred for 2 hr. To the reaction mixture was added saturated aqueous ammonium chloride solution (40 mL) under ice-cooling. To the reaction mixture was added ethyl acetate at room temperature (40 mL), and the layers were separated. The aqueous layer was extracted once with ethyl acetate (10 mL). The combined organic layer was washed with saturated brine and dried over sodium sulfate. Sodium sulfate was removed by filtration, and the filtrate was concentrated under reduced pressure to give the title compound (2.0 g).
$^1$H-NMR (DMSO-$D_6$) δ: 7.34 (1H, br s), 4.07 (1H, br s), 1.70 (6H, s), 1.37 (9H, s), 1.02 (6H, s).

Step 7-4: 2-(3-Aminobicyclo[1.1.1]pentan-1-yl)propan-2-ol monohydrochloride

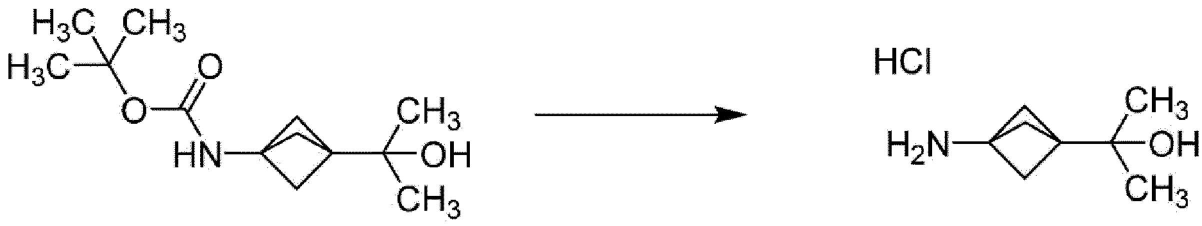

To a solution of tert-butyl (3-(2-hydroxypropan-2-yl)bicyclo[1.1.1]pentan-1-yl)carbamate (414 mg) in ethyl acetate (4.0 mL) was added 4 M hydrogen chloride/ethyl acetate solution (4.0 mL) at room temperature, and the mixture was stirred overnight. To the reaction mixture was added ethyl acetate at room temperature (10 mL), and the precipitated solid was collected by filtration and dried under reduced pressure to give the title compound (271 mg).
$^1$H-NMR (DMSO-$D_6$) δ: 8.79 (3H, br s), 4.21 (1H, br s), 1.80 (6H, s), 1.05 (6H, s).

Step 7-5: 2-(6-Fluoro-3-methyl-1H-indazol-1-yl)-N-(3-(2-hydroxypropan-2-yl)bicyclo[1.1.1]pentan-1-yl)pyrimidine-5-carboxamide

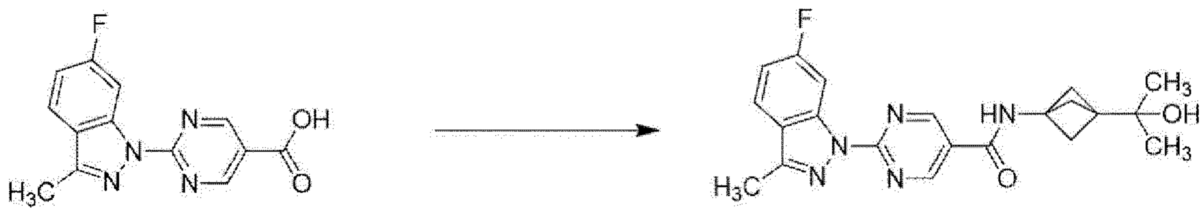

To a suspension of 2-(6-fluoro-3-methyl-1H-indazol-1-yl)pyrimidine-5-carboxylic acid (30 mg), 2-(3-aminobicyclo[1.1.1]pentan-1-yl)propan-2-ol monohydrochloride (28 mg), 1-hydroxy-7-azabenzotriazole (16 mg) and 1-ethyl-3-(3-dimethylaminopropyl)carbodiimide hydrochloride (36 mg) in N,N-dimethylformamide (1.0 mL) was added triethylamine (0.030 mL) at room temperature, and the mixture was stirred overnight. To the reaction mixture were added saturated aqueous sodium hydrogen carbonate (2.0 mL) and water (3.0 mL) at room temperature, and the precipitated solid was collected by filtration and dried under reduced pressure to give the title compound (42.7 mg).
$^1$H-NMR (DMSO-$D_6$) δ: 9.24 (1H, br s), 9.22 (2H, s), 8.39 (1H, dd, J = 10.3, 2.2 Hz), 7.94 (1H, dd, J = 8.8, 5.3 Hz), 7.32-7.26 (1H, m), 4.20 (1H, s), 2.61 (3H, s), 1.96 (6H, s), 1.09 (6H, s). MS(M+H):396 MS(M-H) :394

[Production Example 8] Example 8: Production of 2-(3-(difluoromethyl)-1H-indazol-1-yl)-N-(trans-4-(5-(methoxymethyl)-1,3,4-oxadiazol-2-yl)cyclohexyl)pyrimidine-5-carboxamide

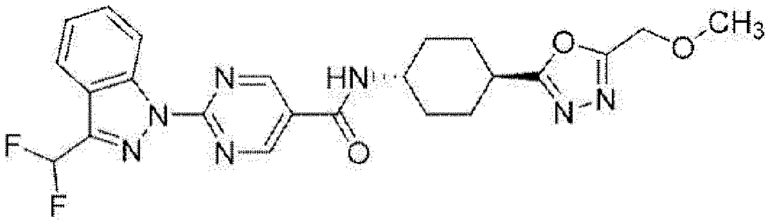

Step 8-1: 3-(Difluoromethyl)-1H-indazole

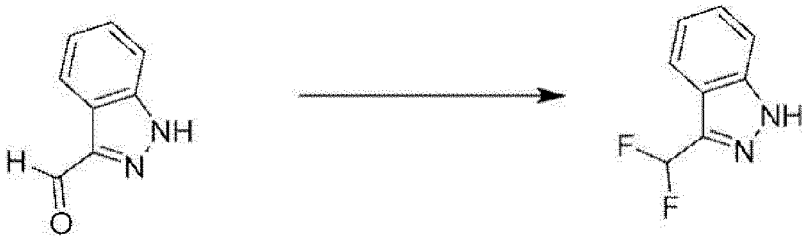

To a solution of 1H-indazole-3-carbaldehyde (1.5 g) in dichloromethane (10 mL) was added N,N-diethylaminosulfur trifluoride (2.71 mL) under ice-cooling, and the mixture was allowed to gradually return to room temperature and stirred for 5 hr. To the reaction mixture was added dropwise saturated aqueous sodium hydrogen carbonate under ice-cooling, and ethyl acetate was added. The layers were separated, and the aqueous layer was extracted once with ethyl acetate. The combined organic layer was washed with saturated brine and dried over magnesium sulfate. Magnesium sulfate was removed by filtration, and the filtrate was concentrated under reduced pressure. The residue was purified by silica gel column chromatography (eluent: n-hexane/ethyl acetate=95/5 to 80/20) and silica gel column chromatography (eluent: n-hexane/ethyl acetate=100/0 to 85/15) to give the title compound (343 mg). The title compound (673 mg) was obtained from the starting material, 1H-indazole-3-carboxaldehyde, by a similar production method.
$^1$H-NMR (DMSO-$D_6$) δ: 13.59 (1H, s), 7.84 (1H, d, J = 8.2 Hz), 7.64 (1H, d, J = 9.0 Hz), 7.46 (1H, t, J = 7.5 Hz), 7.34 (1H, t, J = 53.9 Hz), 7.25 (1H, t, J = 7.5 Hz).

Step 8-2: Ethyl 2-(3-(difluoromethyl)-1H-indazol-1-yl)pyrimidine-5-carboxylate

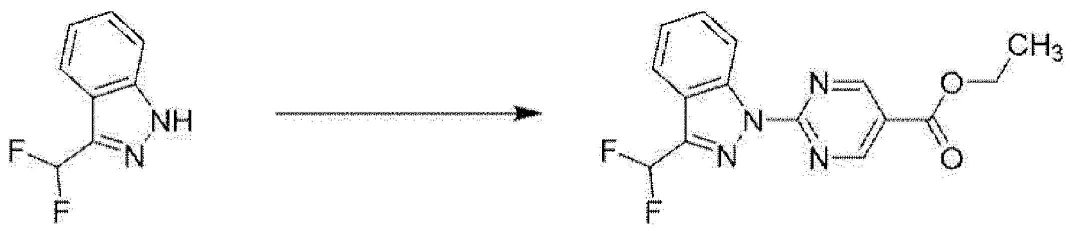

A suspension of 3-(difluoromethyl)-1H-indazole (0.95 g), ethyl 2-chloropyrimidine-carboxylate (1.1 g) and cesium carbonate (2.2 g) in N,N-dimethylformamide (10 mL) was stirred at room temperature for 4 hr. To the reaction mixture was added water at room temperature and the mixture was stirred for 1 hr. The precipitated solid was collected by filtration and dried under reduced pressure to give the title compound (1.73 g).
$^1$H-NMR (DMSO-$D_6$) δ: 9.38 (2H, s), 8.81 (1H, d, J = 8.6 Hz), 8.01 (1H, d, J = 8.1 Hz), 7.77 (1H, t, J = 8.4 Hz), 7.57 (1H, t, J = 54.0 Hz), 7.54 (1H, t, J = 7.6 Hz), 4.42 (2H, q, J = 7.1 Hz), 1.38 (3H, t, J = 7.1 Hz).

Step 8-3: 2-(3-(Difluoromethyl)-1H-indazol-1-yl)pyrimidine-5-carboxylic acid

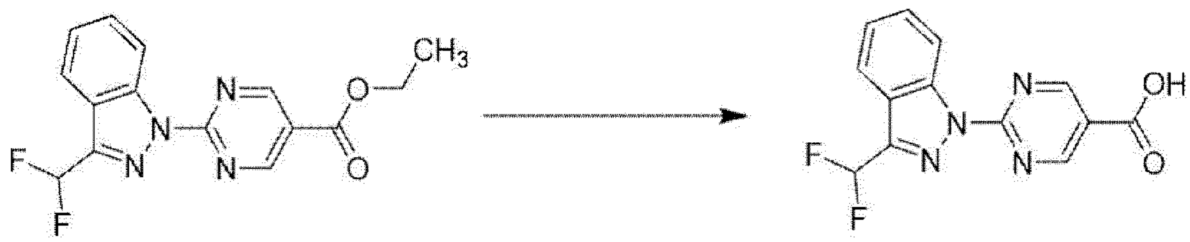

To a solution of ethyl 2-(3-(difluoromethyl)-1H-indazol-1-yl)pyrimidine-5-carboxylate (1.73 g) in tetrahydrofuran (10 mL) was added 2 M aqueous sodium hydroxide solution (5.03 mL) under ice-cooling, and the mixture was allowed to gradually return to room temperature and stirred for 6 hr. To the reaction mixture was added 2 M hydrochloric acid (5.0 mL) at room temperature, and the mixture was stirred for 1 hr. The precipitated solid was collected by filtration and dried under reduced pressure to give the title compound (1.45 g).
$^1$H-NMR (DMSO-$D_6$) δ: 13.80 (1H, br s), 9.35 (2H, s), 8.82 (1H, d, J = 8.5 Hz), 8.01 (1H, d, J = 7.9 Hz), 7.78-7.74 (1H, m), 7.56 (1H, t, J = 54.0 Hz), 7.56-7.51 (1H, m).

Step 8-4: tert-Butyl (trans-4-(2-(2-methoxyacetyl)hydrazine-1-carbonyl) cyclohexyl) carbamate

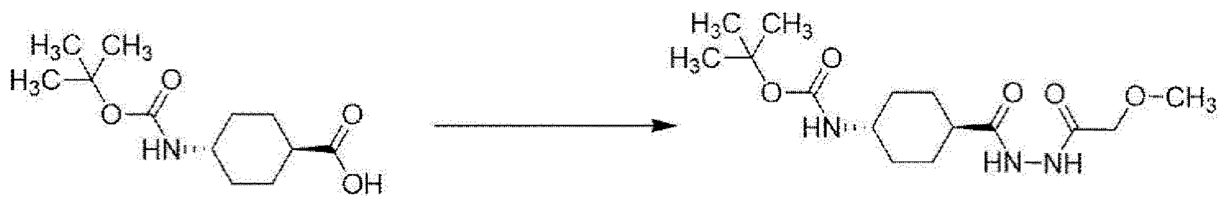

To trans-4-((tert-butoxycarbonyl)amino)cyclohexane-1-carboxylic acid (800 mg), methoxyacetic acid hydrazide (411 mg), 1-hydroxy-7-azabenzotriazole (134 mg) and 1-ethyl-3-(3-dimethylaminopropyl)carbodiimide hydrochloride (819 mg) were added N,N-dimethylformamide (10 mL) and triethylamine (732 mg) in a water bath, and the mixture was stirred for 10 min and stirred at room temperature overnight. To the reaction mixture was added water (20 mL) at room temperature, and the mixture was stirred for 1 hr. The precipitated solid was collected by filtration and dried under reduced pressure to give the title compound (650 mg).
$^1$H-NMR (DMSO-$D_6$) δ: 9.64 (2H, d, J = 7.5 Hz), 6.71 (1H, d, J = 7.5 Hz), 3.87 (2H, s), 3.31 (3H, s), 3.22-3.09 (1H, m), 2.14-2.01 (1H, m), 1.85-1.68 (4H, br m), 1.46-1.30 (11H, m), 1.21-1.09 (2H, m).

Step 8-5: tert-Butyl (trans-4-(5-(methoxymethyl)-1,3,4-oxadiazol-2-yl)cyclohexyl)carbamate

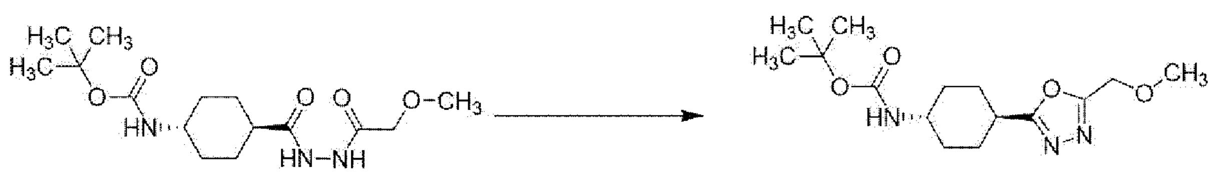

To a solution of tert-butyl (trans-4-(2-(2-methoxyacetyl)hydrazine -1-carbonyl)cyclohexyl)carbamate (650 mg), hexachloroethane (701 mg) and diisopropylethylamine (1.054 mL) in acetonitrile (12 mL) was added two divided portions of triphenylphosphine (725 mg) under ice-cooling, and the mixture was stirred for 15 min. The mixture was stirred at room temperature for 30 min and then at 70°C for 3 hr. To the reaction mixture were added hexachloroethane (350 mg), diisopropylethylamine (0.5 mL) and triphenylphosphine (370 mg) in a water bath, and the mixture was stirred at 80°C for 2 hr. To the reaction mixture were added toluene and water in a water bath, and the layers were separated. The organic layer was washed successively with 5 wt% aqueous potassium hydrogen sulfate solution and saturated brine, and concentrated under reduced pressure. To the residue was added ethyl acetate at room temperature, insoluble material was removed by filtration, and the residue was concentrated under reduced pressure. The residue was purified by silica gel column chromatography (eluent: n-hexane/ethyl acetate=34/66) to give crude product (480 mg) containing the title compound.

Step 8-6: Trans-4-(5-(methoxymethyl)-1,3,4-oxadiazol-2-yl)cyclohexan-1-amine

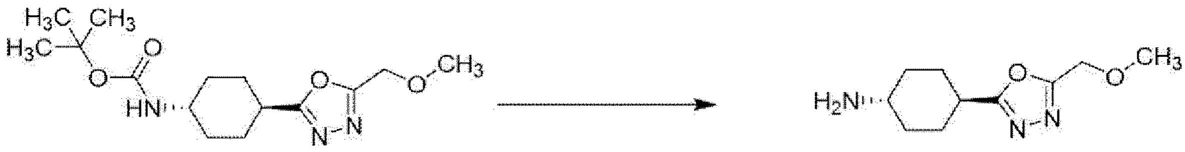

To a solution of a crude product (480 mg) containing tert-butyl (trans-4-(5-(methoxymethyl)-1,3,4-oxadiazol-2-yl) cyclohexyl)carbamate in ethyl acetate (1.0 mL) was added trifluoroacetic acid (1.5 mL) at room temperature, and the mixture was stirred for 3 hr. To the reaction mixture was added trifluoroacetic acid (5.0 mL) at room temperature, and the mixture was stirred at 40°C for 1 hr. The reaction mixture was concentrated under reduced pressure, and ethyl acetate and Wakogel (registered trade mark) $50NH_2$ (1.5 g) were added to the residue. The mixture was stirred for 30 min and filtered through celite, and the filtrate was concentrated under reduced pressure. The residue was purified by Bond Elut SCX (Agilent technologies) (eluent: methanol to 1 M ammonia/methanol solution) to give a crude product (190 mg) containing the title compound.

Step 8-7: 2-(3-(Difluoromethyl)-1H-indazol-1-yl)-N-(trans-4-(5-(methoxymethyl)-1,3,4-oxadiazol-2-yl)cyclohexyl)pyrimidine-5-carboxamide

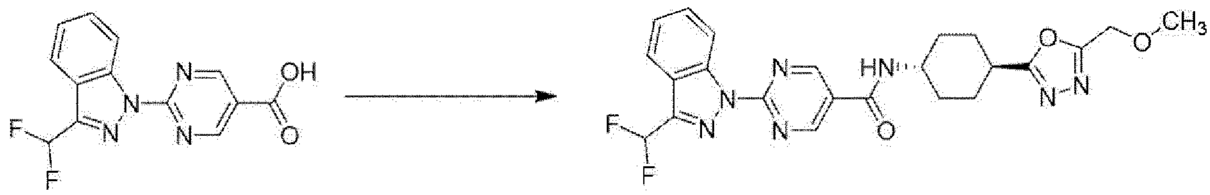

A suspension of a crude product (51 mg) containing trans-4-(5-(methoxymethyl)-1,3,4-oxadiazol-2-yl)cyclohexan-1-amine, 2-(3-(difluoromethyl)-1H-indazol-1-yl)pyrimidine-5-carboxylic acid (50 mg), triethylamine (52.3 mg), 1-hydroxy-7-azabenzotriazole (25.8 mg) and 1-ethyl-3-(3-dimethylaminopropyl)carbodiimide hydrochloride (49.5 mg) in N,N-dimethylformamide (1.2 mL) was stirred overnight at room temperature. To the reaction mixture was added water at room temperature, and the precipitated solid was collected by filtration, and dried under reduced pressure. To this solid was added n-hexane/ethyl acetate (n-hexane/ethyl acetate=1/1) solution, and slurry purification was performed. The solid was collected by filtration, and dried under reduced pressure. The solid was dissolved in methanol and purified by amino silica gel column chromatography (eluent: ethyl acetate/methanol=100/0 to 97/3) to give a crude product containing the title compound. To the crude product containing the title compound was added ethyl acetate, and the mixture was slurry purified. The solid was collected by filtration and dried under reduced pressure to give the title compound (22 mg).

$^1$H-NMR (DMSO-$D_6$) δ: 9.33 (2H, s), 8.79 (1H, d, J = 5.1 Hz), 8.74 (1H, d, J = 7.6 Hz), 8.01 (1H, d, J = 8.1 Hz), 7.78-7.74 (1H, m), 7.57 (1H, t, J = 53.0 Hz), 7.56-7.49 (1H, m), 4.63 (2H, s), 3.95-3.85 (1H, m), 3.35 (3H, s), 3.06-2.96 (1H, m), 2.24-2.22 (2H, br m), 2.18-2.16 (2H, br m), 1.75-1.46 (4H, m).
MS(M+H):484 MS(M-H) :482

[Production Example 9] Example 9: 2-(3-(difluoromethyl)-1H-pyrazolo[3,4-c]pyridin-1-yl)-N-(trans-4-(2-hydroxypropan-2-yl)cyclohexyl)pyrimidine-5-carboxamide

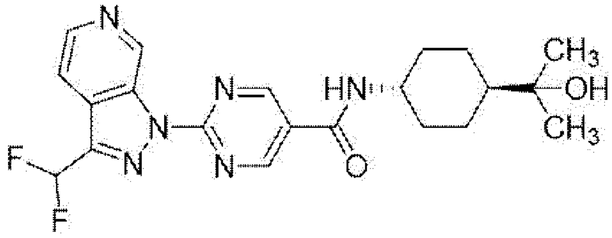

Step 9-1: 3-Iodo-1H-pyrazolo[3,4-c]pyridine

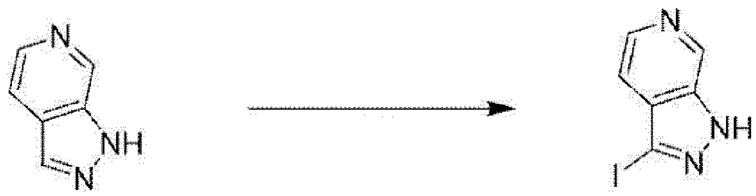

To a suspension of 1H-pyrazolo[3,4-c]pyridine (4.95 g) and potassium carbonate (17.23 g) in N,N-dimethylformamide (49.5 mL) was added iodine (10.55 g) under ice-cooling, and the mixture was stirred at room temperature for 3 hr. The reaction mixture was added to 10 wt% aqueous sodium thiosulfate solution (300 mL), and the mixture was stirred at room temperature for 1 hr. The precipitated solid was collected by filtration and dried under reduced pressure to give the title compound (8.66 g).
$^1$H-NMR (DMSO-$D_6$) δ: 14.06 (1H, s), 9.05 (1H, s), 8.30 (1H, d, J = 6.0 Hz), 7.45 (1H, d, J = 6.0 Hz).

Step 9-2: 3-Iodo-1-((2-(trimethylsilyl)ethoxy)methyl)-1H-pyrazolo[3,4-c]pyridine

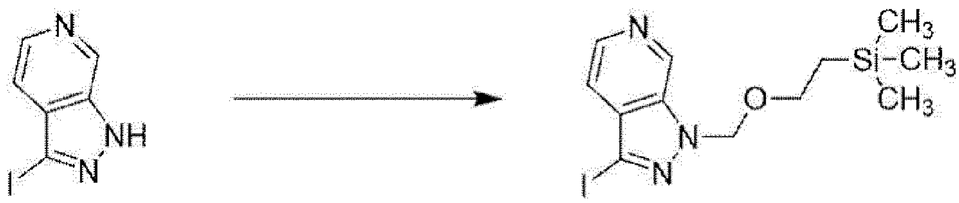

To a suspension of sodium hydride (0.612 g, 60 wt% oil dispersion) in N,N-dimethylformamide (10 mL) was added dropwise N,N-dimethylformamide (30 mL) solution of 3-iodo-1H-pyrazolo[3,4-c]pyridine (3.0 g) under ice-cooling, and the mixture was stirred at the same temperature for 1 hr. To the reaction mixture was added 2-(chloromethoxy)ethyltrimethylsilane (2.7 mL) under ice-cooling, and the mixture was stirred at room temperature for 2 hr. The reaction mixture and ethyl acetate were added to water (60 mL) at room temperature. The layers were separated, and the aqueous layer was extracted once with ethyl acetate. The combined organic layer was washed successively with water and saturated brine, and dried over sodium sulfate. Sodium sulfate was removed by filtration, and the filtrate was concentrated under reduced pressure. The residue was purified by silica gel column chromatography (eluent: n-hexane/ethyl acetate=90/10 to 50/50) to give the title compound (2.55 g).
$^1$H-NMR (DMSO-$D_6$) δ: 9.26 (1H, s), 8.38 (1H, d, J = 6.0 Hz), 7.49 (1H, d, J = 6.0 Hz), 5.88 (2H, s), 3.54 (2H, t, J = 7.9 Hz), 0.80 (2H, t, J = 7.9 Hz), -0.12 (9H, s).

Step 9-3: 1-((2-(Trimethylsilyl)ethoxy)methyl)-1H-pyrazolo[3,4-c]pyridine-3-carbaldehyde

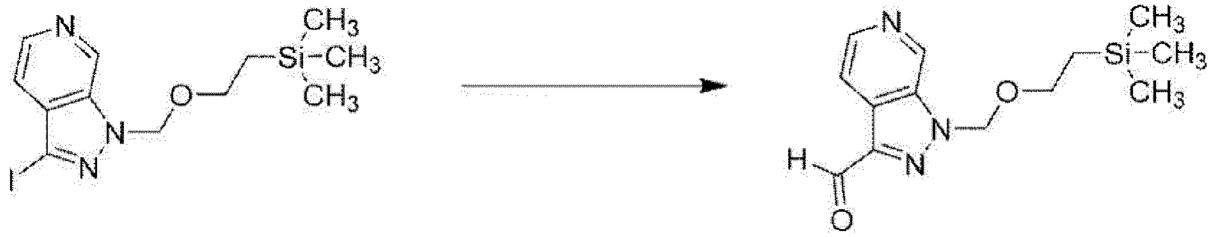

To a solution of 3-iodo-1-((2-(trimethylsilyl)ethoxy)methyl)-1H-pyrazolo[3,4-c]pyridine (1.1 g) in tetrahydrofuran (20 mL) was added dropwise 2.0 M tetrahydrofuran solution (2.198 mL) of isopropylmagnesium chloride under ice-salt bath cooling, and the mixture was stirred for 1 hr. At the same temperature, N,N-dimethylformamide (0.908 mL) was added and the mixture was stirred for 2 hr. To the reaction mixture was added saturated aqueous ammonium chloride solution at the same temperature. Ethyl acetate was added thereto at room temperature, and the layers were separated. The organic layer was washed successively with water and saturated brine, and dried over sodium sulfate. Sodium sulfate was removed by filtration, and the filtrate was concentrated under reduced pressure. The residue was purified by silica gel column chromatography (eluent: n-hexane/ethyl acetate=90/10 to 0/100) to give the title compound (0.26 g).
$^1$H-NMR (DMSO-$D_6$) δ: 10.42 (1H, s), 9.44 (1H, s), 8.42 (1H, d, J = 6.0 Hz), 8.05 (1H, d, J = 6.0 Hz), 6.22 (2H, s), 3.64 (2H, t, J = 7.9 Hz), 0.85 (2H, t, J = 7.9 Hz), -0.10 (9H, s).

Step 9-4: 3-(Difluoromethyl)-1-((2-(trimethylsilyl)ethoxy)methyl)-1H-pyrazolo[3,4-c]pyridine

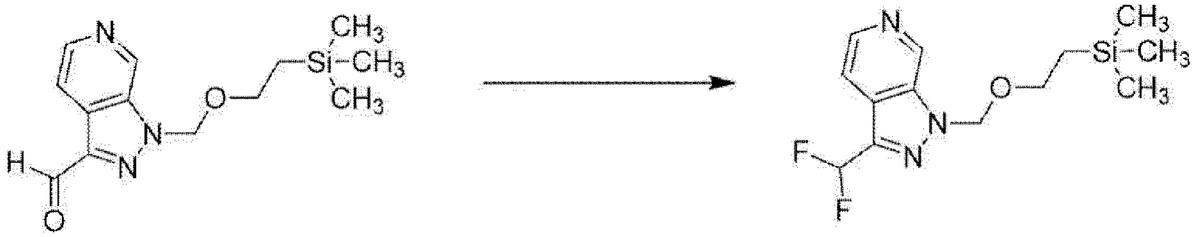

To a solution of 1-((2-(trimethylsilyl)ethoxy)methyl)-1H-pyrazolo[3,4-c]pyridine-3-carbaldehyde (260 mg) in dichloromethane (5.0 mL) was added dropwise Deoxo-Fluor (registered trade mark) (0.518 mL) under ice-cooling. The mixture was stirred at the same temperature for 1 hr, and at room temperature for 4 hr. To the reaction mixture was slowly added saturated aqueous sodium hydrogen carbonate under ice-cooling. To the reaction mixture was added ethyl acetate at room temperature, and the organic layer was washed successively with water and saturated brine. The organic layer was dried over sodium sulfate, sodium sulfate was removed by filtration, and the filtrate was concentrated under reduced pressure. The residue was purified by silica gel column chromatography (eluent: n-hexane/ethyl acetate=90/10 to 0/100) to give the title compound (260 mg).
$^1$H-NMR (DMSO-$D_6$) δ: 9.34 (1H, s), 8.25 (1H, d, J = 5.4 Hz), 7.75 (1H, d, J = 5.4 Hz), 7.75 (1H, t, J = 52.5 Hz), 5.99 (2H, s), 3.59 (2H, t, J = 7.9 Hz), 0.86 (2H, t, J = 7.9 Hz), -0.08 (9H, s).

Step 9-5: 3-(Difluoromethyl)-1H-pyrazolo[3,4-c]pyridine

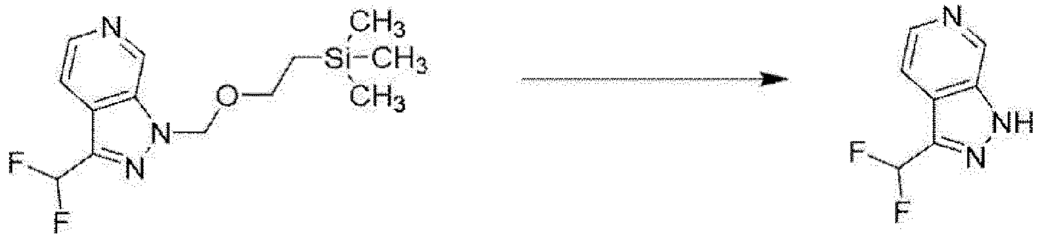

To a solution of 3-(difluoromethyl)-1-((2-(trimethylsilyl)ethoxy)methyl)-1H-pyrazolo[3,4-c]pyridine (260 mg) in dichloromethane (1.5 mL) was added trifluoroacetic acid (1.5 mL) at room temperature, and the mixture was stirred overnight. The reaction mixture was concentrated under reduced pressure, and chloroform and triethylamine (0.182 mL) was added to the residue. The mixture was purified by silica gel column chromatography {eluent: n-hexane/ethyl acetate (containing 5% 2 M ammonia/methanol solution)=90/10 to 0/100} to give a crude product (83 mg) containing the title compound.

Step 9-6: Ethyl 2-(3-(difluoromethyl)-1H-pyrazolo[3,4-c]pyridin-1-yl)pyrimidine-5-carboxylate

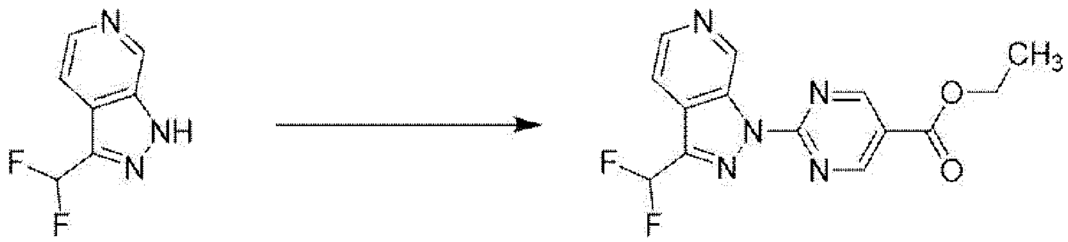

A suspension of a crude product (54 mg) containing 3-(difluoromethyl)-1H-pyrazolo[3,4-c]pyridine, ethyl 2-chloropyrimidine-carboxylate (59.6 mg) and cesium carbonate (156 mg) in N,N-dimethylformamide (0.6 mL) was stirred at room temperature for 1 hr. To the reaction mixture was added water at room temperature, and the precipitated solid was collected by filtration, washed with water, and dried under reduced pressure. The solid was purified by silica gel column chromatography (eluent: n-hexane/ethyl acetate=80/20 to 0/100) to give the title compound (70 mg).
$^1$H-NMR (DMSO-D$_6$) δ: 10.13 (1H, d, J = 1.2 Hz), 9.42 (2H, s), 8.63 (1H, d, J = 5.4 Hz), 8.00 (1H, d, J = 5.4 Hz), 7.63 (1H, t, J = 53.1 Hz), 4.42 (2H, q, J = 7.1 Hz), 1.37 (3H, t, J = 7.1 Hz).

Step 9-7: 2-(3-(Difluoromethyl)-1H-pyrazolo[3,4-c]pyridin-1-yl)pyrimidine-5-carboxylic acid

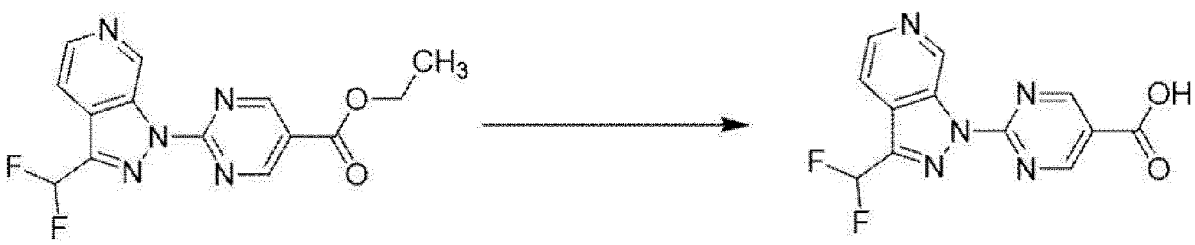

To a solution of ethyl 2-(3-(difluoromethyl)-1H-pyrazolo[3,4-c]pyridin-1-yl)pyrimidine-5-carboxylate (70 mg) in tetrahydrofuran (1.0 mL) was added 2 M aqueous sodium hydroxide solution (0.329 mL) at room temperature, and the mixture was stirred overnight. To the reaction mixture was added 1 M hydrochloric acid (0.658 mL) at room temperature, and the mixture was stirred for 1 hr. The precipitated solid was collected by filtration, washed with water, and dried under reduced pressure to give the title compound (33 mg).
$^1$H-NMR (DMSO-D$_6$) δ: 13.88 (1H, br s), 10.15 (1H, s), 9.41 (2H, s), 8.64 (1H, d, J = 5.4 Hz), 8.01 (1H, d, J = 5.4 Hz), 7.64 (1H, t, J = 53.1 Hz).

Step 9-8: 2-(3-(Difluoromethyl)-1H-pyrazolo[3,4-c]pyridin-1-yl)-N-(trans-4-(2-hydroxypropan-2-yl)cyclohexyl)pyrimidine-5-carboxamide

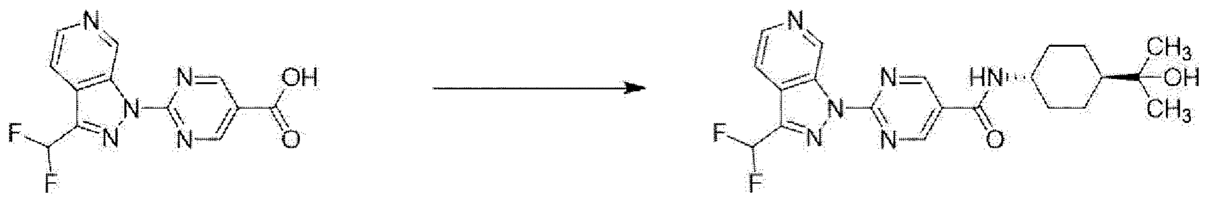

To a suspension of 2-(3-(difluoromethyl)-1H-pyrazolo[3,4-c]pyridin-1-yl)pyrimidine-5-carboxylic acid (33 mg) and 2-(trans-4-aminocyclohexyl)propan-2-ol (26.7 mg) in N,N-dimethylformamide (0.33 mL) were added triethylamine (0.0237 mL), 1-hydroxy-7-azabenzotriazole (15.42 mg) and 1-ethyl-3-(3-dimethylaminopropyl)carbodiimide hydrochloride (26.1 mg) at room temperature, and the mixture was stirred overnight. To the reaction mixture were added water (2.0 mL) and saturated aqueous sodium hydrogen carbonate (1.0 mL) at room temperature, and the mixture was stirred for 1 hr. The precipitated solid was collected by filtration, washed with water, and dried under reduced pressure to give the title compound (42 mg).

$^1$H-NMR (DMSO-$D_6$) δ: 10.12 (1H, s), 9.35 (2H, s), 8.67 (1H, d, J = 7.5 Hz), 8.62 (1H, d, J = 5.4 Hz), 8.00 (1H, d, J = 5.4 Hz), 7.64 (1H, t, J = 53.3 Hz), 4.06 (1H, s), 3.81-3.70 (1H, m), 1.99-1.85 (4H, m), 1.38-1.28 (2H, m), 1.22-1.11 (3H, m), 1.05 (6H, s).
MS(M+H) :431 MS(M-H):429

[Production Example 10] Example 10: 2-(3-(difluoromethyl)-6-fluoro-1H-indazol-1-yl)-N-(3-(2-hydroxypropan-2-yl)bicyclo[1.1.1]pentan-1-yl)pyrimidine-5-carboxamide

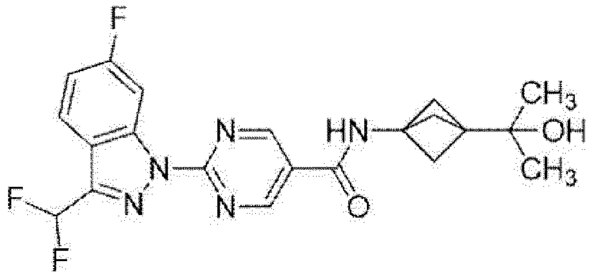

Step 10-1: 3-(Difluoromethyl)-6-fluoro-1H-indazole

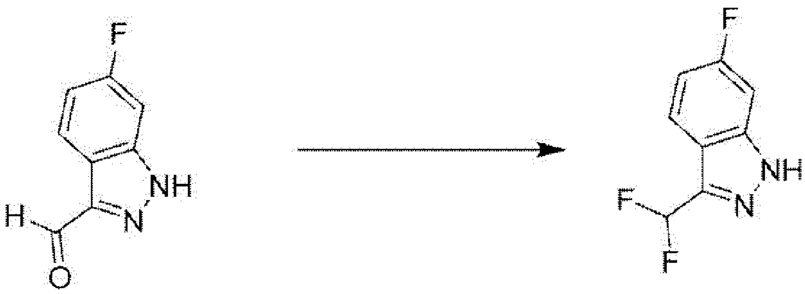

To a suspension of 6-fluoro-1H-indazole-3-carbaldehyde (500 mg) in dichloromethane (15 mL) was added N,N-diethylaminosulfur trifluoride (0.805 mL) under ice-cooling, and the mixture was allowed to gradually return to room temperature and stirred for 4 hr. To the reaction mixture were added saturated aqueous sodium hydrogen carbonate (10

mL) and water (5 mL) under ice-cooling. To the reaction mixture was added chloroform at room temperature, and the layers were separated. The organic layer was concentrated under reduced pressure. The residue was purified by silica gel column chromatography (eluent: n-hexane/ethyl acetate=92/8 to 34/66) to give a crude product (205 mg) containing the title compound.

Step 10-2: Ethyl 2-(3-(difluoromethyl)-6-fluoro-1H-indazol-1-yl)pyrimidine-5-carboxylate

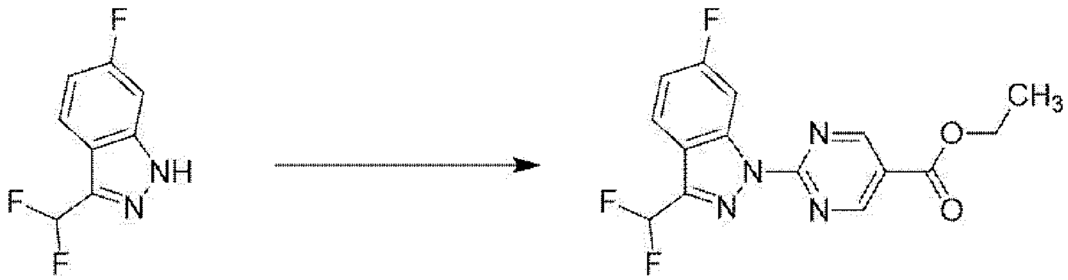

A suspension of a crude product (205 mg) containing 3-(difluoromethyl)-6-fluoro-1H-indazole, ethyl 2-chloropyrimidine-carboxylate (206 mg) and cesium carbonate (456 mg) in N,N-dimethylformamide (4.0 mL) was stirred at 60°C for 5 hr. To the reaction mixture was added water (25 mL) at room temperature, and the precipitated solid was collected by filtration, and dried under reduced pressure. The solid was dissolved in chloroform and purified twice by silica gel column chromatography (eluent: n-hexane/ethyl acetate=92/8 to 34/66) to give the title compound (275 mg).
$^1$H-NMR (DMSO-$D_6$) δ: 9.39 (2H, s), 8.57 (1H, dd, J = 10.4, 2.3 Hz), 8.05 (1H, dd, J = 8.8, 5.3 Hz), 7.57 (1H, t, J = 53.4 Hz), 7.48-7.42 (1H, m), 4.42 (2H, q, J = 7.2 Hz), 1.38 (3H, t, J = 7.2 Hz).

Step 10-3: 2-(3-(Difluoromethyl)-6-fluoro-1H-indazol-1-yl)pyrimidine-5-carboxylic acid

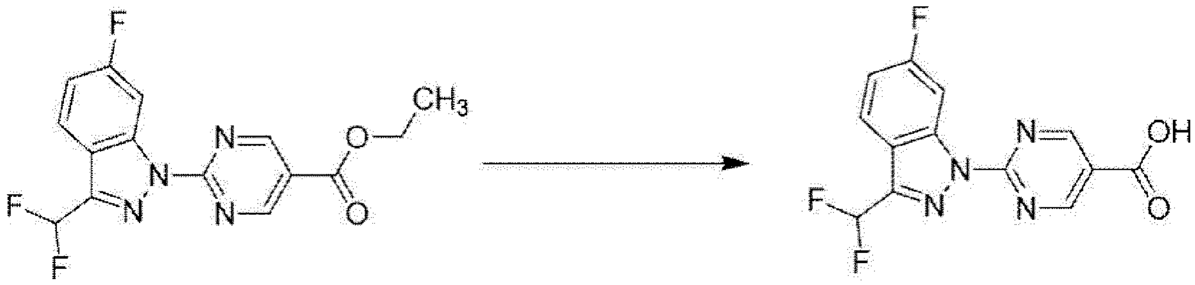

To a suspension of ethyl 2-(3-(difluoromethyl)-6-fluoro-1H-indazol-1-yl)pyrimidine-5-carboxylate (275 mg) in tetrahydrofuran (10 mL) was added 2 M aqueous sodium hydroxide solution (0.85 mL) at room temperature, and the mixture was stirred for 8 hr. To the reaction mixture were added 1 M hydrochloric acid (1.7 mL) and water (15 mL) at room temperature, and the mixture was left standing overnight. The precipitated solid was collected by filtration and dried under reduced pressure to give the title compound (210 mg).
$^1$H-NMR (DMSO-$D_6$) δ: 13.84 (1H, br s), 9.36 (2H, s), 8.56 (1H, dd, J = 10.2, 2.3 Hz), 8.04 (1H, dd, J = 8.8, 5.3 Hz), 7.56 (1H, t, J = 53.2 Hz), 7.47-7.42 (1H, m).

Step 10-4: tert-Butyl (3-(2-hydroxypropan-2-yl)bicyclo[1.1.1]pentan-1-yl)carbamate

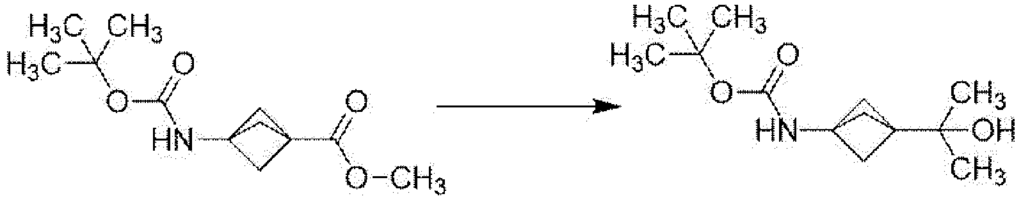

To a solution of methyl 3-((tert-butoxycarbonyl)amino)bicyclo[1.1.1]pentane-1-carboxylate (2.0 g) in tetrahydrofuran (10 mL) was added 1.02 M tetrahydrofuran solution (33 mL) of methylmagnesium bromide under ice-cooling. The mixture was stirred at the same temperature for 1 hr, allowed to gradually return to room temperature, and stirred for 2 hr. To the reaction mixture was added saturated aqueous ammonium chloride solution (40 mL) under ice-cooling. To the reaction mixture was added ethyl acetate (40 mL) at room temperature, the layers were separated, and the aqueous layer was extracted once with ethyl acetate (10 mL). The combined organic layer was washed with saturated brine, and dried over sodium sulfate. Sodium sulfate was removed by filtration, and the filtrate was concentrated under reduced pressure to give the title compound (2.0 g).
$^1$H-NMR (DMSO-D$_6$) δ: 7.34 (1H, br s), 4.07 (1H, br s), 1.70 (6H, s), 1.37 (9H, s), 1.02 (6H, s).

Step 10-5: 2-(3-Aminobicyclo[1.1.1]pentan-1-yl)propan-2-ol monohydrochloride

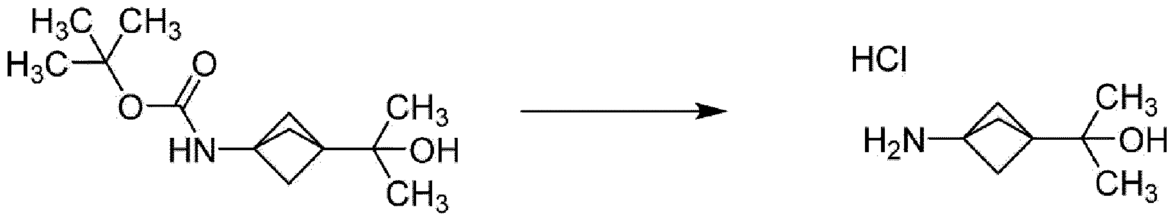

To a solution of tert-butyl (3-(2-hydroxypropan-2-yl)bicyclo[1.1.1]pentan-1-yl)carbamate (414 mg) in ethyl acetate (4.0 mL) was added 4 M hydrogen chloride/ethyl acetate solution (4.0 mL) at room temperature, and the mixture was stirred overnight. To the reaction mixture was added ethyl acetate (10 mL) at room temperature, and the precipitated solid was collected by filtration and dried under reduced pressure to give the title compound (271 mg).
$^1$H-NMR (DMSO-D$_6$) δ: 8.79 (3H, br s), 4.21 (1H, br s), 1.80 (6H, s), 1.05 (6H, s).

Step 10-6: 2-(3-(Difluoromethyl)-6-fluoro-1H-indazol-1-yl)-N-(3-(2-hydroxypropan-2-yl)bicyclo[1.1.1]pentan-1-yl)pyrimidine-5-carboxamide

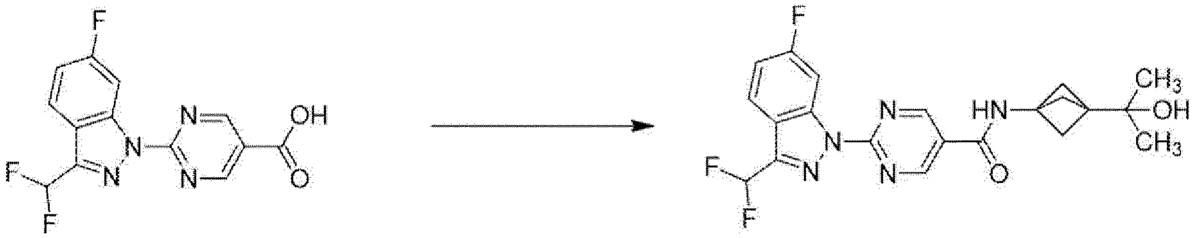

To a suspension of 2-(3-(difluoromethyl)-6-fluoro-1H-indazol-1-yl)pyrimidine-5-carboxylic acid (62 mg), 2-(3-aminobicyclo[1.1.1]pentan-1-yl)propan-2-ol monohydrochloride (48 mg), 1-hydroxy-7-azabenzotriazole (31 mg) and 1-ethyl-3-(3-dimethylaminopropyl)carbodiimide hydrochloride (49 mg) in N,N-dimethylformamide (1.0 mL) was added triethylamine (0.050 mL) at room temperature, and the mixture was stirred overnight. To the reaction mixture were added saturated aqueous sodium hydrogen carbonate (1.0 mL) and water (12 mL) at room temperature, and the precipitated solid was collected by filtration and dried under reduced pressure to give the title compound (74.5 mg).

$^1$H-NMR (DMSO-D$_6$) δ: 9.33 (1H, br s), 9.30 (2H, s), 8.52 (1H, dd, J = 10.3, 2.2 Hz), 8.04 (1H, dd, J = 8.8, 5.3 Hz), 7.56 (1H, t, J = 53.2 Hz), 7.46-7.41 (1H, m), 4.21 (1H, s), 1.96 (6H, s), 1.09 (6H, s).

MS(M+H) :432 MS(M-H):430

[Production Example 11] Example 229: Production of N-(trans-4-(1-hydroxy-2-methylpropoxy)cyclohexyl)-2-(3-methyl-1H-indazol-1-yl)pyrimidine-5-carboxamide

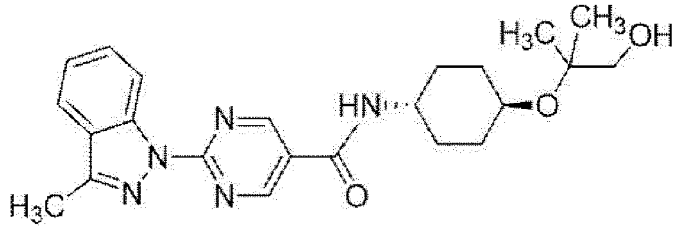

Step 11-1: Ethyl 2-{[trans-4-(dibenzylamino)cyclohexyl]oxy}-propionate

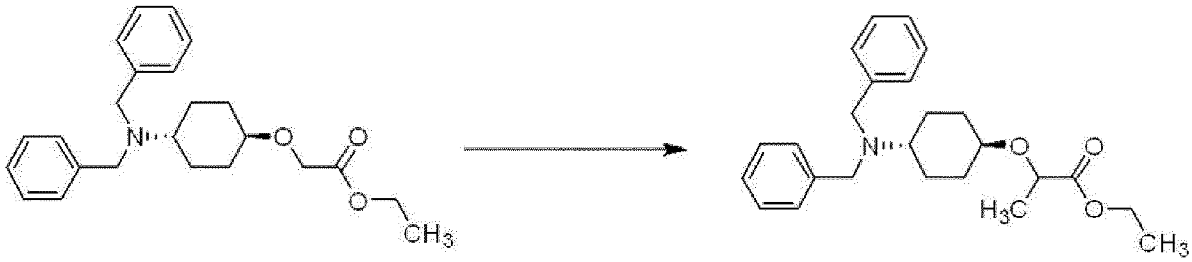

To 1.13 M n-hexane solution (7.81 mL) of lithium bis(trimethylsilyl)amide was added dropwise at -78°C tetrahydrofuran (15.3 mL) solution of 2-{[trans-4-(dibenzylamino)cyclohexyl]oxy}ethyl acetate (1.53 g) synthesized in the same manner as in Production Example 4, step 4-4, and the mixture was stirred at the same temperature for 20 min. To the reaction mixture was added dropwise methyl iodide (1.003 mL) at the same temperature. The mixture was stirred for 1 hr, allowed to gradually return to room temperature, and left standing overnight. To the reaction mixture were added water and ethyl acetate. The layers were separated, and the aqueous layer was extracted once with ethyl acetate. The combined organic layer was concentrated under reduced pressure. The residue was purified by silica gel chromatography (eluent: n-hexane/ethyl acetate=98/2 to 80/20) to give the title compound (422 mg).
$^1$H-NMR (DMSO-$D_6$) δ: 7.33-7.26 (8H, m), 7.23-7.16 (2H, m), 4.13-4.00 (3H, m), 3.55 (4H, s), 3.25-3.15 (1H, m), 2.44-2.31 (1H, m), 2.07-1.88 (2H, m), 1.85-1.73 (2H, m), 1.46-1.30 (2H, m), 1.25-1.13 (6H, m), 1.10-0.88 (2H, m).

Step 11-2: Ethyl 2-{[trans-4-(dibenzylamino)cyclohexyl]oxy}-2-methylpropionate

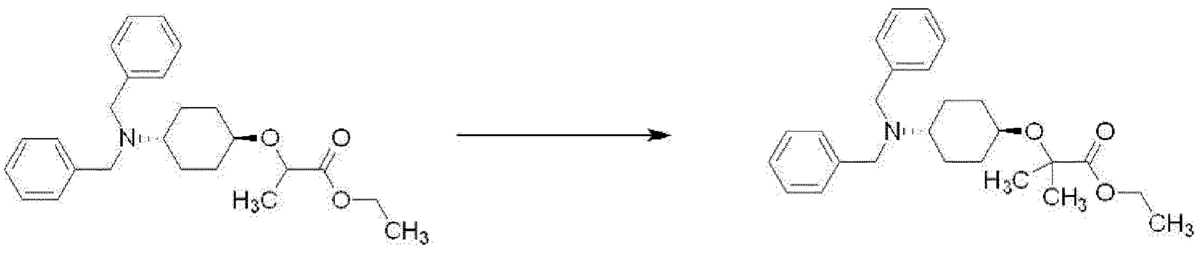

To 2.0 M tetrahydrofuran/n-heptane/ethylbenzene solution (1.174 mL) of lithium diisopropylamide was added dropwise at - 78°C tetrahydrofuran (4.22 mL) solution of ethyl 2-{[trans-4-(dibenzylamino)cyclohexyl]oxy}propionate (422 mg), and the mixture was stirred at the same temperature for 30 min. To the reaction mixture was added dropwise methyl iodide (0.267 mL) at the same temperature, hexamethylphosphoric triamide (0.204 mL) was added, and the mixture was stirred for 40 min. The reaction mixture was allowed to gradually return to room temperature, stirred for 1 hr, and left

standing overnight. To the reaction mixture were added water and ethyl acetate. The layers were separated, and the aqueous layer was extracted once with ethyl acetate. The combined organic layer was concentrated under reduced pressure. The residue was purified by silica gel chromatography (eluent: n-hexane/ethyl acetate=98/2 to 78/22) to give the title compound (388 mg).
$^1$H-NMR (DMSO-$D_6$) δ: 7.36-7.24 (8H, m), 7.23-7.16 (2H, m), 4.09 (2H, q, J = 7.2 Hz), 3.55 (4H, s), 3.27-3.20 (1H, m), 2.37-2.30 (1H, m), 1.95-1.84 (2H, m), 1.81-1.71 (2H, m), 1.50-1.35 (2H, m), 1.29 (6H, s), 1.19 (3H, t, J = 7.0 Hz), 1.11-0.97 (2H, m).

Step 11-3: 2-{[Trans-4-(dibenzylamino)cyclohexyl]oxy}-2-methylpropan-1-ol

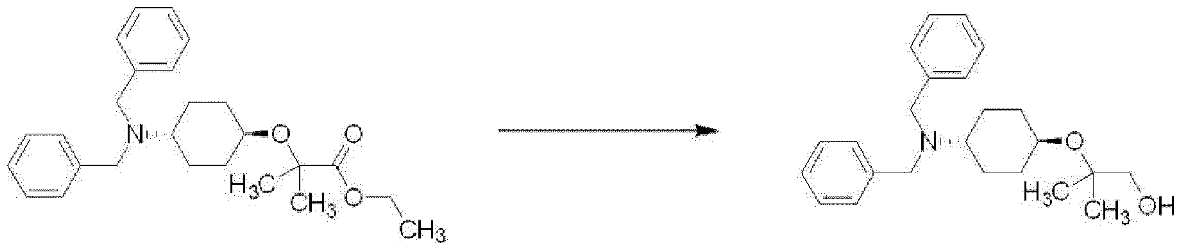

To a suspension of lithium aluminum hydride (76 mg) in tetrahydrofuran (3.88 mL) was added dropwise tetrahydrofuran (1.94 mL) solution of ethyl 2-{[trans-4-(dibenzylamino)cyclohexyl]oxy}-2-methylpropionate (388 mg) under ice-cooling. The mixture was allowed to gradually return to room temperature, stirred for 1 hr, and left standing overnight. To the reaction mixture were successively added water (0.076 mL), 4 M aqueous sodium hydroxide solution (0.076 mL) and water (0.228 mL) under ice-cooling. The mixture was filtered through celite, and the filtrate was concentrated under reduced pressure to give the title compound (329 mg).
$^1$H-NMR (DMSO-$D_6$) δ: 7.36-7.25 (8H, m), 7.22-7.16 (2H, m), 4.45 (1H, t, J = 5.5 Hz), 3.55 (4H, s), 3.49-3.34 (1H, m), 3.16 (2H, d, J = 5.5 Hz), 2.39-2.30 (1H, m), 1.86-1.71 (4H, m), 1.51-1.39 (2H, m), 1.12-0.93 (8H, m).

Step 11-4: 2-((Trans-4-aminocyclohexyl)oxy)-2-methylpropan-1-ol

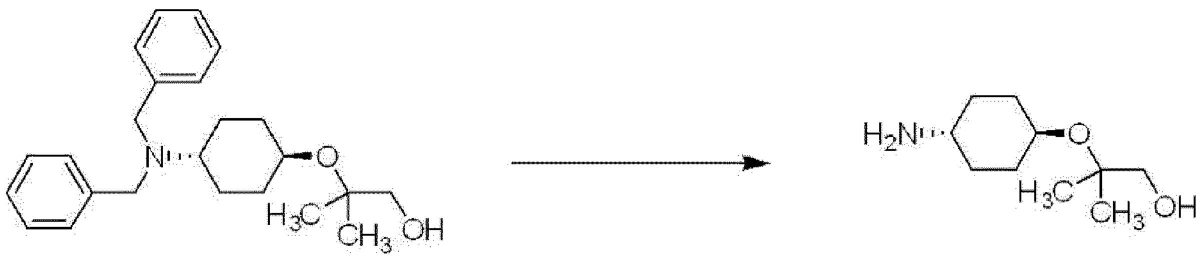

To a solution of 2-{[trans-4-(dibenzylamino)cyclohexyl]oxy}-2-methylpropan-1-ol (150 mg) in tetrahydrofuran/-methanol (1.5 mL, tetrahydrofuran/methanol=1/1) was added 20% palladium hydroxide-carbon (15 mg) at room temperature. Under 1 atm hydrogen, the mixture was stirred at room temperature overnight. The reaction mixture was filtered through celite, and the filtrate was concentrated under reduced pressure to give the title compound (68.8 mg).
$^1$H-NMR (DMSO-$D_6$) δ: 4.46 (1H, t, J = 5.1 Hz), 3.48-3.35 (2H, m), 3.17 (2H, d, J = 5.1 Hz), 2.25-1.91 (2H, m), 1.77-1.63 (4H, m), 1.25-0.96 (10H, m).

Step 11-5: N-(Trans-4-(1-hydroxy-2-methylpropoxy)cyclohexyl)-2-(3-methyl-1H-indazol-1-yl)pyrimidine-5-carboxamide

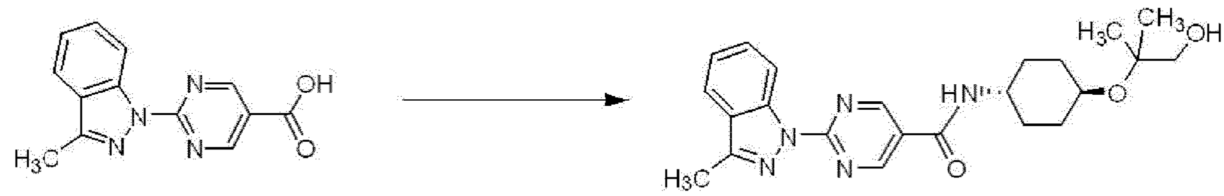

A suspension of 2-(3-methyl-1H-indazol-1-yl)pyrimidine-5-carboxylic acid (30 mg) synthesized in the same manner as in Production Example 4, step 4-2, 2-((trans-4-aminocyclohexyl)oxy)-2-methylpropan-1-ol (24.31 mg), 1-hydroxy-7-azabenzotriazole (4.82 mg) and 1-ethyl-3-(3-dimethylaminopropyl)carbodiimide hydrochloride (33.9 mg) in N,N-dimethylformamide (0.6 mL) was stirred for 3 hr. To the reaction mixture was added water (3.0 mL) at room temperature, and the precipitated solid was collected by filtration, washed with water, and dried under reduced pressure at 70°C to give a crude product (41.6 mg) containing the title compound. The crude product was purified by reversed-phase silica gel chromatography (eluent: water/acetonitrile=95/5 to 0/100) to give the title compound (23.9 mg).
$^1$H-NMR (DMSO-$D_6$) δ: 9.21 (2H, s), 8.67 (1H, d, J = 8.3 Hz), 8.51 (1H, d, J = 7.4 Hz), 7.89 (1H, d, J = 7.6 Hz), 7.63 (1H, dd, J = 8.3, 7.5 Hz), 7.40 (1H, dd, J = 7.6, 7.5 Hz), 4.53 (1H, br s), 3.82-3.67 (1H, m), 3.58-3.45 (1H, m), 3.22 (2H, s), 2.62 (3H, s), 1.97-1.76 (4H, m), 1.51-1.22 (4H, m), 1.09 (6H, s). MS (M+H) :424 MS (M-H) :422

[Production Example 12] Example 241: Production of 3-(trans-4-(2-(6-fluoro3-methyl-1H-indazol-1-yl)pyrimidine-5-carboxyamide)cyclohexyl)-3-methylbutyric acid

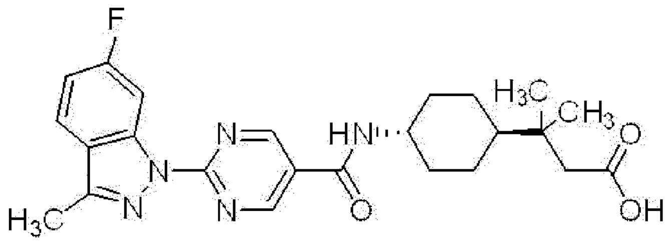

Step 12-1: tert-Butyl {trans-4-[methoxy(methyl)carbamoyl]cyclohexyl}carbamate

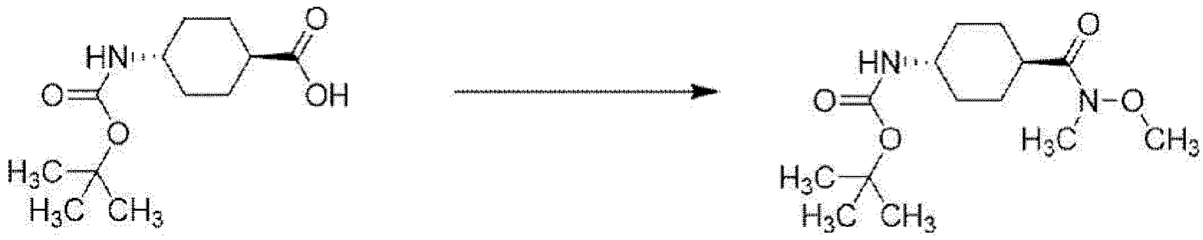

Under a nitrogen atmosphere, to a solution of trans-4-[(tert-butoxycarbonyl)amino]cyclohexanecarboxylic acid (25.0 g), N,O-dimethylhydroxylamine hydrochloride (13.1 g) and N,N-diisopropylethylamine (23.3 mL) in N,N-dimethylformamide (250 mL) were successively added portions of 1-hydroxy-1H-benzotriazole monohydrate (20.5 g) and 1-ethyl-3-(3-dimethylaminopropyl)carbodiimide hydrochloride (25.7 g) under water cooling, and the mixture was stirred for 7 days. To the reaction mixture was added dropwise saturated aqueous sodium hydrogen carbonate/water (250 mL, saturated aqueous sodium hydrogen carbonate/water =1/2) at room temperature, and the mixture was stirred. The precipitated solid was collected by filtration, washed with water (300 mL), and dried under reduced pressure to give the title compound (24.45 g).
$^1$H-NMR ($CDCl_3$) δ: 4.40-4.33 (1H, m), 3.69 (3H, s), 3.48-3.39 (1H, m), 3.17 (3H, s), 2.64-2.57 (1H, m), 2.11-2.07 (2H, m), 1.86-1.81 (2H, m), 1.67-1.57 (2H, m), 1.44 (9H, s), 1.14 (2H, ddd, J = 24.9, 12.7, 3.4 Hz).

Step 12-2: tert-Butyl (trans-4-acetylcyclohexyl)carbamate

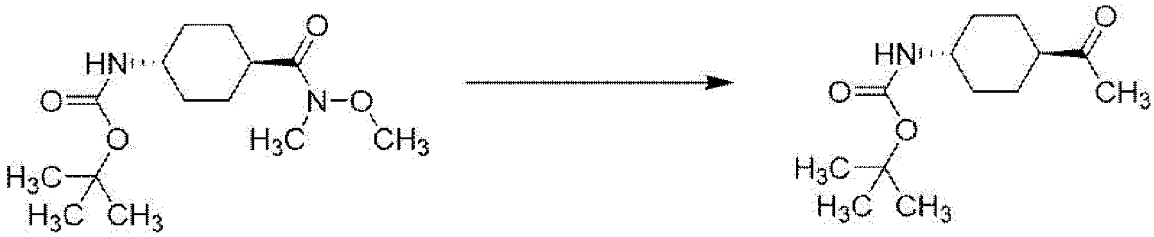

Under an argon atmosphere, to a solution of tert-butyl {trans-4-[methoxy(methyl)carbamoyl]cyclohexyl}carbamate (3.0 g) in tetrahydrofuran (20 mL) was added 1.02 M tetrahydrofuran solution (22.6 mL) of methylmagnesium bromide under ice-cooling, and the mixture was stirred for 3 hr. To the reaction mixture were added saturated aqueous ammonium chloride solution (20 mL), water (10 mL) and ethyl acetate (30 mL) under ice-cooling, and the layers were separated. The aqueous layer was extracted once with ethyl acetate (20 mL). The combined organic layer was washed successively with water and saturated brine, and dried over sodium sulfate. Sodium sulfate was removed by filtration, and the filtrate was concentrated under reduced pressure to give the title compound (2.464 g).
$^1$H-NMR (DMSO-$D_6$) δ: 6.72 (1H, d, J = 7.9 Hz), 3.17-3.07 (1H, m), 2.27-2.20 (1H, m), 2.08 (3H, s), 1.87-1.76 (4H, m), 1.37 (9H, s), 1.26-1.09 (4H, m).

Step 12-3: tert-Butyl (trans-4-(1-(2,2-dimethyl-4,6-dioxo-1,3-dioxan-5-ylidene) ethyl) cyclohexyl) carbamate

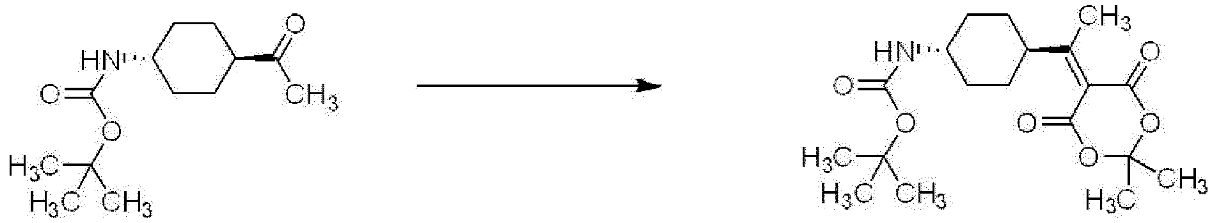

A 1.0 M dichloromethane solution (31 mL) of titanium tetrachloride (IV) was added to tetrahydrofuran (20 mL) under ice-cooling. To the reaction mixture were added tetrahydrofuran (28 mL) solution of tert-butyl (trans-4-acetylcyclohexyl)carbamate (2.464 g) and 2,2-dimethyl-1,3-dioxane-4,6-dione (1.619 g), and pyridine (4.54 mL) under ice-cooling. The mixture was allowed to gradually return to room temperature and stirred overnight. To the reaction mixture were added saturated aqueous ammonium chloride solution (30 mL), water (15 mL) and ethyl acetate (60 mL) at room temperature. After partitioning, the organic layer was washed successively with water and saturated brine, and concentrated under reduced pressure. To the residue were added ethyl acetate (3.0 mL), n-hexane (30 mL) and water (10 mL) and the mixture was slurry stirred for 1 hr. The solid was collected by filtration and dried under reduced pressure to give the title compound (2.254 g) .
$^1$H-NMR (DMSO-$D_6$) δ: 6.75 (1H, d, J = 8.1 Hz), 3.42-3.35 (1H, m), 3.32-3.22 (1H, m), 2.29 (3H, s), 1.85-1.81 (2H, m), 1.67 (6H, s), 1.60-1.49 (4H, m), 1.38 (9H, s), 1.24-1.14 (2H, m).

Step 12-4: tert-Butyl (trans-4-(2-(2,2-dimethyl-4,6-dioxo-1,3-dioxan-5-yl)propan-2-yl)cyclohexyl)carbamate

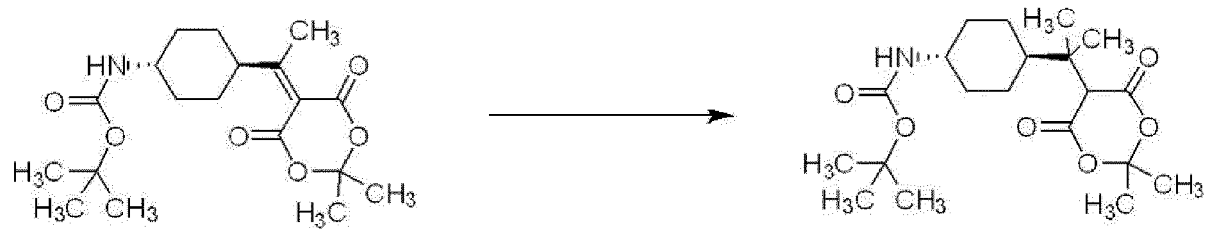

To a solution of copper(I) bromide (2.64 g) in tetrahydrofuran (20 mL) was added 1.0 M tetrahydrofuran solution (37 mL) of methylmagnesium bromide under ice-cooling. To the reaction mixture was added tetrahydrofuran (40 mL) solution of tert-butyl (trans-4-(1-(2,2-dimethyl-4,6-dioxo-1,3-dioxan-5-ylidene)ethyl)cyclohexyl)carbamate (2.254 g) under ice-cooling. The mixture was stirred for 90 min, allowed to gradually return to room temperature, and stirred for 90 min. To the reaction mixture were added saturated aqueous ammonium chloride solution (60 mL) and ethyl acetate (80 mL) at room temperature. After partitioning, the organic layer was washed with saturated brine, and concentrated under reduced pressure. The residue was purified by silica gel column chromatography (eluent: n-hexane/ethyl acetate=92/8 to 34/66) to give the title compound (217 mg).
$^1$H-NMR (DMSO-$D_6$) δ: 6.66 (1H, d, J = 7.9 Hz), 3.81 (1H, s), 3.12 (1H, m), 1.82-1.77 (2H, m), 1.73 (3H, s), 1.69-1.60 (3H, m), 1.65 (3H, s), 1.37 (9H, s), 1.13-1.02 (4H, m), 1.02 (6H, s).

Step 12-5: Methyl 3-(trans-4-((tert-butoxycarbonyl)amino)cyclohexyl)-3-methylbutyrate

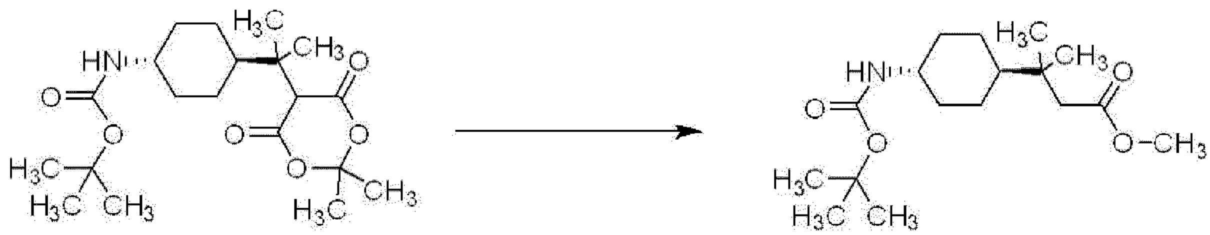

A solution of tert-butyl (trans-4-(2-(2,2-dimethyl-4,6-dioxo-1,3-dioxan-5-yl)propan-2-yl)cyclohexyl)carbamate (205 mg) in methanol (6.0 mL) was stirred at 80°C for 8 hr and left standing overnight. The reaction mixture was concentrated under reduced pressure. To the residue were added dimethyl sulfoxide (2.0 mL), water (0.2 mL) and sodium chloride (70 mg), and the mixture was stirred at 140°C for 7 hr. To the reaction mixture were added water (15 mL) and ethyl acetate (15 mL) at room temperature, and the layers were separated. The aqueous layer was extracted once with ethyl acetate (5.0 mL). The combined organic layer was washed successively with water, and saturated brine, and the residue was concentrated under reduced pressure to give a crude product (179 mg) containing the title compound.

Step 12-6: Methyl 3-(trans-4-aminocyclohexyl)-3-methylbutyrate monohydrochloride

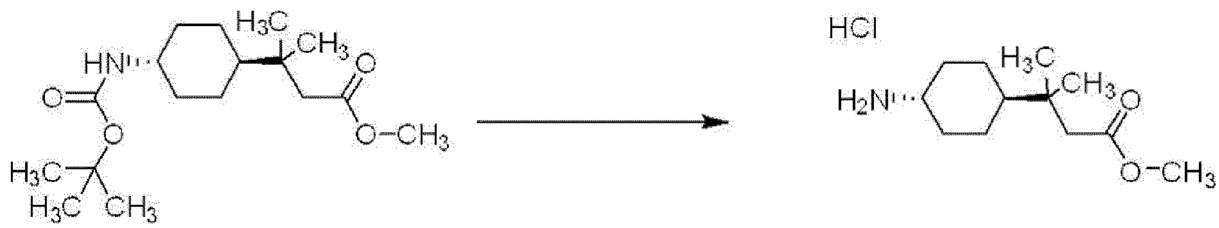

To a solution of a crude product (179 mg) containing methyl 3-(trans-4-((tert-butoxycarbonyl)amino)cyclohexyl)-3-methylbutyrate in ethyl acetate (2.0 mL) was added 4 M hydrogen chloride/ethyl acetate (2.0 mL) solution at room temperature, and the mixture was stirred overnight. The reaction mixture was concentrated under reduced pressure to give the title compound (131 mg).
$^1$H-NMR (DMSO-$D_6$) δ: 7.95 (3H, br s), 3.57 (3H, s), 2.92-2.85 (1H, m), 2.21 (2H, s), 1.99-1.96 (2H, m), 1.77-1.74 (2H, m), 1.33-0.96 (5H, m), 0.90 (6H, s).

Step 12-7: Methyl 3-(trans-4-(2-(6-fluoro-3-methyl-1H-indazol-1-yl)pyrimidine-5-carboxyamido)cyclohexyl)-3-methylbutyrate

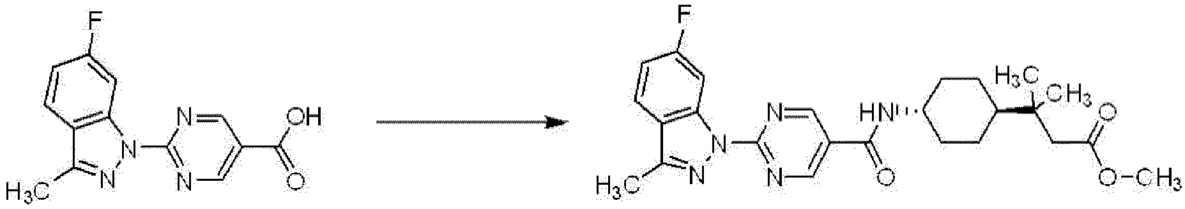

To a suspension of 2-(6-fluoro-3-methyl-1H-indazol-1-yl)pyrimidine-5-carboxylic acid (50 mg) synthesized in the same manner as in Production Example 1, step 1-2, methyl 3-(trans-4-aminocyclohexyl)-3-methylbutyrate monohydrochloride (55 mg), 1-hydroxy-7-azabenzotriazole (25 mg) and 1-ethyl-3-(3-dimethylaminopropyl)carbodiimide hydrochloride (55 mg) in N,N-dimethylformamide (1.5 mL) was added triethylamine (0.040 mL) at room temperature, and the mixture was stirred overnight. To the reaction mixture were successively added saturated aqueous sodium hydrogen carbonate (2.0 mL) and water (3.0 mL) at room temperature, and the precipitated solid was collected by filtration and dried under reduced pressure to give a crude product (77 mg) containing the title compound.

Step 12-8: 3-(Trans-4-(2-(6-fluoro-3-methyl-1H-indazol-1-yl)pyrimidine-5-carboxyamido)cyclohexyl)-3-methylbutyric acid

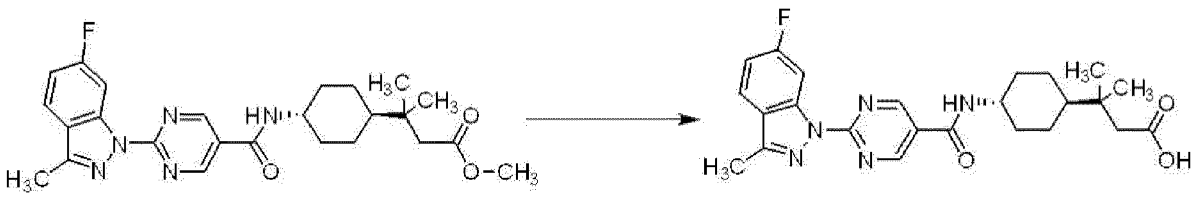

A suspension of a crude product (30 mg) containing methyl 3-(trans-4-(2-(6-fluoro-3-methyl-1H-indazol-1-yl) pyrimidine-5-carboxyamido)cyclohexyl)-3-methylbutyrate, sodium iodide (67 mg) and chlorotrimethylsilane (0.05 mL) in acetonitrile (2.0 mL) was stirred at 85°C for 7 hr. To the reaction mixture were added sodium iodide (63 mg) and chlorotrimethylsilane (0.05 mL) at room temperature, and the mixture was stirred at 85°C for 9 hr. To the reaction mixture were successively added water (4.0 mL) and 1 M hydrochloric acid (0.5 mL) at room temperature, and the solid was collected by filtration. The solid was dissolved in dimethyl sulfoxide, and purified by reversed-phase silica gel chromatography (eluent: water/acetonitrile=90/10 to 0/100) to give the title compound (7.3 mg).

$^1$H-NMR (DMSO-$D_6$) δ: 9.27-9.26 (2H, m), 8.83-8.73 (1H, br m), 8.40 (1H, dd, J = 10.4, 2.3 Hz), 7.94 (1H, dd, J = 8.7, 5.4 Hz), 7.31-7.26 (1H, m), 3.75-3.65 (1H, m), 2.61 (3H, s), 1.94-1.90 (2H, m), 1.86-1.83 (2H, m), 1.85 (2H, s), 1.48-1.41 (1H, m), 1.36-1.27 (2H, m), 1.11-1.02 (2H, m), 0.91 (6H, s).
MS (M+H) :454 MS (M-H) :452

[Production Example 13] <u>Example 306: Production of N-(trans-4-(((S)-1-hydroxypropan-2-yl)oxy)cyclohexyl)-2-(3-methyl-1H-indazol-1-yl)pyrimidine-5-carboxamide</u>

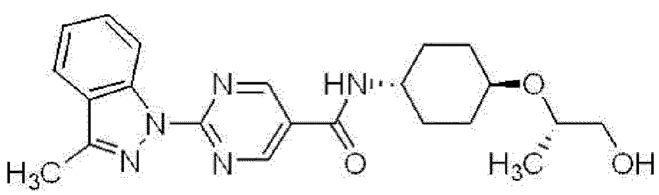

Step 13-1: (S)-((1-(Benzyloxy)propan-2-yl)oxy)trimethylsilane

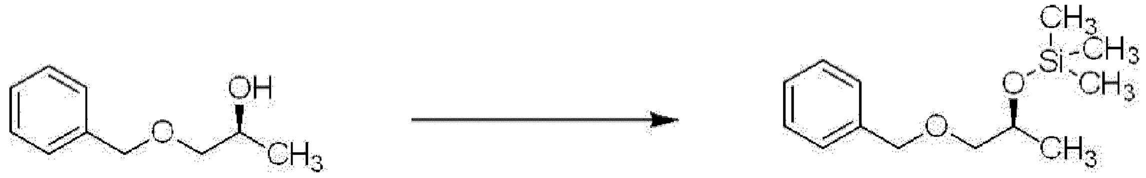

To a solution of (S)-1-benzyloxy-2-propanol (500 mg) and triethylamine (0.922 mL) in tetrahydrofuran (5.0 mL) was added trimethylsilyl chloride (0.408 mL) in a water bath, and the mixture was stirred and left standing overnight. To the reaction mixture was added n-hexane/ethyl acetate (n-hexane/ethyl acetate=10/1) at room temperature, and the mixture was stirred. The insoluble material was filtered off, and the filtrate was concentrated to give a crude product (767.8 mg) containing the title compound.

Step 13-2: Benzyl (trans-4-(((S)-1-(benzyloxy)propan-2-yl)oxy)cyclohexyl)carbamate

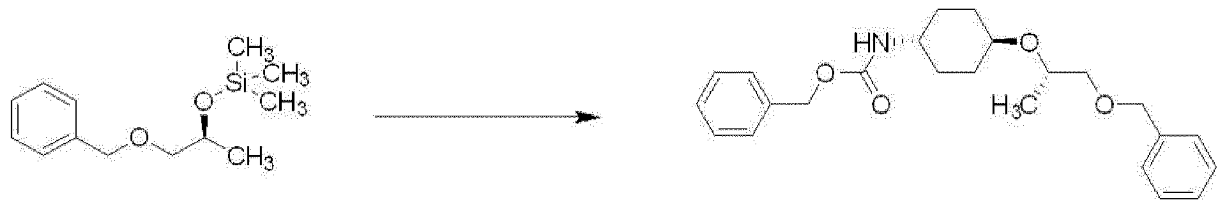

To a solution of a crude product (767.8 mg) containing (S)-((1-(benzyloxy)propan-2-yl)oxy)trimethylsilane, benzyl (4-oxocyclohexyl)carbamate (1481 mg) and triethylsilane (1045 mg) in acetonitrile (20 mL) was added dropwise acetonitrile (2.0 mL) solution of trimethylsilyl trifluoromethanesulfonate (1.094 mL) under ice-cooling, and the mixture was stirred at the same temperature for 3 hr. To the reaction mixture were added 1 M hydrochloric acid and ethyl acetate, and the layers were separated. The organic layer was washed with saturated brine. The organic layer was dried over sodium sulfate, sodium sulfate was removed by filtration, and the filtrate was concentrated under reduced pressure. The residue was purified by silica gel chromatography (eluent: n-hexane/ethyl acetate=80/20 to 76/24) to give the title compound (504.5 mg).
$^1$H-NMR (DMSO-$D_6$) δ: 7.39-7.26 (10H, m), 7.17 (1H, d, J = 7.6 Hz), 4.99 (2H, s), 4.48 (2H, s), 3.74-3.65 (1H, m), 3.40-3.23 (4H, m), 1.91-1.85 (2H, m), 1.82-1.75 (2H, m), 1.21-1.14 (4H, m), 1.04 (3H, d, J = 6.3 Hz).

Step 13-3: (S)-2-((Trans-4-aminocyclohexyl)oxy)propan-1-ol

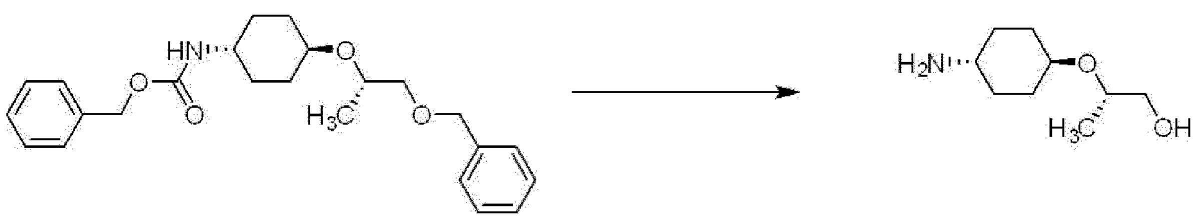

To a solution of benzyl (trans-4-(((S)-1-(benzyloxy)propan-2-yl)oxy)cyclohexyl)carbamate (504 mg) in methanol (10 mL) was added 20% palladium hydroxide-carbon (178 mg) at room temperature. Under 4 atm hydrogen, the mixture was stirred overnight at room temperature. The reaction mixture was filtered through celite, and the filtrate was concentrated under reduced pressure. To a solution of the residue in methanol (10 mL) was added 20% palladium hydroxide-carbon (178 mg) at room temperature. Under 4 atm hydrogen, the mixture was stirred at room temperature for 2 days. The reaction mixture was filtered through celite, and the filtrate was concentrated under reduced pressure. The residue was purified by Bond Elut SCX (Agilent technologies) (eluent: methanol, 1 M ammonia/methanol solution) to give

the title compound (176 mg).
$^1$H-NMR (DMSO-$D_6$) δ: 4.45 (1H, br s), 3.49-3.42 (1H, m), 3.34-3.14 (4H, m), 1.85-1.70 (4H, m), 1.61 (2H, br s), 1.18-0.95 (4H, m), 0.99 (3H, d, J = 5.0 Hz).

Step 13-4: N-(Trans-4-(((S)-1-hydroxypropan-2-yl)oxy)cyclohexyl)-2-(3-methyl-1H-indazol-1-yl)pyrimidine-5-carboxamide

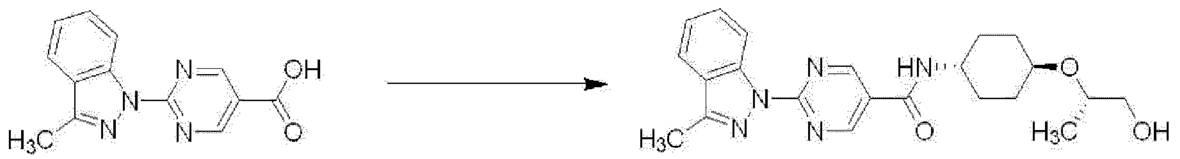

To a suspension of 2-(3-methyl-1H-indazol-1-yl)pyrimidine-5-carboxylic acid (40 mg) synthesized in the same manner as in Production Example 4, step 4-2, and (S)-2-((trans-4-aminocyclohexyl)oxy)propan-1-ol (32.7 mg) in N,N-dimethylformamide (0.6 mL) were successively added triethylamine (0.0329 mL), 1-hydroxy-7-azabenzotriazole (21.41 mg) and 1-ethyl-3-(3-dimethylaminopropyl)carbodiimide hydrochloride (36.2 mg) at room temperature, and the mixture was stirred at the same temperature for 20 hr. To the reaction mixture was added water (0.6 mL) at room temperature, and the mixture was purified by reversed-phase silica gel chromatography (eluent: water (containing 0.1% formic acid)/-acetonitrile=100/0 to 60/40) and concentrated. The precipitated solid was collected by filtration, washed with water, and dried under reduced pressure to give the title compound (46.2 mg).

$^1$H-NMR (DMSO-$D_6$) δ: 9.20 (2H, s), 8.66 (1H, d, J = 8.3 Hz), 8.51 (1H, d, J = 7.4 Hz), 7.87 (1H, d, J = 7.9 Hz), 7.63-7.60 (1H, m), 7.39-7.37 (1H, m), 4.48 (1H, t, J = 5.8 Hz), 3.80-3.73 (1H, m), 3.52-3.48 (1H, m), 3.39-3.31 (2H, m), 3.23-3.17 (1H, m), 2.61 (3H, s), 1.97-1.89 (4H, m), 1.43-1.20 (4H, m), 1.02 (3H, d, J = 6.2 Hz).
MS (M+H) :410 MS (M-H) :408

[Production Example 14] Example 335: Production of (S)-3-(trans-4-(4-(4-fluoro-1-methyl-1H-indazol-3-yl)benzamido) cyclohexyl)butanoic acid

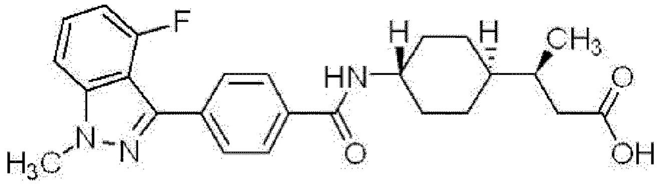

Step 14-1: Methyl 4-(4-fluoro-1-methyl-1H-indazol-3-yl)benzoate

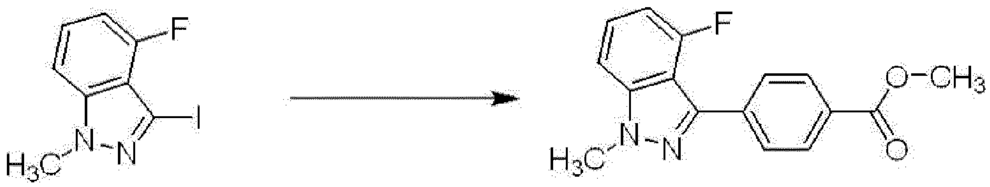

Under a nitrogen atmosphere, a suspension of 4-bromo-1H-pyrrolo[3,2-c]pyridine (202 mg), (4-(methoxycarbonyl)phenyl)boronic acid (359 mg), potassium phosphate (769 mg) and [1,1'-bis(di-tert-butylphosphino)ferrocene] palladium(II) dichloride-dichloromethane adduct (74 mg) in 1,2-dimethoxyethane/water (5.0 mL, 1,2-dimethoxyethane/-water=4/1) was stirred under microwave irradiation at 100°C for 45 min. The reaction mixture was purified by silica gel chromatography (eluent: n-hexane/ethyl acetate=100/0 to 70/30) to give a crude product (352 mg) containing the title compound.

Step 14-2: 4-(4-Fluoro-1-methyl-1H-indazol-3-yl)benzoic acid

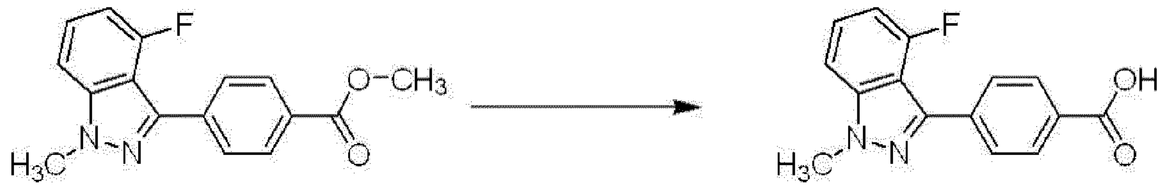

A solution of a crude product (352 mg) containing methyl 4-(4-fluoro-1-methyl-1H-indazol-3-yl)benzoate in methanol (3.0 mL) was stirred under microwave irradiation at 100°C for 20 min. To the reaction mixture was added 1 M hydrochloric acid at room temperature until a solid was precipitated. The precipitated solid was collected by filtration, and dried under reduced pressure at 60°C to give a crude product (315 mg) containing the title compound.

Step 14-3: Ethyl 3-(trans-4-((tert-butoxycarbonyl)amino)cyclohexyl)-3-oxopropionate (assumed to be enol-keto 1/9 mixture)

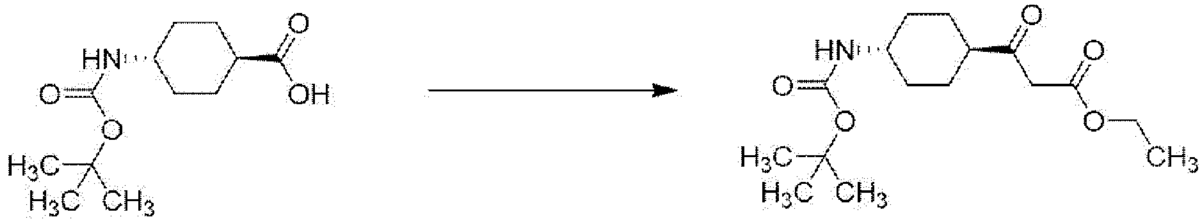

Under a nitrogen atmosphere, to a solution of trans-4-[(tert-butoxycarbonyl)amino]cyclohexanecarboxylic acid (200 g) in tetrahydrofuran (900 mL) was added 1,1'-carbonyldiimidazole (140 g) in portions over 7 min at room temperature while rinsing in with tetrahydrofuran (100 mL). After stirring for 1.5 hr, at room temperature, potassium ethyl malonate (196 g) was added in portions over 2 min, and magnesium chloride (110 g) was added in portions over 2 min while rinsing in with tetrahydrofuran (200 mL). The reaction mixture was stirred at room temperature for 26 hr. To the reaction mixture was added 20 wt% aqueous citric acid solution (1000 mL) at room temperature, and the mixture was stirred for 20 min. Ethyl acetate (1000 mL) was added thereto, and the layers were separated. The organic layer was washed successively with 10 wt% aqueous citric acid solution (1000 mL), water (1000 mL), 7.5 wt% aqueous sodium hydrogen carbonate solution (1000 mL), and saturated brine (1000 mL). The organic layer was dried over sodium sulfate, sodium sulfate was removed by filtration, and the filtrate was concentrated under reduced pressure. The residue was dissolved by adding n-hexane/ethyl acetate (800 mL, n-hexane/ethyl acetate=1/3), and insoluble material was filtered off. The filtrate was concentrated under reduced pressure and dried under reduced pressure to give the title compound (247 g).
$^1$H-NMR ($CDCl_3$) δ: 12.14 (0.1H, s), 4.95 (0.1H, s), 4.36 (1H, br s), 4.19 (2H, q, J = 7.1 Hz), 3.47 (1.8H, s), 3.40 (1H, br s), 2.49-2.33 (0.9H, m), 2.16-2.05 (2H, m), 2.01-1.93 (2H, m), 1.93-1.89 (0.1H, m), 1.53-1.36 (11H, m), 1.27 (3H, t, J = 7.2 Hz), 1.17-1.07 (2H, m).

Step 14-4: Ethyl (Z)-3-(trans-4-((tert-butoxycarbonyl)amino)cyclohexyl)-3-(tosyloxy)acrylate

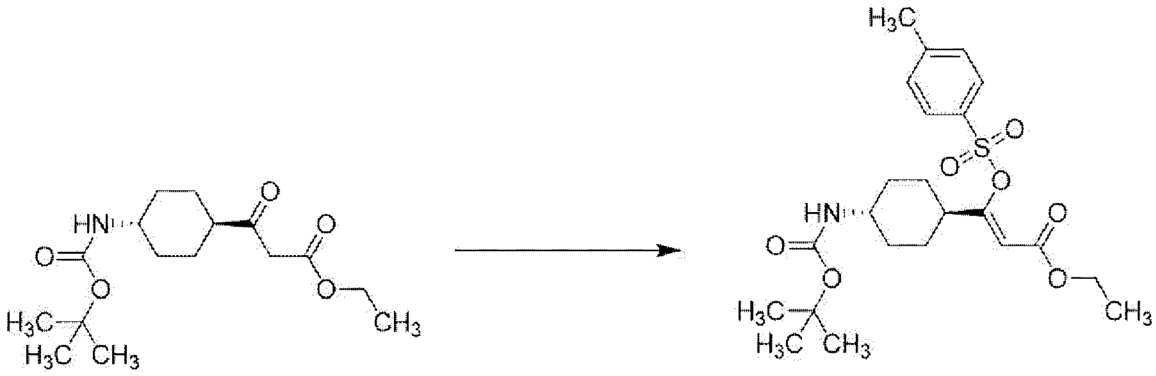

Under a nitrogen atmosphere, to a solution of ethyl 3-(trans-4-((tert-butoxycarbonyl)amino)cyclohexyl)-3-oxopropionate (234.4 g) in acetonitrile/toluene (937 mL, acetonitrile/toluene=1/3) were added tetramethylethylenediamine (169 mL) and lithium chloride (47.5 g) at room temperature, and the mixture was stirred for 30 min. To the reaction mixture was added dropwise acetonitrile/toluene (468 mL, acetonitrile/toluene=1/3) solution of p-toluenesulfonylchloride (214 g) under ice-cooling over 20 min, while rinsing in with acetonitrile/toluene (4118 mL, acetonitrile/toluene=1/3). After stirring for 2 hr, 5.0 wt% aqueous sodium hydrogen carbonate solution (1172 mL) was added dropwise over 1 min under ice-cooling. To the reaction mixture was added toluene (937 mL), and the layers were separated. The organic layer was washed successively with water (234 mL) and saturated brine (1172 mL). The organic layer was dried over sodium sulfate (234 g), CARBORAFFIN-20 (23 g) was added thereto, and sodium sulfate and CARBORAFFIN-20 were removed by filtration, and the mixture was concentrated under reduced pressure. To the residue was added ethyl acetate (200 mL), and the filtrate was dissolved at 85°C, and ethyl acetate (150 mL) was added. n-Hexane (1050 mL) was added dropwise at 50°C, and the seed crystal was added at 40°C. To this suspension was added dropwise n-hexane (2100 mL) at 40°C. The mixture was stirred at the same temperature for 2 hr, allowed to gradually return to room temperature, and stirred for 15 hr. The precipitated solid was collected by filtration, washed with n-hexane/ethyl acetate (500 mL, n-hexane/ethyl acetate=19/1), and dried under reduced pressure to give the title compound (302 g).

Synthesis of seed crystal of the title compound (ethyl (Z)-3-{trans-4-[(tert-butoxycarbonyl)amino]cyclohexyl}-3-(p-toluenesulfonyloxy)acrylate)

Step 14-4-1: Ethyl 3-(trans-4-((tert-butoxycarbonyl)amino)cyclohexyl)-3-oxopropionate

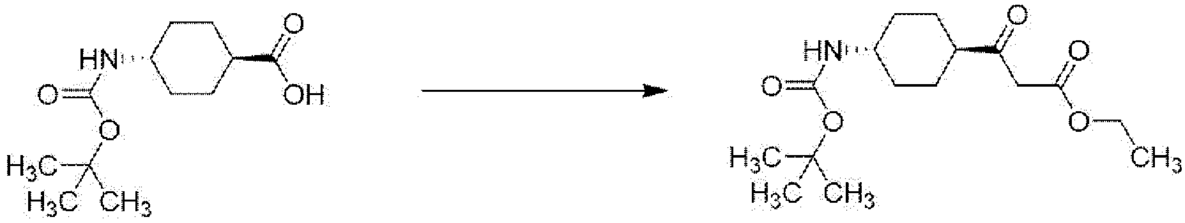

To a solution of trans-4-((tert-butoxycarbonyl)amino)cyclohexanecarboxylic acid (1.0 g) in tetrahydrofuran (5 mL) was added 1,1'-carbonyldiimidazole (700 mg) at room temperature, and the mixture was stirred for 1 hr. Potassium ethyl malonate (839 mg) and magnesium chloride (470 mg) were added at room temperature, and the mixture was stirred at room temperature for 1 hr, and at 70°C for 3 hr. To the reaction mixture was added 10 wt% aqueous citric acid solution (5.0 mL) at room temperature, and the mixture was stirred for 15 min. Ethyl acetate (5.0 mL) was added thereto, and the layers were separated. The organic layer was washed successively once with 10 wt% aqueous citric acid solution (5.0 mL), once with water (5.0 mL), three times with saturated aqueous sodium hydrogen carbonate solution (3.0 mL), and once with saturated brine. The organic layer was dried over sodium sulfate. Sodium sulfate was removed by filtration, and the filtrate was concentrated under reduced pressure. To the residue was dissolved by adding n-hexane/ethyl acetate (n-hexane/ethyl acetate=1/2), and insoluble material was filtered off. The filtrate was concentrated under reduced pressure and dried under reduced pressure to give a crude product (1.16 g) containing the title compound.

Step 14-4-2: Ethyl (Z)-3-(trans-4-((tert-butoxycarbonyl)amino)cyclohexyl)-3-(tosyloxy)acrylate

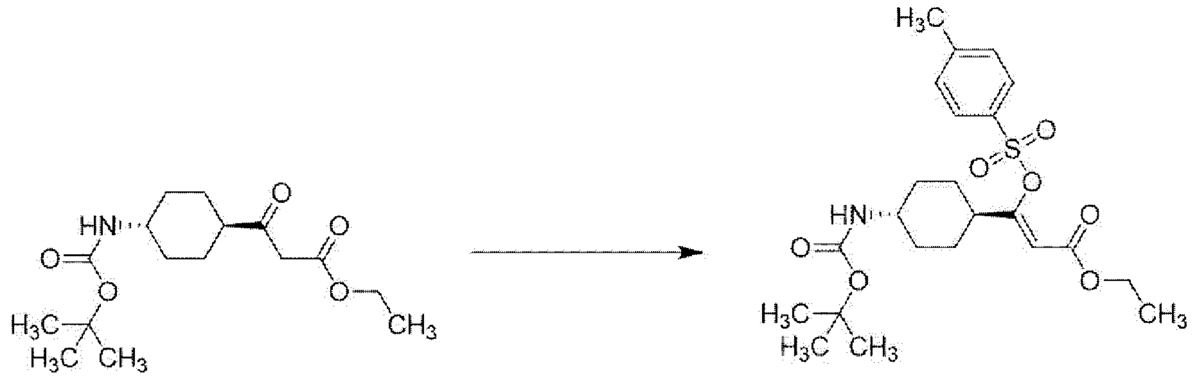

To a solution of ethyl 3-(trans-4-((tert-butoxycarbonyl)amino)cyclohexyl)-3-oxopropionate (1.15 g) in acetonitrile (9.26 mL) were added lithium chloride (235 mg) and tetramethylethylenediamine (0.829 mL) at room temperature, and the mixture was stirred for 15 min. To the reaction mixture was added dropwise acetonitrile (2.3 mL) solution of p-toluenesulfonylchloride (1.056 g) under ice-cooling, and the mixture was stirred for 1 hr. To the reaction mixture were added water (16 mL) and toluene (16 mL), and the layers were separated. The organic layer was washed successively with water, saturated aqueous sodium hydrogen carbonate, and saturated brine. The organic layer was dried over sodium sulfate (234 g), sodium sulfate was removed by filtration, and the filtrate was concentrated under reduced pressure. The residue was purified by silica gel chromatography (eluent: n-hexane/ethyl acetate=85/15 to 65/35) to give the title compound (1.40 g).
$^1$H-NMR ($CDCl_3$) δ: 7.93-7.86 (2H, m), 7.38-7.31 (2H, m), 5.52-5.49 (1H, m), 4.34 (1H, br s), 4.05 (2H, q, J = 7.1 Hz), 3.36 (1H, br s), 2.46 (3H, s), 2.39-2.28 (1H, m), 2.10-1.98 (4H, m), 1.43 (9H, s), 1.29-1.15 (5H, m), 1.13-1.00 (2H, m).

Step 14-5: Ethyl (E)-3-(trans-4-((tert-butoxycarbonyl)amino)cyclohexyl)-2-butenoate

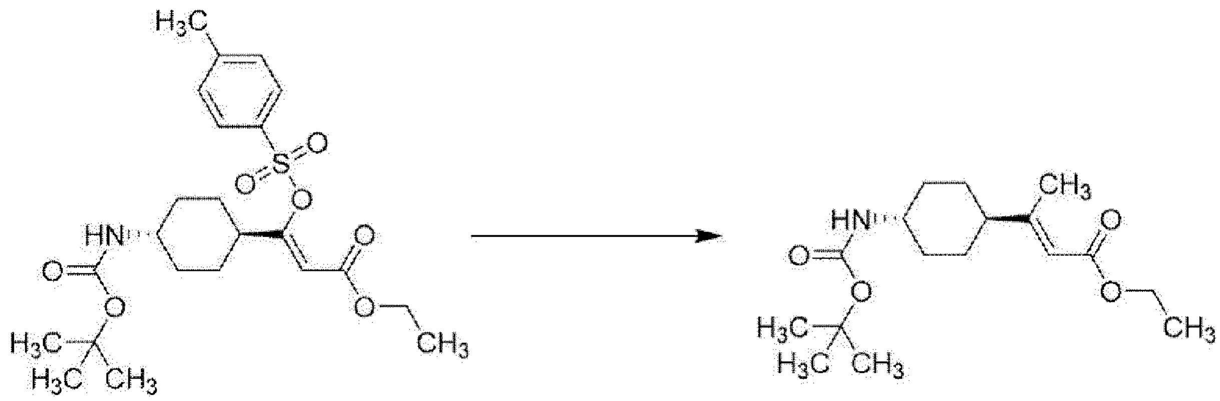

To a solution of ethyl (Z)-3-(trans-4-((tert-butoxycarbonyl)amino)cyclohexyl)-3-(tosyloxy)acrylate (210 g) in tetrahydrofuran (1050 mL) was added iron chloride (III) (3.64 g) under sodium chloride/ice-cooling, and 1.0 M tetrahydrofuran solution (1229 mL) of methylmagnesium bromide was added dropwise. To the reaction mixture was added 10 wt% aqueous citric acid solution (1000 mL) under sodium chloride/ice-cooling, and the mixture was stirred at room temperature for 1 hr. Ethyl acetate (840 mL) was added thereto, and the layers were separated. The organic layer was washed successively with 5.0 wt% aqueous sodium hydrogen carbonate solution (1050 mL), water (1050 mL), and saturated brine (1050 mL). The organic layer was dried over sodium sulfate (210 g), CARBORAFFIN-20 (21 g) was added thereto, sodium sulfate and CARBORAFFIN-20 were removed by filtration, and the filtrate was concentrated under reduced pressure. The residue was dissolved by adding n-hexane/ethyl acetate (1600 mL, n-hexane/ethyl acetate=9/1) at 85°C, and the mixture was allowed to gradually return to room temperature and stirred for 15 hr. The precipitated solid was collected by filtration, washed with n-hexane/ethyl acetate (400 mL, n-hexane/ethyl acetate=19/1), and dried under

reduced pressure to give a crude product (100 g) containing the title compound. The crude products (40 g and 60 g) containing the title compound were purified by silica gel chromatography (eluent: n-hexane/ethyl acetate=95/5 to 50/50) to give the title compound (44.09 g).
$^1$H-NMR ($CDCl_3$) δ: 5.65 (1H, s), 4.37 (1H, br s), 4.14 (2H, q, J = 7.1 Hz), 3.39 (1H, br s), 2.13 (3H, d, J = 1.2 Hz), 2.12-2.03 (2H, m), 1.99-1.88 (1H, m), 1.84-1.74 (2H, m), 1.44 (9H, s), 1.42-1.30 (2H, m), 1.27 (3H, t, J = 7.2 Hz), 1.20-1.06 (2H, m).

Step 14-6: Ethyl (S)-3-(trans-4-((tert-butoxycarbonyl)amino)cyclohexyl)butanoate

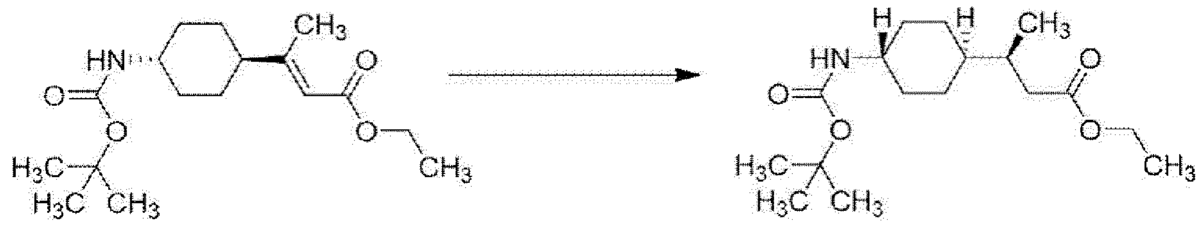

Under a nitrogen atmosphere, a solution of bis[η-(2,5-nornornadiene)]rhodium (I) tetrafluoroborate (0.36 g) and (R)-1-[(Sp)-2-(di-tert-butylphosphino)ferrocenyl]ethyl bis(2-methylphenyl)phosphine (CAS No.: 849924-76-1) (0.583 g) in methanol (120 mL) was stirred at room temperature for 1 hr. To the reaction mixture was added a solution of ethyl (E)-3-(trans-4-((tert-butoxycarbonyl)amino)cyclohexyl)-2-butenoate (30 g) in methanol (210 mL) at room temperature, and the mixture was stirred under 3.9 atm hydrogen for 2 days. The reaction mixture was concentrated under reduced pressure. It was combined with a concentrate obtained from ethyl (E)-3-(trans-4-((tert-butoxycarbonyl)amino)cyclohexyl)-2-butenoate (14 g) by a similar production method to give a crude product (46 g) containing the title compound.

Step 14-7: Ethyl (S)-3-(trans-4-aminocyclohexyl)butanoate hydrochloride

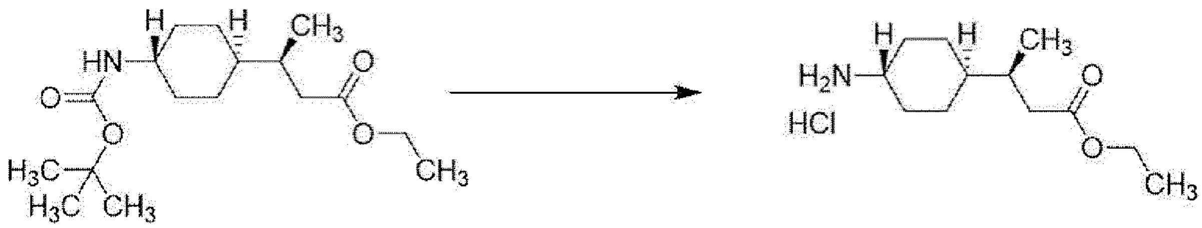

To a solution of a crude product (100 g) containing ethyl (S)-3-(trans-4-((tert-butoxycarbonyl)amino)cyclohexyl) butanoate synthesized in the same manner as in step 14-6 in ethyl acetate (100 mL) was added dropwise 4 M hydrogen chloride/ethyl acetate (500 mL) solution under ice-cooling over 10 min. The mixture was allowed to gradually return to room temperature and stirred for 90 min. To the reaction mixture was added n-hexane (300 mL) and the mixture was stirred for 30 min. The precipitated solid was collected by filtration, washed with n-hexane/ethyl acetate (100 mL, n-hexane/ethyl acetate=9/1), and dried under reduced pressure to give the title compound (56.8 g).
$^1$H-NMR (DMSO-$D_6$) δ: 7.87 (3H, br s), 4.05 (2H, q, J = 7.1 Hz), 2.89 (1H, tt, J = 11.7, 3.8 Hz), 2.35 (1H, dd, J = 14.9, 5.2 Hz), 2.05 (1H, dd, J = 15.0, 9.0 Hz), 2.00-1.87 (2H, m), 1.83-1.71 (1H, m), 1.71-1.59 (2H, m), 1.33-1.20 (2H, m), 1.17 (3H, t, J = 7.2 Hz), 1.14-0.96 (3H, m), 0.83 (3H, d, J = 6.7 Hz).

Step 14-8: Ethyl (S)-3-(trans-4-(4-(4-fluoro-1-methyl-1H-indazol-3-yl)benzamido)cyclohexyl)butanoate

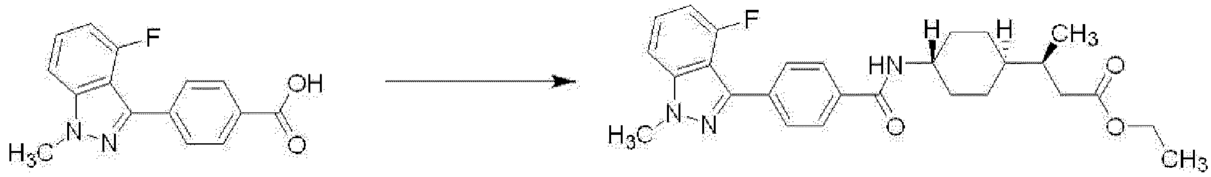

To a suspension of a crude product (50 mg) containing 4-(4-fluoro-1-methyl-1H-indazol-3-yl)benzoic acid, 1-ethyl-3-(3-dimethylaminopropyl)carbodiimide hydrochloride (42.6 mg), 1-hydroxy-1H-benzotriazole monohydrate (31.2 mg), and ethyl (S)-3-(trans-4-aminocyclohexyl)butanoate hydrochloride (50.8 mg) in N,N-dimethylformamide (1.5 mL) was added triethylamine (0.064 mL) at room temperature, and the mixture was stirred for 3 hr. To the reaction mixture was added water at room temperature until a solid was precipitated, and the precipitated solid was collected by filtration and dried under reduced pressure to give a crude product (110 mg) containing the title compound.

Step 14-9: (S)-3-(Trans-4-(4-(4-fluoro-1-methyl-1H-indazol-3-yl)benzamido)cyclohexyl)butanoic acid

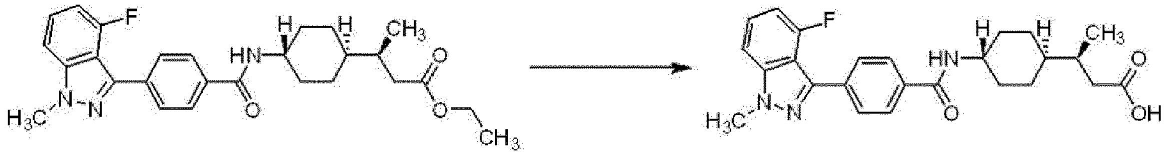

To a solution of a crude product (86.6 mg) containing ethyl (S)-3-(trans-4-(4-(4-fluoro-1-methyl-1H-indazol-3-yl) benzamido)cyclohexyl)butanoate in tetrahydrofuran/methanol (3 mL, tetrahydrofuran/methanol=1/2) was added 2 M aqueous sodium hydroxide solution (0.463 mL) at room temperature, and the mixture was stirred under microwave irradiation at 100°C for 20 min. To the reaction mixture was added 2 M hydrochloric acid (0.463 mL) at room temperature, and the precipitated solid was collected by filtration, and dried under reduced pressure at 60°C to give the title compound (68.7 mg).

$^1$H-NMR (DMSO-D$_6$) δ: 11.98 (1H, br s), 8.30-8.24 (1H, m), 8.06-8.01 (2H, m), 8.00-7.95 (2H, m), 7.93-7.88 (1H, m), 7.81-7.76 (1H, m), 7.43-7.36 (1H, m), 4.14 (3H, s), 3.80-3.68 (1H, m), 2.35-2.27 (1H, m), 2.04-1.95 (1H, m), 1.94-1.86 (2H, m), 1.84-1.74 (1H, m), 1.73-1.65 (2H, m), 1.42-1.27 (2H, m), 1.25-1.06 (3H, m), 0.89-0.85 (3H, m).
MS (M+H) :438 MS (M-H) :436

[Production Example 15] Example 426: Production of N-(trans-4-(2-hydroxypropan-2-yl)cyclohexyl)-2-(3-methylimidazo[1,5-a]pyridin-1-yl)pyrimidine-5-carboxamide

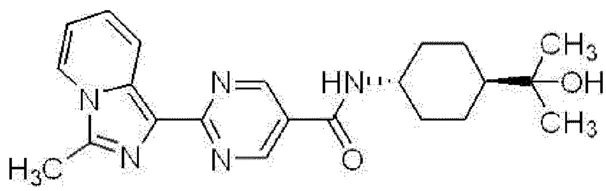

Step 15-1: 2,2,2-Trifluoro-1-(3-methylimidazo[1,5-a]pyridin-1-yl)ethan-1-one

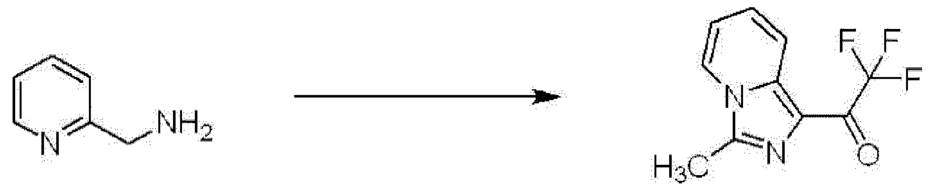

To a solution of 2-picolylamine (2.098 g) and pyridine (6.2 mL) in chloroform (15 mL) was added dropwise acetyl chloride (1.4 mL) under ice-cooling, and the mixture was stirred at the same temperature for 20 min. To the reaction mixture was added dropwise trifluoroacetic anhydride (5.5 mL) under ice-cooling, and the mixture was stirred at room temperature overnight. To the reaction mixture was added saturated aqueous sodium hydrogen carbonate (60 mL) at room temperature, and the layers were separated. The aqueous layer was extracted once with chloroform (15 mL). The combined organic layer was concentrated under reduced pressure, and toluene (30 mL) was added to the residue. The mixture was concentrated under reduced pressure, and the residue was purified by silica gel chromatography (eluent: n-hexane/ethyl acetate=84/16 to 0/100) to give a crude product (1.98 g) containing the title compound.

Step 15-2: 3-Methylimidazo[1,5-a]pyridine-1-carboxylic acid

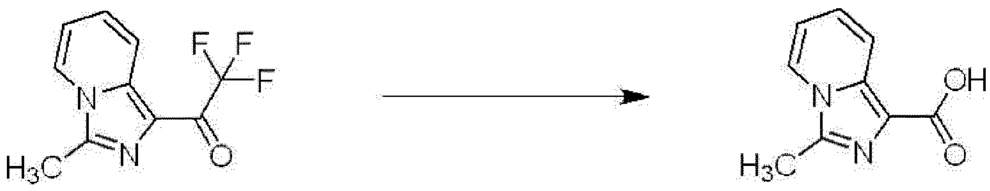

To a solution of a crude product (1.98 g) containing 2,2,2-trifluoro-1-(3-methylimidazo[1,5-a]pyridin-1-yl)ethan-1-one in methanol (20 mL) was added 2 M aqueous sodium hydroxide solution (20 mL) at room temperature, and the mixture was stirred at 80°C for 7 hr. To the reaction mixture was added 6 M hydrochloric acid (6.6 mL) at room temperature, and methanol contained in the reaction mixture was concentrated under reduced pressure. To the residue was added 1 M hydrochloric acid (7.0 mL) at room temperature, and the precipitated solid was collected by filtration and dried under reduced pressure to give the title compound (811 mg).

$^1$H-NMR (DMSO-$D_6$) $\delta$: 8.43-8.39 (1H, m), 8.04-8.01 (1H, m), 7.35-7.28 (1H, m), 7.09-7.03 (1H, m), 2.70 (3H, s).

Step 15-3: 3-Methylimidazo[1,5-a]pyridine-1-carboxamide

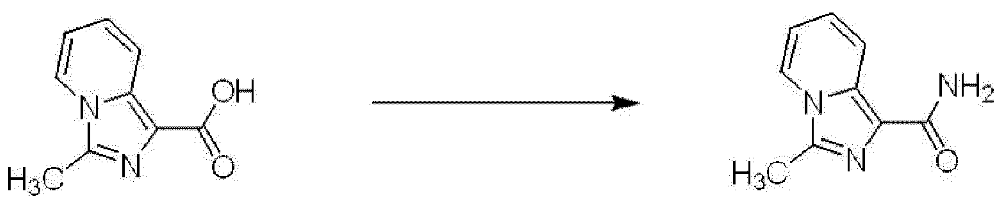

A mixture of 3-methylimidazo[1,5-a]pyridine-1-carboxylic acid (510 mg) and thionyl chloride (3.0 mL) was stirred at 70°C for 7 hr, and left standing overnight at room temperature. The reaction mixture was concentrated under reduced pressure, and 28% aqueous ammonia solution (5.0 mL) was added to the obtained residue under ice-cooling. The mixture was warmed to room temperature and stirred overnight. The precipitated solid was collected by filtration, washed with water, and dried under reduced pressure to give a crude product (352 mg) containing the title compound.

Step 15-4: 3-Methylimidazo[1,5-a]pyridine-1-carbonitrile

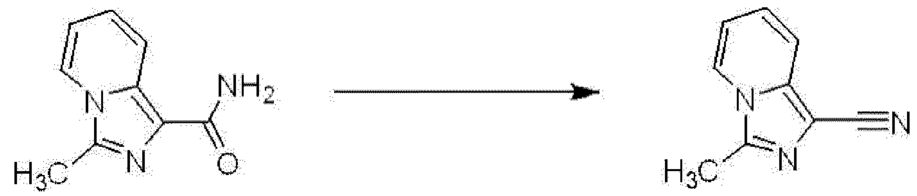

To a solution of a crude product (352 mg) containing 3-methylimidazo[1,5-a]pyridine-1-carboxamide and triethylamine (0.65 mL) in chloroform (5.0 mL) was added trifluoroacetic anhydride (0.37 mL) under ice-cooling, and the mixture was allowed to gradually return to room temperature and stirred overnight. To the reaction mixture were added triethylamine (0.33 mL) and trifluoroacetic acid (0.20 mL) under ice-cooling, and the mixture was allowed to gradually return to room temperature and stirred overnight. To the reaction mixture was added saturated aqueous sodium hydrogen carbonate (5.0 mL), and the layers were separated. The organic layer was concentrated under reduced pressure. The residue was purified by silica gel chromatography (eluent: n-hexane/ethyl acetate=88/12 to 0/100) to give the title compound (254 mg).
$^1$H-NMR (DMSO-$D_6$) δ: 8.41-8.39 (1H, m), 7.76-7.73 (1H, m), 7.29-7.25 (1H, m), 7.02-6.98 (1H, m), 2.64 (3H, s).

Step 15-5: Ethyl 2-(3-methylimidazo[1,5-a]pyridin-1-yl)pyrimidine-5-carboxylate

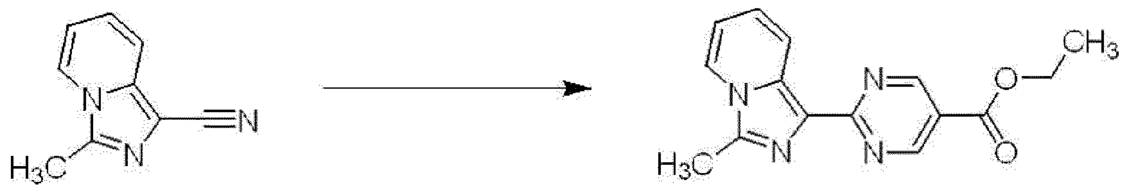

To a solution of 3-methylimidazo[1,5-a]pyridine-1-carbonitrile (254 mg) in methanol (7 mL) were added 5.0 M methanol solution (0.032 mL) of sodium methoxide at room temperature, and the mixture was stirred at the same temperature for 5 hr. To the reaction mixture was added 5.0 M methanol solution (0.32 mL) of sodium methoxide at room temperature, and the mixture was stirred at room temperature overnight. To the reaction mixture was added 5.0 M methanol solution (0.32 mL) of sodium methoxide at room temperature, and the mixture was stirred at room temperature overnight. This step was repeated twice. To the reaction mixture was added ammonium chloride (463 mg) at room temperature, and the mixture was stirred for 72 hr. The mixture was filtered through celite, and concentrated under reduced pressure. To the residue were successively added N,N-dimethylformamide (4.0 mL), potassium carbonate (675 mg) and ethyl 2-formyl-3-oxopropionate (0.611 mL) at room temperature, and the mixture was stirred at 100°C for 7 hr. To the reaction mixture were added ethyl acetate (20 mL) and water (15 mL) at room temperature, and the layers were separated. The aqueous layer was extracted once with ethyl acetate (10 mL). The combined organic layer was washed successively with water and saturated brine, dried over sodium sulfate, and concentrated under reduced pressure. The residue was purified twice by silica gel chromatography (eluent: n-hexane/acetone=66/34 to 34/66, then chloroform/methanol=90/10) to give the title compound (230 mg).
$^1$H-NMR (DMSO-$D_6$) δ: 9.16 (2H, s), 8.55-8.53 (1H, m), 8.35 (1H, d, J = 6.5 Hz), 7.28-7.24 (1H, m), 7.00-6.96 (1H, m), 4.40-4.34 (2H, m), 2.70 (3H, s), 1.38-1.34 (3H, m).

Step 15-6: 2-(3-Methylimidazo[1,5-a]pyridin-1-yl)pyrimidine-5-carboxylic acid

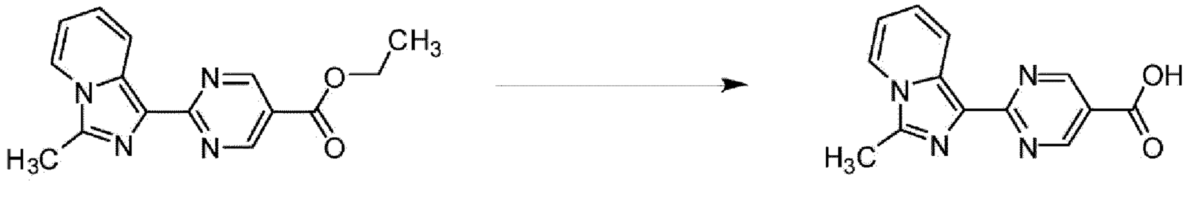

To a suspension of ethyl 2-(3-methylimidazo [1,5-a]pyridin-l-yl)pyrimidine-5-carboxylate (180 mg) in methanol (3.0 mL) was added 2 M aqueos sodium hydroxide solution (0.7 mL) at room temperature, and the mixture was stirred

overnight. To the reaction mixture were successively added 1 M hydrochloric acid (1.4 mL) and water (15.0 mL) at room temperature, and the precipitated solid was collected by filtration and dried under reduced pressure to give the title compound (82 mg).
$^1$H-NMR (DMSO-$D_6$) δ: 13.54 (1H, br s), 9.21 (2H, s), 8.55 (1H, d, J = 9.2 Hz), 8.46-8.44 (1H, m), 7.39-7.35 (1H, m), 7.12-7.08 (1H, m), 2.78 (3H, s).

Step 15-7: N-(Trans-4-(2-hydroxypropan-2-yl) cyclohexyl)-2-(3-methylimidazo[1,5-a]pyridin-1-yl)pyrimidine-5-carboxamide

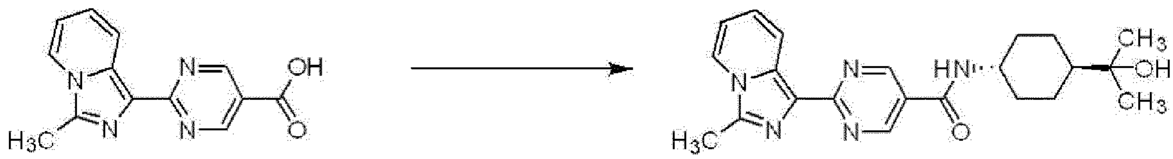

A suspension of 2-(3-methylimidazo[1,5-a]pyridin-1-yl)pyrimidine-5-carboxylic acid (30 mg), 2-(trans-4-aminocyclohexyl)propan-2-ol (25 mg), 1-hydroxy-7-azabenzotriazole (16 mg) and 1-ethyl-3-(3-dimethylaminopropyl)carbodiimide hydrochloride (36 mg) in N,N-dimethylformamide (1.0 mL) was stirred at room temperature overnight. To the reaction mixture were added saturated aqueous sodium hydrogen carbonate (2.0 mL) and water (3.0 mL) at room temperature, and the precipitated solid was collected by filtration and dried under reduced pressure to give the title compound (31.4 mg).

$^1$H-NMR (DMSO-$D_6$) δ: 9.13 (2H, s), 8.51 (1H, d, J = 8.8 Hz), 8.44 (1H, d, J = 7.9 Hz), 8.32 (1H, d, J = 6.7 Hz), 7.23-7.19 (1H, m), 6.97-6.93 (1H, m), 4.04 (1H, br s), 3.78-3.68 (1H, m), 2.70 (3H, s), 1.97-1.91 (2H, m), 1.88-1.82 (2H, m), 1.36-1.09 (5H, m), 1.05 (6H, s).
MS (M+H) :394 MS (M-H) :438

Other Example compounds were obtained by a method similar to the above-mentioned production methods and Production Examples, or using a known method as necessary. The structural formula and property data of each Example compound are shown in the following Table 1.

[Table 1-1]

| Ex.No. | structural formula | 1H-NMR (400 MHz) | MS (positive) | MS (negative) | Comments |
|---|---|---|---|---|---|
| 1 | 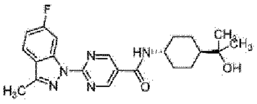 | 1H-NMR (DMSO-D6) δ: 9.23 (2H, s), 8.54 (1H, d, J = 8.2 Hz), 8.40 (1H, dd, J = 10.2, 2.0 Hz), 7.94 (1H, dd, J = 8.6, 5.6 Hz), 7.29 (1H, ddd, J = 10.2, 8.6, 2.0 Hz), 4.05 (1H, br s), 3.81-3.68 (1H, m), 2.61 (3H, s), 1.96 (2H, d, J = 10.5 Hz), 1.86 (2H, d, J = 10.5 Hz), 1.40-1.26 (2H, m), 1.25-1.00 (3H, m), 1.06 (6H, s). | 412 | 410 | |
| 2 | 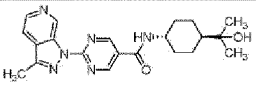 | 1H-NMR (DMSO-D6) δ: 9.99 (1H, s), 9.28 (2H, s), 8.58 (1H, d, J = 7.6 Hz), 8.53 (1H, d, J = 5.3 Hz), 7.94 (1H, d, J = 5.3 Hz), 4.05 (1H, br s), 3.81-3.68 (1H, m), 2.66 (3H, s), 1.97 (2H, d, J = 10.2 Hz), 1.86 (2H, d, J = 10.2 Hz), 1.40-1.26 (2H, m), 1.25-1.03 (3H, m), 1.06 (6H, s). | 395 | 393 | |
| 3 | 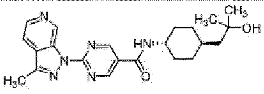 | 1H-NMR (DMSO-D6) δ: 9.99 (1H, s), 9.27 (2H, s), 8.56 (1H, d, J = 7.6 Hz), 8.53 (1H, d, J = 5.3 Hz), 7.94 (1H, d, J = 5.3 Hz), 4.04 (1H, br s), 3.82-3.69 (1H, m), 2.66 (3H, s), 1.88 (4H, d, J = 10.6 Hz), 1.50-1.22 (5H, m), 1.21-0.98 (2H, m), 1.11 (6H, s). | 409 | 407 | |
| 4 | 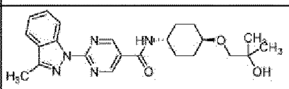 | 1H-NMR (DMSO-D6) δ: 9.22 (2H, s), 8.67 (1H, d, J = 8.6 Hz), 8.53 (1H, d, J = 7.6 Hz), 7.89 (1H, d, J = 7.9 Hz), 7.65-7.61 (1H, m), 7.42-7.38 (1H, m), 4.22 (1H, s), 3.85-3.76 (1H, m), 3.29-3.22 (1H, m), 3.19 (2H, s), 2.62 (3H, s), 2.06-2.01 (2H, m), 1.95-1.91 (2H, m), 1.45-1.23 (4H, m), 1.08 (6H, s). | 424 | 422 | |
| 5 | 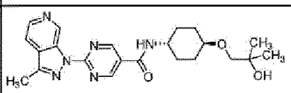 | 1H-NMR (DMSO-D6) δ: 9.99 (1H, s), 9.27 (2H, s), 8.58 (1H, d, J = 7.5 Hz), 8.54 (1H, d, J = 5.2 Hz), 7.94 (1H, d, J = 5.2 Hz), 4.20 (1H, br s), 3.87-3.74 (1H, m), 3.51-3.22 (1H, m), 3.19 (2H, s), 2.67 (3H, s), 2.04 (2H, d, J = 10.5 Hz), 1.93 (2H, d, J = 10.5 Hz), 1.48-1.20 (4H, m), 1.07 (6H, s). | 425 | 423 | |
| 6 | 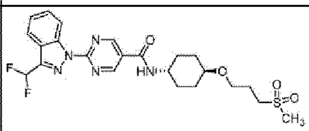 | 1H-NMR (DMSO-D6) δ: 9.30 (2H, s), 8.78 (1H, d, J = 8.6 Hz), 8.63 (1H, d, J = 7.5 Hz), 8.00 (1H, d, J = 8.1 Hz), 7.75 (1H, t, J = 8.1 Hz), 7.56 (1H, t, J = 53.9 Hz), 7.52 (1H, t, J = 7.5 Hz), 3.86-3.76 (1H, m), 3.53 (2H, t, J = 6.2 Hz), 3.30-3.25 (1H, m), 3.16-3.11 (2H, m), 2.99 (3H, s), 2.07-2.02 (2H, m), 1.97-1.87 (4H, m), 1.46-1.36 (2H, m), 1.33-1.23 (2H, m). | 508 | 506 | |
| 7 | 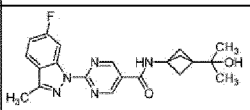 | 1H-NMR (DMSO-D6) δ: 9.24 (1H, br s), 9.22 (2H, s), 8.39 (1H, dd, J = 10.3, 2.2 Hz), 7.94 (1H, dd, J = 8.8, 5.3 Hz), 7.32-7.26 (1H, m), 4.20 (1H, s), 2.61 (3H, s), 1.96 (6H, s), 1.09 (6H, s). | 396 | 394 | |
| 8 | 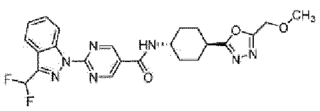 | 1H-NMR (DMSO-D6) δ: 9.33 (2H, s), 8.79 (1H, d, J = 5.1 Hz), 8.74 (1H, d, J = 7.6 Hz), 8.01 (1H, d, J = 8.1 Hz), 7.78-7.74 (1H, m), 7.57 (1H, t, J = 53.0 Hz), 7.56-7.49 (1H, m), 4.63 (2H, s), 3.95-3.85 (1H, m), 3.35 (3H, s), 3.06-2.96 (1H, m), 2.24-2.22 (2H, br m), 2.18-2.16 (2H, br m), 1.75-1.46 (4H, m). | 484 | 482 | |

[Table 1-2]

| Ex.No. | structural formula | 1H-NMR (400 MHz) | MS (positive) | MS (negative) | Comments |
|---|---|---|---|---|---|
| 9 | 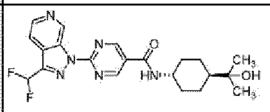 | 1H-NMR (DMSO-D6) δ: 10.12 (1H, s), 9.35 (2H, s), 8.67 (1H, d, J = 7.5 Hz), 8.62 (1H, d, J = 5.4 Hz), 8.00 (1H, d, J = 5.4 Hz), 7.64 (1H, t, J = 53.3 Hz), 4.06 (1H, s), 3.81-3.70 (1H, m), 1.99-1.85 (4H, m), 1.38-1.28 (2H, m), 1.22-1.11 (3H, m), 1.05 (6H, s). | 431 | 429 | |
| 10 | 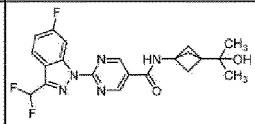 | 1H-NMR (DMSO-D6) δ: 9.33 (1H, br s), 9.30 (2H, s), 8.52 (1H, dd, J = 10.3, 2.2 Hz), 8.04 (1H, dd, J = 8.8, 5.3 Hz), 7.56 (1H, t, J = 53.2 Hz), 7.46-7.41 (1H, m), 4.21 (1H, s), 1.96 (6H, s), 1.09 (6H, s). | 432 | 430 | |
| 11 | 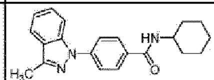 | 1H-NMR (DMSO-D6) δ: 8.28 (1H, d, J = 7.9 Hz), 8.06-8.02 (2H, m), 7.92-7.83 (4H, m), 7.55-7.51 (1H, m), 7.31-7.27 (1H, m), 3.83-3.76 (1H, m), 2.61 (3H, s), 1.87-1.83 (2H, m), 1.77-1.74 (2H, m), 1.65-1.60 (1H, m), 1.40-1.27 (4H, m), 1.20-1.10 (1H, m). | 334 | Not Detected | |
| 12 | 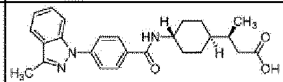 | 1H-NMR (DMSO-D6) δ: 11.99 (1H, s), 8.28 (1H, d, J = 8.1 Hz), 8.05-8.02 (2H, m), 7.92-7.83 (4H, m), 7.55-7.51 (1H, m), 7.31-7.27 (1H, m), 3.79-3.70 (1H, m), 2.60 (3H, s), 2.32 (1H, dd, J = 15.0, 5.2 Hz), 2.00 (1H, dd, J = 15.0, 9.0 Hz), 1.93-1.88 (2H, m), 1.82-1.76 (1H, m), 1.70-1.68 (2H, m), 1.40-1.29 (2H, m), 1.24-1.06 (3H, m), 0.88 (3H, d, J = 6.7 Hz). | 420 | Not Detected | opti-cally active form (S) |
| 13 | 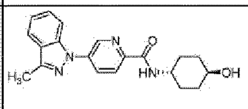 | 1H-NMR (DMSO-D6) δ: 9.08 (1H, d, J = 2.5 Hz), 8.43 (1H, d, J = 8.8 Hz), 8.39 (1H, dd, J = 8.4, 2.4 Hz), 8.19 (1H, d, J = 8.6 Hz), 7.97 (1H, d, J = 8.6 Hz), 7.91 (1H, d, J = 8.3 Hz), 7.59-7.55 (1H, m), 7.35-7.31 (1H, m), 4.56 (1H, d, J = 4.4 Hz), 3.81-3.73 (1H, m), 3.44-3.38 (1H, m), 2.62 (3H, s), 1.88-1.80 (4H, m), 1.54-1.44 (2H, m), 1.32-1.23 (2H, m). | 351 | Not Detected | |
| 14 | 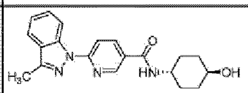 | 1H-NMR (DMSO-D6) δ: 8.96 (1H, dd, J = 2.3, 0.7 Hz), 8.76-8.73 (1H, m), 8.37-8.33 (2H, m), 8.00 (1H, dd, J = 8.7, 0.6 Hz), 7.87 (1H, dt, J = 8.1, 0.9 Hz), 7.62-7.58 (1H, m), 7.35 (1H, td, J = 16.0, 8.0 Hz), 4.56 (1H, d, J = 4.4 Hz), 3.80-3.71 (1H, m), 3.46-3.37 (1H, m), 2.62 (3H, s), 1.88-1.85 (4H, m), 1.44-1.34 (2H, m), 1.31-1.21 (2H, m). | 351 | 349 | |
| 15 | 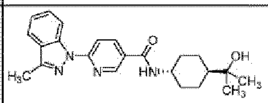 | 1H-NMR (DMSO-D6) δ: 8.97-8.96 (1H, m), 8.75 (1H, d, J = 8.3 Hz), 8.38-8.34 (2H, m), 8.00 (1H, d, J = 8.8 Hz), 7.87 (1H, d, J = 8.1 Hz), 7.62-7.58 (1H, m), 7.37-7.33 (1H, m), 4.04 (1H, s), 3.78-3.69 (1H, m), 2.62 (3H, s), 1.96-1.91 (2H, m), 1.87-1.83 (2H, m), 1.37-1.28 (2H, m), 1.23-1.05 (3H, m), 1.05 (6H, s). | 393 | 391 | |
| 16 | 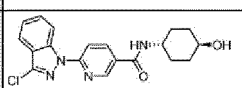 | 1H-NMR (DMSO-D6) δ: 8.99 (1H, d, J = 2.2 Hz), 8.82 (1H, d, J = 8.2 Hz), 8.44-8.39 (2H, m), 8.00 (1H, d, J = 8.2 Hz), 7.85 (1H, d, J = 8.2 Hz), 7.76-7.72 (1H, m), 7.50-7.48 (1H, m), 4.56 (1H, d, J = 4.5 Hz), 3.80-3.72 (1H, m), 3.45-3.40 (1H, m), 1.88-1.86 (4H, m), 1.39-1.26 (4H, m). | 371 | 369 | |

[Table 1-3]

| Ex.No. | structural formula | 1H-NMR (400 MHz) | MS (positive) | MS (negative) | Comments |
|---|---|---|---|---|---|
| 17 | 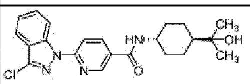 | 1H-NMR (DMSO-D6) δ: 9.00 (1H, d, J = 2.2 Hz), 8.82 (1H, d, J = 8.2 Hz), 8.45-8.40 (2H, m), 8.00 (1H, d, J = 9.0 Hz), 7.85 (1H, d, J = 7.5 Hz), 7.76-7.72 (1H, m), 7.49 (1H, t, J = 7.5 Hz), 4.04 (1H, s), 3.76-3.72 (1H, m), 1.94-1.85 (4H, m), 1.37-1.09 (5H, m), 1.05 (6H, s). | 413 | 411 | |
| 18 | 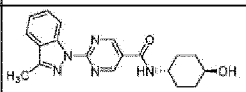 | 1H-NMR (DMSO-D6) δ: 9.22 (2H, s), 8.67 (1H, d, J = 8.2 Hz), 8.51 (1H, d, J = 7.5 Hz), 7.89 (1H, d, J = 7.5 Hz), 7.65-7.61 (1H, m), 7.42-7.38 (1H, m), 4.58 (1H, d, J = 4.5 Hz), 3.81-3.72 (1H, m), 3.46-3.38 (1H, m), 2.62 (3H, s), 1.90-1.87 (4H, m), 1.44-1.35 (2H, m), 1.31-1.22 (2H, m). | 352 | 350 | |
| 19 | 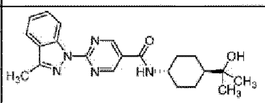 | 1H-NMR (DMSO-D6) δ: 9.22 (2H, s), 8.67 (1H, d, J = 8.5 Hz), 8.53 (1H, d, J = 7.8 Hz), 7.89 (1H, d, J = 8.0 Hz), 7.65-7.61 (1H, m), 7.42-7.38 (1H, m), 4.05 (1H, s), 3.78-3.70 (1H, m), 2.62 (3H, s), 1.98-1.94 (2H, m), 1.88-1.84 (2H, m), 1.37-1.28 (2H, m), 1.24-1.06 (3H, m), 1.06 (6H, s). | 394 | 392 | |
| 20 | 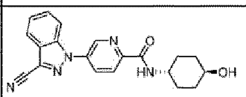 | 1H-NMR (DMSO-D6) δ: 9.11 (1H, d, J = 2.3 Hz), 8.57 (1H, d, J = 8.6 Hz), 8.50 (1H, dd, J = 8.3, 2.5 Hz), 8.28 (1H, d, J = 8.6 Hz), 8.09-8.06 (2H, m), 7.76-7.72 (1H, m), 7.59 (1H, t, J = 7.7 Hz), 4.57 (1H, d, J = 4.4 Hz), 3.84-3.74 (1H, m), 3.45-3.38 (1H, m), 1.88-1.80 (4H, m), 1.56-1.46 (2H, m), 1.32-1.23 (2H, m). | 362 | 360 | |
| 21 | 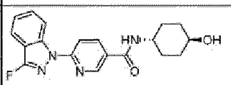 | 1H-NMR (DMSO-D6) δ: 8.97 (1H, d, J = 1.5 Hz), 8.80 (1H, d, J = 9.0 Hz), 8.40-8.37 (2H, m), 7.90-7.86 (2H, m), 7.74 (1H, t, J = 7.9 Hz), 7.45 (1H, t, J = 7.5 Hz), 4.56 (1H, d, J = 3.7 Hz), 3.78-3.72 (1H, m), 3.46-3.35 (1H, m), 1.89-1.84 (4H, m), 1.41-1.23 (4H, m). | 355 | 353 | |
| 22 | 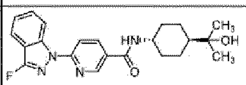 | 1H-NMR (DMSO-D6) δ: 8.98 (1H, d, J = 1.5 Hz), 8.80 (1H, d, J = 9.0 Hz), 8.42-8.38 (2H, m), 7.90-7.86 (2H, m), 7.74 (1H, t, J = 7.9 Hz), 7.46 (1H, t, J = 7.5 Hz), 4.04 (1H, s), 3.76-3.70 (1H, m), 1.93-1.85 (4H, m), 1.37-1.09 (5H, m), 1.05 (6H, s). | 397 | 395 | |
| 23 | 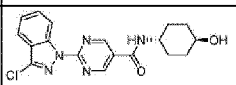 | 1H-NMR (DMSO-D6) δ: 9.25 (2H, s), 8.73 (1H, d, J = 8.8 Hz), 8.55 (1H, d, J = 7.6 Hz), 7.85 (1H, d, J = 8.1 Hz), 7.75 (1H, t, J = 7.6 Hz), 7.51 (1H, t, J = 7.6 Hz), 4.56 (1H, d, J = 4.4 Hz), 3.78-3.72 (1H, m), 3.44-3.39 (1H, m), 1.88-1.85 (4H, m), 1.37-1.27 (4H, m). | 372 | 370 | |
| 24 | 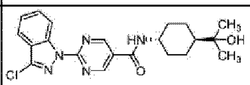 | 1H-NMR (DMSO-D6) δ: 9.26 (2H, s), 8.74 (1H, d, J = 8.8 Hz), 8.57 (1H, d, J = 7.4 Hz), 7.85 (1H, d, J = 7.9 Hz), 7.76 (1H, t, J = 7.9 Hz), 7.51 (1H, t, J = 7.5 Hz), 4.03 (1H, s), 3.74-3.69 (1H, m), 1.95-1.85 (4H, m), 1.32-1.12 (5H, m), 1.04 (6H, s). | 414 | 412 | |

[Table 1-4]

| Ex.No. | structural formula | 1H-NMR (400 MHz) | MS (positive) | MS (negative) | Comments |
|---|---|---|---|---|---|
| 25 | 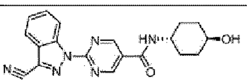 | 1H-NMR (DMSO-D6) δ: 9.34 (2H, s), 8.82 (1H, d, J = 8.2 Hz), 8.65 (1H, d, J = 7.5 Hz), 8.06 (1H, d, J = 8.2 Hz), 7.83 (1H, t, J = 8.2 Hz), 7.62 (1H, t, J = 8.2 Hz), 4.58 (1H, d, J = 4.5 Hz), 3.85-3.71 (1H, m), 3.49-3.38 (1H, m), 1.96-1.83 (4H, m), 1.47-1.20 (4H, m). | 363 | 361 | |
| 26 | 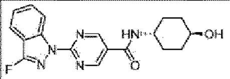 | 1H-NMR (DMSO-D6) δ: 9.23 (2H, s), 8.74 (1H, d, J = 8.2 Hz), 8.53 (1H, d, J = 7.5 Hz), 7.93 (1H, d, J = 7.5 Hz), 7.77 (1H, t, J = 7.5 Hz), 7.50 (1H, t, J = 7.5 Hz), 4.58 (1H, d, J = 3.7 Hz), 3.78-3.73 (1H, m), 3.46-3.39 (1H, m), 1.92-1.85 (4H, m), 1.41-1.25 (4H, m). | 356 | 354 | |
| 27 | 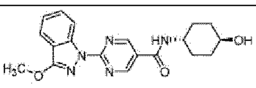 | 1H-NMR (DMSO-D6) δ: 9.18 (2H, s), 8.68 (1H, d, J = 8.2 Hz), 8.46 (1H, d, J = 7.5 Hz), 7.78 (1H, d, J = 8.2 Hz), 7.67 (1H, t, J = 7.9 Hz), 7.38 (1H, t, J = 7.5 Hz), 4.58 (1H, d, J = 4.5 Hz), 4.17 (3H, s), 3.77-3.72 (1H, m), 3.46-3.41 (1H, m), 1.89-1.87 (4H, m), 1.39-1.27 (4H, m). | 368 | 366 | |
| 28 | 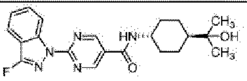 | 1H-NMR (DMSO-D6) δ: 9.24 (2H, s), 8.74 (1H, d, J = 8.2 Hz), 8.55 (1H, d, J = 8.2 Hz), 7.92 (1H, d, J = 8.2 Hz), 7.77 (1H, t, J = 8.2 Hz), 7.50 (1H, t, J = 8.2 Hz), 4.05 (1H, s), 3.78-3.70 (1H, m), 1.99-1.84 (4H, m), 1.36-1.10 (5H, m), 1.08 (6H, d, J = 17.2 Hz). | 398 | 396 | |
| 29 | 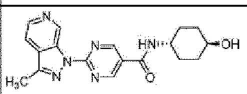 | 1H-NMR (DMSO-D6) δ: 9.99 (1H, s), 9.27 (2H, s), 8.55 (1H, d, J = 7.9 Hz), 8.53 (1H, d, J = 5.5 Hz), 7.94 (1H, d, J = 5.3 Hz), 4.57 (1H, br s), 3.83-3.70 (1H, m), 3.49-3.38 (1H, m), 2.66 (3H, s), 1.96-1.80 (4H, m), 1.48-1.17 (4H, m). | 353 | 351 | |
| 30 | 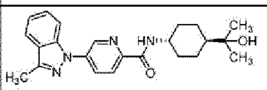 | 1H-NMR (DMSO-D6) δ: 9.08 (1H, d, J = 2.5 Hz), 8.43 (1H, d, J = 8.6 Hz), 8.39 (1H, dd, J = 8.4, 2.7 Hz), 8.20 (1H, d, J = 8.6 Hz), 7.97 (1H, d, J = 8.6 Hz), 7.91 (1H, d, J = 8.1 Hz), 7.59-7.55 (1H, m), 7.35-7.31 (1H, m), 4.04 (1H, s), 3.79-3.71 (1H, m), 2.62 (3H, s), 1.92-1.83 (4H, m), 1.47-1.38 (2H, m), 1.24-1.01 (3H, m), 1.05 (6H, s). | 393 | Not Detected | |
| 31 | 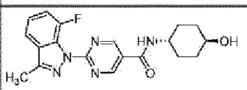 | 1H-NMR (DMSO-D6) δ: 9.22 (2H, s), 8.58 (1H, d, J = 7.6 Hz), 7.72 (1H, dd, J = 7.4, 1.2 Hz), 7.45-7.34 (2H, m), 4.57 (1H, d, J = 4.4 Hz), 3.80-3.71 (1H, m), 3.46-3.38 (1H, m), 2.61 (3H, s), 1.91-1.85 (4H, m), 1.43-1.21 (4H, m). | 370 | 368 | |
| 32 | 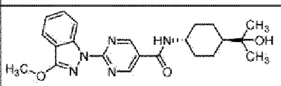 | 1H-NMR (DMSO-D6) δ: 9.19 (2H, s), 8.68 (1H, d, J = 9.0 Hz), 8.48 (1H, d, J = 7.5 Hz), 7.78 (1H, d, J = 7.5 Hz), 7.67 (1H, t, J = 7.5 Hz), 7.38 (1H, t, J = 7.5 Hz), 4.17 (3H, s), 4.05 (1H, s), 3.79-3.68 (1H, m), 1.99-1.84 (4H, m), 1.36-1.09 (5H, m), 1.06 (6H, s). | 410 | 408 | |

[Table 1-5]

| Ex.No. | structural formula | 1H-NMR (400 MHz) | MS (positive) | MS (negative) | Comments |
|---|---|---|---|---|---|
| 33 | 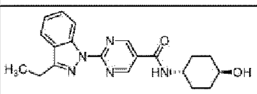 | 1H-NMR (DMSO-D6) δ: 9.22 (2H, s), 8.68 (1H, d, J = 8.2 Hz), 8.51 (1H, d, J = 7.5 Hz), 7.92 (1H, d, J = 8.2 Hz), 7.62 (1H, t, J = 7.9 Hz), 7.39 (1H, t, J = 7.5 Hz), 4.58 (1H, d, J = 4.5 Hz), 3.81-3.72 (1H, m), 3.47-3.39 (1H, m), 3.05 (2H, q, J = 7.5 Hz), 1.91-1.85 (4H, m), 1.44-1.35 (2H, m), 1.39 (3H, t, J = 7.5 Hz), 1.31-1.23 (2H, m). | 366 | 364 | |
| 34 | 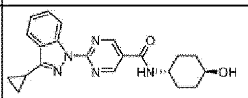 | 1H-NMR (DMSO-D6) δ: 9.20 (2H, s), 8.68 (1H, d, J = 9.0 Hz), 8.49 (1H, d, J = 8.2 Hz), 7.98 (1H, d, J = 8.2 Hz), 7.63 (1H, t, J = 7.5 Hz), 7.40 (1H, t, J = 7.5 Hz), 4.57 (1H, d, J = 4.5 Hz), 3.79-3.71 (1H, m), 3.47-3.39 (1H, m), 2.49-2.42 (1H, m), 1.90-1.86 (4H, m), 1.44-1.34 (2H, m), 1.31-1.22 (2H, m), 1.15-1.13 (4H, m). | 378 | 376 | |
| 35 | 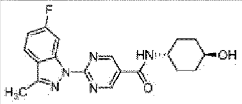 | 1H-NMR (DMSO-D6) δ: 9.23 (2H, s), 8.52 (1H, d, J = 7.5 Hz), 8.41 (1H, dd, J = 10.5, 2.2 Hz), 7.95 (1H, dd, J = 8.2, 5.2 Hz), 7.30 (1H, ddd, J = 8.2, 8.2, 2.2 Hz), 4.58 (1H, br s), 3.83-3.70 (1H, m), 3.48-3.39 (1H, m), 2.61 (3H, s), 1.95-1.82 (4H, m), 1.46-1.20 (4H, m). | 370 | 368 | |
| 36 | 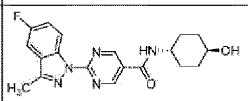 | 1H-NMR (DMSO-D6) δ: 9.21 (2H, s), 8.68 (1H, dd, J = 9.0, 4.5 Hz), 8.52 (1H, d, J = 7.5 Hz), 7.74 (1H, dd, J = 9.0, 2.2 Hz), 7.52 (1H, ddd, J = 9.0, 9.0, 2.2 Hz), 4.58 (1H, br s), 3.83-3.70 (1H, m), 3.48-3.37 (1H, m), 2.60 (3H, s), 1.95-1.83 (4H, m), 1.47-1.20 (4H, m). | 370 | 368 | |
| 37 | 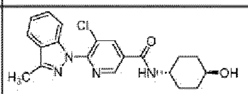 | 1H-NMR (DMSO-D6) δ: 8.93 (1H, d, J = 1.5 Hz), 8.57-8.55 (2H, m), 7.86 (1H, d, J = 7.5 Hz), 7.76 (1H, d, J = 8.2 Hz), 7.50 (1H, t, J = 7.5 Hz), 7.31 (1H, t, J = 7.5 Hz), 4.57 (1H, d, J = 4.5 Hz), 3.80-3.72 (1H, m), 3.46-3.37 (1H, m), 2.60 (3H, s), 1.89-1.86 (4H, m), 1.43-1.22 (4H, m). | 385 | 383 | |
| 38 | 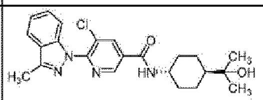 | 1H-NMR (DMSO-D6) δ: 8.94 (1H, s), 8.59-8.57 (2H, m), 7.86 (1H, d, J = 8.2 Hz), 7.77 (1H, d, J = 9.0 Hz), 7.52-7.50 (1H, m), 7.31 (1H, t, J = 7.5 Hz), 4.05 (1H, s), 3.77-3.70 (1H, m), 2.60 (3H, s), 1.97-1.84 (4H, m), 1.37-1.10 (5H, m), 1.06 (6H, s). | 427 | 425 | |
| 39 | 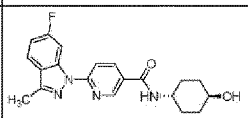 | 1H-NMR (DMSO-D6) δ: 8.97 (1H, s), 8.46 (1H, d, J = 10.5 Hz), 8.40-8.32 (2H, m), 7.99 (1H, d, J = 9.0 Hz), 7.93 (1H, dd, J = 9.0, 5.2 Hz), 7.25 (1H, t, J = 9.0 Hz), 4.56 (1H, d, J = 4.5 Hz), 3.81-3.69 (1H, m), 3.48-3.35 (1H, m), 2.61 (3H, s), 1.92-1.80 (4H, br m), 1.46-1.19 (4H, m). | 369 | 367 | |
| 40 | 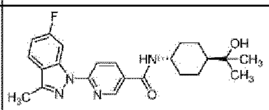 | 1H-NMR (DMSO-D6) δ: 8.98 (1H, s), 8.46 (1H, d, J = 9.7 Hz), 8.38 (2H, t, J = 6.7 Hz), 7.99 (1H, d, J = 8.2 Hz), 7.93 (1H, dd, J = 8.6, 5.6 Hz), 7.25 (1H, t, J = 8.6 Hz), 4.04 (1H, s), 3.80-3.67 (1H, m), 2.61 (3H, s), 1.99-1.89 (2H, br m), 1.89-1.79 (2H, br m), 1.40-1.25 (2H, br m), 1.25-1.00 (9H, br m). | 411 | 409 | |

[Table 1-6]

| Ex.No. | structural formula | 1H-NMR (400 MHz) | MS (positive) | MS (negative) | Comments |
|---|---|---|---|---|---|
| 41 | 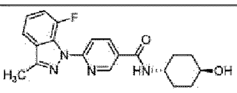 | 1H-NMR (DMSO-D6) δ: 8.92 (1H, d, J = 1.6 Hz), 8.43 (1H, d, J = 7.6 Hz), 8.37 (1H, dd, J = 8.6, 2.3 Hz), 7.88 (1H, d, J = 8.3 Hz), 7.70 (1H, d, J = 7.9 Hz), 7.41-7.36 (1H, m), 7.32 (1H, td, J = 7.9, 4.2 Hz), 4.56 (1H, d, J = 4.4 Hz), 3.79-3.71 (1H, m), 3.45-3.37 (1H, m), 2.61 (3H, s), 1.89-1.85 (4H, m), 1.44-1.34 (2H, m), 1.31-1.21 (2H, m). | 369 | 367 | |
| 42 | 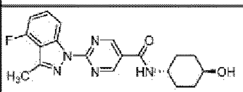 | 1H-NMR (DMSO-D6) δ: 9.23 (2H, s), 8.54 (1H, d, J = 7.5 Hz), 8.49 (1H, d, J = 9.0 Hz), 7.63-7.61 (1H, m), 7.18-7.15 (1H, m), 4.58 (1H, s), 3.81-3.70 (1H, m), 3.47-3.37 (1H, m), 2.70 (3H, s), 1.90-1.87 (4H, m), 1.44-1.23 (4H, m). | 370 | 368 | |
| 43 | 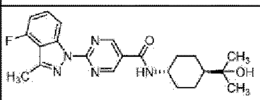 | 1H-NMR (DMSO-D6) δ: 9.24 (2H, s), 8.56 (1H, d, J = 7.5 Hz), 8.50 (1H, d, J = 9.0 Hz), 7.63-7.61 (1H, m), 7.18-7.16 (1H, m), 4.05 (1H, s), 3.77-3.70 (1H, m), 2.70 (3H, s), 1.96-1.86 (4H, m), 1.37-1.10 (5H, m), 1.06 (6H, s). | 412 | 410 | |
| 44 | 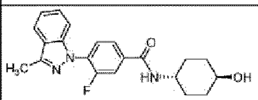 | 1H-NMR (DMSO-D6) δ: 8.39 (1H, d, J = 7.5 Hz), 7.96 (1H, d, J = 12.0 Hz), 7.88 (2H, t, J = 13.1 Hz), 7.75 (1H, t, J = 8.2 Hz), 7.49 (1H, t, J = 7.5 Hz), 7.40 (1H, dd, J = 9.0, 3.7 Hz), 7.28 (1H, t, J = 7.5 Hz), 4.57 (1H, d, J = 4.5 Hz), 3.81-3.70 (1H, m), 3.46-3.36 (1H, m), 2.60 (3H, s), 1.92-1.78 (4H, br m), 1.46-1.33 (2H, br m), 1.32-1.20 (2H, br m). | 368 | 366 | |
| 45 | 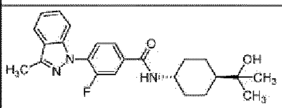 | 1H-NMR (DMSO-D6) δ: 8.41 (1H, d, J = 8.2 Hz), 7.97 (1H, d, J = 12.0 Hz), 7.92-7.84 (2H, m), 7.76 (1H, t, J = 7.9 Hz), 7.50 (1H, t, J = 7.5 Hz), 7.40 (1H, dd, J = 9.0, 3.7 Hz), 7.28 (1H, t, J = 7.5 Hz), 4.04 (1H, s), 3.79-3.68 (1H, m), 2.60 (3H, s), 1.98-1.80 (4H, br m), 1.40-1.26 (2H, br m), 1.23-1.03 (9H, br m). | 410 | 408 | |
| 46 | 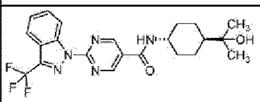 | 1H-NMR (DMSO-D6) δ: 9.34 (2H, s), 8.82 (1H, d, J = 8.8 Hz), 8.65 (1H, d, J = 7.6 Hz), 8.00-7.98 (1H, m), 7.83-7.79 (1H, m), 7.61-7.57 (1H, m), 4.05 (1H, s), 3.79-3.72 (1H, m), 2.00-1.96 (2H, m), 1.88-1.85 (2H, m), 1.38-1.28 (2H, m), 1.25-1.02 (3H, m), 1.06 (6H, s). | 448 | 446 | |
| 47 | 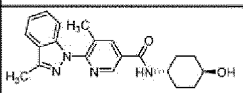 | 1H-NMR (DMSO-D6) δ: 8.80 (1H, d, J = 2.2 Hz), 8.39 (1H, d, J = 7.5 Hz), 8.27 (1H, d, J = 2.2 Hz), 7.95 (1H, d, J = 7.5 Hz), 7.84 (1H, d, J = 7.5 Hz), 7.48 (1H, t, J = 7.5 Hz), 7.29 (1H, t, J = 7.5 Hz), 4.56 (1H, d, J = 4.5 Hz), 3.81-3.72 (1H, m), 3.46-3.38 (1H, m), 2.59 (3H, s), 2.52 (3H, s), 1.88-1.86 (4H, m), 1.43-1.22 (4H, m). | 365 | 363 | |
| 48 | 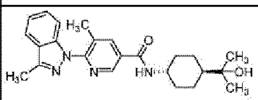 | 1H-NMR (DMSO-D6) δ: 8.81 (1H, s), 8.41 (1H, d, J = 7.5 Hz), 8.28 (1H, s), 7.95 (1H, d, J = 9.0 Hz), 7.84 (1H, d, J = 7.5 Hz), 7.49 (1H, t, J = 7.5 Hz), 7.29 (1H, t, J = 7.5 Hz), 4.04 (1H, s), 3.78-3.70 (1H, m), 2.60 (3H, s), 2.52 (3H, s), 1.94-1.85 (4H, m), 1.37-1.09 (5H, m), 1.06 (6H, s). | 407 | 405 | |

[Table 1-7]

| Ex.No. | structural formula | 1H-NMR (400 MHz) | MS (positive) | MS (negative) | Comments |
|---|---|---|---|---|---|
| 49 | 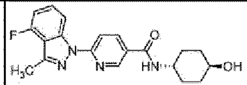 | 1H-NMR (DMSO-D6) δ: 8.96 (1H, d, J = 2.2 Hz), 8.56 (1H, d, J = 9.0 Hz), 8.40-8.36 (2H, m), 8.01 (1H, d, J = 8.2 Hz), 7.58 (1H, td, J = 8.2, 5.7 Hz), 7.12 (1H, dd, J = 10.5, 8.2 Hz), 4.56 (1H, d, J = 4.5 Hz), 3.79-3.72 (1H, m), 3.45-3.38 (1H, m), 2.70 (3H, s), 1.88-1.85 (4H, m), 1.43-1.22 (4H, m). | 369 | 367 | |
| 50 | 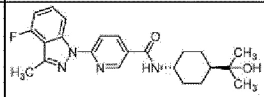 | 1H-NMR (DMSO-D6) δ: 8.98 (1H, s), 8.57 (1H, d, J = 9.0 Hz), 8.41-8.38 (2H, m), 8.01 (1H, d, J = 9.0 Hz), 7.58 (1H, q, J = 7.2 Hz), 7.12 (1H, dd, J = 10.5, 7.2 Hz), 4.04 (1H, s), 3.77-3.70 (1H, m), 2.70 (3H, s), 1.95-1.84 (4H, m), 1.37-1.09 (5H, m), 1.05 (6H, s). | 411 | 409 | |
| 51 | 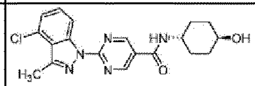 | 1H-NMR (DMSO-D6) δ: 9.23 (2H, s), 8.66 (1H, d, J = 8.6 Hz), 8.54 (1H, d, J = 7.6 Hz), 7.61-7.57 (1H, m), 7.42 (1H, d, J = 7.6 Hz), 4.58 (1H, d, J = 4.6 Hz), 3.81-3.71 (1H, m), 3.47-3.38 (1H, m), 2.78 (3H, s), 1.90-1.87 (4H, m), 1.44-1.35 (2H, m), 1.31-1.22 (2H, m). | 386 | 384 | |
| 52 | 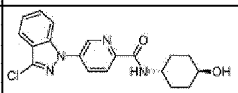 | 1H-NMR (DMSO-D6) δ: 9.06 (1H, d, J = 3.0 Hz), 8.50 (1H, d, J = 8.2 Hz), 8.42 (1H, dd, J = 9.0, 3.0 Hz), 8.22 (1H, d, J = 8.2 Hz), 8.03 (1H, d, J = 8.2 Hz), 7.87 (1H, d, J = 8.2 Hz), 7.70-7.68 (1H, m), 7.47-7.46 (1H, m), 4.57 (1H, d, J = 3.7 Hz), 3.82-3.73 (1H, m), 3.45-3.38 (1H, m), 1.87-1.80 (4H, m), 1.55-1.46 (2H, m), 1.31-1.23 (2H, m). | 371 | 369 | |
| 53 | 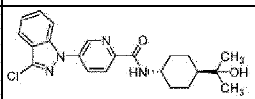 | 1H-NMR (DMSO-D6) δ: 9.07 (1H, s), 8.50 (1H, d, J = 8.2 Hz), 8.42 (1H, d, J = 8.2 Hz), 8.23 (1H, d, J = 8.2 Hz), 8.03 (1H, d, J = 8.2 Hz), 7.87 (1H, d, J = 8.2 Hz), 7.69 (1H, t, J = 8.2 Hz), 7.46 (1H, t, J = 8.2 Hz), 4.04 (1H, s), 3.79-3.70 (1H, m), 1.91-1.83 (4H, m), 1.48-1.39 (2H, m), 1.24-1.09 (3H, m), 1.05 (6H, s). | 413 | 411 | |
| 54 | 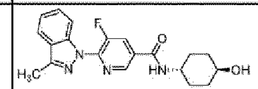 | 1H-NMR (DMSO-D6) δ: 8.84 (1H, s), 8.47 (1H, d, J = 7.5 Hz), 8.35-8.33 (1H, m), 8.20 (1H, d, J = 9.0 Hz), 7.88 (1H, d, J = 8.2 Hz), 7.56 (1H, t, J = 8.2 Hz), 7.35 (1H, dd, J = 9.0, 8.2 Hz), 4.57 (1H, d, J = 4.5 Hz), 3.80-3.70 (1H, m), 3.47-3.37 (1H, m), 2.60 (3H, s), 1.89-1.86 (4H, m), 1.43-1.22 (4H, m). | 369 | 367 | |
| 55 | 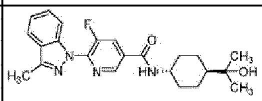 | 1H-NMR (DMSO-D6) δ: 8.85 (1H, s), 8.49 (1H, d, J = 7.5 Hz), 8.35 (1H, d, J = 12.0 Hz), 8.20 (1H, d, J = 8.2 Hz), 7.88 (1H, d, J = 7.5 Hz), 7.56 (1H, t, J = 7.9 Hz), 7.36 (1H, t, J = 7.5 Hz), 4.05 (1H, s), 3.78-3.70 (1H, m), 2.61 (3H, s), 1.96-1.84 (4H, m), 1.37-1.10 (5H, m), 1.06 (6H, s). | 411 | 409 | |
| 56 | 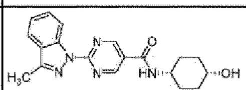 | 1H-NMR (DMSO-D6) δ: 9.24 (2H, s), 8.68 (1H, d, J = 8.2 Hz), 8.54 (1H, d, J = 7.5 Hz), 7.89 (1H, d, J = 7.5 Hz), 7.63 (1H, t, J = 7.1 Hz), 7.40 (1H, t, J = 7.5 Hz), 4.41 (1H, d, J = 3.0 Hz), 3.89-3.79 (2H, m), 2.62 (3H, s), 1.84-1.69 (4H, m), 1.62-1.48 (4H, m). | 352 | 350 | |

[Table 1-8]

| Ex.No. | structural formula | 1H-NMR (400 MHz) | MS (positive) | MS (negative) | Comments |
|---|---|---|---|---|---|
| 57 | 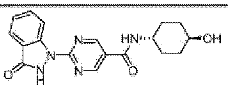 | 1H-NMR (DMSO-D6) δ: 8.95 (2H, s), 8.48 (1H, d, J = 8.8 Hz), 8.27-8.19 (1H, m), 7.46 (1H, d, J = 7.9 Hz), 7.29 (1H, dd, J = 8.8, 7.9 Hz), 7.07 (1H, dd, J = 7.9, 7.9 Hz), 4.55 (1H, d, J = 4.4 Hz), 3.79-3.65 (1H, m), 3.46-3.37 (1H, m), 1.93-1.79 (4H, m), 1.45-1.18 (4H, m). | 354 | 352 | |
| 58 | 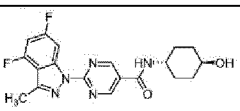 | 1H-NMR (DMSO-D6) δ: 9.24 (2H, s), 8.55 (1H, d, J = 7.6 Hz), 8.28 (1H, dd, J = 10.1, 2.0 Hz), 7.30 (1H, td, J = 10.1, 1.8 Hz), 4.58 (1H, d, J = 4.4 Hz), 3.80-3.72 (1H, m), 3.47-3.39 (1H, m), 2.67 (3H, s), 1.90-1.86 (4H, m), 1.44-1.35 (2H, m), 1.31-1.23 (2H, m). | 388 | 386 | |
| 59 | 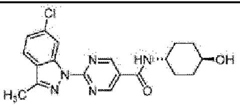 | 1H-NMR (DMSO-D6) δ: 9.24 (2H, s), 8.72 (1H, d, J = 1.8 Hz), 8.51 (1H, d, J = 7.8 Hz), 7.93 (1H, d, J = 8.5 Hz), 7.45 (1H, dd, J = 8.3, 1.8 Hz), 4.58 (1H, d, J = 4.5 Hz), 3.79-3.71 (1H, m), 3.47-3.38 (1H, m), 2.61 (3H, s), 1.90-1.86 (4H, m), 1.43-1.34 (2H, m), 1.31-1.22 (2H, m). | 386 | 384 | |
| 60 | 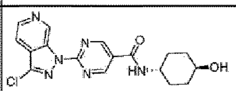 | 1H-NMR (DMSO-D6) δ: 10.07 (1H, s), 9.31 (2H, s), 8.63 (1H, d, J = 6.0 Hz), 8.61 (1H, d, J = 7.5 Hz), 7.92 (1H, d, J = 6.0 Hz), 4.58 (1H, d, J = 4.5 Hz), 3.82-3.71 (1H, m), 3.48-3.39 (1H, m), 1.90-1.87 (4H, m), 1.44-1.23 (4H, m). | 373 | 371 | |
| 61 | 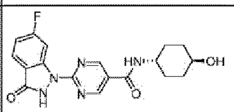 | 1H-NMR (DMSO-D6) δ: 9.11 (2H, s), 8.39 (1H, d, J = 7.6 Hz), 8.32 (1H, dd, J = 10.8, 2.0 Hz), 7.85-7.76 (1H, m), 7.12 (1H, t, J = 8.8 Hz), 4.58 (1H, br s), 3.81-3.69 (1H, m), 3.50-3.18 (1H, m), 1.93-1.80 (4H, m), 1.46-1.17 (4H, m). | 372 | 370 | |
| 62 | 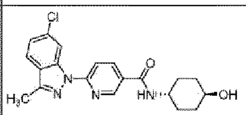 | 1H-NMR (DMSO-D6) δ: 8.99 (1H, d, J = 1.5 Hz), 8.79 (1H, s), 8.36 (2H, d, J = 7.5 Hz), 7.99 (1H, d, J = 9.0 Hz), 7.91 (1H, d, J = 8.2 Hz), 7.40 (1H, d, J = 8.2 Hz), 4.57 (1H, br s), 3.80-3.71 (1H, m), 3.45-3.39 (1H, m), 2.62 (3H, s), 1.89-1.84 (4H, m), 1.43-1.34 (2H, m), 1.31-1.21 (2H, m). | 385 | 383 | |
| 63 | 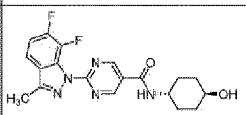 | 1H-NMR (DMSO-D6) δ: 9.23 (2H, s), 8.58 (1H, d, J = 8.2 Hz), 7.75 (1H, dd, J = 9.0, 4.5 Hz), 7.47 (1H, td, J = 9.3, 6.0 Hz), 4.58 (1H, d, J = 3.7 Hz), 3.80-3.72 (1H, m), 3.47-3.39 (1H, m), 2.60 (3H, s), 1.90-1.87 (4H, m), 1.44-1.34 (2H, m), 1.31-1.23 (2H, m). | 388 | 386 | |
| 64 | 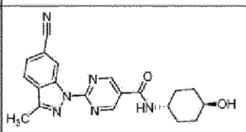 | 1H-NMR (DMSO-D6) δ: 9.26 (2H, s), 9.10 (1H, s), 8.55 (1H, d, J = 7.5 Hz), 8.13 (1H, d, J = 8.2 Hz), 7.78 (1H, d, J = 8.2 Hz), 4.58 (1H, d, J = 3.7 Hz), 3.81-3.71 (1H, m), 3.46-3.39 (1H, m), 2.67 (3H, s), 1.90-1.87 (4H, m), 1.44-1.23 (4H, m). | 377 | 375 | |

[Table 1-9]

| Ex.No. | structural formula | 1H-NMR (400 MHz) | MS (positive) | MS (negative) | Comments |
|---|---|---|---|---|---|
| 65 | 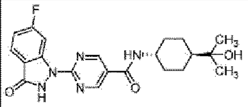 | 1H-NMR (DMSO-D6) δ: 9.09-8.93 (2H, m), 8.36-8.24 (1H, m), 8.22-8.12 (1H, m), 7.59-7.48 (1H, m), 7.09-6.97 (1H, m), 4.02 (1H, s), 3.71-3.57 (1H, m), 1.97-1.74 (4H, m), 1.33-1.07 (5H, m), 1.04 (6H, s). | 414 | 412 | |
| 66 | 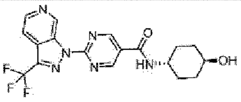 | 1H-NMR (DMSO-D6) δ: 10.16 (1H, s), 9.37 (2H, s), 8.68-8.67 (2H, m), 8.02 (1H, d, J = 5.2 Hz), 4.58 (1H, d, J = 4.5 Hz), 3.82-3.74 (1H, m), 3.47-3.39 (1H, m), 1.91-1.88 (4H, m), 1.45-1.24 (4H, m). | 407 | 405 | |
| 67 | 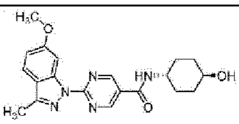 | 1H-NMR (DMSO-D6) δ: 9.22 (2H, s), 8.49 (1H, d, J = 7.6 Hz), 8.20 (1H, d, J = 2.2 Hz), 7.75 (1H, d, J = 8.8 Hz), 7.01 (1H, dd, J = 8.8, 2.2 Hz), 4.57 (1H, d, J = 3.9 Hz), 3.90 (3H, s), 3.81-3.71 (1H, m), 3.47-3.38 (1H, m), 2.55 (3H, s), 1.89-1.86 (4H, m), 1.43-1.22 (4H, m). | 382 | 380 | |
| 68 | 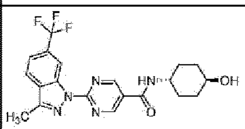 | 1H-NMR (DMSO-D6) δ: 9.27 (2H, s), 9.02-9.02 (1H, m), 8.50 (1H, d, J = 7.6 Hz), 8.15 (1H, d, J = 8.4 Hz), 7.72 (1H, dd, J = 8.4, 1.3 Hz), 4.58 (1H, br s), 3.80-3.72 (1H, m), 3.46-3.39 (1H, m), 2.68 (3H, s), 1.90-1.87 (4H, m), 1.43-1.23 (4H, m). | 420 | 418 | |
| 69 | 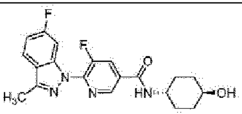 | 1H-NMR (DMSO-D6) δ: 8.84 (1H, s), 8.48 (1H, d, J = 8.2 Hz), 8.35 (1H, d, J = 12.0 Hz), 7.97 (1H, dd, J = 9.7, 2.2 Hz), 7.93 (1H, dd, J = 9.0, 5.2 Hz), 7.28-7.22 (1H, m), 4.57 (1H, d, J = 4.5 Hz), 3.80-3.71 (1H, m), 3.46-3.38 (1H, m), 2.59 (3H, s), 1.88-1.86 (4H, m), 1.43-1.22 (4H, m). | 387 | 385 | |
| 70 | 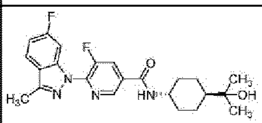 | 1H-NMR (DMSO-D6) δ: 8.85 (1H, s), 8.50 (1H, d, J = 8.2 Hz), 8.36 (1H, d, J = 12.0 Hz), 8.00-7.92 (2H, m), 7.27-7.23 (1H, m), 4.05 (1H, s), 3.79-3.70 (1H, m), 2.60 (3H, s), 1.96-1.84 (4H, m), 1.36-1.09 (5H, m), 1.06 (6H, s). | 429 | 427 | |
| 71 | 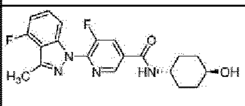 | 1H-NMR (DMSO-D6) δ: 8.84 (1H, s), 8.48 (1H, d, J = 7.5 Hz), 8.35 (1H, d, J = 12.0 Hz), 7.99-7.92 (2H, m), 7.27-7.23 (1H, m), 4.57 (1H, d, J = 4.5 Hz), 3.80-3.71 (1H, m), 3.46-3.38 (1H, m), 2.59 (3H, s), 1.89-1.86 (4H, m), 1.43-1.22 (4H, m). | 387 | 385 | |
| 72 | 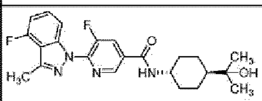 | 1H-NMR (DMSO-D6) δ: 8.85 (1H, s), 8.49 (1H, d, J = 8.2 Hz), 8.36 (1H, d, J = 12.0 Hz), 7.99-7.92 (2H, m), 7.27-7.22 (1H, m), 4.05 (1H, s), 3.77-3.69 (1H, m), 2.59 (3H, s), 1.96-1.84 (4H, m), 1.38-1.06 (5H, m), 1.05 (6H, s). | 429 | 427 | |

[Table 1-10]

| Ex.No. | structural formula | 1H-NMR (400 MHz) | MS (positive) | MS (negative) | Comments |
|---|---|---|---|---|---|
| 73 | 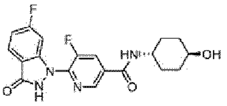 | 1H-NMR (DMSO-D6) δ: 11.93 (1H, br s), 8.78 (1H, s), 8.45-8.33 (1H, m), 8.25 (1H, d, J = 12.0 Hz), 8.10-7.93 (1H, m), 7.89-7.73 (1H, m), 7.27-7.06 (1H, m), 4.56 (1H, d, J = 3.7 Hz), 3.82-3.66 (1H, m), 3.48-3.36 (1H, m), 1.96-1.74 (4H, m), 1.46-1.13 (4H, m). | 389 | 387 | |
| 74 | 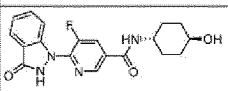 | 1H-NMR (DMSO-D6) δ: 12.73 (1H, s), 8.36-8.25 (3H, m), 7.52 (1H, d, J = 8.2 Hz), 7.40 (2H, t, J = 7.9 Hz), 7.08 (1H, t, J = 7.5 Hz), 4.55 (1H, d, J = 3.7 Hz), 3.76-3.64 (1H, m), 3.44-3.34 (1H, m), 1.90-1.77 (4H, m), 1.42-1.16 (4H, m). | 371 | 369 | |
| 75 | 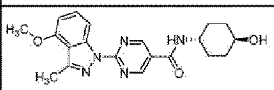 | 1H-NMR (DMSO-D6) δ: 9.20 (2H, s), 8.50 (1H, d, J = 7.6 Hz), 8.22 (1H, d, J = 8.3 Hz), 7.51 (1H, t, J = 8.2 Hz), 6.84 (1H, d, J = 7.9 Hz), 4.57 (1H, d, J = 4.2 Hz), 3.96 (3H, s), 3.82-3.69 (1H, m), 3.48-3.37 (1H, m), 2.66 (3H, s), 1.93-1.82 (4H, m), 1.47-1.17 (4H, m). | 382 | 380 | |
| 76 | 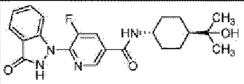 | 1H-NMR (DMSO-D6) δ: 12.73 (1H, s), 8.38-8.26 (3H, m), 7.52 (1H, d, J = 8.6 Hz), 7.40 (2H, t, J = 7.6 Hz), 7.08 (1H, t, J = 7.5 Hz), 4.03 (1H, br s), 3.75-3.61 (1H, m), 1.96-1.77 (4H, m), 1.35-1.06 (5H, m), 1.04 (6H, s). | 413 | 411 | |
| 77 | 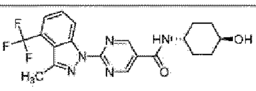 | 1H-NMR (DMSO-D6) δ: 9.26 (2H, s), 9.07 (1H, d, J = 7.2 Hz), 8.57 (1H, d, J = 7.6 Hz), 7.85-7.77 (2H, m), 4.58 (1H, d, J = 4.4 Hz), 3.83-3.71 (1H, m), 3.48-3.37 (1H, m), 2.68 (3H, s), 1.95-1.82 (4H, m), 1.48-1.17 (4H, m). | 420 | 418 | |
| 78 | 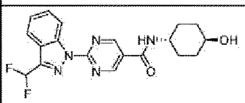 | 1H-NMR (DMSO-D6) δ: 9.30 (2H, s), 8.78 (1H, d, J = 9.0 Hz), 8.60 (1H, d, J = 8.2 Hz), 8.00 (1H, d, J = 8.2 Hz), 7.75 (1H, t, J = 7.9 Hz), 7.70-7.42 (2H, m), 4.58 (1H, d, J = 4.5 Hz), 3.81-3.73 (1H, m), 3.46-3.39 (1H, m), 1.90-1.87 (4H, m), 1.44-1.35 (2H, m), 1.31-1.23 (2H, m). | 388 | 386 | |
| 79 | 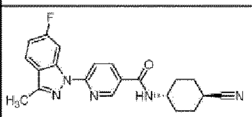 | 1H-NMR (DMSO-D6) δ: 8.97 (1H, d, J = 2.2 Hz), 8.49-8.44 (2H, m), 8.36 (1H, dd, J = 8.2, 2.2 Hz), 8.00 (1H, d, J = 8.2 Hz), 7.93 (1H, dd, J = 8.2, 5.2 Hz), 7.25 (1H, td, J = 9.0, 2.2 Hz), 3.90-3.78 (1H, m), 2.76-2.67 (1H, m), 2.61 (3H, s), 2.13-2.04 (2H, m), 1.95-1.87 (2H, m), 1.70-1.58 (0H, m), 1.45-1.35 (2H, m). | 378 | 376 | |
| 80 | 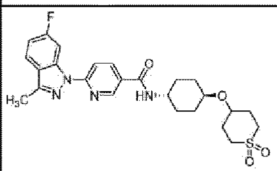 | 1H-NMR (DMSO-D6) δ: 8.97 (1H, d, J = 2.2 Hz), 8.48-8.35 (3H, m), 8.00 (1H, d, J = 8.2 Hz), 7.93 (1H, dd, J = 9.0, 5.2 Hz), 7.25 (1H, td, J = 9.0, 2.2 Hz), 3.84-3.73 (2H, m), 3.43-3.35 (1H, m), 3.15-3.01 (4H, m), 2.62 (3H, s), 2.08-1.87 (8H, m), 1.47-1.24 (4H, m). | 501 | 499 | |

[Table 1-11]

| Ex.No. | structural formula | 1H-NMR (400 MHz) | MS (positive) | MS (negative) | Comments |
|---|---|---|---|---|---|
| 81 | 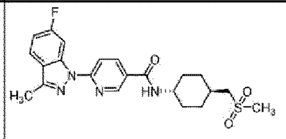 | 1H-NMR (DMSO-D6) δ: 8.98 (1H, d, J = 2.2 Hz), 8.49-8.42 (2H, m), 8.37 (1H, dd, J = 8.2, 2.2 Hz), 8.00 (1H, d, J = 8.2 Hz), 7.93 (1H, dd, J = 9.0, 5.2 Hz), 7.25 (1H, td, J = 9.0, 2.2 Hz), 3.82-3.72 (1H, m), 3.09 (2H, d, J = 6.0 Hz), 2.99 (3H, s), 2.62 (3H, s), 2.01-1.87 (5H, m), 1.46-1.35 (2H, m), 1.28-1.16 (2H, m). | 445 | 443 | |
| 82 | 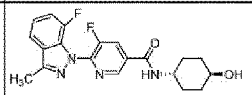 | 1H-NMR (DMSO-D6) δ: 8.82 (1H, s), 8.56 (1H, d, J = 7.5 Hz), 8.38 (1H, d, J = 10.5 Hz), 7.73 (1H, d, J = 8.2 Hz), 7.40-7.28 (2H, m), 4.57 (1H, s), 3.79-3.71 (1H, m), 3.46-3.39 (1H, m), 2.61 (3H, s), 1.89-1.87 (4H, m), 1.43-1.22 (4H, m). | 387 | 385 | |
| 83 | 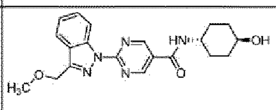 | 1H-NMR (DMSO-D6) δ: 9.25 (2H, s), 8.70 (1H, d, J = 8.8 Hz), 8.54 (1H, d, J = 7.9 Hz), 7.95 (1H, d, J = 7.9 Hz), 7.68-7.62 (1H, m), 7.42 (1H, td, J = 7.6, 0.7 Hz), 4.87 (2H, s), 4.58 (1H, d, J = 4.4 Hz), 3.83-3.71 (1H, m), 3.49-3.38 (1H, m), 3.38 (3H, s), 1.93-1.85 (4H, br m), 1.45-1.33 (2H, br m), 1.33-1.21 (2H, br m). | 382 | 380 | |
| 84 | 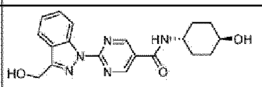 | 1H-NMR (DMSO-D6) δ: 9.23 (2H, s), 8.69 (1H, d, J = 8.6 Hz), 8.52 (1H, d, J = 7.6 Hz), 8.02 (1H, d, J = 7.9 Hz), 7.65-7.61 (1H, m), 7.40 (1H, t, J = 7.2 Hz), 5.57 (1H, t, J = 6.0 Hz), 4.90 (2H, d, J = 5.8 Hz), 4.57 (1H, d, J = 4.4 Hz), 3.81-3.71 (1H, m), 3.48-3.38 (1H, m), 1.93-1.82 (4H, br m), 1.46-1.33 (2H, br m), 1.33-1.21 (2H, br m). | 368 | 366 | |
| 85 | 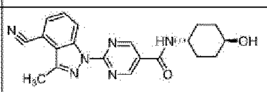 | 1H-NMR (DMSO-D6) δ: 9.25 (2H, s), 9.01 (1H, d, J = 8.2 Hz), 8.57 (1H, d, J = 8.2 Hz), 7.94 (1H, d, J = 6.7 Hz), 7.79 (1H, t, J = 7.9 Hz), 4.59 (1H, d, J = 4.5 Hz), 3.82-3.70 (1H, m), 3.49-3.39 (1H, m), 2.79 (3H, s), 1.95-1.83 (4H, m), 1.49-1.19 (4H, m). | 377 | 375 | |
| 86 | 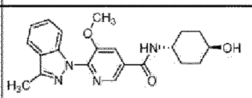 | 1H-NMR (DMSO-D6) δ: 8.58 (1H, d, J = 1.8 Hz), 8.47 (1H, d, J = 7.6 Hz), 8.05 (1H, d, J = 1.8 Hz), 7.81 (1H, d, J = 8.3 Hz), 7.47 (1H, d, J = 8.1 Hz), 7.42 (1H, ddd, J = 8.3, 8.3, 1.9 Hz), 7.24 (1H, ddd, J = 8.3, 8.1, 1.9 Hz), 4.58 (1H, br s), 3.90 (3H, s), 3.84-3.73 (1H, m), 3.48-3.38 (1H, m), 2.57 (3H, s), 1.94-1.83 (4H, m), 1.50-1.20 (4H, m). | 381 | 379 | |
| 87 | 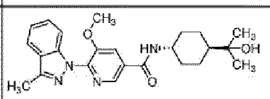 | 1H-NMR (DMSO-D6) δ: 8.59 (1H, d, J = 1.8 Hz), 8.50 (1H, d, J = 7.9 Hz), 8.06 (1H, d, J = 1.8 Hz), 7.81 (1H, d, J = 7.9 Hz), 7.46 (1H, t, J = 7.9 Hz), 7.42 (1H, td, J = 7.9, 1.5 Hz), 7.24 (1H, ddd, J = 7.9, 7.9, 1.5 Hz), 4.05 (1H, s), 3.91 (3H, s), 3.85-3.71 (1H, m), 2.57 (3H, s), 2.02-1.81 (4H, m), 1.41-1.09 (5H, m), 1.06 (6H, s). | 423 | 421 | |
| 88 | 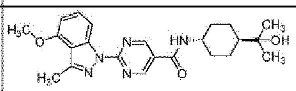 | 1H-NMR (DMSO-D6) δ: 9.21 (2H, s), 8.52 (1H, d, J = 7.9 Hz), 8.23 (1H, d, J = 8.2 Hz), 7.51 (1H, dd, J = 8.2, 8.2 Hz), 6.84 (1H, d, J = 8.2 Hz), 4.05 (1H, br s), 3.96 (3H, s), 3.80-3.67 (1H, m), 2.66 (3H, s), 2.01-1.80 (4H, m), 1.39-1.08 (5H, m), 1.05 (6H, s). | 424 | 422 | |

[Table 1-12]

| Ex.No. | structural formula | 1H-NMR (400 MHz) | MS (positive) | MS (negative) | Comments |
|---|---|---|---|---|---|
| 89 | 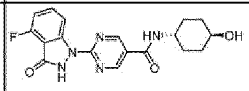 | 1H-NMR (DMSO-D6) δ: 9.03 (2H, s), 8.40 (1H, d, J = 6.9 Hz), 8.33 (1H, d, J = 8.3 Hz), 7.26 (1H, ddd, J = 8.3, 8.3, 5.4 Hz), 6.74 (1H, dd, J = 9.6, 8.3 Hz), 4.56 (1H, d, J = 4.4 Hz), 3.79-3.67 (1H, m), 3.48-3.38 (1H, m), 1.93-1.79 (4H, m), 1.50-1.16 (4H, m). | 372 | 370 | |
| 90 | 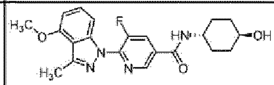 | 1H-NMR (DMSO-D6) δ: 8.82 (1H, d, J = 1.2 Hz), 8.47 (1H, d, J = 7.6 Hz), 8.33 (1H, dd, J = 11.8, 1.8 Hz), 7.68 (1H, d, J = 8.3 Hz), 7.43 (1H, t, J = 8.1 Hz), 6.77 (1H, d, J = 7.9 Hz), 4.56 (1H, br s), 3.95 (3H, s), 3.81-3.70 (1H, m), 3.47-3.38 (1H, m), 2.65 (3H, s), 1.93-1.81 (4H, m), 1.46-1.19 (4H, m). | 399 | 397 | |
| 91 | 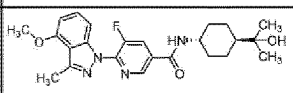 | 1H-NMR (DMSO-D6) δ: 8.83 (1H, d, J = 1.4 Hz), 8.49 (1H, d, J = 7.9 Hz), 8.34 (1H, dd, J = 11.8, 1.8 Hz), 7.68 (1H, d, J = 8.3 Hz), 7.43 (1H, t, J = 8.2 Hz), 6.77 (1H, d, J = 7.9 Hz), 4.04 (1H, s), 3.95 (3H, s), 3.79-3.67 (1H, m), 2.65 (3H, s), 2.00-1.80 (4H, m), 1.39-1.08 (5H, m), 1.05 (6H, s). | 441 | 439 | |
| 92 | 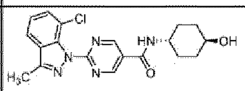 | 1H-NMR (DMSO-D6) δ: 9.27 (2H, s), 8.64 (1H, d, J = 7.9 Hz), 7.89 (1H, dd, J = 7.9, 0.9 Hz), 7.63 (1H, dd, J = 7.6, 0.9 Hz), 7.38-7.34 (1H, m), 4.58 (1H, d, J = 4.2 Hz), 3.81-3.72 (1H, m), 3.46-3.38 (1H, m), 2.60 (3H, s), 1.92-1.86 (4H, m), 1.44-1.22 (4H, m). | 386 | 384 | |
| 93 | 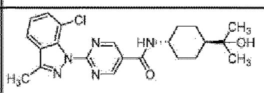 | 1H-NMR (DMSO-D6) δ: 9.28 (2H, s), 8.67 (1H, d, J = 7.9 Hz), 7.90 (1H, dd, J = 8.0, 0.9 Hz), 7.64 (1H, dd, J = 7.6, 0.9 Hz), 7.38-7.34 (1H, m), 4.05 (1H, s), 3.79-3.69 (1H, m), 2.60 (3H, s), 1.99-1.95 (2H, m), 1.88-1.84 (2H, m), 1.37-1.04 (5H, m), 1.06 (6H, s). | 428 | 426 | |
| 94 | 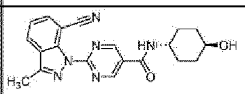 | 1H-NMR (DMSO-D6) δ: 9.29 (2H, s), 8.66 (1H, d, J = 7.9 Hz), 8.29 (1H, dd, J = 8.0, 1.0 Hz), 8.14 (1H, dd, J = 7.5, 1.0 Hz), 7.56-7.52 (1H, m), 4.58 (1H, d, J = 4.6 Hz), 3.82-3.73 (1H, m), 3.46-3.38 (1H, m), 2.66 (3H, d, J = 8.8 Hz), 1.92-1.85 (4H, m), 1.45-1.35 (2H, m), 1.32-1.22 (2H, m). | 377 | 375 | |
| 95 | 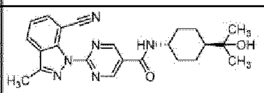 | 1H-NMR (DMSO-D6) δ: 9.30 (2H, s), 8.68 (1H, d, J = 7.6 Hz), 8.29 (1H, dd, J = 7.9, 1.1 Hz), 8.14 (1H, dd, J = 7.4, 1.1 Hz), 7.56-7.52 (1H, m), 4.05 (1H, s), 3.81-3.71 (1H, m), 2.65 (3H, s), 1.99-1.95 (2H, m), 1.89-1.83 (2H, m), 1.38-1.28 (2H, m), 1.25-1.02 (3H, m), 1.06 (6H, s). | 419 | 417 | |
| 96 | 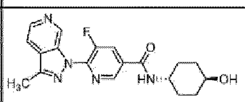 | 1H-NMR (DMSO-D6) δ: 9.67 (1H, d, J = 1.5 Hz), 8.88 (1H, d, J = 1.5 Hz), 8.51-8.48 (2H, m), 8.38 (1H, dd, J = 12.0, 1.5 Hz), 7.93-7.92 (1H, m), 4.58 (1H, d, J = 4.5 Hz), 3.80-3.72 (1H, m), 3.46-3.39 (1H, m), 2.65 (3H, s), 1.89-1.86 (4H, m), 1.44-1.35 (2H, m), 1.32-1.22 (2H, m). | 370 | 368 | |

[Table 1-13]

| Ex.No. | structural formula | 1H-NMR (400 MHz) | MS (positive) | MS (negative) | Comments |
|---|---|---|---|---|---|
| 97 | 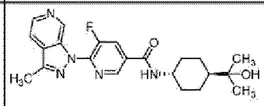 | 1H-NMR (DMSO-D6) δ: 9.67 (1H, s), 8.89 (1H, s), 8.52 (1H, d, J = 7.5 Hz), 8.49 (1H, d, J = 6.0 Hz), 8.39 (1H, d, J = 12.0 Hz), 7.93 (1H, d, J = 5.2 Hz), 4.05 (1H, s), 3.78-3.70 (1H, m), 2.65 (3H, s), 1.97-1.93 (2H, m), 1.88-1.84 (2H, m), 1.37-1.28 (2H, m), 1.24-1.02 (3H, m), 1.06 (6H, s). | 412 | 410 | |
| 98 | 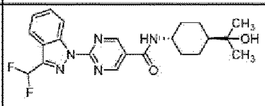 | 1H-NMR (DMSO-D6) δ: 9.31 (2H, s), 8.78 (1H, d, J = 8.6 Hz), 8.62 (1H, d, J = 7.6 Hz), 8.00 (1H, d, J = 8.1 Hz), 7.75 (1H, t, J = 7.9 Hz), 7.56 (1H, t, J = 53.4 Hz), 7.52 (1H, t, J = 7.5 Hz), 4.05 (1H, s), 3.79-3.71 (1H, m), 1.99-1.95 (2H, m), 1.88-1.84 (2H, m), 1.38-1.28 (2H, m), 1.24-1.07 (3H, m), 1.06 (6H, s). | 430 | 428 | |
| 99 | 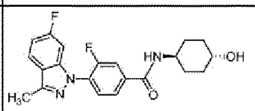 | 1H-NMR (DMSO-D6) δ: 8.40 (1H, d, J = 7.5 Hz), 7.96 (1H, d, J = 12.0 Hz), 7.93-7.87 (2H, m), 7.76 (1H, t, J = 7.9 Hz), 7.23 (1H, d, J = 9.7 Hz), 7.16 (1H, t, J = 9.3 Hz), 4.57 (1H, d, J = 4.5 Hz), 3.81-3.70 (1H, m), 3.47-3.37 (1H, m), 2.58 (3H, s), 1.92-1.80 (4H, br m), 1.46-1.34 (2H, br m), 1.32-1.20 (2H, br m). | 386 | 384 | |
| 100 | 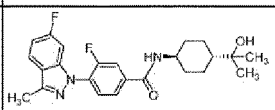 | 1H-NMR (DMSO-D6) δ: 8.41 (1H, d, J = 8.2 Hz), 7.97 (1H, d, J = 12.0 Hz), 7.94-7.87 (2H, m), 7.76 (1H, t, J = 7.9 Hz), 7.23 (1H, d, J = 9.7 Hz), 7.16 (1H, t, J = 9.3 Hz), 4.04 (1H, s), 3.79-3.68 (1H, m), 2.59 (3H, s), 1.96-1.81 (4H, br m), 1.39-1.28 (2H, br m), 1.22-1.03 (9H, br m). | 428 | 426 | |
| 101 | 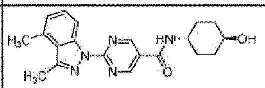 | 1H-NMR (DMSO-D6) δ: 9.20 (2H, s), 8.53 (1H, d, J = 7.9 Hz), 8.50 (1H, d, J = 10.5 Hz), 7.46 (1H, dd, J = 7.9, 7.9 Hz), 7.12 (1H, d, J = 7.9 Hz), 3.83-3.70 (1H, m), 3.49-3.26 (2H, m), 2.75 (3H, s), 2.72 (3H, s), 1.93-1.83 (4H, m), 1.46-1.20 (4H, m). | 366 | 364 | |
| 102 | 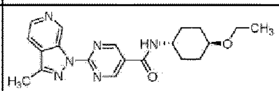 | 1H-NMR (DMSO-D6) δ: 9.98 (1H, d, J = 1.2 Hz), 9.27 (2H, s), 8.58 (1H, d, J = 7.6 Hz), 8.53 (1H, d, J = 5.3 Hz), 7.94 (1H, dd, J = 5.3, 1.2 Hz), 3.86-3.75 (1H, m), 3.47 (2H, q, J = 7.0 Hz), 3.38-3.20 (1H, m), 2.66 (3H, s), 2.08-1.88 (4H, m), 1.47-1.20 (4H, m), 1.10 (3H, t, J = 7.0 Hz). | 381 | 379 | |
| 103 | 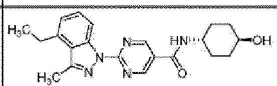 | 1H-NMR (DMSO-D6) δ: 9.21 (2H, s), 8.56 (1H, d, J = 8.3 Hz), 8.50 (1H, d, J = 7.6 Hz), 7.51 (1H, dd, J = 8.3, 7.4 Hz), 7.17 (1H, d, J = 7.4 Hz), 3.82-3.71 (1H, m), 3.54 (1H, br s), 3.46-3.38 (1H, m), 3.09 (2H, q, J = 7.6 Hz), 2.75 (3H, s), 1.93-1.83 (4H, m), 1.46-1.22 (4H, m), 1.30 (3H, t, J = 7.6 Hz). | 380 | 378 | |
| 104 | 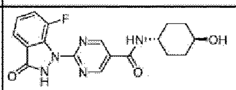 | 1H-NMR (DMSO-D6) δ: 13.38 (1H, s), 8.99 (2H, s), 8.43 (1H, d, J = 7.6 Hz), 7.31-7.20 (2H, m), 7.05 (1H, td, J = 7.9, 4.2 Hz), 4.55 (1H, d, J = 4.4 Hz), 3.78-3.65 (1H, m), 3.46-3.35 (1H, m), 1.92-1.77 (4H, m), 1.44-1.15 (4H, m). | 372 | 370 | |

[Table 1-14]

| Ex.No. | structural formula | 1H-NMR (400 MHz) | MS (positive) | MS (negative) | Comments |
|---|---|---|---|---|---|
| 105 | 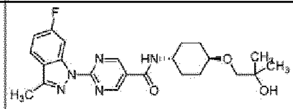 | 1H-NMR (DMSO-D6) δ: 9.23 (2H, s), 8.54 (1H, d, J = 7.6 Hz), 8.40 (1H, dd, J = 10.4, 2.3 Hz), 7.94 (1H, dd, J = 8.7, 5.4 Hz), 7.32-7.27 (1H, m), 4.22 (1H, s), 3.85-3.76 (1H, m), 3.32-3.22 (1H, m), 3.19 (2H, s), 2.61 (3H, s), 2.06-2.01 (2H, m), 1.95-1.91 (2H, m), 1.45-1.35 (2H, m), 1.32-1.23 (2H, m), 1.07 (6H, s). | 442 | 440 | |
| 106 | 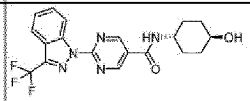 | 1H-NMR (DMSO-D6) δ: 9.33 (2H, s), 8.82 (1H, d, J = 8.6 Hz), 8.64 (1H, d, J = 7.6 Hz), 7.99 (1H, d, J = 8.1 Hz), 7.83-7.79 (1H, m), 7.61-7.57 (1H, m), 4.59 (1H, d, J = 4.4 Hz), 3.83-3.73 (1H, m), 3.48-3.40 (1H, m), 1.92-1.87 (4H, m), 1.46-1.35 (2H, m), 1.33-1.23 (2H, m). | 406 | 404 | |
| 107 | 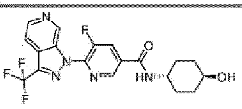 | 1H-NMR (DMSO-D6) δ: 9.77 (1H, s), 8.96 (1H, s), 8.64 (1H, d, J = 5.2 Hz), 8.61 (1H, d, J = 7.5 Hz), 8.51 (1H, d, J = 12.0 Hz), 8.01 (1H, d, J = 5.2 Hz), 4.59 (1H, s), 3.82-3.74 (1H, m), 3.46-3.39 (1H, m), 1.90-1.87 (4H, m), 1.45-1.23 (4H, m). | 424 | 422 | |
| 108 | 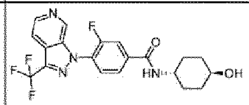 | 1H-NMR (DMSO-D6) δ: 9.18 (1H, d, J = 1.5 Hz), 8.60 (1H, d, J = 6.0 Hz), 8.50 (1H, d, J = 7.5 Hz), 8.07 (1H, d, J = 11.2 Hz), 8.03-7.96 (3H, m), 3.80-3.73 (1H, m), 3.45-3.39 (1H, m), 1.88-1.86 (4H, m), 1.45-1.22 (4H, m). | 423 | 421 | |
| 109 | 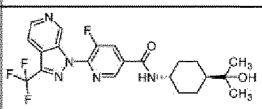 | 1H-NMR (DMSO-D6) δ: 9.77 (1H, s), 8.97 (1H, s), 8.65-8.62 (2H, m), 8.51 (1H, d, J = 11.2 Hz), 8.01 (1H, d, J = 5.2 Hz), 4.05 (1H, s), 3.80-3.72 (1H, m), 1.98-1.85 (4H, m), 1.38-1.10 (5H, m), 1.06 (6H, s). | 466 | 464 | |
| 110 | 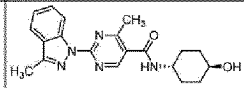 | 1H-NMR (DMSO-D6) δ: 8.74 (1H, s), 8.68 (1H, d, J = 8.3 Hz), 8.44 (1H, d, J = 7.9 Hz), 7.87 (1H, d, J = 8.1 Hz), 7.61 (1H, t, J = 7.9 Hz), 7.37 (1H, t, J = 7.5 Hz), 4.56 (1H, d, J = 4.4 Hz), 3.74-3.68 (1H, m), 3.46-3.38 (1H, m), 2.64 (3H, s), 2.61 (3H, s), 1.92-1.85 (4H, m), 1.37-1.22 (4H, m). | 366 | 364 | |
| 111 | 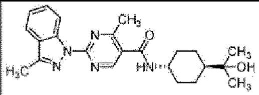 | 1H-NMR (DMSO-D6) δ: 8.75 (1H, s), 8.68 (1H, d, J = 8.6 Hz), 8.45 (1H, d, J = 7.9 Hz), 7.87 (1H, d, J = 7.9 Hz), 7.61 (1H, t, J = 7.7 Hz), 7.38 (1H, t, J = 7.5 Hz), 4.04 (1H, s), 3.72-3.65 (1H, m), 2.65 (3H, s), 2.61 (3H, s), 2.00-1.96 (2H, m), 1.86-1.84 (2H, m), 1.30-1.08 (5H, m), 1.05 (6H, s). | 408 | 406 | |
| 112 | 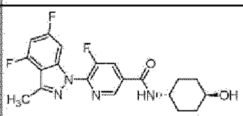 | 1H-NMR (DMSO-D6) δ: 8.84 (1H, d, J = 1.6 Hz), 8.50 (1H, d, J = 7.6 Hz), 8.37 (1H, dd, J = 11.8, 1.6 Hz), 7.81 (1H, dd, J = 9.5, 1.8 Hz), 7.23 (1H, td, J = 10.0, 1.7 Hz), 4.57 (1H, d, J = 4.4 Hz), 3.79-3.72 (1H, m), 3.46-3.38 (1H, m), 2.65 (3H, s), 1.89-1.85 (4H, m), 1.44-1.34 (2H, m), 1.31-1.22 (2H, m). | 405 | 403 | |

[Table 1-15]

| Ex.No. | structural formula | 1H-NMR (400 MHz) | MS (positive) | MS (negative) | Comments |
|---|---|---|---|---|---|
| 113 | 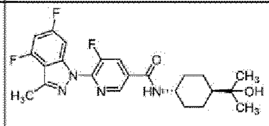 | 1H-NMR (DMSO-D6) δ: 8.85 (1H, d, J = 1.2 Hz), 8.52 (1H, d, J = 7.6 Hz), 8.38 (1H, dd, J = 11.7, 1.7 Hz), 7.81 (1H, dd, J = 9.5, 1.8 Hz), 7.24 (1H, td, J = 10.0, 1.8 Hz), 4.05 (1H, s), 3.77-3.70 (1H, m), 2.66 (3H, s), 1.96-1.93 (2H, m), 1.87-1.84 (2H, m), 1.37-1.27 (2H, m), 1.23-1.05 (3H, m), 1.05 (6H, s). | 447 | 445 | |
| 114 | 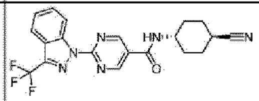 | 1H-NMR (DMSO-D6) δ: 9.33 (2H, s), 8.83-8.81 (1H, m), 8.70 (1H, d, J = 7.6 Hz), 8.00-7.97 (1H, m), 7.83-7.79 (1H, m), 7.61-7.57 (1H, m), 3.92-3.82 (1H, m), 2.78-2.71 (1H, m), 2.12-2.07 (2H, m), 1.97-1.92 (2H, m), 1.71-1.61 (2H, m), 1.47-1.37 (2H, m). | 415 | 413 | |
| 115 | 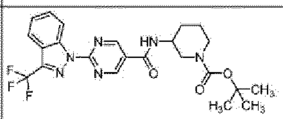 | 1H-NMR (DMSO-D6) δ: 9.34 (2H, s), 8.83 (1H, d, J = 8.8 Hz), 8.72 (1H, d, J = 7.2 Hz), 8.01-7.98 (1H, m), 7.84-7.80 (1H, m), 7.61-7.57 (1H, m), 4.08-3.90 (1H, br m), 3.89-3.71 (2H, m), 2.92-2.83 (2H, m), 1.99-1.95 (1H, m), 1.80-1.75 (1H, m), 1.62-1.53 (1H, m), 1.50-1.42 (1H, m), 1.40 (9H, s). | 491 | 489 | |
| 116 | 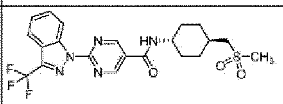 | 1H-NMR (DMSO-D6) δ: 9.34 (2H, s), 8.82 (1H, d, J = 8.8 Hz), 8.70 (1H, d, J = 7.6 Hz), 8.00-7.97 (1H, m), 7.83-7.79 (1H, m), 7.61-7.57 (1H, m), 3.84-3.75 (1H, m), 3.10 (2H, d, J = 6.2 Hz), 3.00 (3H, s), 2.02-1.88 (5H, m), 1.46-1.37 (2H, m), 1.29-1.20 (2H, m). | 482 | 480 | |
| 117 | HCl 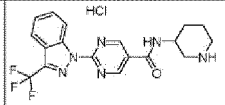 | 1H-NMR (DMSO-D6) δ: 9.46 (2H, s), 9.24 (1H, br s), 9.21-9.18 (1H, m), 8.98 (1H, br s), 8.83 (1H, d, J = 8.8 Hz), 8.01-7.98 (1H, m), 7.84-7.80 (1H, m), 7.62-7.58 (1H, m), 4.31-4.13 (1H, m), 3.37-3.24 (1H, m), 3.19-3.12 (1H, m), 3.02-2.90 (2H, m), 2.00-1.89 (2H, m), 1.78-1.58 (2H, m). | 391 | 389 | race-mate |
| 118 | 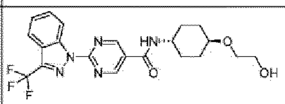 | 1H-NMR (DMSO-D6) δ: 9.33 (2H, s), 8.82 (1H, d, J = 8.8 Hz), 8.66 (1H, d, J = 7.6 Hz), 8.00-7.98 (1H, m), 7.83-7.79 (1H, m), 7.61-7.57 (1H, m), 4.54 (1H, t, J = 5.4 Hz), 3.86-3.77 (1H, m), 3.51-3.44 (4H, m), 3.31-3.25 (1H, m), 2.07-2.03 (2H, m), 1.96-1.92 (2H, m), 1.46-1.36 (2H, m), 1.32-1.23 (2H, m). | 450 | 448 | |
| 119 | 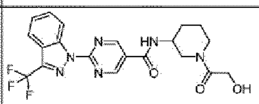 | 1H-NMR (DMSO-D6) δ: 9.34 (1H, s), 9.33 (1H, s), 8.84-8.81 (1.5H, m), 8.76 (0.5H, d, J = 7.4 Hz), 8.00-7.98 (1H, m), 7.84-7.79 (1H, m), 7.61-7.57 (1H, m), 4.60-4.53 (1H, m), 4.37-4.33 (0.5H, m), 4.17-4.07 (2H, m), 3.98-3.85 (1.5H, m), 3.77-3.73 (0.5H, m), 3.62-3.57 (0.5H, m), 3.12-2.97 (1.5H, m), 2.81-2.76 (0.5H, m), 2.02-1.98 (1H, m), 1.83-1.78 (1H, m), 1.74-1.43 (2H, m). | 449 | 447 | race-mate |
| 120 | 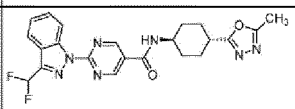 | 1H-NMR (DMSO-D6) δ: 9.32 (2H, s), 8.79 (1H, d, J = 8.6 Hz), 8.73 (1H, d, J = 7.6 Hz), 8.01 (1H, d, J = 8.1 Hz), 7.76 (1H, dt, J = 10.9, 3.9 Hz), 7.57 (1H, t, J = 53.2 Hz), 7.53 (1H, t, J = 7.3 Hz), 3.92-3.85 (1H, m), 2.93 (1H, tt, J = 11.8, 3.5 Hz), 2.47 (3H, s), 2.18-2.11 (2H, br m), 2.08-2.00 (2H, br m), 1.69-1.47 (4H, m). | 454 | 452 | |

[Table 1-16]

| Ex.No. | structural formula | 1H-NMR (400 MHz) | MS (positive) | MS (negative) | Comments |
|---|---|---|---|---|---|
| 121 | 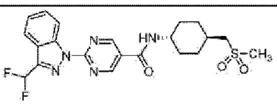 | 1H-NMR (DMSO-D6) δ: 9.31 (2H, s), 8.78 (1H, d, J = 9.0 Hz), 8.67 (1H, d, J = 7.5 Hz), 8.01 (1H, d, J = 7.5 Hz), 7.75 (1H, t, J = 7.9 Hz), 7.56 (1H, t, J = 53.9 Hz), 7.52 (1H, t, J = 7.5 Hz), 3.85-3.74 (1H, m), 3.09 (2H, d, J = 6.7 Hz), 3.00 (3H, s), 2.05-1.85 (5H, br m), 1.48-1.35 (2H, br m), 1.31-1.17 (2H, br m). | 464 | 462 | |
| 122 | 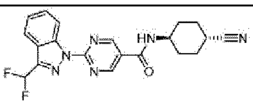 | 1H-NMR (DMSO-D6) δ: 9.30 (2H, s), 8.78 (1H, d, J = 8.2 Hz), 8.67 (1H, d, J = 7.5 Hz), 8.00 (1H, d, J = 8.2 Hz), 7.75 (1H, t, J = 7.9 Hz), 7.56 (1H, t, J = 53.9 Hz), 7.52 (1H, t, J = 7.9 Hz), 3.92-3.81 (1H, m), 2.77-2.71 (1H, m), 2.13-2.05 (2H, br m), 1.99-1.90 (2H, br m), 1.71-1.61 (2H, br m), 1.46-1.37 (2H, br m). | 397 | 395 | |
| 123 | 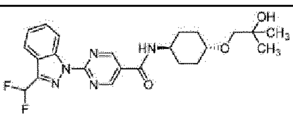 | 1H-NMR (DMSO-D6) δ: 9.30 (2H, s), 8.78 (1H, d, J = 9.0 Hz), 8.62 (1H, d, J = 7.5 Hz), 8.00 (1H, d, J = 8.2 Hz), 7.75 (1H, t, J = 7.9 Hz), 7.56 (1H, t, J = 53.1 Hz), 7.52 (1H, t, J = 7.9 Hz), 4.22 (1H, s), 3.86-3.76 (1H, m), 3.30-3.22 (1H, m), 3.19 (2H, s), 2.08-2.00 (2H, br m), 1.98-1.90 (2H, br m), 1.47-1.34 (2H, br m), 1.34-1.22 (2H, br m), 1.07 (6H, s). | 460 | 458 | |
| 124 | 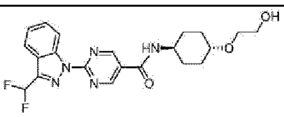 | 1H-NMR (DMSO-D6) δ: 9.30 (2H, s), 8.78 (1H, d, J = 8.2 Hz), 8.63 (1H, d, J = 7.5 Hz), 8.00 (1H, d, J = 8.2 Hz), 7.75 (1H, t, J = 7.9 Hz), 7.56 (1H, t, J = 53.1 Hz), 7.52 (1H, t, J = 7.9 Hz), 4.54 (1H, t, J = 5.2 Hz), 3.87-3.76 (1H, m), 3.52-3.42 (4H, m), 3.30-3.23 (1H, m), 2.10-2.00 (2H, br m), 1.98-1.90 (2H, br m), 1.48-1.34 (2H, br m), 1.33-1.22 (2H, br m). | 432 | 430 | |
| 125 | 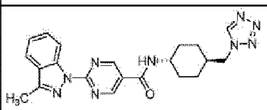 | 1H-NMR (DMSO-D6) δ: 9.21 (2H, s), 8.98 (1H, s), 8.67 (1H, d, J = 8.6 Hz), 8.57 (1H, d, J = 7.6 Hz), 7.89 (1H, d, J = 7.9 Hz), 7.63 (1H, ddd, J = 8.6, 7.4, 1.2 Hz), 7.39 (1H, dd, J = 7.9, 7.4 Hz), 4.63 (2H, d, J = 6.9 Hz), 3.84-3.71 (1H, m), 2.62 (3H, s), 2.03-1.88 (3H, m), 1.67-1.57 (2H, m), 1.42-1.14 (4H, m). | 418 | 416 | |
| 126 | 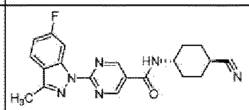 | 1H-NMR (DMSO-D6) δ: 9.23 (2H, s), 8.59 (1H, d, J = 7.6 Hz), 8.40 (1H, dd, J = 10.4, 2.3 Hz), 7.94 (1H, dd, J = 9.0, 5.4 Hz), 7.29 (1H, ddd, J = 9.0, 9.0, 2.3 Hz), 3.91-3.80 (1H, m), 2.79-2.69 (1H, m), 2.61 (3H, s), 2.14-2.04 (2H, m), 1.97-1.89 (2H, m), 1.73-1.59 (2H, m), 1.49-1.34 (2H, m). | 379 | 377 | |
| 127 | 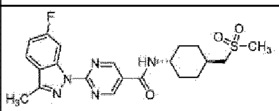 | 1H-NMR (DMSO-D6) δ: 9.24 (2H, s), 8.59 (1H, d, J = 7.9 Hz), 8.41 (1H, dd, J = 10.4, 2.3 Hz), 7.95 (1H, dd, J = 9.0, 5.4 Hz), 7.29 (1H, ddd, J = 9.0, 9.0, 2.3 Hz), 3.86-3.71 (1H, m), 3.09 (2H, d, J = 6.2 Hz), 2.99 (3H, s), 2.61 (3H, s), 2.05-1.85 (4H, m), 1.49-1.17 (5H, m). | 446 | 444 | |
| 128 | 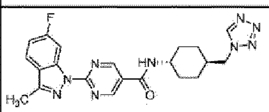 | 1H-NMR (DMSO-D6) δ: 9.23 (2H, s), 8.98 (1H, s), 8.58 (1H, d, J = 7.9 Hz), 8.40 (1H, dd, J = 10.4, 2.3 Hz), 7.94 (1H, dd, J = 9.0, 5.3 Hz), 7.29 (1H, ddd, J = 9.0, 9.0, 2.3 Hz), 4.63 (2H, d, J = 7.2 Hz), 3.84-3.72 (1H, m), 2.61 (3H, s), 2.04-1.88 (3H, m), 1.66-1.56 (2H, m), 1.42-1.14 (4H, m). | 436 | 434 | |

[Table 1-17]

| Ex.No. | structural formula | 1H-NMR (400 MHz) | MS (positive) | MS (negative) | Comments |
|---|---|---|---|---|---|
| 129 | 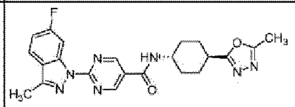 | 1H-NMR (DMSO-D6) δ: 9.25 (2H, s), 8.65 (1H, d, J = 7.6 Hz), 8.41 (1H, dd, J = 10.5, 2.0 Hz), 7.95 (1H, dd, J = 8.9, 5.3 Hz), 7.30 (1H, ddd, J = 8.9, 8.9, 2.0 Hz), 3.94-3.81 (1H, m), 2.97-2.88 (1H, m), 2.61 (3H, s), 2.47 (3H, s), 2.20-1.98 (4H, m), 1.71-1.45 (4H, m). | 436 | 434 | |
| 130 | 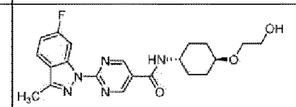 | 1H-NMR (DMSO-D6) δ: 9.23 (2H, s), 8.55 (1H, d, J = 7.6 Hz), 8.40 (1H, dd, J = 10.4, 2.3 Hz), 7.95 (1H, dd, J = 9.0, 5.4 Hz), 7.29 (1H, ddd, J = 9.0, 9.0, 2.3 Hz), 4.54 (1H, t, J = 5.3 Hz), 3.86-3.74 (1H, m), 3.52-3.42 (4H, m), 3.29-3.22 (1H, m), 2.61 (3H, s), 2.10-1.87 (4H, m), 1.47-1.20 (4H, m). | 414 | 412 | |
| 131 | 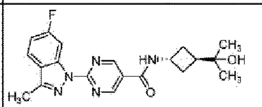 | 1H-NMR (DMSO-D6) δ: 9.25 (2H, s), 8.92 (1H, d, J = 6.9 Hz), 8.41 (1H, dd, J = 10.3, 2.2 Hz), 7.95 (1H, dd, J = 8.9, 5.4 Hz), 7.30 (1H, ddd, J = 8.9, 8.9, 2.2 Hz), 4.42-4.29 (1H, m), 4.25 (1H, br s), 2.61 (3H, s), 2.40-2.21 (3H, m), 2.12-2.00 (2H, m), 1.06 (6H, s). | 384 | 382 | |
| 132 | 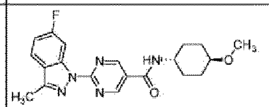 | 1H-NMR (DMSO-D6) δ: 9.23 (2H, s), 8.55 (1H, d, J = 7.5 Hz), 8.40 (1H, d, J = 9.7 Hz), 7.94 (1H, dd, J = 9.0, 5.2 Hz), 7.29 (1H, dd, J = 9.0, 9.0 Hz), 3.86-3.73 (1H, m), 3.25 (3H, s), 3.19-3.10 (1H, m), 2.61 (3H, s), 2.10-1.83 (4H, m), 1.49-1.15 (4H, m). | 384 | 382 | |
| 133 | 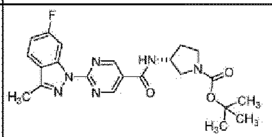 | 1H-NMR (DMSO-D6) δ: 9.25 (2H, s), 8.87 (1H, d, J = 6.0 Hz), 8.41 (1H, dd, J = 10.1, 1.9 Hz), 7.95 (1H, dd, J = 8.8, 5.6 Hz), 7.30 (1H, ddd, J = 8.8, 8.8, 1.9 Hz), 4.53-4.41 (1H, m), 3.66-3.52 (1H, m), 3.50-3.39 (1H, m), 3.39-3.17 (2H, m), 2.61 (3H, s), 2.21-2.08 (1H, m), 2.01-1.87 (1H, m), 1.42 (9H, s). | 441 | 439 | opti-cally active form (R) |
| 134 | 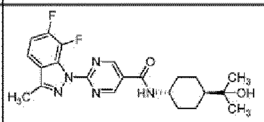 | 1H-NMR (DMSO-D6) δ: 9.24 (2H, s), 8.60 (1H, d, J = 7.6 Hz), 7.75 (1H, dd, J = 8.6, 4.2 Hz), 7.51-7.44 (1H, m), 4.05 (1H, s), 3.77-3.69 (1H, m), 2.60 (3H, s), 1.98-1.94 (2H, m), 1.88-1.84 (2H, m), 1.36-1.27 (2H, m), 1.24-1.01 (3H, m), 1.05 (6H, s). | 430 | 428 | |
| 135 | 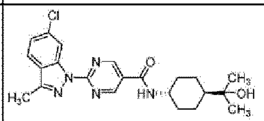 | 1H-NMR (DMSO-D6) δ: 9.24 (2H, s), 8.72 (1H, d, J = 1.8 Hz), 8.53 (1H, d, J = 7.6 Hz), 7.93 (1H, d, J = 8.3 Hz), 7.45 (1H, dd, J = 8.3, 1.8 Hz), 4.05 (1H, s), 3.78-3.70 (1H, m), 2.61 (3H, s), 1.99-1.94 (2H, m), 1.88-1.84 (2H, m), 1.37-1.27 (2H, m), 1.24-1.06 (3H, m), 1.06 (6H, s). | 428 | 426 | |
| 136 | 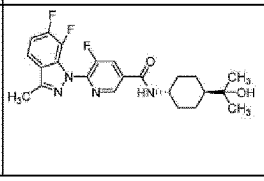 | 1H-NMR (DMSO-D6) δ: 8.83 (1H, d, J = 1.5 Hz), 8.57 (1H, d, J = 7.6 Hz), 8.40 (1H, dd, J = 10.6, 1.8 Hz), 7.76 (1H, dd, J = 8.8, 4.1 Hz), 7.45-7.38 (1H, m), 4.03 (1H, s), 3.78-3.69 (1H, m), 2.59 (3H, s), 1.97-1.94 (2H, m), 1.87-1.84 (2H, m), 1.37-1.27 (2H, m), 1.23-1.05 (3H, m), 1.05 (6H, s). | 447 | 445 | |

[Table 1-18]

| Ex.No. | structural formula | 1H-NMR (400 MHz) | MS (positive) | MS (negative) | Comments |
|---|---|---|---|---|---|
| 137 | 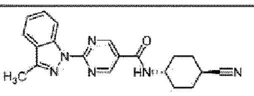 | 1H-NMR (DMSO-D6) δ: 9.22 (2H, s), 8.67 (1H, d, J = 8.6 Hz), 8.57 (1H, d, J = 7.4 Hz), 7.89 (1H, d, J = 7.9 Hz), 7.65-7.61 (1H, m), 7.40 (1H, t, J = 7.5 Hz), 3.90-3.80 (1H, m), 2.78-2.69 (1H, m), 2.62 (3H, s), 2.12-2.05 (2H, m), 1.97-1.89 (2H, m), 1.71-1.58 (2H, m), 1.47-1.35 (2H, m). | 361 | 359 | |
| 138 | 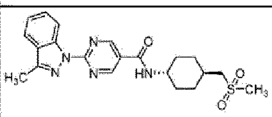 | 1H-NMR (DMSO-D6) δ: 9.22 (2H, s), 8.69-8.66 (1H, m), 8.57 (1H, d, J = 7.6 Hz), 7.91-7.87 (1H, m), 7.65-7.61 (1H, m), 7.42-7.38 (1H, m), 3.82-3.73 (1H, m), 3.09 (2H, d, J = 6.2 Hz), 2.99 (3H, s), 2.62 (3H, s), 2.03-1.86 (5H, m), 1.47-1.35 (2H, m), 1.30-1.18 (2H, m). | 428 | 426 | |
| 139 | 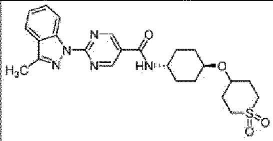 | 1H-NMR (DMSO-D6) δ: 9.22 (2H, s), 8.70-8.66 (1H, m), 8.54 (1H, d, J = 7.6 Hz), 7.90-7.87 (1H, m), 7.65-7.61 (1H, m), 7.42-7.38 (1H, m), 3.84-3.74 (2H, m), 3.46-3.36 (1H, m), 3.14-3.01 (4H, m), 2.62 (3H, s), 2.11-1.89 (8H, m), 1.48-1.25 (4H, m). | 484 | 482 | |
| 140 | 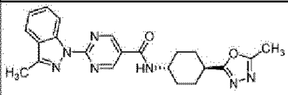 | 1H-NMR (DMSO-D6) δ: 9.24 (2H, s), 8.71-8.61 (2H, m), 7.92-7.87 (1H, m), 7.65-7.61 (1H, m), 7.42-7.38 (1H, m), 3.92-3.83 (1H, m), 2.97-2.88 (1H, m), 2.62 (3H, s), 2.47 (3H, s), 2.17-2.11 (2H, m), 2.07-2.00 (2H, m), 1.68-1.47 (4H, m). | 418 | 416 | |
| 141 | 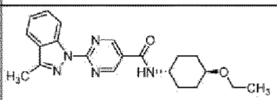 | 1H-NMR (DMSO-D6) δ: 9.22 (2H, s), 8.67 (1H, d, J = 8.3 Hz), 8.53 (1H, d, J = 7.6 Hz), 7.89 (1H, d, J = 7.9 Hz), 7.63 (1H, t, J = 7.6 Hz), 7.40 (1H, t, J = 7.5 Hz), 3.84-3.75 (1H, m), 3.47 (2H, q, J = 7.0 Hz), 3.28-3.20 (1H, m), 2.62 (3H, s), 2.06-1.99 (2H, m), 1.96-1.89 (2H, m), 1.45-1.34 (2H, m), 1.31-1.20 (2H, m), 1.10 (3H, t, J = 6.9 Hz). | 380 | 378 | |
| 142 | 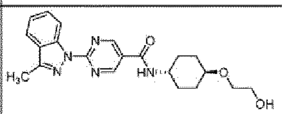 | 1H-NMR (DMSO-D6) δ: 9.22 (2H, s), 8.67 (1H, d, J = 8.3 Hz), 8.53 (1H, d, J = 7.9 Hz), 7.89 (1H, d, J = 7.9 Hz), 7.65-7.61 (1H, m), 7.42-7.38 (1H, m), 4.54 (1H, t, J = 5.3 Hz), 3.84-3.76 (1H, m), 3.51-3.43 (4H, m), 3.31-3.24 (1H, m), 2.62 (3H, s), 2.08-2.01 (2H, m), 1.96-1.89 (2H, m), 1.45-1.34 (2H, m), 1.31-1.21 (2H, m). | 396 | 394 | |
| 143 | 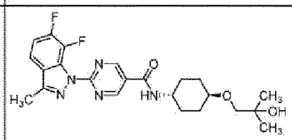 | 1H-NMR (DMSO-D6) δ: 9.23 (2H, s), 8.60 (1H, d, J = 8.2 Hz), 7.75 (1H, dd, J = 8.6, 4.1 Hz), 7.51-7.44 (1H, m), 4.21 (1H, s), 3.83-3.76 (1H, m), 3.30-3.23 (1H, m), 3.18 (2H, s), 2.60 (3H, s), 2.04-1.91 (4H, m), 1.44-1.23 (4H, m), 1.07 (6H, s). | 460 | 458 | |
| 144 | 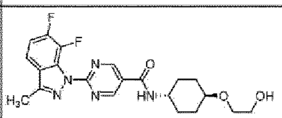 | 1H-NMR (DMSO-D6) δ: 9.23 (2H, s), 8.61 (1H, d, J = 7.5 Hz), 7.75 (1H, dd, J = 9.0, 4.5 Hz), 7.51-7.44 (1H, m), 4.54 (1H, t, J = 4.9 Hz), 3.85-3.75 (1H, m), 3.50-3.43 (4H, m), 3.29-3.23 (1H, m), 2.60 (3H, s), 2.05-1.91 (4H, m), 1.44-1.22 (4H, m). | 432 | 430 | |

[Table 1-19]

| Ex.No. | structural formula | 1H-NMR (400 MHz) | MS (positive) | MS (negative) | Comments |
|---|---|---|---|---|---|
| 145 | 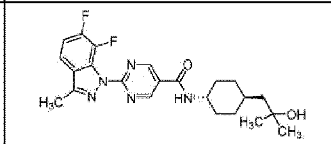 | 1H-NMR (DMSO-D6) δ: 9.23 (2H, s), 8.59 (1H, d, J = 8.2 Hz), 7.77-7.74 (1H, m), 7.51-7.44 (1H, m), 4.04 (1H, s), 3.79-3.68 (1H, m), 2.60 (3H, s), 1.89-1.87 (4H, m), 1.45-1.28 (5H, m), 1.11 (6H, s), 1.10-1.02 (2H, m). | 444 | 442 | |
| 146 | 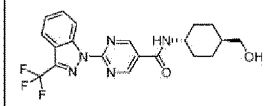 | 1H-NMR (DMSO-D6) δ: 9.34 (2H, s), 8.82 (1H, d, J = 8.6 Hz), 8.67 (1H, d, J = 7.6 Hz), 8.00-7.97 (1H, m), 7.83-7.79 (1H, m), 7.61-7.57 (1H, m), 4.42 (1H, t, J = 5.3 Hz), 3.83-3.73 (1H, m), 3.27-3.23 (2H, m), 1.97-1.93 (2H, m), 1.84-1.80 (2H, m), 1.41-1.31 (3H, m), 1.07-0.97 (2H, m). | 420 | 418 | |
| 147 | 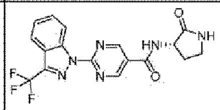 | 1H-NMR (DMSO-D6) δ: 9.37 (2H, s), 9.18 (1H, d, J = 8.1 Hz), 8.85-8.82 (1H, m), 8.00-7.96 (2H, m), 7.84-7.79 (1H, m), 7.61-7.57 (1H, m), 4.66-4.59 (1H, m), 3.26-3.31 (2H, m), 2.47-2.40 (1H, m), 2.10-2.00 (1H, m). | 391 | 389 | opti-cally active form (S) |
| 148 | 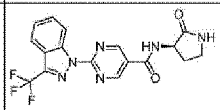 | 1H-NMR (DMSO-D6) δ: 9.37 (2H, s), 9.18 (1H, d, J = 8.1 Hz), 8.85-8.82 (1H, m), 8.00-7.96 (2H, m), 7.84-7.79 (1H, m), 7.61-7.57 (1H, m), 4.66-4.59 (1H, m), 3.26-3.31 (2H, m), 2.47-2.40 (1H, m), 2.10-2.00 (1H, m). | 391 | 389 | opti-cally active form (R) |
| 149 | 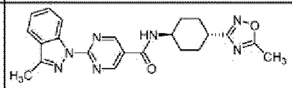 | 1H-NMR (DMSO-D6) δ: 9.24 (2H, s), 8.68 (1H, d, J = 8.2 Hz), 8.64 (1H, d, J = 7.5 Hz), 7.89 (1H, d, J = 8.2 Hz), 7.63 (1H, t, J = 7.5 Hz), 7.40 (1H, t, J = 7.5 Hz), 3.93-3.82 (1H, m), 2.82-2.73 (1H, m), 2.63 (3H, s), 2.56 (3H, s), 2.12-1.98 (4H, br m), 1.65-1.48 (4H, m). | 418 | 416 | |
| 150 | 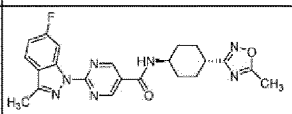 | 1H-NMR (DMSO-D6) δ: 9.25 (2H, s), 8.65 (1H, d, J = 7.5 Hz), 8.41 (1H, dd, J = 10.1, 1.9 Hz), 7.94 (1H, dd, J = 8.6, 5.6 Hz), 7.29 (1H, td, J = 8.8, 1.7 Hz), 3.93-3.81 (1H, m), 2.82-2.74 (1H, m), 2.61 (3H, s), 2.56 (3H, s), 2.11-1.99 (4H, br m), 1.65-1.48 (4H, m). | 436 | 434 | |
| 151 | 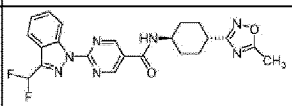 | 1H-NMR (DMSO-D6) δ: 9.32 (2H, s), 8.79 (1H, d, J = 8.2 Hz), 8.73 (1H, d, J = 7.5 Hz), 8.01 (1H, d, J = 8.2 Hz), 7.76 (1H, t, J = 7.9 Hz), 7.57 (1H, t, J = 53.1 Hz), 7.53 (1H, t, J = 7.5 Hz), 3.94-3.83 (1H, m), 2.83-2.74 (1H, m), 2.57 (3H, s), 2.11-2.00 (4H, br m), 1.66-1.48 (4H, m). | 454 | 452 | |
| 152 | 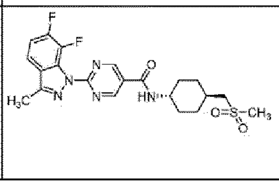 | 1H-NMR (DMSO-D6) δ: 9.22 (2H, s), 8.64 (1H, d, J = 7.6 Hz), 7.75-7.72 (1H, m), 7.49-7.43 (1H, m), 3.80-3.72 (1H, m), 3.07 (2H, d, J = 6.2 Hz), 2.98 (3H, s), 2.59 (3H, s), 1.99-1.90 (5H, m), 1.43-1.17 (4H, m). | 464 | 462 | |

[Table 1-20]

| Ex.No. | structural formula | 1H-NMR (400 MHz) | MS (positive) | MS (negative) | Comments |
|---|---|---|---|---|---|
| 153 | 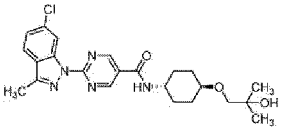 | 1H-NMR (DMSO-D6) δ: 9.22 (2H, s), 8.71 (1H, d, J = 1.8 Hz), 8.51 (1H, d, J = 7.6 Hz), 7.92 (1H, d, J = 8.6 Hz), 7.43 (1H, dd, J = 8.4, 1.7 Hz), 4.20 (1H, s), 3.84-3.73 (1H, m), 3.27-3.21 (1H, m), 3.17 (2H, s), 2.60 (3H, s), 2.03-1.90 (4H, m), 1.43-1.21 (4H, m), 1.06 (6H, s). | 458 | 456 | |
| 154 | 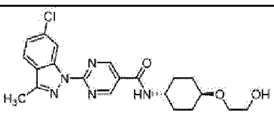 | 1H-NMR (DMSO-D6) δ: 9.22 (2H, s), 8.70 (1H, d, J = 1.7 Hz), 8.52 (1H, d, J = 7.6 Hz), 7.91 (1H, d, J = 8.6 Hz), 7.43 (1H, dd, J = 8.6, 1.7 Hz), 4.53 (1H, t, J = 5.4 Hz), 3.82-3.74 (1H, m), 3.49-3.40 (4H, m), 3.27-3.22 (1H, m), 2.60 (3H, s), 2.04-1.90 (4H, m), 1.43-1.21 (4H, m). | 430 | 428 | |
| 155 | 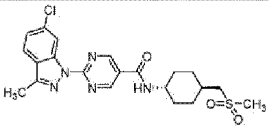 | 1H-NMR (DMSO-D6) δ: 9.23 (2H, s), 8.71 (1H, dd, J = 1.8, 0.6 Hz), 8.56 (1H, d, J = 7.6 Hz), 7.92 (1H, dd, J = 8.3, 0.6 Hz), 7.43 (1H, dd, J = 8.3, 1.8 Hz), 3.80-3.72 (1H, m), 3.07 (2H, d, J = 6.2 Hz), 2.98 (3H, s), 2.60 (3H, s), 1.99-1.90 (5H, m), 1.43-1.17 (4H, m). | 462 | 460 | |
| 156 | 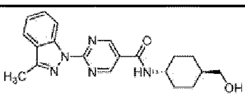 | 1H-NMR (DMSO-D6) δ: 9.23 (2H, s), 8.68 (1H, d, J = 8.2 Hz), 8.55 (1H, d, J = 7.5 Hz), 7.89 (1H, d, J = 8.2 Hz), 7.63 (1H, t, J = 7.5 Hz), 7.40 (1H, t, J = 7.5 Hz), 4.41 (1H, t, J = 5.2 Hz), 3.81-3.73 (1H, m), 3.25 (2H, t, J = 5.6 Hz), 2.62 (3H, s), 1.98-1.90 (2H, m), 1.85-1.77 (2H, m), 1.41-1.29 (3H, m), 1.08-0.95 (2H, m). | 366 | 364 | |
| 157 | 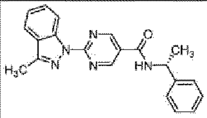 | 1H-NMR (DMSO-D6) δ: 9.28 (2H, s), 9.13 (1H, d, J = 8.2 Hz), 8.68 (1H, d, J = 8.2 Hz), 7.89 (1H, d, J = 7.5 Hz), 7.63 (1H, t, J = 7.5 Hz), 7.46-7.39 (3H, m), 7.39-7.33 (2H, m), 7.28-7.23 (1H, m), 5.25-5.18 (1H, m), 2.63 (3H, s), 1.53 (3H, d, J = 6.7 Hz). | 358 | 356 | optically active form (R) |
| 158 | 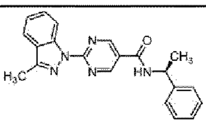 | 1H-NMR (DMSO-D6) δ: 9.28 (2H, s), 9.13 (1H, d, J = 8.2 Hz), 8.68 (1H, d, J = 8.2 Hz), 7.89 (1H, d, J = 8.2 Hz), 7.63 (1H, t, J = 7.5 Hz), 7.46-7.39 (3H, m), 7.39-7.33 (2H, m), 7.28-7.23 (1H, m), 5.25-5.18 (1H, m), 2.63 (3H, s), 1.53 (3H, d, J = 6.7 Hz). | 358 | 356 | optically active form (S) |
| 159 | 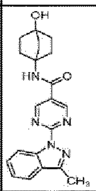 | 1H-NMR (DMSO-D6) δ: 9.15 (2H, s), 8.66 (1H, d, J = 9.0 Hz), 8.01 (1H, s), 7.88 (1H, d, J = 7.5 Hz), 7.62 (1H, t, J = 7.9 Hz), 7.39 (1H, t, J = 7.5 Hz), 4.32 (1H, s), 2.61 (3H, s), 2.08-2.04 (6H, m), 1.66-1.62 (6H, m). | 378 | 376 | |
| 160 | 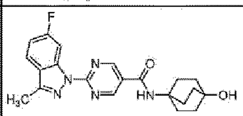 | 1H-NMR (DMSO-D6) δ: 9.16 (2H, s), 8.39 (1H, dd, J = 10.5, 2.2 Hz), 8.02 (1H, s), 7.94 (1H, dd, J = 8.2, 5.2 Hz), 7.29 (1H, td, J = 8.6, 2.0 Hz), 4.32 (1H, s), 2.61 (3H, s), 2.08-2.04 (6H, m), 1.66-1.62 (6H, m). | 396 | 394 | |

[Table 1-21]

| Ex.No. | structural formula | 1H-NMR (400 MHz) | MS (positive) | MS (negative) | Comments |
|---|---|---|---|---|---|
| 161 | 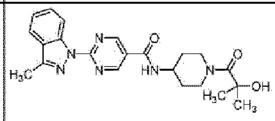 | 1H-NMR (DMSO-D6) δ: 9.23 (2H, s), 8.68 (1H, d, J = 8.6 Hz), 8.61 (1H, d, J = 7.4 Hz), 7.89 (1H, d, J = 8.1 Hz), 7.65-7.61 (1H, m), 7.42-7.38 (1H, m), 5.39 (1H, s), 4.84-4.33 (2H, br m), 4.13-4.05 (1H, m), 3.19-2.76 (2H, br m), 2.62 (3H, s), 1.92-1.88 (2H, m), 1.54-1.43 (2H, m), 1.34 (6H, s). | 423 | 421 | |
| 162 | 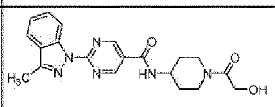 | 1H-NMR (DMSO-D6) δ: 9.23 (2H, s), 8.68 (1H, d, J = 8.6 Hz), 8.63 (1H, d, J = 7.6 Hz), 7.89 (1H, d, J = 7.9 Hz), 7.65-7.61 (1H, m), 7.42-7.38 (1H, m), 4.54 (1H, t, J = 5.5 Hz), 4.34-4.30 (1H, m), 4.14-4.06 (3H, m), 3.75-3.72 (1H, m), 3.14-3.08 (1H, m), 2.88-2.82 (1H, m), 2.62 (3H, s), 1.93-1.89 (2H, m), 1.55-1.39 (2H, m). | 395 | 393 | |
| 163 | 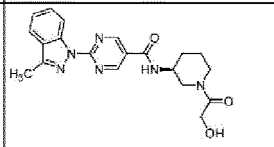 | 1H-NMR (DMSO-D6) δ: 9.23 (2H, d, J = 4.2 Hz), 8.68 (1.5H, d, J = 8.3 Hz), 8.63 (0.5H, d, J = 7.4 Hz), 7.89 (1H, d, J = 7.9 Hz), 7.66-7.62 (1H, m), 7.42-7.38 (1H, m), 4.58-4.52 (1H, m), 4.36-4.33 (0.5H, m), 4.14-4.08 (2H, m), 4.00-3.94 (0.5H, m), 3.90-3.84 (1H, m), 3.75-3.72 (0.5H, m), 3.61-3.57 (0.5H, m), 3.06-2.98 (1.5H, m), 2.78-2.72 (0.5H, m), 2.63 (3H, s), 2.00-1.97 (1H, m), 1.81-1.77 (1H, m), 1.70-1.43 (2H, m). | 395 | 393 | optically active form (S) |
| 164 | 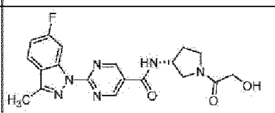 | 1H-NMR (DMSO-D6) δ: 9.25 (2H, d, J = 1.2 Hz), 8.87 (1H, dd, J = 12.8, 6.4 Hz), 8.41 (1H, dd, J = 10.5, 2.2 Hz), 7.95 (1H, dd, J = 8.8, 5.3 Hz), 7.30 (1H, td, J = 8.8, 2.3 Hz), 4.63-4.42 (1H, m), 4.03 (1H, s), 4.00 (1H, s), 3.75-3.61 (1H, m), 3.59-3.40 (4H, m), 2.61 (3H, s), 2.27-1.88 (2H, m). | 399 | 397 | optically active form (R) |
| 165 | 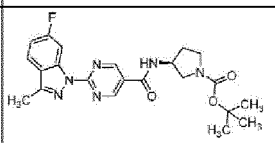 | 1H-NMR (DMSO-D6) δ: 9.25 (2H, s), 8.87 (1H, d, J = 6.0 Hz), 8.41 (1H, dd, J = 10.1, 1.9 Hz), 7.95 (1H, dd, J = 8.8, 5.6 Hz), 7.30 (1H, ddd, J = 8.8, 8.8, 1.9 Hz), 4.53-4.41 (1H, m), 3.66-3.52 (1H, m), 3.50-3.39 (1H, m), 3.39-3.17 (2H, m), 2.61 (3H, s), 2.21-2.08 (1H, m), 2.01-1.87 (1H, m), 1.42 (9H, s). | 441 | 439 | optically active form (S) |
| 166 | 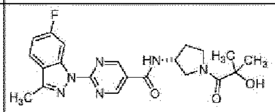 | 1H-NMR (DMSO-D6) δ: 9.25 (2H, d, J = 2.5 Hz), 8.84 (1H, d, J = 6.2 Hz), 8.41 (1H, dd, J = 10.4, 2.3 Hz), 7.95 (1H, dd, J = 8.8, 5.3 Hz), 7.30 (1H, td, J = 9.0, 2.3 Hz), 5.17 (1H, s), 4.53-4.37 (1H, m), 4.15-4.06 (0.5H, m), 4.00-3.84 (1H, m), 3.79-3.54 (1.5H, m), 3.46-3.34 (1H, m), 2.61 (3H, s), 2.24-1.94 (1.5H, m), 1.92-1.79 (0.5H, m), 1.36-1.25 (6H, m). | 427 | 425 | optically active form (R) |
| 167 | 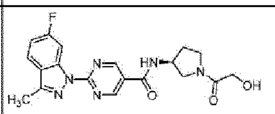 | 1H-NMR (DMSO-D6) δ: 9.25 (2H, s), 8.87 (1H, dd, J = 11.9, 5.7 Hz), 8.41 (1H, dd, J = 10.4, 2.3 Hz), 7.95 (1H, t, J = 7.1 Hz), 7.30 (1H, td, J = 8.9, 2.1 Hz), 4.63-4.43 (2H, m), 4.06-3.97 (2H, m), 3.75-3.62 (1H, m), 3.59-3.40 (3H, m), 2.61 (3H, s), 2.28-1.89 (2H, m). | 399 | 397 | optically active form (S) |
| 168 | 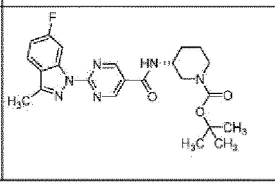 | 1H-NMR (DMSO-D6) δ: 9.24 (2H, s), 8.61 (1H, d, J = 7.2 Hz), 8.41 (1H, dd, J = 10.4, 2.3 Hz), 7.95 (1H, dd, J = 9.0, 5.3 Hz), 7.30 (1H, ddd, J = 9.0, 9.0, 2.3 Hz), 4.10-3.62 (3H, m), 2.93-2.81 (1H, m), 2.61 (3H, s), 2.00-1.89 (1H, m), 1.83-1.72 (1H, m), 1.62-1.50 (1H, m), 1.49-1.29 (2H, m), 1.40 (9H, s). | 455 | 453 | optically active form (R) |

[Table 1-22]

| Ex.No. | structural formula | 1H-NMR (400 MHz) | MS (positive) | MS (negative) | Comments |
|---|---|---|---|---|---|
| 169 | 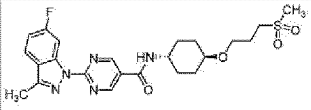 | 1H-NMR (DMSO-D6) δ: 9.23 (2H, s), 8.55 (1H, d, J = 7.6 Hz), 8.40 (1H, dd, J = 10.4, 2.1 Hz), 7.94 (1H, dd, J = 8.9, 5.4 Hz), 7.29 (1H, ddd, J = 8.9, 8.9, 2.1 Hz), 3.86-3.74 (1H, m), 3.53 (2H, t, J = 6.2 Hz), 3.34-3.22 (1H, m), 3.18-3.09 (2H, m), 2.99 (3H, s), 2.61 (3H, s), 2.09-1.99 (2H, m), 1.98-1.84 (4H, m), 1.47-1.21 (4H, m). | 490 | 488 | |
| 170 | 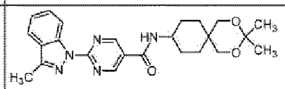 | 1H-NMR (DMSO-D6) δ: 9.22 (2H, s), 8.68 (1H, d, J = 8.6 Hz), 8.54 (1H, d, J = 7.9 Hz), 7.89 (1H, d, J = 8.1 Hz), 7.63 (1H, t, J = 7.7 Hz), 7.40 (1H, t, J = 7.5 Hz), 3.86-3.76 (1H, m), 3.71 (2H, s), 3.45 (2H, s), 2.62 (3H, s), 1.89-1.81 (2H, br m), 1.78-1.69 (2H, br m), 1.50-1.38 (2H, m), 1.33 (6H, s), 1.21-1.11 (2H, m). | 436 | 434 | |
| 171 | 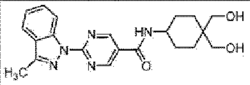 | 1H-NMR (DMSO-D6) δ: 9.22 (2H, s), 8.68 (1H, d, J = 8.5 Hz), 8.54 (1H, d, J = 7.6 Hz), 7.89 (1H, d, J = 7.6 Hz), 7.63 (1H, t, J = 7.4 Hz), 7.40 (1H, t, J = 7.6 Hz), 4.33 (2H, br s), 3.82-3.70 (1H, m), 3.43 (2H, s), 3.20 (2H, s), 2.62 (3H, s), 1.74-1.64 (2H, br m), 1.61-1.43 (4H, br m), 1.28-1.15 (2H, m). | 396 | 394 | |
| 172 | 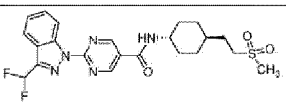 | 1H-NMR (DMSO-D6) δ: 9.31 (2H, s), 8.78 (1H, d, J = 8.6 Hz), 8.64 (1H, d, J = 7.9 Hz), 8.00 (1H, d, J = 8.1 Hz), 7.78-7.73 (1H, m), 7.56 (1H, t, J = 53.4 Hz), 7.55-7.50 (1H, m), 3.80 (1H, tt, J = 11.9, 3.9 Hz), 3.18-3.11 (2H, m), 2.96 (3H, s), 1.98-1.90 (2H, br m), 1.86-1.78 (2H, br m), 1.67-1.58 (2H, m), 1.43-1.30 (3H, br m), 1.15-1.02 (2H, br m). | 478 | 476 | |
| 173 | 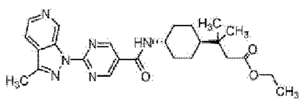 | 1H-NMR (DMSO-D6) δ: 9.99 (1H, s), 9.28 (2H, s), 8.57 (1H, d, J = 8.1 Hz), 8.54 (1H, d, J = 5.3 Hz), 7.95 (1H, d, J = 5.3 Hz), 4.06 (2H, q, J = 7.1 Hz), 3.83-3.70 (1H, m), 2.67 (3H, s), 2.22 (2H, s), 2.04-1.93 (2H, m), 1.85-1.76 (2H, m), 1.39-1.07 (5H, m), 1.20 (3H, t, J = 7.1 Hz), 0.95 (6H, s). | 465 | 463 | |
| 174 | 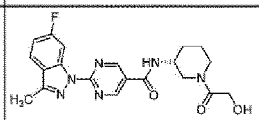 | 1H-NMR (DMSO-D6) δ: 9.24 (2H, d, J = 3.9 Hz), 8.70 (0.5H, d, J = 7.9 Hz), 8.64 (0.5H, d, J = 7.9 Hz), 8.41 (1H, dd, J = 10.4, 2.3 Hz), 7.95 (1H, dd, J = 8.9, 5.3 Hz), 7.30 (1H, ddd, J = 8.9, 8.9, 2.3 Hz), 4.60-4.50 (1H, m), 4.38-4.30 (0.5H, m), 4.19-4.04 (2H, m), 4.00-3.80 (1.5H, m), 3.77-3.69 (0.5H, m), 3.63-3.54 (0.5H, m), 3.12-2.93 (1.5H, m), 2.80-2.71 (0.5H, m), 2.62 (3H, s), 2.04-1.93 (1H, m), 1.84-1.75 (1H, m), 1.73-1.41 (2H, m). | 413 | 411 | optically active form (R) |
| 175 | 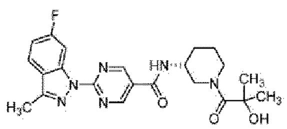 | 1H-NMR (DMSO-D6) δ: 9.24 (2H, s), 8.63 (1H, d, J = 7.6 Hz), 8.41 (1H, dd, J = 10.4, 2.3 Hz), 7.95 (1H, dd, J = 8.9, 5.4 Hz), 7.30 (1H, ddd, J = 8.9, 8.9, 2.3 Hz), 5.39 (1H, s), 5.02-4.07 (2H, m), 3.95-3.81 (1H, m), 3.17-2.58 (2H, m), 2.62 (3H, s), 2.05-1.94 (1H, m), 1.83-1.72 (1H, m), 1.68-1.55 (1H, m), 1.53-1.42 (1H, m), 1.41-1.30 (6H, m). | 441 | 439 | optically active form (R) |
| 176 | 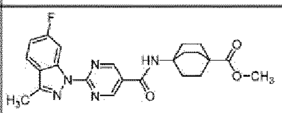 | 1H-NMR (DMSO-D6) δ: 9.17 (2H, s), 8.40 (1H, dd, J = 10.4, 2.1 Hz), 8.10 (1H, br s), 7.94 (1H, dd, J = 8.8, 5.4 Hz), 7.29 (1H, ddd, J = 8.8, 8.8, 2.1 Hz), 3.59 (3H, s), 2.61 (3H, s), 2.06-1.96 (6H, m), 1.89-1.80 (6H, m). | 438 | 436 | |

[Table 1-23]

| Ex.No. | structural formula | 1H-NMR (400 MHz) | MS (positive) | MS (negative) | Comments |
|---|---|---|---|---|---|
| 177 | 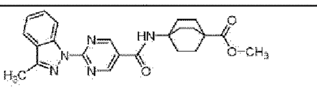 | 1H-NMR (DMSO-D6) δ : 9.16 (2H, s), 8.67 (1H, d, J = 8.6 Hz), 8.09 (1H, br s), 7.89 (1H, d, J = 7.9 Hz), 7.63 (1H, dd, J = 8.6, 7.4 Hz), 7.39 (1H, dd, J = 7.9, 7.4 Hz), 3.59 (3H, s), 2.62 (3H, s), 2.05-1.96 (6H, m), 1.89-1.80 (6H, m). | 420 | 418 | |
| 178 | 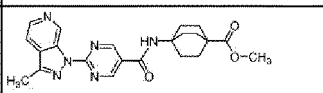 | 1H NMR (DMSO-D6) δ : 9.98 (1H, s), 9.22 (2H, s), 8.53 (1H, d, J = 5.3 Hz), 8.13 (1H, s), 7.94 (1H, dd, J = 5.3, 1.2 Hz), 3.59 (3H, s), 2.66 (3H, s), 2.07-1.96 (6H, m), 1.90-1.80 (6H, m). | 421 | 419 | |
| 179 | 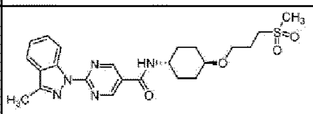 | 1H-NMR (DMSO-D6) δ : 9.22 (2H, s), 8.67 (1H, d, J = 8.3 Hz), 8.54 (1H, d, J = 7.6 Hz), 7.89 (1H, d, J = 7.9 Hz), 7.63 (1H, dd, J = 8.3, 7.5 Hz), 7.40 (1H, dd, J = 7.9, 7.5 Hz), 3.87-3.74 (1H, m), 3.53 (2H, t, J = 6.1 Hz), 3.30-3.22 (1H, m), 3.14 (2H, t, J = 7.9 Hz), 2.99 (3H, s), 2.62 (3H, s), 2.08-1.99 (2H, m), 1.97-1.85 (4H, m), 1.49-1.20 (4H, m). | 472 | 470 | |
| 180 | 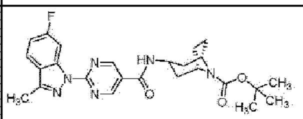 | 1H NMR (DMSO-D6) δ : 9.22 (2H, s), 8.58 (1H, d, J = 7.9 Hz), 8.40 (1H, dd, J = 10.4, 2.1 Hz), 7.94 (1H, dd, J = 8.9, 5.3 Hz), 7.29 (1H, ddd, J = 8.9, 8.9, 2.1 Hz), 4.49-4.34 (1H, m), 4.18-4.07 (2H, m), 2.61 (3H, s), 2.02-1.53 (8H, m), 1.44 (9H, s). | 481 | 479 | exo form |
| 181 | 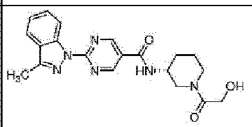 | 1H-NMR (DMSO-D6) δ : 9.24-9.22 (2H, m), 8.70-8.59 (2H, m), 7.89 (1H, d, J = 8.1 Hz), 7.63 (1H, t, J = 7.6 Hz), 7.40 (1H, t, J = 7.4 Hz), 4.59-4.50 (1H, m), 4.36-3.57 (5H, m), 3.11-2.72 (2H, m), 2.62 (3H, s), 2.02-1.95 (1H, m), 1.83-1.76 (1H, m), 1.73-1.40 (2H, m). | 395 | 393 | optically active form (R) |
| 182 | 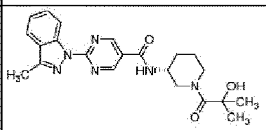 | 1H-NMR (DMSO-D6) δ : 9.23 (2H, s), 8.68 (1H, d, J = 8.5 Hz), 8.61 (1H, d, J = 7.6 Hz), 7.89 (1H, d, J = 8.1 Hz), 7.64 (1H, t, J = 7.6 Hz), 7.40 (1H, t, J = 7.4 Hz), 5.39 (1H, s), 4.90-4.15 (2H, br m), 3.96-3.83 (1H, m), 3.13-2.69 (2H, br m), 2.62 (3H, s), 2.04-1.95 (1H, m), 1.82-1.72 (1H, m), 1.68-1.28 (8H, m). | 423 | 421 | optically active form (R) |
| 183 | 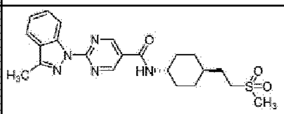 | 1H-NMR (DMSO-D6) δ : 9.22 (2H, s), 8.68 (1H, d, J = 8.5 Hz), 8.54 (1H, d, J = 8.1 Hz), 7.89 (1H, d, J = 8.1 Hz), 7.63 (1H, t, J = 7.9 Hz), 7.40 (1H, t, J = 7.6 Hz), 3.84-3.74 (1H, m), 3.17-3.12 (2H, m), 2.96 (3H, s), 2.62 (3H, s), 1.97-1.90 (2H, m), 1.85-1.78 (2H, m), 1.67-1.58 (2H, m), 1.43-1.30 (3H, m), 1.15-1.01 (2H, m). | 442 | 440 | |
| 184 | 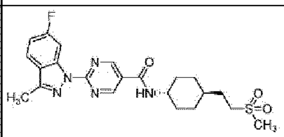 | 1H-NMR (DMSO-D6) δ : 9.23 (2H, s), 8.55 (1H, d, J = 7.6 Hz), 8.43-8.38 (1H, m), 7.97-7.92 (1H, m), 7.32-7.26 (1H, m), 3.84-3.73 (1H, m), 3.18-3.10 (2H, m), 2.96 (3H, s), 2.61 (3H, s), 1.97-1.89 (2H, m), 1.86-1.76 (2H, m), 1.66-1.58 (2H, m), 1.43-1.30 (3H, m), 1.15-1.01 (2H, m). | 460 | 458 | |

[Table 1-24]

| Ex.No. | structural formula | 1H-NMR (400 MHz) | MS (positive) | MS (negative) | Comments |
|---|---|---|---|---|---|
| 185 | 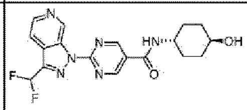 | 1H-NMR (DMSO-D6) δ: 10.12 (1H, s), 9.35 (2H, s), 8.65-8.62 (2H, m), 8.00 (1H, d, J = 5.8 Hz), 7.64 (1H, t, J = 53.0 Hz), 4.58 (1H, s), 3.83-3.71 (1H, m), 3.49-3.40 (1H, m), 1.90-1.88 (4H, m), 1.44-1.23 (4H, m). | 389 | 387 | |
| 186 | 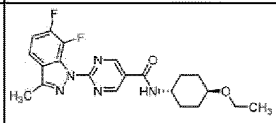 | 1H-NMR (DMSO-D6) δ: 9.23 (2H, s), 8.61 (1H, d, J = 7.6 Hz), 7.77-7.74 (1H, m), 7.51-7.44 (1H, m), 3.84-3.75 (1H, m), 3.47 (2H, q, J = 7.0 Hz), 3.27-3.21 (1H, m), 2.60 (3H, s), 2.04-1.91 (4H, m), 1.44-1.20 (4H, m), 1.10 (3H, t, J = 7.0 Hz). | 416 | 414 | |
| 187 | 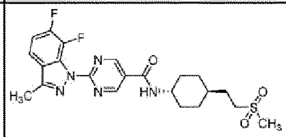 | 1H-NMR (DMSO-D6) δ: 9.24 (2H, s), 8.62 (1H, d, J = 7.6 Hz), 7.77-7.74 (1H, m), 7.51-7.45 (1H, m), 3.82-3.74 (1H, m), 3.16-3.12 (2H, m), 2.96 (3H, s), 2.60 (3H, s), 1.94-1.79 (4H, m), 1.65-1.59 (2H, m), 1.39-1.31 (3H, m), 1.12-1.03 (2H, m). | 478 | 476 | |
| 188 | 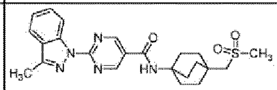 | 1H-NMR (DMSO-D6) δ: 9.17 (2H, s), 8.67 (1H, d, J = 8.6 Hz), 8.04 (1H, s), 7.88 (1H, d, J = 7.9 Hz), 7.65-7.60 (1H, m), 7.39 (1H, t, J = 7.3 Hz), 3.07 (2H, s), 2.95 (3H, s), 2.62 (3H, s), 2.01-1.97 (6H, m), 1.82-1.78 (6H, m). | 454 | 452 | |
| 189 | 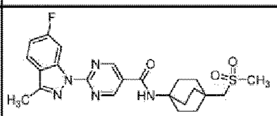 | 1H-NMR (DMSO-D6) δ: 9.18 (2H, s), 8.39 (1H, dd, J = 10.4, 2.3 Hz), 8.06 (1H, s), 7.94 (1H, dd, J = 8.7, 5.4 Hz), 7.29 (1H, td, J = 8.9, 2.3 Hz), 3.07 (2H, s), 2.95 (3H, s), 2.61 (3H, s), 2.01-1.97 (6H, m), 1.82-1.78 (6H, m). | 472 | 470 | |
| 190 | 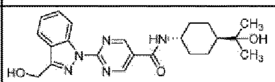 | 1H-NMR (DMSO-D6) δ: 9.24 (2H, s), 8.69 (1H, d, J = 8.6 Hz), 8.55 (1H, d, J = 7.9 Hz), 8.02 (1H, d, J = 7.9 Hz), 7.65-7.61 (1H, m), 7.40 (1H, t, J = 7.2 Hz), 5.57 (1H, t, J = 6.0 Hz), 4.90 (2H, d, J = 6.0 Hz), 4.05 (1H, s), 3.78-3.70 (1H, m), 1.98-1.94 (2H, m), 1.88-1.84 (2H, m), 1.36-1.27 (2H, m), 1.24-1.09 (3H, m), 1.06 (6H, s). | 410 | 408 | |
| 191 | 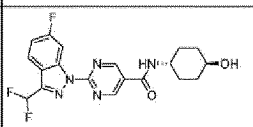 | 1H-NMR (DMSO-D6) δ: 9.30 (2H, s), 8.61 (1H, d, J = 7.4 Hz), 8.54-8.50 (1H, m), 8.04 (1H, dd, J = 8.7, 5.2 Hz), 7.56 (1H, t, J = 53.6 Hz), 7.46-7.41 (1H, m), 4.58 (1H, d, J = 4.6 Hz), 3.81-3.73 (1H, m), 3.47-3.40 (1H, m), 1.90-1.88 (4H, m), 1.45-1.33 (2H, m), 1.32-1.23 (2H, m). | 406 | 404 | |
| 192 | 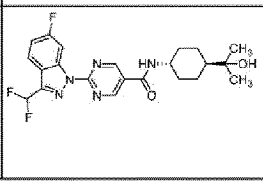 | 1H-NMR (DMSO-D6) δ: 9.31 (2H, s), 8.63 (1H, d, J = 7.6 Hz), 8.54-8.50 (1H, m), 8.04 (1H, dd, J = 8.6, 5.3 Hz), 7.56 (1H, t, J = 53.2 Hz), 7.46-7.40 (1H, m), 4.05 (1H, br s), 3.80-3.70 (1H, m), 1.99-1.95 (2H, m), 1.88-1.84 (2H, m), 1.37-1.06 (5H, m), 1.06 (6H, s). | 448 | 446 | |

[Table 1-25]

| Ex.No. | structural formula | 1H-NMR (400 MHz) | MS (positive) | MS (negative) | Comments |
|---|---|---|---|---|---|
| 193 | 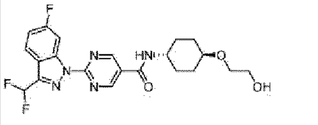 | 1H-NMR (DMSO-D6) δ: 9.30 (2H, s), 8.63 (1H, d, J = 7.6 Hz), 8.54-8.50 (1H, m), 8.06-8.02 (1H, m), 7.56 (1H, t, J = 53.2 Hz), 7.46-7.40 (1H, m), 4.54 (1H, t, J = 5.1 Hz), 3.84-3.77 (1H, m), 3.49-3.40 (4H, m), 3.31-3.24 (1H, m), 2.07-2.02 (2H, m), 1.96-1.91 (2H, m), 1.45-1.36 (2H, m), 1.32-1.22 (2H, m). | 450 | 448 | |
| 194 | 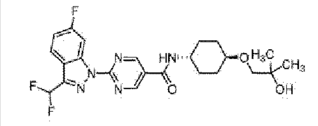 | 1H-NMR (DMSO-D6) δ: 9.30 (2H, d, J = 0.7 Hz), 8.63 (1H, d, J = 7.6 Hz), 8.54-8.51 (1H, m), 8.04 (1H, dd, J = 8.8, 5.3 Hz), 7.56 (1H, t, J = 53.2 Hz), 7.45-7.41 (1H, m), 4.21 (1H, s), 3.86-3.76 (1H, m), 3.19 (2H, s), 3.31-3.22 (1H, m), 2.06-2.01 (2H, m), 1.96-1.91 (2H, m), 1.45-1.23 (4H, m), 1.07 (6H, s). | 478 | 476 | |
| 195 | 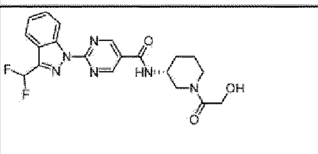 | 1H-NMR (DMSO-D6) δ: 9.30 (1H, s), 9.29 (1H, s), 8.78 (1H, d, J = 8.1 Hz), 8.71 (1H, d, J = 8.3 Hz), 7.99 (1H, d, J = 8.1 Hz), 7.74 (1H, t, J = 8.1 Hz), 7.55 (1H, t, J = 53.0 Hz), 7.51 (2H, t, J = 8.1 Hz), 4.57-4.51 (1H, m), 4.35-3.56 (4H, m), 3.10-2.63 (2H, m), 1.99-1.43 (4H, m). | 431 | 429 | |
| 196 | 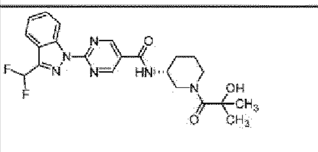 | 1H-NMR (DMSO-D6) δ: 9.30 (2H, s), 8.77 (1H, d, J = 8.1 Hz), 8.69 (1H, d, J = 7.4 Hz), 7.99 (1H, d, J = 8.1 Hz), 7.74 (1H, t, J = 8.1 Hz), 7.55 (1H, t, J = 53.0 Hz), 7.51 (1H, t, J = 8.1 Hz), 5.38 (1H, s), 3.94-3.81 (1H, m), 3.02-2.28 (4H, m), 2.00-1.97 (1H, m), 1.77-1.48 (3H, m), 1.36 (3H, s), 1.33 (3H, s). | 459 | 457 | |
| 197 | 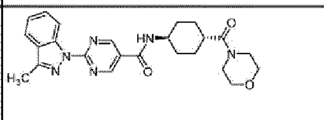 | 1H-NMR (DMSO-D6) δ: 9.23 (2H, s), 8.68 (1H, d, J = 8.6 Hz), 8.59 (1H, d, J = 7.0 Hz), 7.89 (1H, d, J = 7.5 Hz), 7.63 (1H, t, J = 7.8 Hz), 7.40 (1H, t, J = 7.3 Hz), 3.83-3.73 (1H, m), 3.61-3.41 (8H, br m), 2.62 (3H, s), 2.61-2.53 (1H, br m), 2.00-1.92 (2H, br m), 1.80-1.72 (2H, br m), 1.56-1.37 (4H, m). | 449 | 447 | |
| 198 | 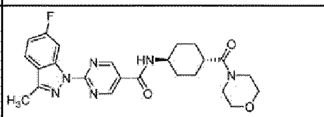 | 1H-NMR (DMSO-D6) δ: 9.23 (2H, s), 8.60 (1H, d, J = 7.5 Hz), 8.41 (1H, d, J = 10.2 Hz), 7.94 (1H, dd, J = 8.6, 5.4 Hz), 7.29 (1H, t, J = 9.2 Hz), 3.84-3.72 (1H, m), 3.62-3.41 (8H, br m), 2.61 (3H, s), 2.60-2.54 (1H, br m), 1.99-1.91 (2H, br m), 1.80-1.72 (2H, br m), 1.56-1.37 (4H, m). | 467 | 465 | |
| 199 | 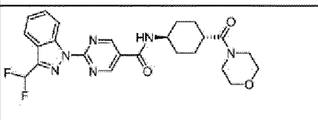 | 1H-NMR (DMSO-D6) δ: 9.31 (2H, s), 8.79 (1H, d, J = 8.6 Hz), 8.69 (1H, d, J = 7.5 Hz), 8.01 (1H, d, J = 8.1 Hz), 7.75 (1H, t, J = 7.8 Hz), 7.57 (1H, t, J = 53.3 Hz), 7.53 (1H, t, J = 7.5 Hz), 3.83-3.74 (1H, m), 3.61-3.41 (8H, br m), 2.63-2.54 (1H, m), 2.01-1.92 (2H, br m), 1.81-1.72 (2H, br m), 1.56-1.38 (4H, m). | 485 | 483 | |
| 200 | 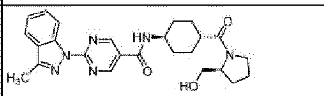 | 1H-NMR (DMSO-D6) δ: 9.22 (2H, s), 8.68 (1H, d, J = 8.3 Hz), 8.59-8.53 (1H, br m), 7.89 (1H, d, J = 7.9 Hz), 7.63 (1H, t, J = 7.7 Hz), 7.40 (1H, t, J = 7.5 Hz), 4.97 (0.4H, t, J = 5.5 Hz), 4.74 (0.6H, t, J = 5.5 Hz), 3.98-3.91 (1H, m), 3.83-3.73 (1H, m), 3.53-3.45 (2H, br m), 3.40-3.28 (1H, m), 3.27-3.19 (1H, m), 2.62 (3H, s), 2.55-2.52 (0.4H, m), 2.42-2.32 (0.6H, m), 2.00-1.33 (12H, br m). | 463 | 461 | optically active form (S) |

[Table 1-26]

| Ex.No. | structural formula | 1H-NMR (400 MHz) | MS (positive) | MS (negative) | Comments |
|---|---|---|---|---|---|
| 201 | 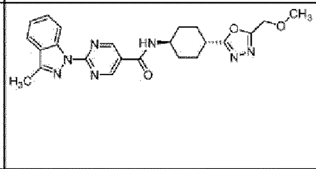 | 1H-NMR (DMSO-D6) δ: 9.24 (2H, s), 8.68 (1H, d, J = 8.6 Hz), 8.64 (1H, d, J = 7.6 Hz), 7.89 (1H, d, J = 7.9 Hz), 7.63 (1H, t, J = 7.7 Hz), 7.40 (1H, t, J = 7.5 Hz), 4.63 (2H, s), 3.92-3.85 (1H, m), 3.35 (3H, s), 3.00 (1H, tt, J = 11.7, 3.5 Hz), 2.63 (3H, s), 2.21-2.13 (2H, br m), 2.08-2.01 (2H, br m), 1.72-1.60 (2H, m), 1.60-1.48 (2H, m). | 448 | 446 | |
| 202 | 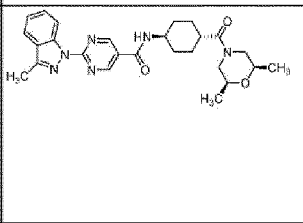 | 1H-NMR (DMSO-D6) δ: 9.22 (2H, s), 8.68 (1H, d, J = 8.6 Hz), 8.58 (1H, d, J = 7.4 Hz), 7.89 (1H, d, J = 8.1 Hz), 7.63 (1H, t, J = 7.7 Hz), 7.40 (1H, t, J = 7.4 Hz), 4.27 (1H, d, J = 12.9 Hz), 3.87 (1H, d, J = 13.4 Hz), 3.82-3.71 (1H, br m), 3.53-3.36 (2H, br m), 2.76-2.67 (1H, m), 2.66-2.56 (4H, br m), 2.25-2.15 (1H, br m), 2.00-1.91 (2H, br m), 1.79-1.69 (2H, br m), 1.57-1.39 (4H, br m), 1.13 (3H, d, J = 6.0 Hz), 1.09 (3H, d, J = 6.0 Hz). | 477 | 475 | |
| 203 | 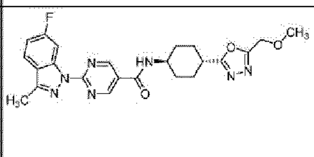 | 1H-NMR (DMSO-D6) δ: 9.25 (2H, s), 8.65 (1H, d, J = 7.4 Hz), 8.41 (1H, d, J = 10.4 Hz), 7.95 (1H, t, J = 6.2 Hz), 7.30 (1H, t, J = 8.9 Hz), 4.63 (2H, s), 3.94-3.84 (1H, br m), 3.35 (3H, s), 3.05-2.96 (1H, m), 2.62 (3H, s), 2.21-2.13 (2H, br m), 2.09-2.01 (2H, br m), 1.60-1.72 (2H, m), 1.60-1.48 (2H, m). | 466 | 464 | |
| 204 | 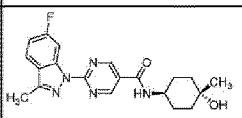 | 1H-NMR (DMSO-D6) δ: 9.22 (2H, s), 8.49 (1H, d, J = 7.5 Hz), 8.43-8.38 (1H, m), 7.97-7.92 (1H, m), 7.33-7.26 (1H, m), 4.30 (1H, s), 3.91-3.81 (1H, m), 2.61 (3H, s), 1.87-1.78 (2H, m), 1.67-1.59 (2H, m), 1.54-1.42 (4H, m), 1.17 (3H, s). | 384 | 382 | |
| 205 | 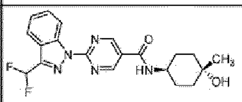 | 1H-NMR (DMSO-D6) δ: 9.30 (2H, s), 8.79 (1H, d, J = 8.6 Hz), 8.57 (1H, d, J = 7.5 Hz), 8.00 (1H, d, J = 8.1 Hz), 7.75 (1H, t, J = 7.5 Hz), 7.57 (1H, t, J = 53.3 Hz), 7.52 (2H, t, J = 7.5 Hz), 4.42-4.19 (1H, m), 3.92-3.82 (1H, m), 1.90-1.78 (2H, m), 1.67-1.59 (2H, m), 1.55-1.42 (4H, m), 1.18 (3H, s). | 402 | 400 | |
| 206 | 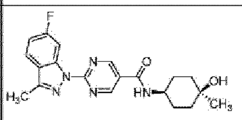 | 1H-NMR (DMSO-D6) δ: 9.25 (2H, s), 8.56 (1H, d, J = 8.1 Hz), 8.40 (1H, dd, J = 10.2, 2.2 Hz), 7.94 (1H, dd, J = 8.6, 5.4 Hz), 7.29 (1H, td, J = 9.0, 2.3 Hz), 4.09 (1H, s), 3.82-3.73 (1H, m), 2.61 (3H, s), 1.83-1.71 (2H, m), 1.66-1.57 (4H, m), 1.43-1.34 (2H, m), 1.13 (3H, s). | 384 | 382 | |
| 207 | 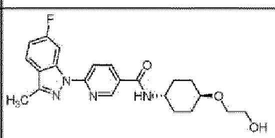 | 1H-NMR (DMSO-D6) δ: 8.98-8.96 (1H, m), 8.49-8.44 (1H, m), 8.42-8.34 (2H, m), 7.99 (1H, d, J = 8.8 Hz), 7.93 (1H, dd, J = 8.7, 5.4 Hz), 7.27-7.23 (1H, m), 4.54 (1H, t, J = 5.2 Hz), 3.83-3.75 (1H, m), 3.52-3.42 (4H, m), 3.30-3.22 (1H, m), 2.61 (3H, s), 2.07-2.00 (2H, m), 1.95-1.86 (2H, m), 1.45-1.34 (2H, m), 1.31-1.20 (2H, m). | 413 | 411 | |
| 208 | 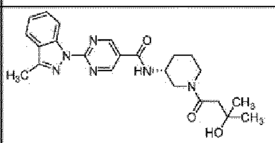 | 1H-NMR (DMSO-D6) δ: 9.25-9.21 (2.0H, m), 8.68-8.60 (2.0H, m), 7.89 (1.0H, d, J = 8.1 Hz), 7.64 (1.0H, t, J = 7.5 Hz), 7.40 (1.0H, t, J = 7.5 Hz), 4.86 (1.0H, d, J = 7.5 Hz), 4.46-4.39 (0.5H, m), 4.13-4.06 (0.5H, m), 4.04-3.98 (0.5H, m), 3.92-3.78 (1.5H, m), 3.16-3.03 (1.0H, m), 2.92-2.84 (0.5H, m), 2.78-2.68 (0.5H, m), 2.62 (3.0H, s), 2.48-2.45 (2.0H, m), 2.04-1.94 (1.0H, m), 1.83-1.74 (1.0H, m), 1.72-1.34 (1.0H, m), 1.22-1.14 (6.0H, m). | 437 | 435 | optically active form (R) |

[Table 1-27]

| Ex.No. | structural formula | 1H-NMR (400 MHz) | MS (positive) | MS (negative) | Comments |
|---|---|---|---|---|---|
| 209 | 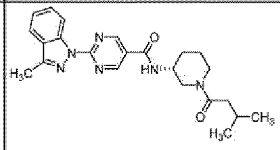 | 1H-NMR (DMSO-D6) δ: 9.27-9.21 (2H, m), 8.69-8.60 (2H, m), 7.89 (1H, d, J = 8.1 Hz), 7.64 (1H, t, J = 7.8 Hz), 7.40 (1H, t, J = 7.5 Hz), 4.45-3.75 (3H, m), 3.10-2.98 (1H, m), 2.83-2.65 (1H, m), 2.62 (3H, s), 2.32-1.98 (4H, m), 1.79-1.41 (3H, m), 0.97-0.86 (6H, m). | 421 | 419 | optically active form (R) |
| 210 | 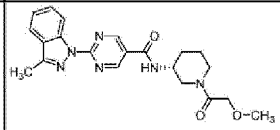 | 1H-NMR (DMSO-D6) δ: 9.25-9.20 (2H, m), 8.72-8.61 (2H, m), 7.89 (1H, d, J = 8.1 Hz), 7.63 (1H, t, J = 7.8 Hz), 7.40 (1H, t, J = 7.5 Hz), 4.37-3.61 (5H, m), 3.32-3.26 (3H, m), 3.11-2.67 (2H, m), 2.62 (3H, s), 2.03-1.94 (1H, m), 1.85-1.75 (1H, m), 1.71-1.38 (2H, m). | 409 | 407 | optically active form (R) |
| 211 | 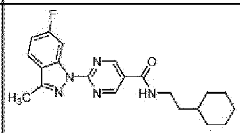 | 1H-NMR (DMSO-D6) δ: 9.23 (2H, s), 8.74 (1H, t, J = 5.4 Hz), 8.41 (1H, dd, J = 10.4, 2.3 Hz), 7.94 (1H, dd, J = 8.7, 5.4 Hz), 7.29 (1H, td, J = 8.9, 2.1 Hz), 3.39-3.32 (2H, m), 2.61 (3H, s), 1.79-1.59 (5H, m), 1.51-1.43 (2H, m), 1.41-1.30 (1H, m), 1.28-1.12 (3H, m), 0.97-0.88 (2H, m). | 382 | 380 | |
| 212 | 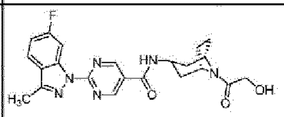 | 1H NMR (DMSO D6) δ: 9.22 (2H, s), 8.57 (1H, d, J = 7.6 Hz), 8.40 (1H, d, J = 9.7 Hz), 7.99-7.90 (1H, m), 7.30 (1H, t, J = 8.9 Hz), 4.66 (1H, t, J = 5.1 Hz), 4.60-4.51 (1H, m), 4.50-4.40 (1H, m), 4.32-4.23 (1H, m), 4.14 (1H, dd, J = 15.0, 5.5 Hz), 4.01 (1H, dd, J = 15.3, 5.5 Hz), 2.61 (3H, s), 2.12-1.56 (8H, m). | 439 | 437 | exo form |
| 213 | 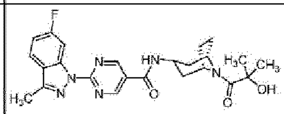 | 1H NMR (DMSO D6) δ: 9.23 (2H, s), 8.58 (1H, d, J = 7.6 Hz), 8.40 (1H, d, J = 10.2 Hz), 7.94 (1H, dd, J = 8.9, 5.5 Hz), 7.29 (1H, dd, J = 8.9, 8.9 Hz), 5.25 (1H, br s), 5.19-5.03 (1H, m), 4.69-4.53 (1H, m), 4.52-4.36 (1H, m), 2.61 (3H, s), 2.10-1.57 (8H, m), 1.34 (6H, s). | 467 | 465 | exo form |
| 214 | 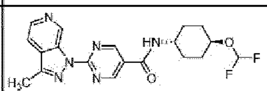 | 1H-NMR (DMSO-D6) δ: 9.98 (1H, s), 9.27 (2H, s), 8.60 (1H, d, J = 7.2 Hz), 8.53 (1H, d, J = 5.1 Hz), 7.94 (1H, d, J = 5.1 Hz), 6.73 (1H, t, J = 76.5 Hz), 4.15-4.02 (1H, m), 3.92-3.76 (1H, m), 2.66 (3H, s), 2.11-1.87 (4H, m), 1.62-1.38 (4H, m). | 403 | 401 | |
| 215 | 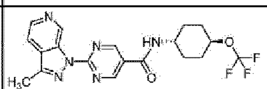 | 1H-NMR (DMSO-D6) δ: 9.99 (1H, s), 9.28 (2H, s), 8.64 (1H, d, J = 7.4 Hz), 8.53 (1H, d, J = 5.3 Hz), 7.94 (1H, d, J = 5.3 Hz), 4.48-4.36 (1H, m), 3.93-3.79 (1H, m), 2.66 (3H, s), 2.17-1.91 (4H, m), 1.73-1.43 (4H, m). | 421 | 419 | |
| 216 | 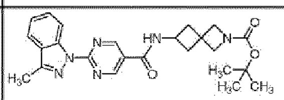 | 1H-NMR (DMSO-D6) δ: 9.22 (2H, s), 8.90 (1H, d, J = 6.9 Hz), 8.67 (1H, d, J = 8.6 Hz), 7.89 (1H, d, J = 7.9 Hz), 7.63 (1H, dd, J = 8.6, 7.5 Hz), 7.40 (1H, dd, J = 7.9, 7.5 Hz), 4.38-4.25 (1H, m), 3.94 (2H, s), 3.82 (2H, s), 2.62 (3H, s), 2.60-2.52 (2H, m), 2.32-2.23 (2H, m), 1.38 (9H, s). | 449 | 447 | |

[Table 1-28]

| Ex.No. | structural formula | 1H-NMR (400 MHz) | MS (positive) | MS (negative) | Comments |
|---|---|---|---|---|---|
| 217 | 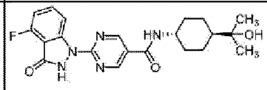 | 1H-NMR (DMSO-D6) δ: 9.01 (2H, s), 8.45-8.24 (2H, m), 7.43-7.21 (1H, m), 6.91-6.66 (1H, m), 4.04 (1H, s), 3.78-3.65 (1H, m), 2.01-1.78 (4H, m), 1.43-1.08 (5H, m), 1.05 (6H, s). | 414 | 412 | |
| 218 | 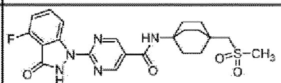 | 1H-NMR (DMSO-D6) δ: 9.32-8.85 (2H, m), 8.41-8.26 (1H, m), 8.20-7.74 (1H, m), 7.46-7.19 (1H, m), 6.95-6.70 (1H, m), 3.07 (2H, s), 2.95 (3H, s), 2.15-1.91 (6H, m), 1.90-1.71 (6H, m). | 474 | 472 | |
| 219 | 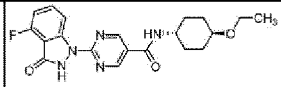 | 1H-NMR (DMSO-D6) δ: 9.19-9.00 (2H, m), 8.54-8.32 (2H, m), 7.44-7.28 (1H, m), 6.91-6.79 (1H, m), 3.83-3.70 (1H, m), 3.47 (2H, q, J = 6.9 Hz), 3.27-3.18 (1H, m), 2.08-1.97 (2H, m), 1.95-1.83 (2H, m), 1.52-1.32 (2H, m), 1.32-1.17 (2H, m), 1.10 (3H, t, J = 6.9 Hz). | 400 | 398 | |
| 220 | 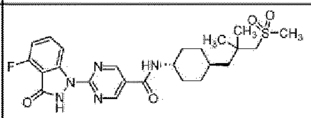 | 1H-NMR (DMSO-D6) δ: 9.05 (2H, s), 8.46-8.30 (2H, m), 7.37-7.24 (1H, m), 6.86-6.73 (1H, m), 3.78-3.64 (1H, m), 3.16 (2H, s), 2.97 (3H, s), 1.91-1.73 (4H, m), 1.49-1.30 (5H, m), 1.21-1.02 (2H, m), 1.15 (6H, s). | 504 | 502 | |
| 221 | 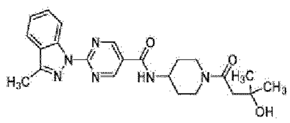 | 1H-NMR (DMSO-D6) δ: 9.23 (2H, s), 8.68 (1H, d, J = 8.6 Hz), 8.62 (1H, d, J = 7.5 Hz), 7.89 (1H, d, J = 7.5 Hz), 7.63 (1H, t, J = 7.8 Hz), 7.40 (1H, t, J = 7.5 Hz), 4.89 (1H, s), 4.42 (1H, d, J = 13.5 Hz), 4.13-4.01 (2H, m), 3.21-3.14 (1H, m), 2.81-2.74 (1H, m), 2.62 (3H, s), 2.54-2.43 (2H, m), 1.93-1.87 (2H, m), 1.56-1.34 (2H, m), 1.19 (6H, s). | 437 | 435 | |
| 222 | 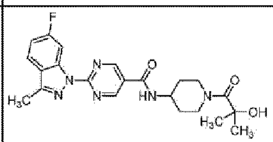 | 1H-NMR (DMSO-D6) δ: 9.24 (2H, s), 8.63 (1H, d, J = 7.5 Hz), 8.40 (1H, dd, J = 10.2, 2.2 Hz), 7.94 (1H, dd, J = 8.6, 5.4 Hz), 7.29 (1H, td, J = 9.0, 2.3 Hz), 5.40 (1H, s), 4.86-4.32 (2H, br m), 4.13-4.05 (1H, m), 3.26-2.73 (2H, br m), 2.61 (3H, s), 1.92-1.88 (2H, m), 1.54-1.43 (2H, m), 1.34 (6H, s). | 441 | 439 | |
| 223 | 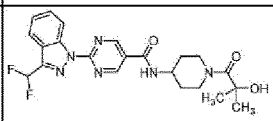 | 1H-NMR (DMSO-D6) δ: 9.32 (2H, s), 8.79 (1H, d, J = 8.6 Hz), 8.71 (1H, d, J = 7.5 Hz), 8.00 (1H, d, J = 8.1 Hz), 7.76 (1H, t, J = 7.8 Hz), 7.57 (1H, t, J = 53.3 Hz), 7.52 (1H, t, J = 7.5 Hz), 5.40 (1H, s), 4.88-4.29 (2H, br m), 4.15-4.07 (1H, m), 3.25-2.73 (2H, br m), 1.93-1.89 (2H, m), 1.53-1.46 (2H, m), 1.34 (6H, s). | 459 | 457 | |
| 224 | 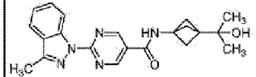 | 1H-NMR (DMSO-D6) δ: 9.22 (1H, d, J = 5.5 Hz), 9.21 (2H, s), 8.68-8.66 (1H, m), 7.90-7.87 (1H, m), 7.65-7.61 (1H, m), 7.42-7.38 (1H, m), 4.20 (1H, s), 2.62 (3H, s), 1.96 (6H, s), 1.09 (6H, s). | 378 | 376 | |

[Table 1-29]

| Ex.No. | structural formula | 1H-NMR (400 MHz) | MS (positive) | MS (negative) | Comments |
|---|---|---|---|---|---|
| 225 | 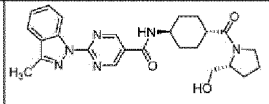 | 1H-NMR (DMSO-D6) δ: 9.22 (2H, s), 8.68 (1H, d, J = 8.3 Hz), 8.59-8.53 (1H, br m), 7.89 (1H, d, J = 7.9 Hz), 7.63 (1H, t, J = 7.7 Hz), 7.40 (1H, t, J = 7.5 Hz), 4.97 (0H, t, J = 5.5 Hz), 4.74 (1H, t, J = 5.5 Hz), 3.98-3.91 (1H, m), 3.83-3.73 (1H, m), 3.53-3.45 (2H, br m), 3.40-3.28 (1H, m), 3.27-3.19 (1H, m), 2.62 (3H, s), 2.55-2.52 (0H, m), 2.42-2.32 (1H, m), 2.00-1.33 (12H, br m). | 463 | 461 | optically active form (R) |
| 226 | 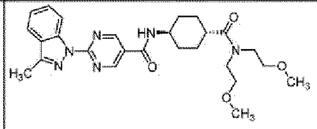 | 1H-NMR (DMSO-D6) δ: 9.22 (2H, s), 8.68 (1H, d, J = 8.6 Hz), 8.55 (1H, d, J = 7.6 Hz), 7.89 (1H, d, J = 7.9 Hz), 7.66-7.60 (1H, m), 7.40 (1H, t, J = 7.4 Hz), 3.84-3.73 (1H, br m), 3.55 (2H, t, J = 5.2 Hz), 3.50-3.36 (6H, br m), 3.28 (3H, s), 3.24 (3H, s), 2.65-2.56 (4H, br m), 1.99-1.91 (2H, br m), 1.76-1.69 (2H, br m), 1.54-1.37 (4H, m). | 495 | 493 | |
| 227 | 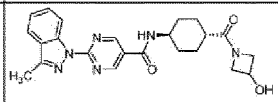 | 1H-NMR (DMSO-D6) δ: 9.22 (2H, s), 8.67 (1H, d, J = 8.3 Hz), 8.56 (1H, d, J = 7.6 Hz), 7.89 (1H, d, J = 7.9 Hz), 7.63 (1H, t, J = 7.9 Hz), 7.40 (1H, t, J = 7.5 Hz), 5.69 (1H, br s), 4.44 (1H, br s), 4.35 (1H, t, J = 7.4 Hz), 4.01 (1H, t, J = 7.6 Hz), 3.89 (1H, dd, J = 9.0, 3.7 Hz), 3.82-3.70 (1H, br m), 3.56 (1H, dd, J = 9.2, 3.5 Hz), 2.62 (3H, s), 2.22-2.13 (1H, br m), 2.01-1.90 (2H, br m), 1.81-1.68 (2H, br m), 1.50-1.32 (4H, br m). | 435 | 433 | |
| 228 | 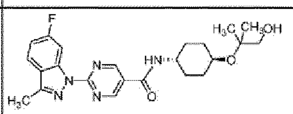 | 1H-NMR (DMSO-D6) δ: 9.22 (2H, s), 8.52 (1H, d, J = 7.6 Hz), 8.41 (1H, dd, J = 10.4, 2.3 Hz), 7.95 (1H, dd, J = 8.9, 5.3 Hz), 7.29 (1H, td, J = 8.9, 2.3 Hz), 4.53 (1H, br s), 3.80-3.68 (1H, m), 3.56-3.46 (1H, m), 3.21 (2H, d, J = 8.8 Hz), 2.61 (3H, s), 1.94-1.77 (4H, m), 1.49-1.22 (4H, m), 1.09 (6H, s). | 442 | 440 | |
| 229 | 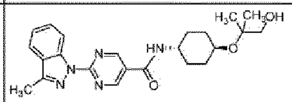 | 1H-NMR (DMSO-D6) δ: 9.21 (2H, s), 8.67 (1H, d, J = 8.3 Hz), 8.51 (1H, d, J = 7.4 Hz), 7.89 (1H, d, J = 7.6 Hz), 7.63 (1H, dd, J = 8.3, 7.5 Hz), 7.40 (1H, dd, J = 7.6, 7.5 Hz), 4.53 (1H, br s), 3.82-3.67 (1H, m), 3.58-3.45 (1H, m), 3.22 (2H, s), 2.62 (3H, s), 1.97-1.76 (4H, m), 1.51-1.22 (4H, m), 1.09 (6H, s). | 424 | 422 | |
| 230 | 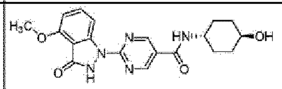 | 1H-NMR (DMSO-D6) δ: 9.02 (2H, br s), 8.35 (1H, br s), 8.15 (1H, d, J = 8.1 Hz), 7.20 (1H, t, J = 8.1 Hz), 6.58 (1H, d, J = 7.5 Hz), 4.57 (1H, br s), 3.84 (3H, s), 3.79-3.66 (1H, m), 3.49-3.39 (1H, m), 1.94-1.78 (4H, m), 1.52-1.16 (4H, m). | 384 | 382 | |
| 231 | 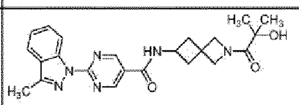 | 1H-NMR (DMSO-D6) δ: 9.22 (2H, s), 8.92 (1H, d, J = 6.9 Hz), 8.68 (1H, d, J = 8.6 Hz), 7.89 (1H, d, J = 8.1 Hz), 7.63 (1H, dd, J = 8.6, 7.5 Hz), 7.40 (1H, dd, J = 8.1, 7.5 Hz), 5.02 (1H, d, J = 6.9 Hz), 4.47 (1H, s), 4.40-4.27 (1H, m), 4.35 (1H, s), 3.94 (1H, s), 3.83 (1H, s), 2.62 (3H, s), 2.60-2.52 (2H, m), 2.35-2.23 (2H, m), 1.23 (6H, s). | 435 | 433 | |
| 232 | 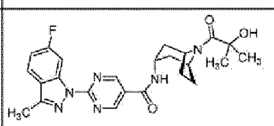 | 1H-NMR (DMSO-D6) δ: 9.18 (2H, s), 8.48 (1H, d, J = 4.6 Hz), 8.44 (1H, dd, J = 10.2, 2.1 Hz), 7.95 (1H, dd, J = 8.9, 5.3 Hz), 7.30 (1H, ddd, J = 8.9, 8.9, 2.1 Hz), 5.27 (1H, br s), 5.08-4.94 (1H, m), 4.62-4.48 (1H, m), 4.09-3.97 (1H, m), 2.62 (3H, s), 2.35-1.70 (8H, m), 1.41-1.23 (6H, m). | 467 | 465 | endo form |

[Table 1-30]

| Ex.No. | structural formula | 1H-NMR (400 MHz) | MS (positive) | MS (negative) | Comments |
|---|---|---|---|---|---|
| 233 | 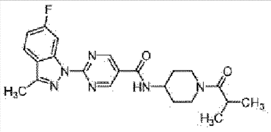 | 1H-NMR (DMSO-D6) δ: 9.24 (2H, s), 8.62 (1H, d, J = 7.6 Hz), 8.41 (1H, dd, J = 10.5, 2.2 Hz), 7.95 (1H, dd, J = 8.9, 5.3 Hz), 7.30 (1H, ddd, J = 8.9, 8.9, 2.2 Hz), 4.43-4.32 (1H, m), 4.16-4.05 (1H, m), 4.03-3.93 (1H, m), 3.25-3.13 (1H, m), 2.97-2.86 (1H, m), 2.80-2.69 (1H, m), 2.61 (3H, s), 1.99-1.83 (2H, m), 1.56-1.32 (2H, m), 1.07-0.96 (6H, m). | 425 | 423 | |
| 234 | 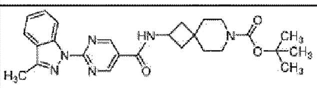 | 1H-NMR (DMSO-D6) δ: 9.23 (2H, s), 8.91 (1H, d, J = 7.2 Hz), 8.68 (1H, d, J = 8.6 Hz), 7.89 (1H, d, J = 8.1 Hz), 7.63 (1H, ddd, J = 8.6, 7.4, 1.9 Hz), 7.40 (1H, dd, J = 8.1, 7.4 Hz), 4.51-4.39 (1H, m), 3.33-3.27 (2H, m), 3.26-3.19 (2H, m), 2.62 (3H, s), 2.30-2.21 (2H, m), 1.91-1.83 (2H, m), 1.55 (2H, t, J = 5.5 Hz), 1.49 (2H, t, J = 5.5 Hz), 1.39 (9H, s). | 477 | 475 | |
| 235 | 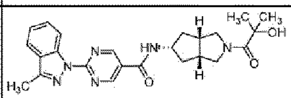 | 1H-NMR (DMSO-D6) δ: 9.22 (2H, s), 8.73 (1H, d, J = 7.4 Hz), 8.67 (1H, d, J = 8.6 Hz), 7.89 (1H, d, J = 7.9 Hz), 7.63 (1H, t, J = 7.7 Hz), 7.40 (1H, t, J = 7.5 Hz), 5.16 (1H, s), 4.39-4.26 (1H, m), 3.99-3.66 (2H, m), 3.57-3.38 (2H, m), 2.71-2.42 (2H, m), 2.62 (3H, s), 2.36-2.14 (2H, m), 1.52-1.37 (2H, m), 1.31 (6H, s). | 449 | 447 | cis form |
| 236 | 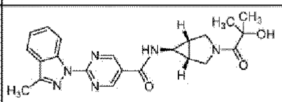 | 1H-NMR (DMSO-D6) δ: 9.20 (2H, s), 8.86 (1H, d, J = 3.9 Hz), 8.67 (1H, d, J = 8.6 Hz), 7.89 (1H, d, J = 7.6 Hz), 7.63 (1H, dd, J = 8.6, 7.4 Hz), 7.40 (1H, dd, J = 7.6, 7.4 Hz), 5.22 (1H, s), 4.43-4.32 (1H, m), 3.86-3.77 (1H, m), 3.72-3.60 (1H, m), 3.43-3.35 (1H, m), 3.34-3.27 (1H, m), 2.62 (3H, s), 2.00-1.90 (1H, m), 1.84-1.74 (1H, m), 1.28 (6H, s). | 421 | 419 | trans form |
| 237 | 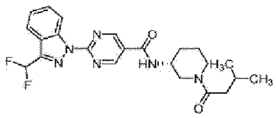 | 1H-NMR (DMSO-D6) δ: 9.31 (1H, s), 9.29 (1H, s), 8.78 (1H, d, J = 7.9 Hz), 8.69 (1H, d, J = 7.6 Hz), 7.99 (1H, d, J = 7.9 Hz), 7.75 (1H, t, J = 7.9 Hz), 7.55 (1H, t, J = 53.0 Hz), 7.51 (1H, t, J = 7.9 Hz), 4.40-3.76 (3H, m), 3.08-2.62 (2H, m), 2.20-1.95 (3H, m), 1.78-1.37 (4H, m), 0.91-0.85 (6H, m). | 457 | 455 | |
| 238 | 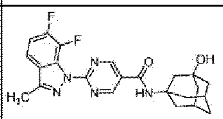 | 1H-NMR (DMSO-D6) δ: 9.17 (2H, s), 8.16 (1H, s), 7.75-7.72 (1H, m), 7.49-7.42 (1H, m), 4.53 (1H, s), 2.58 (3H, s), 2.19-2.17 (2H, br m), 2.01-1.92 (6H, m), 1.60-1.45 (6H, m). | 440 | 438 | |
| 239 | 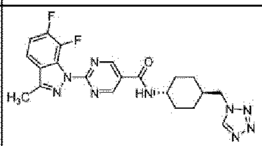 | 1H-NMR (DMSO-D6) δ: 9.21 (2H, s), 8.97 (1H, s), 8.63 (1H, d, J = 7.9 Hz), 7.75-7.72 (1H, m), 7.49-7.43 (1H, m), 4.61 (2H, d, J = 6.9 Hz), 3.80-3.70 (1H, m), 2.58 (3H, s), 1.97-1.90 (3H, m), 1.61-1.58 (2H, m), 1.37-1.14 (4H, m). | 454 | 452 | |
| 240 | 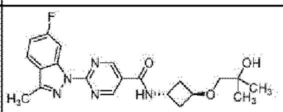 | 1H-NMR (DMSO-D6) δ: 9.25 (2H, s), 8.97 (1H, d, J = 6.5 Hz), 8.41 (1H, dd, J = 10.2, 2.2 Hz), 7.95 (1H, dd, J = 8.6, 5.4 Hz), 7.30 (1H, td, J = 8.9, 2.2 Hz), 4.50-4.45 (1H, m), 4.32 (1H, s), 4.19-4.14 (1H, m), 3.07 (2H, s), 2.61 (3H, s), 2.32-2.29 (4H, m), 1.10 (6H, s). | 414 | 412 | |

[Table 1-31]

| Ex.No. | structural formula | 1H-NMR (400 MHz) | MS (positive) | MS (negative) | Comments |
|---|---|---|---|---|---|
| 241 | 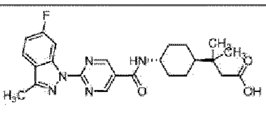 | 1H-NMR (DMSO-D6) δ: 9.27-9.26 (2H, m), 8.83-8.73 (1H, br m), 8.40 (1H, dd, J = 10.4, 2.3 Hz), 7.94 (1H, dd, J = 8.7, 5.4 Hz), 7.31-7.26 (1H, m), 3.75-3.65 (1H, m), 2.61 (3H, s), 1.94-1.90 (2H, m), 1.86-1.83 (2H, m), 1.85 (2H, s), 1.48-1.41 (1H, m), 1.36-1.27 (2H, m), 1.11-1.02 (2H, m), 0.91 (6H, s). | 454 | 452 | |
| 242 | 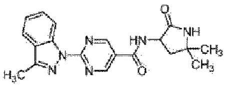 | 1H-NMR (DMSO-D6) δ: 9.25 (2H, s), 9.04 (1H, d, J = 8.0 Hz), 8.68 (1H, d, J = 8.6 Hz), 8.10 (1H, s), 7.89 (1H, d, J = 8.0 Hz), 7.66-7.62 (1H, m), 7.42-7.38 (1H, m), 4.81-4.74 (1H, m), 2.62 (3H, s), 2.31 (1H, dd, J = 12.1, 8.8 Hz), 1.88 (1H, dd, J = 12.1, 10.9 Hz), 1.29 (3H, s), 1.25 (3H, s). | 365 | 363 | race-mate |
| 243 | 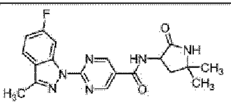 | 1H-NMR (DMSO-D6) δ: 9.26 (2H, s), 9.05 (1H, d, J = 8.1 Hz), 8.41 (1H, dd, J = 10.4, 2.3 Hz), 8.11 (1H, br s), 7.94 (1H, dd, J = 8.7, 5.4 Hz), 7.32-7.27 (1H, m), 4.81-4.74 (1H, m), 2.61 (3H, s), 2.31 (1H, dd, J = 12.3, 8.8 Hz), 1.88 (1H, dd, J = 12.3, 10.9 Hz), 1.29 (3H, s), 1.25 (3H, s). | 383 | 381 | race-mate |
| 244 | 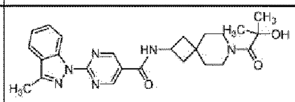 | 1H-NMR (DMSO-D6) δ: 9.23 (2H, s), 8.92 (1H, d, J = 7.2 Hz), 8.68 (1H, d, J = 8.6 Hz), 7.89 (1H, d, J = 8.1 Hz), 7.64 (1H, ddd, J = 8.6, 7.5, 1.9 Hz), 7.40 (1H, dd, J = 8.1, 7.5 Hz), 5.33 (1H, s), 4.54-4.40 (1H, m), 3.97-3.36 (4H, m), 2.62 (3H, s), 2.32-2.23 (2H, m), 1.94-1.86 (2H, m), 1.66-1.49 (4H, m), 1.31 (6H, s). | 463 | 461 | |
| 245 | 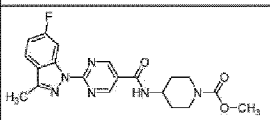 | 1H-NMR (DMSO-D6) δ: 9.24 (2H, s), 8.62 (1H, d, J = 7.4 Hz), 8.41 (1H, dd, J = 10.4, 2.1 Hz), 7.95 (1H, dd, J = 8.9, 5.3 Hz), 7.30 (1H, ddd, J = 8.9, 8.9, 2.1 Hz), 4.11-3.88 (3H, m), 3.61 (3H, s), 3.09-2.87 (2H, m), 2.61 (3H, s), 1.92-1.81 (2H, m), 1.54-1.39 (2H, m). | 413 | 411 | |
| 246 | 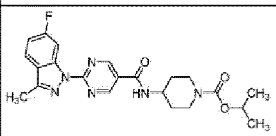 | 1H-NMR (DMSO-D6) δ: 9.24 (2H, s), 8.61 (1H, d, J = 7.6 Hz), 8.41 (1H, dd, J = 10.4, 2.3 Hz), 7.95 (1H, dd, J = 8.9, 5.4 Hz), 7.30 (1H, ddd, J = 8.9, 8.9, 2.3 Hz), 4.83-4.74 (1H, m), 4.10-3.89 (3H, m), 3.05-2.84 (2H, m), 2.61 (3H, s), 1.94-1.81 (2H, m), 1.54-1.38 (2H, m), 1.20 (6H, d, J = 6.2 Hz). | 441 | 439 | |
| 247 | 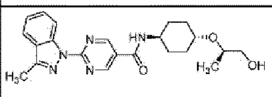 | 1H-NMR (DMSO-D6) δ: 9.22 (2H, s), 8.67 (1H, d, J = 8.6 Hz), 8.53 (1H, d, J = 7.6 Hz), 7.89 (1H, d, J = 7.9 Hz), 7.65-7.61 (1H, m), 7.42-7.38 (1H, m), 4.49 (1H, t, J = 5.8 Hz), 3.82-3.74 (1H, m), 3.42-3.32 (3H, m), 3.25-3.19 (1H, m), 2.62 (3H, s), 2.03-1.87 (4H, br m), 1.46-1.33 (2H, br m), 1.33-1.21 (2H, br m), 1.04 (3H, d, J = 6.2 Hz). | 410 | 408 | opti-cally active form (R) |
| 248 | 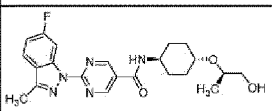 | 1H-NMR (DMSO-D6) δ: 9.23 (2H, s), 8.54 (1H, d, J = 7.6 Hz), 8.40 (1H, dd, J = 10.3, 2.2 Hz), 7.94 (1H, dd, J = 8.7, 5.4 Hz), 7.29 (1H, td, J = 8.9, 2.2 Hz), 4.49 (1H, t, J = 5.8 Hz), 3.81-3.74 (1H, m), 3.56-3.47 (1H, m), 3.42-3.30 (2H, m), 3.25-3.18 (1H, m), 2.61 (3H, s), 2.03-1.88 (4H, br m), 1.46-1.20 (4H, br m), 1.04 (3H, d, J = 6.0 Hz). | 428 | 426 | opti-cally active form (R) |

[Table 1-32]

| Ex.No. | structural formula | 1H-NMR (400 MHz) | MS (positive) | MS (negative) | Comments |
|---|---|---|---|---|---|
| 249 | 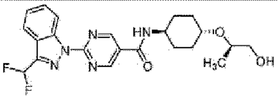 | 1H-NMR (DMSO-D6) δ: 9.30 (2H, s), 8.78 (1H, d, J = 8.6 Hz), 8.62 (1H, d, J = 7.6 Hz), 8.00 (1H, d, J = 8.1 Hz), 7.78-7.73 (1H, m), 7.56 (1H, t, J = 53.4 Hz), 7.55-7.50 (1H, m), 4.50 (1H, t, J = 5.8 Hz), 3.85-3.73 (1H, m), 3.56-3.47 (1H, m), 3.42-3.32 (2H, m), 3.26-3.18 (1H, m), 2.03-1.89 (4H, br m), 1.47-1.34 (2H, m), 1.33-1.21 (2H, m), 1.04 (3H, d, J = 6.0 Hz). | 446 | 444 | optically active form (R) |
| 250 | 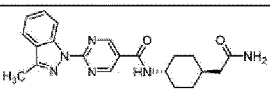 | 1H-NMR (DMSO-D6) δ: 9.22 (2H, s), 8.67 (1H, d, J = 8.6 Hz), 8.55 (1H, d, J = 7.6 Hz), 7.89 (1H, d, J = 7.9 Hz), 7.65-7.61 (1H, m), 7.42-7.38 (1H, m), 7.25 (1H, br s), 6.72 (1H, br s), 3.80-3.73 (1H, m), 2.62 (3H, s), 1.97 (2H, d, J = 6.9 Hz), 1.93-1.89 (2H, m), 1.78-1.74 (2H, m), 1.69-1.62 (1H, m), 1.40-1.32 (2H, m), 1.10-1.01 (2H, m). | 393 | 391 | |
| 251 | 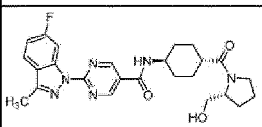 | 1H-NMR (DMSO-D6) δ: 9.23 (2H, s), 8.61-8.55 (1H, br m), 8.41 (1H, dd, J = 10.4, 2.3 Hz), 7.95 (1H, dd, J = 8.8, 5.3 Hz), 7.29 (1H, td, J = 8.9, 2.2 Hz), 4.97 (0.4H, t, J = 5.7 Hz), 4.74 (0.6H, t, J = 5.7 Hz), 3.99-3.91 (1H, m), 3.83-3.72 (1H, m), 3.54-3.45 (2H, m), 3.39-3.31 (1H, m), 3.28-3.19 (1H, m), 2.61 (3H, s), 2.55-2.52 (0.4H, br m), 2.42-2.33 (0.6H, br m), 2.00-1.35 (12H, br m). | 481 | 479 | optically active form (R) |
| 252 | 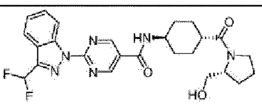 | 1H-NMR (DMSO-D6) δ: 9.30 (2H, s), 8.79 (1H, d, J = 8.8 Hz), 8.68-8.63 (1H, br m), 8.00 (1H, d, J = 8.1 Hz), 7.78-7.72 (1H, m), 7.56 (1H, t, J = 53.4 Hz), 7.52 (1H, t, J = 7.5 Hz), 4.97 (0.4H, t, J = 5.7 Hz), 4.74 (0.6H, t, J = 5.7 Hz), 3.99-3.91 (1H, m), 3.85-3.73 (1H, m), 3.53-3.45 (2H, m), 3.38-3.32 (1H, m), 3.27-3.20 (1H, m), 2.58-2.51 (0.4H, br m), 2.42-2.32 (0.6H, br m), 2.01-1.37 (12H, br m). | 499 | 497 | optically active form (R) |
| 253 | 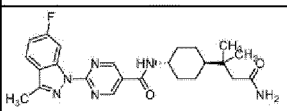 | 1H-NMR (DMSO-D6) δ: 9.23 (2H, t, J = 15.7 Hz), 8.53 (1H, d, J = 7.9 Hz), 8.40 (1H, dd, J = 10.4, 2.2 Hz), 7.94 (1H, dd, J = 8.9, 5.3 Hz), 7.32-7.27 (1H, m), 7.19 (1H, br s), 6.66 (1H, br s), 3.79-3.69 (1H, m), 2.61 (3H, s), 1.99-1.94 (2H, m), 1.98 (2H, s), 1.98-1.95 (2H, m), 1.36-1.19 (3H, m), 1.16-1.06 (2H, m), 0.94 (6H, s). | 453 | 451 | |
| 254 | 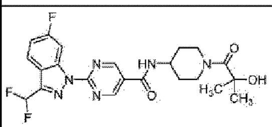 | 1H-NMR (DMSO-D6) δ: 9.32 (2H, s), 8.72 (1H, d, J = 7.6 Hz), 8.53 (1H, dd, J = 10.2, 2.3 Hz), 8.05 (1H, dd, J = 8.8, 5.3 Hz), 7.56 (1H, t, J = 53.4 Hz), 7.46-7.41 (1H, m), 5.40 (1H, s), 4.90-4.25 (2H, br m), 4.16-4.06 (1H, m), 3.31-3.06 (2H, br m), 1.92-1.90 (2H, m), 1.57-1.42 (2H, m), 1.34 (6H, s). | 477 | 475 | |
| 255 | 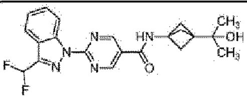 | 1H-NMR (DMSO-D6) δ: 9.32 (1H, br s), 9.30 (2H, s), 8.78 (1H, d, J = 8.6 Hz), 8.01-7.99 (1H, m), 7.77-7.73 (1H, m), 7.56 (1H, t, J = 53.4 Hz), 7.54-7.50 (1H, m), 4.21 (1H, s), 1.97 (6H, s), 1.09 (6H, s). | 414 | 412 | |
| 256 | 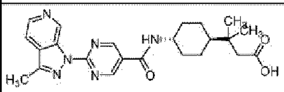 | 1H-NMR (DMSO-D6) δ: 9.99 (1H, s), 9.28 (2H, s), 8.59 (1H, br s), 8.53 (1H, d, J = 5.5 Hz), 7.94 (1H, d, J = 5.5 Hz), 3.81-3.69 (1H, m), 2.66 (3H, s), 2.10 (2H, s), 2.02-1.92 (2H, m), 1.87-1.76 (2H, m), 1.41-1.26 (3H, m), 1.21-1.09 (2H, m), 0.97 (6H, s). | 437 | 435 | |

[Table 1-33]

| Ex.No. | structural formula | 1H-NMR (400 MHz) | MS (positive) | MS (negative) | Comments |
|---|---|---|---|---|---|
| 257 | 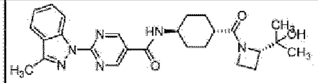 | 1H-NMR (DMSO-D6) δ: 9.22 (2H, s), 8.67 (1H, d, J = 8.6 Hz), 8.57 (1H, d, J = 7.5 Hz), 7.89 (1H, d, J = 8.1 Hz), 7.63 (1H, dd, J = 8.6, 7.5 Hz), 7.40 (1H, dd, J = 8.1, 7.5 Hz), 4.23 (1H, t, J = 7.5 Hz), 4.16-4.07 (1H, m), 4.07-3.96 (1H, m), 3.87-3.28 (2H, m), 2.62 (3H, s), 2.27-2.14 (2H, m), 2.02-1.87 (3H, m), 1.85-1.69 (2H, m), 1.56-1.30 (4H, m), 1.10 (3H, s), 0.97 (3H, s). | 477 | 475 | optically active form (S) |
| 258 | 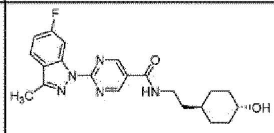 | 1H-NMR (DMSO-D6) δ: 9.23 (2H, s), 8.77-8.73 (1H, m), 8.43-8.38 (1H, m), 7.97-7.92 (1H, m), 7.32-7.26 (1H, m), 4.45 (1H, d, J = 4.3 Hz), 3.37-3.29 (3H, m), 2.61 (3H, s), 1.86-1.71 (4H, m), 1.48-1.42 (2H, m), 1.31-1.22 (1H, m), 1.19-1.07 (2H, m), 1.00-0.88 (2H, m). | 398 | 396 | |
| 259 | 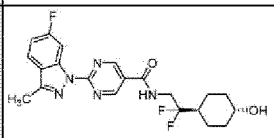 | 1H-NMR (DMSO-D6) δ: 9.28 (2H, s), 9.10-9.06 (1H, m), 8.44-8.39 (1H, m), 7.97-7.92 (1H, m), 7.33-7.27 (1H, m), 4.58 (1H, d, J = 4.3 Hz), 3.83 (2H, td, J = 15.4, 5.9 Hz), 3.39-3.32 (1H, m), 2.61 (3H, s), 1.94-1.82 (5H, m), 1.29-1.10 (4H, m). | 434 | 432 | |
| 260 | 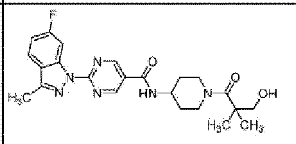 | 1H-NMR (DMSO-D6) δ: 9.25 (2H, s), 8.63 (1H, d, J = 7.0 Hz), 8.41 (1H, d, J = 10.8 Hz), 7.97-7.93 (1H, m), 7.32-7.27 (1H, m), 4.53 (1H, t, J = 5.9 Hz), 4.30-4.26 (2H, m), 4.16-4.05 (1H, m), 3.44-3.43 (2H, m), 3.00-2.94 (2H, m), 2.61 (3H, s), 1.92-1.89 (2H, m), 1.50-1.42 (2H, m), 1.18 (6H, s). | 455 | 453 | |
| 261 | 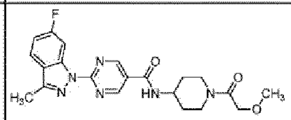 | 1H-NMR (DMSO-D6) δ: 9.25 (2H, s), 8.64 (1H, d, J = 7.5 Hz), 8.41 (1H, d, J = 10.2 Hz), 7.97-7.93 (1H, m), 7.32-7.28 (1H, m), 4.32-4.29 (1H, m), 4.17-4.04 (3H, m), 3.83-3.80 (1H, m), 3.31 (3H, s), 3.17-3.09 (1H, m), 2.83-2.76 (1H, m), 2.61 (3H, s), 1.94-1.89 (2H, m), 1.57-1.36 (2H, m). | 427 | 425 | |
| 262 | 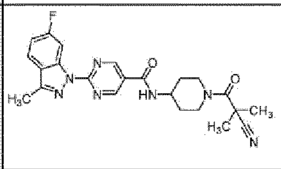 | 1H-NMR (DMSO-D6) δ: 9.25 (2H, s), 8.68 (1H, d, J = 7.5 Hz), 8.42-8.39 (1H, m), 7.95 (1H, dd, J = 8.6, 4.8 Hz), 7.32-7.27 (1H, m), 4.34-4.11 (3H, m), 3.25-2.67 (2H, m), 2.61 (3H, s), 1.99-1.96 (2H, m), 1.67-1.55 (2H, m), 1.55 (6H, s). | 450 | 448 | |
| 263 | 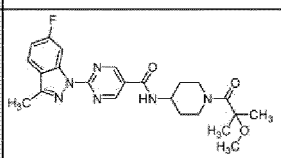 | 1H-NMR (DMSO-D6) δ: 9.24 (2H, s), 8.64 (1H, d, J = 7.5 Hz), 8.41 (1H, d, J = 9.2 Hz), 7.97-7.93 (1H, m), 7.32-7.27 (1H, m), 4.72-4.11 (3H, m), 3.17 (3H, s), 3.16-3.14 (1H, m), 2.74-2.72 (1H, m), 2.61 (3H, s), 1.94-1.92 (2H, m), 1.62-1.36 (2H, m), 1.35 (6H, s). | 455 | 453 | |
| 264 | 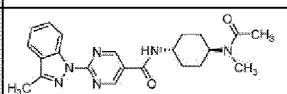 | 1H-NMR (DMSO-D6) δ: 9.23 (2H, s), 8.68 (1H, d, J = 8.1 Hz), 8.57 (1H, d, J = 7.5 Hz), 7.89 (1H, d, J = 8.1 Hz), 7.63 (1H, dd, J = 8.1, 7.5 Hz), 7.40 (1H, dd, J = 8.1, 7.5 Hz), 4.34-4.22 (0.6H, m), 3.90-3.76 (1H, m), 3.67-3.56 (0.4H, m), 2.83 (1.8H, s), 2.70 (1.2H, s), 2.62 (3H, s), 2.06 (1.2H, s), 2.03-1.92 (2H, m), 1.98 (1.8H, s), 1.82-1.38 (6H, m). | 407 | 405 | |

[Table 1-34]

| Ex.No. | structural formula | 1H-NMR (400 MHz) | MS (positive) | MS (negative) | Comments |
|---|---|---|---|---|---|
| 265 | 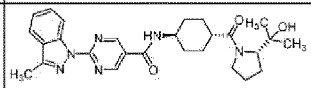 | 1H-NMR (DMSO-D6) δ : 9.23 (2H, s), 8.68 (1H, d, J = 8.1 Hz), 8.59 (1H, d, J = 7.5 Hz), 7.89 (1H, d, J = 8.1 Hz), 7.63 (1H, dd, J = 8.1, 7.6 Hz), 7.40 (1H, dd, J = 8.1, 7.6 Hz), 5.59 (1H, br s), 4.00 (1H, t, J = 6.5 Hz), 3.85-3.72 (2H, m), 3.50-3.20 (2H, m), 2.62 (3H, s), 2.05-1.83 (5H, m), 1.82-1.62 (3H, m), 1.57-1.33 (4H, m), 1.03 (3H, s), 0.98 (3H, s). | 491 | 489 | optically active form (S) |
| 266 | 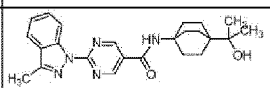 | 1H-NMR (DMSO-D6) δ : 9.16 (2H, s), 8.67 (1H, d, J = 8.6 Hz), 8.00 (1H, s), 7.88 (1H, d, J = 8.1 Hz), 7.62 (1H, dd, J = 8.6, 7.5 Hz), 7.39 (1H, dd, J = 8.1, 7.5 Hz), 3.87 (1H, br s), 2.62 (3H, s), 1.92 (6H, t, J = 7.8 Hz), 1.57 (6H, t, J = 7.8 Hz), 1.01 (6H, s). | 420 | 418 | |
| 267 | 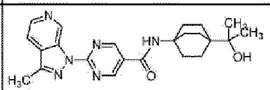 | 1H-NMR (DMSO-D6) δ : 9.98 (1H, s), 9.22 (2H, s), 8.53 (1H, d, J = 5.4 Hz), 8.05 (1H, br s), 7.94 (1H, d, J = 5.4 Hz), 3.87 (1H, br s), 2.66 (3H, s), 1.93 (6H, t, J = 7.8 Hz), 1.57 (6H, t, J = 7.8 Hz), 1.01 (6H, s). | 421 | 419 | |
| 268 | 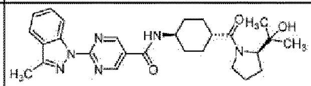 | 1H-NMR (DMSO-D6) δ : 9.23 (2H, s), 8.68 (1H, d, J = 8.1 Hz), 8.59 (1H, d, J = 7.5 Hz), 7.89 (1H, d, J = 8.1 Hz), 7.63 (1H, dd, J = 8.1, 7.6 Hz), 7.40 (1H, dd, J = 8.1, 7.6 Hz), 5.59 (1H, br s), 4.00 (1H, t, J = 6.5 Hz), 3.85-3.72 (2H, m), 3.50-3.20 (2H, m), 2.62 (3H, s), 2.05-1.83 (5H, m), 1.82-1.62 (3H, m), 1.57-1.33 (4H, m), 1.03 (3H, s), 0.98 (3H, s). | 491 | 489 | optically active form (R) |
| 269 | 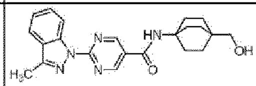 | 1H-NMR (DMSO-D6) δ : 9.16 (2H, s), 8.67 (1H, d, J = 8.6 Hz), 8.00 (1H, br s), 7.88 (1H, d, J = 8.1 Hz), 7.62 (1H, dd, J = 8.6, 7.3 Hz), 7.39 (1H, dd, J = 8.1, 7.3 Hz), 3.49 (1H, br s), 3.05 (2H, s), 2.62 (3H, s), 1.96 (6H, t, J = 8.1 Hz), 1.46 (6H, t, J = 8.1 Hz). | 392 | 390 | |
| 270 | 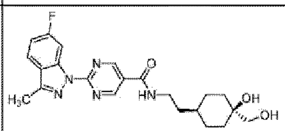 | 1H-NMR (DMSO-D6) δ : 9.23 (2H, s), 8.74 (1H, t, J = 5.4 Hz), 8.41 (1H, dd, J = 10.8, 2.2 Hz), 7.94 (1H, dd, J = 8.6, 5.4 Hz), 7.32-7.27 (1H, m), 4.43 (1H, t, J = 5.7 Hz), 3.78 (1H, s), 3.38-3.33 (2H, m), 3.13 (2H, d, J = 5.4 Hz), 2.61 (3H, s), 1.57-1.21 (11H, m). | 428 | 426 | |
| 271 | 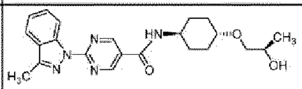 | 1H-NMR (DMSO-D6) δ : 9.22 (2H, s), 8.67 (1H, d, J = 8.1 Hz), 8.53 (1H, d, J = 7.4 Hz), 7.89 (1H, d, J = 8.1 Hz), 7.63 (1H, t, J = 7.9 Hz), 7.40 (1H, t, J = 7.4 Hz), 4.49 (1H, d, J = 4.4 Hz), 3.85-3.75 (1H, m), 3.73-3.64 (1H, m), 3.35-3.29 (1H, m), 3.29-3.19 (2H, m), 2.62 (3H, s), 2.08-2.00 (2H, br m), 1.97-1.89 (2H, br m), 1.46-1.33 (2H, m), 1.33-1.21 (2H, m), 1.04 (3H, d, J = 6.0 Hz). | 410 | 408 | optically active form (R) |
| 272 | 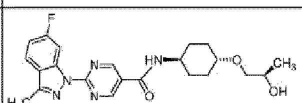 | 1H-NMR (DMSO-D6) δ : 9.23 (2H, s), 8.54 (1H, d, J = 7.6 Hz), 8.40 (1H, dd, J = 10.4, 2.3 Hz), 7.94 (1H, dd, J = 8.7, 5.4 Hz), 7.29 (1H, td, J = 8.9, 2.3 Hz), 4.49 (1H, d, J = 4.6 Hz), 3.85-3.74 (1H, m), 3.73-3.64 (1H, m), 3.35-3.30 (1H, m), 3.29-3.18 (2H, m), 2.61 (3H, s), 2.07-2.00 (2H, br m), 1.96-1.88 (2H, br m), 1.46-1.33 (2H, m), 1.32-1.20 (2H, m), 1.04 (3H, d, J = 6.2 Hz). | 428 | 426 | optically active form (R) |

[Table 1-35]

| Ex.No. | structural formula | 1H-NMR (400 MHz) | MS (positive) | MS (negative) | Comments |
|---|---|---|---|---|---|
| 273 | 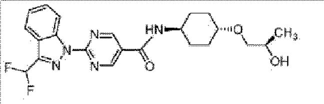 | 1H-NMR (DMSO-D6) δ: 9.30 (2H, s), 8.78 (1H, d, J = 8.6 Hz), 8.63 (1H, d, J = 7.6 Hz), 8.00 (1H, d, J = 8.1 Hz), 7.75 (1H, t, J = 7.9 Hz), 7.56 (1H, t, J = 53.4 Hz), 7.52 (1H, t, J = 7.6 Hz), 4.49 (1H, d, J = 4.6 Hz), 3.86-3.75 (1H, m), 3.74-3.63 (1H, m), 3.35-3.29 (1H, m), 3.28-3.19 (2H, m), 2.08-2.00 (2H, br m), 1.98-1.89 (2H, br m), 1.46-1.35 (2H, m), 1.33-1.22 (2H, m), 1.04 (3H, d, J = 6.2 Hz). | 446 | 444 | optically active form (R) |
| 274 | 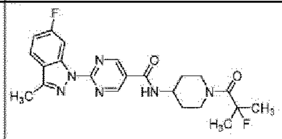 | 1H-NMR (DMSO-D6) δ: 9.23 (2H, s), 8.62 (1H, d, J = 7.9 Hz), 8.39 (1H, dd, J = 10.3, 2.2 Hz), 7.93 (1H, dd, J = 8.8, 5.3 Hz), 7.31-7.25 (1H, m), 4.31-4.28 (2H, m), 4.18-4.08 (1H, m), 3.27-3.13 (1H, m), 2.96-2.76 (1H, m), 2.60 (3H, s), 1.94-1.92 (2H, m), 1.55 (6H, d, J = 22.0 Hz), 1.52-1.41 (2H, m). | 443 | 441 | |
| 275 | 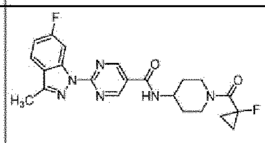 | 1H-NMR (DMSO-D6) δ: 9.25 (2H, s), 8.65 (1H, d, J = 7.6 Hz), 8.41 (1H, dd, J = 10.4, 2.3 Hz), 7.95 (1H, dd, J = 8.8, 5.3 Hz), 7.30 (1H, td, J = 8.8, 2.3 Hz), 4.25-4.15 (3H, m), 3.22-2.98 (2H, m), 2.61 (3H, s), 1.98-1.96 (2H, m), 1.59-1.46 (2H, m), 1.33-1.13 (4H, m). | 441 | 439 | |
| 276 | 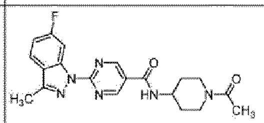 | 1H-NMR (DMSO-D6) δ: 9.24 (2H, s), 8.63 (1H, d, J = 7.6 Hz), 8.41 (1H, dd, J = 10.3, 2.2 Hz), 7.95 (1H, dd, J = 8.8, 5.2 Hz), 7.30 (1H, td, J = 8.8, 2.2 Hz), 4.35-4.32 (1H, m), 4.12-4.03 (1H, m), 3.87-3.83 (1H, m), 3.21-3.15 (1H, m), 2.78-2.72 (1H, m), 2.62 (3H, s), 2.03 (3H, s), 1.94-1.85 (2H, m), 1.54-1.36 (2H, m). | 397 | 395 | |
| 277 | 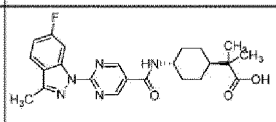 | 1H-NMR (DMSO-D6) δ: 12.09 (1H, br s), 9.23 (2H, s), 8.56 (1H, d, J = 7.6 Hz), 8.40 (1H, dd, J = 10.4, 2.3 Hz), 7.94 (1H, dd, J = 8.9, 5.3 Hz), 7.29 (1H, td, J = 8.9, 2.3 Hz), 3.79-3.70 (1H, m), 2.61 (3H, s), 1.99-1.95 (2H, m), 1.68-1.65 (2H, m), 1.58-1.51 (1H, m), 1.39-1.30 (2H, m), 1.21-1.09 (2H, m), 1.05 (6H, s). | 440 | 438 | |
| 278 | 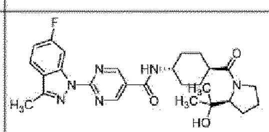 | 1H-NMR (DMSO-D6) δ: 9.24 (2H, s), 8.60 (1H, d, J = 7.4 Hz), 8.41 (1H, dd, J = 10.4, 2.3 Hz), 7.94 (1H, dd, J = 8.7, 5.4 Hz), 7.32-7.27 (1H, m), 5.59 (1H, s), 4.01-3.98 (1H, m), 3.81-3.74 (2H, m), 3.43-3.36 (1H, m), 2.61 (3H, s), 2.57-2.45 (1H, m), 1.99-1.84 (5H, m), 1.66-1.81 (3H, m), 1.56-1.38 (4H, m), 1.13 (0.4H, s), 1.08 (0.4H, s), 1.03 (2.6H, s), 0.98 (2.6H, s). | 509 | 507 | optically active form (S) |
| 279 | 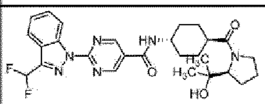 | 1H-NMR (DMSO-D6) δ: 9.31 (2H, s), 8.79 (1H, d, J = 8.6 Hz), 8.68 (1H, d, J = 7.4 Hz), 8.01 (1H, d, J = 7.9 Hz), 7.77-7.73 (1H, m), 7.57 (1H, t, J = 53.2 Hz), 7.54-7.51 (1H, m), 5.59 (1H, s), 4.02-3.98 (1H, m), 3.82-3.75 (2H, m), 3.43-3.36 (1H, m), 2.57-2.46 (1H, m), 2.01-1.84 (5H, m), 1.80-1.66 (3H, m), 1.56-1.36 (4H, m), 1.13 (0.3H, s), 1.08 (0.3H, s), 1.03 (2.7H, s), 0.98 (2.7H, s). | 527 | 525 | optically active form (S) |
| 280 | 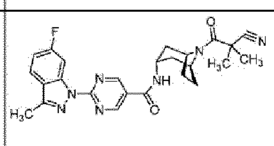 | 1H-NMR (DMSO-D6) δ: 9.19 (2H, s), 8.54 (1H, d, J = 4.0 Hz), 8.44 (1H, d, J = 10.3 Hz), 7.95 (1H, dd, J = 9.0, 5.4 Hz), 7.30 (1H, dd, J = 9.0, 9.0 Hz), 4.77-4.52 (2H, m), 4.11-3.99 (1H, m), 2.62 (3H, s), 2.38-1.77 (8H, m), 1.53 (6H, s). | 476 | 474 | endo form |

[Table 1-36]

| Ex.No. | structural formula | 1H-NMR (400 MHz) | MS (positive) | MS (negative) | Comments |
|---|---|---|---|---|---|
| 281 | 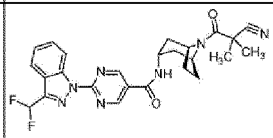 | 1H-NMR (DMSO-D6) δ: 9.27 (2H, s), 8.81 (1H, d, J = 8.5 Hz), 8.62 (1H, d, J = 4.5 Hz), 8.01 (1H, d, J = 8.1 Hz), 7.76 (1H, dd, J = 8.5, 7.9 Hz), 7.57 (1H, t, J = 53.4 Hz), 7.53 (1H, dd, J = 8.1, 7.9 Hz), 4.78-4.53 (2H, m), 4.13-4.02 (1H, m), 2.43-1.79 (8H, m), 1.54 (6H, s). | 494 | 492 | endo form |
| 282 | 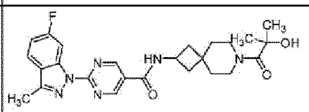 | 1H-NMR (DMSO-D6) δ: 9.25 (2H, s), 8.94 (1H, d, J = 7.2 Hz), 8.41 (1H, dd, J = 10.3, 1.8 Hz), 7.95 (1H, dd, J = 8.5, 5.4 Hz), 7.30 (1H, ddd, J = 8.5, 8.5, 1.8 Hz), 5.33 (1H, s), 4.53-4.41 (1H, m), 4.00-3.36 (4H, m), 2.62 (3H, s), 2.27 (2H, t, J = 10.3 Hz), 1.89 (2H, t, J = 10.3 Hz), 1.67-1.44 (4H, m), 1.31 (6H, s). | 481 | 479 | |
| 283 | 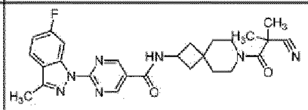 | 1H-NMR (DMSO-D6) δ: 9.25 (2H, s), 8.95 (1H, d, J = 7.2 Hz), 8.41 (1H, dd, J = 10.3, 2.2 Hz), 7.95 (1H, dd, J = 8.8, 5.2 Hz), 7.30 (1H, ddd, J = 8.8, 8.8, 2.2 Hz), 4.53-4.41 (1H, m), 3.75-3.41 (4H, m), 2.62 (3H, s), 2.30 (2H, t, J = 10.1 Hz), 1.92 (2H, t, J = 10.1 Hz), 1.74-1.55 (4H, m), 1.52 (6H, s). | 490 | 488 | |
| 284 | 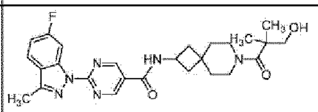 | 1H-NMR (DMSO-D6) δ: 9.25 (2H, s), 8.94 (1H, d, J = 7.6 Hz), 8.41 (1H, dd, J = 10.3, 2.2 Hz), 7.95 (1H, dd, J = 8.5, 5.4 Hz), 7.30 (1H, ddd, J = 8.5, 8.5, 2.2 Hz), 4.53-4.41 (2H, m), 3.55-3.37 (6H, m), 2.62 (3H, s), 2.27 (2H, t, J = 10.3 Hz), 1.89 (2H, t, J = 10.1 Hz), 1.63-1.48 (4H, m), 1.15 (6H, s). | 495 | 493 | |
| 285 | 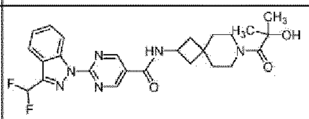 | 1H-NMR (DMSO-D6) δ: 9.32 (2H, s), 9.02 (1H, d, J = 7.2 Hz), 8.79 (1H, d, J = 8.5 Hz), 8.01 (1H, d, J = 8.1 Hz), 7.76 (1H, dd, J = 8.5, 7.6 Hz), 7.57 (1H, t, J = 53.4 Hz), 7.53 (1H, dd, J = 8.1, 7.6 Hz), 5.34 (1H, s), 4.55-4.41 (1H, m), 4.02-3.37 (4H, m), 2.29 (2H, t, J = 10.1 Hz), 1.90 (2H, t, J = 9.9 Hz), 1.69-1.44 (4H, m), 1.31 (6H, s). | 499 | 497 | |
| 286 | 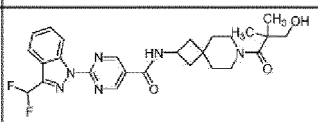 | 1H-NMR (DMSO-D6) δ: 9.32 (2H, s), 9.02 (1H, d, J = 7.2 Hz), 8.79 (1H, d, J = 8.5 Hz), 8.01 (1H, d, J = 8.1 Hz), 7.76 (1H, dd, J = 8.5, 7.9 Hz), 7.57 (1H, t, J = 53.4 Hz), 7.53 (1H, dd, J = 8.1, 7.9 Hz), 4.54-4.40 (2H, m), 3.58-3.37 (6H, m), 2.28 (2H, t, J = 10.1 Hz), 1.90 (2H, t, J = 10.3 Hz), 1.59 (2H, t, J = 5.2 Hz), 1.53 (2H, t, J = 5.2 Hz), 1.15 (6H, s). | 513 | 511 | |
| 287 | 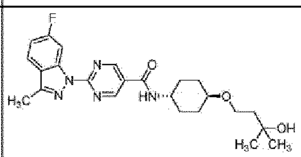 | 1H-NMR (DMSO-D6) δ: 9.23 (2H, s), 8.55 (1H, d, J = 7.6 Hz), 8.40 (1H, dd, J = 10.5, 2.5 Hz), 7.94 (1H, dd, J = 8.5, 5.4 Hz), 7.29 (1H, td, J = 8.9, 2.4 Hz), 4.17 (1H, s), 3.84-3.76 (1H, m), 3.53 (2H, t, J = 7.2 Hz), 3.27-3.20 (1H, m), 2.61 (3H, s), 2.07-1.99 (2H, m), 1.96-1.88 (2H, m), 1.61 (2H, t, J = 7.2 Hz), 1.47-1.34 (2H, m), 1.32-1.19 (2H, m), 1.10 (6H, s). | 456 | 454 | |
| 288 | 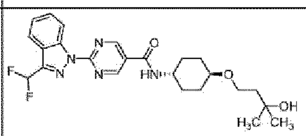 | 1H-NMR (DMSO-D6) δ: 9.30 (2H, s), 8.78 (1H, d, J = 8.5 Hz), 8.63 (1H, d, J = 7.6 Hz), 8.00 (1H, d, J = 8.1 Hz), 7.75 (1H, t, J = 7.9 Hz), 7.56 (1H, t, J = 53.2 Hz), 7.52 (1H, t, J = 7.4 Hz), 4.17 (1H, s), 3.85-3.76 (1H, m), 3.53 (2H, t, J = 7.2 Hz), 3.28-3.20 (1H, m), 2.07-1.99 (2H, m), 1.96-1.90 (2H, m), 1.61 (2H, t, J = 7.2 Hz), 1.45-1.36 (2H, m), 1.32-1.21 (2H, m), 1.10 (6H, s). | 474 | 472 | |

[Table 1-37]

| Ex.No. | structural formula | 1H-NMR (400 MHz) | MS (positive) | MS (negative) | Comments |
|---|---|---|---|---|---|
| 289 | 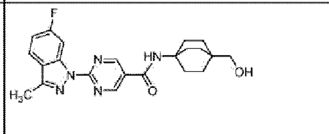 | 1H-NMR (DMSO-D6) δ: 9.17 (2H, s), 8.39 (1H, dd, J = 10.5, 2.0 Hz), 8.01 (1H, br s), 7.94 (1H, dd, J = 8.9, 5.6 Hz), 7.29 (1H, ddd, J = 8.9, 8.9, 2.0 Hz), 4.36 (1H, br s), 3.05 (2H, s), 2.61 (3H, s), 1.95 (6H, t, J = 7.9 Hz), 1.46 (6H, t, J = 7.9 Hz). | 410 | 408 | |
| 290 | 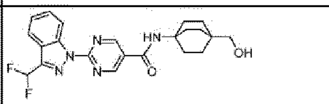 | 1H-NMR (DMSO-D6) δ: 9.25 (2H, s), 8.78 (1H, d, J = 9.0 Hz), 8.10 (1H, br s), 8.00 (1H, d, J = 8.1 Hz), 7.75 (1H, dd, J = 9.0, 7.6 Hz), 7.56 (1H, t, J = 53.4 Hz), 7.52 (1H, dd, J = 8.1, 7.6 Hz), 4.37 (1H, br s), 3.05 (2H, s), 1.96 (6H, t, J = 7.9 Hz), 1.46 (6H, t, J = 7.9 Hz). | 428 | 426 | |
| 291 | 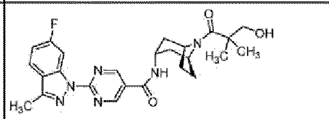 | 1H-NMR (DMSO-D6) δ: 9.18 (2H, s), 8.49 (1H, d, J = 4.5 Hz), 8.43 (1H, dd, J = 10.3, 1.8 Hz), 7.95 (1H, dd, J = 8.8, 5.2 Hz), 7.30 (1H, ddd, J = 8.8, 8.8, 1.8 Hz), 4.64-4.51 (2H, m), 4.55 (1H, t, J = 5.8 Hz), 4.05-3.95 (1H, m), 3.43 (2H, d, J = 5.8 Hz), 2.61 (3H, s), 2.22-2.01 (4H, m), 1.96-1.80 (4H, m), 1.15 (6H, s). | 481 | 479 | endo form |
| 292 | 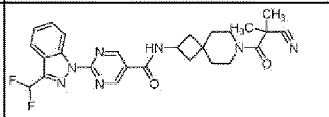 | 1H-NMR (DMSO-D6) δ: 9.32 (2H, s), 9.03 (1H, d, J = 7.2 Hz), 8.79 (1H, d, J = 8.5 Hz), 8.01 (1H, d, J = 7.6 Hz), 7.76 (1H, dd, J = 8.5, 7.6 Hz), 7.57 (1H, t, J = 53.4 Hz), 7.53 (1H, dd, J = 7.6, 7.6 Hz), 4.54-4.43 (1H, m), 3.77-3.40 (4H, m), 2.32 (2H, t, J = 10.5 Hz), 1.93 (2H, t, J = 10.5 Hz), 1.75-1.57 (4H, m), 1.53 (6H, s). | 508 | 506 | |
| 293 | 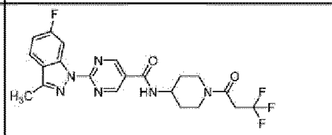 | 1H-NMR (DMSO-D6) δ: 9.25 (2H, s), 8.65 (1H, d, J = 7.6 Hz), 8.41 (1H, dd, J = 9.9, 2.1 Hz), 7.95 (1H, dd, J = 8.5, 5.4 Hz), 7.30 (1H, td, J = 8.5, 2.1 Hz), 4.35-4.31 (1H, m), 4.17-4.03 (1H, m), 3.89-3.86 (1H, m), 3.77-3.61 (2H, m), 3.23-3.17 (1H, m), 2.86-2.81 (1H, m), 2.61 (3H, s), 1.93-1.89 (2H, m), 1.58-1.38 (2H, m). | 465 | 463 | |
| 294 | 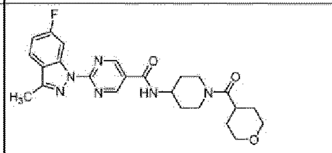 | 1H-NMR (DMSO-D6) δ: 9.24 (2H, d, J = 0.9 Hz), 8.63 (1H, d, J = 7.6 Hz), 8.41 (1H, dd, J = 10.3, 2.1 Hz), 7.95 (1H, dd, J = 8.5, 5.4 Hz), 7.30 (1H, td, J = 8.5, 2.1 Hz), 4.38-4.35 (1H, m), 4.15-4.00 (1H, m), 3.86-3.84 (2H, m), 3.44-3.37 (2H, m), 3.23-3.16 (1H, m), 2.97-2.89 (1H, m), 2.78-2.72 (1H, m), 2.61 (3H, s), 1.96-1.86 (3H, m), 1.66-1.38 (6H, m). | 467 | 465 | |
| 295 | 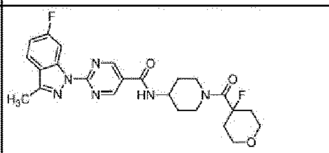 | 1H-NMR (DMSO-D6) δ: 9.24 (2H, s), 8.64 (1H, d, J = 6.7 Hz), 8.42-8.40 (1H, m), 7.97-7.93 (1H, m), 7.32-7.28 (1H, m), 4.33-4.16 (3H, m), 3.79-3.63 (4H, m), 3.29-3.22 (1H, m), 2.98-2.82 (1H, m), 2.61 (3H, s), 2.14-1.97 (6H, m), 1.61-1.42 (2H, m). | 485 | 483 | |
| 296 | 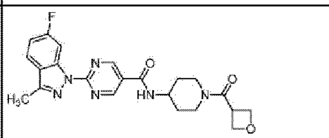 | 1H-NMR (DMSO-D6) δ: 9.24 (2H, s), 8.63 (1H, d, J = 7.6 Hz), 8.41 (1H, dd, J = 10.5, 2.1 Hz), 7.95 (1H, dd, J = 8.8, 5.6 Hz), 7.30 (1H, td, J = 8.8, 2.1 Hz), 4.75-4.64 (4H, m), 4.36-4.33 (1H, m), 4.20-4.04 (2H, m), 3.45-3.42 (1H, m), 3.10-3.04 (1H, m), 2.86-2.80 (1H, m), 2.61 (3H, s), 1.94-1.86 (2H, m), 1.46-1.38 (2H, m). | 439 | 437 | |

[Table 1-38]

| Ex.No. | structural formula | 1H-NMR (400 MHz) | MS (positive) | MS (negative) | Comments |
|---|---|---|---|---|---|
| 297 | 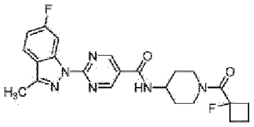 | 1H-NMR (DMSO-D6) δ: 9.24 (2H, s), 8.63 (1H, d, J = 7.6 Hz), 8.40 (1H, dd, J = 10.1, 2.0 Hz), 7.95 (1H, dd, J = 8.5, 5.4 Hz), 7.29 (1H, td, J = 8.5, 2.0 Hz), 4.34-4.30 (1H, m), 4.13 (1H, d, J = 6.7 Hz), 3.83-3.80 (1H, m), 3.24-3.18 (1H, m), 2.94-2.88 (1H, m), 2.70-2.62 (2H, m), 2.61 (3H, s), 2.44-2.32 (2H, m), 1.95-1.84 (3H, m), 1.56-1.44 (3H, m). | 455 | 453 | |
| 298 | 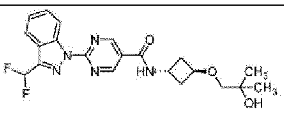 | 1H-NMR (DMSO-D6) δ: 9.32 (2H, s), 9.06 (1H, d, J = 6.7 Hz), 8.79 (1H, d, J = 9.0 Hz), 8.01 (1H, d, J = 7.6 Hz), 7.76 (1H, t, J = 7.9 Hz), 7.57 (1H, t, J = 53.4 Hz), 7.53 (1H, t, J = 7.4 Hz), 4.54-4.44 (1H, m), 4.33 (1H, br s), 4.20-4.14 (1H, m), 3.08 (2H, s), 2.34-2.30 (4H, m), 1.10 (6H, s). | 432 | 430 | |
| 299 | 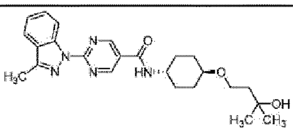 | 1H-NMR (DMSO-D6) δ: 9.22 (2H, s), 8.67 (1H, d, J = 8.5 Hz), 8.54 (1H, d, J = 7.6 Hz), 7.89 (1H, d, J = 8.1 Hz), 7.63 (1H, t, J = 7.9 Hz), 7.40 (1H, t, J = 7.4 Hz), 4.17 (1H, s), 3.85-3.74 (1H, m), 3.53 (2H, t, J = 7.2 Hz), 3.28-3.18 (1H, m), 2.62 (3H, s), 2.07-1.99 (2H, m), 1.97-1.88 (2H, m), 1.61 (2H, t, J = 7.2 Hz), 1.47-1.34 (2H, m), 1.32-1.19 (2H, m), 1.10 (6H, s). | 438 | 436 | |
| 300 | 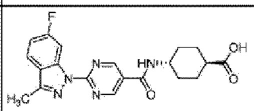 | 1H-NMR (DMSO-D6) δ: 9.23 (2H, s), 8.58 (1H, d, J = 7.6 Hz), 8.40 (1H, dd, J = 10.4, 2.2 Hz), 7.94 (1H, dd, J = 8.8, 5.3 Hz), 7.29 (1H, td, J = 8.8, 2.2 Hz), 3.81-3.73 (1H, m), 3.34 (1H, br s), 2.61 (3H, s), 2.12-2.06 (1H, m), 1.97-1.93 (4H, m), 1.46-1.32 (4H, m). | 398 | 396 | |
| 301 | 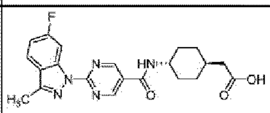 | 1H-NMR (DMSO-D6) δ: 12.03 (1H, br s), 9.23 (2H, s), 8.56 (1H, d, J = 7.9 Hz), 8.40 (1H, dd, J = 10.4, 2.3 Hz), 7.94 (1H, dd, J = 8.8, 5.3 Hz), 7.29 (1H, td, J = 8.9, 2.3 Hz), 3.82-3.73 (1H, m), 2.61 (3H, s), 2.14 (2H, d, J = 7.4 Hz), 1.94-1.90 (2H, m), 1.80-1.77 (2H, m), 1.71-1.61 (1H, m), 1.42-1.32 (2H, m), 1.15-1.06 (2H, m). | 412 | 410 | |
| 302 | 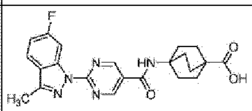 | 1H-NMR (DMSO-D6) δ: 12.06 (1H, br s), 9.17 (2H, s), 8.39 (1H, dd, J = 10.4, 2.3 Hz), 8.08 (1H, s), 7.94 (1H, dd, J = 8.8, 5.4 Hz), 7.29 (1H, td, J = 8.8, 2.3 Hz), 2.61 (3H, s), 2.02-1.98 (6H, m), 1.84-1.80 (6H, m). | 424 | 422 | |
| 303 | 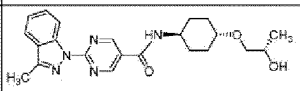 | 1H-NMR (DMSO-D6) δ: 9.22 (2H, s), 8.67 (1H, d, J = 8.1 Hz), 8.53 (1H, d, J = 7.4 Hz), 7.89 (1H, d, J = 8.1 Hz), 7.63 (1H, t, J = 7.9 Hz), 7.40 (1H, t, J = 7.4 Hz), 4.49 (1H, d, J = 4.4 Hz), 3.85-3.75 (1H, m), 3.73-3.64 (1H, m), 3.35-3.29 (1H, m), 3.29-3.19 (2H, m), 2.62 (3H, s), 2.08-2.00 (2H, br m), 1.97-1.89 (2H, br m), 1.46-1.33 (2H, m), 1.33-1.21 (2H, m), 1.04 (3H, d, J = 6.0 Hz). | 410 | 408 | optically active form (S) |
| 304 | 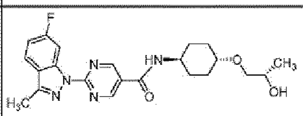 | 1H-NMR (DMSO-D6) δ: 9.23 (2H, s), 8.54 (1H, d, J = 7.6 Hz), 8.40 (1H, dd, J = 10.4, 2.3 Hz), 7.94 (1H, dd, J = 8.7, 5.4 Hz), 7.29 (1H, td, J = 8.9, 2.3 Hz), 4.49 (1H, d, J = 4.6 Hz), 3.85-3.74 (1H, m), 3.73-3.64 (1H, m), 3.35-3.30 (1H, m), 3.29-3.18 (2H, m), 2.61 (3H, s), 2.07-2.00 (2H, br m), 1.96-1.88 (2H, br m), 1.46-1.33 (2H, m), 1.32-1.20 (2H, m), 1.04 (3H, d, J = 6.2 Hz). | 428 | 426 | optically active form (S) |

[Table 1-39]

| Ex.No. | structural formula | 1H-NMR (400 MHz) | MS (positive) | MS (negative) | Comments |
|---|---|---|---|---|---|
| 305 | 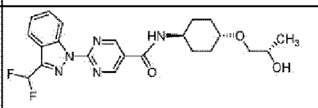 | 1H-NMR (DMSO-D6) δ: 9.30 (2H, s), 8.78 (1H, d, J = 8.6 Hz), 8.63 (1H, d, J = 7.6 Hz), 8.00 (1H, d, J = 8.1 Hz), 7.75 (1H, t, J = 7.9 Hz), 7.56 (1H, t, J = 53.4 Hz), 7.52 (1H, t, J = 7.6 Hz), 4.49 (1H, d, J = 4.6 Hz), 3.86-3.75 (1H, m), 3.74-3.63 (1H, m), 3.35-3.29 (1H, m), 3.28-3.19 (2H, m), 2.08-2.00 (2H, br m), 1.98-1.89 (2H, br m), 1.46-1.35 (2H, m), 1.33-1.22 (2H, m), 1.04 (3H, d, J = 6.2 Hz). | 446 | 444 | optically active form (S) |
| 306 | 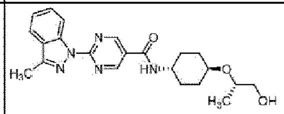 | 1H-NMR (DMSO-D6) δ: 9.20 (2H, s), 8.66 (1H, d, J = 8.3 Hz), 8.51 (1H, d, J = 7.4 Hz), 7.87 (1H, d, J = 7.9 Hz), 7.63-7.60 (1H, m), 7.39-7.37 (1H, m), 4.48 (1H, t, J = 5.8 Hz), 3.80-3.73 (1H, m), 3.52-3.48 (1H, m), 3.39-3.31 (2H, m), 3.23-3.17 (1H, m), 2.61 (3H, s), 1.97-1.89 (4H, m), 1.43-1.20 (4H, m), 1.02 (3H, d, J = 6.2 Hz). | 410 | 408 | optically active form (S) |
| 307 | 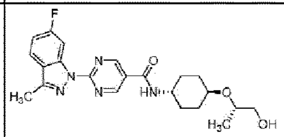 | 1H NMR (DMSO-D6) δ: 9.21 (2H, s), 8.52 (1H, d, J = 7.6 Hz), 8.39 (1H, dd, J = 10.4, 2.2 Hz), 7.93 (1H, dd, J = 8.8, 5.3 Hz), 7.28 (1H, td, J = 8.8, 2.2 Hz), 4.48 (1H, t, J = 5.8 Hz), 3.80-3.71 (1H, m), 3.53-3.46 (1H, m), 3.40-3.30 (2H, m), 3.23-3.17 (1H, m), 2.60 (3H, s), 2.01-1.89 (4H, m), 1.42-1.19 (4H, m), 1.02 (3H, d, J = 6.0 Hz). | 428 | 426 | optically active form (S) |
| 308 | 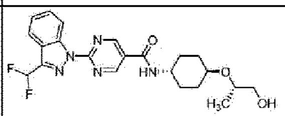 | 1H-NMR (DMSO-D6) δ: 9.28 (2H, s), 8.77 (1H, d, J = 8.6 Hz), 8.60 (1H, d, J = 7.6 Hz), 7.99 (1H, d, J = 8.1 Hz), 7.76-7.72 (1H, m), 7.55 (1H, t, J = 52.0 Hz), 7.52-7.50 (1H, m), 4.48 (1H, t, J = 5.8 Hz), 3.81-3.74 (1H, m), 3.53-3.48 (1H, m), 3.37-3.33 (2H, m), 3.23-3.17 (1H, m), 2.01-1.89 (4H, m), 1.43-1.19 (4H, m), 1.02 (3H, d, J = 6.0 Hz). | 446 | 444 | optically active form (S) |
| 309 | 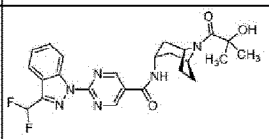 | 1H NMR (DMSO-D6) δ: 9.25 (2H, s), 8.81 (1H, d, J = 8.6 Hz), 8.55 (1H, d, J = 4.6 Hz), 8.01 (1H, d, J = 8.1 Hz), 7.76 (1H, dd, J = 8.6, 7.9 Hz), 7.57 (1H, t, J = 53.4 Hz), 7.53 (1H, dd, J = 8.1, 7.9 Hz), 5.27 (1H, s), 5.10-4.97 (1H, m), 4.61-4.49 (1H, m), 4.10-3.99 (1H, m), 2.35-1.72 (8H, m), 1.40-1.25 (6H, m). | 485 | 483 | endo form |
| 310 | 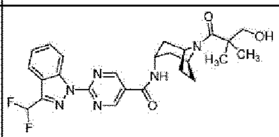 | 1H-NMR (DMSO-D6) δ: 9.26 (2H, s), 8.81 (1H, d, J = 8.6 Hz), 8.57 (1H, d, J = 4.6 Hz), 8.01 (1H, d, J = 7.9 Hz), 7.76 (1H, ddd, J = 8.6, 7.9, 2.0 Hz), 7.57 (1H, t, J = 53.4 Hz), 7.53 (1H, dd, J = 7.9, 7.9 Hz), 4.63-4.51 (2H, m), 4.55 (1H, t, J = 5.8 Hz), 4.07-3.96 (1H, m), 3.43 (2H, d, J = 5.8 Hz), 2.23-2.02 (4H, m), 1.95-1.79 (4H, m), 1.15 (6H, s). | 499 | 497 | endo form |
| 311 | 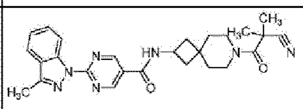 | 1H-NMR (DMSO-D6) δ: 9.24 (2H, s), 8.94 (1H, d, J = 7.2 Hz), 8.68 (1H, d, J = 8.6 Hz), 7.89 (1H, d, J = 7.9 Hz), 7.63 (1H, dd, J = 8.6, 7.7 Hz), 7.40 (1H, dd, J = 7.9, 7.7 Hz), 4.53-4.39 (1H, m), 3.76-3.36 (4H, m), 2.62 (3H, s), 2.30 (2H, t, J = 10.2 Hz), 1.92 (2H, t, J = 10.2 Hz), 1.75-1.56 (4H, m), 1.52 (6H, s). | 472 | 470 | |
| 312 | 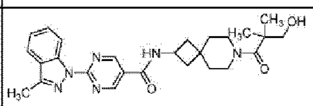 | 1H-NMR (DMSO-D6) δ: 9.23 (2H, s), 8.93 (1H, d, J = 7.4 Hz), 8.68 (1H, d, J = 8.3 Hz), 7.89 (1H, d, J = 7.9 Hz), 7.63 (1H, ddd, J = 8.3, 7.9, 2.1 Hz), 7.40 (1H, dd, J = 7.9, 7.9 Hz), 4.52-4.40 (1H, m), 4.48 (1H, t, J = 5.9 Hz), 3.55-3.47 (2H, m), 3.46-3.38 (2H, m), 3.40 (2H, d, J = 5.9 Hz), 2.62 (3H, s), 2.27 (2H, t, J = 10.3 Hz), 1.89 (2H, t, J = 10.3 Hz), 1.59 (2H, t, J = 5.3 Hz), 1.53 (2H, t, J = 5.3 Hz), 1.15 (6H, s). | 477 | 475 | |

[Table 1-40]

| Ex.No. | structural formula | 1H-NMR (400 MHz) | MS (positive) | MS (negative) | Comments |
|---|---|---|---|---|---|
| 313 | 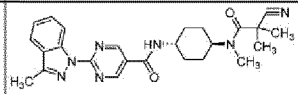 | 1H-NMR (DMSO-D6) δ: 9.23 (2H, s), 8.68 (1H, d, J = 8.5 Hz), 8.58 (1H, d, J = 8.1 Hz), 7.89 (1H, d, J = 8.1 Hz), 7.64 (1H, dd, J = 8.5, 7.6 Hz), 7.40 (1H, dd, J = 8.1, 7.6 Hz), 4.23 (0.5H, s), 4.07-3.78 (1.5H, m), 3.10 (1H, s), 2.78 (2H, s), 2.62 (3H, s), 2.11-1.58 (6H, m), 1.58-1.42 (2H, m), 1.54 (6H, s). | 460 | 458 | |
| 314 | 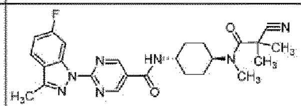 | 1H-NMR (DMSO-D6) δ: 9.25 (2H, s), 8.60 (1H, d, J = 8.1 Hz), 8.41 (1H, dd, J = 10.3, 2.2 Hz), 7.95 (1H, dd, J = 8.5, 5.4 Hz), 7.30 (1H, ddd, J = 8.5, 8.5, 2.2 Hz), 4.23 (0.5H, s), 4.07-3.78 (1.5H, m), 3.10 (1H, s), 2.78 (2H, s), 2.61 (3H, s), 2.10-1.66 (6H, m), 1.65-1.41 (2H, m), 1.54 (6H, s). | 478 | 476 | |
| 315 | 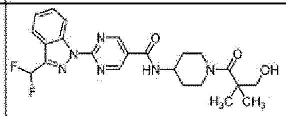 | 1H-NMR (DMSO-D6) δ: 9.32 (2H, d, J = 0.9 Hz), 8.78 (1H, d, J = 8.2 Hz), 8.72 (1H, d, J = 7.2 Hz), 8.01 (1H, d, J = 8.2 Hz), 7.76 (1H, t, J = 8.2 Hz), 7.57 (1H, t, J = 53.0 Hz), 7.53 (1H, t, J = 8.2 Hz), 4.53 (1H, t, J = 5.8 Hz), 4.30-4.27 (2H, m), 4.15-4.06 (1H, m), 3.44 (2H, d, J = 5.8 Hz), 3.00-2.94 (2H, m), 1.93-1.91 (2H, m), 1.49-1.45 (2H, m), 1.18 (6H, s). | 473 | 471 | |
| 316 | 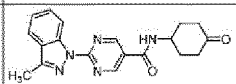 | 1H-NMR (DMSO-D6) δ: 9.25 (2H, s), 8.69-8.67 (2H, m), 7.89 (1H, d, J = 7.9 Hz), 7.66-7.61 (1H, m), 7.40 (1H, t, J = 7.5 Hz), 4.38-4.30 (1H, m), 2.62 (3H, s), 2.58-2.50 (2H, m), 2.37-2.31 (2H, m), 2.18-2.13 (2H, m), 1.89-1.79 (2H, m). | 350 | 348 | |
| 317 | 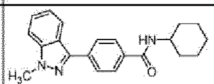 | 1H-NMR (DMSO-D6) δ: 8.27 (1H, d, J = 8.1 Hz), 8.12 (1H, dt, J = 8.3, 0.9 Hz), 8.07-8.04 (2H, m), 8.00-7.97 (2H, m), 7.73-7.71 (1H, m), 7.50-7.46 (1H, m), 7.30-7.26 (1H, m), 4.13 (3H, s), 3.83-3.76 (1H, m), 1.86-1.74 (4H, m), 1.64-1.61 (1H, m), 1.40-1.27 (4H, m), 1.18-1.14 (1H, m). | 334 | Not Detected | |
| 318 | 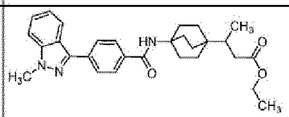 | 1H-NMR (DMSO-D6) δ: 8.13-8.08 (1H, m), 8.05-8.00 (2H, m), 7.94-7.88 (2H, m), 7.74-7.66 (2H, m), 7.51-7.45 (1H, m), 7.30-7.24 (1H, m), 4.13 (3H, s), 4.10-4.02 (2H, m), 2.00-1.90 (6H, m), 2.50-2.40 (1H, m), 1.90-1.80 (1H, m), 1.68-1.57 (1H, m), 1.53-1.38 (6H, m), 1.22-1.15 (3H, m), 0.81-0.75 (3H, m). | 474 | 472 | optically active form (R) |
| 319 | 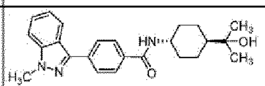 | 1H-NMR (DMSO-D6) δ: 8.29-8.23 (1H, m), 8.14-8.10 (1H, m), 8.08-8.03 (2H, m), 8.01-7.95 (2H, m), 7.75-7.70 (1H, m), 7.52-7.45 (1H, m), 7.31-7.24 (1H, m), 4.13 (3H, s), 4.03 (1H, s), 3.79-3.66 (1H, m), 1.96-1.80 (4H, m), 1.40-1.26 (2H, m), 1.25-1.07 (3H, m), 1.05 (6H, s). | 392 | Not Detected | |
| 320 | 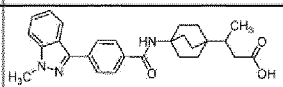 | 1H-NMR (DMSO-D6) δ: 11.98 (1H, br s), 8.13-8.08 (1H, m), 8.05-8.00 (2H, m), 7.95-7.88 (2H, m), 7.75-7.67 (2H, m), 7.51-7.45 (1H, m), 7.30-7.24 (1H, m), 4.13 (3H, s), 2.43-2.36 (1H, m), 2.00-1.89 (6H, m), 1.80-1.72 (1H, m), 1.65-1.55 (1H, m), 1.54-1.37 (6H, m), 0.82-0.77 (3H, m). | 446 | 444 | optically active form (R) |

[Table 1-41]

| Ex.No. | structural formula | 1H-NMR (400 MHz) | MS (positive) | MS (negative) | Comments |
|---|---|---|---|---|---|
| 321 | 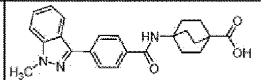 | 1H-NMR (DMSO-D6) δ: 12.04 (1H, br s), 8.13-8.08 (1H, m), 8.05-8.00 (2H, m), 7.93-7.89 (2H, m), 7.76-7.70 (2H, m), 7.51-7.45 (1H, m), 7.30-7.24 (1H, m), 4.13 (3H, s), 2.04-1.96 (6H, m), 1.85-1.77 (6H, m). | 404 | 402 | |
| 322 | 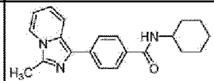 | 1H-NMR (DMSO-D6) δ: 8.15 (2H, d, J = 7.2 Hz), 7.98-7.89 (5H, m), 6.93 (1H, ddd, J = 9.2, 6.5, 0.9 Hz), 6.78-6.74 (1H, m), 3.83-3.74 (1H, m), 2.65 (3H, t, J = 16.0 Hz), 1.91-1.74 (4H, m), 1.64-1.60 (1H, m), 1.40-1.26 (4H, m), 1.19-1.09 (1H, m). | 334 | Not Detected | |
| 323 | 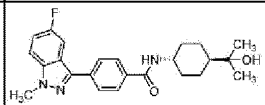 | 1H-NMR (DMSO-D6) δ: 8.28-8.22 (1H, m), 7.99-7.89 (4H, m), 7.59-7.54 (1H, m), 7.51-7.43 (1H, m), 7.05-6.98 (1H, m), 4.15 (3H, s), 4.03 (1H, s), 3.79-3.66 (1H, m), 1.96-1.79 (4H, m), 1.40-1.25 (2H, m), 1.24-1.07 (3H, m), 1.05 (6H, s). | 410 | Not Detected | |
| 324 | 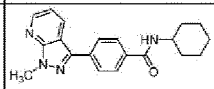 | 1H-NMR (DMSO-D6) δ: 8.67-8.63 (2H, m), 8.32-8.27 (1H, m), 8.12-8.08 (2H, m), 8.02-7.97 (2H, m), 7.38-7.33 (1H, m), 4.15 (3H, s), 3.85-3.73 (1H, m), 1.90-1.81 (2H, m), 1.81-1.70 (2H, m), 1.68-1.57 (1H, m), 1.38-1.24 (4H, m), 1.21-1.09 (1H, m). | 335 | Not Detected | |
| 325 | 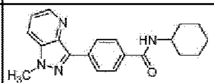 | 1H-NMR (DMSO-D6) δ: 8.69-8.66 (1H, m), 8.58-8.53 (2H, m), 8.26-8.21 (2H, m), 8.00-7.95 (2H, m), 7.53-7.47 (1H, m), 4.17 (3H, s), 3.85-3.73 (1H, m), 1.91-1.80 (2H, m), 1.79-1.70 (2H, m), 1.68-1.58 (1H, m), 1.40-1.27 (4H, m), 1.22-1.08 (1H, m). | 335 | Not Detected | |
| 326 | 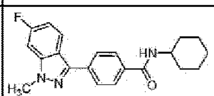 | 1H-NMR (DMSO-D6) δ: 8.31-8.25 (1H, m), 8.17-8.11 (1H, m), 8.06-8.01 (2H, m), 8.00-7.95 (2H, m), 7.65-7.60 (1H, m), 7.18-7.11 (1H, m), 4.09 (3H, s), 3.84-3.74 (1H, m), 1.89-1.80 (2H, m), 1.79-1.69 (2H, m), 1.67-1.58 (1H, m), 1.40-1.28 (4H, m), 1.21-1.08 (1H, m). | 352 | Not Detected | |
| 327 | 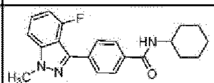 | 1H-NMR (DMSO-D6) δ: 8.30-8.25 (1H, m), 8.06-8.02 (2H, m), 8.00-7.95 (2H, m), 7.92-7.88 (1H, m), 7.81-7.76 (1H, m), 7.43-7.36 (1H, m), 4.14 (3H, s), 3.84-3.74 (1H, m), 1.88-1.80 (2H, m), 1.78-1.70 (2H, m), 1.66-1.58 (1H, m), 1.39-1.28 (4H, m), 1.20-1.09 (1H, m). | 352 | Not Detected | |
| 328 | 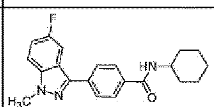 | 1H-NMR (DMSO-D6) δ: 8.29-8.23 (1H, m), 7.98-7.89 (4H, m), 7.59-7.54 (1H, m), 7.50-7.42 (1H, m), 7.05-6.98 (1H, m), 4.14 (3H, s), 3.85-3.72 (1H, m), 1.89-1.81 (2H, m), 1.78-1.70 (2H, m), 1.67-1.57 (1H, m), 1.39-1.27 (4H, m), 1.21-1.08 (1H, m). | 352 | 350 | |

[Table 1-42]

| Ex.No. | structural formula | 1H-NMR (400 MHz) | MS (positive) | MS (negative) | Comments |
|---|---|---|---|---|---|
| 329 | 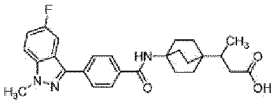 | 1H-NMR (DMSO-D6) δ: 11.98 (1H, br s), 7.91-7.87 (4H, m), 7.70-7.67 (1H, m), 7.58-7.54 (1H, m), 7.50-7.42 (1H, m), 7.04-6.97 (1H, m), 4.14 (3H, s), 2.43-2.31 (1H, m), 1.98-1.89 (6H, m), 1.81-1.70 (1H, m), 1.66-1.54 (1H, m), 1.53-1.37 (6H, m), 0.81-0.76 (3H, m). | 464 | 462 | optically active form (R) |
| 330 | 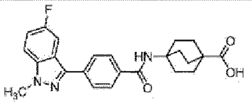 | 1H-NMR (DMSO-D6) δ: 12.02 (1H, br s), 7.90-7.88 (4H, m), 7.76-7.74 (1H, m), 7.58-7.54 (1H, m), 7.50-7.43 (1H, m), 7.04-6.97 (1H, m), 4.14 (3H, s), 2.03-1.94 (6H, m), 1.84-1.76 (6H, m). | 422 | 420 | |
| 331 | 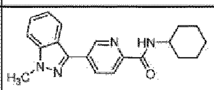 | 1H-NMR (DMSO-D6) δ: 9.24-9.19 (1H, m), 8.58-8.53 (1H, m), 8.51-8.45 (1H, m), 8.19-8.12 (2H, m), 7.80-7.74 (1H, m), 7.55-7.48 (1H, m), 7.34-7.27 (1H, m), 4.17 (3H, s), 3.88-3.76 (1H, m), 1.88-1.79 (2H, m), 1.79-1.68 (2H, m), 1.66-1.56 (1H, m), 1.50-1.27 (4H, m), 1.25-1.11 (1H, m). | 335 | Not Detected | |
| 332 | 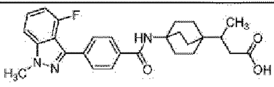 | 1H-NMR (DMSO-D6) δ: 11.96 (1H, br s), 8.03-7.98 (2H, m), 7.92-7.85 (3H, m), 7.81-7.76 (1H, m), 7.70-7.67 (1H, m), 7.43-7.36 (1H, m), 4.14 (3H, s), 2.43-2.31 (1H, m), 1.99-1.90 (6H, m), 1.80-1.72 (1H, m), 1.64-1.55 (1H, m), 1.52-1.37 (6H, m), 0.81-0.77 (3H, m). | 464 | 462 | optically active form (R) |
| 333 | 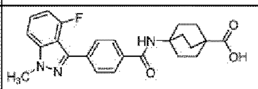 | 1H-NMR (DMSO-D6) δ: 12.09-11.95 (1H, m), 8.03-7.99 (2H, m), 7.93-7.85 (3H, m), 7.81-7.73 (2H, m), 7.43-7.36 (1H, m), 4.14 (3H, s), 2.03-1.95 (6H, m), 1.84-1.76 (6H, m). | 422 | 420 | |
| 334 | 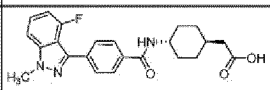 | 1H-NMR (DMSO-D6) δ: 12.01 (1H, br s), 8.31-8.26 (1H, m), 8.06-8.02 (2H, m), 7.99-7.95 (2H, m), 7.92-7.88 (1H, m), 7.81-7.77 (1H, m), 7.43-7.36 (1H, m), 4.14 (3H, s), 3.82-3.68 (1H, m), 2.15-2.11 (2H, m), 1.91-1.82 (2H, m), 1.82-1.73 (2H, m), 1.69-1.57 (1H, m), 1.46-1.31 (2H, m), 1.16-1.00 (2H, m). | 410 | 408 | |
| 335 | 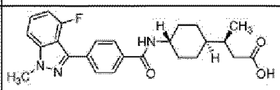 | 1H-NMR (DMSO-D6) δ: 11.98 (1H, br s), 8.30-8.24 (1H, m), 8.06-8.01 (2H, m), 8.00-7.95 (2H, m), 7.93-7.88 (1H, m), 7.81-7.76 (1H, m), 7.43-7.36 (1H, m), 4.14 (3H, s), 3.80-3.68 (1H, m), 2.35-2.27 (1H, m), 2.04-1.95 (1H, m), 1.94-1.86 (2H, m), 1.84-1.74 (1H, m), 1.73-1.65 (2H, m), 1.42-1.27 (2H, m), 1.25-1.06 (3H, m), 0.89-0.85 (3H, m). | 438 | 436 | optically active form (S) |
| 336 | 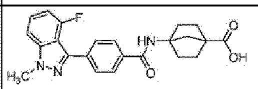 | 1H-NMR (DMSO-D6) δ: 12.12 (1H, br s), 8.54-8.51 (1H, m), 8.06-8.01 (2H, m), 7.99-7.94 (2H, m), 7.93-7.87 (1H, m), 7.82-7.76 (1H, m), 7.43-7.36 (1H, m), 4.14 (3H, s), 2.02-1.96 (6H, m), 1.96-1.85 (2H, m), 1.69-1.60 (2H, m). | 408 | 406 | |

[Table 1-43]

| Ex.No. | structural formula | 1H-NMR (400 MHz) | MS (positive) | MS (negative) | Comments |
|---|---|---|---|---|---|
| 337 | 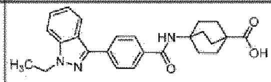 | 1H-NMR (DMSO-D6) δ: 12.04 (1H, br s), 8.12-8.08 (1H, m), 8.05-8.00 (2H, m), 7.94-7.89 (2H, m), 7.78-7.73 (2H, m), 7.49-7.43 (1H, m), 7.29-7.23 (1H, m), 4.57-4.49 (2H, m), 2.04-1.95 (6H, m), 1.85-1.77 (6H, m), 1.49-1.42 (3H, m). | 418 | 416 | |
| 338 | 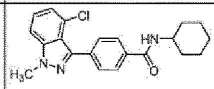 | 1H-NMR (DMSO-D6) δ: 8.31-8.26 (1H, m), 7.96-7.90 (2H, m), 7.75-7.68 (3H, m), 7.48-7.41 (1H, m), 7.28-7.23 (1H, m), 4.14 (3H, s), 3.85-3.72 (1H, m), 1.90-1.81 (2H, m), 1.79-1.70 (2H, m), 1.67-1.57 (1H, m), 1.40-1.26 (4H, m), 1.22-1.09 (1H, m). | 368 | Not Detected | |
| 339 | 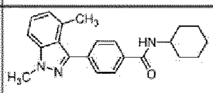 | 1H-NMR (DMSO-D6) δ: 8.32-8.25 (1H, m), 7.96-7.91 (2H, m), 7.67-7.61 (2H, m), 7.53-7.48 (1H, m), 7.36-7.30 (1H, m), 6.95-6.92 (1H, m), 4.08 (3H, s), 3.86-3.74 (1H, m), 2.31 (3H, s), 1.89-1.81 (2H, m), 1.79-1.70 (2H, m), 1.66-1.58 (1H, m), 1.38-1.27 (4H, m), 1.20-1.09 (1H, m). | 348 | Not Detected | |
| 340 | 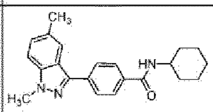 | 1H-NMR (DMSO-D6) δ: 8.28-8.22 (1H, m), 8.06-8.01 (2H, m), 8.00-7.94 (2H, m), 7.90-7.87 (1H, m), 7.63-7.59 (1H, m), 7.33-7.28 (1H, m), 4.10 (3H, s), 3.85-3.73 (1H, m), 2.47 (3H, s), 1.90-1.79 (2H, m), 1.79-1.70 (2H, m), 1.68-1.58 (1H, m), 1.39-1.26 (4H, m), 1.22-1.08 (1H, m). | 348 | Not Detected | |
| 341 | 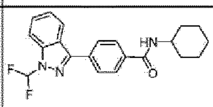 | 1H-NMR (DMSO-D6) δ: 8.41-8.37 (1H, m), 8.21-7.90 (3H, m), 7.79-7.75 (1H, m), 7.74-7.68 (2H, m), 7.63-7.58 (1H, m), 7.48-7.42 (1H, m), 7.25-7.19 (1H, m), 3.87-3.75 (1H, m), 1.90-1.81 (2H, m), 1.80-1.71 (2H, m), 1.67-1.59 (1H, m), 1.41-1.25 (4H, m), 1.22-1.08 (1H, m). | 370 | Not Detected | |
| 342 | 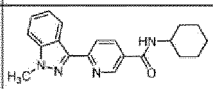 | 1H-NMR (DMSO-D6) δ: 9.14-9.10 (1H, m), 8.61-8.56 (1H, m), 8.46-8.39 (1H, m), 8.30-8.25 (1H, m), 8.21-8.16 (1H, m), 7.76-7.71 (1H, m), 7.51-7.46 (1H, m), 7.33-7.27 (1H, m), 4.16 (3H, s), 3.85-3.74 (1H, m), 1.91-1.82 (2H, m), 1.81-1.70 (2H, m), 1.67-1.58 (1H, m), 1.39-1.26 (4H, m), 1.22-1.08 (1H, m). | 335 | Not Detected | |
| 343 | 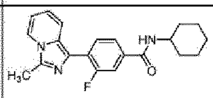 | 1H-NMR (DMSO-D6) δ: 8.28 (1H, d, J = 7.9 Hz), 8.17-8.15 (1H, m), 7.92-7.88 (1H, m), 7.79-7.75 (2H, m), 7.62-7.58 (1H, m), 6.92 (1H, ddd, J = 9.2, 6.5, 0.9 Hz), 6.79-6.76 (1H, m), 3.83-3.74 (1H, m), 2.67 (3H, s), 1.86-1.73 (4H, m), 1.64-1.60 (1H, m), 1.38-1.23 (4H, m), 1.19-1.09 (1H, m). | 352 | Not Detected | |
| 344 | 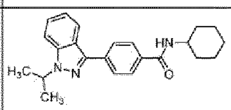 | 1H-NMR (DMSO-D6) δ: 8.26 (1H, d, J = 7.9 Hz), 8.11-8.09 (1H, m), 8.06-8.03 (2H, m), 8.00-7.97 (2H, m), 7.80-7.77 (1H, m), 7.47-7.43 (1H, m), 7.28-7.24 (1H, m), 5.13-5.03 (1H, m), 3.85-3.75 (1H, m), 1.88-1.72 (4H, m), 1.65-1.60 (1H, m), 1.56 (6H, d, J = 6.7 Hz), 1.40-1.27 (4H, m), 1.20-1.10 (1H, m). | 362 | Not Detected | |

[Table 1-44]

| Ex.No. | structural formula | 1H-NMR (400 MHz) | MS (positive) | MS (negative) | Comments |
|---|---|---|---|---|---|
| 345 | 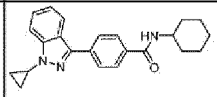 | 1H-NMR (DMSO-D6) δ: 8.27 (1H, d, J = 8.1 Hz), 8.12-8.09 (1H, m), 8.05-8.02 (2H, m), 7.99-7.96 (2H, m), 7.79-7.77 (1H, m), 7.52-7.48 (1H, m), 7.32-7.28 (1H, m), 3.87-3.76 (1H, m), 3.86-3.75 (1H, m), 1.87-1.72 (4H, m), 1.65-1.60 (1H, m), 1.40-1.27 (4H, m), 1.20-1.18 (4H, m), 1.20-1.13 (1H, m). | 360 | Not Detected | |
| 346 | 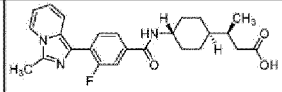 | 1H-NMR (DMSO-D6) δ: 11.99 (1H, s), 8.27 (1H, d, J = 7.9 Hz), 8.17-8.15 (1H, m), 7.92-7.88 (1H, m), 7.78-7.74 (2H, m), 7.62-7.58 (1H, m), 6.92 (1H, ddd, J = 9.4, 6.4, 0.9 Hz), 6.79-6.76 (1H, m), 3.78-3.68 (1H, m), 2.67 (3H, s), 2.31 (1H, dd, J = 15.0, 5.2 Hz), 1.99 (1H, dd, J = 15.0, 9.0 Hz), 1.91-1.88 (2H, m), 1.82-1.75 (1H, m), 1.70-1.67 (2H, m), 1.39-1.28 (2H, m), 1.23-1.05 (3H, m), 0.87 (3H, d, J = 7.2 Hz). | 438 | Not Detected | opti-cally active form (S) |
| 347 | 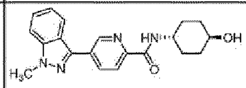 | 1H-NMR (DMSO-D6) δ: 9.21 (1H, dd, J = 2.3, 0.7 Hz), 8.54 (1H, dd, J = 8.2, 2.3 Hz), 8.46 (1H, d, J = 8.6 Hz), 8.17-8.13 (2H, m), 7.77 (1H, d, J = 8.6 Hz), 7.54-7.49 (1H, m), 7.33-7.29 (1H, m), 4.56 (1H, d, J = 4.4 Hz), 4.17 (3H, s), 3.83-3.73 (1H, m), 3.45-3.38 (1H, m), 1.88-1.80 (4H, m), 1.55-1.45 (2H, m), 1.32-1.22 (2H, m). | 351 | Not Detected | |
| 348 | 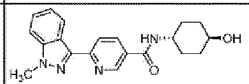 | 1H-NMR (DMSO-D6) δ: 9.11 (1H, d, J = 2.2 Hz), 8.58 (1H, d, J = 8.2 Hz), 8.40 (1H, d, J = 7.5 Hz), 8.26 (1H, dd, J = 8.2, 2.2 Hz), 8.18 (1H, d, J = 8.2 Hz), 7.73 (1H, d, J = 9.0 Hz), 7.48 (1H, t, J = 7.1 Hz), 7.29 (1H, t, J = 7.5 Hz), 4.56 (1H, s), 4.16 (3H, s), 3.80-3.72 (1H, m), 3.42-3.40 (1H, m), 1.88-1.85 (4H, m), 1.39-1.26 (4H, m). | 351 | 349 | |
| 349 | 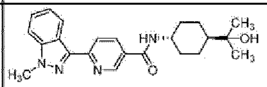 | 1H-NMR (DMSO-D6) δ: 9.12 (1H, d, J = 2.2 Hz), 8.58 (1H, d, J = 8.2 Hz), 8.42 (1H, d, J = 7.5 Hz), 8.28 (1H, dd, J = 8.2, 2.2 Hz), 8.19 (1H, d, J = 8.2 Hz), 7.73 (1H, d, J = 8.2 Hz), 7.48 (1H, t, J = 8.2 Hz), 7.30 (1H, t, J = 8.2 Hz), 4.17 (3H, s), 4.04 (1H, s), 3.78-3.70 (1H, m), 1.99-1.84 (4H, m), 1.37-1.09 (5H, m), 1.03 (6H, d, J = 15.0 Hz). | 393 | 391 | |
| 350 | 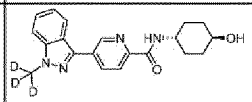 | 1H-NMR (DMSO-D6) δ: 9.21 (1H, d, J = 2.2 Hz), 8.55 (1H, dd, J = 8.2, 2.2 Hz), 8.46 (1H, d, J = 8.2 Hz), 8.15 (2H, dd, J = 8.2, 5.2 Hz), 7.77 (1H, d, J = 8.2 Hz), 7.51 (1H, t, J = 7.5 Hz), 7.31 (1H, t, J = 7.5 Hz), 4.56 (1H, s), 3.79-3.75 (1H, m), 3.43-3.40 (1H, m), 1.84 (4H, dd, J = 13.8, 10.8 Hz), 1.38 (4H, dq, J = 88.6, 11.3 Hz). | 354 | Not Detected | |
| 351 | 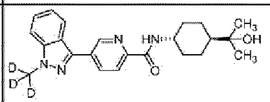 | 1H-NMR (DMSO-D6) δ: 9.21 (1H, d, J = 2.2 Hz), 8.55 (1H, dd, J = 8.2, 2.2 Hz), 8.47 (1H, d, J = 9.0 Hz), 8.19-8.13 (2H, m), 7.77 (1H, d, J = 9.0 Hz), 7.52 (1H, t, J = 7.5 Hz), 7.31 (1H, t, J = 7.5 Hz), 4.04 (1H, s), 3.79-3.71 (1H, m), 1.90-1.82 (4H, m), 1.47-1.10 (5H, m), 1.05 (6H, s). | 396 | Not Detected | |
| 352 | 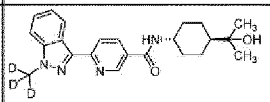 | 1H-NMR (DMSO-D6) δ: 9.12 (1H, d, J = 2.2 Hz), 8.58 (1H, d, J = 8.2 Hz), 8.42 (1H, d, J = 7.5 Hz), 8.27 (1H, dd, J = 8.2, 2.2 Hz), 8.19 (1H, d, J = 8.2 Hz), 7.73 (1H, d, J = 9.0 Hz), 7.48 (1H, t, J = 7.1 Hz), 7.29 (1H, t, J = 7.5 Hz), 4.04 (1H, s), 3.76-3.70 (1H, m), 1.94-1.85 (4H, m), 1.36-1.29 (2H, m), 1.20-1.12 (3H, m), 1.05 (6H, s). | 396 | 394 | |

[Table 1-45]

| Ex.No. | structural formula | 1H-NMR (400 MHz) | MS (positive) | MS (negative) | Comments |
|---|---|---|---|---|---|
| 353 | 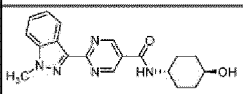 | 1H-NMR (DMSO-D6) δ: 9.26 (2H, s), 8.57-8.53 (2H, m), 7.78 (1H, d, J = 8.6 Hz), 7.53-7.49 (1H, m), 7.36-7.32 (1H, m), 4.58 (1H, d, J = 4.4 Hz), 4.21 (3H, s), 3.81-3.72 (1H, m), 3.47-3.39 (1H, m), 1.90-1.87 (4H, m), 1.45-1.35 (2H, m), 1.31-1.23 (2H, m). | 352 | 350 | |
| 354 | 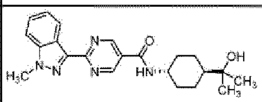 | 1H-NMR (DMSO-D6) δ: 9.27 (2H, s), 8.59-8.54 (2H, m), 7.78 (1H, d, J = 8.6 Hz), 7.53-7.49 (1H, m), 7.37-7.33 (1H, m), 4.21 (3H, s), 4.05 (1H, s), 3.79-3.71 (1H, m), 1.99-1.95 (2H, m), 1.88-1.84 (2H, m), 1.38-1.28 (2H, m), 1.24-1.08 (3H, m), 1.06 (6H, s). | 394 | 392 | |
| 355 | 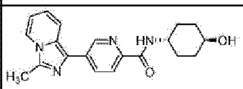 | 1H-NMR (DMSO-D6) δ: 9.13 (1H, d, J = 1.6 Hz), 8.40 (1H, dd, J = 8.2, 2.2 Hz), 8.33 (1H, d, J = 8.6 Hz), 8.21 (1H, d, J = 7.2 Hz), 8.05-8.00 (2H, m), 7.03-6.99 (1H, m), 6.83-6.80 (1H, m), 4.56 (1H, d, J = 4.2 Hz), 3.80-3.70 (1H, m), 3.46-3.38 (1H, m), 2.67 (3H, s), 1.88-1.80 (4H, m), 1.53-1.43 (2H, m), 1.32-1.22 (2H, m). | 351 | Not Detected | |
| 356 | 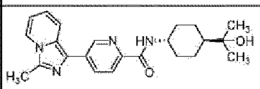 | 1H-NMR (DMSO-D6) δ: 9.13 (1H, d, J = 1.8 Hz), 8.40 (1H, dd, J = 8.3, 2.3 Hz), 8.34 (1H, d, J = 8.6 Hz), 8.21 (1H, d, J = 7.2 Hz), 8.05 (1H, d, J = 8.3 Hz), 8.03-8.01 (1H, m), 7.03-6.99 (1H, m), 6.83-6.80 (1H, m), 4.04 (1H, s), 3.77-3.68 (1H, m), 2.67 (3H, s), 1.91-1.82 (4H, m), 1.45-1.36 (2H, m), 1.24-1.03 (3H, m), 1.05 (6H, s). | 393 | Not Detected | |
| 357 | 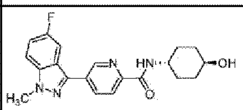 | 1H-NMR (DMSO-D6) δ: 9.18 (1H, d, J = 0.9 Hz), 8.55-8.52 (1H, m), 8.44 (1H, d, J = 8.8 Hz), 8.14 (1H, d, J = 8.1 Hz), 7.96-7.93 (1H, m), 7.83 (1H, dd, J = 9.1, 4.3 Hz), 7.45-7.40 (1H, m), 4.57 (1H, d, J = 4.4 Hz), 4.17 (3H, s), 3.81-3.73 (1H, m), 3.45-3.38 (1H, m), 1.88-1.80 (4H, m), 1.55-1.44 (2H, m), 1.32-1.22 (2H, m). | 369 | Not Detected | |
| 358 | 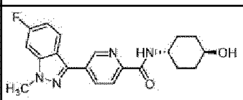 | 1H-NMR (DMSO-D6) δ: 9.18 (1H, d, J = 2.1 Hz), 8.53 (1H, dd, J = 8.0, 2.1 Hz), 8.46 (1H, d, J = 8.6 Hz), 8.18-8.14 (2H, m), 7.67 (1H, dd, J = 9.7, 2.1 Hz), 7.20-7.15 (1H, m), 4.57 (1H, d, J = 4.4 Hz), 4.12 (3H, s), 3.81-3.73 (1H, m), 3.45-3.39 (1H, m), 1.88-1.80 (4H, m), 1.55-1.45 (2H, m), 1.32-1.22 (2H, m). | 369 | Not Detected | |
| 359 | 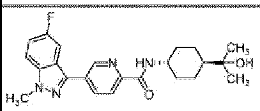 | 1H-NMR (DMSO-D6) δ: 9.18 (1H, d, J = 2.1 Hz), 8.53 (1H, dd, J = 8.2, 1.7 Hz), 8.44 (1H, d, J = 8.6 Hz), 8.14 (1H, d, J = 8.2 Hz), 7.97-7.94 (1H, m), 7.83 (1H, dd, J = 9.1, 4.3 Hz), 7.45-7.40 (1H, m), 4.17 (3H, s), 4.04 (1H, s), 3.79-3.71 (1H, m), 1.92-1.83 (4H, m), 1.47-1.37 (2H, m), 1.25-1.01 (3H, m), 1.05 (6H, s). | 411 | Not Detected | |
| 360 | 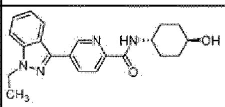 | 1H-NMR (DMSO-D6) δ: 9.21 (1H, d, J = 2.2 Hz), 8.54 (1H, dd, J = 8.2, 2.2 Hz), 8.45 (1H, d, J = 9.0 Hz), 8.15 (2H, t, J = 8.2 Hz), 7.81 (1H, d, J = 8.2 Hz), 7.50 (1H, t, J = 8.2 Hz), 7.30 (1H, t, J = 7.1 Hz), 4.56 (2H, q, J = 7.1 Hz), 4.56 (1H, s), 3.80-3.78 (1H, m), 3.45-3.36 (1H, m), 1.88-1.78 (4H, m), 1.54-1.45 (2H, m), 1.47 (3H, t, J = 7.1 Hz), 1.32-1.23 (2H, m). | 365 | 363 | |

[Table 1-46]

| Ex.No. | structural formula | 1H-NMR (400 MHz) | MS (positive) | MS (negative) | Comments |
|---|---|---|---|---|---|
| 361 | 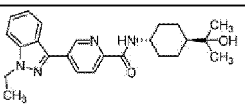 | 1H-NMR (DMSO-D6) δ: 9.21 (1H, d, J = 2.2 Hz), 8.54 (1H, dd, J = 8.2, 2.2 Hz), 8.46 (1H, d, J = 8.2 Hz), 8.16-8.14 (2H, m), 7.81 (1H, d, J = 9.0 Hz), 7.50 (1H, t, J = 7.5 Hz), 7.30 (1H, t, J = 7.5 Hz), 4.57 (2H, q, J = 7.2 Hz), 4.04 (1H, s), 3.77-3.71 (1H, m), 1.91-1.81 (4H, m), 1.47 (3H, t, J = 7.2 Hz), 1.47-1.38 (2H, m), 1.24-1.10 (3H, m), 1.05 (6H, s). | 407 | Not Detected | |
| 362 | 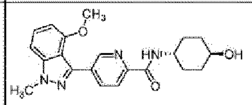 | 1H-NMR (DMSO-D6) δ: 9.07 (1H, d, J = 2.1 Hz), 8.49 (1H, d, J = 8.6 Hz), 8.40 (1H, dd, J = 8.1, 2.1 Hz), 8.11 (1H, d, J = 8.1 Hz), 7.41 (1H, t, J = 8.1 Hz), 7.28 (1H, d, J = 8.6 Hz), 6.71 (1H, d, J = 7.6 Hz), 4.56 (1H, d, J = 4.4 Hz), 4.10 (3H, s), 3.90 (3H, s), 3.81-3.74 (1H, m), 3.44-3.38 (1H, m), 1.88-1.80 (4H, m), 1.56-1.45 (2H, m), 1.32-1.23 (2H, m). | 381 | Not Detected | |
| 363 | 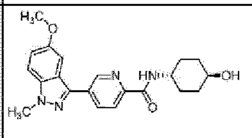 | 1H-NMR (DMSO-D6) δ: 9.20 (1H, d, J = 2.1 Hz), 8.52 (1H, dd, J = 8.2, 2.1 Hz), 8.44 (1H, d, J = 8.6 Hz), 8.15 (1H, d, J = 8.2 Hz), 7.68 (1H, d, J = 9.1 Hz), 7.45 (1H, d, J = 2.3 Hz), 7.16 (1H, dd, J = 9.1, 2.3 Hz), 4.57 (1H, d, J = 4.4 Hz), 4.13 (3H, s), 3.88 (3H, s), 3.81-3.76 (1H, m), 3.45-3.39 (1H, m), 1.88-1.81 (4H, m), 1.54-1.44 (2H, m), 1.32-1.22 (2H, m). | 381 | Not Detected | |
| 364 | 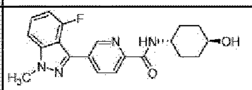 | 1H-NMR (DMSO-D6) δ: 9.08 (1H, s), 8.51 (1H, d, J = 8.6 Hz), 8.40 (1H, d, J = 8.3 Hz), 8.15 (1H, d, J = 8.1 Hz), 7.61 (1H, d, J = 8.3 Hz), 7.53-7.47 (1H, m), 7.06 (1H, dd, J = 11.6, 7.6 Hz), 4.56 (1H, br s), 4.18 (3H, s), 3.81-3.73 (1H, m), 3.45-3.37 (1H, m), 1.88-1.79 (4H, m), 1.56-1.45 (2H, m), 1.32-1.22 (2H, m). | 369 | Not Detected | |
| 365 | 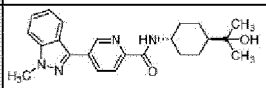 | 1H-NMR (DMSO-D6) δ: 9.21 (1H, s), 8.55 (1H, dd, J = 8.1, 2.1 Hz), 8.46 (1H, d, J = 8.7 Hz), 8.17-8.14 (2H, m), 7.77 (1H, d, J = 8.7 Hz), 7.54-7.50 (1H, m), 7.33-7.29 (1H, m), 4.17 (3H, s), 4.04 (1H, s), 3.79-3.70 (1H, m), 1.92-1.83 (4H, m), 1.47-1.38 (2H, m), 1.24-1.05 (3H, m), 1.05 (6H, s). | 393 | Not Detected | |
| 366 | 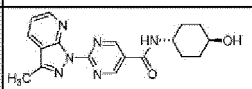 | 1H-NMR (DMSO-D6) δ: 9.24 (2H, s), 8.71 (1H, d, J = 4.4 Hz), 8.55 (1H, d, J = 7.9 Hz), 8.38 (1H, d, J = 7.9 Hz), 7.43 (1H, dd, J = 7.9, 4.4 Hz), 4.57 (1H, d, J = 4.2 Hz), 3.83-3.70 (1H, m), 3.49-3.37 (1H, m), 2.63 (3H, s), 1.97-1.82 (4H, m), 1.47-1.17 (4H, m). | 353 | 351 | |
| 367 | 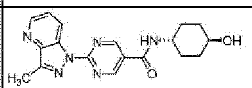 | 1H-NMR (DMSO-D6) δ: 9.24 (2H, s), 8.96 (1H, d, J = 8.6 Hz), 8.71 (1H, d, J = 4.4 Hz), 8.54 (1H, d, J = 7.6 Hz), 7.65 (1H, dd, J = 8.6, 4.4 Hz), 4.57 (1H, d, J = 3.9 Hz), 3.83-3.65 (1H, m), 3.51-3.38 (1H, m), 2.66 (3H, s), 1.96-1.82 (4H, m), 1.47-1.19 (4H, m). | 353 | 351 | |
| 368 | 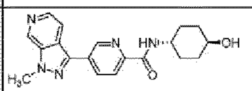 | 1H-NMR (DMSO-D6) δ: 9.29 (1H, d, J = 1.1 Hz), 9.23 (1H, dd, J = 2.2, 0.7 Hz), 8.57 (1H, dd, J = 8.2, 2.2 Hz), 8.46 (1H, d, J = 8.6 Hz), 8.39 (1H, d, J = 5.8 Hz), 8.17 (1H, dd, J = 8.2, 0.7 Hz), 8.13 (1H, dd, J = 5.8, 1.1 Hz), 4.57 (1H, d, J = 4.4 Hz), 4.29 (3H, s), 3.83-3.73 (1H, m), 3.46-3.38 (1H, m), 1.89-1.81 (4H, m), 1.55-1.45 (2H, m), 1.33-1.22 (2H, m). | 352 | Not Detected | |

[Table 1-47]

| Ex.No. | structural formula | 1H-NMR (400 MHz) | MS (positive) | MS (negative) | Comments |
|---|---|---|---|---|---|
| 369 | 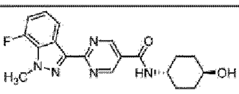 | 1H-NMR (DMSO-D6) δ: 9.27 (2H, s), 8.58 (1H, d, J = 7.5 Hz), 8.36 (1H, d, J = 7.5 Hz), 7.35-7.26 (2H, m), 4.58 (1H, d, J = 4.5 Hz), 4.33 (3H, s), 3.81-3.73 (1H, m), 3.46-3.39 (1H, m), 1.90-1.87 (4H, m), 1.44-1.35 (2H, m), 1.31-1.23 (2H, m). | 370 | 368 | |
| 370 | 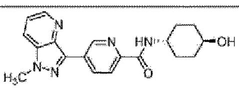 | 1H-NMR (DMSO-D6) δ: 9.63-9.61 (1H, br m), 9.00-8.97 (1H, m), 8.71-8.69 (1H, br m), 8.48-8.46 (1H, m), 8.30-8.27 (1H, m), 8.20-8.17 (1H, m), 7.56-7.52 (1H, m), 4.58-4.55 (1H, br m), 4.20 (3H, s), 3.82-3.73 (1H, m), 3.46-3.38 (1H, m), 1.90-1.78 (4H, m), 1.55-1.45 (2H, m), 1.32-1.23 (2H, m). | 352 | Not Detected | |
| 371 | 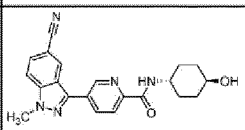 | 1H-NMR (DMSO-D6) δ: 9.22 (1H, s), 8.79 (1H, s), 8.63-8.60 (1H, m), 8.46 (1H, d, J = 8.1 Hz), 8.15 (1H, d, J = 8.3 Hz), 7.97 (1H, d, J = 9.0 Hz), 7.84 (1H, d, J = 8.3 Hz), 4.57 (1H, d, J = 4.2 Hz), 4.21 (3H, s), 3.83-3.73 (1H, m), 3.46-3.39 (1H, m), 1.88-1.81 (4H, m), 1.55-1.45 (2H, m), 1.33-1.23 (2H, m). | 376 | 420 | |
| 372 | 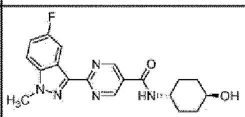 | 1H-NMR (DMSO-D6) δ: 9.26 (2H, s), 8.56 (1H, d, J = 7.4 Hz), 8.19 (1H, dd, J = 9.4, 2.4 Hz), 7.85 (1H, dd, J = 9.2, 4.4 Hz), 7.43 (1H, td, J = 9.1, 2.3 Hz), 4.57 (1H, d, J = 4.4 Hz), 4.22 (3H, s), 3.82-3.72 (1H, m), 3.46-3.39 (1H, m), 1.90-1.87 (4H, m), 1.44-1.35 (2H, m), 1.31-1.22 (2H, m). | 370 | 368 | |
| 373 | 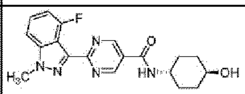 | 1H-NMR (DMSO-D6) δ: 9.25 (2H, s), 8.59 (1H, d, J = 7.9 Hz), 7.61 (1H, d, J = 8.3 Hz), 7.49 (1H, td, J = 8.0, 4.6 Hz), 7.04 (1H, dd, J = 11.0, 7.7 Hz), 4.57 (1H, d, J = 4.4 Hz), 4.20 (3H, s), 3.80-3.72 (1H, m), 3.45-3.39 (1H, m), 1.90-1.87 (4H, m), 1.43-1.35 (2H, m), 1.31-1.22 (2H, m). | 370 | 368 | |
| 374 | 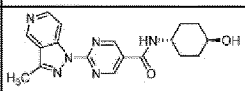 | 1H-NMR (DMSO-D6) δ: 9.25 (2H, s), 9.23 (1H, s), 8.65 (1H, d, J = 5.8 Hz), 8.56 (1H, d, J = 7.4 Hz), 8.50 (1H, d, J = 5.8 Hz), 4.57 (1H, d, J = 3.7 Hz), 3.84-3.70 (1H, m), 3.33-3.21 (1H, m), 2.70 (3H, s), 1.93-1.84 (4H, m), 1.46-1.20 (4H, m). | 353 | 351 | |
| 375 | 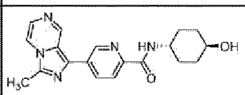 | 1H-NMR (DMSO-D6) δ: 9.45 (1H, d, J = 1.6 Hz), 9.21 (1H, d, J = 2.0 Hz), 8.55 (1H, dd, J = 8.2, 2.0 Hz), 8.41 (1H, d, J = 8.8 Hz), 8.22 (1H, dd, J = 5.0, 1.6 Hz), 8.09 (1H, d, J = 8.2 Hz), 7.67 (1H, d, J = 5.0 Hz), 4.56 (1H, d, J = 4.4 Hz), 3.80-3.72 (1H, m), 3.45-3.38 (1H, m), 2.70 (3H, s), 1.88-1.80 (4H, m), 1.54-1.44 (2H, m), 1.32-1.23 (2H, m). | 352 | Not Detected | |
| 376 | 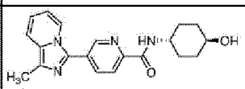 | 1H-NMR (DMSO-D6) δ: 9.08 (1H, d, J = 1.6 Hz), 8.53 (1H, d, J = 7.4 Hz), 8.46 (1H, d, J = 8.6 Hz), 8.43 (1H, dd, J = 8.2, 2.2 Hz), 8.13 (1H, d, J = 8.2 Hz), 7.69-7.66 (1H, m), 6.86-6.82 (1H, m), 6.77-6.74 (1H, m), 4.56 (1H, d, J = 4.4 Hz), 3.82-3.72 (1H, m), 3.46-3.37 (1H, m), 2.51 (3H, s), 1.88-1.79 (4H, m), 1.55-1.45 (2H, m), 1.32-1.22 (2H, m). | 351 | Not Detected | |

[Table 1-48]

| Ex.No. | structural formula | 1H-NMR (400 MHz) | MS (positive) | MS (negative) | Comments |
|---|---|---|---|---|---|
| 377 | 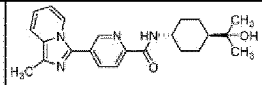 | 1H-NMR (DMSO-D6) δ: 9.08 (1H, d, J = 2.2 Hz), 8.54 (1H, d, J = 7.2 Hz), 8.47 (1H, d, J = 8.2 Hz), 8.43 (1H, dd, J = 8.2, 2.2 Hz), 8.13 (1H, d, J = 8.2 Hz), 7.68 (1H, d, J = 9.0 Hz), 6.84 (1H, dd, J = 9.0, 6.2 Hz), 6.78-6.74 (1H, m), 4.04 (1H, s), 3.79-3.70 (1H, m), 2.51 (3H, s), 1.91-1.82 (4H, m), 1.47-1.38 (2H, m), 1.24-1.05 (3H, m), 1.05 (6H, s). | 393 | Not Detected | |
| 378 | 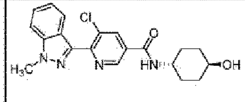 | 1H-NMR (DMSO-D6) δ: 9.08 (1H, d, J = 1.5 Hz), 8.54 (1H, d, J = 8.2 Hz), 8.43 (1H, d, J = 1.5 Hz), 8.17 (1H, d, J = 7.5 Hz), 7.76 (1H, d, J = 7.5 Hz), 7.49 (1H, t, J = 7.5 Hz), 7.27 (1H, t, J = 7.5 Hz), 4.57 (1H, s), 4.18 (3H, s), 3.79-3.71 (1H, m), 3.46-3.38 (1H, m), 1.89-1.86 (4H, m), 1.43-1.22 (4H, m). | 385 | 383 | |
| 379 | 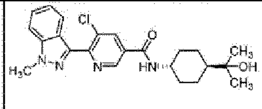 | 1H-NMR (DMSO-D6) δ: 9.09 (1H, d, J = 1.5 Hz), 8.56 (1H, d, J = 8.2 Hz), 8.44 (1H, d, J = 1.5 Hz), 8.17 (1H, d, J = 7.9 Hz), 7.76 (1H, d, J = 7.9 Hz), 7.49 (1H, t, J = 7.9 Hz), 7.28 (1H, t, J = 7.9 Hz), 4.18 (3H, s), 4.04 (1H, s), 3.77-3.68 (1H, m), 1.97-1.84 (4H, m), 1.37-1.09 (5H, m), 1.06 (6H, s). | 427 | 425 | |
| 380 | 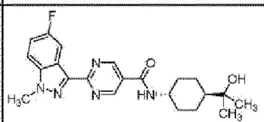 | 1H-NMR (DMSO-D6) δ: 9.27 (2H, s), 8.58 (1H, d, J = 7.9 Hz), 8.19 (1H, dd, J = 9.5, 2.3 Hz), 7.85 (1H, dd, J = 9.1, 4.3 Hz), 7.43 (1H, td, J = 9.0, 2.5 Hz), 4.22 (3H, s), 4.05 (1H, s), 3.79-3.70 (1H, m), 1.99-1.94 (2H, m), 1.88-1.84 (2H, m), 1.37-1.28 (2H, m), 1.24-1.09 (3H, m), 1.06 (6H, s). | 412 | 410 | |
| 381 | 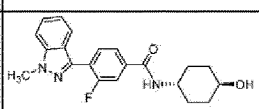 | 1H-NMR (DMSO-D6) δ: 8.35 (1H, d, J = 7.5 Hz), 7.90-7.82 (3H, m), 7.78 (1H, dd, J = 8.2, 3.0 Hz), 7.73 (1H, d, J = 8.2 Hz), 7.48 (1H, t, J = 7.9 Hz), 7.25 (1H, t, J = 7.5 Hz), 4.56 (1H, d, J = 4.5 Hz), 4.15 (3H, s), 3.81-3.69 (1H, m), 3.47-3.38 (1H, m), 1.91-1.80 (4H, br m), 1.46-1.33 (2H, br m), 1.32-1.20 (2H, br m). | 368 | 366 | |
| 382 | 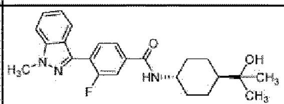 | 1H-NMR (DMSO-D6) δ: 8.37 (1H, d, J = 7.5 Hz), 7.91-7.82 (3H, m), 7.78 (1H, dd, J = 8.2, 3.0 Hz), 7.73 (1H, d, J = 9.0 Hz), 7.48 (1H, t, J = 7.9 Hz), 7.25 (1H, t, J = 7.9 Hz), 4.15 (3H, s), 4.04 (1H, s), 3.79-3.68 (1H, m), 1.96-1.80 (4H, br m), 1.39-1.26 (2H, br m), 1.24-1.02 (9H, m). | 410 | 408 | |
| 383 | 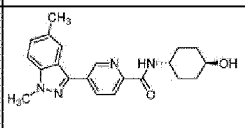 | 1H-NMR (DMSO-D6) δ: 9.19 (1H, dd, J = 2.2, 0.8 Hz), 8.52 (1H, dd, J = 8.2, 2.2 Hz), 8.43 (1H, d, J = 8.3 Hz), 8.15 (1H, dd, J = 8.2, 0.8 Hz), 7.92 (1H, s), 7.65 (1H, d, J = 8.6 Hz), 7.36-7.33 (1H, m), 4.56 (1H, d, J = 4.4 Hz), 4.13 (3H, s), 3.82-3.72 (1H, m), 3.46-3.38 (1H, m), 2.48 (3H, s), 1.88-1.81 (4H, m), 1.55-1.44 (2H, m), 1.32-1.22 (2H, m). | 365 | Not Detected | |
| 384 | 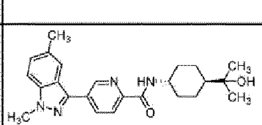 | 1H-NMR (DMSO-D6) δ: 9.20 (1H, dd, J = 2.3, 0.7 Hz), 8.53 (1H, dd, J = 8.2, 2.3 Hz), 8.43 (1H, d, J = 8.6 Hz), 8.15 (1H, dd, J = 8.2, 0.7 Hz), 7.93 (1H, s), 7.65 (1H, d, J = 8.6 Hz), 7.36-7.33 (1H, m), 4.13 (3H, s), 4.04 (1H, s), 3.79-3.71 (1H, m), 2.48 (3H, s), 1.93-1.83 (4H, m), 1.47-1.37 (2H, m), 1.24-1.01 (3H, m), 1.05 (6H, s). | 407 | Not Detected | |

[Table 1-49]

| Ex.No. | structural formula | 1H-NMR (400 MHz) | MS (positive) | MS (negative) | Comments |
|---|---|---|---|---|---|
| 385 | 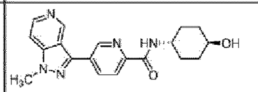 | 1H-NMR (DMSO-D6) δ: 9.49 (1H, d, J = 0.9 Hz), 9.27-9.26 (1H, m), 8.64 (1H, dd, J = 8.2, 2.1 Hz), 8.50-8.48 (2H, m), 8.18 (1H, d, J = 8.2 Hz), 7.79 (1H, dd, J = 6.0, 1.2 Hz), 4.56 (1H, d, J = 4.4 Hz), 4.18 (3H, s), 3.82-3.74 (1H, m), 3.46-3.38 (1H, m), 1.88-1.80 (4H, m), 1.56-1.45 (2H, m), 1.32-1.22 (2H, m). | 352 | Not Detected | |
| 386 | 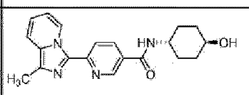 | 1H-NMR (DMSO-D6) δ: 9.85-9.83 (1H, m), 9.05-9.05 (1H, m), 8.38 (1H, d, J = 7.6 Hz), 8.26-8.26 (2H, m), 7.77-7.74 (1H, m), 6.99-6.91 (2H, m), 4.56 (1H, d, J = 4.4 Hz), 3.80-3.71 (1H, m), 3.45-3.37 (1H, m), 2.53 (3H, s), 1.90-1.83 (4H, m), 1.44-1.21 (4H, m). | 351 | 349 | |
| 387 | 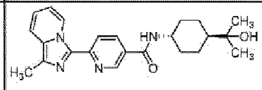 | 1H-NMR (DMSO-D6) δ: 9.85-9.83 (1H, m), 9.06 (1H, t, J = 1.5 Hz), 8.39 (1H, d, J = 8.1 Hz), 8.29-8.24 (2H, m), 7.77-7.74 (1H, m), 6.99-6.91 (2H, m), 4.04 (1H, s), 3.78-3.68 (1H, m), 2.53 (3H, s), 1.95-1.91 (2H, m), 1.87-1.83 (2H, m), 1.37-1.27 (2H, m), 1.23-1.01 (3H, m), 1.05 (6H, s). | 393 | 437 | |
| 388 | 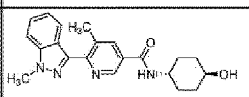 | 1H-NMR (DMSO-D6) δ: 8.98 (1H, s), 8.42 (1H, d, J = 8.2 Hz), 8.37 (1H, d, J = 7.5 Hz), 8.14 (1H, s), 7.72 (1H, d, J = 8.2 Hz), 7.47 (1H, t, J = 8.2 Hz), 7.25 (1H, t, J = 8.2 Hz), 4.56 (1H, d, J = 3.7 Hz), 4.16 (3H, s), 3.80-3.71 (1H, m), 3.46-3.37 (1H, m), 2.73 (3H, s), 1.88-1.86 (4H, m), 1.44-1.22 (4H, m). | 365 | 363 | |
| 389 | 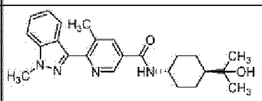 | 1H-NMR (DMSO-D6) δ: 8.99 (1H, d, J = 1.5 Hz), 8.42 (1H, d, J = 8.2 Hz), 8.39 (1H, d, J = 7.5 Hz), 8.15 (1H, d, J = 1.5 Hz), 7.72 (1H, d, J = 8.2 Hz), 7.47 (1H, t, J = 8.2 Hz), 7.26 (1H, t, J = 8.2 Hz), 4.16 (3H, s), 4.04 (1H, s), 3.78-3.70 (1H, m), 2.73 (3H, s), 1.95-1.84 (4H, m), 1.37-1.09 (5H, m), 1.06 (6H, s). | 407 | 405 | |
| 390 | 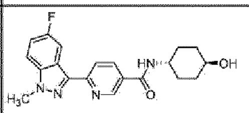 | 1H-NMR (DMSO-D6) δ: 9.11 (1H, dd, J = 2.3, 0.7 Hz), 8.40 (1H, d, J = 7.9 Hz), 8.27 (1H, dd, J = 8.3, 2.3 Hz), 8.23 (1H, dd, J = 9.5, 2.3 Hz), 8.16 (1H, dd, J = 8.3, 0.7 Hz), 7.81 (1H, dd, J = 9.1, 4.3 Hz), 7.43-7.38 (1H, m), 4.56 (1H, brs), 4.18 (3H, s), 3.80-3.71 (1H, m), 3.44-3.38 (1H, m), 1.89-1.84 (4H, m), 1.45-1.34 (2H, m), 1.31-1.21 (2H, m). | 369 | 413 | |
| 391 | 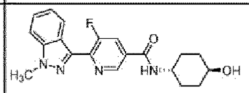 | 1H-NMR (DMSO-D6) δ: 9.01 (1H, s), 8.48 (1H, d, J = 7.5 Hz), 8.44 (1H, d, J = 8.2 Hz), 8.20 (1H, d, J = 11.2 Hz), 7.76 (1H, d, J = 7.5 Hz), 7.50 (1H, t, J = 7.5 Hz), 7.30 (1H, t, J = 7.5 Hz), 4.57 (1H, d, J = 4.5 Hz), 4.18 (3H, s), 3.80-3.72 (1H, m), 3.45-3.37 (1H, m), 1.89-1.86 (4H, m), 1.44-1.22 (4H, m). | 369 | 367 | |
| 392 | 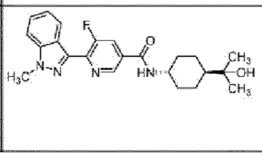 | 1H-NMR (DMSO-D6) δ: 9.02 (1H, s), 8.50 (1H, d, J = 7.5 Hz), 8.44 (1H, d, J = 8.2 Hz), 8.22 (1H, d, J = 12.0 Hz), 7.77 (1H, d, J = 7.5 Hz), 7.50 (1H, t, J = 7.5 Hz), 7.31 (1H, t, J = 7.5 Hz), 4.18 (3H, s), 4.05 (1H, s), 3.78-3.70 (1H, m), 1.96-1.84 (4H, m), 1.37-1.10 (5H, m), 1.06 (6H, s). | 411 | 409 | |

[Table 1-50]

| Ex.No. | structural formula | 1H-NMR (400 MHz) | MS (positive) | MS (negative) | Comments |
|---|---|---|---|---|---|
| 393 | 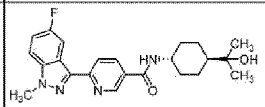 | 1H-NMR (DMSO-D6) δ: 9.12 (1H, dd, J = 2.3, 0.8 Hz), 8.42 (1H, d, J = 7.9 Hz), 8.28 (1H, dd, J = 8.3, 2.3 Hz), 8.24 (1H, dd, J = 9.4, 2.3 Hz), 8.17 (1H, dd, J = 8.3, 0.7 Hz), 7.81 (1H, dd, J = 9.1, 4.3 Hz), 7.43-7.38 (1H, m), 4.18 (3H, s), 4.04 (1H, br s), 3.79-3.69 (1H, m), 1.96-1.92 (2H, m), 1.87-1.83 (2H, m), 1.38-1.27 (2H, m), 1.24-1.02 (3H, m), 1.06 (6H, s). | 411 | 455 | |
| 394 | 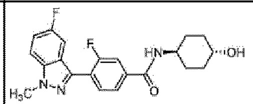 | 1H-NMR (DMSO-D6) δ: 8.36 (1H, d, J = 7.5 Hz), 7.91-7.78 (4H, m), 7.53-7.48 (1H, br m), 7.40 (1H, td, J = 9.0, 2.2 Hz), 4.56 (1H, d, J = 4.5 Hz), 4.16 (3H, s), 3.80-3.69 (1H, m), 3.46-3.36 (1H, m), 1.91-1.80 (4H, br m), 1.46-1.33 (2H, br m), 1.32-1.20 (2H, br m). | 386 | 384 | |
| 395 | 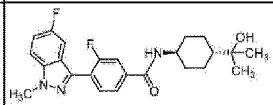 | 1H-NMR (DMSO-D6) δ: 8.37 (1H, d, J = 8.2 Hz), 7.91-7.78 (4H, m), 7.53-7.48 (1H, br m), 7.40 (1H, td, J = 9.7, 2.2 Hz), 4.16 (3H, s), 4.04 (1H, s), 3.79-3.67 (1H, br m), 1.97-1.80 (4H, br m), 1.39-1.26 (2H, br m), 1.23-1.02 (9H, br m). | 428 | 426 | |
| 396 | 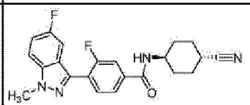 | 1H-NMR (DMSO-D6) δ: 8.43 (1H, d, J = 7.6 Hz), 7.90-7.79 (4H, m), 7.50 (1H, dt, J = 9.3, 2.7 Hz), 7.40 (1H, td, J = 9.1, 2.5 Hz), 4.16 (3H, s), 3.89-3.78 (1H, m), 2.70 (1H, tt, J = 11.6, 3.5 Hz), 2.12-2.04 (2H, br m), 1.93-1.86 (2H, br m), 1.70-1.58 (2H, br m), 1.46-1.35 (2H, br m). | 395 | 393 | |
| 397 | 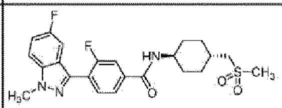 | 1H-NMR (DMSO-D6) δ: 8.42 (1H, d, J = 7.9 Hz), 7.91-7.78 (4H, m), 7.51 (1H, dt, J = 9.6, 2.6 Hz), 7.40 (1H, td, J = 9.1, 2.4 Hz), 4.16 (3H, s), 3.82-3.71 (1H, m), 3.09 (2H, d, J = 6.2 Hz), 2.99 (3H, s), 2.03-1.95 (2H, br m), 1.94-1.84 (3H, br m), 1.47-1.35 (2H, br m), 1.28-1.16 (2H, br m). | 462 | 460 | |
| 398 | 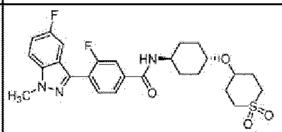 | 1H-NMR (DMSO-D6) δ: 8.39 (1H, d, J = 7.5 Hz), 7.91-7.78 (4H, m), 7.53-7.47 (1H, br m), 7.40 (1H, td, J = 9.0, 1.5 Hz), 4.16 (3H, s), 3.83-3.74 (2H, m), 3.43-3.34 (1H, m), 3.14-3.00 (4H, m), 2.10-1.95 (6H, br m), 1.93-1.85 (2H, br m), 1.48-1.36 (2H, br m), 1.35-1.23 (2H, br m). | 518 | 516 | |
| 399 | 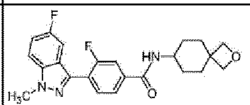 | 1H-NMR (DMSO-D6) δ: 8.33 (1H, d, J = 7.9 Hz), 7.91-7.77 (4H, m), 7.50 (1H, dt, J = 9.5, 2.8 Hz), 7.40 (1H, td, J = 9.1, 2.5 Hz), 4.35 (2H, s), 4.24 (2H, s), 4.16 (3H, s), 3.83-3.71 (1H, m), 2.14-2.05 (2H, br m), 1.82-1.73 (2H, br m), 1.58-1.47 (2H, br m), 1.40-1.28 (2H, br m). | 412 | 410 | |
| 400 | 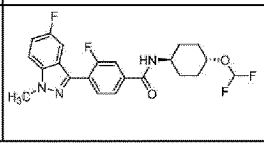 | 1H-NMR (DMSO-D6) δ: 8.41 (1H, d, J = 7.9 Hz), 7.91-7.78 (4H, m), 7.50 (1H, dt, J = 9.5, 2.6 Hz), 7.40 (1H, td, J = 9.1, 2.4 Hz), 6.73 (1H, t, J = 76.6 Hz), 4.16 (3H, s), 4.10-4.02 (1H, m), 3.87-3.77 (1H, m), 2.07-1.99 (2H, br m), 1.96-1.88 (2H, br m), 1.57-1.40 (4H, br m). | 436 | 434 | |

[Table 1-51]

| Ex.No. | structural formula | 1H-NMR (400 MHz) | MS (positive) | MS (negative) | Comments |
|---|---|---|---|---|---|
| 401 | 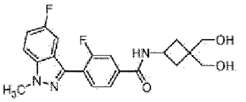 | 1H-NMR (DMSO-D6) δ: 8.72 (1H, d, J = 7.5 Hz), 7.92-7.78 (4H, m), 7.54-7.48 (1H, br m), 7.40 (1H, td, J = 9.0, 1.5 Hz), 4.60 (1H, t, J = 5.2 Hz), 4.48 (1H, t, J = 5.2 Hz), 4.43-4.31 (1H, m), 4.16 (3H, s), 3.45 (2H, d, J = 5.2 Hz), 3.38 (2H, d, J = 5.2 Hz), 2.09 (2H, t, J = 10.5 Hz), 1.90 (2H, t, J = 10.1 Hz). | 402 | 400 | |
| 402 | 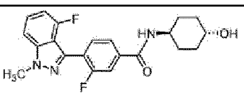 | 1H-NMR (DMSO-D6) δ: 8.36 (1H, d, J = 7.5 Hz), 7.83-7.76 (2H, m), 7.71 (1H, t, J = 7.5 Hz), 7.58 (1H, d, J = 9.0 Hz), 7.51-7.43 (1H, m), 6.97 (1H, dd, J = 10.8, 7.9 Hz), 4.56 (1H, d, J = 4.5 Hz), 4.15 (3H, s), 3.80-3.69 (1H, m), 3.47-3.36 (1H, m), 1.92-1.80 (4H, br m), 1.45-1.33 (2H, br m), 1.32-1.20 (2H, br m). | 386 | Not Detected | |
| 403 | 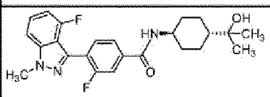 | 1H-NMR (DMSO-D6) δ: 8.38 (1H, d, J = 8.2 Hz), 7.85-7.77 (2H, m), 7.71 (1H, t, J = 7.5 Hz), 7.58 (1H, d, J = 8.2 Hz), 7.51-7.43 (1H, m), 6.97 (1H, dd, J = 11.2, 7.5 Hz), 4.15 (3H, s), 4.04 (1H, s), 3.78-3.66 (1H, m), 1.97-1.80 (4H, br m), 1.39-1.26 (2H, br m), 1.22-1.02 (9H, br m). | 428 | 426 | |
| 404 | 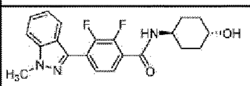 | 1H-NMR (DMSO-D6) δ: 8.37 (1H, d, J = 7.5 Hz), 7.79 (1H, dd, J = 8.2, 2.2 Hz), 7.75 (1H, d, J = 9.0 Hz), 7.66 (1H, t, J = 7.1 Hz), 7.52-7.44 (2H, m), 7.27 (1H, t, J = 7.5 Hz), 4.56 (1H, d, J = 4.5 Hz), 4.16 (3H, s), 3.76-3.66 (1H, m), 3.46-3.37 (1H, m), 1.91-1.82 (4H, br m), 1.38-1.21 (4H, m). | 386 | 384 | |
| 405 | 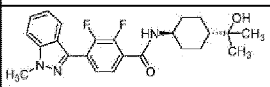 | 1H-NMR (DMSO-D6) δ: 8.37 (1H, d, J = 7.5 Hz), 7.80 (1H, dd, J = 8.2, 3.0 Hz), 7.75 (1H, d, J = 8.2 Hz), 7.66 (1H, t, J = 7.1 Hz), 7.53-7.44 (2H, m), 7.27 (1H, t, J = 7.5 Hz), 4.16 (3H, s), 4.04 (1H, s), 3.74-3.63 (1H, m), 1.99-1.90 (2H, br m), 1.88-1.80 (2H, br m), 1.33-1.21 (2H, br m), 1.20-1.01 (9H, br m). | 428 | 426 | |
| 406 | 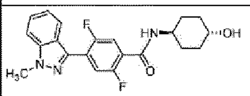 | 1H-NMR (DMSO-D6) δ: 8.29 (1H, d, J = 8.2 Hz), 7.78 (1H, dd, J = 8.2, 3.0 Hz), 7.75 (1H, d, J = 9.0 Hz), 7.64 (1H, dd, J = 10.1, 5.6 Hz), 7.56 (1H, dd, J = 10.5, 6.0 Hz), 7.49 (1H, t, J = 7.9 Hz), 7.26 (1H, t, J = 7.9 Hz), 4.56 (1H, d, J = 4.5 Hz), 4.16 (3H, s), 3.76-3.65 (1H, m), 3.46-3.36 (1H, m), 1.91-1.81 (4H, br m), 1.41-1.19 (4H, m). | 386 | 384 | |
| 407 | 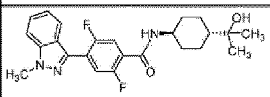 | 1H-NMR (DMSO-D6) δ: 8.30 (1H, d, J = 8.2 Hz), 7.79 (1H, dd, J = 8.2, 3.7 Hz), 7.75 (1H, d, J = 8.2 Hz), 7.65 (1H, dd, J = 10.1, 5.6 Hz), 7.57 (1H, dd, J = 10.5, 6.0 Hz), 7.49 (1H, t, J = 7.9 Hz), 7.26 (1H, t, J = 7.5 Hz), 4.16 (3H, s), 4.04 (1H, s), 3.74-3.62 (1H, m), 1.99-1.90 (2H, br m), 1.88-1.79 (2H, br m), 1.33-1.21 (2H, br m), 1.19-1.03 (9H, br m). | 428 | 426 | |
| 408 | 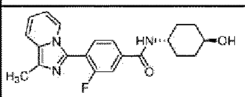 | 1H-NMR (DMSO-D6) δ: 8.38 (1H, d, J = 7.9 Hz), 7.91 (1H, dd, J = 7.2, 4.4 Hz), 7.87-7.85 (2H, m), 7.80-7.76 (1H, m), 7.66-7.63 (1H, m), 6.83-6.79 (1H, m), 6.72-6.69 (1H, m), 4.56 (1H, d, J = 4.4 Hz), 3.80-3.70 (1H, m), 3.45-3.37 (1H, m), 2.50 (3H, s), 1.89-1.82 (4H, m), 1.45-1.35 (2H, m), 1.31-1.20 (2H, m). | 368 | 412 | |

[Table 1-52]

| Ex.No. | structural formula | 1H-NMR (400 MHz) | MS (positive) | MS (negative) | Comments |
|---|---|---|---|---|---|
| 409 | 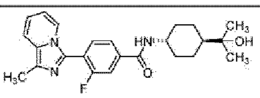 | 1H-NMR (DMSO-D6) δ: 8.40 (1H, d, J = 7.9 Hz), 7.92-7.89 (1H, m), 7.88-7.85 (2H, m), 7.80-7.76 (1H, m), 7.66-7.63 (1H, m), 6.81 (1H, dd, J = 9.2, 6.2 Hz), 6.72-6.69 (1H, m), 4.04 (1H, s), 3.78-3.68 (1H, m), 2.50 (3H, s), 1.94-1.90 (2H, m), 1.87-1.83 (2H, m), 1.38-1.04 (5H, m), 1.05 (6H, s). | 410 | 454 | |
| 410 | 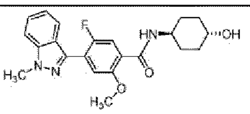 | 1H-NMR (DMSO-D6) δ: 8.04 (1H, d, J = 7.5 Hz), 7.78 (1H, d, J = 5.2 Hz), 7.73 (1H, d, J = 8.2 Hz), 7.63 (1H, d, J = 11.2 Hz), 7.48 (1H, t, J = 7.5 Hz), 7.41 (1H, d, J = 6.0 Hz), 7.24 (1H, t, J = 7.5 Hz), 4.56 (1H, d, J = 3.7 Hz), 4.16 (3H, s), 3.94 (3H, s), 3.79-3.68 (1H, m), 3.48-3.39 (1H, m), 1.93-1.80 (4H, br m), 1.42-1.20 (4H, br m). | 398 | 442 | |
| 411 | 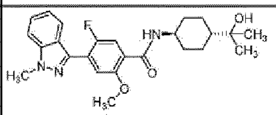 | 1H-NMR (DMSO-D6) δ: 8.03 (1H, d, J = 8.2 Hz), 7.78 (1H, dd, J = 8.2, 3.0 Hz), 7.73 (1H, d, J = 9.0 Hz), 7.63 (1H, d, J = 11.2 Hz), 7.48 (1H, t, J = 7.9 Hz), 7.41 (1H, d, J = 6.0 Hz), 7.24 (1H, t, J = 7.5 Hz), 4.16 (3H, s), 4.04 (1H, s), 3.95 (3H, s), 3.74-3.64 (1H, m), 2.01-1.92 (2H, br m), 1.88-1.79 (2H, br m), 1.33-1.02 (11H, m). | 440 | 484 | |
| 412 | 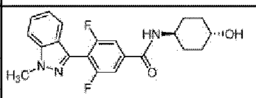 | 1H-NMR (DMSO-D6) δ: 8.43 (1H, d, J = 7.5 Hz), 7.75 (3H, d, J = 8.2 Hz), 7.59 (1H, d, J = 8.2 Hz), 7.49 (1H, t, J = 7.5 Hz), 7.23 (1H, t, J = 7.5 Hz), 4.57 (1H, d, J = 3.7 Hz), 4.16 (3H, s), 3.80-3.69 (1H, m), 3.46-3.36 (1H, m), 1.93-1.81 (4H, br m), 1.46-1.33 (2H, br m), 1.32-1.20 (2H, br m). | 386 | 384 | |
| 413 | 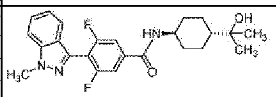 | 1H-NMR (DMSO-D6) δ: 8.45 (1H, d, J = 7.5 Hz), 7.76 (3H, d, J = 9.0 Hz), 7.59 (1H, d, J = 8.2 Hz), 7.49 (1H, t, J = 7.9 Hz), 7.23 (1H, t, J = 7.5 Hz), 4.16 (3H, s), 4.05 (1H, s), 3.78-3.67 (1H, m), 1.98-1.89 (2H, br m), 1.89-1.81 (2H, br m), 1.39-1.26 (2H, br m), 1.24-1.01 (9H, br m). | 428 | 426 | |
| 414 | 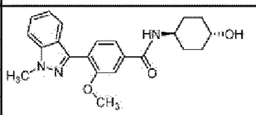 | 1H-NMR (DMSO-D6) δ: 8.25 (1H, d, J = 7.9 Hz), 7.64 (2H, t, J = 8.3 Hz), 7.60-7.52 (3H, m), 7.41 (1H, t, J = 7.7 Hz), 7.15 (1H, t, J = 7.5 Hz), 4.56 (1H, d, J = 4.4 Hz), 4.10 (3H, s), 3.86 (3H, s), 3.82-3.72 (1H, m), 3.47-3.36 (1H, m), 1.92-1.81 (4H, br m), 1.48-1.35 (2H, br m), 1.33-1.21 (2H, br m). | 380 | 378 | |
| 415 | 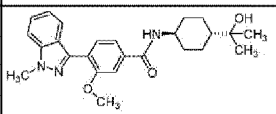 | 1H-NMR (DMSO-D6) δ: 8.27 (1H, d, J = 8.1 Hz), 7.64 (2H, t, J = 7.9 Hz), 7.61-7.53 (3H, m), 7.44-7.39 (1H, m), 7.18-7.12 (1H, m), 4.10 (3H, s), 4.04 (1H, s), 3.86 (3H, s), 3.81-3.70 (1H, m), 1.97-1.89 (2H, br m), 1.89-1.82 (2H, br m), 1.42-1.28 (2H, br m), 1.25-1.03 (9H, br m). | 422 | 420 | |
| 416 | 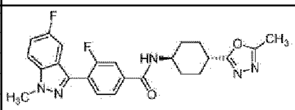 | 1H-NMR (DMSO-D6) δ: 8.49 (1H, d, J = 8.2 Hz), 7.92-7.79 (4H, m), 7.51 (1H, d, J = 9.0 Hz), 7.41 (1H, t, J = 9.0 Hz), 4.17 (3H, s), 3.92-3.81 (1H, m), 2.95-2.86 (1H, m), 2.47 (3H, s), 2.18-2.09 (2H, br m), 2.04-1.96 (2H, br m), 1.69-1.46 (4H, m). | 452 | 450 | |

[Table 1-53]

| Ex.No. | structural formula | 1H-NMR (400 MHz) | MS (positive) | MS (negative) | Comments |
|---|---|---|---|---|---|
| 417 | 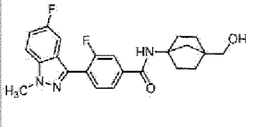 | 1H-NMR (DMSO-D6) δ: 8.55 (1H, s), 7.89-7.78 (4H, m), 7.50 (1H, dt, J = 9.7, 2.2 Hz), 7.40 (1H, td, J = 9.0, 1.5 Hz), 4.47 (1H, t, J = 5.2 Hz), 4.16 (3H, s), 3.44 (2H, d, J = 5.2 Hz), 1.97-1.83 (4H, br m), 1.71-1.59 (4H, br m), 1.36-1.26 (2H, br m). | 412 | 410 | |
| 418 | 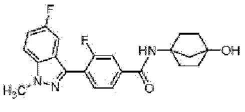 | 1H-NMR (DMSO-D6) δ: 8.49 (1H, s), 7.89-7.77 (4H, m), 7.50 (1H, d, J = 9.0 Hz), 7.40 (1H, t, J = 9.0 Hz), 4.92 (1H, s), 4.16 (3H, s), 2.05-1.83 (6H, br m), 1.76-1.66 (2H, br m), 1.61-1.52 (2H, br m). | 398 | 396 | |
| 419 | 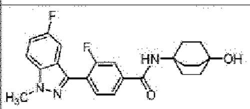 | 1H-NMR (DMSO-D6) δ: 7.87-7.73 (5H, m), 7.48 (1H, d, J = 9.7 Hz), 7.40 (1H, t, J = 9.0 Hz), 4.30 (1H, s), 4.16 (3H, s), 2.10-2.01 (6H, br m), 1.68-1.58 (6H, br m). | 412 | 410 | |
| 420 | 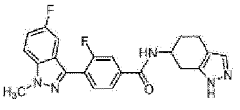 | 1H-NMR (DMSO-D6) δ: 12.33 (1H, s), 8.62 (1H, d, J = 7.5 Hz), 7.95-7.86 (3H, m), 7.81 (1H, dd, J = 9.0, 3.7 Hz), 7.52 (1H, d, J = 9.7 Hz), 7.44-7.38 (1H, m), 7.33 (1H, br s), 4.26-4.18 (1H, br m), 4.17 (3H, s), 3.01-2.92 (1H, br m), 2.71-2.61 (2H, br m), 2.60-2.53 (1H, br m), 2.05-1.97 (1H, br m), 1.82-1.68 (1H, m). | 408 | 406 | race-mate |
| 421 | 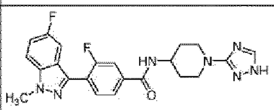 | 1H-NMR (DMSO-D6) δ: 13.05 (0.4H, br s), 12.57 (0.6H, br s), 8.47 (1H, d, J = 7.5 Hz), 8.17 (0.4H, br s), 7.92-7.78 (4H, m), 7.51 (1H, d, J = 9.7 Hz), 7.47 (0.6H, br s), 7.41 (1H, td, J = 9.0, 2.2 Hz), 4.16 (3H, s), 4.11-3.83 (3H, br m), 3.10-2.80 (2H, br m), 1.92-1.80 (2H, br m), 1.68-1.55 (2H, br m). | 438 | 436 | tauto-mer |
| 422 | 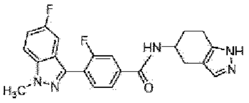 | 1H-NMR (DMSO-D6) δ: 12.33 (1H, s), 8.59 (1H, d, J = 7.5 Hz), 7.93-7.84 (3H, m), 7.81 (1H, dd, J = 9.0, 4.5 Hz), 7.52 (1H, d, J = 9.7 Hz), 7.41 (1H, td, J = 9.0, 2.2 Hz), 7.32 (1H, br s), 4.20-4.10 (4H, br m), 2.90-2.63 (3H, br m), 2.59-2.51 (1H, m), 2.09-2.01 (1H, br m), 1.90-1.78 (1H, m). | 408 | 406 | race-mate |
| 423 | 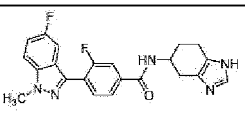 | 1H-NMR (DMSO-D6) δ: 11.64 (1H, br s), 8.61 (1H, d, J = 7.6 Hz), 7.92-7.86 (3H, m), 7.81 (1H, dd, J = 9.1, 4.3 Hz), 7.51 (1H, dt, J = 9.5, 2.5 Hz), 7.45-7.38 (2H, m), 4.28-4.18 (1H, m), 4.17 (3H, s), 2.87-2.79 (1H, br m), 2.66-2.58 (3H, br m), 2.07-2.00 (1H, br m), 1.89-1.79 (1H, br m). | 408 | 406 | race-mate |
| 424 | 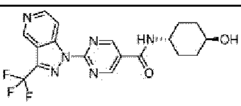 | 1H-NMR (DMSO-D6) δ: 9.34 (1H, s), 9.33 (2H, s), 8.80 (1H, d, J = 6.0 Hz), 8.67-8.64 (2H, m), 4.57 (1H, s), 3.80-3.72 (1H, m), 3.46-3.37 (1H, m), 1.89-1.86 (4H, m), 1.43-1.22 (4H, m). | 407 | 405 | |

[Table 1-54]

| Ex.No. | structural formula | 1H-NMR (400 MHz) | MS (positive) | MS (negative) | Comments |
|---|---|---|---|---|---|
| 425 | 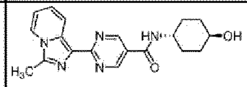 | 1H-NMR (DMSO-D6) δ: 9.11 (2H, s), 8.50 (1H, d, J = 9.1 Hz), 8.41 (1H, d, J = 7.6 Hz), 8.31 (1H, d, J = 7.2 Hz), 7.19 (1H, dd, J = 9.1, 6.5 Hz), 6.95-6.91 (1H, m), 4.57 (1H, d, J = 4.4 Hz), 3.79-3.70 (1H, m), 3.46-3.37 (1H, m), 2.68 (3H, s), 1.90-1.83 (4H, m), 1.43-1.21 (4H, m). | 352 | 396 | |
| 426 | 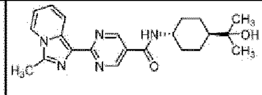 | 1H-NMR (DMSO-D6) δ: 9.13 (2H, s), 8.51 (1H, d, J = 8.8 Hz), 8.44 (1H, d, J = 7.9 Hz), 8.32 (1H, d, J = 6.7 Hz), 7.23-7.19 (1H, m), 6.97-6.93 (1H, m), 4.04 (1H, br s), 3.78-3.68 (1H, m), 2.70 (3H, s), 1.97-1.91 (2H, m), 1.88-1.82 (2H, m), 1.36-1.09 (5H, m), 1.05 (6H, s). | 394 | 438 | |
| 427 | 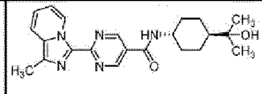 | 1H-NMR (DMSO-D6) δ: 9.79 (1H, d, J = 6.9 Hz), 9.23 (2H, s), 8.54 (1H, d, J = 7.9 Hz), 7.83 (1H, d, J = 8.8 Hz), 7.11-7.02 (2H, m), 4.05 (1H, s), 3.79-3.69 (1H, m), 2.55 (3H, s), 1.98-1.94 (2H, m), 1.89-1.83 (2H, m), 1.37-1.23 (2H, m), 1.24-1.02 (3H, m), 1.06 (6H, s). | 394 | 392 | |
| 428 | 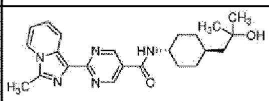 | 1H-NMR (DMSO-D6) δ: 9.11 (2H, s), 8.52-8.49 (1H, m), 8.41 (1H, d, J = 7.9 Hz), 8.31 (1H, d, J = 7.2 Hz), 7.21-7.17 (1H, m), 6.95-6.91 (1H, m), 4.04 (1H, s), 3.78-3.68 (1H, m), 2.69 (3H, s), 1.90-1.84 (4H, m), 1.46-1.26 (3H, m), 1.29 (2H, d, J = 5.3 Hz), 1.11 (6H, s), 1.14-0.95 (2H, m). | 408 | 452 | |
| 429 | 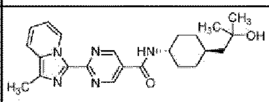 | 1H-NMR (DMSO-D6) δ: 9.79 (1H, d, J = 6.9 Hz), 9.22 (2H, s), 8.53 (1H, d, J = 7.6 Hz), 7.83 (1H, d, J = 8.8 Hz), 7.11-7.01 (2H, m), 4.04 (1H, s), 3.79-3.70 (1H, m), 2.55 (3H, s), 1.90-1.85 (4H, m), 1.46-1.23 (3H, m), 1.29 (2H, d, J = 5.1 Hz), 1.11 (6H, s), 1.14-1.00 (2H, m). | 408 | 406 | |

**Experimental Example 1: Measurement of human H-PGDS binding inhibitory activity**

The H-PGDS binding inhibitory activity of the test compound was measured by AlphaLISA antagonistic assay. A specific protocol is as follows.

In an AlphaPlate-384 Shallow well plate (Cat. 6008350, PerkinElmer) were added and mixed 240 nL/well of DMSO solution as a compound solution or a solvent control, 3 μL/well (final concentration: 45 nmol/L) of biotinylated H-PGDS inhibitor (see below for the production method) diluted with 2%(v/v) DMSO-containing Assay Buffer (50 mM HEPES-NaOH (pH 7.5), 2 mM GSH, 150 mM NaCl, 2 mM $MgCl_2$, 0.005% Surfactant P20), and 2 μL/well (final concentration: 0.3 nmol/L) of His-tagged human H-PGDS (Cat. ATGP1557, ATgen) diluted with Assay Buffer. As a blank, 2 μL/well of Assay Buffer was added instead of His-tagged human H-PGDS. This plate was allowed to stand at room temperature for 60 min, and Nickel Chelate AlphaLISA Acceptor Beads (Cat. AL108C, Perkin Elmer) at 5 μL/well (final concentration: 20 μg/mL) were added and mixed. After allowing to stand at room temperature for 30 min under shading, AlphaScreen Streptavidin donor beads (Cat. 6760002S, Perkin Elmer) were added at 5 μL/well (final concentration: 30 μg/mL) and mixed. After allowing to stand at room temperature for 60 min under shading, AlphaLISA signals (excitation wavelength: 680 nm, measurement wavelength: 615 nm) were measured using Enspire (Perkin Elmer).

The H-PGDS binding inhibitory activity (inhibitory rate) of each test compound was calculated as follows, and the $IC_{50}$ value was calculated from the inhibitory rate at two concentrations sandwiching 50%.

$$\text{inhibitory rate } (\%) = \left( 1 - \frac{\text{fluorescence of test compound well} - \text{fluorescence of blank well}}{\text{fluorescence of solvent control well} - \text{fluorescence of blank well}} \right) \times 100$$

The results of human H-PGDS binding inhibitory activity are shown in the following Table 2.

[Table 2-1]

| Example No. | human H-PGDS binding inhibitory activity $IC_{50}$ (nM) | Example No. | human H-PGDS binding inhibitory activity $IC_{50}$ (nM) | Example No. | human H-PGDS binding inhibitory activity $IC_{50}$ (nM) |
|---|---|---|---|---|---|
| 1 | 3 | 41 | 374 | 81 | 32 |
| 2 | 30 | 42 | 200 | 82 | 359 |
| 3 | 21 | 43 | 15 | 83 | 9%* |
| 4 | 5 | 44 | 541 | 84 | 418 |
| 5 | 60 | 45 | 85 | 85 | 48%* |
| 6 | 3 | 46 | 10 | 86 | 9%* |
| 7 | 11 | 47 | 25%* | 87 | 8%* |
| 8 | 6 | 48 | 423 | 88 | 497 |
| 9 | 13 | 49 | 589 | 89 | 373 |
| 10 | 6 | 50 | 74 | 90 | 6%* |
| 11 | 171 | 51 | 155 | 91 | 39%* |
| 12 | 31 | 52 | 36%* | 92 | 42%* |
| 13 | 578 | 53 | 322 | 93 | 280 |
| 14 | 159 | 54 | 41 | 94 | 871 |
| 15 | 12 | 55 | 3 | 95 | 144 |
| 16 | 372 | 56 | 54 | 96 | 490 |
| 17 | 27 | 57 | 372 | 97 | 33 |
| 18 | 45 | 58 | 172 | 98 | 2 |
| 19 | 4 | 59 | 68 | 99 | 487 |
| 20 | 11%* | 60 | 503 | 100 | 74 |
| 21 | 885 | 61 | 491 | 101 | 328 |
| 22 | 129 | 62 | 319 | 102 | 55 |
| 23 | 53 | 63 | 152 | 103 | 473 |
| 24 | 3 | 64 | 827 | 104 | 15%* |
| 25 | 973 | 65 | 289 | 105 | 5 |
| 26 | 166 | 66 | 42%* | 106 | 131 |
| 27 | 225 | 67 | 18%* | 107 | 21%* |
| 28 | 13 | 68 | 8%* | 108 | 10%* |
| 29 | 262 | 69 | 27 | 109 | 492 |
| 30 | 49 | 70 | 3 | 110 | 37%* |
| 31 | 167 | 71 | 29 | 111 | 222 |
| 32 | 19 | 72 | 3 | 112 | 481 |
| 33 | 415 | 73 | 9%* | 113 | 42 |
| 34 | 42%* | 74 | 7%* | 114 | 113 |
| 35 | 33 | 75 | 22%* | 115 | 138 |
| 36 | 772 | 76 | 7%* | 116 | 33 |
| 37 | 684 | 77 | 32%* | 117 | 39%* |
| 38 | 81 | 78 | 15 | 118 | 31 |
| 39 | 71 | 79 | 54 | 119 | 244 |
| 40 | 7 | 80 | 17 | 120 | 15 |

*Inhibition rate (%) at 1000 nM

[Table 2-2]

| Example No. | human H-PGDS binding inhibitory activity $IC_{50}$ (nM) | Example No. | human H-PGDS binding inhibitory activity $IC_{50}$ (nM) | Example No. | human H-PGDS binding inhibitory activity $IC_{50}$ (nM) |
|---|---|---|---|---|---|
| 121 | 6 | 161 | 30 | 201 | 19 |
| 122 | 16 | 162 | 81 | 202 | 53 |
| 123 | 4 | 163 | 41%** | 203 | 13 |
| 124 | 8 | 164 | 25%** | 204 | 14 |
| 125 | 14 | 165 | 48 | 205 | 8 |
| 126 | 19 | 166 | 22%** | 206 | 37 |
| 127 | 10 | 167 | 30%** | 207 | 18 |
| 128 | 5 | 168 | 12 | 208 | 26 |
| 129 | 17 | 169 | 7 | 209 | 10 |
| 130 | 8 | 170 | 4 | 210 | 23 |
| 131 | 33 | 171 | 47 | 211 | 22 |
| 132 | 6 | 172 | 3 | 212 | 47%** |
| 133 | 96 | 173 | 14 | 213 | 42 |
| 134 | 19 | 174 | 19 | 214 | 40 |
| 135 | 6 | 175 | 32 | 215 | 37 |
| 136 | 48 | 176 | 4 | 216 | 31 |
| 137 | 33 | 177 | 8 | 217 | 373 |
| 138 | 18 | 178 | 60 | 218 | 405 |
| 139 | 7 | 179 | 11 | 219 | 421 |
| 140 | 25 | 180 | 52 | 220 | 596 |
| 141 | 6 | 181 | 26 | 221 | 69 |
| 142 | 16 | 182 | 36 | 222 | 17 |
| 143 | 24 | 183 | 6 | 223 | 10 |
| 144 | 41 | 184 | 5 | 224 | 12 |
| 145 | 10 | 185 | 236 | 225 | 7 |
| 146 | 50 | 186 | 19 | 226 | 34 |
| 147 | 258 | 187 | 22 | 227 | 34 |
| 148 | 349 | 188 | 15 | 228 | 3 |
| 149 | 19 | 189 | 6 | 229 | 3 |
| 150 | 14 | 190 | 21 | 230 | 25%** |
| 151 | 10 | 191 | 11 | 231 | 62 |
| 152 | 46 | 192 | 2 | 232 | 29 |
| 153 | 18 | 193 | 3 | 233 | 10 |
| 154 | 72 | 194 | 3 | 234 | 8 |
| 155 | 31 | 195 | 11 | 235 | 41%** |
| 156 | 17 | 196 | 14 | 236 | 53 |
| 157 | 544 | 197 | 19 | 237 | 5 |
| 158 | 155 | 198 | 13 | 238 | 81 |
| 159 | 26 | 199 | 8 | 239 | 19 |
| 160 | 20 | 200 | 16 | 240 | 33 |

**Inhibition rate (%) at 100 nM

[Table 2-3]

| Example No. | human H-PGDS binding inhibitory activity $IC_{50}$ (nM) | Example No. | human H-PGDS binding inhibitory activity $IC_{50}$ (nM) | Example No. | human H-PGDS binding inhibitory activity $IC_{50}$ (nM) |
|---|---|---|---|---|---|
| 241 | 2 | 281 | 15 | 321 | 143 |
| 242 | 49%** | 282 | 17 | 322 | 335 |
| 243 | 88 | 283 | 9 | 323 | 32 |
| 244 | 17 | 284 | 11 | 324 | 21%* |
| 245 | 12 | 285 | 12 | 325 | 30%* |
| 246 | 13 | 286 | 8 | 326 | 22%* |
| 247 | 5 | 287 | 12 | 327 | 146 |
| 248 | 4 | 288 | 7 | 328 | 417 |
| 249 | 3 | 289 | 9 | 329 | 34 |
| 250 | 19 | 290 | 3 | 330 | 289 |
| 251 | 6 | 291 | 8 | 331 | 352 |
| 252 | 5 | 292 | 5 | 332 | 7 |
| 253 | 2 | 293 | 43 | 333 | 66 |
| 254 | 14 | 294 | 67 | 334 | 186 |
| 255 | 7 | 295 | 7 | 335 | 15 |
| 256 | 22 | 296 | 60 | 336 | 39%** |
| 257 | 27 | 297 | 51%*** | 337 | 46%* |
| 258 | 36 | 298 | 31 | 338 | 36%* |
| 259 | 17 | 299 | 14 | 339 | 48%* |
| 260 | 11 | 300 | 57 | 340 | 46%* |
| 261 | 63 | 301 | 41 | 341 | 25%* |
| 262 | 10 | 302 | 28 | 342 | 207 |
| 263 | 26 | 303 | 13 | 343 | 145 |
| 264 | 27 | 304 | 9 | 344 | 23%* |
| 265 | 13 | 305 | 7 | 345 | 32%* |
| 266 | 3 | 306 | 12 | 346 | 26 |
| 267 | 16 | 307 | 8 | 347 | 100 |
| 268 | 3 | 308 | 6 | 348 | 585 |
| 269 | 9 | 309 | 20 | 349 | 63 |
| 270 | 38 | 310 | 7 | 350 | 546 |
| 271 | 13 | 311 | 11 | 351 | 39 |
| 272 | 9 | 312 | 13 | 352 | 39 |
| 273 | 5 | 313 | 19 | 353 | 136 |
| 274 | 5 | 314 | 14 | 354 | 13 |
| 275 | 7 | 315 | 5 | 355 | 589 |
| 276 | 36 | 316 | 60 | 356 | 51 |
| 277 | 4 | 317 | 176 | 357 | 478 |
| 278 | 10 | 318 | 56 | 358 | 14%* |
| 279 | 8 | 319 | 30 | 359 | 50 |
| 280 | 20 | 320 | 10 | 360 | 32%* |

*Inhibition rate (%) at 1000 nM; **Inhibition rate (%) at 100 nM; ***Inhibition rate (%) at 3 nM

[Table 2-4]

| Example No. | human H-PGDS binding inhibitory activity $IC_{50}$ (nM) | Example No. | human H-PGDS binding inhibitory activity $IC_{50}$ (nM) |
|---|---|---|---|
| 361 | 293 | 401 | 453 |
| 362 | 13%* | 402 | 510 |
| 363 | 13%* | 403 | 101 |
| 364 | 413 | 404 | 37%* |
| 365 | 39 | 405 | 338 |
| 366 | 19%* | 406 | 33%* |
| 367 | 45%* | 407 | 443 |
| 368 | 8%* | 408 | 510 |
| 369 | 520 | 409 | 107 |
| 370 | 21%* | 410 | 16%* |
| 371 | 11%* | 411 | 21%* |
| 372 | 50 | 412 | 506 |
| 373 | 957 | 413 | 152 |
| 374 | 43%* | 414 | 12%* |
| 375 | 4%* | 415 | 13%* |
| 376 | 556 | 416 | 144 |
| 377 | 57 | 417 | 150 |
| 378 | 589 | 418 | 325 |
| 379 | 75 | 419 | 109 |
| 380 | 3 | 420 | 401 |
| 381 | 238 | 421 | 238 |
| 382 | 25 | 422 | 267 |
| 383 | 25%* | 423 | 461 |
| 384 | 386 | 424 | 16%* |
| 385 | 7%* | 425 | 31%** |
| 386 | 56 | 426 | 27 |
| 387 | 6 | 427 | 7 |
| 388 | 19%* | 428 | 25 |
| 389 | 663 | 429 | 6 |
| 390 | 308 | | |
| 391 | 142 | | |
| 392 | 6 | | |
| 393 | 46 | | |
| 394 | 181 | | |
| 395 | 23 | | |
| 396 | 129 | | |
| 397 | 51 | | |
| 398 | 37 | | |
| 399 | 93 | | |
| 400 | 56 | | |

*Inhibition rate (%) at 1000 nM; **Inhibition rate (%) at 100 nM

Production of biotinylated H-PGDS inhibitor

The biotinylated H-PGDS inhibitor used in Experimental Example 1 was produced by biotinylating the H-PGDS inhibitor produced by the following steps. The H-PGDS inhibitor subjected to biotinylation may be any of enantiomers A and B described later, and enantiomer B was used in this Experimental Example.

Production of H-PGDS inhibitor (3-{4-[4-(3,7-difluoro-1H-pyrrolo[3,2-c]pyridin-4-yl)piperidine-1-carboxamido]bicyclo[2.2.2]octan-1-yl}butyric acid)

The title compound represented by the following formula:

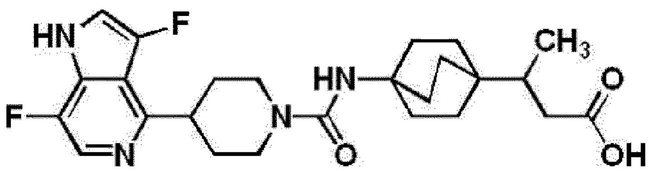

has an asymmetric carbon at the β-position of the carboxy group.

In this Production Example, asymmetric reduction is performed using a chiral phosphine ligand in the following step 10A or step 10B. A compound obtained using R-Sp form as the chiral phosphine ligand is referred to as enantiomer A, and a compound obtained using S-Rp form is referred to as enantiomer B.

Step 1: tert-Butyl 4-(7-fluoro-1H-pyrrolo [3,2-c]pyridin-4-yl)-3,6-dihydropyridine-1(2H)-carboxylate

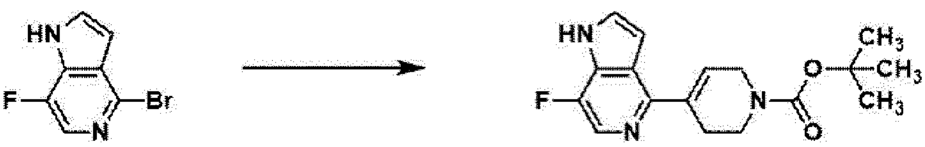

Under an argon atmosphere, to a solution of 4-bromo-7-fluoro-1H-pyrrolo[3,2-c]pyridine (4.5 g) and tert-butyl 4-(4,4,5,5-tetramethyl-1,3,2-dioxaborolan-2-yl)-3,6-dihydropyridine-1(2H)-carboxylate (9.6 g) in toluene/ethanol (42 mL, toluene/ethanol=2.5/1) were added 2 M aqueous sodium carbonate solution (21 mL) and [1,1'-bis(di-tert-butylphosphino)ferrocene]palladium(II) dichloride-dichloromethane adduct (1.7 g) at room temperature, and the mixture was stirred at 100°C overnight. The reaction mixture was filtered through celite, and water and ethyl acetate were added thereto. After partitioning, the organic layer was washed with saturated brine. The organic layer was dried over sodium sulfate, sodium sulfate was removed by filtration, and the filtrate was concentrated under reduced pressure. The residue was purified by silica gel column chromatography (eluent: n-hexane/ethyl acetate=70/30 to 35/65) to give the title compound (6.3 g).
$^1$H-NMR (DMSO-$D_6$) δ: 1.44 (9H, s), 2.68-2.71 (2H, m), 3.54-3.58 (2H, m), 4.08-4.11 (2H, m), 6.49-6.51 (1H, m), 6.86-6.88 (1H, m), 7.55-7.57 (1H, m), 8.10 (1H, d, J = 2.1 Hz), 12.20 (1H, br s).

Step 2: tert-Butyl 4-(7-fluoro-1H-pyrrolo[3,2-c]pyridin-4-yl)piperidine-1-carboxylate

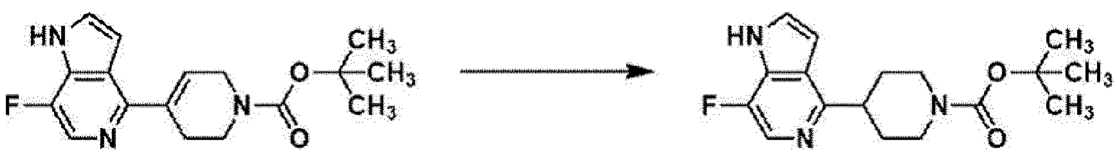

To a solution of tert-butyl 4-(7-fluoro-1H-pyrrolo[3,2-c]pyridin-4-yl)-3,6-dihydropyridine-1(2H)-carboxylate (6.3 g) in tetrahydrofuran/methanol (63 mL, tetrahydrofuran/methanol=1/1) was added 10% palladium-carbon (1.3 g) at room temperature, and the mixture was stirred overnight under 1 atm hydrogen atmosphere. The reaction mixture was filtered through celite, and the filtrate was concentrated under reduced pressure to give the title compound (6.3 g).
$^1$H-NMR (DMSO-$D_6$) δ: 1.42 (9H, s), 1.70-1.80 (4H, m), 2.83-2.99 (2H, m), 3.26-3.36 (1H, m), 4.04-4.10 (2H, m), 6.75-6.77

(1H, m), 7.49-7.51 (1H, m), 8.03 (1H, d, J = 2.7 Hz), 12.09 (1H, br s).

Step 3:tert-Butyl 4-(3,7-difluoro-1H-pyrrolo[3,2-c]pyridin-4-yl)piperidine-1-carboxylate

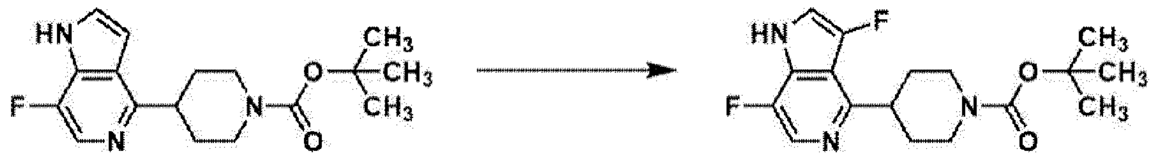

Under an argon atmosphere, to a suspension of tert-butyl 4-(7-fluoro-1H-pyrrolo[3,2-c]pyridin-4-yl)piperidine-1-carboxylate (3.3 g) in acetonitrile (33 mL) was added acetic acid (6.6 mL) at room temperature. To the reaction mixture was added N-fluoro-N'-(chloromethyl)triethylenediamine bis(tetrafluoroborate) (5.5 g) in several divided portions at room temperature, and the mixture was stirred for 2 hr. The reaction mixture was added dropwise to saturated aqueous sodium hydrogen carbonate solution (120 mL) over 10 min at room temperature. To the reaction mixture was added sodium dithionite (2.5 g) at room temperature, and the mixture was stirred for 30 min. To the reaction mixture was added ethyl acetate and, after partitioning, the organic layer was washed with saturated brine. The organic layer was dried over sodium sulfate, sodium sulfate was removed by filtration, and the filtrate was concentrated under reduced pressure. The residue was purified twice by silica gel column chromatography (eluent: n-hexane/ethyl acetate=80/20 to 50/50) to give the title compound (0.99 g).
$^1$H-NMR ($CDCl_3$) δ: 1.48 (9H, s), 1.87-1.97 (4H, m), 2.84-2.94 (2H, m), 3.35-3.43 (1H, m), 4.22-4.34 (2H, m), 7.06 (1H, t, J = 2.8 Hz), 8.11 (1H, d, J = 2.7 Hz), 8.22 (1H, br s).

Step 4: 3,7-Difluoro-4-(piperidin-4-yl)-1H-pyrrolo[3,2-c]pyridine dihydrochloride

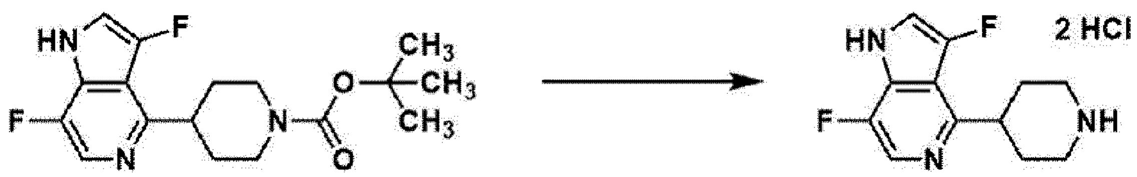

To a solution of tert-butyl 4-(3,7-difluoro-1H-pyrrolo[3,2-c]pyridin-4-yl)piperidine-1-carboxylate (0.99 g) in ethyl acetate (5.0 mL) was added 4 M hydrogen chloride/ethyl acetate (9.9 mL) solution at room temperature, and the mixture was stirred for 3 hr. To the reaction mixture was added ethyl acetate (5.0 mL) at room temperature, and the mixture was stirred for 1 hr. The precipitated solid was collected by filtration and dried under reduced pressure to give the title compound (0.78 g).
$^1$H-NMR (DMSO-$D_6$) δ: 1.99-2.20 (4H, m), 3.04-3.13 (2H, m), 3.37-3.43 (2H, m), 3.53-3.60 (1H, m), 7.76 (1H, s), 8.27 (1H, s), 8.80 (1H, br s), 9.05 (1H, br s), 12.56 (1H, br s).

Step 5: Methyl 4-[(tert-butoxycarbonyl)amino]bicyclo[2.2.2]octane-1-carboxylate

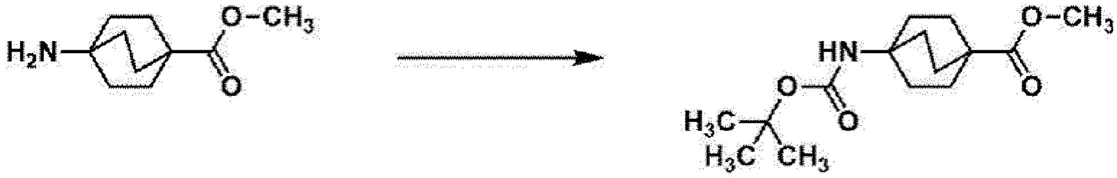

To a suspension of methyl 4-aminobicyclo[2.2.2]octane-1-carboxylate (25 g) and triethylamine (23 mL) in tetrahydrofuran (250 mL) was added di-tert-butyl bicarbonate (38 mL) at room temperature, and the mixture was stirred for 15 hr. The reaction mixture was concentrated under reduced pressure, and n-hexane/ethyl acetate (100 mL, n-hexane/ethyl acetate=90/10) was added to the residue. The precipitated solid was collected by filtration, washed with n-hexane/ethyl acetate (60 mL, n-hexane/ethyl acetate=90/10), and dried under reduced pressure to give the title compound (37 g).
$^1$H-NMR (DMSO-$D_6$) $\delta$: 1.35 (9H, s), 1.73 (12H, s), 3.55 (3H, s), 6.44 (1H, br s).

Step 6: 4-[(tert-Butoxycarbonyl)amino]bicyclo[2.2.2]octane-1-carboxylic acid

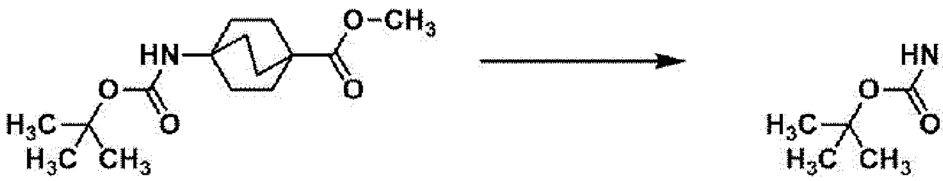

To a suspension of methyl 4-[(tert-butoxycarbonyl)amino]bicyclo[2.2.2]octane-1-carboxylate (37 g) in tetrahydrofuran/methanol (370 mL, tetrahydrofuran/methanol=1/1) was added 2 M aqueous sodium hydroxide solution (130 mL) at room temperature, and the mixture was stirred at 60°C for 4 hr. To the reaction mixture was added 2 M hydrochloric acid (130 mL) under ice-cooling, and the mixture was stirred for 1 hr. The precipitated solid was collected by filtration and dried under reduced pressure to give the title compound (27 g).
$^1$H-NMR (DMSO-$D_6$) $\delta$: 1.35 (9H, s), 1.72 (12H, s), 6.41 (1H, br s), 11.98 (1H, s).

Step 7: tert-Butyl {4-[methoxy(methyl)carbamoyl]bicyclo[2.2.2]octan-1-yl}carbamate

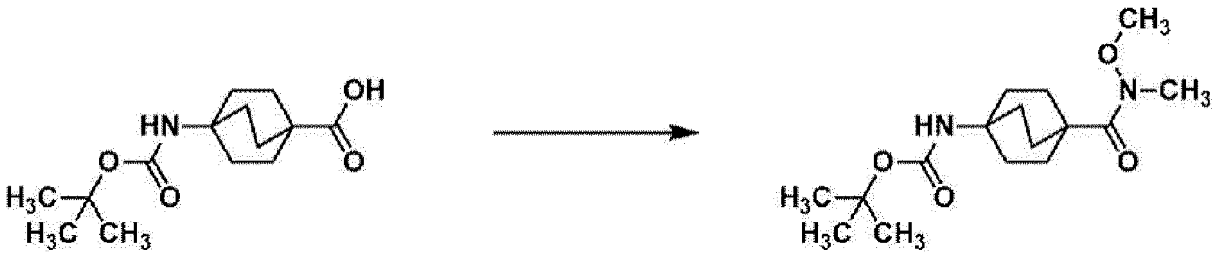

Under an argon atmosphere, to a solution of 4-[(tert-butoxycarbonyl)amino]bicyclo[2.2.2]octane-1-carboxylic acid (10 g) in N,N-dimethylformamide (100 mL) were added O-(7-azabenzotriazol-1-yl)-N,N,N',N'-tetramethyluronium hexafluorophosphate (17 g), N,O-dimethylhydroxylamine hydrochloride (4.4 g) and N,N-diisopropylethylamine (16 mL) at room temperature, and the mixture was stirred for 4 hr. To the reaction mixture were added N,N-diisopropylethylamine (2.6 mL) and N,O-dimethylhydroxylamine hydrochloride (1.5 g) at room temperature, and the mixture was stirred for 18 hr. To the reaction mixture were added water (100 mL) and ethyl acetate (200 mL) at room temperature. After partitioning, the organic layer was washed successively with water (200 mL), saturated aqueous sodium hydrogen carbonate solution (100 mL) and saturated brine (50 mL). The organic layer was dried over sodium sulfate, sodium sulfate was removed by filtration, and the filtrate was concentrated under reduced pressure to give the title compound (12 g).
$^1$H-NMR ($CDCl_3$) $\delta$: 1.42 (9H, s), 1.82-1.87 (6H, m), 1.95-1.99 (6H, m), 3.14 (3H, s), 3.64 (3H, s), 4.35 (1H, br s).

Step 8: tert-Butyl (4-acetylbicyclo[2.2.2]octan-1-yl)carbamate

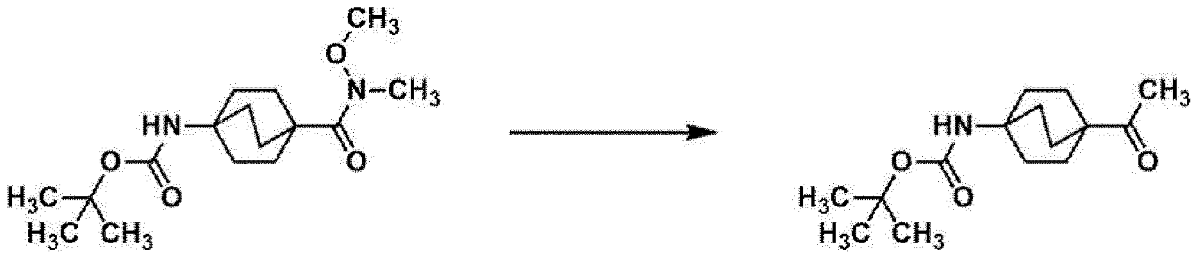

Under an argon atmosphere, to a solution of tert-butyl {4-[methoxy(methyl)carbamoyl]bicyclo[2.2.2]octan-1-yl} carbamate (8.0 g) in tetrahydrofuran (80 mL) was added dropwise 1.1 M tetrahydrofuran (57 mL) solution of methylmagnesium bromide over 15 min under ice-cooling, and the mixture was stirred for 40 min. To the reaction mixture was added 0.95 M tetrahydrofuran (22 mL) solution of methylmagnesium bromide under ice-cooling, and the mixture was stirred at the same temperature for 30 min and at room temperature for 2.5 hr. To the reaction mixture were added 10 wt% aqueous citric acid solution and ethyl acetate (100 mL) at room temperature. After partitioning, the organic layer was washed with saturated brine. The organic layer was dried over sodium sulfate, sodium sulfate was removed by filtration, and the filtrate was concentrated under reduced pressure. To the residue was added n-hexane/ethyl acetate (13 mL, n-hexane/ethyl acetate=2/1), and the mixture was stirred. The precipitated solid was collected by filtration, washed with n-hexane/ethyl acetate (n-hexane/ethyl acetate=10/1) and dried under reduced pressure to give the title compound (2.5 g). In addition, the filtrate was concentrated under reduced pressure, and the residue was purified by silica gel column chromatography (eluent: n-hexane/ethyl acetate=95/5 to 60/40) to give the title compound (3.6 g).
$^1$H-NMR ($CDCl_3$) δ: 1.42 (9H, s), 1.78-1.83 (6H, m), 1.84-1.89 (6H, m), 2.08 (3H, s), 4.35 (1H, br s).

Step 9: Ethyl 3-{4-[(tert-butoxycarbonyl)amino]bicyclo[2.2.2]octan-1-yl}-2-butenoate

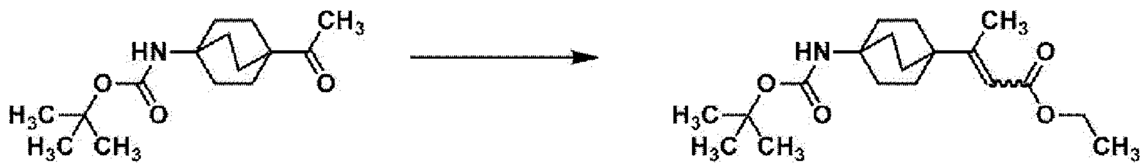

Under an argon atmosphere, to a suspension of sodium hydride (1.8 g, 60 wt% oil dispersion) in tetrahydrofuran (60 mL) was added dropwise tetrahydrofuran (6.0 mL) solution of triethyl phosphonoacetate (10 g) over 20 min under ice-cooling, and the mixture was stirred for 1 hr. To the reaction mixture was added dropwise tetrahydrofuran (30 mL) solution of tert-butyl (4-acetylbicyclo[2.2.2]octan-1-yl)carbamate (6.0 g) over 10 min under ice-cooling. The reaction mixture was stirred at room temperature for 3 hr and at 50°C for 15 hr. To the reaction mixture were added saturated aqueous ammonium chloride solution (100 mL) and ethyl acetate (200 mL) at room temperature. After partitioning, the organic layer was washed with saturated brine (50 mL). The organic layer was dried over sodium sulfate, sodium sulfate was removed by filtration, and the filtrate was concentrated under reduced pressure. The residue was purified by silica gel column chromatography (eluent: n-hexane/ethyl acetate=95/5 to 60/40) to give the title compound (4.1 g).
$^1$H-NMR ($CDCl_3$) δ: 1.27 (3H, t, J = 7.2 Hz), 1.42 (9H, s), 1.68-1.72 (6H, m), 1.85-1.88 (6H, m), 2.10 (3H, s), 4.13 (2H, q, J = 7.2 Hz), 4.34 (1H, br s), 5.63 (1H, s).

Step 10A: Ethyl (S)-3-{4-[(tert-butoxycarbonyl)amino]bicyclo[2.2.2]octan-1-yl}butyrate (enantiomer A)

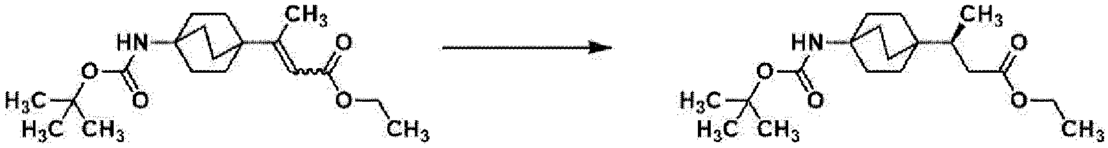

Under an argon atmosphere, a solution of bis[η-(2,5-norbornadiene)]rhodium (I) tetrafluoroborate (28 mg) and (R)-1-[(Sp)-2-(di-tert-butylphosphino)ferrocenyl]ethyl bis(2-methylphenyl)phosphine (R-Sp form) (CAS No.: 849924-76-1) (42 mg) in methanol (1.5 mL) was stirred at room temperature for 1 hr. To the reaction mixture was added

a solution of ethyl 3-{4-[(tert-butoxycarbonyl)amino]bicyclo[2.2.2]octan-1-yl}-2-butenoate (0.50 g) in methanol (4.0 mL) at room temperature, and the mixture was stirred under 4 atm hydrogen atmosphere for 60 hr. The reaction mixture was concentrated under reduced pressure, and the residue was purified by silica gel column chromatography (eluent: n-hexane/ethyl acetate=98/2 to 80/20) to give the title compound (0.46 g).
$^1$H-NMR ($CDCl_3$) δ: 0.81 (3H, d, J = 6.7 Hz), 1.25 (3H, t, J = 7.2 Hz), 1.41-1.48 (6H, m), 1.42 (9H, s), 1.68-1.74 (1H, m), 1.76-1.81 (6H, m), 1.87 (1H, dd, J = 14.6, 10.9 Hz), 2.41 (1H, dd, J = 14.6, 3.2 Hz), 4.12 (2H, q, J = 7.2 Hz), 4.30 (1H, s).

Step 11A: Ethyl (S)-3-(4-aminobicyclo[2.2.2]octan-1-yl)butyrate monohydrochloride (enantiomer A)

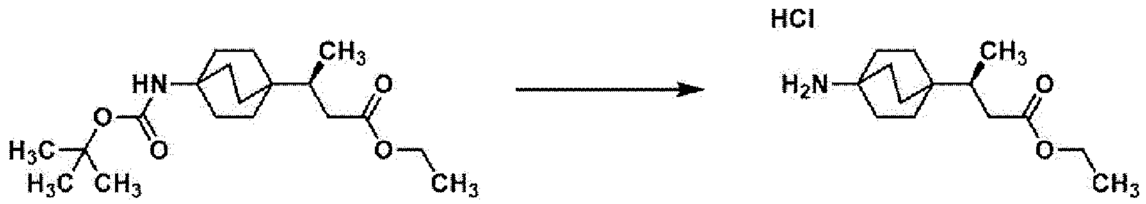

To ethyl (S)-3-{4-[(tert-butoxycarbonyl)amino]bicyclo[2.2.2]octan-1-yl}butyrate (enantiomer A) (0.46 g) was added 4 M hydrogen chloride/ethyl acetate (4.6 mL) solution at room temperature, and the mixture was stirred for 2 hr. The reaction mixture was concentrated under reduced pressure and azeotropically distilled with toluene to give the title compound (0.36 g).
$^1$H-NMR (DMSO-$D_6$) δ: 0.75 (3H, d, J = 6.7 Hz), 1.17 (3H, t, J = 7.1 Hz), 1.38-1.50 (6H, m), 1.59-1.68 (7H, m), 1.83 (1H, dd, J = 14.9, 10.6 Hz), 2.42 (1H, dd, J = 14.9, 3.1 Hz), 4.05 (2H, q, J = 7.1 Hz), 7.86-8.04 (3H, m).

Step 12A: Ethyl (S)-3-{4-[4-(3,7-difluoro-1H-pyrrolo[3,2-c]pyridin-4-yl)piperidine-1-carboxamido]bicyclo[2.2.2]octan-1-yl}butyrate (enantiomer A)

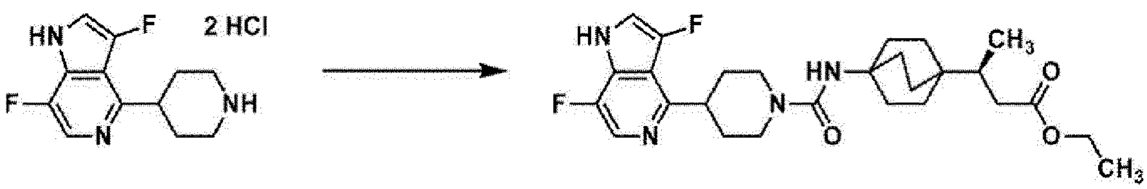

Under an argon atmosphere, to a solution of triphosgene (36 mg) in tetrahydrofuran (1.5 mL) was added dropwise a suspension of ethyl (S)-3-(4-aminobicyclo[2.2.2]octan-1-yl)butyrate monohydrochloride (enantiomer A) (0.10 g) and N,N-diisopropylethylamine (0.14 mL) in tetrahydrofuran (3.0 mL) under ice-cooling, and the mixture was stirred for 1 hr. To the reaction mixture were added N,N-diisopropylethylamine (0.16 mL), water (0.45 mL) and 3,7-difluoro-4-(piperidin-4-yl)-1H-pyrrolo[3,2-c]pyridine dihydrochloride (80 mg) under ice-cooling, and the mixture was stirred at room temperature for 15 hr. The reaction mixture was concentrated under reduced pressure and purified by silica gel column chromatography (eluent: n-hexane/ethyl acetate=85/15 to 30/70) to give the title compound (0.12 g).
$^1$H-NMR ($CDCl_3$) δ: 0.83 (3H, d, J = 6.7 Hz), 1.25 (3H, t, J = 7.1 Hz), 1.46-1.51 (6H, m), 1.71-1.73 (1H, m), 1.85-1.97 (11H, m), 2.44 (1H, dd, J = 14.8, 3.2 Hz), 2.88-2.96 (2H, m), 3.33-3.41 (1H, m), 4.00-4.05 (2H, m), 4.12 (2H, q, J = 7.2 Hz), 4.20 (1H, s), 7.06 (1H, t, J = 2.9 Hz), 8.10 (1H, d, J = 2.8 Hz), 8.31 (1H, br s).

Step 13A: (S)-3-{4-[4-(3,7-Difluoro-1H-pyrrolo[3,2-c]pyridin-4-yl)piperidine-1-carboxamido]bicyclo[2.2.2]octan-1-yl}butyric acid (enantiomer A)

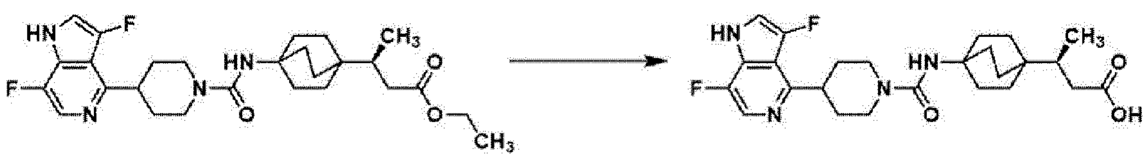

To a solution of ethyl (S)-3-{4-[4-(3,7-difluoro-1H-pyrrolo[3,2-c]pyridin-4-yl)piperidine-1-carboxamido]bicyclo[2.2.2]octan-1-yl}butyrate (enantiomer A) (0.12 g) in tetrahydrofuran/methanol (2.4 mL, tetrahydrofuran/methanol=1/1) was added 2 M aqueous sodium hydroxide solution (0.59 mL) at room temperature, and the mixture was stirred at 65°C for 2 hr. To the reaction mixture were added 2 M hydrochloric acid (0.59 mL), water and ethyl acetate under ice-cooling. After partitioning, the organic layer was washed with saturated brine. The organic layer was dried over sodium sulfate, sodium sulfate was removed by filtration, and the filtrate was concentrated under reduced pressure to give the title compound (0.094 g).

$^1$H-NMR (DMSO-$D_6$) δ: 0.76 (3H, d, J = 6.7 Hz), 1.31-1.46 (6H, m), 1.50-1.61 (1H, m), 1.67-1.82 (11H, m), 2.33-2.39 (1H, m), 2.68-2.77 (2H, m), 3.21-3.29 (1H, m), 4.03-4.09 (2H, m), 5.60 (1H, s), 7.54 (1H, d, J = 2.5 Hz), 8.08 (1H, d, J = 3.0 Hz), 11.95 (2H, br s).
MS (M+H) :475 MS (M-H) :473

Step 10B: Ethyl (R)-3-{4-[(tert-butoxycarbonyl)amino]bicyclo[2.2.2]octan-1-yl}butyrate (enantiomer B)

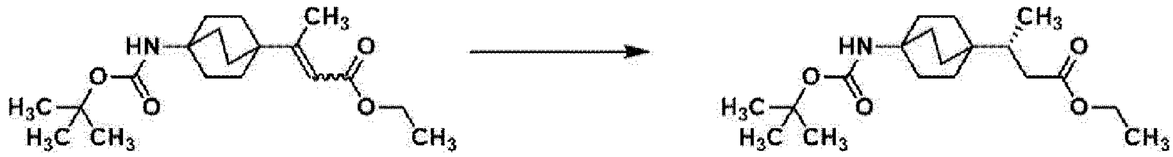

Under an argon atmosphere, a solution of bis[η-(2,5-norbornadiene)]rhodium (I) tetrafluoroborate (28 mg) and (S)-1-[(Rp)-2-(di-tert-butylphosphino)ferrocenyl]ethyl bis(2-methylphenyl)phosphine (S-Rp form) (CAS No.: 849924-77-2) (45 mg) in methanol (1.5 mL) was stirred at room temperature for 1 hr. To the reaction mixture was added a solution of ethyl 3-{4-[(tert-butoxycarbonyl)amino]bicyclo[2.2.2]octan-1-yl}-2-butenoate (0.50 g) in methanol (4.0 mL) at room temperature, and the mixture was stirred under a 4 atm hydrogen atmosphere for 65 hr. The reaction mixture was concentrated under reduced pressure, and the residue was purified by silica gel column chromatography (eluent: n-hexane/ethyl acetate=98/2 to 80/20) to give the title compound (0.54 g).
$^1$H-NMR ($CDCl_3$) δ: 0.81 (3H, d, J = 6.7 Hz), 1.25 (3H, t, J = 7.2 Hz), 1.41-1.48 (6H, m), 1.42 (9H, s), 1.68-1.74 (1H, m), 1.76-1.81 (6H, m), 1.87 (1H, dd, J = 14.6, 10.9 Hz), 2.41 (1H, dd, J = 14.6, 3.2 Hz), 4.12 (2H, q, J = 7.2 Hz), 4.30 (1H, s).

Step 11B: Ethyl (R)-3-(4-aminobicyclo[2.2.2]octan-1-yl)butyrate monohydrochloride (enantiomer B)

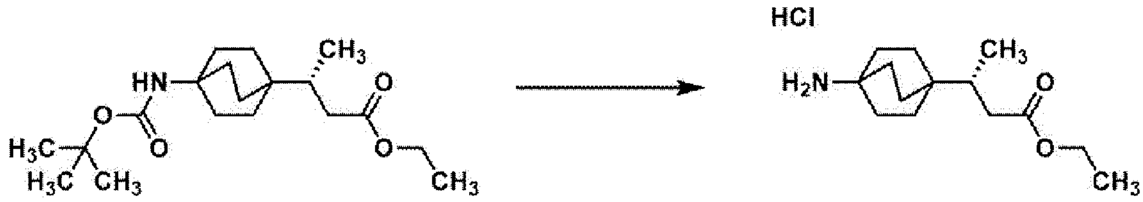

To ethyl (R)-3-{4-[(tert-butoxycarbonyl)amino]bicyclo[2.2.2]octan-1-yl}butyrate (enantiomer B) (0.54 g) was added 4 M hydrogen chloride/ethyl acetate (4.8 mL) solution at room temperature, and the mixture was stirred for 3 hr. The reaction mixture was concentrated under reduced pressure and azeotropically distilled with toluene to give the title compound (0.36 g).
$^1$H-NMR ($CDCl_3$) δ: 0.82 (3H, d, J = 5.3 Hz), 1.23-1.27 (3H, m), 1.48-1.55 (6H, m), 1.73-1.79 (1H, m), 1.85-1.93 (7H, m), 2.38 (1H, d, J = 14.8 Hz), 4.09-4.15 (2H, m), 8.23 (3H, br s).

Step 12B: Ethyl (R)-3-{4-[4-(3,7-difluoro-1H-pyrrolo[3,2-c]pyridin-4-yl)piperidine-1-carboxamido]bicyclo[2.2.2]octan-1-yl}butyrate (enantiomer B)

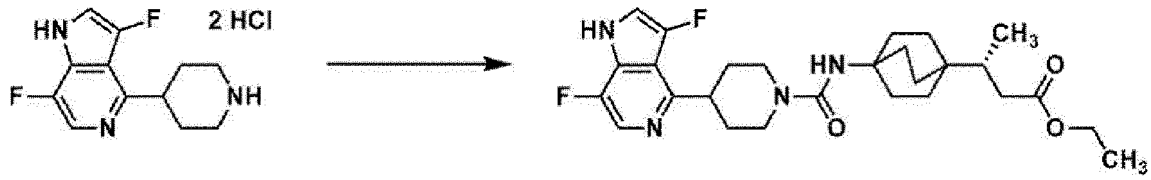

Under an argon atmosphere, to a solution of triphosgene (36 mg) in tetrahydrofuran (0.8 mL) was added dropwise a suspension of ethyl (R)-3-(4-aminobicyclo[2.2.2]octan-1-yl)butyrate monohydrochloride (enantiomer B) (100 mg) and N,N-diisopropylethylamine (0.14 mL) in tetrahydrofuran (1.6 mL) under ice-cooling, and the mixture was stirred for 1 hr. To the reaction mixture were added N,N-diisopropylethylamine (0.16 mL), 3,7-difluoro-4-(piperidin-4-yl)-1H-pyrrolo[3,2-c]pyridine dihydrochloride (80 mg) and water (0.24 mL) under ice-cooling, and the mixture was stirred at room temperature for 15 hr. The reaction mixture was concentrated, and the residue was purified by silica gel column chromatography (eluent: n-hexane/ethyl acetate=80/20 to 0/100) to give the title compound (62 mg).
$^1$H-NMR ($CDCl_3$) δ: 0.83 (3H, d, J = 6.7 Hz), 1.25 (3H, t, J = 7.1 Hz), 1.46-1.51 (6H, m), 1.71-1.73 (1H, m), 1.85-1.97 (11H, m), 2.44 (1H, dd, J = 14.8, 3.2 Hz), 2.88-2.96 (2H, m), 3.33-3.41 (1H, m), 4.00-4.05 (2H, m), 4.12 (2H, q, J = 7.2 Hz), 4.20 (1H, s), 7.06 (1H, t, J = 2.9 Hz), 8.10 (1H, d, J = 2.8 Hz), 8.19 (1H, br s).

Step 13B: (R)-3-{4-[4-(3,7-Difluoro-1H-pyrrolo[3,2-c]pyridin-4-yl)piperidine-1-carboxamido]bicyclo[2.2.2]octan-1-yl}butyric acid (enantiomer B)

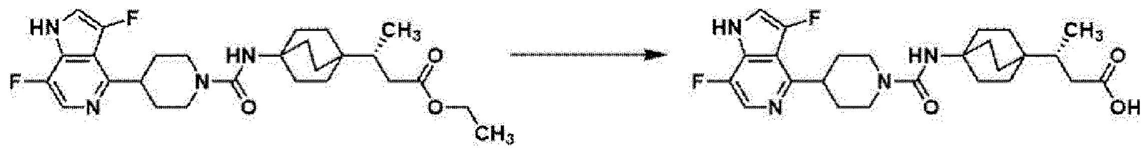

To a solution of ethyl (R)-3-{4-[4-(3,7-difluoro-1H-pyrrolo[3,2-c]pyridin-4-yl)piperidine-1-carboxamido]bicyclo[2.2.2]octan-1-yl}butyrate (enantiomer B) (62 mg) in tetrahydrofuran/methanol (1.2 mL, tetrahydrofuran/methanol=1/1) was added 2 M aqueous sodium hydroxide solution (0.31 mL) at room temperature, and the mixture was stirred at 65°C for 2 hr. To the reaction mixture were added 2 M hydrochloric acid (0.31 mL) and ethyl acetate at room temperature. After partitioning, the organic layer was washed with saturated brine. The organic layer was dried over sodium sulfate, sodium sulfate was removed by filtration, and the filtrate was concentrated under reduced pressure to give the title compound (48 mg).

$^1$H-NMR (DMSO-$D_6$) δ: 0.74 (3H, d, J = 6.7 Hz), 1.30-1.40 (6H, m), 1.51-1.58 (1H, m), 1.66-1.79 (11H, m), 2.31-2.37 (1H, m), 2.65-2.74 (2H, m), 3.21-3.27 (1H, m), 4.01-4.06 (2H, m), 5.59 (1H, s), 7.52-7.53 (1H, m), 8.06 (1H, d, J = 3.0 Hz), 11.90-11.97 (2H, m).
MS (M+H) :475 MS(M-H) :473

Biotinylation of H-PGDS inhibitor ((R)-3-{4-[4-(3,7-difluoro-1H-pyrrolo[3,2-c]pyridin-4-yl)piperidine-1-carboxamido]bicyclo[2.2.2]octan-1-yl}butyric acid) (enantiomer B)

To a solution of (R)-3-{4-[4-(3,7-difluoro-1H-pyrrolo[3,2-c]pyridin-4-yl)piperidine-1-carboxamido]bicyclo[2.2.2]octan-1-yl}butyric acid (enantiomer B) (10 mg) and N-(35-amino-3,6,9,12,15,18,21,24,27,30,33-undecaoxapentatriacontyl)-5-(2-oxohexahydro-1H-thieno[3,4-d]imidazol-4-yl)pentanamide (16.25 mg) in N,N-dimethylformamide (0.5 mL) were added 1-ethyl-3-(3-dimethylaminopropyl)carbodiimide hydrochloride (4.85 mg) and 1H-benzo[d] [1,2,3]triazol-1-ol hydrate at room temperature, and the mixture was left standing overnight. The reaction mixture was purified by silica gel reversed-phase column chromatography (eluent: water/trifluoroacetic acid/acetonitrile) and lyophilized. The residue was added to VariPure IPE column (manufactured by Agilent) and eluted with methanol. The eluate was concentrated under reduced pressure and dried under reduced pressure to give the title compound (14 mg).

Measurement of human H-PGDS inhibitory activity of compound ((R)-3-{4-[4-(3,7-difluoro-1H-pyrrolo[3,2-c]pyridin-4-yl)piperidine-1-carboxamido]bicyclo[2.2.2]octan-1-yl}butyric acid) (enantiomer B)

(1) Preparation of human H-PGDS purified fraction

Using the human H-PGDS expression plasmid DNA (pET28b/hH-PGDS) as a template, PCR (Polymerase Chain Reaction) was performed to amplify a DNA fragment containing human H-PGDS with a NotI recognition cleavage sequence immediately before the translation initiation codon and a BamHI recognition cleavage sequence immediately below the translation end codon. The purified DNA fragment was fused to pET15b (manufactured by Merck KGaA, model number 69661) digested with NotI and BamHI, by using In-Fusion HD Cloning Kit (manufactured by Takara Bio Inc., model number 639649). Human H-PGDS expression plasmid DNA was isolated from Escherichia coli DH5α (manufactured by TOYOBO, model number DNA-903) transformed with the obtained In-Fusion reaction product. The base sequence of human H-PGDS cloned into the vector was determined by the Dye Terminator method using BigDye Terminator v3.1 Cycle Sequencing Kit (manufactured by Applied Biosystems, model number 4337456). The determined sequence was identical to the sequence of the protein translation region of human H-PGDS (Accession number NM_014485.2) registered in the NCBI Reference Database.

Escherichia coli BL21 (DE3) (Merck KGaA, model number 69450-4) transformed with human H-PGDS-expressing plasmid DNA was cultured at 37°C using 2×YT medium (manufactured by Becton Dickinson, model number 244020) until the optical concentration at 620 nm reached 0.6 or higher, and then cultured at 30°C for 6 hr in the presence of 1 mmol/L isopropyl β-D-1-thiogalactopyranoside. After completion of the culture, bacterial cells were collected and suspended in Homogenate Buffer (PBS(-), cOmplete (registered trade mark), EDTA-free (F. Hoffmann-La Roche Ltd, model number 1873580). Using an Ultrasonic disruptor UD-201 (manufactured by TOMY DIGITAL BIOLOGY CO., LTD.), the bacterial cells suspended for 15 seconds at out put:4, duty cycle:50 were disrupted, and this operation was repeated three times. After removing the precipitate by centrifugation (13,100×g, 15 min, 4°C) and ammonium sulfate was added to the supernatant to 40% saturation and stirred at 4°C for 1 hr or more. After removing the precipitate by centrifugation (13,100×g, 10 min, 4°C), ammonium sulfate was added to the supernatant to 60% saturation, and the mixture was stirred at 4°C for 1 hr or more. Thereafter, the precipitate obtained by centrifugation was suspended in PBS(-) and dialyzed twice for 2 hr or more against a dialysis fluid (50 mmol/L potassium phosphate (pH 6.0), 150 mmol/L NaCl, 1 mmol/L $MgCl_2$). The supernatant fraction obtained by centrifuging (21,100×g, 10 min, 4°C) the sample after dialysis was added to a Glutathione Sepharose 4B column (manufactured by GE Healthcare, model number 17075604). After washing the column with the dialysis fluid, human H-PGDS was eluted with the eluent (5 mmol/L Tris-HCl (pH 8.0), 150 mmol/L NaCl, 1 mmol/L $MgCl_2$, 15 mmol/L Glutathione). Glutathione Sepharose 4B column eluate was added to a gel filtration column (Superdex-200 30/100 GL (manufactured by GE HealthCare, model number 17-5175-01)), and eluted with gel filtration Buffer (5 mmol/L Tris-HCl (pH 8.0), 150 mmol/L NaCl, 1 mmol/L $MgCl_2$). Eluted fractions were collected and used as human H-PGDS purified fractions.

The protein concentration of the human H-PGDS purified fraction was measured with Pierce 660nm Protein Assay Reagent (manufactured by Thermo Fisher Scientific Inc., model number 22660). Purified fractions were stored at -80°C after rapid freezing using liquid nitrogen. Human H-PGDS was detected by Western blotting using a rabbit anti-H-PGDS polyclonal antibody (manufactured by LifeSpan BioSciences, Inc, model number LS-B6886).

(2) Evaluation of human H-PGDS inhibitory activity

Calculation of the H-PGDS inhibitory activity of a compound was performed according to the following protocol attached to Prostaglandin D Synthase Inhibitor Screening Assay Kit (Cat. No. 10006595, Cayman Chemical Company) and Prostaglandin D2 Express ELISA Kit (Cat. No. 512041, Cayman Chemical Company). Human recombinant H-PGDS prepared as described above was used.

In a 96 well V-bottom plate (Cat. No. 3363, Costar) were added and mixed lightly 10 μL/well of DMSO solution (10 % v/v) diluted with Assay buffer (0.1 mol/L Tris-HCl, pH 8.0) as a compound solution or a solvent control diluted with Assay buffer, 10 μL/well of $MgCl_2$ solution at a final concentration of 2 mmol/L, 10 μL/well of GSH solution at a final concentration of 0.8 mmol/L, and human recombinant H-PGDS at a final concentration of 1.6 μg/mL, or 10 μL/well of Assay buffer as a blank. This plate was allowed to stand at room temperature for 10 min, 10 μL/well of PGH2 at a final concentration of 50 μmol/L was added to initiate an enzyme reaction, and the reaction was carried out for 1 min while mixing with a plate mixer. After the enzyme reaction, 0.5 mol/L of HCl was added at 10 μL/well to discontinue the reaction. Furthermore, a $FeCl_2$ solution dissolved in 0.1 mol/L hydrochloric acid to a concentration of 0.4 mg/mL was added at 10 μL/well to perform reduction of PGH2. This enzyme reaction solution was diluted 4000-fold with EIA buffer, and the diluted enzyme reaction solution was added to an ELISA plate at 50 μL/well. Prostaglandin D2 Express AChE Tracer and Prostaglandin D2 Express EIA Monoclonal Antibody were added to this plate at 50 μL/well each. As a Non-Specific Binding sample (NSB), 100 μL/well of EIA buffer and 50 μL/well of Prostaglandin D2 Express AChE Tracer were added in another well, and 50

μL/well of EIA buffer and 50 μL/well of Prostaglandin D2 Express AChE Tracer, and 50 μL/well of prostaglandin D2 Express EIA Monoclonal Antibody were added as B0 samples. This plate was reacted for 2 hr while mixing with a plate mixer. After completion of the reaction, the reaction solution was removed and Wash buffer was added at 200 μL/well. This process was repeated 5 times to wash the plate. After washing the plate, 200 μL/well of Ellman's Reagent was added to all wells, and the mixture was reacted for 30 min in shading while mixing with a plate mixer. After completion of the reaction, the absorbance at 420 nm was measured using a microplate reader.

The H-PGDS binding inhibitory activity (inhibitory rate) of the compound was calculated as follows, and the $IC_{50}$ value for H-PGDS activity was calculated from the inhibitory rate at two concentrations sandwiching 50%.

$$\text{inhibitory rate (\%)} = \left( 1 - \frac{B^{*1}/B0^{*2}\ \text{of blank} - B^{*1}/B0^{*2}\ \text{of compound}}{B^{*1}/B0^{*2}\ \text{of blank} - B^{*1}/B0^{*2}\ \text{of solvent control}} \right) \times 100$$

*1: B = $OD_{420}$ of each well - $OD_{420}$ of NSB
*2: B0 = $OD_{420}$ of B0 - $OD_{420}$ of NSB

Also, the human H-PGDS inhibitory activity ($IC_{50}$ value) was calculated for the compound (enantiomer A) according to the above-mentioned method.

The evaluation results of each compound are shown in the following Table 3.

[Table 3]

| compound | | human H-PGDS inhibitory activity $IC_{50}$ (nM) |
|---|---|---|
| (3-{4-[4-(3,7-difluoro-1H-pyrrolo[3,2-c]pyridin-4-yl)piperidine-1-carboxamido]-bicyclo[2.2.2]octan-1-yl}-butyric acid) | enantiomer A | 5 |
| | enantiomer B | 6 |

Formulation Examples of the present invention include the following formulations. However, the present invention is not limited by these Formulation Examples.

Formulation Example 1: Production of capsule

| | |
|---|---|
| 1) compound of Example 1 | 30 mg |
| 2) microcrystalline cellulose | 10 mg |
| 3) lactose | 19 mg |
| 4) magnesium stearate | 1 mg |

1), 2), 3) and 4) are mixed and filled in a gelatin capsule.

Formulation Example 2: Production of tablet

| | |
|---|---|
| 1) compound of Example 1 | 10 g |
| 2) lactose | 50 g |
| 3) cornstarch | 15 g |
| 4) carmellose calcium | 44 g |
| 5) magnesium stearate | 1 g |

A total amount of 1), 2) and 3) and 30 g of 4) are kneaded with water, vacuum dried, and sieved. The sieved powder is mixed with 14 g of 4) and 1 g of 5), and the mixture is punched with a tableting machine. As a result, 1000 tablets containing 10 mg of the compound of Example 1 per tablet are obtained.

[Industrial Applicability]

The compound of the present invention or a pharmaceutically acceptable salt thereof has H-PGDS inhibitory activity, and is useful for the treatment and/or prophylaxis of a disease selected from the group consisting of peripheral arterial diseases (e.g., intermittent claudication and comprehensive severe chronic lower extremity ischemia), cardiovascular diseases, allergic asthma, chronic obstructive pulmonary diseases, allergic rhinitis, sarcopenia, and Duchenne muscular dystrophy.

## Claims

**1.** A compound of the formula [I]:

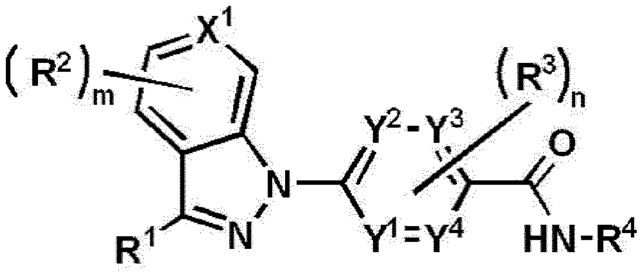

[I]

wherein

$X^1$, $Y^1$, $Y^2$, $Y^3$ and $Y^4$ are each independently a carbon or a nitrogen atom (wherein the total number of the nitrogen atoms for $Y^1$, $Y^2$, $Y^3$ and $Y^4$ is 0, 1 or 2),
m is 0, 1 or 2,
n is 0, 1 or 2,
$R^1$ is

(1) hydroxy,
(2) cyano,
(3) $C_{1-4}$ alkyl (wherein the alkyl is optionally substituted by hydroxy or $C_{1-4}$ alkoxy),
(4) $C_{1-4}$ alkoxy,
(5) halogen,
(6) $C_{1-4}$ haloalkyl, or
(7) $C_{3-6}$ cycloalkyl,

$R^2$ in the number of m are each independently

(1) cyano,
(2) $C_{1-4}$ alkyl,
(3) $C_{1-4}$ alkoxy,
(4) halogen, or
(5) $C_{1-4}$ haloalkyl,

$R^3$ in the number of n are each independently

(1) $C_{1-4}$ alkyl,
(2) $C_{1-4}$ alkoxy, or
(3) halogen, and

$R^4$ is

(1) ring Cy [wherein the ring Cy is

(a) $C_{4-6}$ cycloalkyl (wherein the cycloalkyl is optionally substituted by 1 or 2 substituents independently selected from the group consisting of

(I) hydroxy,
(II) cyano,
(III) oxo,
(IV) $C_{1-6}$ alkyl (wherein the alkyl is optionally substituted by

(i) hydroxy,
(ii) carboxy,
(iii) $-CONH_2$,
(iv) $-CO-C_{1-4}$ alkoxy,
(v) $-SO_2-C_{1-4}$ alkyl, or
(vi) a group represented by the formula:

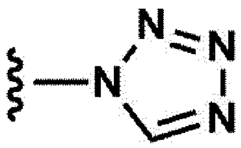
),

(V) $C_{1-6}$ alkoxy (wherein the alkoxy is optionally substituted by hydroxy or $-SO_2-C_{1-4}$ alkyl),
(VI) carboxy,
(VII) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(i) hydroxy, and
(ii) $C_{1-6}$ alkyl (wherein the alkyl is optionally substituted by hydroxy)}],

(VIII) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(IX) $-O-C_{1-6}$ haloalkyl,
(X) a group represented by the formula:

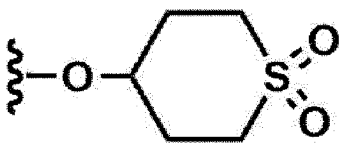
,

(XI) a group represented by the formula:

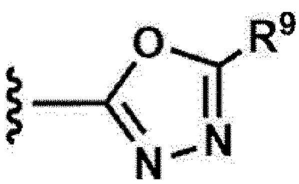

{wherein $R^9$ is $C_{1-4}$ alkyl (wherein the alkyl is optionally substituted by $C_{1-4}$ alkoxy)}, and

(XII) a group represented by the formula:

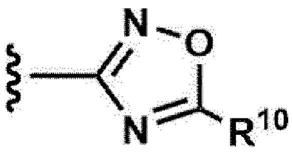

wherein $R^{10}$ is $C_{1-4}$ alkyl)),

(b) $C_{5-8}$ bridged cycloalkyl {wherein the bridged cycloalkyl is optionally substituted by

(I) hydroxy,
(II) $C_{1-6}$ alkyl (wherein the alkyl is optionally substituted by

(i) hydroxy,
(ii) carboxy,
(iii) $-CONH_2$,
(iv) -CO-$C_{1-4}$ alkoxy,
(v) $-SO_2$-$C_{1-4}$ alkyl, or
(vi) a group represented by the formula:

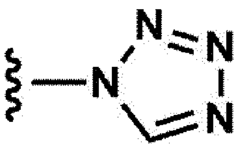 ),

(III) $C_{1-6}$ alkoxy (wherein the alkoxy is optionally substituted by hydroxy or $-SO_2$-$C_{1-4}$ alkyl),
(IV) carboxy,
(V) -CO-$C_{1-4}$ alkoxy,
(VI) -O-$C_{1-6}$ haloalkyl, or
(VII) a group represented by the formula:

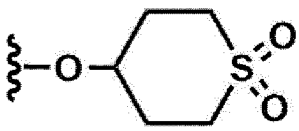 },

(c) 5- or 6-membered heterocycloalkyl containing one nitrogen atom [wherein the heterocycloalkyl is optionally substituted by 1 to 3 substituents independently selected from the group consisting of

(I) oxo,
(II) $C_{1-6}$ alkyl,
(III) -CO-$R^{11}$ {wherein $R^{11}$ is

(i) $C_{1-6}$ alkyl (wherein the alkyl is optionally substituted by

(A) hydroxy,
(B) cyano, or
(C) $C_{1-4}$ alkoxy),

(ii) $C_{1-6}$ alkoxy,
(iii) $C_{1-6}$ haloalkyl,
(iv) $C_{3-6}$ cycloalkyl (wherein the cycloalkyl is optionally substituted by halogen), or
(v) 4- to 6-membered heterocycloalkyl containing one oxygen atom (wherein the heterocycloalkyl is optionally substituted by halogen)}, and

(IV) a group represented by the formula:

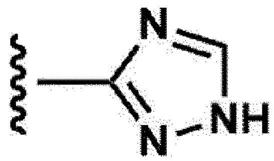

],

(d) 8-membered bridged heterocycloalkyl containing one nitrogen atom {wherein the bridged heterocycloalkyl is optionally substituted by -CO-$R^{11}$},
(e) 7- to 11-membered spiro heterocycloalkyl containing one nitrogen atom {wherein the spiro heterocycloalkyl is optionally substituted by -CO-$R^{11}$},
(f) 6- to 9-membered saturated or partially unsaturated fused ring group containing 1 or 2 nitrogen atoms (wherein the fused ring group is optionally substituted by -CO-$R^{11}$),
(g) a group represented by the formula:

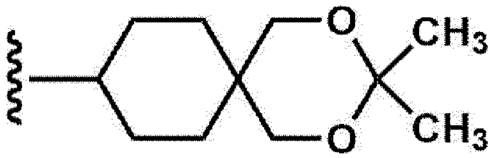

,

(h) a group represented by the formula:

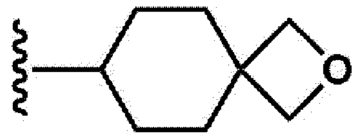

,

or
(i) a group represented by the formula:

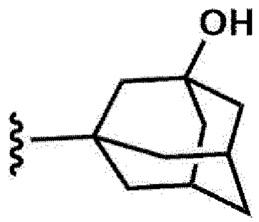

],

(2) $C_{1-4}$ alkyl {wherein the alkyl is optionally substituted by

(a) $C_{3-6}$ cycloalkyl (wherein the cycloalkyl is optionally substituted by 1 or 2 substituents independently selected from the group consisting of hydroxy and $C_{1-4}$ alkyl optionally substituted by hydroxy), or
(b) phenyl}, or

(3) $C_{1-4}$ haloalkyl (wherein the haloalkyl is optionally substituted by $C_{3-6}$ cycloalkyl optionally substituted by hydroxy)],

or a pharmaceutically acceptable salt thereof.

2. The compound of Claim 1, wherein the total number of the nitrogen atoms for $Y^1$, $Y^2$, $Y^3$ and $Y^4$ is 1 or 2, or a pharmaceutically acceptable salt thereof.

3. The compound of Claim 1, which is represented by the formula [IA]:

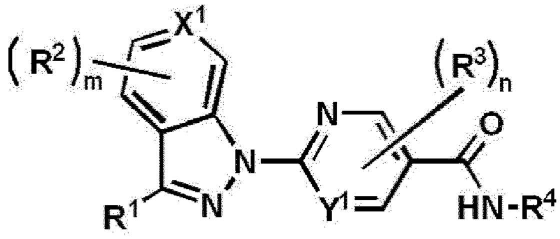

[IA]

(wherein each symbol is as defined for Claim 1), or a pharmaceutically acceptable salt thereof.

**4.** The compound of any one of Claims 1 to 3, wherein $R^3$ is halogen, or a pharmaceutically acceptable salt thereof.

**5.** The compound of any one of Claims 1 to 4, wherein $R^4$ is ring Cy, or a pharmaceutically acceptable salt thereof.

**6.** The compound of Claim 1, which is represented by the formula [IB]:

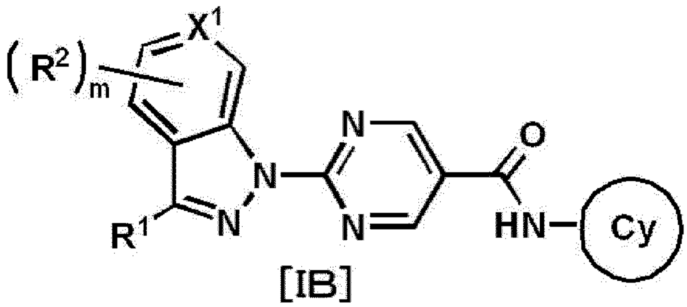

[IB]

(wherein each symbol is as defined for Claim 1), or a pharmaceutically acceptable salt thereof.

**7.** The compound of any one of Claims 1 to 6, wherein $R^1$ is

(1) $C_{1-4}$ alkyl (wherein the alkyl is optionally substituted by hydroxy or $C_{1-4}$ alkoxy),
(2) $C_{1-4}$ alkoxy,
(3) halogen, or
(4) $C_{1-4}$ haloalkyl,

or a pharmaceutically acceptable salt thereof.

**8.** The compound of any one of Claims 1 to 7, wherein $R^2$ is halogen, or a pharmaceutically acceptable salt thereof.

**9.** The compound of any one of Claims 1 to 8, wherein ring Cy is

(1) $C_{4-6}$ cycloalkyl (wherein the cycloalkyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) $-CONH_2$,

(IV) -CO-$C_{1-4}$ alkoxy,
(V) -$SO_2$-$C_{1-4}$ alkyl, or
(VI) a group represented by the formula:

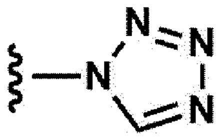
),

(e) $C_{1-6}$ alkoxy (wherein the alkoxy is optionally substituted by hydroxy or -$SO_2$-$C_{1-4}$ alkyl),
(f) carboxy,
(g) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) -$NR^7R^8$ {wherein $R^7$ and $R^8$ are each independently $C_{1-4}$ alkyl, or -CO-$C_{1-6}$ alkyl (wherein the alkyl is optionally substituted by cyano)},
(i) -O-$C_{1-6}$ haloalkyl,
(j) a group represented by the formula:

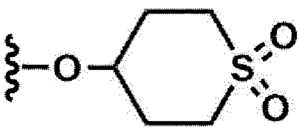
,

(k) a group represented by the formula:

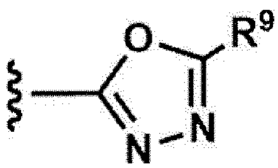

{wherein $R^9$ is $C_{1-4}$ alkyl (wherein the alkyl is optionally substituted by $C_{1-4}$ alkoxy)}, and
(m) a group represented by the formula:

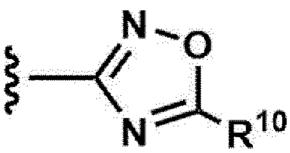

wherein $R^{10}$ is $C_{1-4}$ alkyl)),

(2) $C_{5-8}$ bridged cycloalkyl {wherein the bridged cycloalkyl is optionally substituted by

(a) hydroxy,
(b) $C_{1-6}$ alkyl (wherein the alkyl is optionally substituted by

(I) hydroxy,
(II) carboxy,
(III) -$CONH_2$,

(IV) -CO-$C_{1-4}$ alkoxy,
(V) -$SO_2$-$C_{1-4}$ alkyl, or
(VI) a group represented by the formula:

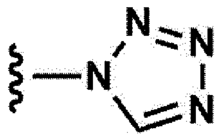
),

(c) $C_{1-6}$ alkoxy (wherein the alkoxy is optionally substituted by hydroxy or -$SO_2$-$C_{1-4}$ alkyl),
(d) carboxy,
(e) -CO-$C_{1-4}$ alkoxy,
(f) -O-$C_{1-6}$ haloalkyl, or
(g) a group represented by the formula:

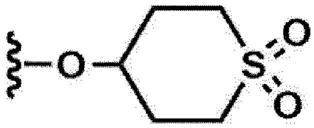
},

(3) 5- or 6-membered heterocycloalkyl containing one nitrogen atom [wherein the heterocycloalkyl is optionally substituted by 1 to 3 substituents independently selected from the group consisting of

(a) oxo,
(b) $C_{1-6}$ alkyl,
(c) -CO-$R^{11}$ {wherein $R^{11}$ is

(I) $C_{1-6}$ alkyl (wherein the alkyl is optionally substituted by

(i) hydroxy,
(ii) cyano, or
(iii) $C_{1-4}$ alkoxy),

(II) $C_{1-6}$ alkoxy,
(III) $C_{1-6}$ haloalkyl,
(IV) $C_{3-6}$ cycloalkyl (wherein the cycloalkyl is optionally substituted by halogen), or
(V) 4- to 6-membered heterocycloalkyl containing one oxygen atom (wherein the heterocycloalkyl is optionally substituted by halogen)}, and

(d) a group represented by the formula:

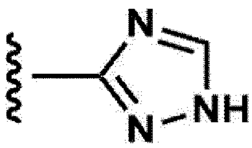
],

(4) 8-membered bridged heterocycloalkyl containing one nitrogen atom {wherein the bridged heterocycloalkyl is optionally substituted by -CO-$R^{11}$},
(5) 7- to 11-membered spiro heterocycloalkyl containing one nitrogen atom {wherein the spiro heterocycloalkyl is optionally substituted by -CO-$R^{11}$}, or
(6) a 6- to 9-membered saturated or partially unsaturated fused ring group containing 1 or 2 nitrogen atoms (wherein the fused ring group is optionally substituted by -CO-$R^{11}$),

or a pharmaceutically acceptable salt thereof.

**10.** The compound of any one of Claims 1 to 9, wherein ring Cy is

(1) $C_{4-6}$ cycloalkyl (wherein the cycloalkyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

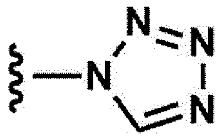

),

(e) $C_{1-6}$ alkoxy (wherein the alkoxy is optionally substituted by hydroxy or $-SO_2-C_{1-4}$ alkyl),
(f) carboxy,
(g) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl, or $-CO-C_{1-6}$ alkyl (wherein the alkyl is optionally substituted by cyano)},
(i) $-O-C_{1-6}$ haloalkyl,
(j) a group represented by the formula:

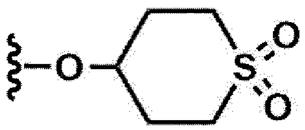

,

(k) a group represented by the formula:

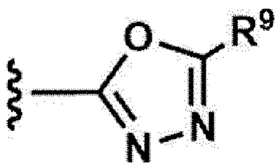

{wherein $R^9$ is $C_{1-4}$ alkyl (wherein the alkyl is optionally substituted by $C_{1-4}$ alkoxy)}, and
(m) a group represented by the formula:

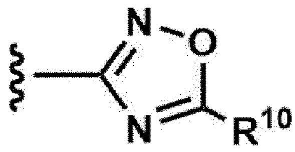

wherein $R^{10}$ is $C_{1-4}$ alkyl)),

(2) $C_{5-8}$ bridged cycloalkyl {wherein the bridged cycloalkyl is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

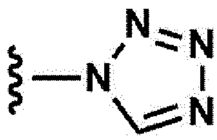 ),

(c) $C_{1-6}$ alkoxy (wherein the alkoxy is optionally substituted by hydroxy or $-SO_2-C_{1-4}$ alkyl),
(d) carboxy,
(e) $-CO-C_{1-4}$ alkoxy,
(f) $-O-C_{1-6}$ haloalkyl, or
(g) a group represented by the formula:

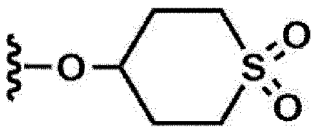 },

(3) 5- or 6-membered heterocycloalkyl containing one nitrogen atom [wherein the heterocycloalkyl is optionally substituted by 1 to 3 substituents independently selected from the group consisting of

(a) oxo,
(b) $C_{1-6}$ alkyl,
(c) $-CO-R^{11}$ {wherein $R^{11}$ is

(I) $C_{1-6}$ alkyl (wherein the alkyl is optionally substituted by

(i) hydroxy,
(ii) cyano, or
(iii) $C_{1-4}$ alkoxy),

(II) $C_{1-6}$ alkoxy,
(III) $C_{1-6}$ haloalkyl,
(IV) $C_{3-6}$ cycloalkyl (wherein the cycloalkyl is optionally substituted by halogen), or
(V) 4- to 6-membered heterocycloalkyl containing one oxygen atom (wherein the heterocycloalkyl is optionally substituted by halogen)}, and

(d) a group represented by the formula:

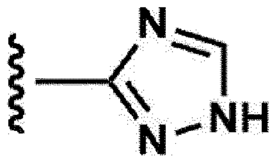 ],

or

(4) 8-membered bridged heterocycloalkyl containing one nitrogen atom {wherein the bridged heterocycloalkyl is optionally substituted by -CO-$R^{11}$},

or a pharmaceutically acceptable salt thereof.

**11.** The compound of any one of Claims 1 to 8, wherein ring Cy is

(1) $C_{4-6}$ cycloalkyl (wherein the cycloalkyl is optionally substituted by 1 or 2 substituents independently selected from the group consisting of

(a) hydroxy,
(b) cyano,
(c) oxo,
(d) $C_{1-6}$ alkyl (wherein the alkyl is optionally substituted by

(I) hydroxy,
(II) carboxy,
(III) -$CONH_2$,
(IV) -CO-$C_{1-4}$ alkoxy,
(V) -$SO_2$-$C_{1-4}$ alkyl, or
(VI) a group represented by the formula:

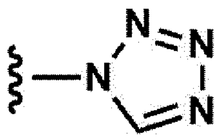 ),

(e) $C_{1-6}$ alkoxy (wherein the alkoxy is optionally substituted by hydroxy or -$SO_2$-$C_{1-4}$ alkyl),
(f) carboxy,
(g) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) -$NR^7R^8$ {wherein $R^7$ and $R^8$ are each independently $C_{1-4}$ alkyl, or -CO-$C_{1-6}$ alkyl (wherein the alkyl is optionally substituted by cyano)},
(i) -O-$C_{1-6}$ haloalkyl,
(j) a group represented by the formula:

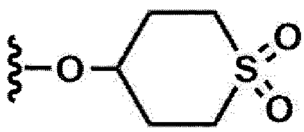,

(k) a group represented by the formula:

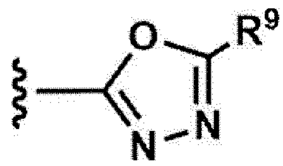

{wherein $R^9$ is $C_{1-4}$ alkyl (wherein the alkyl is optionally substituted by $C_{1-4}$ alkoxy)}, and
(m) a group represented by the formula:

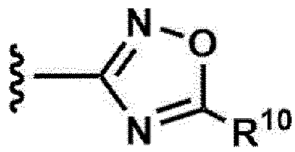

wherein $R^{10}$ is $C_{1-4}$ alkyl)), or

(2) $C_{5-8}$ bridged cycloalkyl {wherein the bridged cycloalkyl is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

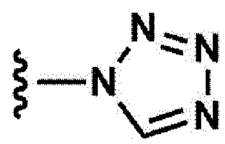

),

(c) $C_{1-6}$ alkoxy (wherein the alkoxy is optionally substituted by hydroxy or $-SO_2-C_{1-4}$ alkyl),
(d) carboxy,
(e) $-CO-C_{1-4}$ alkoxy,
(f) $-O-C_{1-6}$ haloalkyl, or
(g) a group represented by the formula:

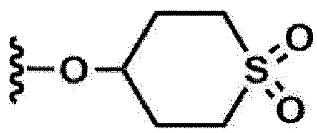

},

or a pharmaceutically acceptable salt thereof.

**12.** The compound of any one of Claims 1 to 8, wherein ring Cy is

(1) cyclohexyl (wherein the cyclohexyl is optionally substituted by 1 or 2 substituents independently selected from the group consisting of

(a) hydroxy,
(b) cyano,

(c) oxo,
(d) $C_{1-6}$ alkyl (wherein the alkyl is optionally substituted by

(I) hydroxy,
(II) carboxy,
(III) $-CONH_2$,
(IV) -CO-$C_{1-4}$ alkoxy,
(V) $-SO_2$-$C_{1-4}$ alkyl, or
(VI) a group represented by the formula:

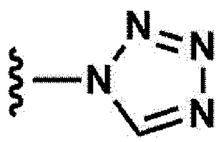

), 

(e) $C_{1-6}$ alkoxy (wherein the alkoxy is optionally substituted by hydroxy or $-SO_2$-$C_{1-4}$ alkyl),
(f) carboxy,
(g) -CO-$NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl, or -CO-$C_{1-6}$ alkyl (wherein the alkyl is optionally substituted by cyano)},
(i) -O-$C_{1-6}$ haloalkyl,
(j) a group represented by the formula:

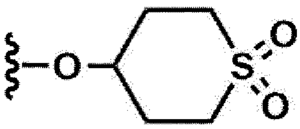

,

(k) a group represented by the formula:

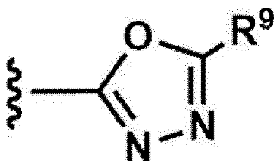

{wherein $R^9$ is $C_{1-4}$ alkyl (wherein the alkyl is optionally substituted by $C_{1-4}$ alkoxy)}, and
(m) a group represented by the formula:

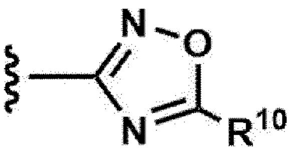

wherein $R^{10}$ is $C_{1-4}$ alkyl)), or

(2) a group represented by the formula:

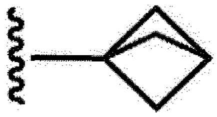

{wherein the group represented by the formula is optionally substituted by

(a) hydroxy,
(b) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

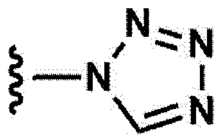

),

(c) $C_{1-6}$ alkoxy (wherein the alkoxy is optionally substituted by hydroxy or $-SO_2-C_{1-4}$ alkyl),
(d) carboxy,
(e) $-CO-C_{1-4}$ alkoxy,
(f) $-O-C_{1-6}$ haloalkyl, or
(g) a group represented by the formula:

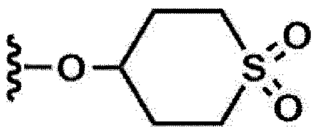

},

or a pharmaceutically acceptable salt thereof.

**13.** The compound of any one of Claims 1 to 8, wherein ring Cy is

(1) a group represented by the formula:

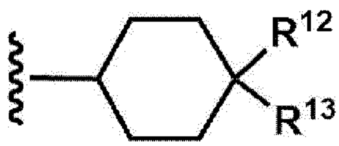

wherein $R^{12}$ and $R^{13}$ are each independently

(a) hydrogen,
(b) hydroxy,
(c) cyano,
(d) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or

(VI) a group represented by the formula:

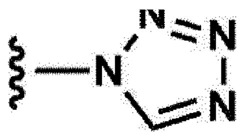
),

(e) $C_{1-6}$ alkoxy (wherein the alkoxy is optionally substituted by hydroxy or $-SO_2-C_{1-4}$ alkyl),
(f) carboxy,
(g) $-CO-NR^5R^6$ [wherein $R^5$ and $R^6$ are each independently $C_{1-4}$ alkyl (wherein the alkyl is optionally substituted by $C_{1-4}$ alkoxy), or $R^5$ and $R^6$ optionally form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by 1 or 2 substituents independently selected from the group consisting of

(I) hydroxy, and
(II) $C_{1-6}$ alkyl (wherein the alkyl is optionally substituted by hydroxy)}],

(h) $-NR^7R^8$ {wherein $R^7$ and $R^8$ are each independently $C_{1-4}$ alkyl or $-CO-C_{1-6}$ alkyl (wherein the alkyl is optionally substituted by cyano)},
(i) $-O-C_{1-6}$ haloalkyl,
(j) a group represented by the formula:

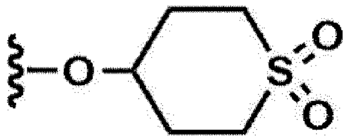

(k) a group represented by the formula:

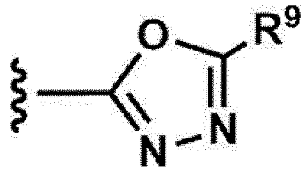

{wherein $R^9$ is $C_{1-4}$ alkyl (wherein the alkyl is optionally substituted by $C_{1-4}$ alkoxy)}, or
(m) a group represented by the formula:

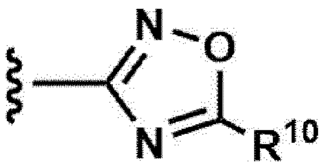

wherein $R^{10}$ is $C_{1-4}$ alkyl), or $R^{12}$ and $R^{13}$ are optionally joined to form oxo), or

(2) a group represented by the formula:

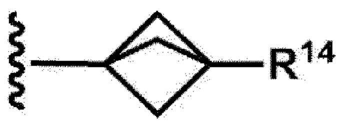

wherein $R^{14}$ is

(a) hydrogen,
(b) hydroxy,
(c) $C_{1-6}$ alkyl (wherein the alkyl is optionally substituted by

(I) hydroxy,
(II) carboxy,
(III) $-CONH_2$,
(IV) $-CO-C_{1-4}$ alkoxy,
(V) $-SO_2-C_{1-4}$ alkyl, or
(VI) a group represented by the formula:

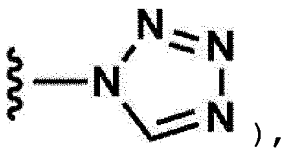

(d) $C_{1-6}$ alkoxy (wherein the alkoxy is optionally substituted by hydroxy or $-SO_2-C_{1-4}$ alkyl),
(e) carboxy,
(f) $-CO-C_{1-4}$ alkoxy,
(g) $-O-C_{1-6}$ haloalkyl, or
(h) a group represented by the formula:

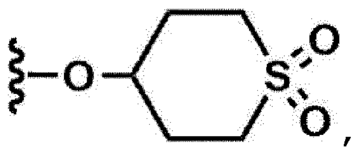

or a pharmaceutically acceptable salt thereof.

**14.** The compound of Claim 1 or 13, which is represented by the formula [IC]:

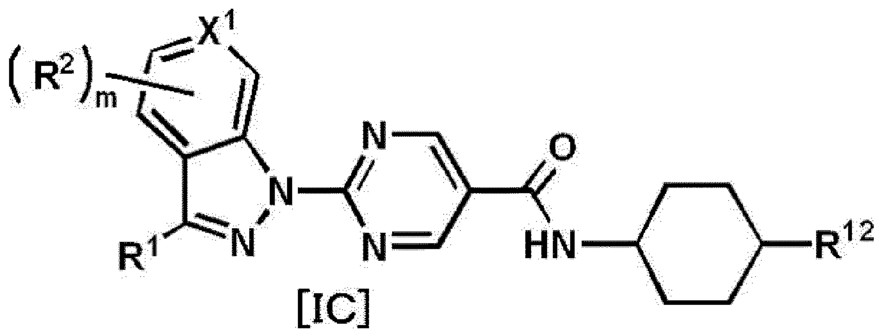

[IC]

wherein

$X^1$, $R^1$, $R^2$ and m are as defined for Claim 1, and
$R^{12}$ is as defined for Claim 13,
or a pharmaceutically acceptable salt thereof.

**15.** The compound of Claim 14, wherein
$R^{12}$ is

(1) hydroxy,
(2) cyano,
(3) $C_{1-6}$ alkyl (wherein the alkyl is optionally substituted by

(a) hydroxy,
(b) carboxy,
(c) $-CONH_2$,
(d) -CO-$C_{1-4}$ alkoxy,
(e) $-SO_2$-$C_{1-4}$ alkyl, or
(f) a group represented by the formula:

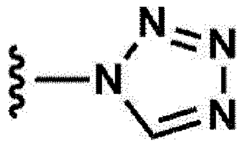

),

(4) $C_{1-6}$ alkoxy (wherein the alkoxy is optionally substituted by hydroxy or $-SO_2$-$C_{1-4}$ alkyl),
(5) -CO-$NR^5R^6$ [wherein $R^5$ and $R^6$ form, together with the nitrogen atom bonded thereto, 4- to 6-membered heterocycloalkyl containing 1 or 2 hetero atoms independently selected from the group consisting of nitrogen and oxygen atoms {wherein the heterocycloalkyl is optionally substituted by $C_{1-6}$ alkyl (wherein the alkyl is optionally substituted by hydroxy)}],
(6) $-NR^7R^8$ {wherein $R^7$ and $R^8$ are each independently $C_{1-4}$ alkyl or -CO-$C_{1-6}$ alkyl (wherein the alkyl is optionally substituted by cyano) },
(7) a group represented by the formula:

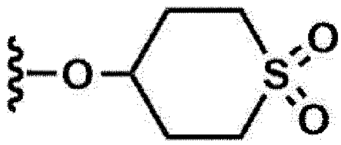

,

(8) a group represented by the formula:

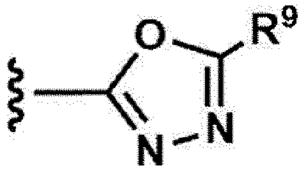

{wherein $R^9$ is $C_{1-4}$ alkyl (wherein the alkyl is optionally substituted by $C_{1-4}$ alkoxy)}, or
(9) a group represented by the formula:

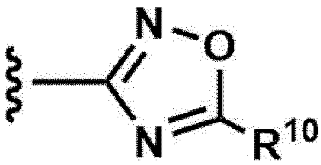

wherein $R^{10}$ is $C_{1-4}$ alkyl,

or a pharmaceutically acceptable salt thereof.

**16.** The compound of Claim 1 or 13, which is represented by the formula [ID] :

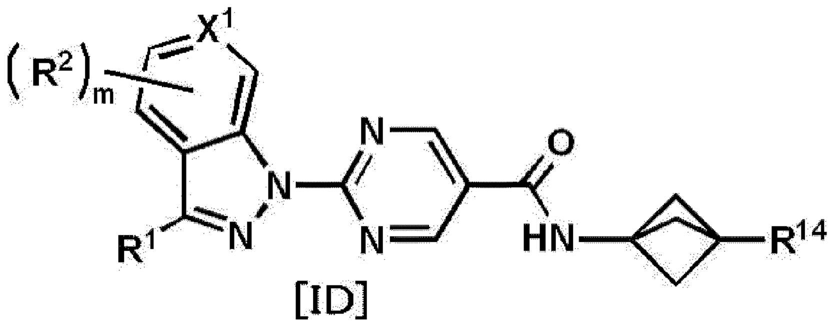

[ID]

wherein

$X^1$, $R^1$, $R^2$ and m are as defined for Claim 1, and
$R^{14}$ is as defined for Claim 13,
or a pharmaceutically acceptable salt thereof.

**17.** The compound of Claim 16, wherein $R^{14}$ is $C_{1-6}$ alkyl (wherein the alkyl is optionally substituted by hydroxy), or a pharmaceutically acceptable salt thereof.

**18.** A compound selected from the group consisting of the following structural formulas:

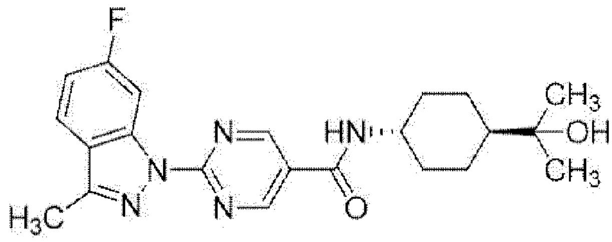

,

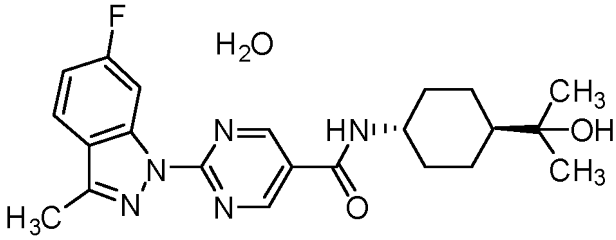

,

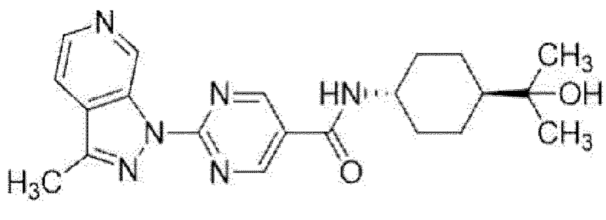

,

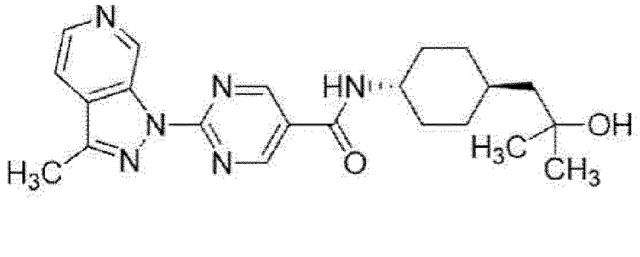

,

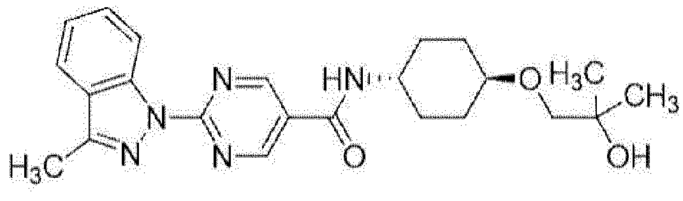

,

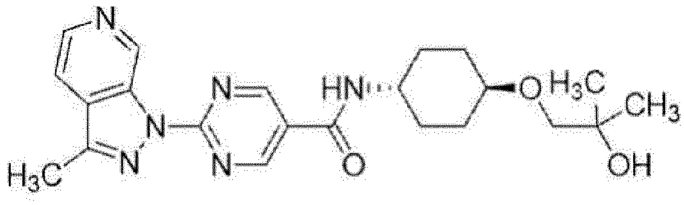

,

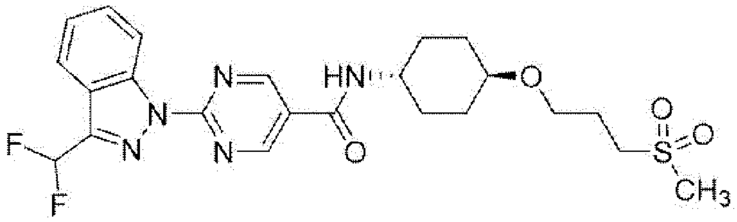

,

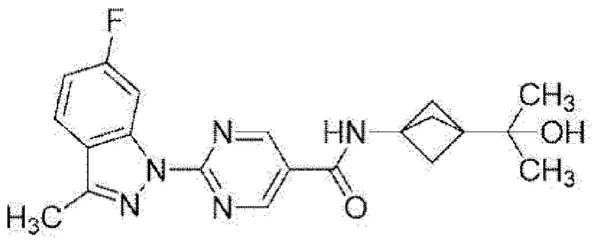

,

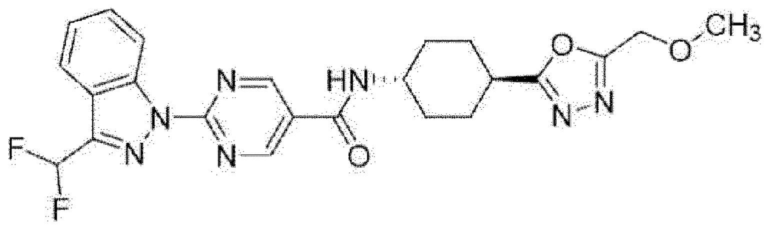

,

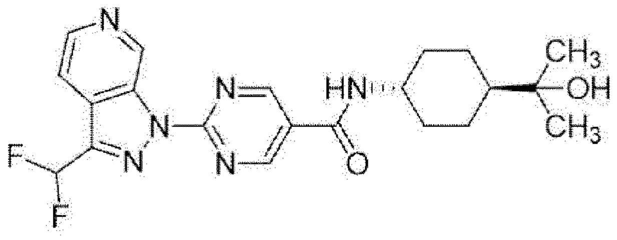

,

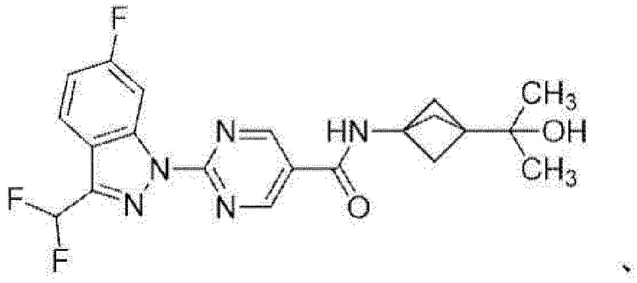

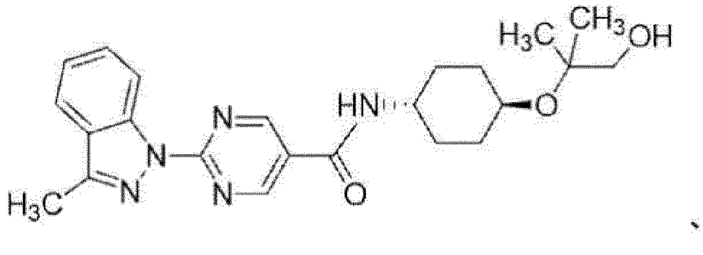

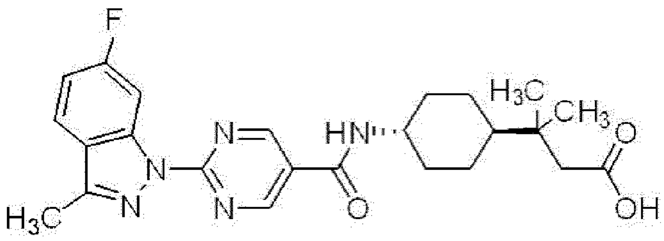

and

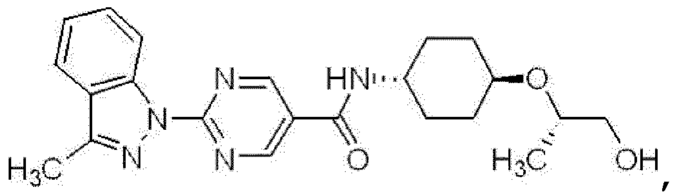

or a pharmaceutically acceptable salt thereof.

**19.** A pharmaceutical composition comprising the compound of any one of Claims 1 to 18 or a pharmaceutically acceptable salt thereof, and a pharmaceutically acceptable carrier.

**20.** An H-PGDS inhibitor comprising the compound of any one of Claims 1 to 18 or a pharmaceutically acceptable salt thereof.

**21.** A therapeutic or prophylactic agent for a disease selected from the group consisting of peripheral arterial diseases, cardiovascular diseases, allergic asthma, chronic obstructive pulmonary diseases, allergic rhinitis, sarcopenia, and Duchenne muscular dystrophy, the agent comprising the compound of any one of Claims 1 to 18 or a pharmaceutically acceptable salt thereof.

**22.** The therapeutic or prophylactic agent of Claim 21, wherein the peripheral arterial disease is intermittent claudication or comprehensive severe chronic lower extremity ischemia based on PAD.

**23.** A method for inhibiting H-PGDS in a mammal, comprising administering a pharmaceutically effective amount of the compound of any one of Claims 1 to 18 or a pharmaceutically acceptable salt thereof to the mammal.

**24.** A method for treating or preventing a disease selected from the group consisting of peripheral arterial diseases,

cardiovascular diseases, allergic asthma, chronic obstructive pulmonary diseases, allergic rhinitis, sarcopenia, and Duchenne muscular dystrophy in a mammal, comprising administering a pharmaceutically effective amount of the compound of any one of Claims 1 to 18 or a pharmaceutically acceptable salt thereof to the mammal.

**25.** The method of Claim 24, wherein the peripheral arterial disease is intermittent claudication or comprehensive severe chronic lower extremity ischemia based on PAD.

**26.** Use of the compound of any one of Claims 1 to 18 or a pharmaceutically acceptable salt thereof in producing an H-PGDS inhibitor.

**27.** Use of the compound of any one of Claims 1 to 18 or a pharmaceutically acceptable salt thereof in producing a therapeutic or prophylactic agent for a disease selected from the group consisting of peripheral arterial diseases, cardiovascular diseases, allergic asthma, chronic obstructive pulmonary diseases, allergic rhinitis, sarcopenia, and Duchenne muscular dystrophy.

**28.** The use of Claim 27, wherein the peripheral arterial disease is intermittent claudication or comprehensive severe chronic lower extremity ischemia based on PAD.

**29.** The compound of any one of Claims 1 to 18 or a pharmaceutically acceptable salt thereof for use in inhibiting H-PGDS.

**30.** The compound of any one of Claims 1 to 18 or a pharmaceutically acceptable salt thereof for use in the treatment or prophylaxis of a disease selected from the group consisting of peripheral arterial diseases, cardiovascular diseases, allergic asthma, chronic obstructive pulmonary diseases, allergic rhinitis, sarcopenia, and Duchenne muscular dystrophy.

**31.** The compound of Claim 30 or a pharmaceutically acceptable salt thereof, wherein the peripheral arterial disease is intermittent claudication or comprehensive severe chronic lower extremity ischemia based on PAD.

## INTERNATIONAL SEARCH REPORT

International application No.
**PCT/JP2023/006921**

**A. CLASSIFICATION OF SUBJECT MATTER**

***C07D 403/04***(2006.01)i; ***A61K 31/416***(2006.01)i; ***A61K 31/4439***(2006.01)i; ***A61K 31/444***(2006.01)i; ***A61K 31/506***(2006.01)i; ***A61K 31/5377***(2006.01)i; ***A61P 9/00***(2006.01)i; ***A61P 11/00***(2006.01)i; ***A61P 11/06***(2006.01)i; ***A61P 21/00***(2006.01)i; ***A61P 37/08***(2006.01)i; ***C07D 231/56***(2006.01)i; ***C07D 401/04***(2006.01)i; ***C07D 401/14***(2006.01)i; ***C07D 403/14***(2006.01)i; ***C07D 405/14***(2006.01)i; ***C07D 409/14***(2006.01)i; ***C07D 413/14***(2006.01)i; ***C07D 451/04***(2006.01)i; ***C07D 471/04***(2006.01)i

FI: C07D403/04 CSP; A61K31/506; A61P9/00; A61P11/00; A61P11/06; A61P37/08; A61P21/00; C07D413/14; C07D471/04 106Z; C07D231/56 A; C07D401/04; C07D409/14; C07D401/14; C07D403/14; C07D451/04; C07D405/14; A61K31/416; A61K31/4439; A61K31/444; A61K31/5377

According to International Patent Classification (IPC) or to both national classification and IPC

**B. FIELDS SEARCHED**

Minimum documentation searched (classification system followed by classification symbols)

C07D403/04; A61K31/416; A61K31/4439; A61K31/444; A61K31/506; A61K31/5377; A61P9/00; A61P11/00; A61P11/06; A61P21/00; A61P37/08; C07D231/56; C07D401/04; C07D401/14; C07D403/14; C07D405/14; C07D409/14; C07D413/14; C07D451/04; C07D471/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2021/256569 A1 (SATO PHARMACEUTICAL CO., LTD.) 23 December 2021 (2021-12-23)<br>claims, examples | 1-31 |
| A | TRUJILLO, John I. et al. Investigation of the binding pocket of human hematopoietic prostaglandin (PG) D2 synthase (hH-PGDS): A tale of two waters. Bioorganic & Medicinal Chemistry Letters. 2012, 22(11), pages 3795-3799, DOI: 10.1016/j.bmcl.2012.04.004<br>entire text | 1-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 April 2023** | **18 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

## INTERNATIONAL SEARCH REPORT

International application No.
**PCT/JP2023/006921**

**C. DOCUMENTS CONSIDERED TO BE RELEVANT**

| Category* | Citation of document, with indication, where appropriate, of the relevant passages | Relevant to claim No. |
|---|---|---|
| A | CHRIST, Angelika N. et al. Development and Characterization of New Inhibitors of the Human and Mouse Hematopoietic Prostaglandin D2 Synthases. Journal of Medicinal Chemistry. 2010, 53(15), pages 5536-5548, DOI: 10.1021/jm100194a<br>entire text, compound 29 | 1-31 |
| A | JP 2021-534166 A (AMGEN INC.) 09 December 2021 (2021-12-09)<br>claims, examples 1-27 | 1-31 |
| P, A | WO 2022/194781 A1 (CHIESI FARMACEUTICI S.P.A.) 22 September 2022 (2022-09-22)<br>intermediate 9f | 1-31 |

Form PCT/ISA/210 (second sheet) (January 2015)

**INTERNATIONAL SEARCH REPORT**
**Information on patent family members**

International application No.
**PCT/JP2023/006921**

| Patent document cited in search report | Publication date (day/month/year) | Patent family member(s) | Publication date (day/month/year) |
|---|---|---|---|
| WO 2021/256569 A1 | 23 December 2021 | (Family: none) | |
| JP 2021-534166 A | 09 December 2021 | US 2022/0363694 A1<br>claims, examples 1-27<br>WO 2020/036940 A1<br>EP 3837262 A1 | |
| WO 2022/194781 A1 | 22 September 2022 | WO 2022/194779 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011052628 A
- WO 2019203296 A


### Non-patent literature cited in the description


- **MCDERMOTT MM et al.** *JACC Cardiovasc Imaging.*, June 2013, vol. 6 (6), 687-94
- **STEG PG et al.** *Circulation.*, 29 June 2010, vol. 121 (25), 2724-30
- **MATSUOKA T et al.** *Science*, 17 March 2000, vol. 287 (5460), 2013-7
- **GEORGE L et al.** *Ther Adv Chronic Dis.*, January 2016, vol. 7 (1), 34-51
- **SARGENT C et al.** *Br J Pharmacol*, 2009, vol. 7, 003P
- **OKINAGA T et al.** *Acta Neuropathol.*, October 2002, vol. 104 (4), 377-84
- **MOHRI I et al.** *Am J Pathol.*, May 2009, vol. 174 (5), 1735-44
- **HAMBURG NM et al.** *Circulation J*, 2017, vol. 81, 281-289
- **TRAN B**. *Heart*, 2021, vol. 107, 1835-1843
- **KAJIWARA D et al.** *European Journal of Pharmacology*, 2011, vol. 667, 389-395
- **BERGE et al.** *J. Pharm. Sci.*, 1977, vol. 66, 1-19
- **STAHL et al.** Handbook of Pharmaceutical Salts: Properties, Selection, and Use. Wiley-VCH, 2002
- **PAULEKUHN et al.** *J. Med. Chem.*, 2007, vol. 50, 6665-6672
- *CHEMICAL ABSTRACTS*, 849924-76-1
- *CHEMICAL ABSTRACTS*, 849924-77-2